(19) Europäisches Patentamt
European Patent Office
Office européen des brevets

(11) EP 2 617 837 A2

# (12) EUROPEAN PATENT APPLICATION

(43) Date of publication:
**24.07.2013 Bulletin 2013/30**

(51) Int Cl.:
***C12Q 1/68*** *(2006.01)*

(21) Application number: **13160408.4**

(22) Date of filing: **06.06.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **08.06.2007 US 942937 P**

(62) Document number(s) of the earlier application(s) in accordance with Art. 76 EPC:
**08770385.6 / 2 167 687**

(71) Applicants:
- **Biogen Idec MA Inc.**
  **Cambridge, Massachusetts 02142 (US)**
- **The Feinstein Institute for Medical Research**
  **Manhasset, NY 11030 (US)**

(72) Inventors:
- **Carulli, John, P.**
  **Southborough, MA 01772 (US)**
- **Gregersen, Peter, K.**
  **Larchmont, NY 10538 (US)**
- **Batliwalla, Franak**
  **Port Washington, NY 11050 (US)**
- **Bienkowska, Jadwiga**
  **Cambridge, MA 02139 (US)**
- **Liu, Chunyu**
  **Sharon, MA 02067 (US)**

(74) Representative: **Pohlman, Sandra M.**
**Df-mp**
**Fünf Höfe**
**Theatinerstraße 16**
**80333 München (DE)**

Remarks:
- This application was filed on 21-03-2013 as a divisional application to the application mentioned under INID code 62.
- The references to the drawing(s) Fig.: 1-10 are deemed to be deleted (Rule 56(4) EPC).
- A request for correction of the description has been filed pursuant to Rule 139 EPC. A decision on the request will be taken during the proceedings before the Examining Division (Guidelines for Examination in the EPO, A-V, 3.).

## (54) Biomarkers for predicting anti-TNF responsiveness or non-responsiveness

(57) The present disclosure provides biomarkers that are predictive a subject's responsiveness or non-responsiveness to an anti-TNF therapy. The biomarkers, compositions, and methods described herein are useful in selecting appropriate treatment modalities (e.g., an anti-TNF therapy or a non-anti-TNF therapy) for a subject suffering from a disease such as an immune disorder.

EP 2 617 837 A2

## Description

### CROSS-REFERENCE TO RELATED APPLICATIONS

This application claims priority to U.S. Provisional Application No. 60/942,937, filed on June 8, 2007, the contents of which are hereby incorporated by reference in their entirety.

### STATEMENT REGARDING FEDERALLY SPONSORED RESEARCH OR

### DEVELOPMENT

The research described in this application was supported by grant number 1-AR-1-2256 from the National Institute of Arthritis and Musculoskeletal and Skin Diseases of the National Institutes of Health. The Government may have certain rights in the invention.

### BACKGROUND

Tumor Necrosis Factor-Alpha (TNF) is a cytokine that plays a key role in the pathogenesis of inflammatory diseases such as rheumatoid arthritis. Blockade of TNF signaling through the use of anti-TNF monoclonal antibodies (e.g., adalimumab or infliximab) or TNF receptor fusion proteins (e.g., etaneracept) can be used to ameliorate symptoms of certain inflammatory diseases. While anti-TNF treatments have been tremendously successful, they do not produce significant clinical responses in all patients who receive them.

Identification of a patients as being likely or unlikely to respond to anti-TNF treatment is a critical for optimal patient management. Patients exhibiting biomarkers suggesting they are unlikely to respond to anti-TNF treatment can be steered to other therapies immediately (i.e., without administering an anti-TNF treatment) or upon failure with a first anti-TNF treatment.

### SUMMARY

The present invention is based, at least in part, on the discovery of biomarkers that are predictive of a subject's responsiveness or non-responsiveness to an anti-TNF therapy. For example, the expression level of one or more of the genes depicted in Table 1 or the presence of one or more of the single nucleotide polymorphisms depicted in Tables 2-4 or 13 can predict the likelihood that a given subject will or will not respond to an anti-TNF therapy. The biomarkers, compositions, and methods described herein are thus useful in selecting appropriate treatment modalities (e.g., an anti-TNF therapy or a non-anti-TNF therapy) for a subject suffering from a disease such as an immune or inflammatory disorder (e.g., rheumatoid arthritis or Crohn's disease).

In one aspect, the disclosure provides a method of treating an immune disorder, which method includes the step of administering to a subject in need thereof an effective amount of a therapy comprising an anti-TNF agent, wherein the subject has been identified as having at least one (e.g., at least two, at least three, at least four, at least five, at least six, at least seven, at least eight, at least nine, at least 10, at least 11, at least 12, at least 13, at least 14, at least 15, at least 16, at least 17, at least 18, at least 19, at least 20, at least 21, at least 22, or at least 24) of (i) an elevated expression level, as compared to a healthy individual, of one or more of ANKIB 1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1, or (ii) a reduced expression level, as compared to a healthy individual, of one or more of ANKRD12, CAMK2G-, CASP5, CXorf52, DNAH1, EEA1, FAM44A, FOXJ3, HDAC4, MNT, MXRA7, PTCH1, SEL1L, or SFRS2.

In another aspect, the disclosure provides a method of treating an immune disorder, which method includes the step of administering to a subject in need thereof an effective amount of a therapy comprising a non-anti-TNF agent, wherein the subject has been identified as having at least one (e.g., at least two, at least three, at least four, at least five, at least six, at least seven, at least eight, at least nine, at least 10, at least 11, at least 12, at least 13, at least 14, at least 15, at least 16, at least 17, at least 18, at least 19, at least 20, at least 21, at least 22, or at least 24) of (i) an elevated expression level, as compared to a healthy individual, of one or more of ANKRD12, CAMK2G-, CASP5, CXorf52, DNAH1, EEA1, FAM44A, FOXJ3, HDAC4, MNT, MXRA7, PTCH1, SEL1L, or SFRS2, or (ii) a reduced expression level, as compared to a healthy individual, of one or more of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1.

In some embodiments of either of the methods above, the subject has been identified as having elevated or reduced expression levels, as compared to a healthy individual, of at least two (e.g., at least three, at least four, at least five, at least six, at least seven, at least eight, at least nine, at least 10, at least 11, at least 12, at least 13, at least 14, at least 15, at least 16, at least 17, at least 18, at least 19, at least 20, at least 21, at least 22, at least 23, or at least 24 or more) genes selected from the group consisting of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2. The at least five genes can include, e.g., ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6. The at least five genes can include, e.g., CLTB, COL4A3BP, CXorf52, FAM44A, MXRA7, PGK1, SFRS2, or YIPF6. The at least eight genes can include, e.g., ANKIB 1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and/or YIPF6. The at least eight genes can consist of, or include, e.g., CLTB, COL4A3BP, CXorf52, FAM44A, MXRA7, PGK1, SFRS2, and/or YIPF6. The at least 24 genes can consist of, or include, e.g., ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6.

In another aspect, the disclosure features a method of predicting the response of a subject to a therapy comprising an anti-TNF agent. The method includes the steps of: providing a biological sample obtained from a subject that has an immune disorder; and measuring the expression level of one or more genes in the biological sample, wherein the one or more genes comprise at least one (e.g., at least two, at least three, at least four, at least five, at least six, at least seven, at least eight, at least nine, at least 10, at least 11, at least 12, at least 13, at least 14, at least 15, at least 16, at least 17, at least 18, at least 19, at least 20, at least 21, at least 22, or at least 24) gene selected from the group consisting of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2, wherein at least one of (i) an elevated expression level, as compared to a healthy individual, of one or more of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1, or (ii) a reduced expression level, as compared to a healthy individual, of one or more of ANKRD12, CAMK2G-, CASP5, CXorf52, DNAH1, EEA1, FAM44A, FOXJ3, HDAC4, MNT, MXRA7, PTCH1, SEL1L, or SFRS2 predicts that the subject will respond to a therapy comprising an anti-TNF agent, and wherein at least one of (i) an elevated expression level, as compared to a healthy individual, of one or more of ANKRD12, CAMK2G-, CASP5, CXorf52, DNAH1, EEA1, FAM44A, FOXJ3, HDAC4, MNT, MXRA7, PTCH1, SEL1L, or SFRS2, or (ii) a reduced expression level, as compared to a healthy individual, of one or more of ANKIB 1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 predicts that the subject will not respond to a therapy comprising an anti-TNF agent. The RNA or protein expression of the one or more genes can be measured. The RNA expression level of the one or more genes can be measured using, e.g., microarray analysis and/or quantitative polymerase chain reaction.

In some embodiments, the method includes determining that the expression level of one or more of (i) ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 are elevated, as compared to a healthy individual, or (ii) ANKRD12, CAMK2G-, CASP5, CXorf52, DNAH1, EEA1, FAM44A, FOXJ3, HDAC4, MNT, MXRA7, PTCH1, SEL1L, or SFRS2 are reduced, as compared to a healthy individual; and selecting a therapy comprising an anti-TNF agent for the subject. The method can further include administering the therapy comprising an anti-TNF agent to the subject.

In some embodiments, the method can further include creating a record indicating that the subject is likely to respond to a therapy comprising an anti-TNF agent, if the expression level of one or more of (i) ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 are elevated, as compared to a healthy individual, or (ii) ANKRD12, CAMK2G-, CASP5, CXorf52, DNAH1, EEA1, FAM44A, FOXJ3, HDAC4, MNT, MXRA7, PTCH1, SEL1L, or SFRS2 are reduced, as compared to a healthy individual. The record can be created, e.g., on a computer readable medium.

In some embodiments, the method can include determining that the expression level of one or more of (i) ANKRD12, CAMK2G-, CASP5, CXorf52, DNAH1, EEA1, FAM44A, FOXJ3, HDAC4, MNT, MXRA7, PTCH1, SEL1L, or SFRS2 are elevated, as compared to a healthy individual, or (ii) ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 are reduced, as compared to a healthy individual; and selecting a therapy comprising a non-anti-TNF agent for the subject. The method can further include administering the therapy comprising a non-anti-TNF agent to the subject.

In some embodiments, the method can further include creating a record indicating that the subject is not likely to respond to a therapy comprising an anti-TNF agent, if the expression level of one or more of (i) ANKRD12, CAMK2G-, CASP5, CXorf52, DNAH1, EEA1, FAM44A, FOXJ3, HDAC4, MNT, MXRA7, PTCH1, SEL1L, or SFRS2 are elevated, as compared to a healthy individual, or (ii) ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 are reduced, as compared to a healthy individual. The record can be created, e.g., on a computer readable medium.

In some embodiments of any of the methods described herein, the expression level of at least one gene selected from the group consisting of ANKIB 1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2 is elevated or reduced, as compared to a healthy individual, by at least about 1.5 fold (e.g., at least about 2 fold, at least about 2.5 fold, at least about 3.0 fold, at least about 3.5 fold, at least about 4.0 fold, or at least about 5 fold or more). The methods described herein can include measuring the expression level of at least two (e.g., at least three, at least four, at least five, at least six, at least seven, at least eight, at least nine, at least 10, at least 11, at least 12, at least 13, at least 14, at least 15, at least 16, at least 17, at least 18, at least 19, at least 20, at least 21, at least 22, or at least 24) genes selected from the group consisting of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2. The at least five genes can include, e.g., ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6. The at least five genes can include, e.g., CLTB, COL4A3BP, CXorf52, FAM44A, MXRA7, PGK1, SFRS2, or YIPF6. The at least eight genes can include, e.g., ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and/or YIPF6. The at least eight genes can consist of, or include, e.g., CLTB, COL4A3BP, CXorf52, FAM44A, MXRA7, PGK1, SFRS2, and/or YIPF6. The at least 24 genes can consist of, or include, e.g., ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6.

In another aspect, the disclosure provides a method of predicting the response of a subject to therapy comprising an anti-TNF agent, which method includes the step of assessing the expression level (e.g., the RNA or protein expression level) of one or more genes in a biological sample from a subject, wherein the one or more genes comprise at least one (e.g., at least two, at least three, at least four, at least five, at least six, at least seven, at least eight, at least nine, at least 10, at least 11, at least 12, at least 13, at least 14, at least 15, at least 16, at least 17, at least 18, at least 19, at least 20, at least 21, at least 22, or at least 24) gene selected from the group consisting of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2, wherein an elevated expression level of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 or a reduced expression level of ANKRD12, CAMK2G-, CASP5, CXorf52, DNAH1, EEA1, FAM44A, FOXJ3, HDAC4, MNT, MXRA7, PTCH1, SEL1L, or SFRS2 predicts that the subject will respond to an anti-TNF therapy, and wherein an elevated expression level of ANKRD12, CAMK2G-, CASP5, CXorf52, DNAH1, EEA1, FAM44A, FOXJ3, HDAC4, MNT, MXRA7, PTCH1, SEL1L, or SFRS2 or a reduced expression level of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 predicts that the subject will not respond to an anti-TNF therapy.

In some embodiments, the method can include determining that the expression level of one or more (e.g., two or more, three or more, four or more, five or more, six or more, seven or more, eight or more, nine or more, 10 or more, 11 or more, 12 or more, 13 or more, 14 or more, 15 or more, 16 or more, 17 or more, 18 or more, 19 or more, 20 or more, 21 or more, or 22 or more) of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 are elevated or ANKRD12, CAMK2G-, CASP5, CXorf52, DNAH1, EEA1, FAM44A, FOXJ3, HDAC4, MNT, MXRA7, PTCH1, SEL1L, or SFRS2 are reduced; and predicting that the subject will respond to an anti-TNF therapy. In some embodiments, the methods include, after predicting that the subject will respond to the anti-TNF therapy, administering to the subject the anti-TNF therapy.

In some embodiments, the method can include determining that the expression level of one or more (e.g., two or more, three or more, four or more, five or more, six or more, seven or more, eight or more, nine or more, 10 or more, 11 or more, 12 or more, 13 or more, 14 or more, 15 or more, 16 or more, 17 or more, 18 or more, 19 or more, 20 or more, 21 or more, or 22 or more) of ANKRD12, CAMK2G-, CASP5, CXorf52, DNAH1, EEA1, FAM44A, FOXJ3, HDAC4, MNT, MXRA7, PTCH1, SEL1L, or SFRS2 are elevated or ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 are reduced; and predicting that the subject will not respond to an anti-TNF therapy. In some embodiments, the methods include, after predicting that the subject will not respond to the anti-TNF therapy, administering to the subject a non-anti-TNF therapy.

In yet another aspect, the disclosure features a method of treating an immune disorder. The method includes the step of administering to a subject in need thereof an effective amount of a therapy comprising an anti-TNF agent, wherein the subject has been identified as having one or more (e.g., two or more, three or more, four or more, five or more, six or more, seven or more, eight or more, nine or more, ten or more, 11 or more, 12 or more, 13, or more, 14 or more, 15 or more, 16 or more, 17 or more, 18 or more, 19 or more, 20 or more, or all of the) single nucleotide polymorphisms (SNPs) genotypes selected from the group consisting of rs11778767 (A/A), rs11780500 (A/A), rs 11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), and rs868856 (G/G).

In another aspect, the disclosure features a method of treating an immune disorder, which method includes the step of administering to a subject in need thereof an effective amount of a therapy comprising a non-anti-TNF agent, wherein the subject has been identified as having one or more (e.g., two or more, three or more, four or more, five or more, six or more, seven or more, eight or more, nine or more, ten or more, 11 or more, 12 or more, 13, or more, 14 or more, 15 or more, 16 or more, 17 or more, 18 or more, 19 or more, 20 or more, or all of the) SNP genotypes selected from the group consisting of rs11778767 (A/G), rs11778767 (G/G), rs11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), rs868856 (A/A), rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), and any of the SNP genotypes depicted in Table 13.

In some embodiments, the subject has been identified as having one or more (e.g., two or more, three or more, four or more, five or more, six or more, seven or more, eight or more, nine or more, ten or more, 11 or more, 12 or more, 13, or more, 14 or more, 15 or more, 16 or more, 17 or more, 18 or more, 19 or more, 20 or more, or all of the) SNP genotypes selected from the group consisting of rs11778767 (A/G), rs11778767 (G/G), rs 11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), and rs868856 (A/A).

In some embodiments, the subject has been identified as having one or more (e.g., two or more, three or more, four or more, five or more, six or more, seven or more, eight or more, nine or more, ten or more, 11 or more, 12 or more, 13, or more, 14 or more, 15 or more, 16 or more, 17 or more, 18 or more, 19 or more, 20 or more, or all of the) SNP genotypes selected from the group consisting of any of rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), and rs6071980 (C/C).

In some embodiments, the subject has been identified as having one or more (e.g., two or more, three or more, four or more, five or more, six or more, seven or more, eight or more, nine or more, ten or more, 11 or more, 12 or more, 13, or more, 14 or more, 15 or more, 16 or more, 17 or more, 18 or more, 19 or more, 20 or more, or all of the) SNP genotypes selected from the group consisting of rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), and rs4803455 (C/C).

In some embodiments, the subject has been identified as having one or more (e.g., two or more, three or more, four or more, five or more, six or more, seven or more, eight or more, nine or more, ten or more, 11 or more, 12 or more, 13, or more, 14 or more, 15 or more, 16 or more, 17 or more, 18 or more, 19 or more, 20 or more, or all of the) SNP genotypes selected from the group consisting of any of the SNP genotypes depicted in Table 13.

In another aspect, the disclosure features a method of treating an immune disorder, which method includes the step of administering to a subject in need thereof an effective amount of a therapy comprising a non-anti-TNF agent, wherein the subject has been identified as having one or more (e.g., two or more, three or more, four or more, five or more, six or more, seven or more, eight or more, nine or more, ten or more, 11 or more, 12 or more, 13, or more, 14 or more, 15 or more, 16 or more, 17 or more, 18 or more, 19 or more, 20 or more, or all of the) SNP genotypes selected from the group consisting of rs 11778767 (A/G), rs11778767 (G/G), rs 11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), or rs868856 (A/A).

In another aspect, the disclosure provides a method of predicting the response of a subject to a therapy comprising an anti-TNF agent. The method includes the steps of: providing a biological sample obtained from a subject that has an immune disorder; and detecting the presence or absence of one or more SNP genotypes in the biological sample, wherein the one or more SNP genotypes comprise at least one (e.g., at least two, at least three, at least four, at least five, at least six, at least seven, at least eight, at least nine, at least 10, at least 11, at least 12, at least 13, at least 14, at least 15, at least 16, at least 17, at least 18, at least 19, or at least 20) SNP genotype selected from the group consisting of rs 11778767 (A/A), rs11780500 (A/A), rs11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), rs868856 (G/G), rs11778767 (A/G), rs11778767 (G/G), rs11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), rs868856 (A/A), rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs 1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), and any of the SNP genotypes depicted in Table 13, wherein the presence of one or more of rs 11778767 (A/A), rs11780500 (A/A), rs 11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), or rs868856 (G/G) predicts that the subject will respond to a therapy comprising an anti-TNF agent, and wherein the presence of one or more of rs11778767 (A/G), rs 11778767 (G/G), rs11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), rs868856 (A/A), rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), or any of the SNP genotypes depicted in Table 13 predicts that the subject will not respond to a therapy comprising an anti-TNF agent.

In another aspect, the disclosure provides a method of predicting the response of a subject to a therapy comprising an anti-TNF agent. The method includes the steps of: providing a biological sample obtained from a subject that has an immune disorder; and detecting the presence or absence of one or more SNP genotypes in the biological sample, wherein the one or more SNP genotypes comprise at least one (e.g., at least two, at least three, at least four, at least five, at least six, at least seven, at least eight, at least nine, at least 10, at least 11, at least 12, at least 13, at least 14, at least 15, at least 16, at least 17, at least 18, at least 19, or at least 20) SNP genotype selected from the group consisting of rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), and any of the SNP genotypes depicted in Table 13, wherein the presence of one or more of any of rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), or any of the SNP genotypes depicted in Table 13 predicts that the subject will not respond to a therapy comprising an anti-TNF agent.

In another aspect, the disclosure provides a method of predicting the response of a subject to a therapy comprising an anti-TNF agent. The method includes the steps of: providing a biological sample obtained from a subject that has an immune disorder; and detecting the presence or absence of one or more SNP genotypes in the biological sample, wherein the one or more SNP genotypes comprise at least one (e.g., at least two, at least three, at least four, at least five, at least six, at least seven, at least eight, at least nine, at least 10, at least 11, at least 12, at least 13, at least 14, at least 15, at least 16, at least 17, at least 18, at least 19, or at least 20) SNP genotype selected from the group consisting of any of rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), and rs6071980 (C/C), wherein the presence of one or more of any of rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), or rs6071980 (C/C) predicts that the subject will not respond to a therapy comprising an anti-TNF agent.

In another aspect, the disclosure provides a method of predicting the response of a subject to a therapy comprising an anti-TNF agent. The method includes the steps of: providing a biological sample obtained from a subject that has an immune disorder; and detecting the presence or absence of one or more SNP genotypes in the biological sample, wherein the one or more SNP genotypes comprise at least one (e.g., at least two, at least three, at least four, at least five, at least six, at least seven, at least eight, at least nine, at least 10, at least 11, at least 12, at least 13, at least 14, at least 15, at least 16, at least 17, at least 18, at least 19, or at least 20) SNP genotype selected from the group consisting of rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), and rs4803455 (C/C), wherein the presence of one or more of any of rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs 1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), or rs4803455 (C/C) predicts that the subject will not respond to a therapy comprising an anti-TNF agent.

In another aspect, the disclosure provides a method of predicting the response of a subject to a therapy comprising an anti-TNF agent. The method includes the steps of: providing a biological sample obtained from a subject that has an immune disorder; and detecting the presence or absence of one or more SNP genotypes in the biological sample, wherein the one or more SNP genotypes comprise at least one (e.g., at least two, at least three, at least four, at least five, at least six, at least seven, at least eight, at least nine, at least 10, at least 11, at least 12, at least 13, at least 14, at least 15, at least 16, at least 17, at least 18, at least 19, or at least 20) SNP genotype selected from the group consisting of any of the SNP genotypes depicted in Table 13, wherein the presence of one or more of any of the SNP genotypes depicted in Table 13 predicts that the subject will not respond to a therapy comprising an anti-TNF agent.

In another aspect, the disclosure provides a method of predicting the response of a subject to a therapy comprising an anti-TNF agent. The method includes the steps of: providing a biological sample obtained from a subject that has an immune disorder; and detecting the presence or absence of one or more SNP genotypes in the biological sample, wherein the one or more SNP genotypes comprise at least one (e.g., at least two, at least three, at least four, at least five, at least six, at least seven, at least eight, at least nine, at least 10, at least 11, at least 12, at least 13, at least 14, at least 15, at least 16, at least 17, at least 18, at least 19, or at least 20) SNP genotype selected from the group consisting of rs11778767 (A/A), rs11780500 (A/A), rs11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), rs868856 (G/G), rs11778767 (A/G), rs11778767 (G/G), rs11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), and rs868856 (A/A), wherein the presence of one or more of rs11778767 (A/A), rs11780500 (A/A), rs11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), or rs868856 (G/G) predicts that the subject will respond to a therapy comprising an anti-TNF agent, and wherein the presence of one or more of rs 11778767 (A/G), rs11778767 (G/G), rs11780500 (A/G), rs 1422422 (A/G), rs 1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), or rs868856 (A/A) predicts that the subject will not respond to a therapy comprising an anti-TNF agent.

In some embodiments, the method can include detecting the presence of one or more of rs11778767 (A/A), rs11780500 (A/A), rs 11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), or rs868856 (G/G); and selecting a therapy comprising an anti-TNF agent for the subject. The method can further include administering the therapy comprising an anti-TNF agent to the subject.

In some embodiments, the method can include creating a record indicating that the subject is likely to respond to a therapy comprising an anti-TNF agent, if one or more of rs11778767 (A/A), rs 11780500 (A/A), rs 11780500 (G/G), rs1422422 (A/A), rs1441209 (A/A), rs 1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), or rs868856 (G/G) are present. The record can be created on a computer readable medium.

In some embodiments, the method can include detecting the presence of one or more of rs 11778767 (A/G), rs11778767 (G/G), rs 11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), or rs868856 (A/A); and selecting a therapy comprising a non-anti-TNF agent for the subject. The method can further include administering the therapy comprising a non-anti-TNF agent to the subject.

In some embodiments, the method can include detecting the presence of one or more of rs 11778767 (A/G), rs 11778767 (G/G), rs 11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), rs868856 (A/A), rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), or any of the SNP genotypes depicted in Table 13; and selecting a therapy comprising a non-anti-TNF agent for the subject. The method can further include administering the therapy comprising a non-anti-TNF agent to the subject.

In some embodiments, the method can include detecting the presence of one or more of any of rs983332 (A/C), rs928655 (A/G), rs13393173 (A/G), rs437943 (A/G), rs 10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 ((C/T), rs 1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), or any of the SNP genotypes depicted in Table 13; and selecting a therapy comprising a non-anti-TNF agent for the subject. The method can further include administering the therapy comprising a non-anti-TNF agent to the subject.

In some embodiments, the method can include detecting the presence of one or more of any of rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), or rs6071980 (C/C); and selecting a therapy comprising a non-anti-TNF agent for the subject. In some embodiments, the method can include detecting the presence of one or more of rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), or rs4803455 (C/C); and selecting a therapy comprising a non-anti-TNF agent for the subject. In some embodiments, the method can include detecting the presence of one or more of any of the SNP genotypes depicted in Table 13; and selecting a therapy comprising a non-anti-TNF agent for the subject. The method can further include administering the therapy comprising a non-anti-TNF agent to the subject.

In some embodiments, the method can include creating a record indicating that the subject is not likely to respond to a therapy comprising an anti-TNF agent, if one or more of rs 11778767 (A/G), rs11778767 (G/G), rs11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), rs868856 (A/A), rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), or any of the SNP genotypes depicted in Table 13 are present. The record can be created on a computer readable medium.

In some embodiments, the method can include creating a record indicating that the subject is not likely to respond to a therapy comprising an anti-TNF agent, if one or more of any of rs983332 (A/C), rs928655 (A/G), rs13393173 (A/G), rs437943 (A/G), rs 10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), or any of the SNP genotypes depicted in Table 13 are present. The record can be created on a computer readable medium.

In some embodiments, the method can include creating a record indicating that the subject is not likely to respond to a therapy comprising an anti-TNF agent, if one or more of any of rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs983332 (A/A), rs928655 (A/A), rs13393173 (A/A), rs437943 (G/G), rs 10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), or rs6071980 (C/C) are present. In some embodiments, the method can include creating a record indicating that the subject is not likely to respond to a therapy comprising an anti-TNF agent, if one or more of rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), or rs4803455 (C/C) are present. In some embodiments, the method can include creating a record indicating that the subject is not likely to respond to a therapy comprising an anti-TNF agent, if one or more of any of the SNP genotypes depicted in Table 13 are present. The record can be created on a computer readable medium.

In some embodiments, the method can include creating a record indicating that the subject is not likely to respond to a therapy comprising an anti-TNF agent, if one or more of rs 11778767 (A/G), rs 11778767 (G/G), rs 11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), or rs868856 (A/A) are present. The record can be created on a computer readable medium.

In some embodiments, the subject can be one identified as having two or more (e.g., three or more, four or more, five or more, six or more, seven or more, eight or more, nine or more, 10 or more, 11 or more, 12 or more, 13 or more, 14 or more, 15 or more, 16 or more, 17 or more, 18 or more, 19 or more, 20 or more, or all of the) SNP genotypes selected from the group consisting of rs11778767 (A/A), rs11780500 (A/A), rs11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), and rs868856 (G/G).

In some embodiments, the subject can be one identified as having two or more (e.g., three or more, four or more, five or more, six or more, seven or more, eight or more, nine or more, 10 or more, 11 or more, 12 or more, 13 or more, 14 or more, 15 or more, 16 or more, 17 or more, 18 or more, 19 or more, 20 or more, or all of the) SNP genotypes selected from the group consisting of rs 11778767 (A/G), rs11778767 (G/G), rs 11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), rs868856 (A/A), rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), and any of the SNP genotypes depicted in Table 13.

In some embodiments, the subject can be one identified as having two or more (e.g., three or more, four or more, five or more, six or more, seven or more, eight or more, nine or more; 10 or more, 11 or more, 12 or more, 13 or more, 14 or more, 15 or more, 16 or more, 17 or more, 18 or more, 19 or more, 20 or more, or all of the) SNP genotypes selected from the group consisting of rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), and any of the SNP genotypes depicted in Table 13.

In some embodiments, the subject can be one identified as having two or more (e.g., three or more, four or more, five or more, six or more, seven or more, eight or more, nine or more, 10 or more, 11 or more, 12 or more, 13 or more, 14 or more, 15 or more, 16 or more, 17 or more, 18 or more, 19 or more, 20 or more, or all of the) SNP genotypes selected from the group consisting of rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), and rs6071980 (C/C).

In some embodiments, the subject can be one identified as having two or more (e.g., three or more, four or more, five or more, six or more, seven or more, eight or more, nine or more, 10 or more, 11 or more, 12 or more, 13 or more, 14 or more, 15 or more, 16 or more, 17 or more, 18 or more, 19 or more, 20 or more, or all of the) SNP genotypes selected from the group consisting of rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), and rs4803455 (C/C).

In some embodiments, the subject can be one identified as having two or more (e.g., three or more, four or more, five or more, six or more, seven or more, eight or more, nine or more, 10 or more, 11 or more, 12 or more, 13 or more, 14 or more, 15 or more, 16 or more, 17 or more, 18 or more, 19 or more, 20 or more, or all of the) SNP genotypes selected from Table 13.

In some embodiments, the subject can be one identified as having two or more (e.g., three or more, four or more, five or more, six or more, seven or more, eight or more, nine or more, 10 or more, 11 or more, 12 or more, 13 or more, 14 or more, 15 or more, 16 or more, 17 or more, 18 or more, 19 or more, 20 or more, or all of the) SNP genotypes selected from the group consisting of rs 11778767 (A/G), rs 11778767 (G/G), rs 11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), and rs868856 (A/A).

In some embodiments, the method can include detecting the presence or absence of two or more (e.g., three or more, four or more, five or more, six or more, seven or more, eight or more, nine or more, 10 or more, 11 or more, 12 or more, 13 or more, 14 or more, 15 or more, 16 or more, 17 or more, 18 or more, 19 or more, 20 or more, or all of the) SNP genotypes selected from the group consisting of rs 11778767 (A/A), rs11780500 (A/A), rs11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), and rs868856 (G/G).

In some embodiments, the method can include detecting the presence or absence of two or more (e.g., three or more, four or more, five or more, six or more, seven or more, eight or more, nine or more, 10 or more, 11 or more, 12 or more, 13 or more, 14 or more, 15 or more, 16 or more, 17 or more, 18 or more, 19 or more, 20 or more, or all of the) SNP genotypes selected from the group consisting of rs11778767 (A/G), rs11778767 (G/G), rs11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), rs868856 (A/A), rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs 1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), xs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), and any of the SNP genotypes depicted in Table 13.

In some embodiments, the method can include detecting the presence or absence of two or more (e.g., three or more, four or more, five or more, six or more, seven or more, eight or more, nine or more, 10 or more, 11 or more, 12 or more, 13 or more, 14 or more, 15 or more, 16 or more, 17 or more, 18 or more, 19 or more, 20 or more, or all of the) SNP genotypes selected from the group consisting of rs 11778767 (A/G), rs11778767 (G/G), rs11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), and rs868856 (A/A).

In some embodiments, the method can include detecting the presence or absence of two or more (e.g., three or more, four or more, five or more, six or more, seven or more, eight or more, nine or more, 10 or more, 11 or more, 12 or more, 13 or more, 14 or more, 15 or more, 16 or more, 17 or more, 18 or more, 19 or more, 20 or more, or all of the) SNP genotypes selected from the group consisting of rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), and any of the SNP genotypes depicted in Table 13.

In some embodiments, the method can include detecting the presence or absence of two or more (e.g., three or more, four or more, five or more, six or more, seven or more, eight or more, nine or more, 10 or more, 11 or more, 12 or more, 13 or more, 14 or more, 15 or more, 16 or more, 17 or more, 18 or more, 19 or more, 20 or more, or all of the) SNP genotypes selected from the group consisting of rs 11778767 (A/G), rs 11778767 (G/G), rs 11780500 (A/G), rs 1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), and rs868856 (A/A).

Any of the methods described herein can further include a step of prescribing a therapy comprising an anti-TNF agent or non-anti-TNF agent (the choice of which depends upon the outcome of the predictive methods described herein) for the subject.

The biological sample in any of the methods described herein can consist of, or contain, e.g., blood.

In some embodiments of any of the methods described herein, the subject can have a disease such as an immune (e.g., an inflammatory) disorder, an infection, or any disease treatable by a therapy comprising an anti-TNF agent described herein. For example, the subject can have rheumatoid arthritis or Crohn's disease. The subject can be a human.

In some embodiments of any of the methods described herein, the anti-TNF agent can consist of, or contain, an anti-TNF antibody or a soluble TNF receptor. The anti-TNF antibody can be, e.g., adalimumab or infliximab. The soluble TNF receptor can be, e.g., etanercept.

In some embodiments, the non-anti-TNF agent can consist of, or contain, a non-steroidal anti-inflammatory drug (NSAID), a corticosteroid, a disease-modifying antirheumatic drug (DMARD), an anti-CD20 antibody, a TWEAK inhibitor, an IL-6 inhibitor, an IL-6 receptor inhibitor, a soluble lymphotoxin beta receptor, or a soluble BAFF antagonist. The NSAID can be a COX-2 inhibitor. The DMARD can be methotrexate, gold, penicillamine, or hydroxychloroquine.

In another aspect, the disclosure provides a composition containing at least two (e.g., at least three, at least four, at least five, at least six, at least seven, at least eight, at least nine, at least 10, at least 11, at least 12, at least 15, at least 20, at least 22, or at least 24 or more) polynucleotides that selectively hybridize to all of part of each of at least two (e.g., at least three, at least four, at least five, at least six, at least seven, at least eight, at least nine, at least 10, at least 11, at least 12, at least 15, at least 20, at least 22, or at least 24 or more, respectively) genes selected from the group consisting of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNA1, EEA1, FAM44A, FOXJ3, HDAC4, MNT, MXRA7, PTCH1, SEL1L, or SFRS2. The at least five genes can include, e.g., ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6. The at least five genes can include, e.g., CLTB, COL4A3BP, CXorf52, FAM44A, MXRA7, PGK1, SFRS2, or YIPF6. The at least eight genes can include, e.g., ANKIB 1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and/or YIPF6. The at least eight genes can consist of, or include, e.g., CLTB, COL4A3BP, CXorf52, FAM44A, MXRA7, PGK1, SFRS2, and/or YIPF6. The at least 24 genes can consist of, or include, e.g., ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6.

In yet another aspect, the disclosure provides a composition containing at least two (e.g., at least three, at least four, at least five, at least six, at least seven, at least eight, at least nine, at least 10, at least 11, at least 12, at least 15, or at least 20 or more) polynucleotides that selectively hybridize to each of at least two (e.g., at least three, at least four, at least five, at least six, at least seven, at least eight, at least nine, at least 10, at least 11, at least 12, at least 15, or at least 20 or more, respectively) SNP genotypes selected from the group consisting of rs11778767 (A/A), rs 11780500 (A/A), rs11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), rs868856 (G/G), rs11778767 (A/G), rs11778767 (G/G), rs 11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), rs868856 (A/A), rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/G), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs 10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), and any of the SNP genotypes depicted in Table 13.

In yet another aspect, the disclosure provides a composition containing at least two (e.g., at least three, at least four, at least five, at least six, at least seven, at least eight, at least nine, at least 10, at least 11, at least 12, at least 15, or at least 20 or more) polynucleotides that selectively hybridize to each of at least two (e.g., at least three, at least four, at least five, at least six, at least seven, at least eight, at least nine, at least 10, at least 11, at least 12, at least 15, or at least 20 or more, respectively) SNP genotypes selected from the group consisting of any of rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/G), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), and any of the SNP genotypes depicted in Table 13.

In another aspect, the disclosure provides a composition containing at least two (e.g., at least three, at least four, at least five, at least six, at least seven, at least eight, at least nine, at least 10, at least 11, at least 12, at least 15, or at least 20 or more) polynucleotides that selectively hybridize to each of at least two (e.g., at least three, at least four, at least five, at least six, at least seven, at least eight, at least nine, at least 10, at least 11, at least 12, at least 15, or at least 20 or more, respectively) SNP genotypes selected from the group consisting of rs11778767 (A/A), rs11780500 (A/A), rs11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), rs868856 (G/G), rs 11778767 (A/G), rs 11778767 (G/G), rs11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), and rs868856 (A/A).

In some embodiments of any of the compositions described above, the at least two polynucleotides can be bound to a solid support. The solid support can be a microarray chip, a particle (e.g., an encoded, magnetic, or magnetic and encoded particle), or any other solid support described herein.

Any of the compositions described above can contain less than 100,000 (e.g., less than 90,000; less than 80,000; less than 70,000; less than 60,000; less than 50,000; less than 40,000; less than 30,000; less than 20,000; less than 15,000; less than 10,000; less than 5,000; less than 4,000; less than 3,000; less than 2,000; less than 1,500; less than 1,000; less than 750; less than 500, less than 200, less than 100, or less than 50) different polynucleotides.

In yet another aspect, the disclosure provides a kit for determining expression levels or detecting the presence or absence of one or more SNP genotypes. The kit can include: any of the compositions described above and, optionally, instructions for determining expression levels (e.g., RNA and/or protein expression levels) or instructions for detecting one or more SNP genotypes. The kit can also include, e.g., one or more additional reagents for determining expression levels or detecting the presence or absence of one or more SNP genotypes. For example, the kit can include one or more of an antibody specific for a protein encoded by a gene of interest, primers (e.g., random hexamers or oligo(dT) primers), reverse transcriptase, a DNA polymerase (e.g., Taq polymerase), T4 polynucleotide kinase, one or more detectable labels (such as any described herein), or any other reagents described herein.

In some embodiments, the kit can include instructions for administering a therapy containing an anti-TNF agent if the expression level of one or more of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 are determined to be elevated or the expression level of one or more of ANKRD12, CAMK2G-, CASP5, CXorf52, DNAH1, EEA1, FAM44A, FOXJ3, HDAC4, MNT, MXRA7, PTCH1, SEL1L, or SFRS2 are determined to be reduced.

In some embodiments, the kit can include instructions for administering a therapy containing an anti-TNF agent if one or more of rs11778767 (A/A), rs11780500 (A/A), rs 11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), or rs868856 (G/G) are detected.

In another aspect, the disclosure provides an anti-TNF therapy response profile for a subject obtained by a method that includes the steps of: providing a biological sample from a subject; measuring the expression level of one or more genes in the biological sample, wherein the one or more genes comprise at least one gene selected from the group consisting of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2; and assessing the expression level of at least two of the one or more genes in the biological sample to obtain an anti-TNF response profile for the subject.

In yet another aspect, the disclosure features an anti-TNF therapy response profile for a subject obtained by the method that includes the steps of: providing a biological sample from a subject; and detecting the presence or absence of two or more SNP genotypes in a biological sample from a subject, wherein the one or more SNP genotypes comprise at least one SNP genotype selected from the group consisting of rs11778767 (A/A), rs11780500 (A/A), rs 11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), rs868856 (G/G), rs11778767 (A/G), rs 11778767 (G/G), rs 11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), and rs868856 (A/A) to obtain an anti-TNF response profile for the subject.

In yet another aspect, the disclosure features an anti-TNF therapy response profile for a subject obtained by the method that includes the steps of: providing a biological sample from a subject; and detecting the presence or absence of two or more SNP genotypes in a biological sample from a subject, wherein the one or more SNP genotypes comprise at least one SNP genotype selected from the group consisting of rs 11778767 (A/A), rs 11780500 (A/A), rs 11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), rs868856 (G/G), rs11778767 (A/G), rs11778767 (G/G), rs11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), rs868856 (A/A), rs983332 (A/C), rs928655 (A/G), rs13393173 (A/G), rs437943 (A/G), rs 10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), and any of the SNP genotypes depicted in Table 13 to obtain an anti-TNF response profile for the subject.

In yet another aspect, the disclosure features an anti-TNF therapy response profile for a subject obtained by the method that includes the steps of: providing a biological sample from a subject; and detecting the presence or absence of two or more SNP genotypes in a biological sample from a subject, wherein the one or more SNP genotypes comprise at least one SNP genotype selected from the group consisting of rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), and any of the SNP genotypes depicted in Table 13 to obtain an anti-TNF response profile for the subject.

Any of the response profiles described above can be used for predicting the response of a subject to a therapy comprising an anti-TNF agent.

As used herein, an anti-TNF agent is one that inhibits the activity of TNF. Inhibition of the activity of TNF includes, for example, inhibition of the expression (mRNA or protein expression) of TNF, inhibition of the release of TNF from a cell in which it is produced, or inhibition of the ability of TNF to bind to and/or activate its cognate receptor. Agents that inhibit the activity of TNF include, but are not limited to, small molecules, small interfering RNAs (siRNAs), anti-sense RNAs, antibodies that specifically bind to TNF, soluble TNF receptors, or dominant negative-TNF molecules (such as a dominant negative TNF protein or a nucleic acid encoding a dominant negative TNF protein). It is understood that an agent that inhibits TNF can be one that inhibits the ability of TNF to activate a receptor, but does not inhibit the binding of TNF to the receptor. Anti-TNF antibodies include, e.g., infliximab (Remicade®), D2E7 (adalumimab; Humira™), certolizumab (CDP-870), and CDP-571 (see, e.g., Sandborn et al. (2004) Gut 53(10):1485-1493; Choy et al. (2002) Rheumatology 41(10):1133-1137; and Kaushik et al. (2005) Expert Opinion on Biological Therapy 5(4):601-606(6)). Soluble TNF receptors include, e.g., etanercept (sTNF-RII:Fc; Enbrel®). Exemplary anti-TNF therapies are described in, e.g., U.S. Patent No. 6,270,766.

A non-anti-TNF agent can be, e.g., a non-steroidal anti-inflammatory drug (NSAID), a corticosteroid (e.g., glucocorticoid or mineralocorticoids), or a disease-modifying antirheumatic drug (DMARD). NSAIDS include, e.g., COX-2 inhibitors (e.g., celecoxib, etoricoxib, or lumiracoxib), salicylates (e.g., aspirin, amoxiprin, benorilate, choline magnesium salicylate, diflunisal, faislamine, methyl salicylate, magnesium salicylate, or salicyl salicylate (salsalate)), arylalkanoic acids (e.g., diclofenac, aceclofenac, acemetacin, bromfenac, etodolac, indometacin, ketorolac, nabumetone, sulindac, or tolmetin), or pyrazolidine derivatives (such as phenylbutazone, azapropazone, metamizole, oxyphenbutazone, or sulfinprazone). DMARDS include, but are not limited to, adalimumab, azathioprine, anti-malarials (e.g., chloroquine or hydroxychloroquine), cyclosporine A, D-penicillamine, gold salts (e.g., sodium aurothiomalate or auranofin), leflunomide, methotrexate (MTX), minocycline, or sulfasalazine (SSZ). Non-anti-TNF therapies also include anti-CD20 antibodies (e.g., rituximab (Rituxan®)), TWEAK inhibitors (e.g., anti-TWEAK antibodies, see, e.g., WO 06/122187, the disclosure of which is incorporated herein by reference in its entirety), soluble lymphotoxin beta receptors (e.g., LTBR-Fc), BAFF antagonists such as BR3-Fc; and IL-6 inhibitors (e.g., TL-6 antagonist antibodies, soluble IL-6 receptors) and IL-6 receptor inhibitors (e.g., IL-6 receptor antagonist antibodies such as tocilizumab or atlizumab (Actemra™) see, e.g., WO/2004/096273; EP1536012, WO/2006/119115, U.S. Patent Nos. 5,559,012 and 5,888,510; and U.S. Publication No. 20010001663, the disclosures of each of which are incorporated herein by reference in their entirety). In some embodiments, the non-anti-TNF agent can be an anti-inflammatory agent that does not contain an anti-TNF agent.

Diseases treatable by a therapy comprising an anti-TNF agent include, e.g., immune (e.g., inflammatory) disorders, infections, neurodegenerative diseases, malignant pathologies involving TNF-secreting tumors or other malignancies involving TNF, and alcohol-induced hepatitis.

Immune or inflammatory disorders include, but are not limited to, allergic bronchopulmonary aspergillosis; allergic rhinitis, autoimmune hemolytic anemia; acanthosis nigricans; allergic contact dermatitis; Addison's disease; atopic dermatitis; alopecia areata; alopecia universalis; amyloidosis; anaphylactoid purpura; anaphylactoid reaction; aplastic anemia; angioedema, hereditary; angioedema, idiopathic; ankylosing spondylitis; arteritis, cranial; arteritis, giant cell; arteritis, Takayasu's; arteritis, temporal; asthma; ataxia-telangiectasia; autoimmune oophoritis; autoimmune orchitis; autoimmune polyendocrine failure; Behcet's disease; Berger's disease; Buerger's disease; bronchitis; bullous pemphigus; candidiasis, chronic mucocutaneous; Caplan's syndrome; post-myocardial infarction syndrome; post- pericardiotomy syndrome; carditis; celiac sprue; Chagas's disease; Chediak-Higashi syndrome; Churg-Strauss disease; Cogan's syndrome; cold agglutinin disease; CREST syndrome; Crohn's disease; cryoglobulinemia; cryptogenic fibrosing alveolitis; dermatitis herpetifomis; dermatomyositis; diabetes mellitus; Diamond-Blackfan syndrome; DiGeorge syndrome; discoid lupus erythematosus; eosinophilic fasciitis; episcleritis; drythema elevatum diutinum; erythema marginatum; erythema multiforme; erythema nodosum; Familial Mediterranean fever; Felty's syndrome; pulmonary fibrosis; glomerulonephritis, anaphylactoid; glomerulonephritis, autoimmune; glomerulonephritis, post-streptococcal; glomerulonephritis, post-transplantation; glomerulopathy, membranous; Goodpasture's syndrome; granulocytopenia, immune-mediated; granuloma annulare; granulomatosis, allergic; granulomatous myositis; Grave's disease; Hashimoto's thyroiditis; hemolytic disease of the newborn; hemochromatosis, idiopathic; Henoch- Schoenlein purpura; hepatitis, chronic active and chronic progressive; histiocytosis X; hypereosinophilic syndrome; idiopathic thrombocytopenic purpura; Job's syndrome; juvenile dermatomyositis; juvenile rheumatoid arthritis (Juvenile chronic arthritis); Kawasaki's disease; keratitis; keratoconjunctivitis sicca; Landry-Guillain-Barre-Strohl syndrome; leprosy, lepromatous; Loeffler's syndrome; lupus; Lyell's syndrome; lyme disease; lymphomatoid granulomatosis; mastocytosis, systemic; mixed connective tissue disease; mononeuritis multiplex; Muckle-Wells syndrome; mucocutaneous lymph node syndrome; mucocutaneous lymph node syndrome; multicentric reticulohistiocytosis; multiple sclerosis; myasthenia gravis; mycosis fungoides; necrotizing vasculitis, systemic; nephrotic syndrome; overlap syndrome; panniculitis; paroxysmal cold hemoglobinuria; paroxysmal nocturnal hemoglobinuria; pemphigoid; pemphigus; pemphigus erythernatosus; pemphigus foliaceus; pemphigus vulgaris; pigeon breeder's disease; pneumonitis, hypersensitivity; polyarteritis nodosa; polymyalgia rheumatic; polymyositis; polyneuritis, idiopathic; portuguese familial polyneuropathies; pre-eclampsia/eclampsia; primary biliary cirrhosis; progressive systemic sclerosis (scleroderma); psoriasis; psoriatic arthritis; pulmonary alveolar proteinosis; pulmonary fibrosis, Raynaud's phenomenon/syndrome; Reidel's thyroiditis; Reiter's syndrome, relapsing polychrondritis; rheumatic fever; rheumatoid arthritis; sarcoidosis; scleritis; sclerosing cholangitis; serum sickness; Sezary syndrome; Sjogren's syndrome; Stevens- Johnson syndrome; Still's disease; subacute sclerosing panencephalitis; sympathetic ophthalmia; systemic lupus erythematosus; yransplant rejection; ulcerative colitis; undifferentiated connective tissue disease; urticaria, chronic; urticaria, cold; uveitis; vitiligo; Weber-Christian disease; Wegener's granulomatosis, or Wiskott-Aldrich syndrome.

Infections can include, e.g., sepsis syndrome, cachexia, circulatory collapse and shock resulting from acute or chronic bacterial infection, acute and chronic parasitic and/or infectious diseases, bacterial infections, viral infections, or fungal infections.

Neurodegenerative diseases include, e.g., demyelinating diseases (such as multiple sclerosis and acute transverse myelitis); extrapyramidal and cerebellar disorders' such as lesions of the corticospinal system; disorders of the basal ganglia or cerebellar disorders; hyperkinetic movement disorders such as Huntington's Chorea and senile chorea; drug-induced movement disorders, such as those induced by drugs which block CNS dopamine receptors; hypokinetic movement disorders, such as Parkinson's disease; progressive supranucleo palsy; cerebellar and spinocerebellar disorders, such as astructural lesions of the cerebellum; spinocerebellar degenerations (spinal ataxia, Friedreich's ataxia, cerebellar cortical degenerations, multiple systems degenerations (Mencel, Dejerine-Thomas, Shi-Drager, and Machado-Joseph); and systemic disorders (Refsum's disease, abetalipoprotemia, ataxia telangiectasia, and mitochondrial multi-system disorder); demyelinating core disorders, such as multiple sclerosis, acute transverse myelitis; disorders of the motor unit, such as neurogenic muscular atrophies (anterior horn cell degeneration, such as amyotrophic lateral sclerosis, infantile spinal muscular atrophy and juvenile spinal muscular atrophy); Alzheimer's disease; Down's Syndrome in middle age; Diffuse Lewy body disease; Senile Dementia of Lewy body type; Wernicke-Korsakoff syndrome; chronic alcoholism; Creutzfeldt-Jakob disease; subacute sclerosing panencephalitis; Hallerrorden-Spatz disease; and dementia pugilistica.

Malignant pathologies involving TNF-secreting tumors or other malignancies involving TNF include, but not limited to leukemias (acute, chronic myelocytic, chronic lymphocytic and/or myelodyspastic syndrome); or lymphomas (Hodgkin's and non-Hodgkin's lymphomas, such as malignant lymphomas (Burkitt's lymphoma or mycosis fungoides).

Additional disorders that can be treated according to the methods described herein are described in, e.g., WO 06/74399, WO 06/122187, or U.S. Patent Nos. 5,656,272 and 5,919,452, the disclosures of each of which are incorporated herein by reference in their entirety.

Sequence "complementarity," as used herein, refers to the chemical affinity between specific nitrogenous bases as a result of their hydrogen bonding properties (i.e., the property of two nucleic acid chains having base sequences such that an antiparallel duplex can form where the adenines and uracils (or thymine, in the case of DNA or modified RNA) are apposed to each other, and the guanines and cytosines are apposed to each other). Fully complementary sequences, thus, would be two sequences that have complete one-to-one correspondence (i.e., adenine to uracil and guanine to cytosine) of the base sequences when the nucleotide sequences form an antiparallel duplex.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, the exemplary methods and materials are described below. All publications, patent applications, patents, and other references mentioned herein are incorporated by reference in their entirety. In case of conflict, the present application, including definitions, will control. The materials, methods, and examples are illustrative only and not intended to be limiting.

Other features and advantages of the invention will be apparent from the following detailed description, and from the claims.

**BRIEF DESCRIPTION OF THE DRAWINGS**

**Fig.1** is a scatter plot of P-values for 317,000 single nucleotide polymorphisms (SNPs) from the ANOVA model for 22 chromosomes. The X-axis represents chromosomes 1 to 22 and the Y-axis represents the $-\log_{10}$(p-value). Each dot represents a p-value for an association. Two parallel lines to the X-axis representing $10^{-5}$ and $10^{-7}$ levels of significance are drawn in the plot. The p-values between $10^{-5}$ and $10^{-7}$ are plotted and indicated by asterisk.

**Fig. 2** is a cluster diagram depicting population stratification among the 102 subjects in the ABCoN data. The 102 subjects are divided into 3 distinct clusters - cluster 1, 2, and 3, which are located in the 3 corners in the plot. Cluster 4 seems to be admixed population. Cluster 1 is the largest population cluster, including 89 patients with major European origin, among them 83 were self-reported to be white and 6 had missing ethnicity data. Cluster 2, 3, and 4 include 2, 4, and 6 patients; respectively.

**Fig. 3** is a scatter plot depicting Genome-wide association p-value plots showing the association of SNPs with relative change in DAS28 score (*RelDAS*28). The X-axis represents chromosomes 1 to 22 and the Y-axis represents the $-\log_{10}$(p-value). Each dot represents a p-value for an association. Two parallel lines to the X-axis representing $10^{-5}$ and $10^{-7}$ levels of significance are drawn in the plot. The p-values between $10^{-5}$ and $10^{-7}$ are plotted and indicated by asterisk.

**Figs. 4A** and **4B** are a pair of graphs depicting phenotypic differences among 89 individuals. Phenotypic differences appear not to be correlated to the top principal components. Fig 2A represents DAS change/responding status versus PC1 and the right figure is in a finer scale. Fig. 2B represents DAS change/responding status versus PC2 and the right figure is in a finer scale.

**Fig. 5** is a dendrogram depicting the clustering of 40 genes (Table 11) predictive of the anti-TNF response in rheumatoid arthritis patients. The clustering tree is first cut into two clusters. From each cluster, genes with highest variable importance were selected. The selected genes from 8 subclusters (k =8) are shown as diamonds. The procedure is repeated until the tree is cut into a pre-defined (see the text) number of clusters.

**Figs. 6A** and **6B** are a pair of line graphs depicting the median (6A) and standard deviation (6B) of OOB error rate for different *ntree* values (*mtry* = 22 (default)). The standard deviation of the error rate stabilized at *ntree* = 15400.

**Figs. 7A** and **7B** are a pair of line graphs depicting the change of median (7A) and standard deviation (7B) of the error rate with *mtry*. *Mtry* values with minimum error rate and maximum stability can be selected as optimum values (*mtry* = {45}, circled) for further analysis.

**Fig. 8** is a dot plot depicting the change of median OOB error rate (Y-axis) with *mtry* (X-axis) for different *ntree* values.

**Fig. 9** is a box plot depicting error rate distributions at *mtry* ={45} (10 runs each) for four groups: (1) all 166 genes: x label a-45 (2) 40 genes that converged at *mtry* ={45}: label f-45 indicates final set of converged genes (3) first 40 genes selected by importance: label imp-45 indicating first 40 most important genes among 166.

**Fig. 10** is a line graph depicting change of OOB error rate with the number of genes selected by two methods importance and clustering ranking. Black line corresponds to genes selected by importance ranking of the initial 166 genes set. Open circles represent 40 convergent genes ranked by the importance measure. Minimum error (11%) is obtained with k = 24 genes, circled in red. Filled circles correspond to the error rate with k-best genes selected from k clusters (x axis). Minimum error (11%) is obtained with k = 8 genes (large circles).

## DETAILED DESCRIPTION

Provided herein are methods and compositions (e.g., nucleic acid arrays and kits) for predicting the response of a subject (such as a human patient) to an anti-TNF therapy. In addition, the disclosure provides predictive biomarkers (e.g., gene expression levels or SNP genotypes) to identify those subjects for whom administering an anti-TNF therapy is likely to be effective or ineffective. Such biomarkers, compositions, and methods are useful in selecting appropriate therapeutic modalities (e.g., an anti-TNF therapy or a non-anti-TNF therapy) for subjects suffering from diseases such as rheumatoid arthritis, Crohn's disease, psoriatic arthritis, Behçet' disease, ankylosing spondylitis, and other immune disorders.

Gene Expression and Responsiveness to Anti-TNF Therapy

A series of genes have been identified whose expression levels (e.g., mRNA or protein expression levels) are predictive of the responsiveness or non-responsiveness of a subject to an anti-TNF therapy. The genes (and their corresponding Entrez Gene ID Nos.) are depicted in Table 1.

Table 1.

| Gene Name | Entrez Gene ID No. |
|---|---|
| ANKIB1 | 54467 |
| ANKRD12 | 23253 |
| ARF1 | 375 |
| ARF5 | 381 |
| BRWD2 | 55717 |
| C9orf80 | 58493 |
| CALM2 | 805 |
| CAMK2G- | 818 |
| CASP5 | 838 |
| CLTB | 1212 |
| COL4A3BP | 10087 |
| CXorf52 | 286967 |
| DNAH1 | 25981 |
| EEA1 | 8411 |
| EGLN2 | 112398 |
| FAM44A | 259282 |
| FOXJ3 | 22887 |
| HDAC4 | 9759 |
| HDAC5 | 10014 |

(continued)

| Gene Name | Entrez Gene ID No. |
|---|---|
| LGALS9 | 3965 |
| MNT | 4335 |
| MXRA7 | 439921 |
| MYLIP | 29116 |
| PCBP2 | 5094 |
| PGK1 | 5230 |
| PTCH1 | 5727 |
| RBBP4 | 5928 |
| RER1 | 11079 |
| RPA3 | 6119 |
| SEL1L | 6400 |
| SERF2 | 10169 |
| SFRS2 | 6427 |
| SLC25A39 | 51629 |
| SRGAP2 | 23380 |
| TUG1 | 65000 |
| YIPF6 | 286451 |
| ZFP36L1 | 677 |
| ZNF294 | 26046 |

An elevated expression level of a gene can be predictive of either responsiveness or non-responsiveness to an anti-TNF therapy. For example, an elevated expression level of one or more of HDAC4, CXorf52, ANKRD12, CAMK2G-, CASP5, MXRA7, FAM44A, MNT, SEL1L, EEA1, FOXJ3, DNAH1, PITCH1, or SFRS2 predicts that a subject will not respond to an anti-TNF therapy whereas an elevated expression level of one or more of CLTB, RBBP4, COL4A3BP, C9orf80, PCBP2, YIPF6, MYLIP, ZNF294, RER1, CALM2, ARF5, ARF1, HDAC5, ANKIB1, BRWD2, PGK1, ZFP36L1, SERF2, SRGAP2, TUG1, LGALS9, SLC25A39, EGLN2, or RPA3 predicts that a subject will respond to an anti-TNF therapy.

A decreased expression level of a gene can be predictive of either responsiveness or non-responsiveness to an anti-TNF therapy. For example, a decreased expression level of one or more of CLTB, RBBP4, COL4A3BP, C9orf80, PCBP2, YIPF6, MYLIP, ZNF294, RER1, CALM2, ARF5, ARF1, HDAC5, ANKIB1, BRWD2, PGK1, ZFP36L1, SERF2, SRGAP2, TUG1, LGALS9, SLC25A39, EGLN2, or RPA3 predicts that a subject will not respond to an anti-TNF therapy whereas a decreased expression level of one or more of HDAC4, CXorf52, ANKRD12, CAMK2G-, CASP5, MXRA7, FAM44A, MNT, SEL1L, EEA1, FOXJ3, DNAH1, PTCH1, or SFRS2 predicts that a subject will respond to an anti-TNF therapy.

In predicting responsiveness or non-responsiveness to an anti-TNF therapy, the expression level of one or more of the genes depicted in Table 1 can be elevated or reduced by at least about 1.5 fold (e.g., at least about 2 fold, at least about 2.5 fold, at least about 3.0 fold, at least about 3.5 fold, at least about 4.0 fold, or at least about 5 fold or more).

Gene expression can be detected as, e.g., protein or mRNA expression of a target gene. That is, the presence or expression level (amount) of a gene can be determined by detecting and/or measuring the level of mRNA or protein expression of the gene. In some embodiments, gene expression can be detected as the activity of a protein encoded by a gene such as a gene depicted in Table 1.

A variety of suitable methods can be employed to detect and/or measure the level of mRNA expression of a gene. For example, mRNA expression can be determined using Northern blot or dot blot analysis, reverse transcriptase-PCR (RT-PCR; e.g., quantitative RT-PCR), *in situ* hybridization (e.g., quantitative *in situ* hybridization) or nucleic acid array (e.g., oligonucleotide arrays or gene chips) analysis. Details of such methods are described below and in, e.g., Sambrook et al., Molecular Cloning: A Laboratory Manual Second Edition vol. 1, 2 and 3. Cold Spring Harbor Laboratory Press: Cold Spring Harbor, New York, USA, Nov. 1989; Gibson et al. (1999) Genome Res. 6(10):995-1001; and Zhang et al. (2005) Environ. Sci. Technol. 39(8):2777-2785; U.S. Publication No. 2004086915; European Patent No. 0543942; and U.S. Patent No. 7,101,663; the disclosures of each of which are incorporated herein by reference in their entirety.

In one example, the presence or amount of one or more discrete mRNA populations in a biological sample can be determined by isolating total mRNA from the biological sample (see, e.g., Sambrook et al. (*supra*) and U.S. Patent No. 6,812,341) and subjecting the isolated mRNA to agarose gel electrophoresis to separate the mRNA by size. The size-separated mRNAs are then transferred (e.g., by diffusion) to a solid support such as a nitrocellulose membrane. The presence or amount of one or more mRNA populations in the biological sample can then be determined using one or more detectably-labeled-polynucleotide probes, complementary to the mRNA sequence of interest, which bind to and thus render detectable their corresponding mRNA populations. Detectable-labels include, e.g., fluorescent (e.g., umbelliferone, fluorescein, fluorescein isothiocyanate, rhodamine, dichlorotriazinylamine fluorescein, dansyl chloride, allophycocyanin (APC), or phycoerythrin), luminescent (e.g., europium, terbium, Qdot™ nanoparticles supplied by the Quantum Dot Corporation, Palo Alto, CA), radiological (e.g., $^{125}I$, $^{131}I$, $^{31}S$, $^{32}P$, $^{33}P$, or $^{3}H$), and enzymatic (horseradish peroxidase, alkaline phosphatase, beta-galactosidase, or acetylcholinesterase) labels.

In another example, the presence or amount of discrete populations of mRNA (e.g., mRNA encoded by one or more genes depicted in Table 1) in a biological sample can be determined using nucleic acid (or oligonucleotide) arrays (e.g., an array described below under "Arrays and Kits"). For example, isolated mRNA from a biological sample can be amplified using RT-PCR with random hexamer or oligo(dT)-primer mediated first strand synthesis. The RT-PCR step can be used to detectably-label the amplicons, or, optionally, the amplicons can be detectably-labeled subsequent to the RT-PCR step. For example, the detectable-label can be enzymatically (e.g., by nick-translation or kinase such as T4 polynucleotide kinase) or chemically conjugated to the amplicons using any of a variety of suitable techniques (see, e.g., Sambrook et al., *supra*). The detectably-labeled-amplicons are then contacted to a plurality of polynucleotide probe sets, each set containing one or more of a polynucleotide (e.g., an oligonucleotide) probe specific for (and capable of binding to) a corresponding amplicon, and where the plurality contains many probe sets each corresponding to a different amplicon. Generally, the probe sets are bound to a solid support and the position of each probe set is predetermined on the solid support. The binding of a detectably-labeled amplicon to a corresponding probe of a probe set indicates the presence or amount of a target mRNA in the biological sample. Additional methods for detecting mRNA expression using nucleic acid arrays are described in, e.g., U.S. Patent Nos. 5,445,934; 6,027,880; 6,057,100; 6,156,501; 6,261,776; and 6,576,424; the disclosures of each of which are incorporated herein by reference in their entirety.

Methods of detecting and/or for quantifying a detectable label depend on the nature of the label. The products of reactions catalyzed by appropriate enzymes (where the detectable label is an enzyme; see above) can be, without limitation, fluorescent, luminescent, or radioactive or they may absorb visible or ultraviolet light. Examples of detectors suitable for detecting such detectable labels include, without limitation, x-ray film, radioactivity counters, scintillation counters, spectrophotometers, colorimeters, fluorometers, luminometers, and densitometers.

The expression of a gene can also be determined by detecting and/or measuring expression of a protein encoded by the gene. Methods of determining protein expression generally involve the use of antibodies specific for the target protein of interest. For example, methods of determining protein expression include, but are not limited to, western blot or dot blot analysis, immunohistochemistry (e.g., quantitative immunohistochemistry), immunocytochemistry, enzyme-linked immunosorbent assay (ELISA), enzyme-linked immunosorbent spot (ELISPOT; Coligan, J. E., et al., eds. (1995) Current Protocols in Immunology. Wiley, New York), or antibody array analysis (see, e.g., U.S. Publication Nos. 20030013208 and 2004171068, the disclosures of each of which are incorporated herein by reference in their entirety). Further description of many of the methods above and additional methods for detecting protein expression can be found in, e.g., Sambrook et al. (*supra*).

In one example, the presence or amount of protein expression of a gene (e.g., a gene depicted in Table 1) can be determined using a western blotting technique. For example, a lysate can be prepared from a biological sample, or the biological sample itself, can be contacted with Laemmli buffer and subjected to sodium-dodecyl sulfate polyacrylamide gel electrophoresis (SDS-PAGE). SDS-PAGE-resolved proteins, separated by size, can then be transferred to a filter membrane (e.g., nitrocellulose) and subjected to immunoblotting techniques using a detectably-labeled antibody specific to the protein of interest. The presence or amount of bound detectably-labeled antibody indicates the presence or amount of protein in the biological sample.

In another example, an immunoassay can be used for detecting and/or measuring the protein expression of a gene (e.g., a gene depicted in Table 1). As above, for the purposes of detection, an immunoassay can be performed with an antibody that bears a detection moiety (e.g., a fluorescent agent or enzyme). Proteins from a biological sample can be conjugated directly to a solid-phase matrix (e.g., a multi-well assay plate, nitrocellulose, agarose, sepharose, encoded particles, or magnetic beads) or it can be conjugated to a first member of a specific binding pair (e.g., biotin or streptavidin) that attaches to a solid-phase matrix upon binding to a second member of the specific binding pair (e.g., streptavidin or biotin). Such attachment to a solid-phase matrix allows the proteins to be purified away from other interfering or irrelevant components of the biological sample prior to contact with the detection antibody and also allows for subsequent washing of unbound antibody. Here as above, the presence or amount of bound detectably-labeled antibody indicates the presence or amount of protein in the biological sample.

Methods for generating antibodies or antibody fragments specific for a protein encoded by one or more genes can be generated by immunization, e.g., using an animal, or by *in vitro* methods such as phage display. A polypeptide that includes all or part of a target protein can be used to generate an antibody or antibody fragment. The antibody can be a monoclonal antibody or a preparation of polyclonal antibodies.

Methods for detecting or measuring gene expression (e.g., mRNA or protein expression) can optionally be performed in formats that allow for rapid preparation, processing, and analysis of multiple samples. This can be, for example, in multi-welled assay plates (e.g., 96 wells or 386 wells) or arrays (e.g., nucleic acid chips or protein chips). Stock solutions for various reagents can be provided manually or robotically, and subsequent sample preparation (e.g., RT-PCR, labeling, or cell fixation), pipetting, diluting, mixing, distribution, washing, incubating (e.g., hybridization), sample readout, data collection (optical data) and/or analysis (computer aided image analysis) can be done robotically using commercially available analysis software, robotics, and detection instrumentation capable of detecting the signal generated from the assay. Examples of such detectors include, but are not limited to, spectrophotometers, luminometers, fluorimeters, and devices that measure radioisotope decay. Exemplary high-throughput cell-based assays (e.g., detecting the presence or level of a target protein in a cell) can utilize ArrayScan® VTI HCS Reader or KineticScan® HCS Reader technology (Cellomics Inc., Pittsburg, PA).

In some embodiments, the expression level (or activity) of at least two genes (e.g., at least three genes, at least four genes, at least five genes, at least six genes, at least seven genes, at least eight genes, at least nine genes, at least 10 genes, at least 11 genes, at least 12 genes, at least 13 genes, at least 14 genes, at least 15 genes, at least 16 genes, at least 17 genes, at least 18 genes, at least 19 genes, at least 20 genes, at least 21 genes, at least 22 genes, at least 23 genes, or at least 24 genes or more) can be assessed and/or measured.

To aid in detecting the presence or level of expression of one or more of the genes depicted in Table 1, the nucleic acid sequences of the genes can be used, e.g., as hybridization polynucleotide probes or primers (e.g., for amplification or reverse transcription). The probes and primers can be oligonucleotides of sufficient length to provide specific hybridization to a RNA or DNA target derived from a biological sample. Depending on the specific application, varying hybridization conditions can be employed to achieve varying degrees of selectivity of a probe or primer towards target sequence.

Standard stringency conditions are described by Sambrook, et al. (*supra*) and Haymes, et al. Nucleic Acid Hybridization, A Practical Approach, IRL Press, Washington, D.C. (1985). In order for a nucleic acid molecule to serve as a primer or probe it need only be sufficiently complementary in sequence to be able to form a stable double-stranded structure under the particular hybridization conditions (e.g., solvent and salt concentrations) employed.

Appropriate stringency conditions that promote DNA hybridization, for example, 6.0x sodium chloride/sodium citrate (SSC) at about 45° C, followed by a wash of 2.0×SSC at 50° C, are known to those skilled in the art or can be found in Ausubel, et al., Current Protocols in Molecular Biology, John Wiley & Sons, N.Y. (1989), the disclosure of which is incorporated herein by reference in its entirety. For example, the salt concentration in the wash step can be selected from a low stringency of about 2.0×SSC at 50°C to a high stringency of about 0.2×SSC at 50°C. The temperature used in the wash step can be increased from low stringency conditions at room temperature (about 22° C) to high stringency conditions at about 65°C. Temperature and salt conditions may be varied independently.

Primers and probes can be used in hybridization assays or techniques or in a variety of PCR-type methods. The primers and probes can be detectably-labeled with reagents that facilitate detection (e.g., fluorescent labels, chemical labels (see, e.g., U.S. Patent Nos. 4,582,789 and 4,563,417), or modified bases).

In addition, the nucleic acid sequences (e.g., oligonucleotide probes) can be used in nucleic acid arrays (such as the nucleic acid arrays described below under "Arrays and Kits") for detection and/or quantitation of gene expression.

Single Nucleotide Polymorphisms and Responsiveness to Anti-TNF Therapy

A series of SNP genotypes have been identified that are associated with the response of a subject to an anti-TNF therapy. Thus, the presence of one or more of these SNP genotypes can be used to predict the responsiveness or non-responsiveness of a subject to an anti-TNF therapy. The SNP genotypes (identified by their chromosomal location, SNP ID No., and genotype) are depicted in Tables 2-4 and 13.

Table 2.

| Chromsomal Location | SNP ID No. | Genotype |
|---|---|---|
| | | |
| 1p22 | rs2170331 | A/A |

(continued)

| Chromsomal Location | SNP ID No. | Genotype |
|---|---|---|
| | | A/G |
| | | G/G |
| | | |
| | rs6665006 | A/A |
| | | A/G |
| | | G/G |
| | | |
| 5q35 | rs1422422 | A/A |
| | | A/G |
| | | G/G |
| | rs4976592 | A/A |
| | | A/G |
| | | G/G |
| | | |
| 7q22 | rs1968201 | A/A |
| | | A/G |
| | | |
| | | |
| | rs3087615 | A/C |
| | | C/C |
| | | |
| 9p21 | rs7046653 | A/A |
| | | A/G |
| | | G/G |
| | | |
| | rs868856 | A/A |
| | | A/G |
| | | G/G |
| | | |
| | rs2814707 | A/A |
| | | A/G |
| | | G/G |
| | | |
| | rs774359 | A/A |
| | | A/G |
| | | G/G |
| | | |
| | rs3849942 | A/A |

(continued)

| Chromsomal Location | SNP ID No. | Genotype |
|---|---|---|
| | | A/G |
| | | G/G |
| | | |
| 4p14 | rs437943 | A/A |
| | | A/G |
| | | G/G |
| | | |
| | rs1441209 | A/A |
| | | A/G |
| | | G/G |
| | | |
| | rs6531358 | A/A |
| | | A/G |
| | | G/G |
| | rs2028446 | A/A |
| | | A/G |
| | | G/G |
| | | |
| 8q22 | rs11778767 | A/A |
| | | A/G |
| | | G/G |
| | | |
| | rs11780500 | A/A |
| | | A/G |
| | | G/G |
| | | |
| | rs4562286 | A/A |
| | | A/G |
| | | G/G |
| | | |
| | rs3019293 | A/A |
| | | A/G |
| | | G/G |

Table 3.

| Chromsomal Location | SNP ID No. | Genotype |
|---|---|---|
| 1 | rs983332 | A/C |
| | | A/A |

(continued)

| Chromsomal Location | SNP ID No. | Genotype |
|---|---|---|
| | | |
| 1 | rs928655 | A/G |
| | | A/A |
| | | |
| 2 | rs13393173 | A/G |
| | | A/A |
| | | |
| 4 | rs437943 | A/G |
| | | G/G |
| | | |
| 6 | rs10945919 | A/G |
| | | G/G |
| | | |
| 7 | rs854555 | A/C |
| | | A/A |
| 7 | rs854548 | A/G |
| | | A/A |
| | | |
| 7 | rs854547 | A/G |
| | | A/A |
| | | |
| 9 | rs7046653 | A/G |
| | | A/A |
| | | |
| 9 | rs868856 | C/T |
| | | T/T |
| | | |
| 9 | rs774359 | C/T |
| | | C/C |
| | | |
| 9 | rs2814707 | A/G |
| | | A/A |
| | | |
| 9 | rs3849942 | A/G |
| | | A/A |
| | | |
| 20 | rs6028945 | G/T |
| | | T/T |

(continued)

| Chromsomal Location | SNP ID No. | Genotype |
|---|---|---|
| | | |
| 20 | rs6138150 | C/T |
| | | T/T |
| | | |
| 20 | rs6071980 | C/T |
| | | C/C |

Table 4.

| Chromsomal Location | SNP ID No. | Genotype |
|---|---|---|
| 1 | rs1800896 | A/G |
| | | A/A |
| | | |
| 1 | rs3024490 | A/C |
| | | A/A |
| | | |
| 2 | rs231726 | A/G |
| | | A/A |
| 2 | rs3096851 | A/C |
| | | C/C |
| | | |
| 2 | rs6708660 | A/G |
| | | A/A |
| | | |
| 6 | rs2523619 | A/G |
| | | G/G |
| | | |
| 6 | rs3915971 | A/G |
| | | A/A |
| | | |
| 6 | rs9264869 | A/G |
| | | A/A |
| | | |
| 6 | rs2239804 | A/G |
| | | A/A |
| | | |
| 6 | rs2395175 | A/G |
| | | G/G |
| | | |

(continued)

| Chromsomal Location | SNP ID No. | Genotype |
|---|---|---|
| 6 | rs2395185 | A/C |
| | | C/C |
| | | |
| 6 | rs2516049 | A/G |
| | | A/A |
| | | |
| 6 | rs660895 | A/G |
| | | A/A |
| | | |
| 9 | rs7026551 | A/C |
| | | C/C |
| | | |
| 9 | rs4803455 | A/C |
| | | C/C |

The presence of a particular SNP genotype can be predictive of responsiveness to an anti-TNF therapy. For example, the presence of one or more of rs11778767 (A/A), rs11780500 (A/A), rs11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), or rs868856 (G/G) is predictive that a subject will respond to an anti-TNF therapy.

The presence of a particular SNP genotype can be predictive of non-responsiveness to an anti-TNF therapy. For example, the presence of one or more of rs11778767 (A/G), rs11778767 (G/G), rs11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), or rs868856 (A/A) is predictive that a subject will not respond to an anti-TNF therapy. In another example, the presence of one of more of SNP genotypes from any one of Tables 3, 4, and 13 can be predictive of non-responsiveness of a subject to an anti-TNF therapy.

Methods for detecting the presence of a SNP are known in the art and set forth the accompanying Examples. Suitable methods for determining a SNP genotype include, e.g., Southern blot (see, e.g., Sambrook et al. (*supra*)), real-time PCR analysis (see, e.g., Oliver et al. (2000) J. Mol. Diagnostics 2(4):202-208), nucleic acid array analysis, allele-specific PCR (e.g., quantitative allele-specific PCR), pyroseqeuncing, DNA sequencing (e.g., Sanger chemistry sequencing), or through the use of molecular beacons (e.g., Tyagi et al. (1998) Nat. Biotechnol. 16:49-53; Abravaya et al. (2003) Clin. Chem. Lab. Med. 41:468-474; and Mullah et al. (1999) Nucleos Nucleot. 18:1311-1312, the disclosures of each of which are incorporated herein by reference in their entirety).

To determine a SNP genotype using Southern blot analysis, first, genomic DNA is isolated from a biological sample from a subject (e.g., a human patient), e.g., using a NP40 detergent, SDS, and proteinase K digestion, followed by NaCl extraction, and ethanol wash. Regions of DNA containing the SNP of interest can be amplified using PCR. The amplicons can be subjected to gel-electrophoresis to separate the nucleic acids by size, and then transferred to a solid support such as a nitrocellulose membrane. To detect the presence of a SNP in the biological sample, the solid support containing the amplicons can be contacted with a detectably-labeled, complementary oligonucleotide probe that specifically hybridizes to the SNP under appropriate stringency conditions. The binding of the probe to an amplicon indicates the presence of the corresponding SNP in the biological sample.

In another example, the a SNP genotype can also be detected using nucleic acid arrays. For example, genomic DNA isolated from a biological sample can be amplified using PCR as described above. The amplicons can be detectably-labeled during the PCR amplification process (e.g., using one or more detectably labeled dNTPs) or subsequent to the amplification process using a variety of chemical or enzymatic techniques such as nick-translation. Following amplification and labeling, the detectably-labeled-amplicons are then contacted to a plurality of polynucleotide probe sets, each set containing one or more of a polynucleotide (e.g., an oligonucleotide) probe specific for (and capable of binding to) a corresponding amplicon, and where the plurality contains many probe sets each corresponding to a different amplicon. Generally, the probe sets are bound to a solid support and the position of each probe set is predetermined on the solid support. The binding of a detectably-labeled amplicon to a corresponding probe of a probe set indicates the presence of the SNP so amplified in the biological sample. Suitable conditions and methods for detecting SNP using nucleic acid arrays are further described in, e.g., Lamy et al. (2006) Nucleic Acids Research 34(14): e100; European Patent Publication No. 1234058; U.S. Publication Nos. 20060008823 and 20030059813; and U.S. Patent No. 6,410,231; the disclosures of each of which are incorporated herein by reference in their entirety.

In some embodiments, the presence or absence of at least two (e.g., at least three, at least four, at least five, at least six, at least seven, at least eight, at least nine, at least 10, at least 11, at least 12, at least 13, at least 14, or at least 15) SNP genotypes depicted in Tables 2-4 or 13 can be detected and/or used to predict the responsiveness or non-responsiveness of a subject to an anti-TNF therapy.

Any of the methods of detecting a SNP can, optionally, be performed in formats that allow for rapid preparation, processing, and analysis of multiple samples (see above).

The detection of one or more of the SNP genotypes depicted in Tables 2-4 or 13 can use the nucleic acid sequences of the SNPs themselves, and surrounding sequence, e.g., as hybridization polynucleotide probes or primers (e.g., for amplification or reverse transcription). SNP probes should contain a sequence of sufficient length and complementarity to a corresponding SNP region to specifically hybridize with that SNP region under suitable hybridization conditions. For example, the SNP probes can include at least one (e.g., at least two, at least three, at least four, at least five, at least six, at least seven, at least eight, at least nine, at least 10, at least 11, at least 12, at least 15, at least 20, at least 25, at least 30, at least 35, at least 40, at least 45, at least 50, or 55 or more) nucleotides 5' or 3' to the SNP of interest. The polymorphic site of each probe (i.e., the SNP region) is generally flanked on one or both sides by sequence that is common among the different alleles.

Samples and Sample Collection

Suitable biological samples for the methods described herein include any biological fluid, cell, tissue, or fraction thereof, which includes analyte biomolecules of interest such as nucleic acid (e.g., DNA or mRNA) or protein. A biological sample can be, for example, a specimen obtained from a subject (e.g., a mammal such as a human) or can be derived from such a subject. For example, a sample can be a tissue section obtained by biopsy, or cells that are placed in or adapted to tissue culture. A biological sample can also be a biological fluid such as urine, blood, plasma, serum, saliva, semen, sputum, cerebral spinal fluid, tears, or mucus, or such a sample absorbed onto a paper or polymer substrate. A biological sample can be further fractionated, if desired, to a fraction containing particular cell types. For example, a blood sample can be fractionated into serum or into fractions containing particular types of blood cells such as red blood cells or white blood cells (leukocytes). If desired, a sample can be a combination of samples from a subject such as a combination of a tissue and fluid sample.

The biological samples can be obtained from a subject, e.g., a subject having, suspected of having, or at risk of developing, an inflammatory disease such as rheumatoid arthritis or Crohn's disease. Any suitable methods for obtaining the biological samples can be employed, although exemplary methods include, e.g., phlebotomy, swab (e.g., buccal swab), or fine needle aspirate biopsy procedure. Non-limiting examples of tissues susceptible to fine needle aspiration include lymph node, lung, thyroid, breast, and liver. Samples can also be collected, e.g., by microdissection (e.g., laser capture microdissection (LCM) or laser microdissection (LMD)), bladder wash, smear (PAP smear), or ductal lavage.

Methods for obtaining and/or storing samples that preserve the activity or integrity of molecules (e.g., nucleic acids or proteins) in the sample are well known to those skilled in the art. For example, a biological sample can be further contacted with one or more additional agents such as appropriate buffers and/or inhibitors, including nuclease, protease and phosphatase inhibitors, which preserve or minimize changes in the molecules (e.g., nucleic acids or proteins) in the sample. Such inhibitors include, for example, chelators such as ethylenediamine tetraacetic acid (EDTA), ethylene glycol bis(P-aminoethyl ether) N,N,N1,N1-tetraacetic acid (EGTA), protease inhibitors such as phenylmethylsulfonyl fluoride (PMSF), aprotinin, leupeptin, antipain and the like, and phosphatase inhibitors such as phosphate, sodium fluoride, vanadate and the like. Appropriate buffers and conditions for isolating molecules are well known to those skilled in the art and can be varied depending, for example, on the type of molecule in the sample to be characterized (see, for example, Ausubel et al. Current Protocols in Molecular Biology (Supplement 47), John Wiley & Sons, New York (1999); Harlow and Lane, Antibodies: A Laboratory Manual (Cold Spring Harbor Laboratory Press (1988); Harlow and Lane, Using Antibodies: A Laboratory Manual, Cold Spring Harbor Press (1999); Tietz Textbook of Clinical Chemistry, 3rd ed. Burtis and Ashwood, eds. W.B. Saunders, Philadelphia, (1999)). A sample also can be processed to eliminate or minimize the presence of interfering substances. For example, a biological sample can be fractionated or purified to remove one or more materials that are not of interest. Methods of fractionating or purifying a biological sample include, but are not limited to, chromatographic methods such as liquid chromatography, ionexchange chromatography, size-exclusion chromatography, or affinity chromatography.

For use in the methods described herein, a sample can be in a variety of physical states. For example, a sample can be a liquid or solid, can be dissolved or suspended in a liquid, can be in an emulsion or gel, and can be absorbed onto a material.

Exemplary biological samples, methods for obtaining the samples, and purification methods (e.g., mRNA purification methods) are detailed in the accompanying Examples.

Applications

The methods and compositions described herein can be used to, e.g., (a) predict the responsiveness or non-responsiveness of a subject (e.g., a human) to an anti-TNF therapy and/or (b) generate an anti-TNF therapy response profile for a subject. The profile can include information that indicates whether one or more genes, such as one or more genes depicted in Table 1, are expressed (e.g., yes or no) and/or information that indicates the expression level of one or more genes (e.g., one or more genes depicted in Table 1). The profile can also (or in the alternative) include information indicating the presence or absence of one or more SNP genotypes depicted in Table 2-4 or 13. An anti-TNF therapy response profile can include the expression level of one or more additional genes and/or one or more additional SNP genotypes.

The response profiles described herein can contain information on the expression or expression level of at least two or more (e.g., at least three or more, at least four or more, at least five or more, at least six or more, at least seven or more, at least eight or more, at least nine or more, at least 10 or more, at least 11 or more, at least 12 or more, at least 13 or more, at least 14 or more, at least 15 or more, at least 16 or more, at least 17 or more, at least 18 or more, at least 19 or more, at least 20 or more, at least 21 or more, at least 22 or more, at least 23 or more, or at least 24 or more) genes depicted in Table 1.

Grouping of multiple genes (e.g., 2, 3, 4, 5, 6, 7, 8, 9, 10, 11, 12, 13, 14, 15, 16, 17, 18, 19, 20, 21, 22, 23, or 24 or more genes depicted in Table 1) into sets or clusters can improve the sensitivity or specificity of the classifiers for response or non-response of a subject to an anti-TNF therapy. For example, the accompanying Examples describe the grouping of the genes depicted in Table 1 into 8 clusters. A group of genes comprising individual genes selected from each of the clusters can then be tested for predictive accuracy and the classifiers can be recalculated based on the group of genes. This can result in, e.g., a classifier (e.g., an 8 gene or a 24 gene classifier) with an improved accuracy of prediction as compared to a single gene-based classifier. Any grouping of genes from each cluster can lead to a more accurate classifier. Genes within each cluster can in many cases be substituted for one another without significantly compromising accuracy. Furthermore, any gene depicted in Table 1 could be added back to the grouping (e.g., the grouping of 8 genes or 24 genes).

It is understood that some genes (e.g., some of the genes depicted in Table 1) can be expressed in a cell- or tissue-specific manner. However, such differences in expression can be exploited in several ways. For example, in embodiments where a particular cell type (e.g. neutrophils, monocyte, or B cells) is being analyzed, it can be useful to select one or more gene (or a group of genes) that are expressed in that cell type. Such a strategy can also be useful, e.g., where a biological sample contains multiple cell or tissue types, but only a particular cell or tissue type is to be analyzed. Thus, selecting a particular set of genes expressed only, or predominantly, in the target cell type of interest can provide more a focused prediction.

The response profiles described herein can also (or alternatively) include information on the presence or absence of at least two or more (e.g., at least three or more, at least four or more, at least five or more, at least six or more, at least seven or more, at least eight or more, at least nine or more, at least 10 or more, at least 11 or more, at least 12 or more, at least 13 or more, at least 14 or more, at least 15 or more, at least 16 or more, at least 17 or more, at least 18 or more, at least 19 or more, at least 20 or more, at least 21 or more, at least 22, or at least 24 or more) SNP genotypes depicted in Tables 2-4 or 13.

The resultant information (anti-TNF therapy response profile) can be used for predicting the response of a subject (e.g., a human patient) to an anti-TNF therapy, such as any of the anti-TNF therapies described herein. In addition, the response profiles can be used in predicting the response of a subject to a variety of therapies and/or a variety of disease states since, e.g., the expression levels of one or more of the genes (e.g., one or more of the genes depicted in Table 1) examined can be indicative of such responses or disorders, whether or not physiologic or behavioral symptoms of the disorder have become apparent.

Responsiveness (and, conversely, non-responsiveness) of a subject to a therapy comprising an anti-TNF agent can be classified in several ways and classification is dependent on the subject's disease (e.g., rheumatoid arthritis, Crohn's disease, or any other of the diseases treatable by therapy comprising an anti-TNF agent), the severity of the disease, and the particular medicament the subject is administered. For example, responsiveness of a subject (e.g., a human) with rheumatoid arthritis can be classified as achieving at least about 20% (e.g., at least about 25%, at least about 30%, at least about 35%, at least about 40%, at least about 45%, at least about 50%, at least least 55%, at least about 60%, at least about 65%, or at least about 70% or more) improvement in one or more (e.g., two or more, three or more, four or more, five or more, or more than six) of a number of objective clinical indicia (e.g., American College of Rheumatology (ACR)-core set measures) such as, but not limited to, tender joint count, swollen joint count, pain, global self-assessment of improvement by a subject, global assessment of improvement of the subject by a physician, improvement of a subject's disability, or presence or amount of an acute-phase reactant (e.g., as determined by erythrocyte sedimentation rate (ESR) or presence or level of C-reactive protein). That is, a subject can be classified as responsive to an anti-TNF therapy if, e.g., he or she exhibits about 20% improvement in tender and swollen joint counts and 20% improvement in 3 of the 5 remaining ACR-core set measures (see above). In some instances, a subject is classified as responsive to an anti-TNF therapy using an ACR criteria of 20% (ACR20%), 50% (ACR50%), or 70% (ACR70%). Such improvements can be compared to, e.g., the responsiveness of the same subject to a placebo.

The responsiveness of a subject to an anti-TNF therapy can also be classified using a Disease Activity Score (DAS or DAS28), which is a measure of the number of swollen and tender joints and the ESR or CRP. For example, the DAS28 can be measured by analyzing the number of swollen joints, the number of tender joints, the ESR (in mm/hr) and the VAS (visual analog scale) general health of the patient. Under DAS criteria, responsiveness of a subject to an anti-TNF therapy is a DAS improvement of at least about 1.2 (e.g., at least about 1.3, at least about 1.4, at least about 1.5, at least about 1.6. at least about 1.7, at least about 1.8, at least about 1.9, at least about 2.0, at least about 2.1, at least about 2.2, at least about 2.3, at least about 2.4, at least about 2.5 or at least about 2.6 or more), e.g., at least about 1 month (e.g., at least about 1.5 months, at least about 2.0 months, at least about 2.5 months, at least about 3.0 months, at least about 4.0 months, at least about 5 months, or at least about 6 months). The results of the DAS and the DAS28 are not directly interchangeable as the DAS has a range varying from 1 to 9 and the DAS28 has a range of from 2 to 10; however, a transformation formula can be used to calculate the DAS28 from the DAS: DAS28= (1,072 x DAS) + 0,938.

Additional information regarding the DAS (e.g., methods to calculate the DAS) and ACR classifications of responsiveness of a subject to an anti-TNF therapy can be found in, e.g., Braun et al. (2003) Ann. Rheum. Dis. 62: 1023-1024; Felson et al. (1995) Arthritis and Rheumatism 38(6); Verhoeven et al. (2000) 59:966-974; at the American College of Rheumatology website; or at the website for the Department of Rheumatology, University Medical Centre Nijmegen, Netherlands.

Subjects of all ages can be affected by disorders treatable by an anti-TNF therapy. For example, the first symptoms of Crohn's disease can manifest in early adolescence to mid-life. Therefore, a biological sample used in a methods described herein can be obtained from a subject (e.g., a human) of any age, including a child, an adolescent, or an adult, such as an adult having, or suspected of having, a disease treatable by an anti-TNF therapy (e.g., rheumatoid arthritis). The methods can also be applied to individuals at risk of developing a disorder treatable by an anti-TNF therapy. Such individuals include those who have (i) a family history of (a genetic predisposition for) such disorders or (ii) one or more risk factors for developing such disorders.

The methods described herein can involve, e.g., assessing the expression level (e.g., mRNA or protein expression level) of one or more genes (e.g., one or more genes depicted in Table 1), wherein the expression level of one or more of the genes predicts the response of a subject to an anti-TNF therapy. "Assessing" can include, e.g., comparing the expression of one or more genes in a test biological sample with a known or a control expression level (e.g., in a reference biological sample) of the particular gene(s) of interest. For example, the expression level of one or more genes in a test biological sample can be compared to the corresponding expression levels in a healthy subject, or an average expression level of multiple (e.g., two, three, four, five, six, seven, eight, nine, 10, 15, 20, 25, 30, 35, or 40 or more) healthy subjects, of the same species. The control sample can also be the expression level (or average expression level) of one or more subjects who have either responded to an anti-TNF therapy or not responded to an anti-TNF therapy. Assessing can also include determining if the expression level of one or more genes (e.g., one or more genes as depicted in Table 1) falls within a range of values predetermined as predictive of responsiveness or non-responsiveness of a subject to an anti-TNF therapy. In some embodiments, assessing can be, or include, determining if the expression of one or more genes (e.g., one or more of the genes depicted in Table 1) falls above or below a predetermined cut-off value. A cut-off value is typically an expression level of a gene, or ratio of the expression level of a gene with the expression level of another gene, above or below which is considered predictive of responsiveness or non-responsiveness of a subject to an anti-TNF therapy or, e.g., cause for retest. Thus, in accordance with the methods (and compositions) described herein, a reference expression level of a gene (e.g., a gene depicted in Table 1) is identified as a cut-off value, above or below of which is predictive of responsiveness or non-responsiveness to an anti-TNF therapy. It is understood that an anti-TNF therapy response profile can be interpreted as a whole (the expression level of all genes in the profile), in parts (certain collections or groups of genes (e.g., 8 or 24 genes) within the profile) or on a gene-by-gene basis.

Some cut-off values are not absolute in that clinical correlations can still remain significant over a range of values on either side of the cutoff; however, it is possible to select an optimal cut-off value (e.g. varying H-scores) of expression levels of genes for a particular sample types. Cut-off values determined for use in the methods described herein can be compared with, e.g., published ranges of expression levels but can be individualized to the methodology used and patient population. It is understood that improvements in optimal cut-off values could be determined depending on the sophistication of statistical methods used and on the number and source of samples used to determine reference level values for the different genes and sample types. Therefore, established cut-off values can be adjusted up or down, on the basis of periodic re-evaluations or changes in methodology or population distribution.

The reference expression level of one or more genes can be determined by a variety of methods. The reference level can be determined by comparison of the expression level of a gene of interest in, e.g., populations of subjects (e.g., patients) that are healthy, responsive to an anti-TNF therapy, or not responsive to an anti-TNF therapy. This can be accomplished, for example, by histogram analysis, in which an entire cohort of patients are graphically presented, wherein a first axis represents the expression level of a gene and a second axis represents the number of subjects in the cohort whose sample contain one or more expression levels at a given amount. Determination of the reference expression level of a gene can then be made based on an amount which best distinguishes these separate groups. The reference level can be a single number, equally applicable to every subject, or the reference level can vary, according to specific subpopulations of subjects. For example, older subjects can have a different reference level than younger subjects for the same metabolic disorder. In addition, a subject with more advanced disease (e.g., a more advanced form of a disease treatable by an anti-TNF therapy) can have a different reference value than one with a milder form of the disease.

Methods for determining reference expression levels of one or more genes are detailed in the accompanying Examples.

After predicting that a subject will respond or will not respond to an anti-TNF therapy, a medical practitioner (e.g., a doctor) can select the appropriate therapeutic modality for the subject (e.g., an anti-TNF therapy or a non-anti-TNF therapy, respectively). Selecting a therapy for a subject can be, e.g.: (i) writing a prescription for a medicament; (ii) giving (but not necessarily administering) a medicament to a subject (e.g., handing a sample of a prescription medication to a patient while the patient is at the physician's office); (iii) communication (verbal, written (other than a prescription), or electronic (email, post to a secure site)) to the patient of the suggested or recommended therapeutic modality (e.g., an anti-TNF therapy or a non-anti-TNF therapy); or (iv) identifying a suitable therapeutic modality for a subject and disseminating the information to other medical personnel, e.g., by way of patient record. The latter (iv) can be useful in a case where, e.g., more than one therapeutic agent are to be administered to a patient by different medical practitioners.

It is understood that an anti-TNF response profile can be in electronic form (e.g., an electronic patient record stored on a computer or other electronic (computer-readable) media such as a DVD, CD, or floppy disk) or written form. The anti-TNF response profile can also include information for several (e.g., two, three, four, five, 10, 20, 30, 50, or 100 or more) subjects (e.g., human patients). Such multi-subject response profiles can be used, e.g., in analyses (e.g., statistical analyses) of particular characteristics of subject cohorts.

After predicting that a subject will respond or will not respond to an anti-TNF therapy, a medical practitioner (e.g., a doctor) can administer the appropriate therapeutic modality to the subject (e.g., an anti-TNF therapy or a non-anti-TNF therapy, respectively). Methods of administering anti-TNF therapies such as an anti-TNF antibody or soluble TNF receptor are known in the art and described in, e.g., U.S. Patent Nos. 5,656,272; 6,193,969; 5,919,452, the disclosures of each of which are incorporated herein by reference in their entirety. Likewise, methods for administering non-anti-TNF therapies such as a soluble lymphotoxin beta receptor or anti-CD20 antibody are known in the art and described in, for example, U.S. Patent Nos. 6,403,087; 5,925,351; 6,896,885; and 7,060,667, the disclosures of each of which are incorporated by reference in their entirety.

It is understood that any therapy described herein (e.g., a therapy comprising an anti-TNF agent or a therapy that does not comprise an anti-TNF therapy) can include one or more additional therapeutic agents. That is, any therapy described herein can be co-administered (administered in combination) with one or more additional therapeutic agents such as, but not limited to, one or more of the non-anti-TNF agents described herein. For example, a therapy comprising an anti-TNF agent can include more than one anti-TNF agent (e.g., a soluble TNF receptor and an anti-TNF antibody). A therapy comprising an anti-TNF agent can also include, e.g., one or more non-anti-TNF agents such as methotrexate (MTX). Likewise, a non-anti-TNF therapy can include two or more non-anti-TNF agents, e.g., a TWEAK inhibitor and an IL-6 inhibitor or an IL-6 inhibitor and MTX. Furthermore, any therapy described herein can include one or more agents for treating, for example, pain, nausea, and/or one or more side-effects of a therapy comprising an anti-TNF agent or a non-anti-TNF therapy.

Combination therapies (e.g., co-administration of a therapy comprising an anti-TNF agent or non-anti-TNF therapy and one or more additional therapeutic agents) can be, e.g., simultaneous or successive. For example, an anti-TNF agent and one or more additional therapeutic agents can be administered at the same time or an and-TNF agent can be administered first in time and the one or more additional therapeutic agents administered second in time. In some embodiments, the one or more additional therapeutic agents can be administered first in time and the anti-TNF agent administered second in time.

In cases where the subject predicted to respond to an anti-TNF therapy has been prior administered one or more non-anti-TNF therapies, the therapy comprising an anti-TNF agent can replace or augment a previously or currently administered therapy. For example, upon treating with the therapy comprising an anti-TNF agent, administration of the one non-anti-TNF therapies can cease or diminish, e.g., be administered at lower levels. Administration of the previous therapy can be maintained while the therapy comprising the anti-TNF agent is administered. In some embodiments, a previous therapy can be maintained until the level of the therapy comprising an anti-TNF agent reaches a level sufficient to provide a therapeutic effect.

In other embodiments, the subject can be monitored for a first pre-selected result, e.g., an improvement in one or more symptoms of a disease treatable by a therapy comprising an anti-TNF agent, e.g., rheumatoid arthritis or any other diseases treatable by therapy comprising an anti-TNF agent described herein. In some embodiments, when the first pre-selected result is observed, treatment with the therapy comprising an anti-TNF agent can be decreased or halted. In some embodiments, the subject can then be monitored for a second pre-selected result after treatment with the therapy comprising an anti-TNF agent is halted, e.g., a worsening (e.g., a worsening of a symptom) of a disease treatable by an anti-TNF agent. When the second pre-selected result is observed, administration of the therapy comprising an anti-TNF agent to the subject can be reinstated or increased, or administration of the first therapy can be reinstated, or the subject can be administered both a therapy comprising an anti-TNF agent, or an increased amount of a therapy comprising an anti-TNF agent, and the first therapeutic regimen.

<u>Arrays and Kits</u>

Nucleic acid arrays and kits including the nucleic acid arrays are useful in, e.g., detecting (and/or measuring) gene expression levels or detecting the presence of one or more SNP genotypes. The kits and compositions are also useful for predicting the response of a subject to an anti-TNF therapy.

The nucleic acid arrays can include at least two (e.g., at least three, at least four, at least five, at least six, at least seven, at least eight, at least nine, at least 10, at least 11, at least 12, at least 15, at least 20, at least 22, or at least 24) polynucleotides that hybridize to each of at least two (e.g., at least three, at least four, at least five, at least six, at least seven, at least eight, at least nine, at least 10, at least 11, at least 12, at least 15, at least 20, at least 22, or at least 24, respectively) genes depicted in Table 1 or at least two SNP genotypes depicted in Tables 2-4 or 13. A polynucleotide can include coding sequence or non-coding sequence (e.g., exons, introns, or 5' or 3' regulatory sequences), e.g., of a gene depicted in Table 1. The polynucleotide can include sequence of the sense strand or the anti-sense strand of the coding sequence of a gene depicted in Table 1 or a gene depicted in Tables 2-4 or 13. The polynucleotide can also be single or double-stranded and of variable length. In some embodiments, the length of one strand of a polynucleotide that hybridizes to a gene (e.g., depicted in Table 1) or a SNP genotype (e.g., depicted in Tables 2-4 or 13) can be about six nucleotides (e.g., about seven nucleotides, about eight nucleotides, about nine nucleotides, about 10 nucleotides, about 12 nucleotides, about 13 nucleotides, about 14 nucleotides, about 15 nucleotides, about 20 nucleotides, about 25 nucleotides, about 30 nucleotides, about 35 nucleotides, about 40 nucleotides, about 50 nucleotides, about 75 nucleotides, about 100 nucleotides, or about 150 or more nucleotides) in length. A longer polynucleotide often allows for higher stringency hybridization and wash conditions. The polynucleotide can be DNA, RNA, modified DNA or RNA, or a hybrid, where the nucleic acid contains any combination of deoxyribo- and ribo-nucleotides, and any combination of uracil, adenine, thymine, cytosine and guanine, as well as other bases such as inosine, xanthine, and hypoxanthine.

The polynucleotide arrays can be attached to a solid support, e.g., a porous or non-porous material that is insoluble. The substrate can be associated with the support in variety of ways, e.g., covalently or non-covalently bound.

A support can be composed of a natural or synthetic material, an organic or inorganic material. The compositions of the solid support on which the polynucleotide sequences are attached (either 5' or 3' terminal attachment) generally depend on the method of attachment (e.g., covalent attachment). Suitable solid supports include, but are not limited to, plastics, resins, polysaccharides, silica or silica-based materials, functionalized glass, modified silicon, carbon, metals, inorganic glasses, membranes, nylon, natural fibers such as silk, wool and cotton, or polymers. The material comprising the solid support can have reactive groups such as carboxy, amino, or hydroxyl groups, which are used for attachment of the polynucleotides. Polymeric solid supports can include, e.g., polystyrene, polyethylene glycol tetraphthalate, polyvinyl acetate, polyvinyl chloride, polyvinyl pyrrolidone, polyacrylonitrile, polymethyl methacrylate, polytetrafluoroethylene, butyl rubber, styrenebutadiene rubber, natural rubber, polyethylene, polypropylene, (poly)tetrafluoroethylene, (poly) vinylidenefluoride, polycarbonate, or polymethylpentene (see, e.g., U.S. Patent No. 5,427,779, the disclosure of which is hereby incorporated by reference in its entirety). Alternatively, the polynucleotide sequences can be attached to the solid support without the use of such functional groups.

Each polynucleotide (of a plurality of polynucleotides) on an array can be immobilized at predetermined positions such that each polynucleotide can be identified by its position. Exemplary polynucleotide arrays for use in the methods and kits described herein are described in, e.g., U.S. Patent Nos. 6,197,599; 5,902,723; and 5,871,928; the disclosures of each of which are incorporated herein by reference in their entirety).

In some embodiments of any of the arrays described herein, the array of polynucleotides can have less than 100,000 (e.g., less than 90,000; less than 80,000; less than 70,000; less than 60,000; less than 50,000; less than 40,000; less than 30,000; less than 20,000; less than 15,000; less than 10,000; less than 5,000; less than 4,000; less than 3,000; less than 2,000; less than 1,500; less than 1,000; less than 750; less than 500, less than 200, less than 100, or less than 50) different polynucleotides.

The polynucleotide arrays can also be conjugated to solid support particles. Many suitable solid support particles are known in the art and illustratively include, e.g., particles, such as Luminex®-type encoded particles, magnetic particles, and glass particles.

Exemplary particles that can be used can have a variety of sizes and physical properties. Particles can be selected to have a variety of properties useful for particular experimental formats. For example, particles can be selected that remain suspended in a solution of desired viscosity or to readily precipitate in a solution of desired viscosity. Particles can be selected for ease of separation from sample constituents, for example, by including purification tags for separation with a suitable tag-binding material, paramagnetic properties for magnetic separation, and the like.

In some embodiments, encoded particles are used. Each particle includes a unique code (such as a bar code, luminescence code, fluorescence code, a nucleic acid code, and the like). Encoding can be used to provide particles for evaluating different nucleic acids in a single biological sample. The code is embedded (for example, within the interior of the particle) or otherwise attached to the particle in a manner that is stable through hybridization and analysis. The code can be provided by any detectable means, such as by holographic encoding, by a fluorescence property, color, shape, size, weight, light emission, quantum dot emission and the like to identify particle and thus the capture probes immobilized thereto. Encoding can also be the ratio of two or more dyes in one particle that is different than the ratio present in another particle. For example, the particles may be encoded using optical, chemical, physical, or electronic tags. Examples of such coding technologies are optical bar codes fluorescent dyes, or other means. In some embodiments, the particle code is a nucleic acid, e.g., a single stranded nucleic acid.

Different encoded particles can be used to detect or measure multiple nucleic acids (e.g., SNP genotypes or mRNAs) in parallel, so long as the encoding can be used to identify the polynucleotide (corresponding to an analyte nucleic acid) on a particular particle, and hence the presence or amount of the analyte nucleic acid (e.g., a SNP genotypes or mRNA from a biological sample) being evaluated. A sample can be contacted with a plurality of such coded particles. When the particles are evaluated, e.g., using a fluorescent scanner, the particle code is read as is the fluorescence associated with the particle from any probe used to evaluate modification of the intact substrate associated with the particles.

One exemplary platform utilizes mixtures of fluorescent dyes impregnated into polymer particles as the means to identify each member of a particle set to which a specific capture probe has been immobilized. Another exemplary platform uses holographic barcodes to identify cylindrical glass particles. For example, Chandler et al. (U.S. Patent No. 5,981,180) describes a particle-based system in which different particle types are encoded by mixtures of various proportions of two or more fluorescent dyes impregnated into polymer particles. Soini (U.S. Patent No. 5,028,545) describes a particle-based multiplexed assay system that employs time-resolved fluorescence for particle identification. Fulwyler (U.S. Patent No. 4,499,052) describes an exemplary method for using particle distinguished by color and/or size. U.S. Publication Nos. 2004-0179267, 2004-0132205, 2004-0130786, 2004-0130761, 2004-0126875, 2004-0125424, and 2004-0075907 describe exemplary particles encoded by holographic barcodes.

U.S. Patent No. 6,916,661 describes polymeric microparticles that are associated with nanoparticles that have dyes that provide a code for the particles. The polymeric microparticles can have a diameter of less than one millimeter, e.g., a size ranging from about 0.1 to about 1,000 micrometers in diameter, e.g., 3-25 μm or about 6-12 μm. The nanoparticles can have, e.g., a diameter from about 1 nanometer (nm) to about 100,000 nm in diameter, e.g., about 10 - 1,000 nm or 200 - 500 nm.

Also provided are kits containing any of the nucleic acid arrays described herein. The kits can, optionally, contain instructions for detecting and/or measuring the level of one or more genes in a biological sample or instructions for detecting one or more SNP genotypes (e.g., one or more SNP genotypes depicted in Tables 2-4 or 13).

The kits can optionally include, e.g., a control biological sample or control labeled-amplicon set containing known amounts of one or more amplicons recognized by nucleic acid probes of the array. In some instances, the control can be an insert (e.g., a paper insert or electronic medium such as a CD, DVD, or floppy disk) containing expression level ranges of one or more genes predictive of a response to an anti-TNF therapy.

In some embodiments, the kits can include one or more reagents for processing a biological sample. For example, a kit can include reagents for isolating mRNA or genomic DNA from a biological sample and/or reagents for amplifying isolated mRNA (e.g., reverse transcriptase, primers for reverse transcription or PCR amplification, or dNTPs) and/or genomic DNA. The kits can also, optionally, contain one or more reagents for detectably-labeling an mRNA, mRNA amplicon, genomic DNA or DNA amplicon, which reagents can include, e.g., an enzyme such as a Klenow fragment of DNA polymerase, T4 polynucleotide kinase, one or more detectably-labeled dNTPs, or detectably-labeled gamma phosphate ATP (e.g., $^{33}$P-ATP).

In some embodiments, the kits can include a software package for analyzing the results of, e.g., a microarray analysis or expression profile.

The kits can also include one or more antibodies for detecting the protein expression of any of the genes described herein. For example, a kit can include (or in some cases consist of) a plurality of antibodies capable of specifically binding to one or more proteins encoded by any of the genes depicted in Table 1 and optionally, instructions for detecting the one or more proteins and/or a detection antibody comprising a detectably-labeled antibody that is capable of binding to at least one antibody of the plurality. In some embodiments, the kits can include antibodies that recognize at least two (e.g., three, four, five, six, seven, eight, nine, 10, 11, 12, 13, 14, 15, 16, 17, 18, 19, 20, 21, 22, 23, or 24) proteins encoded by genes depicted in Table 1.

The kits described herein can also, optionally, include instructions for administering an anti-TNF therapy where the expression level of one or more genes, or the presence of one or more SNP genotypes, detectable by the array predicts that a subject will response to an anti-TNF therapy. The kits can contain instructions for administering a variety of non-anti-TNF therapies where the expression level of one or more genes, or the presence of one or more SNP genotypes, detectable by the array predicts that a subject will not respond to an anti-TNF therapy.

The following are examples of the practice of the invention. They are not to be construed as limiting the scope of the invention in any way.

## EXAMPLES

### Example 1. Materials and Methods

*Genetic Analysis Methods*: Clinical data: DAS28 scores were obtained for 109 patients at the baseline; and for 104 patients at 14 weeks. A difference in DAS28 score at the baseline and 14 weeks was calculated to measure a response to anti-TNFs therapies. Table 5 lists descriptive statistics for the DAS28 scores at baseline (DAS_v1) and 14 weeks (DAS_v3) and the difference of these score (ΔDAS_v1_v3: DAS_v1_DAS_v3, a positive ΔDAS_v1_v3 indicates a good response to anti-TNF therapies). ΔDAS_v1_v3 was used as the dependent variable to evaluate associations between SNP markers and response/non-response to the anti-TNF therapies.

Table 5: DAS28 scores at baseline, 14 weeks and difference at the two points: descriptive statistics

| | DAS_v1 | DAS_v3 | ΔDAS_v1_v3 |
|---|---|---|---|
| **N** | 109 | 108 | 104 |
| **Mean** | 5.23 (0.79) | 3.78 (1.27) | |
| **Skewness** | -0.24 | 0.2 | -0.05 |
| **Kurtosis** | -0.07 | -0.38 | 0.08 |

"Skewness" and "kurtosis" measure the normality of a distribution. Kurtosis is the "peakedness" of a probability distribution. Skewness is the deviation of a probability distribution away from a central peak. Skewness and kurtosis of the values in Table 5 are low, indicating the DAS28 and ΔDAS28 values are normally distributed.

*Genotyping methods*: Genomic DNA was purified from whole blood leukocytes by standard methods. For the genome-wide association studies, approximately 750ng of genomic DNA was used to genotype each sample on the Illumina® HumanHap300 BeadChip (Illumina®, San Diego). Samples were processed according to the Illumina® Infinium 2 assay manual. Briefly, each sample was whole-genome amplified, fragmented, precipitated and resuspended in appropriate hybridization buffer. Denatured samples were hybridized on prepared HumanHap300 BeadChips for a minimum of 16 hours at 48°C. Following hybridization, the beadchips were processed for the single base extension reaction, stained and imaged on an Illumina® Bead Array Reader. Normalized bead intensity data obtained for each sample were loaded into the Illumina® Beadstudio 2.0 software which converted fluorescent intensities into Single Nucleotide Polymorphism (SNP) genotypes.

Table 6: 317,000 SNPs chip: distribution on each chromosome

| chr | # SNPs | chr | # SNPs | chr | # SNPs | chr | # SNPs |
|---|---|---|---|---|---|---|---|
| 1 | 23240 | 7 | 16653 | 13 | 11510 | 19 | 5905 |
| 2 | 25334 | 8 | 18266 | 14 | 9825 | 20 | 7833 |
| 3 | 21558 | 9 | 15816 | 15 | 8889 | 21 | 5488 |
| 4 | 19106 | 10 | 15576 | 16 | 8990 | 22 | 5491 |

(continued)

| chr | # SNPs | chr | # SNPs | chr | # SNPs | chr | # SNPs |
|---|---|---|---|---|---|---|---|
| 5 | 19238 | 11 | 14650 | 17 | 8326 | X | 9168 |
| 6 | 20772 | 12 | 15016 | 18 | 10482 | XY | 2 |

To ensure SNP marker quality and reduce the possibility of putative associations, data cleaning procedures were performed on all 317,000 SNPs before association studies were carried out. The distribution of the SNP number on each chromosome is shown in Table 6. The major cleaning procedures include 3 check-ups on each marker. All calculations were carried out using the R software package (Version 2.2.1).

1) Call rates (i.e., the proportion of subjects was genotyped for a certain SNP marker or the proportion of SNPs was genotyped for a certain subject) were calculated to ensure if the DNA samples and SNP markers were in good quality. Bad DNA samples and SNP markers were not used for subsequent statistical analysis.
2) Hardy-Weinberg Equilibrium (HWE) checking is used to ensure the study subjects were obtained from a randomly mating population at each marker locus. The Fisher's exact test probability is applied to testing for HWE p-values. p = 0.001 was used as a threshold to measure if the SNP marker failed the HWE test.
3) A SNP marker may contain two alleles and three genotypes. Minor allele frequencies (MAFs) were calculated to ensure a marker has enough variation (i.e., there are enough subjects in genotype groups) in the population level for statistical analysis.

Analysis of variance (ANOVA) model was carried out to evaluate an association between a SNP marker and response to anti-TNF therapies (i.e., ΔDAS_v1_v3). Phenotypic means of ΔDAS_v1_v3 were compared for different genotype groups of a SNP marker. Because more than 317,000 tests were involved in this study, permutation test was carried out to account for multiple testing issues. To obtain adjusted genome wide p-values, the phenotypic values were randomly shuffled to break relationship between phenotype and genotype. The entire analysis was repeated on the shuffled data; therefore, the shuffled data was representative of the null hypothesis. The smallest p-values were obtained from N = 1,000 permutation iterations. An empirical p-value (or permutation p-value) for an association was obtained by the following

$$\text{equation: } \frac{\#\, p-values\ from\ \ permutation \leq the\ \ observed\ \ \ p-value}{1000}.$$

<u>Example 2. Genetic data</u>

P-values for 317,000 single nucleotide polymorphisms (SNPs) from the ANOVA model were plotted for 22 chromosomes (Fig. 1). The x-axis represents chromosomes 1 to 22, the y-axis represents the $-\log_{10}$(p-value). Each blue dot represents a p-value for an association. Two parallel lines to the x-axis representing $10^{-5}$ and $10^{-7}$ levels of significance were drawn in the plot. The p-values between $10^{-5}$ and $10^{-7}$ were plotted using the symbol of asterisk.

For each SNP (Tables 7A and 7B), a genotyping test can be carried out to predict a patient's response to anti-TNF treatment. Genotypes corresponding to a negative or low ΔDAS_v1_v3 are predictive of non-response to anti-TNF treatment. Genotypes corresponding to positive or high ΔDAS_v1_v3 are predictive of response to anti-TNF. For example, for SNP rs7046653, the AA genotype is associated with a poor response to treatment, while the GG genotype is associated with a good response. Nearly all of the SNPs exhibit additive models of inheritance: each copy of an individual allele increases or decreases the response to anti-TNF treatment, with the heterozygotes showing an intermediate response. The exceptions to this additive inheritance are the SNPs on chromosome 8. For these SNPs, the heterozygous genotypes are associated with more extreme phenotypes than either of the homozygotes.

<u>Table 7A. SNPs with ANOVA p-values < 10-4 for ΔDAS_v1_v3</u>

| Chr | SNP | P-Values | | Genotype | N | Mean * (ΔDAS_ v1_v3) | Stderr (ΔDAS_ v1_v3) |
|---|---|---|---|---|---|---|---|
| | | ANOVA | Perm. | | | | |
| 1p22 | rs2170331 | 0.000074 | 0.72 | A/A | 5 | -0.88 | 0.53 |
| | | | | A/G | 37 | 2.48 | 0.56 |
| | | | | G/G | 60 | 2.47 | 0.55 |

(continued)

| Chr | SNP | P-Values | | Genotype | N | Mean * (ΔDAS_ v1_v3) | Stderr (ΔDAS_ v1_v3) |
|---|---|---|---|---|---|---|---|
| | | ANOVA | Perm. | | | | |
| | rs6665006 | 0.000074 | 0.72 | A/A | 60 | 1.59 | 0.15 |
| | | | | A/G | 37 | 0.01 | 0.25 |
| | | | | G/G | 5 | -2.47 | 0.55 |
| 5q35 | rs1422422 | 0.000019 | 0.24 | A/A | 9 | 2.82 | 0.39 |
| | | | | A/G | 59 | -1.17 | 0.42 |
| | | | | G/G | 34 | -2 | 0.44 |
| | rs4976592 | 0.000039 | 0.4 | A/A | 4 | 3.16 | 0.58 |
| | | | | A/G | 55 | -1.36 | 0.61 |
| | | | | G/G | 43 | -2.25 | 0.61 |
| 7q22 | rs1968201 | 8.3E-06 | 0.09 | A/A | 85 | 1.72 | 0.13 |
| | | | | A/G | 17 | -1.46 | 0.31 |
| | rs3087615 | 0.000024 | 0.24 | A/C | 15 | 0.25 | 0.3 |
| | | | | C/C | 85 | 1.46 | 0.33 |
| 9p21 | rs7046653 | 0.000002 | 0.03 | A/A | 5 | -0.03 | 0.51 |
| | | | | A/G | 46 | 1.03 | 0.54 |
| | | | | G/G | 51 | 2.07 | 0.53 |
| | rs868856 | 0.000002 | 0.03 | A/A | 5 | -0.03 | 0.51 |
| | | | | A/G | 46 | 1.03 | 0.54 |
| | | | | G/G | 51 | 2.07 | 0.53 |
| | rs2814707 | 0.000006 | 0.08 | A/A | 4 | -0.71 | 0.58 |
| | | | | A/G | 36 | 1.75 | 0.61 |
| | | | | G/G | 61 | 2.59 | 0.6 |
| | rs774359 | 0.000007 | 0.08 | A/A | 59 | 1.88 | 0.15 |
| | | | | A/G | 39 | -0.81 | 0.24 |
| | | | | G/G | 4 | -2.6 | 0.59 |
| | rs3849942 | 0.000021 | 0.22 | A/A | 4 | -0.71 | 0.58 |
| | | | | A/G | 37 | 1.81 | 0.61 |
| | | | | G/G | 61 | 2.55 | 0.6 |

*Chr.* refers to the chromosomal location.
*SNP* refers to the SNP identifier.
*Mean* refers to the genotypic mean values of ΔDAS_v1_v3 for different genotypes
*Stderr* refers to the standard error for the genotypic means

A negative mean of ΔDAS_v1_v3 indicates non-response to anti-TNF therapies. *Perm.* refers to the p-value for permutation test.

Table 7B. SNPs with ANOVA p-values < 10-4 for ΔDAS_v1_v3

| Chr | SNP | P-Vales | | Genotype | N | Mean (ΔDAS_ v1_v3) | Stderr (ΔDAS_ v1_v3) |
|---|---|---|---|---|---|---|---|
| | | ANOVA | Perm. | | | | |
| 4p14 | rs437943 | 0.000015 | 0.17 | A/A | 35 | 2.26 | 0.2 |
| | | | | A/G | 59 | -1.15 | 0.25 |
| | | | | G/G | 7 | -1.55 | 0.48 |
| | rs1441209 | 0.000039 | 0.39 | A/A | 56 | 1.97 | 0.16 |
| | | | | A/G | 42 | -1.06 | 0.24 |
| | | | | G/G | 4 | -1.45 | 0.61 |
| | rs6531358 | 0.000092 | 0.78 | A/A | 57 | 1.95 | 0.16 |
| | | | | A/G | 41 | -1.05 | 0.24 |
| | | | | G/G | 4 | -1.44 | 0.61 |
| | rs2028446 | 0.000053 | 0.54 | A/A | 4 | 0.51 | 0.59 |
| | | | | A/G | 41 | 0.4 | 0.62 |
| | | | | G/G | 56 | 1.45 | 0.61 |
| 8q22 | rs11778767 | 0.000023 | 0.21 | A/A | 15 | 2.29 | 0.3 |
| | | | | A/G | 43 | -1.45 | 0.35 |
| | | | | G/G | 44 | -0.48 | 0.35 |
| | rs11780500 | 0.000023 | 0.21 | A/A | 44 | 1.81 | 0.18 |
| | | | | A/G | 43 | -0.97 | 0.25 |
| | | | | G/G | 15 | 0.48 | 0.35 |
| | rs4562286 | 0.000023 | 0.21 | A/A | 15 | 2.28 | 0.3 |
| | | | | A/G | 43 | -1.44 | 0.35 |
| | | | | G/G | 44 | -0.47 | 0.35 |
| | rs3019293 | 0.000048 | 0.41 | A/A | 16 | 2.2 | 0.29 |
| | | | | A/G | 42 | -1.36 | 0.34 |
| | | | | G/G | 44 | -0.39 | 0.34 |

*Chr.* refers to the chromosomal location.
*SNP* refers to the SNP identifier.
*Mean* refers to the genotypic mean values of ΔDAS_v1_v3 for different genotypes
*Stderr* refers to the standard error for the genotypic means
A negative mean of ΔDAS_v1_v3 indicates non-response to anti-TNF therapies.
*Perm*. refers to the p-value for permutation test.

The genotypic data presented here were all obtained using allele specific amplification protocols on an Illumina® Beadstation.

Example 3. Genetic Data

Methods

**Patients.** The Autoimmune Biomarkers Collaborative Network (ABCoN) was established in order to explore the use of new technologies for biomarker discovery in both RA and Systemic Lupus (SLE). The ABCoN Rheumatoid Arthritis cohort includes 116 active RA patients followed prospectively to evaluate efficacy of the three available anti-TNF agents. In order to examine the response to anti-TNF therapy in RA, blood samples, laboratory and clinical data were collected at baseline (prior to anti-TNF therapy), 6 weeks, 3 months, 6 months and 1-year post treatment. DNA, RNA, peripheral blood cells, plasma, serum and urine were obtained at the time of each study visit. All the patients satisfied the ACR 1987 revised criteria for RA, and provided written informed consent. The study protocols were approved by local ethics committees. Among the 116 patients, 102 subjects provided DNA samples for genotyping.

**Efficacy measurements.** Disease severity was evaluated using the DAS28 score which is the Disease Activity Score that includes 28-joint counts and C-reactive protein (Prevoo et al. (1995) Arthritis and rheumatism 38: 44-48). DAS28 was measured at three time points: baseline, 6 weeks, and 14 weeks. Two scales were considered to evaluate efficacy of anti-TNF treatment. First, a relative improvement in disease activity was calculated for each patient using the DAS28 scores at baseline and at week 14:

$$relDAS28 = \left(\frac{DAS28visit1 - DAS28visit3}{DAS28visit1}\right)\%.$$

*RelDAS28* has a continuous scale and is approximately normal. Second, according to the EULAR definition published elsewhere (van Gestel et al. (1998) Arthritis and rheumatism 41: 1845-1850), patients are classified as good, moderate or non-responders, using the individual amount of change in the DAS28 (ΔDAS28) and DAS28 values at 14 weeks (van Gestel et al., *supra*). Briefly, a good responder is classified if ΔDAS28 ≥ 1.2 and DAS28 at 14 weeks ≤ 3.2; a moderate responders are patients with (ΔDAS28 ≥ 1.2 and DAS28 at 14 weeks > 3.2) or (0.6 <ΔDAS28 ≤ 1.2 and DAS28 at 14 weeks ≤ 5.1). Patients are classified as non-responders if they do not fall into any of these categories van Gestel et al., *supra*).

**Genotyping and quality control.** The patients' genomic DNA was extracted from peripheral blood using standard protocols. 317,000 Single Nucleotide Polymorphisms (SNPs) on 102 anti-TNF treated patients were genotyped using an Illumina Beadstation and Illumina HAP300 chips according to the IlluminaInfinium 2 assay manual (Illumina) (Duerr et al. (2006) Science 314: 1461-1463). The HAP300 chip includes, on average, a SNP every 10 kilobases across all the autosomes, and interrogates approximately 87% of the common genetic variation in populations of European descent (Pe'er et al. (2006) Nat Genet 38: 663-667).

To ensure SNP marker quality and reduce the possibility of false associations, quality control procedures were performed on each of the 317,000 SNPs before association tests were carried out. The SNP set was filtered on the basis of genotype call rates (≥ 95%), minor allele frequency (MAF (≥ 0.05). Hardy-Weinberg equilibrium (HWE) was calculated for individual SNPs using the Exact test. All of the SNPs reported in this manuscript have HWE p-values > 0.001. After filtering, 283,348 polymorphic SNPs were analyzed on chromosome 1 through chromosome 22. The average call rate for the filtered SNPs was 99.5%. Patients were removed if their percentage of missing genotypes was more than 5% or if there was evidence of possible contamination in their DNA sample.

**Statistical analysis.** Associations between SNP markers and response to the anti-TNF therapies were evaluated in two stages. In the first stage of our analysis, we used *relDAS*28 as a continuous dependent variable to evaluate the associations. Because the sample size is relatively small in our study, a continuous scale of the dependent variable has more power than a categorized variable. Linear regression was carried out to evaluate association between individual SNP markers and response to anti-TNF therapies (i.e., *relDAS*28) in the context of additive genetic effect model. A t-statistic was derived from regression and used to evaluate association between individual SNP markers and response to anti-TNF therapies (i.e., *relDAS*28). The t-statistic is robust, and thus some departure from normality for the dependent variable is acceptable. Because more than 283,348 tests were involved in this study, a permutation test was carried out to account for multiple testing on each chromosome. The permutation test can also address the slight deviation from normality in the dependent variable. To obtain adjusted p-values, the phenotypic values were randomly shuffled to break the relationship between phenotype and genotype. The entire analysis was repeated on the shuffled data; therefore, the shuffled data is representative of the null hypothesis. The 1,000 smallest p-values were obtained from each of the N = 1,000 permutation iterations for the whole set of SNPs on individual chromosomes.

In the second stage analysis, a categorized dependent variable - response status to the anti-TNF therapy (i.e., non-responders vs. good responders) was used to evaluate association with SNPs selected from the first stage. The probability of being a non-responder was modeled using logistic regression with additive genetic effect model. Unless specified, all calculations for statistical analysis were carried out using the R software package (Version 2.2.1).

Population admixture (i.e., sampling of subjects from two or more subpopulations) has been recognized as a major cause for inconsistent results and spurious associations for genetic studies (Deng et al. (2001) Genetics 159: 1319-1323 and Seldin et al. (2008) PLoS genetics 4: e5). U.S. populations are genetically admixed. Although self-reported ethnicity data was recorded in this study, it is incomplete and may be inaccurate. To accurately classify individuals according to ancestry and to remove any possible related individuals, pair-wise identity-by-state (IBS) distance for the 102 subjects was calculated and subsequent complete linkage agglomerative clustering and multidimensional scaling were performed using genome-wide SNP markers in Plink software (version 1.00, Shaun Purcell et al., Center for Human Genetic Research (CHGR), Massachusetts General Hospital (MGH), and the Broad Institute of Harvard University and Massachusetts Institute of Technology (MIT)). Clustering data were plotted to identify major population subdivisions. In addition to removing outliers from the dataset, the potential effect from subpopulations (e.g. northern and southern Europeans) was further evaluated using the EIGENSTRAT program with genome wide SNP data (Price et al. (2006) Nat Genet 38: 904-909). The top ten principal components (PCs) were obtained. Correlation analysis between the top PCs and the phenotypes *(delDAS*28 and dichotomous response status to anti-TNF) was performed to detect if the phenotypic difference among individuals were due to population stratification. If the spread of samples in these principal components was purely due to population stratification, it can be removed by forcing all samples to have zero value in these principal components. Then a "virtual" genotype can be obtained by rotating the corrected principal components back to the original genotype space. Pearson's chi-square test was performed for association between selected SNPs and response status to the anti-TNF therapy (i.e., non-responders vs. good responders)

Results

**Characteristics of Patients.** Among the 102 active RA patients with complete genotypic and phenotypic data, self-reported ancestry included 83 Europeans, four Asians, three American Africans, as well as three subjects with reported Latino ethnicity. Nine patients have missing information for their ethnicity. Linkage agglomerative clustering and multidimensional scaling identified 89 patients with primarily European ancestry (Fig. 2), among them 83 were self-reported to be white and 6 had missing ethnicity data. These 89 patients were used for subsequent association analysis.

The baseline characteristics of the 89 patients are summarized in Table 8. At the time of diagnosis, they were 47±14 (mean±S.D) yr old, their disease duration was 8±9 years, 75% were women, and 15% were current smokers, and the average serum CRP level (mg/dl) at baseline was 1.7 (S.D. = 2.0). On average, the DAS28 at the baseline (before anti-TNF therapy) was 5.22 (S.D. = 0.80), indicating that the RA disease activity was high for most of the patients (van Gestel et al., *supra*). 54 subjects were treated with Etanercept, 37 with Infliximab, and 25 with Adalimumab.

Table 8. Characteristics of patients for 89 patients of European descent

| Characteristic | European descent (N=89) |
|---|---|
| Age at diagnosis | 47 ± 15 |
| Women: (%) | 75 |
| Disease duration (yr) | 8 ± 8 |
| Current Smokers (%) | 15 |
| Pain VAS | 50.0 ± 22.3 |
| Health VAS | 46.4 ± 17.5 |
| Tender (no.) | 11.8 ± 6.2 |
| Swollen (no.) | 11.2 ± 4.8 |
| HAQ | 1.13 ± 0.61 |
| Physician's global assessment | 49.4 ± 19.0 |
| RF at baseline | 238.2 ± 369.6 |
| Serum CRP level (mg/dl) at baseline | 1.7 ± 2.0 |
| CCP | 127.4 ± 143.4 |
| DAS 28 at baseline | 5.22 ± 0.80 |
| DAS 28 at 6 weeks | 3.99 ± 1.13 |
| DAS 28 at 14 weeks | 3.72 ± 1.32 |

**Genome-wide association studies: 1st stage analysis.** The GWA analyses were performed to test for association between 283,348 polymorphic SNPs and the relative change in DAS28 (*relDAS*28) using an additive genetic model. The principal components in the regression model were not further adjusted because they did not significantly correlate with *relDAS*28 (i.e. the major phenotypic difference among individuals was not due to population stratification) (Table 9).

Table 9. No significant correction between the top 10 principle components and changes in DAS 28 score (delDAS28)

| | PC1 | PC2 | PC3 | PC4 | PC5 | PC6 | PC7 | PC8 | PC9 | PC10 |
|---|---|---|---|---|---|---|---|---|---|---|
| Pearson correlation p-value | 0.72 | 0.63 | 0.14 | 0.93 | 0.52 | 0.16 | 0.55 | 0.92 | 0.95 | 0.2 |

The chromosomal distribution of p-values for the genome wide association is shown in Fig. 3. To address multiple testing issues, a chromosome-wide permutation test was performed. Sixteen SNPs remain significant (permutation exact p (≤ 0.05) or borderline significant (0.05 < permutation exact p≤0.1) after permutation test to obtain exact significance levels. The p-values along with annotation information for these 16 SNPs are shown in Table 10.

Table 10. Sixteen SNPs with smallest p-values with permutation exact p-values ≤ 0.1

| SNP | P-value[i] of *RelDAS28* | Permut. p-value (*RelDAS28*) | Polymorphism | Allele associated with non-response (Allele Freqnency) | Chr | Physical position | P-value Non-responders vs. Good responders (Adjusted)[4] | | Odds Ratio (OR) being non-responder (95% CI)[2] |
|---|---|---|---|---|---|---|---|---|---|
| rs983332 | 0.000005 | 0.008 | A/C | A (0.21) | 1 | 87844401 | 0.00007 | (0.00009) | 10.2 (2.6, 59.2) |
| rs928655 | 0.00003 | 0.07 | A/G | A (0.77) | 1 | 89561595 | 0.0009 | (0.0004) | 5.5 (1.8, 20.2) |
| rs13393173 | 0.000004 | 0.02 | A/G | A (0.12) | 2 | 169214598 | 0.004 | (0.02) | 6.8 (1.7, 40.3) |
| rs437943 | 0.000004 | 0.1 | A/G | G (0.33) | 4 | 35194664 | 0.0007 | (0.002) | 4.6 (1.8, 12.3) |
| rs10945919 | 0.0000003 | 0.004 | A/G | G (0.32) | 6 | 164157088 | 0.0007 | (0.0008) | 4.6 (1.8, 12.3) |
| rs854555 | 0.000002 | 0.03 | A/C | A (0.34) | 7 | 94575042 | 0.0006 | (0.001) | 4.6 (1.8,12.3) |
| rs854548 | 0.000003 | 0.06 | A/G | A (0.27) | 7 | 94570471 | 0.00004 | (0.0003) | 8.5 (2.6, 36.5) |
| rs854547 | 0.000006 | 0.1 | A/G | A (0.63) | 7 | 94568507 | 0.003 | (0.004) | 3.6 (1.5, 9.3) |
| rs7046653 | 0.0000005 | 0.01 | A/G | A (0.26) | 9 | 27480967 | 0.0004 | (0.002) | 4.9 (1.8, 14.0) |
| rs868856 | 0.0000005 | 0.01 | C/T | T (0.26) | 9 | 27479251 | 0.0005 | (0.002) | 4.9 (1.8,14.0) |
| rs774359 | 0.0000006 | 0.01 | C/T | C (0.22) | 9 | 27551049 | 0.0005 | (0.005) | 5.4 (1.9,17.3) |
| rs2814707 | 0.000002 | 0.04 | A/G | A (0.22) | 9 | 27526397 | 0.0006 | (0.007) | 5.2 (1.8, 16.7) |
| rs3849942 | 0.000005 | 0.07 | A/G | A (0.21) | 9 | 27533281 | 0.001 | (0.01) | 5.0 (1.7,15.8) |
| rs6028945 | 0.0000002 | 0.003 | G/T | T (0.12) | 20 | 38254219 | 0.0004 | (0.006) | 11.2 (2.3, 108.1) |
| rs6138150 | 0.000003 | 0.05 | C/T | T (0.84) | 20 | 23795009 | 0.0002 | (0.002) | 11.1 (2.5, 103.3) |
| rs6071980 | 0.000003 | 0.05 | C/T | C (0.13) | 20 | 38301990 | 0.0009 | (0.01) | 7.6 (1.9, 44.6) |

Among these sixteen SNPs, four are located within genes, one is in the 3'UTR, and eleven are in the flanking regions of genes. All these SNPs are common polymorphisms (minor allele frequency > 0.1). In addition to Table 10, data for 2985 SNPs with *relDAS*28 p-values ≤ 0.01 is depicted in Table 9. The Illumina annotation file "HumanHap317K_geneannotation.txt" together with the UCSC Genome Browser were used to annotate SNP details (Kent et al. (2002) Genome Res 12: 996-1006).

Among these leading associations, the rs6028945 marker ~500Kb 3' of the MAFB locus on chromosome 20 is marginally the strongest association (p=0.003 corrected) when the relative change in DAS28 is considered as a continuous variable; a second marker in the region of MAFB, rs6071980 also shows evidence of association (p=0.05, corrected). Likewise, multimarker evidence of association is seen with markers in the Paraoxinase 1 gene (PON1) as well as in a region of chromosome 9 that contains the interferon kappa (IFNK), MOBKL2B and and C9orf72 loci. Other loci showing some evidence of association include a guanylate nucleotide binding protein (GBP6) at 1p22.2, LAG1 (longevity assurance homolog 6, LASS6) at chr2, cystatin D (CST5) at 20p11.21, centaurin, delta 1 (CENTD1) at 4p14, quaking homolog, and KH domain RNA binding (QK1) at 6q26-q27 (Table 2).

**Association between SNPs and response to anti-TNF therapy: 2$^{nd}$ stage analysis.** According to the criteria of EULAR (van Gestel, *supra*), the 89 patients under study can be categorized into three groups: 23 non-responders, 31 good responders and 35 moderate responders using the baseline DAS28 and the change in DAS28 at 14 weeks. Allele frequencies were compared between non-responders and good responders using the Fisher's exact test (Fisher (1922) Journal of the Royal Statistical Society 85: 87-94). The last two columns of Table 10 display Fishers exact p-values and Odds Ratios (ORs) with 95% confidence intervals (CIs) for the non-responder status to the anti-TNF therapies. Note that these 95% CIs are very wide, reflecting the small sample size (i.e., 23 non-responders vs. 31 good-responders) in this study. The major principal components did not appear to predict good responder vs. nonresponder status (Figs. 4A and 4B). Because the sample size is quite small (23 non-responders vs. 31 responders), a minor effect due to subpopulation may have large impact on the 2X2 table estimate. Therefore, subpopulation structure was further adjusted to give adjusted Chi-square p-values using EIGENSTRAT. As shown in Table 10, these adjusted Chi-square p-values are generally larger than the Fisher's exact test p-values.

**SNPs in candidate genes.** Of the markers present on the Illumina HapMap 300 chip, four SNP associations with TNF response have been reported previously in candidate gene studies: rs1800896 in IL10 (Padyukov et al. (2003) Ann Rheum Dis 62: 526-529 and Turner et al. (1997) Eur J Immunogenet 24: 1-8), rs419598 in interleukin 1 receptor antagonist (EL1RN) (Marotte et al. (2006) Ann Rheum Dis 65: 342-347), rs1041981 in LTA (Kang et al. (2005) Rheumatology (Oxford) 44: 547-552; Criswell et al. (2004) Arthritis and rheumatism 50: 2750-2756; and Koss et al. (2000) Genes Immun 1: 185-190), and rs4149570 in TNFRSF1A (Criswell et al., *supra*). These markers were evaluated separately in view of their increased prior probability. In the current study, only rs1800896 shows evidence of association, with a p-value of 0.0132 (uncorrected) with the *delDAS*28 and 0.0183 (corrected for stratification only) with responding status to anti-TNF therapy.

Example 4. Whole blood gene expression profiles from RA patients predict non-response to anti-TNFs therapy

**Clinical Evaluation of Patients**. Human patients with active disease fulfilling the American College of Rheumatology (ACR) criteria and were previously naïve to anti-TNF agents were enrolled in the study. 116 patients beginning anti-TNF therapies (54 etaneracept, 25 adalimumab, 37 infliximab) were consented for this study. Clinical data and baseline gene expression profiles were available for 75 of these patients. The inclusion criteria were: 18 years of age or older, meeting the 1987 ACR criteria, disease activity of at least 6 swollen joints, no previous exposure to anti- TNF medication for a six month duration. The exclusion criteria for our study were those who take 10 mg or more oral steroid therapy per day at the time of enrollment in this study. Peripheral blood samples were collected to isolate RNA using PaxGene tubes. The clinical information required for evaluating response to therapy was also recorded for each patient at baseline and 14 weeks post-treatment. The blood of 42 healthy controls was also collected and profiled using the same procedures. Written informed consent was obtained from all subjects.

**Blood sample acquisition and RNA processing.** Peripheral blood was collected directly into Paxgene tubes according to manufacturer's specifications (Qiagen, Valencia, CA). Blood was collected from RA patients during three visits: pre-treatment, 6 weeks, and 14 weeks post-treatment. Peripheral blood from 89 healthy control patients, for a single time point, was also collected into Paxgene tubes. Total RNA was extracted using the RNeasy kit according to manufacturer's specifications (Qiagen, Valencia, CA). For detailed methods see, e.g., Batliwalla et al, 2005.

**RNA labeling and microarray hybridization.** Sample labeling and hybridization were carried out using the NuGEN Ovation Biotin systems (NuGEN Technologies, Inc, San Carlos, CA) as described in the NuGEN Ovation Biotin system for 96 well plates (version 5). Washing, staining, and scanning of the hybridized arrays was completed as described in the Eukaryotic Target Preparation protocol in the Affymetrix® expression analysis technical manual (702064 rev 2) for Genechip® cartridge arrays using the Genechip® Array Station (Affymetrix, Santa Clara, CA). In summary, 20ng of PaxGene (preAnalytiX GmbH, Hombrechtikon, Germany) purified total RNA in 2μL was annealed with 2μL "first

strand primer mix" (A1) at 65°C for 5 minutes and chilled on ice. The annealed template was incubated at 48°C for 60 minutes with 6μL of first strand master mix (A2 and A3) and then chilled to 4°C. To the completed first stand reaction, 10μL of second strand master mix (B1 and B2) was added and the reaction incubated at 37°C for 30 minutes, 75°C for 15 minutes, and then cooled to 4C. To amplify cDNA, 120μL of SPIA™ master mix (Nugen, San Carlos, CA) was added the 20μL completed second strand reaction and the reaction incubated at 48°C for 60 minutes. The amplified cDNA was purified in using the 96 well dye terminator removal kit (AB gene, Surrey, UK-cat# AB-0943/A) according to the manufacturer's recommendations. To fragment the purified and amplified cDNA, 5μL of fragmentation buffer (F1) and enzyme mix (F2) was added to 25μL and the reaction incubated at 50°C for 30 minutes and then chilled to 4C. Biotin labeling of the fragmented cDNA was completed by addition 5μL biotin labeling buffer (F3) and 2.5μL of labeling enzyme mix (F4) with incubation at 50°C for 30 minutes and then cooled to 4C. The fragmented and labeled cDNA was then purified using the 96 well dye terminator removal kit (AB gene, Surrey, UK-cat# AB-0943/A) according to the manufacturer's recommendations. Three μg of fragmented labeled cDNA was resuspended in 300 μL 1X hybridization buffer containing 100 mM MES (2-(N-morpholino)ethanesulfonic acid), 1 M [Na+], 20 mM EDTA, 0.01% Tween 20,0.5 mg/mL Aceylated BSA, 0.1 mg/mL herring sperm DNA, control oligo B2, and control transcripts bioB 1.5 pM, bioC 5 pM, bioD 25 pM, and cre 100 pM, and hybridized to Human Genome U133 plus 2.0 Genechip® probe arrays according to manufacturer's protocol (Affymetrix®, Santa Clara, CA). The hybridized GeneChip® probe arrays were washed and stained using Streptavidin-Phycoerythrinin (Molecular Probes, Eugene, OR) and amplified with biotinylated anti-streptavidin (Vector Laboratories, Burlingame, CA) (Sigma, Saint Louis, MO) GeneChip® Fluidics Station 400 (Affymetrix, Santa Clara, CA) using an antibody amplification protocol. The GeneChip® probe arrays were scanned using GeneArray Scanner 3000 (Hewlett Packard, Corvallis, OR).

**Gene Expression Data Acquisition and analysis.** Following GeneChip hybridization and scanning, signals from the scans were normalized using the CGRMA algorithm (Li et al. (2001) Genome Biol 2, RESEARCH0032; Irizarry et al. (2003) Nucleic Acids Res 31: e15; and Wu et al. (2004) Journal of the American Statistical Association 99: 909-917). All calculations were performed using R and Bioconductor computational tools (Genetelman et al. (2005) Bioinformatics and Computational Biology Solutions using R and Bioconductor (Springer)). Development of predictors for anti-TNF response from gene expression required several data reduction steps. To identify genes correlated with anti-TNF response over time, the general linear modeling tool implemented in R (Genetelman et al. (2005) Bioinformatics and Computational Biology Solutions using R and Bioconductor (Springer)) was used. The significance of the linear model was assessed by t- statistic and for each random sample selection and gene this t-value was recorded. To further characterize differentially expressed genes, genes were identified that have significantly different expression levels between any of the three patient groups: non-responders, medium, responders and the normal controls, plus those genes that are significantly different between non-responders and responders. A linear modeling approach was applied to the patient data grouping the samples into those 4 pre-defined groups. Standard linear Modeling data analysis approach (MANOVA) was applied to identify genes with a significantly different expression. The significance of each difference was evaluated using t-statistics. In order to take into account the multiple hypotheses testing effect, the significance of the fold changes were evaluated using the Bayesian analysis of variations in the samples, groups, and genes (Smyth, G. K. (2004) Stat. Appl. Genet. Mol. Biol. 3, Article3). The genes with a significance p-value< 0.05 of Bayesian posterior probability of being differentially expressed were selected. The selection also required that the genes have fold changes of at least 1.5.

When sets of suitable transcripts were identified from the data reduction methods, they were further selected to include only genes with biological annotation in publicly available genome databases. This gene set was used to develop predictors of anti-TNF response using the Random Forest machine learning method (Breiman, L. (2001) Machine Learning 45: 5-32). For each patient identified as a "RESPONDER" or "NON-RESPONDER," gene expression levels were determined by GeneChip® RMA (GCRMA; Affymetrix®). The Random Forest approach builds a number of decision trees from randomly selected k-genes and about 2/3 of the patient samples. The algorithm repeats this process many times while a priori user can choose any k to select random k variables for each tree. For each variable (gene expression) the Random Forest approach calculates its importance to the predictions. Given the randomness of the forest construction, the importance of a gene is not a deterministic value. That is the importance of a gene can vary depending on the content of the forest.

**Initial data processing and gene filtering.** Gene expression profiles from whole blood collected pre-treatment from 116 RA patients using the Affymetrix HGU133Plus2 chip. In addition to the RA patients we have collected and profiled blood from 42 healthy controls. The patients were treated with anti-TNF drugs (Remicade, Enbrel, Humira). Disease severity was evaluated using the DAS28 score and EULAR classification (Fransen et al. (2005) Clin Exp Rheumatol 23: S93-99). Patients were followed up 8 weeks and 14 weeks after the baseline visit and blood was collected at those 3 visits. Patients' disease score was assessed at those visits. Typically patients with moderate to severe RA are treated with anti-TNF and in consequence in our patient cohort there were no good responders only non-responders and moderate responders. According to EULAR classification good responders are patients with mild disease (DAS28 < 3.2) who improved their DAS28 by at least 40% when compared to the baseline.

The 14 weeks after baseline visit (visit3) was selected to identify in our cohort the good responders in our group as it is a common time-point for making clinical decisions about effectiveness of therapy. The DAS28 score improvement was calculated for each patient using the following formula:

$$\Delta DAS28 = \%(DAS28visit1 - DAS28visit3).$$

Following the EULAR classification (van Gestel et al. (1998), *supra*), responders, intermediate responders, and non-responders were defined. For the purpose of predictor development, the patient cohort can thus consist of three groups: non-responders (22 patients), intermediate responders (29 patients), and responders (24 patients).

Samples from all patients and healthy controls were processed using first GCRMA data normalization method (Li et al. (2001) Genome Biol 2, RESEARCH0032; Irizarry et al. (2003) Nucleic Acids Res 31: e15; and Wu et al. (2004) Journal of the American Statistical Association 99: 909-917). Data was also independently processed using RMA (Li et al. (2001) Genome Biol 2, RESEARCH0032; Irizarry et al. (2003) Nucleic Acids Res 31, e15; and Wu et al. (2004) Journal of the American Statistical Association 99: 909-917). All calculations were done using R and Bioconductor computational tools (see, e.g., Genetelman et al. (2005) Bioinformatics and Computational Biology Solutions using R and Bioconductor (Springer)).

As the preliminary step of data reduction the MAS5 algorithm (as implemented in Bioconductor) was used to identify transcripts present in the samples. It was required that transcripts were called present with p.value < 0.01 in at least 50% of the samples belonging to one of the four groups: controls, responders, intermediate responders and non-responders. Thus the subsequent analysis was applied to 23,686 transcripts considered as present.

The first step of data reduction identified all baseline gene expressions that significantly correlated with relative DAS28 score. The general linear modeling tool implemented in R (Gentelman et al., *supra*) was used to model ΔDAS28 scores by gene expression values using all baseline samples: medium, responders and non-responders. The significance of the linear model fit was assessed by t-statistic. For each gene and a random selection of 90% of samples a t-value was recorded. The random sampling and fitting procedure were repeated 100 times for each gene. From the significance of those 100-modelling steps, the mean significance for each gene was calculated by removing the top and bottom 5% of the highest and lowest t-values. In addition, patient responses were randomly permuted 100 times and fitted the expression values to the permuted responses. The t.values of the permuted fits generated a distribution of expected by chance fit. Using this random distribution, the fit of the true response values was evaluated to determine if it was significantly different form a random fit by more then two standard deviations. 894 gene probes were selected as correlating significantly with relDAS28 score with the p-value < 0.025 for the fit (t.value better then 2), and significant when compared to fits generated with permuted response. Since these calculations are CPU intensive, the data was processed using the 100 nodes (200 CPU) LINUX cluster.

As a second step in our evaluation of the candidate genes we have identified those that have significantly different expression between any of the three patient groups: non-responders, medium, responders and the normal controls, plus those genes that are significantly different between non-responders and responders. We have applied a linear modeling approach to patient data grouping the samples into those 4 pre-defined groups. We have then applied standard linear Modeling data analysis approach (MANOVA) to identify genes with a significantly different expression. The significance of each difference was evaluated using t-statistics. In order to take into account the multiple hypotheses we further evaluated the significance of the fold changes using the Bayesian analysis of variations as implemented by the limma package (Smyth, G. K. (2004) Stat Appl Genet Mol Biol 3, Article3). Genes with a significance p-value < 0.05 of Bayesian posterior probability of being differentially expressed gene and with at least 1.5 fold difference were selected. The total number of gene-probes determined as significantly differentially expressed with above requirements was 7,972.

From the 894 genes that were significantly correlated with relDAS28 score 291 genes were also considered as being differentially expressed by selection described above. We further selected from those 291 gene probes 166 probes that had the log2 expression value as estimated by the GCRMA of at least 6 and only those genes that have been reviewed in the ENTREZ NCBI human genome database. That is we chose only those genes that had a name assigned and excluded probe sets not associated with an annotated gene or an open reading frame pending human review.

**Finding optimum parameters for random forest.**

The 166 gene probes constituted the starting point for construction of the predictor of the anti-TNF response between the responder and non-responder patients. The random forest (Brieman (2001) Machine Learning 45: 5-32) machine learning method was applied to these probes to identify the best predictor or set of predictors.

Two parameters that are important in random forest algorithm are number of trees used in the forest (*ntree*) and number of random variables used in each split (*mtry*). To find the number of trees required for a stable random forest classifier, the random forest test was run with different *ntree* values *i.e. ntree* = {1, 500, 1000...., 120,000}. 10 runs were performed for each *ntree* value, recorded OOB error rate and calculated the median and standard deviation of error rate. The optimum *ntree* was selected for which the standard deviation of error stabilized.

To find the optimum *mtry*, a similar procedure was applied such that RF was run 10 times for a range of *mtry* values which consist of multiples of the default *mtry* $(\sqrt{\text{number of variables}} = 22)$ starting from 0.05x*mtry* up to *mtry* = number of samples with increments of 0.05. The OOB error rate was recorded was each run. Optimum *mtry* value(s) were selected for which the error rate has stabilized and reached to minimum. In particular, *mtry* values were first ranked according to OOB error rate then ranked based on the following formula:

$$\text{abs}(\text{m.OOB}[mtry_i] - \text{m.OOB}[mtry_{i-1}) + \text{abs}(\text{m.OOB}[mtry_{i+1}] - \text{m.OOB}[mtry_i]) + \text{std.OOB}[mtry_i] \quad (i = 2,n),$$

where m.OOB[$mtry_i$] is the median OOB error rate for ith mtry, n is the total number of *mtry* values, and std.OOB is the standard deviation of the error rate. *mtry* values that satisfy both the lowest error rate and the highest stability (lowest value from the equation) criteria were selected as optimum values.

**Selecting genes consistently important in prediction.** A "convergent random forest" algorithm was developed in order to select genes that are consistently important in prediction. The steps of the algorithm are:

1. Run random forest 10 times.
2. Select genes that are considered important in all 10 runs (variable importance > 0)
3. Repeat 1-2 until the number of genes selected does not change.
4. Repeat 1-3 for different *mtry* values selecting a "convergent" gene set for each *mtry* value.
5. Select the common genes between the 'convergent' gene sets.

**Selecting minimum number of genes with maximum prediction accuracy.** A series of steps were carried out to find a non-redundant set with minimum number of genes and maximum accuracy using selection techniques based on importance ranking and clustering. The selection techniques and the whole workflow are outlined below.

***Selection based on importance ranking***

1. Rank the genes according to variable importance in decreasing order: $g_1$, $g_2$,.., $g_n$
2. Set x = 1.
3. Select x top genes, run RF and record OOB error.
4. Set x = x + 1. Repeat from Step 3 until x = n.
5. Select x which has minimum OOB error rate.

***Selection based on clustering***

1. Cluster the genes using the correlation of the gene expression values as the distance matrix.
2. Set k = 2.
3. Cut the tree into k clusters. From each cluster select the gene with the highest variable importance (k genes in total).
4. Set k = k+1. Repeat from Step 3 until k = x (see *Selection based on importance ranking*)
5. Select k-genes which have minimum OOB error rate.

*Complete procedure to select minimum genes:*

for *i* = 1:50 {

- Select genes based on importance ranking: $\text{predictive_gene_set}_{i,j}$. Record x as $x_i$.
- Select genes based on clustering: $\text{predictive_gene_set}_{i,2}$. Record k as $k_i$. }

- Combine predictive_gene_set$_{i,1}$ and predictive_gene_set$_{i,2}$.
- Calculate Gene Predictive Index (GPI) for each gene *g*:

$$GPI(g)=\left(\sum_i \frac{1-OOB[x_{min}]}{\left|G_i^{x_{min}}\right|}+\sum_i \frac{1-OOB[k_{min}]}{\left|G_i^{k_{min}}\right|}\right)/\max(i)$$

where NPG is the Number of Predictive Genes in run i where gene g is selected either by importance ranking or by clustering. Where $OOB[x_{min}]$ is the minimal error rate and $|G_i^{x_{min}}|$ the number of best genes selected by importance. Similarly, $OOB[k_{min}]$ is the minimal error rate and $|G_i^{k_{min}}|$ the number of genes selected by cluster cutting. Thus $NPG_i = x_i$ if gene *g* is selected by importance ranking and $NPG_i = k_i$ if it is selected by clustering. Thus if the number of predictive genes including gene g identified in that run is small, that gene is given a higher index. Moreover, as the frequency of appearance of the gene in the runs increases, GPI of the gene also increases (through summation).

- Rank the genes according to GPI in decreasing order.
- Perform steps 2-5 outlined in *Selection based on importance ranking.*

**Different classification algorithms.** A final set of genes were run with different classification algorithms to compare the performances with respect to random forest. k nearest neighbor classifier (kNN) is run with k ∈ {1, 3, 5, ..., 21} and k with the smallest error was chosen. SVM with radial kernel was run with combinations of cost parameter c ∈ {1, 2, 4} and the parameter $\gamma \in \{2^{-1}/18, 1/18, 2/18\}$ as suggested by Gentleman et al. (Bioinformatics and Computational Biology Solutions using R and Bioconductor. New York, Springer). A random forest test was run with 100,000 trees and default *mtry*. The analysis was performed using MLInterfaces package in R (Bioconductor).

Example 5. Construction of a Stable Predictor with Minimum Number of Genes and Maximum Performance

Initial data processing and gene filtering resulted in 166 gene probes which are significantly correlated with the anti-TNF response and differentially expressed between responders and non-responders as well as between patients and normal controls. Starting from 166 gene probes, the goal was to find a stable predictor with minimum number of genes and maximum performance.

This goal was achieved by completing two tasks: (1) To construct a stable random forest classifier and (2) to select 'important' yet minimum number of genes that should be used in the final predictor. Each of these tasks is elaborated on below in detail.

**1. Constructing a stable random forest classifier**

The following two questions were addressed to construct a stable RF classifier: How many trees are needed in the forest to have a stable classifier? How many random variables should be selected randomly per split?

First, the optimum number of trees for which a stable classifier exists was determined i.e., where the performance does not change as more trees are added to the forest. For this a random forest was run 10 times for different *ntree* values, each time recording OOB error rate. Then, the median and the standard deviation (std) across these 10 runs was calculated. An example run is shown in Fig. 6 where the median OOB error rate (Fig. 6A) and standard deviation (Fig. 6B) was plotted for each *ntree.* The Error rate quickly stabilized while the standard deviation continued to decline as more trees were added to the forest. Therefore, an *ntree* after which the standard deviation stabilized (Fig. 6B, *ntree* =15400) was determined. This procedure gives an initial estimate of the optimum *ntree* for a dataset however it should not be taken as a static value. The researcher should continually explore the robustness of the results as the dataset (number of genes and samples) changes and add more trees when necessary.

The next question was how the performance changes with *mtry.* For this, RF was run 10 times for a range of *mtry* values recording OOB error rate at each run. The change of median and standard deviation of error rate with *mtry* is shown for *ntree* = 40,000 (Fig. 7A). Since an *ntree* for which the standard deviation stabilized was selected at the previous step, the standard deviation does not change at all with *mtry* (Fig. 7B). After ranking *mtry* values based on error rate and stability, one can select an optimum *mtry* or a range of *mtry* values that are reasonably different from each other (*e.g.* ≥ 1.5 x previous *mtry*). The results proved that *mtry* effects the performance and it is therefore important to experiment different values and evaluate the consistency of the results.

The procedure was repeated with different *ntree* values as shown in Fig. 8. The change of OOB error rate with *mtry* is independent of *ntree* (see, e.g., Diaz-Uriarte et al. (2006) BMC Bioinformatics 7 (3)). Alternatively, the results can imply that the *ntree* range determined in the previous step is really optimum such that adding more trees has no effect on the error rate for the same *mtry* values.

**Gene selection.** After the optimum parameters have been determined for the random forest classifier, the next goal was to select genes that will be used in the final predictor. The final goal was to have a predictor with minimum number of genes and maximum prediction accuracy. Therefore, a set of genes that were informative, non-redundant, and consistently important in prediction were selected as described below.

*Selecting genes consistently important in prediction*

A frequency-based gene selection algorithm that takes into account both the variable importance and consistency was selected. The'convergent random forest' algorithm (see Materials and Methods II) was run using an *mtry* value of *mtry* = {45}. The algorithm resulted in a "convergent" set of 40 genes. The performance of this gene set was calculated with 10 independent RF runs. The results are shown in Fig. 9 for *ntree* = 16,000. The first panel shows the performance of all 166 genes as the initial gene set. In the second panel are the performances of the convergent sets obtained at each *mtry* value. The third panel of Fig. 9 shows the performance of the method when selecting top important genes as the same number as final converging genes.

Overall, the classifier with 40 genes selected by convergence resulted in an improved accuracy over the initial 166 gene set. The 40 genes are shown in Table 11.

Table 11.

| Gene Symbol | Log2 Fold difference Between Non-responders and responders | p-value | Log2 expression in healthy controls | Log2 expression in RA Moderate patients visit 1 | Log2 expression in RA non-responder patients visit 1 | Log2 expression in RA responder patients visit 1 |
|---|---|---|---|---|---|---|
| CLTB | -0.67 | 2.12E-04 | 5.68 | 5.96 | 5.85 | 6.52 |
| HDAC4 | 0.38 | 4.74E-04 | 8.65 | 9.13 | 9.34 | 8.96 |
| CXorf52 | 0.51 | 5.05E-04 | 8.32 | 7.89 | 8.13 | 7.62 |
| RBBP4 | -0.5 | 8.30E-04 | 6.49 | 5.99 | 5.80 | 6.30 |
| COL4A3BP | -0.45 | 9.20E-04 | 6.53 | 5.84 | 5.64 | 6.09 |
| C9orf80 | -0.46 | 9.79E-04 | 7.03 | 6.05 | 5.73 | 6.19 |
| ANKRD12 | 0.69 | 1.29E-03 | 7.24 | 7.86 | 8.08 | 7.39 |
| CAMK2G- | 0.58 | 1.45E-03 | 6.92 | 5.98 | 5.75 | 6.33 |
| PCBP2 | -0.37 | 1.55E-03 | 9.59 | 8.09 | 7.97 | 8.34 |
| COL4A3BP | -0.48 | 1.57E-03 | 6.54 | 5.87 | 5.60 | 6.08 |
| YIPF6 | -0.41 | 1.85E-03 | 8.29 | 7.21 | 6.88 | 7.28 |
| MYLIP | -0.52 | 2.58E-03 | 6.87 | 5.87 | 5.98 | 6.50 |
| ZNF294 | -0.27 | 4.78E-03 | 9.26 | 8.46 | 8.35 | 8.62 |
| RER1 | -0.26 | 4.79E-03 | 8.23 | 8.75 | 8.65 | 8.92 |
| CALM2 | -0.47 | 5.18E-03 | 5.51 | 6.75 | 6.58 | 7.05 |
| ARF5 | -0.42 | 5.96E-03 | 6.77 | 7.31 | 7.21 | 7.63 |
| ARF1 | -0.45 | 6.04E-03 | 6.05 | 5.45 | 5.21 | 5.66 |
| HDAC5 | -0.38 | 6.06E-03 | 5.38 | 5.87 | 5.74 | 6.11 |
| CASP5 | 0.9 | 6.34E-03 | 4.78 | 5.70 | 6.35 | 5.45 |

(continued)

| Gene Symbol | Log2 Fold difference Between Non-responders and responders | p-value | Log2 expression in healthy controls | Log2 expression in RA Moderate patients visit 1 | Log2 expression in RA non-responder patients visit 1 | Log2 expression in RA responder patients visit 1 |
|---|---|---|---|---|---|---|
| MXRA7 | 0.87 | 6.39E-03 | 5.45 | 6.26 | 6.55 | 5.68 |
| ANKIB1 | -0.38 | 6.89E-03 | 6.37 | 4.73 | 4.53 | 4.91 |
| BRWD2 | -0.61 | 7.84E-03 | 6.16 | 4.85 | 4.59 | 5.20 |
| FAM44A | 0.54 | 8.04E-03 | 9.22 | 7.69 | 8.00 | 7.45 |
| PGK1 | -0.31 | 9.75E-03 | 8.11 | 8.91 | 8.79 | 9.10 |
| ZFP36L1 | -0.88 | 9.95E-03 | 9.75 | 7.71 | 7.42 | 8.30 |
| SERF2 | -0.39 | 1.11E-02 | 9.14 | 9.89 | 9.72 | 0.11 |
| SERF2 | -0.39 | 1.18E-02 | 8.22 | 8.71 | 8.63 | 9.02 |
| SRGAP2 | -0.52 | 1.42E-02 | 6.37 | 4.18 | 4.03 | 4.55 |
| MNT | 0.26 | 1.79E-02 | 7.95 | 8.46 | 8.61 | 8.34 |
| SEL1L | 0.38 | 2.55E-02 | 10.00 | 19.50 | 19.68 | 9.30 |
| TUG1 | -0.27 | 2.72E-02 | 9.35 | 7.95 | 7.88 | 8.15 |
| LGALS9 | -0.44 | 2.81E-02 | 6.20 | 6.58 | 6.63 | 7.07 |
| SLC25A39 | -0.68 | 3.46E-02 | 9.06 | 9.02 | 8.25 | 8.93 |
| EEA1 | 0.32 | 3.70E-02 | 6.63 | 5.90 | 6.01 | 5.68 |
| EGLN2 | -0.26 | 5.97E-02 | 7.27 | 7.92 | 7.91 | 8.17 |
| RPA3 | -0.34 | 6.47E-02 | 7.56 | 6.63 | 6.39 | 6.73 |
| FOXJ3 | 0.24 | 6.76E-02 | 8.75 | 8.00 | 8.23 | 7.99 |
| DNAH1 | 0.24 | 8.55E-02 | 5.03 | 6.00 | 6.28 | 6.04 |
| PTCH1 | 0.48 | 1.55E-01 | 7.74 | 6.76 | 6.70 | 6.22 |
| SFRS2 | 0.15 | 1.74E-01 | 12.41 | 11.86 | 11.97 | 11.82 |

For the selection of the most accurate and non-redundant set of genes that differentiate between responders and non-responders. First, how the error rate changes with the number of genes selected by the importance measure was explored. For this, RF was run with the first ranked gene, then the second gene was added, and so on until all 166 genes were added sequentially. The results are shown in Fig. 10. The step-wise increase of the error rate was most probably due to the correlations between the genes. As the aim was to select a minimum number of non-redundant genes, a clustering procedure was developed to select uncorrelated, more informative, and less noisy gene set.

Starting with the convergent gene set (40 genes), first the procedure described above was repeated i.e., the change of error rate was calculated with the number of genes selected by the importance measure (Fig. 10). A minimum error rate (11%) was obtained for the top 24 genes (circled in Fig. 10). The top 24 genes were as follows: ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6. Then, 40 genes were clustered using the hierarchical clustering with the correlation between gene expressions as a measure of distance. The clustering dendrogram is shown in Fig. 5. Starting from the clustering tree, the tree was recursively cut into k = 2, 3, 4, 5.... up to the number of genes for which minimum error is obtained by importance ranking. Once the k-subclusters of genes were identified, genes were selected from those subclusters that have been judged as most important for classification. For k-subclusters, k-best genes for each k selection were selected. The performance of the RF predictors constructed from k-best genes is shown in Table 12. The selection of k = 8 resulted in the best performance of the RF predictor with the accuracy of 89%. The best 8 genes were: CLTB, COL4A3BP, CXorf52, FAM44A, MXRA7, PGK1, SFRS2, and YIPF6.

Different sets of k-best genes are selected in each run and the union of all those is a set of 24 best genes. The RF predictor build from all best genes has the accuracy of 87%.

**Table 12. Performance of the two best gene predictor sets. The prediction was done with the RF predictor and the accuracies were calculated with the OOB cross-validation.**

| Gene set | Predicted | Non-responder | Responder | |
|---|---|---|---|---|
| 8-genes | NON-RESPONDER | 20 | 2 | Specificity =91% |
| | RESPONDER | 3 | 21 | Sensitivity =88% |
| | Overall accuracy is 89% | NPV = 91% | PPV = 88% | |
| 24-genes | NON-RESPONDER | 19 | 3 | Specificity =86% |
| | RESPONDER | 3 | 21 | Sensitivity =88% |
| | Overall accuracy is 83% | NPV = 86% | PPV = 88% | |

Table 13. SNPs associated with Non-responsiveness to anti-TNF therapy

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs6028945 | 2.02E-07 | A/C | A (0.1214) | 20 | 38254219 | 0.00038 | 11.17 (2.32,108.09) |
| rs10945919 | 3.41E-07 | A/G | G (0.319) | 6 | 164157088 | 0.00067 | 4.56 (1.79,12.27) |
| rs868856 | 5.10E-07 | A/G | A (0.2619) | 9 | 27479251 | 0.00048 | 4.87 (1.83,13.95) |
| rs7046653 | 5.10E-07 | A/G | A (0.2619) | 9 | 27480967 | 0.00048 | 4.87 (1.83,13.95) |
| rs774359 | 6.04E-07 | A/G | G (0.2238) | 9 | 27551049 | 5.00E-04 | 5.43 (1.91,17.27) |
| rs854555 | 1.56E-06 | A/C | C (0.6619) | 7 | 94575042 | 6.00E-04 | 4.61 (1.8,12.78) |
| rs2814707 | 1.91E-06 | A/G | A (0.2163) | 9 | 27526397 | 0.00058 | 5.24 (1.83,16.67) |
| rs6138150 | 2.84E-06 | A/G | A (0.8381) | 20 | 23795009 | 0.00023 | 11.06 (2.45,103.25) |
| rs6071980 | 2.89E-06 | A/G | G (0.1286) | 20 | 38301990 | 0.00091 | 7.6 (1.9,44.59) |
| rs854548 | 3.25E-06 | A/G | G (0.7333) | 7 | 94570471 | 3.50E-05 | 8.48 (2.61,36.53) |
| rs13393173 | 3.66E-06 | A/G | A (0.1238) | 2 | 169214598 | 0.0036 | 6.82 (1.68,40.27) |
| rs437943 | 3.90E-06 | A/G | G (0.3381) | 4 | 35194664 | 0.00067 | 4.56 (1.79,12.27) |
| rs983332 | 4.55E-06 | A/C | A (0.2143) | 1 | 87844401 | 6.90E-05 | 10.26 (2.65,59.2) |
| rs3849942 | 4.68E-06 | A/G | A (0.2095) | 9 | 27533281 | 0.001 | 4.97 (1.73,15.85) |
| rs854547 | 5.60E-06 | A/G | A (0.6286) | 7 | 94568507 | 0.0029 | 3.58 (1.46,9.3) |
| rs1968201 | 7.30E-06 | A/G | G (0.9143) | 7 | 102401386 | 0.0038 | 8.18 (1.61,80.71) |
| rs616804 | 1.10E-05 | A/C | A (0.9143) | 12 | 97840812 | 0.0038 | 8.18 (1.61,80.71) |
| rs7334194 | 1.10E-05 | A/G | A (0.1333) | 13 | 105632881 | 0.014 | 5.38 (1.27,32.42) |
| rs6932793 | 1.10E-05 | A/G | G (0.1298) | 6 | 117878836 | 0.00034 | 11.56 (2.4,111.74) |
| rs3087615 | 1.20E-05 | A/C | A (0.9183) | 7 | 102546074 | 0.0072 | 7.57 (1.45,75.8) |
| rs3811272 | 1.30E-05 | A/G | A (0.2041) | 14 | 21811574 | 0.00075 | 5.66 (1.87,19.64) |
| rs1441209 | 1.40E-05 | A/G | G (0.2381) | 4 | 35161518 | 0.0014 | 4.68 (1.69,14.05) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2028446 | 1.40E-05 | A/G | A (0.2356) | 4 | 35171618 | 0.0014 | 4.68 (1.69,14.05) |
| rs6531358 | 1.60E-05 | A/G | G (0.2333) | 4 | 35178895 | 0.0014 | 4.68 (1.69,14.05) |
| rs1422422 | 1.70E-05 | A/G | G (0.6476) | 5 | 167665378 | 0.0014 | 4.04 (1.62,10.81) |
| rs5997597 | 1.90E-05 | A/G | A (0.9286) | 22 | 28959686 | 0.0044 | 12.58 (1.58,577.21) |
| rs4976592 | 2.10E-05 | A/G | G (0.7143) | 5 | 167676785 | 0.0064 | 3.62 (1.37,10.5) |
| rs3811266 | 2.40E-05 | A/G | G (0.2333) | 14 | 21816895 | 0.00068 | 5.11 (1.86,15.31) |
| rs928655 | 3.50E-05 | A/G | A (0.7667) | 1 | 89561595 | 0.00094 | 5.46 (1.8,20.16) |
| rs11615624 | 3.90E-05 | A/G | A (0.9286) | 12 | 61574610 | 0.0049 | Inf (1.7,Inf) |
| rs1565948 | 4.70E-05 | A/G | G (0.4905) | 9 | 27549733 | 0.00099 | 3.82 (1.6,9.51) |
| rs823920 | 4.70E-05 | A/G | G (0.1971) | 9 | 101742140 | 0.00038 | 5.89 (1.97,20.24) |
| rs4802262 | 5.30E-05 | A/G | A (0.2857) | 19 | 50841726 | 0.03 | 2.65 (1.04,7.01) |
| rs8012626 | 5.40E-05 | A/C | C (0.5238) | 14 | 29822859 | 1.50E-05 | 6.09 (2.47,15.81) |
| rs4243175 | 6.10E-05 | A/G | G (0.8571) | 16 | 78037924 | 0.0018 | 8.18 (1.77,77.07) |
| rs11645514 | 6.10E-05 | A/G | A (0.8571) | 16 | 78041992 | 0.0018 | 8.18 (1.77,77.07) |
| rs9356148 | 7.00E-05 | A/C | A (0.419) | 6 | 164163929 | 0.01 | 2.88 (1.22,6.99) |
| rs6465908 | 7.20E-05 | A/G | A (0.119) | 7 | 102422288 | 0.0071 | 6.08 (1.47,36.22) |
| rs7385345 | 7.20E-05 | A/G | A (0.119) | 7 | 102438882 | 0.0071 | 6.08 (1.47,36.22) |
| rs10257369 | 7.20E-05 | A/G | G (0.119) | 7 | 102565350 | 0.0071 | 6.08 (1.47,36.22) |
| rs1325466 | 7.30E-05 | A/C | A (0.7714) | 13 | 74839922 | 7.00E-04 | 6.1 (1.85,26.42) |
| rs179071 | 7.40E-05 | A/C | C (0.4804) | 14 | 29850480 | 1.40E-05 | 6.29 (2.54,16.52) |
| rs6745717 | 7.50E-05 | A/G | A (0.8333) | 2 | 212402737 | 0.0033 | 5.78 (1.53,32.82) |
| rs6084265 | 8.50E-05 | A/G | G (0.5) | 20 | 3018898 | 0.0031 | 3.52 (1.48,8.72) |
| rs4749463 | 8.50E-05 | A/G | G (0.4286) | 10 | 29910692 | 0.0034 | 3.29 (1.4,7.99) |
| rs10029689 | 8.90E-05 | A/G | G (0.152) | 4 | 1502214 | 0.0023 | 5.24 (1.61,20.23) |
| rs464805 | 9.10E-05 | A/G | G (0.9286) | unknown | 0 | 0.00077 | 16.57 (2.2,744.21) |
| rs370534 | 9.10E-05 | A/G | A (0.9286) | 22 | 21581605 | 0.00077 | 16.57 (2.2,744.21) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2242411 | 9.90E-05 | A/G | A (0.5429) | 5 | 1806306 | 0.00086 | 3.98 (1.67,9.86) |
| rs4379724 | 1.00E-04 | A/G | A (0.1562) | 10 | 67288253 | 0.047 | 2.84 (0.94,9.35) |
| rs10183226 | 0.00012 | A/C | C (0.4619) | 2 | 1383763 | 0.00089 | 3.88 (1.63,9.58) |
| rs8064241 | 0.00012 | A/G | G (0.5385) | 17 | 43288340 | 0.00018 | 4.67 (1.93,11.8) |
| rs562034 | 0.00012 | A/C | C (0.1381) | 6 | 85901908 | 0.0083 | 7.17 (1.38,71.92) |
| rs1624922 | 0.00013 | A/G | G (0.9272) | 17 | 71112486 | 0.00028 | Inf (3.05,Inf) |
| rs7614718 | 0.00013 | A/C | C (0.6058) | 3 | 80481144 | 0.027 | 2.63 (1.08,6.7) |
| rs1400316 | 0.00013 | A/G | G (0.3971) | 11 | 83264485 | 0.00039 | 4.68 (1.87,12.29) |
| rs17525249 | 0.00013 | A/G | G (0.1381) | 15 | 91273984 | 0.0081 | 4.43 (1.33,17.33) |
| rs10097597 | 0.00014 | A/G | G (0.9048) | 8 | 139721988 | 0.0044 | 12.58 (1.58,577.21) |
| rs17426089 | 0.00015 | A/G | A (0.1762) | 1 | 87860097 | 0.00078 | 10.37 (2.12,100.84) |
| rs10787841 | 0.00015 | A/G | A (0.1779) | 10 | 120244899 | 0.035 | 2.82 (1.02,8.26) |
| rs3024444 | 0.00016 | A/G | G (0.0673) | 6 | 6120173 | 0.074 | 6.83 (0.86,313.42) |
| rs5009257 | 0.00016 | A/C | A (0.3) | 18 | 58390947 | 0.005 | 3.52 (1.37,9.48) |
| rs10135523 | 0.00016 | A/C | C (0.3894) | 14 | 87884771 | 0.002 | 3.72 (1.5,9.58) |
| rs10266523 | 0.00017 | A/G | G (0.1238) | 7 | 102568009 | 0.0071 | 6.08 (1.47,36.22) |
| rs1889155 | 0.00017 | A/G | G (0.9429) | 9 | 134324406 | 0.081 | 7.32 (0.78,356.8) |
| rs7406505 | 0.00018 | A/G | A (0.7837) | 17 | 77073994 | 0.00042 | 7.59 (2.03,42.78) |
| rs10057536 | 0.00019 | A/G | G (0.1762) | 5 | 117677228 | 0.035 | 3.26 (1.02,11.61) |
| rs10489397 | 2.00E-04 | A/C | C (0.8857) | 1 | 183330267 | 0.0011 | Inf (2.36,Inf) |
| rs2383513 | 2.00E-04 | A/G | A (0.8857) | 1 | 183331181 | 0.0011 | Inf (2.36,Inf) |
| rs1826733 | 0.00021 | A/G | A (0.6476) | 12 | 62909775 | 0.00036 | 5.41 (1.9,17.9) |
| rs12759907 | 0.00021 | A/G | G (0.1762) | 1 | 168191165 | 0.0017 | 4.9 (1.61,16.97) |
| rs4400935 | 0.00022 | A/G | A (0.9381) | 13 | 65129961 | 0.0044 | 12.58 (1.58,577.21) |
| rs8002390 | 0.00022 | A/G | G (0.3429) | 13 | 69809017 | 0.011 | 3.13 (1.26,8.06) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2781400 | 0.00022 | A/G | A (0.1346) | 14 | 97351978 | 0.012 | 4.87 (1.34,22.38) |
| rs10144091 | 0.00022 | A/G | A (0.181) | 14 | 97710205 | 0.0021 | 5.43 (1.67,20.94) |
| rs2294418 | 0.00023 | A/G | G (0.9087) | 6 | 11867362 | 0.00093 | 16.02 (2.12,720.3) |
| rs1107920 | 0.00023 | A/G | A (0.1905) | 19 | 38382536 | 0.001 | 5.97 (1.86,22.94) |
| rs3001167 | 0.00023 | A/G | G (0.919) | 1 | 191262571 | 0.00077 | 16.57 (2.2,744.21) |
| rs3009384 | 0.00023 | A/G | G (0.919) | 1 | 191274940 | 0.00077 | 16.57 (2.2,744.21) |
| rs1556122 | 0.00024 | A/G | G (0.3381) | 13 | 109783608 | 0.035 | 2.61 (1.06,6.62) |
| rs1522811 | 0.00024 | A/C | C (0.7048) | 2 | 226817959 | 0.0036 | 4.45 (1.46,16.54) |
| rs1014610 | 0.00025 | A/G | A (0.9286) | 6 | 14889846 | 0.0019 | 14.52 (1.88,658.49) |
| rs496184 | 0.00025 | A/G | A (0.5476) | 3 | 62699909 | 0.011 | 2.83 (1.2,6.89) |
| rs7241357 | 0.00026 | A/G | G (0.5667) | 18 | 25335967 | 0.033 | 2.34 (1.01,5.56) |
| rs2492448 | 0.00026 | A/G | G (0.7286) | 10 | 35235412 | 0.014 | 3.62 (1.25,12.12) |
| rs1094039 | 0.00027 | A/G | A (0.4381) | 9 | 6685472 | 0.011 | 2.83 (1.2,6.89) |
| rs928163 | 0.00027 | A/G | G (0.7067) | 20 | 55823160 | 0.0028 | 4.2 (1.53,12.97) |
| rs10512432 | 0.00028 | A/G | A (0.3714) | 17 | 25836246 | 0.0041 | 3.39 (1.38,8.57) |
| rs1328325 | 0.00028 | A/G | G (0.3798) | 10 | 29885898 | 0.0022 | 3.73 (1.54,9.37) |
| rs2012025 | 0.00029 | A/G | G (0.5095) | 6 | 1571543 | 0.0065 | 3 (1.28,7.24) |
| rs680500 | 0.00029 | A/G | G (0.2571) | 5 | 73547443 | 0.0084 | 3.3 (1.25,9.2) |
| rs9897045 | 0.00031 | A/G | A (0.1333) | 17 | 72173649 | 0.006 | 5.04 (1.39,23.16) |
| rs1422531 | 0.00031 | A/G | A (0.2067) | 5 | 166725971 | 0.00033 | 7.32 (2.11,32.79) |
| rs606073 | 0.00032 | A/G | G (0.3619) | 18 | 46739965 | 0.041 | 2.39 (0.99,5.9) |
| rs6509807 | 0.00032 | A/G | G (0.3143) | 19 | 58859082 | 0.024 | 2.61 (1.07,6.51) |
| rs2472814 | 0.00033 | A/G | A (0.9333) | 6 | 14898195 | 0.0019 | 14.52 (1.88,658.49) |
| rs790377 | 0.00033 | A/G | G (0.2524) | 11 | 83310837 | 0.00023 | 5.93 (2.09,18.82) |
| rs3757446 | 0.00034 | A/G | A (0.9223) | 7 | 22668017 | 0.064 | 4.67 (0.78,49.6) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs857229 | 0.00034 | A/G | A (0.7429) | 14 | 97740677 | 0.0013 | 4.75 (1.66,15.75) |
| rs10485770 | 0.00035 | A/C | C (0.1324) | 20 | 13943214 | 0.15 | 2.65 (0.73,10.91) |
| rs9365597 | 0.00035 | A/G | G (0.3667) | 6 | 164077150 | 0.0023 | 3.68 (1.52,9.22) |
| rs7526286 | 0.00036 | A/G | A (0.1) | 1 | 197396246 | 0.00078 | 10.37 (2.12,100.84) |
| rs738651 | 0.00037 | A/G | G (0.0857) | 22 | 24942459 | 0.07 | 4.44 (0.75,47.1) |
| rs2067885 | 0.00037 | A/G | G (0.3238) | 15 | 48009237 | 0.00022 | 5.31 (2,15.2) |
| rs12916154 | 0.00037 | A/G | G (0.4) | 15 | 48020898 | 0.0023 | 3.68 (1.52,9.22) |
| rs6566822 | 0.00037 | A/C | A (0.1442) | 18 | 52376631 | 0.0036 | 6.82 (1.68,40.27) |
| rs1796135 | 0.00037 | A/G | A (0.4905) | 12 | 77491016 | 0.00099 | 3.82 (1.6,9.51) |
| rs759390 | 0.00037 | A/C | A (0.6408) | 12 | 95066993 | 0.0073 | 3.33 (1.29,9.26) |
| rs6732469 | 0.00037 | A/G | G (0.3857) | 2 | 128723691 | 0.0041 | 3.39 (1.38,8.57) |
| rs316369 | 0.00038 | A/G | A (0.5286) | 16 | 9636463 | 0.0065 | 3 (1.28,7.24) |
| rs10832052 | 0.00038 | A/C | C (0.1476) | 11 | 13501228 | 0.0038 | 8.18 (1.61,80.71) |
| rs10500785 | 0.00038 | A/C | A (0.1476) | 11 | 13530459 | 0.0038 | 8.18 (1.61,80.71) |
| rs757139 | 0.00038 | A/C | C (0.4905) | 7 | 24752595 | 0.00037 | 4.64 (1.88,12.15) |
| rs8182360 | 0.00038 | A/G | A (0.8667) | 17 | 77086490 | 0.0017 | 14.11 (2.01,615.92) |
| rs235900 | 0.00038 | A/G | A (0.1857) | 1 | 168310189 | 0.0043 | 4.13 (1.42,13.28) |
| rs17202792 | 0.00038 | A/G | A (0.9143) | 2 | 169229228 | 0.0044 | 12.58 (1.58,577.21) |
| rs2850542 | 0.00039 | A/C | C (0.4381) | 18 | 46657558 | 0.01 | 2.86 (1.2,6.98) |
| rs7804867 | 0.00039 | A/G | A (0.181) | 7 | 102627708 | 0.0058 | 4.45 (1.45,15.49) |
| rs1403364 | 0.00039 | A/G | G (0.7143) | 2 | 226729255 | 0.0036 | 4.45 (1.46,16.54) |
| rs10493826 | 4.00E-04 | A/G | A (0.7905) | 1 | 89682184 | 0.024 | 3.14 (1.08,10.57) |
| rs543679 | 0.00041 | A/G | G (0.481) | 18 | 20318292 | 0.011 | 2.86 (1.22,6.89) |
| rs416707 | 0.00041 | A/C | A (0.726) | 21 | 21048681 | 0.00015 | 7.37 (2.24,31.89) |
| rs1885147 | 0.00041 | A/G | A (0.4429) | 14 | 28063661 | 0.0033 | 3.37 (1.43,8.21) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs12458686 | 0.00041 | A/G | A (0.0952) | 18 | 41118655 | 0.0044 | 12.58 (1.58,577.21) |
| rs475150 | 0.00041 | A/C | A (0.0619) | 10 | 116146793 | 0.03 | Inf (0.92,Inf) |
| rs1983833 | 0.00042 | A/G | A (0.1429) | 1 | 52554291 | 0.02 | 3.63 (1.15,12.83) |
| rs10129697 | 0.00042 | A/C | A (0.8762) | 14 | 62494352 | 0.032 | 4.13 (1.05,23.93) |
| rs7551313 | 0.00042 | A/G | A (0.1095) | 1 | 109111923 | 0.0083 | 7.17 (1.38,71.92) |
| rs1341152 | 0.00042 | A/G | G (0.8702) | 1 | 210387829 | 0.017 | 4.72 (1.21,27.18) |
| rs674965 | 0.00043 | A/G | A (0.3571) | 18 | 46746491 | 0.041 | 2.39 (0.99,5.9) |
| rs9573531 | 0.00043 | A/C | A (0.7762) | 13 | 74843577 | 0.0013 | 5.69 (1.72,24.72) |
| rs4740861 | 0.00044 | A/C | A (0.5686) | 9 | 6808299 | 0.017 | 2.74 (1.15,6.64) |
| rs1444139 | 0.00044 | A/G | G (0.6381) | 6 | 92952385 | 0.017 | 2.81 (1.16,7.12) |
| rs6460169 | 0.00045 | A/G | A (0.7571) | 7 | 63492623 | 0.0013 | 5.69 (1.72,24.72) |
| rs6069767 | 0.00046 | A/C | C (0.432) | 20 | 54516885 | 0.0033 | 3.4 (1.4,8.65) |
| rs6750888 | 0.00047 | A/G | G (0.1429) | 2 | 6717102 | 0.006 | 5.04 (1.39,23.16) |
| rs10501819 | 0.00047 | A/G | A (0.4571) | 11 | 94362363 | 0.0066 | 3.03 (1.28,7.38) |
| rs10486281 | 0.00048 | A/G | G (0.7714) | 7 | 8795946 | 2.00E-04 | 8.31 (2.25,46.51) |
| rs2225979 | 0.00048 | A/G | A (0.5333) | 9 | 9720381 | 0.0034 | 3.34 (1.41,8.26) |
| rs34080 | 0.00048 | A/G | A (0.6714) | 7 | 12736909 | 0.0064 | 3.62 (1.37,10.5) |
| rs924465 | 0.00048 | A/G | A (0.3143) | 16 | 58246745 | 0.0092 | 3.22 (1.25,8.7) |
| rs1997823 | 0.00048 | A/G | A (0.9143) | 6 | 72296703 | 0.09 | 2.75 (0.76,11.29) |
| rs7200930 | 0.00048 | A/G | G (0.6095) | 16 | 75105130 | 6.90E-05 | 5.6 (2.19,15.58) |
| rs6079319 | 0.00049 | A/G | G (0.2095) | 20 | 14076212 | 0.031 | 2.92 (1.01,9) |
| rs1038046 | 5.00E-04 | A/G | A (0.7596) | unknown | 0 | 0.00042 | 7.59 (2.03,42.78) |
| rs2392015 | 5.00E-04 | A/G | G (0.7286) | 7 | 31804120 | 0.02 | 2.97 (1.12,8.65) |
| rs7797162 | 5.00E-04 | A/G | A (0.7905) | 7 | 37249108 | 0.0081 | 4.58 (1.36,20.06) |
| rs4755714 | 5.00E-04 | A/G | A (0.5238) | 11 | 43490844 | 0.0018 | 3.54 (1.5,8.64) |
| rs874400 | 5.00E-04 | A/G | G (0.4095) | 20 | 46228063 | 0.0018 | 3.54 (1.5,8.64) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs8072173 | 5.00E-04 | A/C | C (0.3905) | 17 | 48985002 | 0.0084 | 3.1 (1.29,7.66) |
| rs16954658 | 5.00E-04 | A/C | C (0.0962) | 16 | 53868795 | 0.013 | 10.16 (1.4,450.25) |
| rs7667609 | 5.00E-04 | A/G | A (0.2692) | 4 | 101838719 | 0.045 | 2.59 (0.99,7.04) |
| rs1493383 | 5.00E-04 | A/G | G (0.8238) | 5 | 152971591 | 0.0059 | 5.34 (1.4,30.46) |
| rs4406717 | 0.00051 | A/G | G (0.2381) | 10 | 5920072 | 0.024 | 3.02 (1.13,8.43) |
| rs11647703 | 0.00051 | A/G | A (0.3524) | 16 | 49471078 | 0.015 | 2.85 (1.19,7.01) |
| rs2400000 | 0.00052 | A/G | A (0.6429) | 11 | 94324949 | 0.0051 | 3.34 (1.33,8.92) |
| rs1563966 | 0.00053 | A/G | A (0.3095) | 17 | 2042704 | 0.01 | 3.17 (1.25,8.33) |
| rs7725968 | 0.00053 | A/G | G (0.8143) | 5 | 160119984 | 0.0024 | 5.3 (1.6,23.09) |
| rs10160013 | 0.00054 | A/G | G (0.3238) | 10 | 29879793 | 0.011 | 3.13 (1.26,8.06) |
| rs11073301 | 0.00054 | A/G | G (0.6048) | 15 | 36279546 | 0.00081 | 4.13 (1.7,10.58) |
| rs1392468 | 0.00055 | A/G | G (0.3413) | 5 | 1797753 | 0.027 | 2.51 (1.05,6.13) |
| rs12794558 | 0.00055 | A/C | C (0.0952) | 11 | 130606990 | 0.0066 | 10.63 (1.46,470.67) |
| rs4663335 | 0.00055 | A/G | A (0.1333) | 2 | 234471090 | 0.00091 | 7.6 (1.9,44.59) |
| rs2075973 | 0.00056 | A/C | A (0.726) | 1 | 9290163 | 0.012 | 3.21 (1.21,9.36) |
| rs307245 | 0.00056 | A/C | A (0.1429) | 11 | 13438575 | 0.0038 | 8.18 (1.61,80.71) |
| rs10835138 | 0.00056 | A/G | G (0.4952) | 11 | 27215475 | 0.0017 | 3.71 (1.56,9.14) |
| rs6979413 | 0.00056 | A/G | G (0.6143) | 7 | 51530272 | 0.032 | 2.42 (1.02,5.92) |
| rs6748811 | 0.00056 | A/C | C (0.0619) | 2 | 156703282 | 0.0049 | Inf (1.7,Inf) |
| rs3849448 | 0.00056 | A/G | A (0.0673) | 3 | 171462521 | 0.02 | Inf (1.13,Inf) |
| rs11563251 | 0.00056 | A/G | A (0.9327) | 2 | 234461384 | 0.054 | 3.95 (0.88,24.56) |
| rs11759185 | 0.00057 | A/G | A (0.1762) | 6 | 5903614 | 0.0082 | 3.75 (1.28,12.13) |
| rs10498668 | 0.00057 | A/G | G (0.9327) | 6 | 6284445 | 0.081 | 7.32 (0.78,356.8) |
| rs229079 | 0.00057 | A/G | G (0.381) | 21 | 27219920 | 0.048 | 2.25 (0.95,5.38) |
| rs11153668 | 0.00057 | A/G | G (0.2714) | 6 | 117959754 | 0.0084 | 3.3 (1.25,9.2) |
| rs2990505 | 0.00058 | A/G | A (0.203) | 20 | 14014276 | 0.082 | 2.6 (0.88,8.12) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs16923429 | 0.00058 | A/G | A (0.1048) | 11 | 95624950 | 0.094 | 3.49 (0.74,22.16) |
| rs17649734 | 0.00061 | A/G | A (0.1714) | 5 | 16432078 | 0.0043 | 4.13 (1.42,13.28) |
| rs11052116 | 0.00061 | A/G | G (0.1571) | 12 | 32673097 | 0.006 | 5.04 (1.39,23.16) |
| rs6664266 | 0.00061 | A/G | A (0.4571) | 1 | 114531036 | 0.0018 | 3.63 (1.5,9.24) |
| rs12547337 | 0.00061 | A/G | A (0.919) | 8 | 139738893 | 0.081 | 7.32 (0.78,356.8) |
| rs16827953 | 0.00061 | A/G | A (0.0857) | 2 | 232026855 | 0.0022 | Inf (2.1,Inf) |
| rs4757637 | 0.00062 | A/C | C (0.3381) | 11 | 18264151 | 0.00048 | 4.42 (1.79,11.42) |
| rs6056558 | 0.00063 | A/G | A (0.519) | 20 | 933530 | 0.019 | 2.68 (1.15,6.41) |
| rs11765761 | 0.00063 | A/G | G (0.201) | 7 | 8882102 | 0.001 | 4.97 (1.73,15.85) |
| rs156396 | 0.00063 | A/C | C (0.4238) | 9 | 23519626 | 0.0029 | 3.43 (1.45,8.39) |
| rs26534 | 0.00063 | A/G | A (0.5667) | 5 | 115203600 | 0.0065 | 3 (1.28,7.24) |
| rs16952025 | 0.00064 | A/G | A (0.619) | 17 | 2063548 | 0.0051 | 3.34 (1.33,8.92) |
| rs11082439 | 0.00064 | A/G | A (0.1143) | 18 | 41124301 | 0.0071 | 6.08 (1.47,36.22) |
| rs2307143 | 0.00065 | A/G | A (0.281) | 17 | 2036504 | 0.033 | 2.63 (1.05,6.79) |
| rs903160 | 0.00065 | A/G | A (0.281) | 17 | 2038515 | 0.033 | 2.63 (1.05,6.79) |
| rs1505687 | 0.00065 | A/C | A (0.2714) | 2 | 6818838 | 0.088 | 2.19 (0.88,5.57) |
| rs10754227 | 0.00065 | A/C | A (0.4048) | 1 | 185466993 | 0.048 | 2.25 (0.95,5.38) |
| rs3890694 | 0.00065 | A/G | G (0.4048) | 1 | 185478053 | 0.048 | 2.25 (0.95,5.38) |
| rs1418707 | 0.00066 | A/G | A (0.7952) | 6 | 150800 | 0.032 | 3.32 (1.06,12.5) |
| rs10141935 | 0.00066 | A/G | A (0.6333) | 14 | 28076505 | 0.011 | 2.86 (1.2,7.13) |
| rs11124430 | 0.00066 | A/G | G (0.6143) | 2 | 35513043 | 0.0018 | 3.63 (1.5,9.24) |
| rs4969367 | 0.00066 | A/G | A (0.12) | 17 | 76646610 | 0.0044 | 6.13 (1.5,36.28) |
| rs4899951 | 0.00066 | A/G | A (0.3107) | 14 | 87887099 | 0.0045 | 3.61 (1.37,10.03) |
| rs17130136 | 0.00066 | A/C | A (0.1286) | 1 | 87925848 | 1.80E-05 | 25.99 (3.65,1139.56) |
| rs12609177 | 0.00067 | A/G | G (0.2714) | 19 | 38397295 | 0.00095 | 4.11 (1.64,10.79) |
| rs317802 | 0.00067 | A/G | A (0.4087) | 6 | 159061740 | 0.016 | 2.73 (1.14,6.69) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs7823711 | 0.00068 | A/G | A (0.581) | 8 | 804529 | 0.0013 | 4.06 (1.59,11.23) |
| rs676147 | 0.00068 | A/G | A (0.1893) | 18 | 10114648 | 0.00017 | 9.32 (2.38,54.05) |
| rs9378784 | 0.00069 | A/G | A (0.6238) | 6 | 3261745 | 0.0019 | 4.12 (1.57,11.93) |
| rs764189 | 7.00E-04 | A/G | A (0.5962) | 10 | 6396216 | 0.0013 | 4.06 (1.61,10.92) |
| rs4981223 | 7.00E-04 | A/G | G (0.4524) | 14 | 20041624 | 0.011 | 2.93 (1.24,7.21) |
| rs12127343 | 7.00E-04 | A/G | G (0.5286) | 1 | 21615079 | 0.0017 | 3.71 (1.56,9.14) |
| rs6902731 | 7.00E-04 | A/C | A (0.1095) | 6 | 75274664 | 0.069 | 3.49 (0.89,16.63) |
| rs17053352 | 7.00E-04 | A/G | G (0.219) | 6 | 125903635 | 0.12 | 2.45 (0.78,8.21) |
| rs6775443 | 7.00E-04 | A/G | G (0.6346) | 3 | 140360622 | 0.001 | 4.38 (1.66,12.74) |
| rs843451 | 7.00E-04 | A/G | A (0.6952) | 2 | 240067248 | 0.0071 | 3.39 (1.28,9.85) |
| rs10517554 | 0.00071 | A/G | A (0.5667) | 4 | 40912356 | 0.0065 | 3 (1.28,7.24) |
| rs3128477 | 0.00071 | A/G | A (0.3857) | 9 | 87511733 | 0.0084 | 3.1 (1.29,7.66) |
| rs2112270 | 0.00071 | A/C | C (0.5905) | 5 | 89210540 | 0.00021 | 5.48 (2.02,16.81) |
| rs3848777 | 0.00072 | A/G | A (0.1381) | 20 | 13919235 | 0.17 | 2.25 (0.65,8.38) |
| rs4814277 | 0.00072 | A/G | A (0.1381) | 20 | 13989207 | 0.17 | 2.25 (0.65,8.38) |
| rs1466199 | 0.00072 | A/G | G (0.5143) | 11 | 44843130 | 0.077 | 2.15 (0.93,5.08) |
| rs6492252 | 0.00072 | A/G | G (0.5952) | 13 | 109720695 | 0.0051 | 3.34 (1.33,8.92) |
| rs7125373 | 0.00072 | A/G | G (0.1635) | 11 | 115532483 | 0.0081 | 4.43 (1.33,17.33) |
| rs1024528 | 0.00072 | A/G | G (0.4) | 7 | 146663529 | 0.01 | 2.86 (1.2,6.98) |
| rs1890397 | 0.00072 | A/G | G (0.4429) | 1 | 189502590 | 0.0016 | 3.87 (1.6,9.88) |
| rs1945168 | 0.00073 | A/G | G (0.5286) | 18 | 20378416 | 0.012 | 2.75 (1.18,6.58) |
| rs3809140 | 0.00073 | A/G | G (0.8) | 12 | 26169711 | 0.0018 | 8.18 (1.77,77.07) |
| rs4083633 | 0.00073 | A/G | A (0.7921) | 10 | 29921494 | 0.0031 | 5.93 (1.57,33.71) |
| rs3756894 | 0.00073 | A/C | C (0.9476) | 6 | 117964181 | 0.012 | Inf (1.3,Inf) |
| rs902966 | 0.00074 | A/G | A (0.7381) | 17 | 1180385 | 0.093 | 2.26 (0.89,6.07) |
| rs6110153 | 0.00074 | A/C | C (0.1762) | 20 | 13984529 | 0.13 | 2.36 (0.79,7.41) |
| rs919432 | 0.00074 | A/G | G (0.4095) | 2 | 16465684 | 0.048 | 2.27 (0.97,5.42) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2074189 | 0.00074 | A/C | A (0.5286) | 17 | 43242950 | 0.0063 | 3.07 (1.31,7.41) |
| rs7622037 | 0.00074 | A/G | A (0.6566) | 3 | 176451255 | 0.0059 | 3.67 (1.38,10.75) |
| rs4421618 | 0.00074 | A/G | G (0.0952) | 1 | 197386451 | 0.0018 | 9.24 (1.86,90.63) |
| rs6029388 | 0.00075 | A/G | A (0.9286) | 20 | 38925458 | 0.0044 | 12.58 (1.58,577.21) |
| rs16845267 | 0.00075 | A/G | A (0.2548) | 2 | 141657972 | 0.037 | 2.71 (0.98,7.96) |
| rs9790242 | 0.00076 | A/C | C (0.9048) | 3 | 65078890 | 0.054 | 3.76 (0.95,21.94) |
| rs149851 | 0.00077 | A/G | G (0.1048) | 12 | 97813855 | 0.027 | 4.71 (1.09,28.83) |
| rs4814278 | 0.00078 | A/G | G (0.219) | 20 | 13999325 | 0.094 | 2.3 (0.81,6.84) |
| rs10475944 | 0.00078 | A/G | G (0.9238) | 5 | 169873337 | 0.094 | 3.49 (0.74,22.16) |
| rs3743887 | 0.00079 | A/G | A (0.9333) | 16 | 364278 | 0.01 | 10.74 (1.3,500.03) |
| rs7229222 | 0.00079 | A/G | A (0.2) | 18 | 58362041 | 0.053 | 2.63 (0.9,8.18) |
| rs6655965 | 0.00079 | A/G | G (0.5524) | 1 | 200412149 | 0.00099 | 3.82 (1.6,9.51) |
| rs1040223 | 0.00079 | A/G | A (0.5476) | 2 | 226742278 | 0.003 | 3.48 (1.47,8.49) |
| rs1504772 | 8.00E-04 | A/G | G (0.5721) | 8 | 4087225 | 0.0016 | 3.81 (1.55,9.93) |
| rs733175 | 8.00E-04 | A/G | G (0.1238) | 4 | 9726410 | 0.012 | 4.49 (1.21,20.85) |
| rs10485771 | 8.00E-04 | A/G | G (0.181) | 20 | 13999103 | 0.18 | 2.17 (0.67,7.37) |
| rs6708660 | 8.00E-04 | A/G | A (0.6381) | 2 | 43664033 | 0.0029 | 3.56 (1.42,9.5) |
| rs4513310 | 8.00E-04 | A/G | A (0.2524) | 2 | 84767024 | 0.0014 | 4.68 (1.69,14.05) |
| rs7957408 | 8.00E-04 | A/G | A (0.8381) | 12 | 95323764 | 0.052 | 3.07 (0.97,11.61) |
| rs9323994 | 8.00E-04 | A/C | C (0.1429) | 14 | 98159331 | 0.02 | 3.63 (1.15,12.83) |
| rs796063 | 8.00E-04 | A/G | G (0.4519) | 2 | 101187961 | 0.00075 | 4.19 (1.71,10.79) |
| rs223502 | 8.00E-04 | A/G | G (0.2905) | 4 | 103997961 | 0.0045 | 3.61 (1.37,10.03) |
| rs709274 | 8.00E-04 | A/C | A (0.7952) | 2 | 240068225 | 0.0031 | 6.24 (1.66,35.3) |
| rs4273904 | 0.00081 | A/G | A (0.4762) | 9 | 9714976 | 0.0034 | 3.34 (1.41,8.26) |
| rs6487429 | 0.00081 | A/C | A (0.5952) | 12 | 24885299 | 6.00E-04 | 4.61 (1.8,12.78) |
| rs7967945 | 0.00081 | A/G | A (0.5952) | 12 | 24887168 | 6.00E-04 | 4.61 (1.8,12.78) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs728939 | 0.00082 | A/G | G (0.3) | 10 | 3321092 | 0.0075 | 3.11 (1.27,7.86) |
| rs10502353 | 0.00082 | A/C | C (0.3286) | 18 | 7137079 | 0.00056 | 4.69 (1.81,12.98) |
| rs2025392 | 0.00082 | A/G | A (0.5286) | 9 | 9723309 | 0.0034 | 3.34 (1.41,8.26) |
| rs6470399 | 0.00082 | A/G | A (0.381) | 8 | 127078098 | 0.03 | 2.45 (1.04,5.88) |
| rs2525055 | 0.00083 | A/C | A (0.6474) | 12 | 46473005 | 0.0019 | 3.94 (1.52,10.9) |
| rs581170 | 0.00083 | A/G | A (0.1524) | 6 | 85938437 | 0.039 | 3.97 (1.05,18.68) |
| rs9428452 | 0.00083 | A/G | A (0.1905) | 1 | 235492270 | 0.02 | 3.63 (1.15,12.83) |
| rs12041734 | 0.00084 | A/G | G (0.2571) | 1 | 18306896 | 0.0058 | 4.45 (1.45,15.49) |
| rs885813 | 0.00084 | A/G | G (0.4476) | 1 | 21622380 | 0.018 | 2.75 (1.16,6.75) |
| rs2038926 | 0.00084 | A/G | G (0.3143) | 1 | 195332854 | 0.01 | 3.17 (1.25,8.33) |
| rs1998843 | 0.00084 | A/G | A (0.3143) | 1 | 195364013 | 0.01 | 3.17 (1.25,8.33) |
| rs11078629 | 0.00085 | A/G | G (0.7333) | 17 | 1151162 | 0.012 | 3.18 (1.2,9.23) |
| rs7191941 | 0.00085 | A/G | A (0.8738) | 16 | 7552509 | 0.0067 | 10.57 (1.45,468.96) |
| rs11766273 | 0.00085 | A/G | A (0.9286) | 7 | 22548903 | 0.035 | 5.3 (0.94,54.86) |
| rs11725410 | 0.00085 | A/G | G (0.3667) | 4 | 26177641 | 0.048 | 2.25 (0.95,5.38) |
| rs4236552 | 0.00087 | A/G | G (0.7087) | 7 | 100204582 | 0.00047 | 5.76 (1.9,21.35) |
| rs9693430 | 0.00087 | A/C | A (0.9238) | 8 | 130034016 | 0.0019 | 14.52 (1.88,658.49) |
| rs11576706 | 0.00087 | A/G | G (0.2714) | 1 | 168180055 | 0.01 | 3.17 (1.25,8.33) |
| rs450718 | 0.00089 | A/G | G (0.1058) | 22 | 16868534 | 0.00031 | 18.72 (2.53,834.74) |
| rs9884830 | 0.00089 | A/G | A (0.1524) | 4 | 25704066 | 0.035 | 3.26 (1.02,11.61) |
| rs2076756 | 0.00089 | A/G | G (0.2667) | 16 | 49314382 | 0.024 | 3.02 (1.13,8.43) |
| rs11996826 | 0.00089 | A/G | A (0.6619) | 8 | 99228770 | 0.0071 | 3.39 (1.28,9.85) |
| rs2282507 | 0.00089 | A/G | A (0.1394) | 11 | 126268589 | 0.12 | 2.45 (0.78,8.21) |
| rs11161755 | 9.00E-04 | A/G | G (0.9238) | 1 | 86328292 | 0.017 | 6.21 (1.16,63.16) |
| rs2240379 | 0.00091 | A/G | A (0.9333) | 8 | 183585 | 0.081 | 7.32 (0.78,356.8) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2314095 | 0.00091 | A/C | C (0.9279) | 4 | 162731398 | 0.041 | 8.69 (1,412.92) |
| rs7915320 | 0.00092 | A/G | G (0.3667) | 10 | 7553254 | 0.0015 | 3.69 (1.54,9.16) |
| rs8070753 | 0.00092 | A/G | A (0.3619) | 17 | 9205453 | 0.014 | 2.84 (1.17,7.09) |
| rs716796 | 0.00092 | A/G | A (0.1923) | 6 | 25501125 | 0.028 | 2.69 (1.03,7.31) |
| rs6887552 | 0.00092 | A/G | G (0.6667) | 5 | 67028978 | 0.0019 | 4.12 (1.57,11.93) |
| rs3949948 | 0.00092 | A/G | G (0.351) | 13 | 106716741 | 0.0015 | 3.71 (1.55,9.16) |
| rs9521936 | 0.00092 | A/G | G (0.481) | 13 | 110234390 | 0.0065 | 3 (1.28,7.24) |
| rs1520760 | 0.00092 | A/G | G (0.5) | 12 | 118326079 | 0.032 | 2.39 (1.03,5.67) |
| rs1716470 | 0.00092 | A/G | A (0.5) | 12 | 118334213 | 0.032 | 2.39 (1.03,5.67) |
| rs2714174 | 0.00092 | A/G | A (0.3317) | 2 | 141952733 | 0.02 | 2.75 (1.1,7.11) |
| rs7150478 | 0.00093 | A/G | A (0.2095) | 14 | 33241754 | 0.0082 | 3.75 (1.28,12.13) |
| rs1983249 | 0.00093 | A/G | A (0.6476) | 18 | 40388445 | 0.0087 | 3.13 (1.25,8.37) |
| rs10498470 | 0.00093 | A/G | G (0.1381) | 14 | 54174774 | 0.014 | 5.38 (1.27,32.42) |
| rs8003820 | 0.00093 | A/G | A (0.1524) | 14 | 77038334 | 0.1 | 2.57 (0.77,9.39) |
| rs10518398 | 0.00093 | A/G | A (0.9095) | 1 | 87904321 | 0.00011 | Inf (3.55,Inf) |
| rs1880269 | 0.00094 | A/G | A (0.3476) | 6 | 25333492 | 0.0045 | 3.61 (1.37,10.03) |
| rs7531139 | 0.00094 | A/G | A (0.7892) | 1 | 71268188 | 0.0045 | 4.93 (1.48,21.54) |
| rs229126 | 0.00095 | A/G | A (0.5476) | 21 | 27168837 | 0.053 | 2.19 (0.94,5.17) |
| rs721936 | 0.00095 | A/G | A (0.3905) | 9 | 87512703 | 0.01 | 2.86 (1.2,6.98) |
| rs11067138 | 0.00095 | A/G | A (0.1286) | 12 | 113383090 | 0.0038 | 8.18 (1.61,80.71) |
| rs17089603 | 0.00096 | A/G | G (0.181) | 8 | 23668901 | 0.072 | 2.75 (0.89,9.1) |
| rs7024464 | 0.00096 | A/C | A (0.3762) | 9 | 79034117 | 0.048 | 2.25 (0.95,5.38) |
| rs2309464 | 0.00096 | A/C | C (0.2286) | 4 | 182616557 | 0.018 | 3.23 (1.13,9.87) |
| rs11877743 | 0.00097 | A/G | G (0.6143) | 18 | 37650755 | 0.0043 | 3.57 (1.39,9.89) |
| rs7536830 | 0.00097 | A/G | A (0.3667) | 1 | 142886991 | 0.0056 | 3.18 (1.35,7.72) |
| rs7031325 | 0.00099 | A/G | G (0.3333) | 9 | 6645056 | 0.067 | 2.21 (0.92,5.38) |
| rs10484749 | 0.00099 | A/G | G (0.9471) | 6 | 8326004 | 0.081 | 7.32 (0.78,356.8) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2831475 | 0.00099 | A/G | G (0.3835) | 21 | 28374216 | 0.0017 | 3.58 (1.5,8.84) |
| rs11854769 | 0.00099 | A/G | G (0.7476) | 15 | 36289535 | 0.0013 | 4.75 (1.66,15.75) |
| rs1402611 | 0.00099 | A/G | G (0.4048) | 12 | 77467203 | 0.01 | 2.91 (1.24,7.03) |
| rs4743853 | 0.00099 | A/G | A (0.6619) | 9 | 89946917 | 0.0024 | 3.81 (1.49,10.54) |
| rs1860487 | 0.00099 | A/G | G (0.5095) | 7 | 123309087 | 0.0033 | 3.37 (1.43,8.21) |
| rs2350423 | 0.001 | A/C | A (0.3048) | 7 | 8890814 | 0.00045 | 4.48 (1.79,11.77) |
| rs2389409 | 0.001 | A/G | G (0.581) | 7 | 15280029 | 0.019 | 2.65 (1.13,6.44) |
| rs8092489 | 0.001 | A/C | A (0.4899) | 18 | 19151312 | 0.025 | 2.63 (1.09,6.55) |
| rs11848278 | 0.001 | A/G | A (0.1333) | 14 | 22793634 | 0.12 | 2.45 (0.78,8.21) |
| rs9956546 | 0.001 | A/G | G (0.0857) | 18 | 32136446 | 0.012 | 9.56 (1.3,426.01) |
| rs476861 | 0.001 | A/G | G (0.8714) | 21 | 43659936 | 0.0019 | 12.9 (1.82,565.51) |
| rs6573233 | 0.001 | A/G | A (0.1667) | 14 | 58539490 | 0.072 | 2.75 (0.89,9.1) |
| rs180167 | 0.001 | A/G | A (0.0922) | 17 | 65547344 | 0.035 | 5.3 (0.94,54.86) |
| rs9662349 | 0.001 | A/G | A (0.4143) | 1 | 81890073 | 0.02 | 2.56 (1.1,6.1) |
| rs857230 | 0.001 | A/G | A (0.6143) | 14 | 97685742 | 0.0033 | 3.4 (1.4,8.65) |
| rs2902643 | 0.001 | A/G | A (0.4524) | 10 | 106318781 | 0.011 | 2.83 (1.2,6.89) |
| rs1410201 | 0.001 | A/G | A (0.8286) | 9 | 118072323 | 0.0059 | 5.34 (1.4,30.46) |
| rs10487809 | 0.001 | A/G | A (0.381) | 7 | 123306032 | 0.0084 | 3.1 (1.29,7.66) |
| rs1396566 | 0.001 | A/G | G (0.3381) | 12 | 126146401 | 0.00053 | 4.39 (1.8,11.19) |
| rs10505698 | 0.001 | A/G | G (0.2429) | 8 | 139368001 | 0.028 | 2.69 (1.03,7.31) |
| rs1500837 | 0.001 | A/G | A (0.4381) | 4 | 143172461 | 0.0033 | 3.46 (1.46,8.5) |
| rs2396297 | 0.001 | A/C | A (0.4286) | 2 | 226811479 | 0.0056 | 3.18 (1.35,7.72) |
| rs12451892 | 0.0011 | A/G | A (0.619) | 17 | 2194732 | 0.0051 | 3.34 (1.33,8.92) |
| rs11083340 | 0.0011 | A/G | A (0.9048) | 18 | 25339059 | 0.0044 | 12.58 (1.58,577.21) |
| rs12587100 | 0.0011 | A/G | A (0.3143) | 14 | 29021671 | 0.011 | 3.13 (1.26,8.06) |
| rs2684847 | 0.0011 | A/C | C (0.2143) | 18 | 43024315 | 0.0034 | 4.09 (1.52,11.75) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs11897836 | 0.0011 | A/G | G (0.2095) | 2 | 43335566 | 0.064 | 2.49 (0.92,7.05) |
| rs135809 | 0.0011 | A/G | A (0.9381) | 22 | 48271069 | 0.017 | 6.21 (1.16,63.16) |
| rs9538644 | 0.0011 | A/C | A (0.5146) | 13 | 59793265 | 0.048 | 2.27 (0.97,5.42) |
| rs1395442 | 0.0011 | A/G | G (0.8269) | 3 | 62650337 | 0.032 | 4.13 (1.05,23.93) |
| rs8081909 | 0.0011 | A/G | G (0.2143) | 17 | 64299023 | 5.40E-05 | 9.12 (2.67,40.56) |
| rs2120192 | 0.0011 | A/G | G (0.2143) | 17 | 64301554 | 5.40E-05 | 9.12 (2.67,40.56) |
| rs1529210 | 0.0011 | A/G | G (0.2429) | 18 | 66610529 | 0.001 | 4.97 (1.73,15.85) |
| rs1859983 | 0.0011 | A/C | A (0.3317) | 17 | 66933589 | 0.003 | 3.72 (1.49,9.67) |
| rs4527771 | 0.0011 | A/G | A (0.3905) | 7 | 67328938 | 0.015 | 2.85 (1.19,7.01) |
| rs7407221 | 0.0011 | A/G | G (0.601) | 18 | 71168570 | 0.011 | 2.86 (1.21,7.01) |
| rs9543928 | 0.0011 | A/G | G (0.1635) | 13 | 74916808 | 0.061 | 2.9 (0.89,10.47) |
| rs9930233 | 0.0011 | A/G | G (0.6923) | 16 | 81438595 | 0.0013 | 4.69 (1.63,15.65) |
| rs8054307 | 0.0011 | A/C | C (0.2095) | 16 | 82173233 | 0.00063 | 6.88 (1.97,30.99) |
| rs6840362 | 0.0011 | A/G | A (0.3238) | 4 | 89257099 | 0.011 | 3.13 (1.26,8.06) |
| rs4237573 | 0.0011 | A/G | A (0.5905) | 11 | 94877571 | 0.018 | 2.75 (1.16,6.75) |
| rs593499 | 0.0011 | A/C | C (0.3381) | 11 | 99678704 | 0.00021 | 4.88 (1.95,12.86) |
| rs1216192 | 0.0011 | A/G | A (0.1429) | 11 | 99712710 | 0.069 | 3.49 (0.89,16.63) |
| rs953412 | 0.0011 | A/G | G (0.0905) | 6 | 118546711 | 0.00077 | 16.57 (2.2,744.21) |
| rs2248246 | 0.0011 | A/G | A (0.3333) | 12 | 126129494 | 0.0011 | 4.02 (1.65,10.23) |
| rs6988340 | 0.0011 | A/G | G (0.0667) | 8 | 132037651 | 0.041 | 7.53 (0.98,341.68) |
| rs3799488 | 0.0011 | A/G | G (0.1238) | 6 | 137561473 | 0.094 | 3.49 (0.74,22.16) |
| rs9659073 | 0.0011 | A/G | G (0.5913) | 1 | 147340427 | 0.00037 | 4.49 (1.83,11.6) |
| rs6455920 | 0.0011 | A/G | G (0.2143) | 6 | 164161330 | 0.018 | 3.23 (1.13,9.87) |
| rs3820403 | 0.0011 | A/G | G (0.3476) | 1 | 164990038 | 0.069 | 2.22 (0.94,5.37) |
| rs1505485 | 0.0011 | A/C | C (0.5762) | 4 | 173338072 | 0.047 | 2.36 (0.99,5.87) |
| rs6663339 | 0.0011 | A/G | A (0.1095) | 1 | 177273982 | 0.00012 | 21.01 (2.88,930.49) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs17632548 | 0.0011 | A/G | A (0.0913) | 3 | 178765511 | 0.0048 | Inf (1.65,Inf) |
| rs7594399 | 0.0011 | A/C | C (0.9) | 2 | 232075903 | 0.0022 | Inf (2.1,Inf) |
| rs3786284 | 0.0012 | A/G | A (0.1619) | 18 | 12000646 | 0.061 | 2.9 (0.89,10.47) |
| rs11906933 | 0.0012 | A/G | A (0.181) | 20 | 14072369 | 0.13 | 2.36 (0.79,7.41) |
| rs3087948 | 0.0012 | A/G | G (0.7905) | 5 | 14821308 | 0.0036 | 4.45 (1.46,16.54) |
| rs11990415 | 0.0012 | A/G | G (0.319) | 8 | 16647231 | 0.05 | 2.42 (0.94,6.43) |
| rs7783402 | 0.0012 | A/G | A (0.7476) | 7 | 19853669 | 0.002 | 4.77 (1.57,17.68) |
| rs527790 | 0.0012 | A/G | A (0.4381) | 18 | 20314913 | 0.01 | 2.86 (1.2,6.98) |
| rs11028138 | 0.0012 | A/G | G (0.9293) | 11 | 24729758 | 0.029 | 5.9 (1.05,61.29) |
| rs2328853 | 0.0012 | A/G | A (0.2356) | 6 | 25456168 | 0.036 | 2.5 (1.01,6.36) |
| rs2052148 | 0.0012 | A/G | A (0.5579) | 7 | 43172203 | 0.0027 | 3.54 (1.47,8.86) |
| rs6025794 | 0.0012 | A/G | A (0.6667) | 20 | 55818425 | 0.001 | 4.39 (1.67,12.71) |
| rs1596727 | 0.0012 | A/G | A (0.6429) | 12 | 63609374 | 0.00014 | 5.32 (2.03,15.42) |
| rs2394156 | 0.0012 | A/G | A (0.181) | 10 | 67445913 | 0.071 | 2.45 (0.93,6.69) |
| rs372395 | 0.0012 | A/G | G (0.5962) | 16 | 84332418 | 0.0032 | 3.42 (1.4,8.75) |
| rs2660899 | 0.0012 | A/C | A (0.2143) | 12 | 94932888 | 0.02 | 3.63 (1.15,12.83) |
| rs2091918 | 0.0012 | A/G | A (0.5143) | 14 | 104673664 | 0.012 | 2.75 (1.17,6.62) |
| rs1537733 | 0.0012 | A/C | C (0.6515) | 9 | 115062559 | 0.011 | 3.16 (1.22,8.71) |
| rs617727 | 0.0012 | A/C | C (0.4272) | 11 | 121515390 | 0.0059 | 3.13 (1.33,7.63) |
| rs11200334 | 0.0012 | A/G | G (0.4078) | 10 | 123766885 | 0.00073 | 4.06 (1.7,10.08) |
| rs10240712 | 0.0012 | A/G | G (0.8857) | 7 | 137300127 | 0.089 | 3.4 (0.84,20.02) |
| rs9787394 | 0.0012 | A/G | G (0.9286) | 1 | 168882157 | 0.17 | 2.92 (0.58,19.09) |
| rs10026892 | 0.0012 | A/G | G (0.3592) | 4 | 184886414 | 0.00031 | 4.92 (1.89,13.63) |
| rs7932964 | 0.0013 | A/G | A (0.2048) | 11 | 2027146 | 0.02 | 3.11 (1.13,9.04) |
| rs1454626 | 0.0013 | A/C | C (0.2304) | 9 | 2611030 | 0.045 | 2.88 (0.97,9.04) |
| rs7207552 | 0.0013 | A/G | G (0.9423) | 17 | 3843556 | 0.0055 | Inf (1.65,Inf) |
| rs10164492 | 0.0013 | A/C | A (0.4619) | 2 | 12361646 | 0.051 | 2.26 (0.97,5.41) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs1481587 | 0.0013 | A/G | G (0.8429) | 8 | 13580540 | 0.054 | 3.76 (0.95,21.94) |
| rs2224738 | 0.0013 | A/G | A (0.3762) | 20 | 14103881 | 0.01 | 2.91 (1.24,7.03) |
| rs994868 | 0.0013 | A/G | A (0.3762) | 20 | 14107442 | 0.01 | 2.91 (1.24,7.03) |
| rs7222875 | 0.0013 | A/G | G (0.2048) | 17 | 16497233 | 0.028 | 2.69 (1.03,7.31) |
| rs2028208 | 0.0013 | A/G | G (0.2095) | 2 | 20072445 | 0.039 | 2.75 (1.02,7.72) |
| rs7014143 | 0.0013 | A/C | C (0.6019) | 8 | 21632277 | 3.00E-04 | 4.91 (1.92,13.64) |
| rs134806 | 0.0013 | A/G | A (0.1667) | 22 | 25997765 | 0.0029 | 5.62 (1.57,25.6) |
| rs130334 | 0.0013 | A/G | G (0.4143) | 22 | 41391029 | 0.0055 | 3.12 (1.31,7.63) |
| rs197925 | 0.0013 | A/G | G (0.6238) | 17 | 42365720 | 0.0058 | 3.19 (1.32,8.1) |
| rs1048570 | 0.0013 | A/G | A (0.1905) | 15 | 60907059 | 0.011 | 3.41 (1.25,9.87) |
| rs4280128 | 0.0013 | A/G | G (0.6442) | 13 | 74876840 | 0.015 | 2.95 (1.19,7.69) |
| rs480177 | 0.0013 | A/C | C (0.2136) | 10 | 78659030 | 0.014 | 3.62 (1.23,11.71) |
| rs1527721 | 0.0013 | A/G | A (0.8714) | 7 | 94468022 | 0.0035 | 11.74 (1.64,517.13) |
| rs10861467 | 0.0013 | A/G | A (0.4143) | 12 | 104559604 | 0.0036 | 3.23 (1.37,7.83) |
| rs4730274 | 0.0013 | A/G | A (0.5) | 7 | 107073670 | 0.02 | 2.57 (1.1,6.19) |
| rs1838467 | 0.0013 | A/G | A (0.1476) | 5 | 160998740 | 0.006 | 5.04 (1.39,23.16) |
| rs7607967 | 0.0013 | A/G | G (0.5962) | 2 | 166938843 | 0.0021 | 4.06 (1.58,11.29) |
| rs12518180 | 0.0013 | A/G | A (0.2286) | 5 | 175992222 | 0.018 | 2.9 (1.14,7.65) |
| rs3791575 | 0.0013 | A/G | A (0.3077) | 2 | 239852916 | 0.0032 | 3.62 (1.44,9.52) |
| rs1609446 | 0.0014 | A/C | C (0.8952) | 5 | 3273349 | 0.04 | 4.69 (0.95,45.79) |
| rs2026828 | 0.0014 | A/G | G (0.6905) | 9 | 4533307 | 0.0064 | 3.62 (1.37,10.5) |
| rs199002 | 0.0014 | A/G | A (0.3619) | 6 | 23576219 | 0.013 | 2.85 (1.16,7.22) |
| rs199072 | 0.0014 | A/G | A (0.3619) | 6 | 23583452 | 0.013 | 2.85 (1.16,7.22) |
| rs229055 | 0.0014 | A/G | G (0.5485) | 21 | 27185091 | 0.078 | 2.08 (0.89,4.92) |
| rs4879515 | 0.0014 | A/G | A (0.3952) | 9 | 27472235 | 0.01 | 2.86 (1.2,6.98) |
| rs6837194 | 0.0014 | A/G | A (0.9238) | 4 | 29451545 | 0.49 | 1.76 (0.35,9.43) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs5753298 | 0.0014 | A/G | G (0.2048) | 22 | 29453317 | 0.001 | 4.97 (1.73,15.85) |
| rs6468026 | 0.0014 | A/G | A (0.4429) | 8 | 31194597 | 0.0025 | 3.79 (1.52,10.14) |
| rs2016586 | 0.0014 | A/C | A (0.3238) | 22 | 34449764 | 0.018 | 2.9 (1.14,7.65) |
| rs3923022 | 0.0014 | A/G | G (0.3269) | 15 | 36725830 | 0.0013 | 3.86 (1.57,9.82) |
| rs6707783 | 0.0014 | A/G | A (0.0905) | 2 | 51965542 | 0.0022 | Inf (2.1,Inf) |
| rs2125489 | 0.0014 | A/G | A (0.1476) | 4 | 55794411 | 0.0058 | 4.45 (1.45,15.49) |
| rs1553107 | 0.0014 | A/G | G (0.2381) | 15 | 60888291 | 0.0026 | 3.84 (1.5,10.33) |
| rs699619 | 0.0014 | A/G | A (0.181) | 12 | 61665783 | 0.0036 | 6.82 (1.68,40.27) |
| rs812796 | 0.0014 | A/G | G (0.181) | 12 | 61685535 | 0.0036 | 6.82 (1.68,40.27) |
| rs6546177 | 0.0014 | A/C | A (0.4095) | 2 | 65889084 | 0.048 | 2.27 (0.97,5.42) |
| rs2170331 | 0.0014 | A/G | A (0.2762) | 1 | 86027745 | 0.03 | 2.65 (1.04,7.01) |
| rs6665006 | 0.0014 | A/G | G (0.2762) | 1 | 86136671 | 0.03 | 2.65 (1.04,7.01) |
| rs4076053 | 0.0014 | A/G | G (0.4286) | 6 | 88683580 | 0.11 | 1.94 (0.83,4.59) |
| rs1545189 | 0.0014 | A/C | A (0.2887) | 15 | 91361335 | 0.0054 | 3.92 (1.39,11.98) |
| rs2795380 | 0.0014 | A/G | G (0.1381) | 9 | 101758440 | 0.016 | 3.97 (1.18,15.68) |
| rs2995211 | 0.0014 | A/G | A (0.1286) | 9 | 101788575 | 0.016 | 3.97 (1.18,15.68) |
| rs12464067 | 0.0014 | A/G | G (0.3905) | 2 | 105874630 | 0.03 | 2.48 (1.06,5.92) |
| rs552125 | 0.0014 | A/G | G (0.8857) | 13 | 109736814 | 0.0011 | Inf (2.36,Inf) |
| rs7300860 | 0.0014 | A/G | A (0.8286) | 12 | 110217317 | 0.019 | 3.58 (1.15,13.44) |
| rs12573699 | 0.0014 | A/G | A (0.2837) | 10 | 120567218 | 0.016 | 3.1 (1.2,8.38) |
| rs7460718 | 0.0014 | A/C | C (0.0714) | 8 | 132039547 | 0.041 | 7.53 (0.98,341.68) |
| rs1542986 | 0.0014 | A/G | A (0.101) | 4 | 134475682 | 0.0083 | 7.17 (1.38,71.92) |
| rs305347 | 0.0014 | A/G | A (0.9238) | 8 | 137722126 | 0.17 | 2.92 (0.58,19.09) |
| rs2882618 | 0.0014 | A/C | C (0.9143) | 2 | 156719385 | 0.00028 | Inf (3.05,Inf) |
| rs254850 | 0.0014 | A/G | A (0.2333) | 5 | 158599309 | 0.024 | 3.02 (1.13,8.43) |
| rs10924249 | 0.0014 | A/C | A (0.219) | 1 | 242096683 | 0.024 | 3.02 (1.13,8.43) |
| rs3135123 | 0.0015 | A/G | G (0.4571) | 4 | 1954169 | 0.00045 | 4.24 (1.76,10.72) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs7011009 | 0.0015 | A/G | A (0.1143) | 8 | 5245904 | 0.00077 | 16.57 (2.2,744.21) |
| rs722661 | 0.0015 | A/C | C (0.481) | 7 | 13684885 | 0.016 | 2.95 (1.2,7.67) |
| rs7639295 | 0.0015 | A/G | A (0.0905) | 3 | 18052061 | 0.027 | 4.71 (1.09,28.83) |
| rs2152686 | 0.0015 | A/G | A (0.9476) | 13 | 18811270 | 0.16 | 5.72 (0.54,290.23) |
| rs6050867 | 0.0015 | A/G | G (0.9238) | 20 | 25678968 | 0.0083 | 7.17 (1.38,71.92) |
| rs2076672 | 0.0015 | A/G | G (0.7286) | 22 | 34447583 | 0.093 | 2.26 (0.89,6.07) |
| rs6563533 | 0.0015 | A/C | C (0.9238) | 13 | 36863814 | 0.0049 | Inf (1.7,Inf) |
| rs1006108 | 0.0015 | A/G | A (0.7212) | 7 | 42935862 | 0.00069 | 5.07 (1.78,16.8) |
| rs9534613 | 0.0015 | A/G | G (0.2067) | 13 | 46841830 | 0.028 | 3.11 (1.08,9.64) |
| rs9536467 | 0.0015 | A/G | A (0.4048) | 13 | 53047417 | 0.024 | 2.61 (1.07,6.51) |
| rs10878126 | 0.0015 | A/C | C (0.4857) | 12 | 62930978 | 0.00084 | 4.07 (1.65,10.61) |
| rs10095433 | 0.0015 | A/G | A (0.819) | 8 | 65354626 | 0.0031 | 6.24 (1.66,35.3) |
| rs6414806 | 0.0015 | A/G | G (0.4709) | 5 | 66856030 | 0.019 | 2.65 (1.13,6.44) |
| rs1954338 | 0.0015 | A/G | A (0.2095) | 1 | 80453696 | 0.094 | 2.3 (0.81,6.84) |
| rs1395370 | 0.0015 | A/C | A (0.0931) | 4 | 83916757 | 0.13 | 4.14 (0.7,44.04) |
| rs2436974 | 0.0015 | A/G | A (0.219) | 1 | 87952087 | 0.011 | 4.03 (1.3,14.12) |
| rs7951671 | 0.0015 | A/G | G (0.3238) | 11 | 99761460 | 0.00021 | 4.88 (1.95,12.86) |
| rs10516464 | 0.0015 | A/G | A (0.2067) | 4 | 101690536 | 0.11 | 2.14 (0.8,5.89) |
| rs12117918 | 0.0015 | A/G | G (0.6571) | 1 | 102062768 | 0.071 | 2.17 (0.89,5.51) |
| rs9489181 | 0.0015 | A/G | G (0.9048) | 6 | 117865821 | 0.0038 | 8.18 (1.61,80.71) |
| rs10091528 | 0.0015 | A/G | A (0.9095) | 8 | 139672293 | 0.07 | 4.44 (0.75,47.1) |
| rs1856269 | 0.0015 | A/G | G (0.4524) | 6 | 145745866 | 0.0029 | 3.58 (1.46,9.3) |
| rs4896802 | 0.0015 | A/G | A (0.4524) | 6 | 145767354 | 0.0029 | 3.58 (1.46,9.3) |
| rs4708463 | 0.0015 | A/G | G (0.6048) | 6 | 168618109 | 0.11 | 1.95 (0.81,4.85) |
| rs1454694 | 0.0015 | A/G | G (0.1942) | 4 | 182573096 | 0.014 | 4.21 (1.24,16.7) |
| rs7375589 | 0.0015 | A/C | C (0.0714) | 4 | 190671494 | 0.02 | Inf (1.36,Inf) |
| rs6593903 | 0.0015 | A/G | G (0.0905) | 1 | 200316507 | 0.039 | 3.97 (1.05,18.68) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs6598048 | 0.0016 | A/G | G (0.1905) | 11 | 266012 | 0.029 | 3.54 (1.03,14.13) |
| rs867814 | 0.0016 | A/G | G (0.119) | 1 | 3385413 | 0.0083 | 7.17 (1.38,71.92) |
| rs7256147 | 0.0016 | A/G | G (0.8381) | 19 | 6872868 | 0.089 | 3.4 (0.84,20.02) |
| rs12919774 | 0.0016 | A/G | A (0.7619) | 16 | 8455709 | 0.0024 | 5.3 (1.6,23.09) |
| rs10271818 | 0.0016 | A/G | G (0.8077) | 7 | 9910878 | 0.0096 | 5.14 (1.33,29.5) |
| rs362128 | 0.0016 | A/G | G (0.9286) | 22 | 16949269 | 0.31 | 4.2 (0.32,226.71) |
| rs2817204 | 0.0016 | A/G | A (0.0714) | 6 | 24584673 | 0.0096 | Inf (1.59,Inf) |
| rs229078 | 0.0016 | A/C | A (0.335) | 21 | 27218006 | 0.098 | 2.1 (0.86,5.19) |
| rs176338 | 0.0016 | A/G | A (0.4429) | 14 | 28081367 | 0.011 | 2.86 (1.22,6.89) |
| rs2833505 | 0.0016 | A/G | G (0.4381) | 21 | 32080833 | 0.077 | 2.15 (0.93,5.08) |
| rs502806 | 0.0016 | A/G | G (0.1524) | 15 | 32851160 | 0.006 | 5.04 (1.39,23.16) |
| rs983743 | 0.0016 | A/G | A (0.2381) | 15 | 36953365 | 0.0097 | 3.55 (1.26,10.8) |
| rs2484252 | 0.0016 | A/G | A (0.2048) | 10 | 37047727 | 0.0043 | 4.13 (1.42,13.28) |
| rs2837767 | 0.0016 | A/G | A (0.5095) | 21 | 40951763 | 0.032 | 2.44 (1.05,5.81) |
| rs1888303 | 0.0016 | A/G | G (0.2238) | 13 | 43272981 | 0.011 | 3.41 (1.25,9.87) |
| rs719428 | 0.0016 | A/G | A (0.5238) | 5 | 57746496 | 0.0063 | 3.07 (1.31,7.41) |
| rs11006132 | 0.0016 | A/G | G (0.1881) | 10 | 59826172 | 0.0049 | 4.86 (1.49,18.82) |
| rs1657050 | 0.0016 | A/G | A (0.3095) | 12 | 62913326 | 0.001 | 4.05 (1.64,10.45) |
| rs3923754 | 0.0016 | A/G | G (0.9143) | 18 | 65482922 | 0.017 | 6.21 (1.16,63.16) |
| rs917343 | 0.0016 | A/C | A (0.3221) | 17 | 66910901 | 0.0039 | 3.41 (1.38,8.78) |
| rs636471 | 0.0016 | A/G | A (0.0625) | 11 | 79326149 | 0.011 | Inf (1.36,Inf) |
| rs7942499 | 0.0016 | A/G | A (0.2571) | 11 | 83287906 | 5.00E-04 | 5.43 (1.91,17.27) |
| rs6840263 | 0.0016 | A/C | A (0.9118) | 4 | 95066089 | 0.0092 | 6.93 (1.33,69.55) |
| rs2781413 | 0.0016 | A/G | G (0.2667) | 14 | 97038740 | 0.024 | 3.02 (1.13,8.43) |
| rs1506085 | 0.0016 | A/G | A (0.8798) | 6 | 100961343 | 0.037 | 4.88 (0.98,47.67) |
| rs1007468 | 0.0016 | A/G | A (0.2905) | 9 | 101808460 | 0.002 | 3.72 (1.5,9.58) |
| rs668660 | 0.0016 | A/G | G (0.7667) | 11 | 101869729 | 0.0036 | 4.45 (1.46,16.54) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2819419 | 0.0016 | A/C | C (0.5721) | 14 | 104477283 | 0.005 | 3.37 (1.37,8.81) |
| rs1026975 | 0.0016 | A/C | C (0.2143) | 4 | 114328978 | 0.071 | 2.45 (0.93,6.69) |
| rs1711882 | 0.0016 | A/G | G (0.1333) | 10 | 120320249 | 0.15 | 2.06 (0.71,6.2) |
| rs1032463 | 0.0016 | A/G | G (0.1893) | 6 | 125876988 | 0.2 | 2.11 (0.69,6.69) |
| rs2683829 | 0.0016 | A/C | A (0.2524) | 2 | 141945650 | 0.039 | 2.75 (1.02,7.72) |
| rs7735908 | 0.0016 | A/G | A (0.319) | 5 | 161035603 | 0.0045 | 3.61 (1.37,10.03) |
| rs692403 | 0.0016 | A/G | G (0.119) | 5 | 176167681 | 0.26 | 1.95 (0.54,7.42) |
| rs6443571 | 0.0016 | A/G | G (0.3143) | 3 | 179932608 | 0.003 | 3.77 (1.5,9.89) |
| rs2177086 | 0.0016 | A/G | A (0.3762) | 2 | 202846796 | 0.0029 | 3.43 (1.45,8.39) |
| rs3811390 | 0.0016 | A/G | A (0.0577) | 1 | 204692448 | 0.0022 | Inf (2.06,Inf) |
| rs10495439 | 0.0016 | A/G | G (0.0952) | 1 | 235609344 | 0.17 | 2.25 (0.65,8.38) |
| rs11137110 | 0.0017 | A/G | A (0.9286) | 8 | 859170 | 0.17 | 2.92 (0.58,19.09) |
| rs11137116 | 0.0017 | A/G | A (0.9286) | 8 | 917220 | 0.17 | 2.92 (0.58,19.09) |
| rs7721902 | 0.0017 | A/G | A (0.1381) | 5 | 3349496 | 0.011 | 4.03 (1.3,14.12) |
| rs17151244 | 0.0017 | A/C | C (0.0571) | 8 | 10066862 | 0.16 | 5.72 (0.54,290.23) |
| rs1874206 | 0.0017 | A/G | A (0.4272) | 4 | 14547924 | 0.076 | 2.11 (0.91,5.01) |
| rs2522314 | 0.0017 | A/G | G (0.5762) | 22 | 16284552 | 0.018 | 2.75 (1.16,6.75) |
| rs4977686 | 0.0017 | A/C | C (0.5971) | 9 | 21189680 | 0.029 | 2.52 (1.05,6.3) |
| rs2128997 | 0.0017 | A/G | G (0.2857) | 14 | 22030942 | 0.02 | 3.11 (1.13,9.04) |
| rs7325483 | 0.0017 | A/G | A (0.2381) | 13 | 23006468 | 0.039 | 2.75 (1.02,7.72) |
| rs10186544 | 0.0017 | A/G | A (0.315) | 2 | 28594825 | 0.0013 | 3.86 (1.57,9.82) |
| rs1328326 | 0.0017 | A/G | A (0.4712) | 10 | 29899885 | 0.0098 | 3.07 (1.28,7.7) |
| rs7260196 | 0.0017 | A/G | A (0.4095) | 19 | 33696776 | 0.04 | 2.39 (0.98,5.97) |
| rs11591534 | 0.0017 | A/G | A (0.125) | 10 | 44252086 | 0.032 | 3.42 (0.99,13.65) |
| rs13036196 | 0.0017 | A/G | G (0.1905) | 2 | 45483832 | 0.035 | 3.26 (1.02,11.61) |
| rs2066309 | 0.0017 | A/G | A (0.919) | 14 | 46501281 | 0.07 | 4.44 (0.75,47.1) |
| rs1288706 | 0.0017 | A/G | A (0.1667) | 1 | 52466892 | 0.018 | 3.23 (1.13,9.87) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs789748 | 0.0017 | A/C | A (0.4571) | 12 | 62855528 | 0.0062 | 3.13 (1.32,7.71) |
| rs2643665 | 0.0017 | A/G | A (0.4619) | 12 | 62867251 | 0.0062 | 3.13 (1.32,7.71) |
| rs433309 | 0.0017 | A/G | A (0.7083) | 16 | 64392614 | 0.0036 | 4.35 (1.49,14.69) |
| rs12782697 | 0.0017 | A/G | G (0.1524) | 10 | 71749400 | 0.006 | 5.04 (1.39,23.16) |
| rs435958 | 0.0017 | A/G | G (0.5048) | 5 | 73748965 | 0.011 | 2.8 (1.2,6.72) |
| rs6858879 | 0.0017 | A/C | C (0.1031) | 4 | 74212912 | 0.029 | 5.83 (1.03,60.8) |
| rs2190097 | 0.0017 | A/G | A (0.7762) | 7 | 76683596 | 0.051 | 2.59 (0.97,7.58) |
| rs1907729 | 0.0017 | A/C | C (0.8429) | 10 | 78348173 | 0.087 | 2.6 (0.81,9.94) |
| rs725514 | 0.0017 | A/C | A (0.6298) | 16 | 81412395 | 0.017 | 2.81 (1.16,7.12) |
| rs997721 | 0.0017 | A/G | G (0.919) | 4 | 83702471 | 0.027 | 4.71 (1.09,28.83) |
| rs2950849 | 0.0017 | A/G | A (0.881) | 5 | 90430381 | 0.0035 | 11.74 (1.64,517.13) |
| rs10493865 | 0.0017 | A/G | A (0.0952) | 1 | 93193661 | 0.0038 | 8.18 (1.61,80.71) |
| rs1943504 | 0.0017 | A/G | A (0.5) | 11 | 94892375 | 0.019 | 2.68 (1.15,6.41) |
| rs6734569 | 0.0017 | A/C | A (0.2333) | 2 | 98614814 | 0.02 | 3.11 (1.13,9.04) |
| rs13426867 | 0.0017 | A/G | G (0.851) | 2 | 101139193 | 0.01 | 4.92 (1.28,28.16) |
| rs7965538 | 0.0017 | A/G | A (0.226) | 12 | 111804318 | 0.016 | 2.95 (1.14,7.98) |
| rs2809380 | 0.0017 | A/G | A (0.0857) | 9 | 117931133 | 0.0018 | 9.24 (1.86,90.63) |
| rs1649200 | 0.0017 | A/G | A (0.8476) | 10 | 123233720 | 0.012 | 6.33 (1.34,60.52) |
| rs741606 | 0.0017 | A/G | G (0.8476) | 7 | 133548689 | 0.026 | 3.61 (1.05,16.04) |
| rs563275 | 0.0017 | A/G | A (0.2) | 3 | 189141078 | 0.02 | 3.11 (1.13,9.04) |
| rs502658 | 0.0017 | A/G | G (0.148) | 1 | 197661662 | 0.01 | 3.98 (1.25,14.18) |
| rs11774254 | 0.0018 | A/G | G (0.149) | 8 | 839254 | 0.038 | 3.14 (0.98,11.21) |
| rs7027170 | 0.0018 | A/G | A (0.4238) | 9 | 6819942 | 0.03 | 2.48 (1.06,5.92) |
| rs9303252 | 0.0018 | A/C | C (0.2087) | 17 | 10128654 | 0.0023 | 5.24 (1.61,20.23) |
| rs2093892 | 0.0018 | A/G | G (0.4952) | 20 | 12636384 | 0.032 | 2.44 (1.05,5.81) |
| rs10845745 | 0.0018 | A/G | A (0.3095) | 12 | 13357620 | 0.035 | 2.61 (1.06,6.62) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs10513131 | 0.0018 | A/G | A (0.1143) | 5 | 14140481 | 0.027 | 4.71 (1.09,28.83) |
| rs10065052 | 0.0018 | A/G | A (0.9) | 5 | 16419662 | 0.023 | 8.53 (1.13,383.04) |
| rs891275 | 0.0018 | A/G | G (0.4381) | 2 | 16470091 | 0.082 | 2.01 (0.87,4.72) |
| rs1890431 | 0.0018 | A/G | A (0.4429) | 1 | 21597384 | 0.0034 | 3.29 (1.4,7.99) |
| rs1983579 | 0.0018 | A/G | G (0.3762) | 6 | 25445614 | 0.03 | 2.48 (1.06,5.92) |
| rs6050757 | 0.0018 | A/G | A (0.9231) | 20 | 25577800 | 0.0092 | 6.93 (1.33,69.55) |
| rs7119999 | 0.0018 | A/G | A (0.5476) | 11 | 43503274 | 0.0034 | 3.34 (1.41,8.26) |
| rs2331175 | 0.0018 | A/G | A (0.9048) | 7 | 44733163 | 0.28 | 2.38 (0.44,16.17) |
| rs4785408 | 0.0018 | A/G | A (0.819) | 16 | 48919240 | 0.0034 | 7.53 (1.62,71.51) |
| rs2202950 | 0.0018 | A/G | A (0.365) | 16 | 58227657 | 0.017 | 2.97 (1.15,8.06) |
| rs343134 | 0.0018 | A/G | G (0.9048) | 13 | 59102990 | 0.089 | 3.4 (0.84,20.02) |
| rs1553106 | 0.0018 | A/G | G (0.2095) | 15 | 60890871 | 0.0084 | 3.3 (1.25,9.2) |
| rs1966963 | 0.0018 | A/C | C (0.4471) | 16 | 64345386 | 0.05 | 2.33 (0.99,5.64) |
| rs2268957 | 0.0018 | A/G | A (0.0619) | 14 | 65182986 | 0.04 | 8.98 (1.03,426.65) |
| rs4976079 | 0.0018 | A/G | G (0.5) | 5 | 66842798 | 0.012 | 2.75 (1.17,6.62) |
| rs2549516 | 0.0018 | A/G | A (0.7381) | 16 | 78057087 | 0.0044 | 4.16 (1.45,13.84) |
| rs7661056 | 0.0018 | A/G | G (0.1667) | 4 | 83927958 | 0.061 | 2.9 (0.89,10.47) |
| rs10496309 | 0.0018 | A/C | A (0.0857) | 2 | 84527130 | 0.0083 | 7.17 (1.38,71.92) |
| rs10035607 | 0.0018 | A/C | C (0.6095) | 5 | 89151688 | 0.00083 | 4.82 (1.77,14.8) |
| rs10493864 | 0.0018 | A/G | G (0.1048) | 1 | 93182735 | 0.0071 | 6.08 (1.47,36.22) |
| rs953504 | 0.0018 | A/G | G (0.919) | 13 | 96747390 | 0.0083 | 7.17 (1.38,71.92) |
| rs223500 | 0.0018 | A/G | A (0.281) | 4 | 104004247 | 0.03 | 2.65 (1.04,7.01) |
| rs2072162 | 0.0018 | A/G | G (0.6286) | 7 | 105230409 | 0.0087 | 3.13 (1.25,8.37) |
| rs595325 | 0.0018 | A/C | A (0.8) | 13 | 109717206 | 0.0081 | 4.58 (1.36,20.06) |
| rs4565713 | 0.0018 | A/G | G (0.3762) | 1 | 115581541 | 0.0018 | 3.54 (1.5,8.64) |
| rs838916 | 0.0018 | A/C | A (0.481) | 12 | 123775128 | 0.012 | 2.75 (1.17,6.62) |
| rs1432655 | 0.0018 | A/G | A (0.419) | 5 | 162961360 | 0.011 | 3.13 (1.26,8.06) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs1433781 | 0.0018 | A/G | A (0.419) | 5 | 162966587 | 0.011 | 3.13 (1.26,8.06) |
| rs9458896 | 0.0018 | A/G | A (0.6143) | 6 | 164134644 | 0.0024 | 3.81 (1.49,10.54) |
| rs764206 | 0.0018 | A/G | G (0.3571) | 1 | 168276709 | 0.03 | 2.42 (1.02,5.86) |
| rs2331452 | 0.0018 | A/C | A (0.1381) | 4 | 170326125 | 0.0036 | 6.82 (1.68,40.27) |
| rs4972806 | 0.0018 | A/G | A (0.3714) | 2 | 176838085 | 0.0015 | 3.75 (1.58,9.23) |
| rs17033401 | 0.0019 | A/G | A (0.1381) | 3 | 1103245 | 0.035 | 3.26 (1.02,11.61) |
| rs2281727 | 0.0019 | A/G | G (0.3333) | 17 | 2064695 | 0.035 | 2.61 (1.06,6.62) |
| rs2493314 | 0.0019 | A/C | A (0.1019) | 1 | 3423451 | 0.01 | 6.69 (1.28,67.18) |
| rs2240492 | 0.0019 | A/G | G (0.0721) | 4 | 5958959 | 0.04 | 7.82 (1.02,355.05) |
| rs1528345 | 0.0019 | A/G | G (0.7427) | 16 | 8439436 | 0.0023 | 5.57 (1.67,24.3) |
| rs2713344 | 0.0019 | A/G | G (0.2333) | 7 | 9315555 | 0.0012 | 4.3 (1.65,11.91) |
| rs10487881 | 0.0019 | A/C | A (0.2476) | 7 | 9690596 | 0.016 | 2.95 (1.14,7.98) |
| rs6475057 | 0.0019 | A/G | A (0.8381) | 9 | 16468688 | 0.018 | 4.52 (1.16,26.01) |
| rs4721857 | 0.0019 | A/G | A (0.8143) | 7 | 19823730 | 0.00094 | 8.85 (1.93,83.4) |
| rs525380 | 0.0019 | A/C | A (0.519) | 1 | 20154680 | 0.0034 | 3.29 (1.4,7.99) |
| rs7291023 | 0.0019 | A/C | A (0.0857) | 22 | 25420552 | 0.051 | 4.09 (0.91,25.4) |
| rs4820899 | 0.0019 | A/G | G (0.3476) | 22 | 29455091 | 0.0075 | 3.11 (1.27,7.86) |
| rs241424 | 0.0019 | A/G | A (0.4143) | 6 | 32912912 | 0.052 | 2.23 (0.96,5.28) |
| rs3091599 | 0.0019 | A/G | G (0.9333) | 20 | 39830225 | 0.01 | 10.74 (1.3,500.03) |
| rs7237741 | 0.0019 | A/G | G (0.4238) | 18 | 40464806 | 0.03 | 2.52 (1.08,6) |
| rs928508 | 0.0019 | A/G | G (0.3381) | 1 | 40892507 | 0.067 | 2.21 (0.92,5.38) |
| rs205795 | 0.0019 | A/G | G (0.6857) | 20 | 55806890 | 0.00083 | 4.82 (1.77,14.8) |
| rs11079516 | 0.0019 | A/G | G (0.8952) | 17 | 59396573 | 0.0019 | 12.9 (1.82,565.51) |
| rs4238372 | 0.0019 | A/G | G (0.1714) | 15 | 61138541 | 0.043 | 3.06 (1.01,10.05) |
| rs2363838 | 0.0019 | A/G | A (0.2429) | 17 | 62450618 | 0.0027 | 4.28 (1.54,12.89) |
| rs4976123 | 0.0019 | A/G | A (0.4905) | 5 | 67048553 | 0.011 | 2.94 (1.25,7.11) |
| rs7074141 | 0.0019 | A/G | A (0.1905) | 10 | 67452748 | 0.08 | 2.21 (0.85,5.88) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2278701 | 0.0019 | A/G | A (0.4095) | 15 | 78482170 | 0.01 | 2.88 (1.22,6.99) |
| rs1874795 | 0.0019 | A/G | A (0.4048) | 11 | 80407307 | 0.018 | 2.64 (1.12,6.39) |
| rs7810390 | 0.0019 | A/G | G (0.3857) | 7 | 82306748 | 0.0055 | 3.12 (1.31,7.63) |
| rs2309938 | 0.0019 | A/C | A (0.5238) | 2 | 101158112 | 0.012 | 2.75 (1.18,6.58) |
| rs1133186 | 0.0019 | A/C | A (0.2429) | 5 | 115448314 | 0.035 | 2.82 (1.02,8.26) |
| rs6534078 | 0.0019 | A/G | G (0.8286) | 4 | 119311504 | 0.012 | 6.33 (1.34,60.52) |
| rs1317514 | 0.0019 | A/G | A (0.6571) | 11 | 120019124 | 0.024 | 2.75 (1.09,7.37) |
| rs1321991 | 0.0019 | A/G | G (0.9333) | 1 | 182357628 | 0.32 | 2.16 (0.48,11.1) |
| rs2059516 | 0.0019 | A/G | A (0.3606) | 4 | 185845063 | 0.026 | 2.62 (1.1,6.39) |
| rs10494845 | 0.0019 | A/G | G (0.2333) | 1 | 200423836 | 0.0034 | 4.09 (1.52,11.75) |
| rs450015 | 0.002 | A/G | A (0.4714) | 18 | 7154754 | 0.00018 | 4.88 (2.01,12.43) |
| rs9650614 | 0.002 | A/G | A (0.1619) | 8 | 8730796 | 0.043 | 3.06 (1.01,10.05) |
| rs8099036 | 0.002 | A/G | G (0.5429) | 18 | 9746056 | 0.018 | 2.75 (1.16,6.75) |
| rs6074678 | 0.002 | A/G | A (0.3619) | 20 | 13979392 | 0.03 | 2.45 (1.04,5.88) |
| rs9907451 | 0.002 | A/G | A (0.9381) | 17 | 16745331 | 0.49 | 1.76 (0.35,9.43) |
| rs2957566 | 0.002 | A/G | A (0.9286) | 2 | 18135050 | 0.017 | 6.21 (1.16,63.16) |
| rs9470224 | 0.002 | A/G | A (0.9429) | 6 | 36248614 | 0.081 | 7.32 (0.78,356.8) |
| rs1997980 | 0.002 | A/G | G (0.781) | 6 | 42259213 | 0.00041 | 7.75 (2.09,43.54) |
| rs4459374 | 0.002 | A/G | G (0.6095) | 12 | 42837334 | 0.0016 | 3.81 (1.55,9.93) |
| rs1273109 | 0.002 | A/G | G (0.8667) | 12 | 61698032 | 0.0033 | 5.78 (1.53,32.82) |
| rs1322727 | 0.002 | A/G | A (0.9314) | 13 | 65633595 | 0.17 | 2.82 (0.56,18.46) |
| rs1330284 | 0.002 | A/G | A (0.4143) | 9 | 72805768 | 0.01 | 2.88 (1.22,6.99) |
| rs3742729 | 0.002 | A/G | A (0.919) | 14 | 77012331 | 0.28 | 2.38 (0.44,16.17) |
| rs6480848 | 0.002 | A/G | G (0.1683) | 10 | 78411312 | 0.019 | 3.72 (1.18,13.22) |
| rs4704619 | 0.002 | A/G | G (0.7095) | 5 | 79473195 | 0.057 | 2.41 (0.92,6.72) |
| rs2550766 | 0.002 | A/G | G (0.519) | 16 | 80018470 | 0.012 | 2.75 (1.18,6.58) |
| rs1567385 | 0.002 | A/G | A (0.6699) | 2 | 101773575 | 0.023 | 2.75 (1.09,7.44) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2085548 | 0.002 | A/G | A (0.3286) | 4 | 103754193 | 0.019 | 2.87 (1.15,7.4) |
| rs11212094 | 0.002 | A/G | G (0.481) | 11 | 106625752 | 0.12 | 1.91 (0.83,4.48) |
| rs7084183 | 0.002 | A/G | G (0.2762) | 10 | 132083965 | 0.028 | 2.69 (1.03,7.31) |
| rs6430637 | 0.002 | A/G | A (0.1952) | 2 | 137018567 | 0.058 | 2.55 (0.91,7.53) |
| rs2693547 | 0.002 | A/G | A (0.4476) | 4 | 138942427 | 0.011 | 2.94 (1.25,7.11) |
| rs6424377 | 0.002 | A/G | A (0.5524) | 1 | 142917444 | 0.012 | 2.75 (1.17,6.62) |
| rs3921860 | 0.002 | A/G | A (0.2571) | 3 | 178171199 | 0.0043 | 4.13 (1.42,13.28) |
| rs12123988 | 0.002 | A/G | G (0.1333) | 1 | 242364057 | 0.039 | 3.97 (1.05,18.68) |
| rs10413199 | 0.0021 | A/G | A (0.1667) | 19 | 382187 | 0.13 | 2.36 (0.79,7.41) |
| rs12828016 | 0.0021 | A/C | C (0.4333) | 12 | 868626 | 0.18 | 1.72 (0.75,4.01) |
| rs3810420 | 0.0021 | A/G | A (0.1714) | 19 | 3316583 | 0.031 | 2.92 (1.01,9) |
| rs189772 | 0.0021 | A/G | G (0.7286) | 17 | 6898357 | 0.037 | 2.57 (0.99,7.17) |
| rs2282534 | 0.0021 | A/G | A (0.5952) | 18 | 11456335 | 0.033 | 2.41 (1.03,5.78) |
| rs7867569 | 0.0021 | A/G | A (0.1346) | 9 | 14653394 | 0.029 | 3.54 (1.03,14.13) |
| rs4519132 | 0.0021 | A/G | A (0.781) | 12 | 37905772 | 0.014 | 4.24 (1.26,18.66) |
| rs877605 | 0.0021 | A/C | C (0.7952) | 12 | 41384482 | 0.0063 | 4.15 (1.35,15.46) |
| rs7116965 | 0.0021 | A/G | G (0.8857) | 11 | 46217272 | 0.012 | 9.56 (1.3,426.01) |
| rs135783 | 0.0021 | A/G | A (0.0857) | 22 | 48263681 | 0.069 | 3.49 (0.89,16.63) |
| rs2071896 | 0.0021 | A/C | C (0.0857) | 22 | 48264357 | 0.069 | 3.49 (0.89,16.63) |
| rs4785434 | 0.0021 | A/G | A (0.0577) | 16 | 49145477 | 0.31 | 4.06 (0.31,219.41) |
| rs9395692 | 0.0021 | A/C | A (0.9286) | 6 | 51542879 | 0.2 | 2.58 (0.61,12.85) |
| rs289754 | 0.0021 | A/G | G (0.6714) | 16 | 55623057 | 0.0018 | 4.24 (1.55,13.03) |
| rs11152187 | 0.0021 | A/G | G (0.4286) | 18 | 55699259 | 0.018 | 2.7 (1.15,6.49) |
| rs7177755 | 0.0021 | A/G | G (0.2549) | 15 | 60886781 | 0.0026 | 3.84 (1.5,10.33) |
| rs1543239 | 0.0021 | A/G | A (0.1905) | 7 | 68117747 | 0.011 | 4.03 (1.3,14.12) |
| rs9899648 | 0.0021 | A/G | G (0.0714) | 17 | 68224189 | 0.023 | 8.53 (1.13,383.04) |
| rs9564765 | 0.0021 | A/G | G (0.281) | 13 | 70431786 | 0.016 | 2.95 (1.14,7.98) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs10493493 | 0.0021 | A/C | C (0.6762) | 1 | 72281330 | 0.022 | 2.94 (1.14,8.16) |
| rs1157449 | 0.0021 | A/G | G (0.8524) | 8 | 73439958 | 0.0059 | 5.34 (1.4,30.46) |
| rs868265 | 0.0021 | A/G | G (0.8476) | 9 | 87351433 | 0.054 | 3.76 (0.95,21.94) |
| rs6665058 | 0.0021 | A/G | A (0.6286) | 1 | 94889389 | 0.0029 | 3.56 (1.42,9.5) |
| rs42998 | 0.0021 | A/G | A (0.9333) | 7 | 95409696 | 0.07 | 4.44 (0.75,47.1) |
| rs3798514 | 0.0021 | A/G | A (0.8981) | 6 | 100950989 | 0.062 | 4.53 (0.89,44.74) |
| rs7811378 | 0.0021 | A/G | A (0.2524) | 7 | 102646682 | 0.0063 | 3.74 (1.38,10.77) |
| rs10115465 | 0.0021 | A/G | A (0.7238) | 9 | 109590497 | 0.093 | 2.26 (0.89,6.07) |
| rs492560 | 0.0021 | A/G | A (0.6381) | 13 | 109717977 | 0.013 | 3.14 (1.22,8.7) |
| rs6585776 | 0.0021 | A/C | A (0.5882) | 10 | 123760084 | 0.0012 | 3.92 (1.6,9.91) |
| rs2676441 | 0.0021 | A/G | A (0.4327) | 10 | 131037682 | 0.00018 | 4.67 (1.93,11.8) |
| rs6106524 | 0.0022 | A/G | A (0.3095) | 20 | 2256430 | 0.024 | 2.61 (1.07,6.51) |
| rs10795250 | 0.0022 | A/G | G (0.3894) | 10 | 5154836 | 0.0014 | 3.77 (1.56,9.43) |
| rs150596 | 0.0022 | A/C | C (0.0625) | 5 | 6039480 | 0.16 | 5.72 (0.54,290.23) |
| rs749958 | 0.0022 | A/G | G (0.519) | 1 | 7506528 | 0.012 | 2.75 (1.17,6.62) |
| rs2255576 | 0.0022 | A/C | C (0.2857) | 10 | 7554589 | 0.00095 | 4.11 (1.64,10.79) |
| rs2719647 | 0.0022 | A/C | A (0.8762) | 16 | 10739945 | 0.00053 | Inf (2.64,Inf) |
| rs2710995 | 0.0022 | A/G | G (0.5667) | 7 | 24757906 | 0.0013 | 4.06 (1.59,11.23) |
| rs4466027 | 0.0022 | A/G | A (0.4524) | 4 | 26924971 | 0.033 | 2.41 (1.03,5.78) |
| rs12436395 | 0.0022 | A/G | G (0.519) | 14 | 29775777 | 0.00089 | 3.88 (1.63,9.58) |
| rs8096947 | 0.0022 | A/C | A (0.2143) | 18 | 35033179 | 0.11 | 2.23 (0.83,6.12) |
| rs970497 | 0.0022 | A/G | G (0.7238) | 15 | 36269186 | 0.00048 | 5.83 (1.93,21.5) |
| rs2242936 | 0.0022 | A/G | G (0.1571) | 21 | 39345341 | 0.011 | 4.03 (1.3,14.12) |
| rs4340035 | 0.0022 | A/C | C (0.0524) | 11 | 40772633 | 0.081 | 7.32 (0.78,356.8) |
| rs6749108 | 0.0022 | A/G | A (0.3654) | 2 | 43408637 | 0.013 | 2.98 (1.21,7.56) |
| rs3128247 | 0.0022 | A/C | C (0.5381) | 10 | 43465828 | 0.019 | 2.61 (1.12,6.24) |
| rs6583458 | 0.0022 | A/G | G (0.5857) | 7 | 48057231 | 0.03 | 2.52 (1.05,6.26) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs12451912 | 0.0022 | A/G | A (0.3238) | 17 | 62396346 | 0.0023 | 3.94 (1.51,10.93) |
| rs4671600 | 0.0022 | A/G | A (0.7524) | 2 | 64747746 | 0.037 | 3.06 (1.04,10.35) |
| rs7190459 | 0.0022 | A/C | C (0.9476) | 16 | 65467586 | 0.0044 | 12.58 (1.58,577.21) |
| rs7465525 | 0.0022 | A/G | A (0.5762) | 8 | 67157701 | 0.053 | 2.19 (0.93,5.29) |
| rs6460715 | 0.0022 | A/G | G (0.4429) | 7 | 71262550 | 0.011 | 2.86 (1.22,6.89) |
| rs1863414 | 0.0022 | A/G | G (0.1095) | 4 | 74141305 | 0.017 | 6.21 (1.16,63.16) |
| rs965351 | 0.0022 | A/C | A (0.1095) | 4 | 74143070 | 0.017 | 6.21 (1.16,63.16) |
| rs7206380 | 0.0022 | A/G | A (0.719) | 16 | 78029215 | 0.022 | 2.94 (1.14,8.16) |
| rs10862181 | 0.0022 | A/G | A (0.381) | 12 | 79562689 | 0.014 | 2.84 (1.17,7.09) |
| rs10865593 | 0.0022 | A/G | A (0.1571) | 3 | 81294204 | 0.02 | 3.63 (1.15,12.83) |
| rs7615367 | 0.0022 | A/C | A (0.1571) | 3 | 81296754 | 0.02 | 3.63 (1.15,12.83) |
| rs4324526 | 0.0022 | A/G | A (0.6238) | 4 | 88726009 | 0.00055 | 4.68 (1.78,13.55) |
| rs2722222 | 0.0022 | A/G | G (0.1333) | 12 | 88741732 | 0.0044 | 12.58 (1.58,577.21) |
| rs4905727 | 0.0022 | A/G | G (0.3619) | 14 | 98152977 | 0.0084 | 3.1 (1.29,7.66) |
| rs10989615 | 0.0022 | A/C | A (0.2163) | 9 | 101613529 | 0.0058 | 3.76 (1.34,11.39) |
| rs7681405 | 0.0022 | A/G | A (0.1238) | 4 | 101916518 | 0.09 | 2.75 (0.76,11.29) |
| rs7390631 | 0.0022 | A/C | C (0.6971) | 9 | 106875752 | 0.039 | 2.58 (1.02,6.91) |
| rs7772821 | 0.0022 | A/C | C (0.1857) | 6 | 132934199 | 0.00063 | 6.88 (1.97,30.99) |
| rs6600191 | 0.0023 | A/G | A (0.8143) | 16 | 235796 | 0.026 | 3.61 (1.05,16.04) |
| rs7730084 | 0.0023 | A/G | A (0.125) | 5 | 1778206 | 0.015 | 5.2 (1.23,31.35) |
| rs4786015 | 0.0023 | A/G | A (0.5524) | 16 | 5225395 | 0.0066 | 3.05 (1.28,7.61) |
| rs3087689 | 0.0023 | A/G | A (0.719) | 19 | 10525632 | 0.0019 | 4.12 (1.57,11.93) |
| rs1865307 | 0.0023 | A/G | G (0.5144) | 18 | 19134288 | 0.051 | 2.19 (0.94,5.23) |
| rs1071962 | 0.0023 | A/G | A (0.1429) | 22 | 28548695 | 0.12 | 3.02 (0.75,14.69) |
| rs6737948 | 0.0023 | A/G | A (0.8) | 2 | 33276895 | 0.025 | 3.92 (1.15,17.32) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs6029138 | 0.0023 | A/C | A (0.1429) | 20 | 38546320 | 0.0038 | 8.18 (1.61,80.71) |
| rs1662576 | 0.0023 | A/G | G (0.6286) | 17 | 42351244 | 0.01 | 2.99 (1.24,7.59) |
| rs197922 | 0.0023 | A/G | G (0.6714) | 17 | 42363569 | 0.024 | 2.75 (1.09,7.37) |
| rs1924632 | 0.0023 | A/G | A (0.5238) | 13 | 42776786 | 0.03 | 2.45 (1.04,5.88) |
| rs9536474 | 0.0023 | A/G | A (0.4143) | 13 | 53064956 | 0.067 | 2.21 (0.92,5.38) |
| rs1277281 | 0.0023 | A/G | G (0.1381) | 4 | 57697020 | 0.016 | 3.97 (1.18,15.68) |
| rs7899961 | 0.0023 | A/C | A (0.919) | 10 | 59696431 | 0.13 | 3.62 (0.56,39.69) |
| rs11151371 | 0.0023 | A/G | G (0.3762) | 18 | 63326628 | 0.042 | 2.41 (1.01,5.86) |
| rs8017202 | 0.0023 | A/G | G (0.9333) | 14 | 64924711 | 0.04 | 8.98 (1.03,426.65) |
| rs1953417 | 0.0023 | A/G | A (0.9333) | 14 | 64948662 | 0.04 | 8.98 (1.03,426.65) |
| rs754217 | 0.0023 | A/G | A (0.3619) | 16 | 65306407 | 0.01 | 2.88 (1.22,6.99) |
| rs6499096 | 0.0023 | A/C | A (0.3619) | 16 | 65316955 | 0.01 | 2.88 (1.22,6.99) |
| rs4246766 | 0.0023 | A/G | A (0.4857) | 5 | 66951186 | 0.033 | 2.41 (1.03,5.78) |
| rs1337269 | 0.0023 | A/G | A (0.6048) | 13 | 85247830 | 0.053 | 2.19 (0.93,5.29) |
| rs4271562 | 0.0023 | A/G | G (0.2837) | 15 | 86033594 | 0.056 | 2.39 (0.96,6.08) |
| rs10515329 | 0.0023 | A/C | A (0.0524) | 5 | 101877918 | 0.16 | 5.72 (0.54,290.23) |
| rs8180652 | 0.0023 | A/G | G (0.7619) | 6 | 107772723 | 0.096 | 2.5 (0.84,8.52) |
| rs11616523 | 0.0023 | A/G | A (0.2333) | 13 | 110234671 | 0.17 | 2.01 (0.75,5.58) |
| rs2604294 | 0.0023 | A/G | G (0.719) | 11 | 113661528 | 0.037 | 2.57 (0.99,7.17) |
| rs1146179 | 0.0023 | A/G | G (0.181) | 1 | 115471435 | 0.011 | 4.03 (1.3,14.12) |
| rs6827477 | 0.0023 | A/G | A (0.9238) | 4 | 118753533 | 0.081 | 7.32 (0.78,356.8) |
| rs4242363 | 0.0023 | A/G | A (0.2619) | 8 | 125275684 | 0.0092 | 3.22 (1.25,8.7) |
| rs2683824 | 0.0023 | A/G | G (0.4) | 2 | 141942729 | 0.025 | 2.61 (1.09,6.43) |
| rs2140652 | 0.0023 | A/G | G (0.0571) | 6 | 161550943 | 0.02 | Inf (1.13,Inf) |
| rs1554011 | 0.0023 | A/G | A (0.399) | 2 | 177377393 | 0.17 | 1.75 (0.75,4.11) |
| rs984893 | 0.0023 | A/G | A (0.9286) | 2 | 180314980 | 0.035 | 5.3 (0.94,54.86) |
| rs4915206 | 0.0023 | A/G | G (0.0857) | 1 | 197376708 | 0.0083 | 7.17 (1.38,71.92) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs740901 | 0.0024 | A/G | A (0.2429) | 12 | 5314401 | 0.03 | 2.65 (1.04,7.01) |
| rs2059912 | 0.0024 | A/G | A (0.9286) | 2 | 5961298 | 0.01 | 10.74 (1.3,500.03) |
| rs2169014 | 0.0024 | A/G | A (0.7905) | 18 | 8354598 | 0.025 | 3.92 (1.15,17.32) |
| rs3756948 | 0.0024 | A/G | A (0.7429) | 6 | 10663275 | 0.0045 | 4.93 (1.48,21.54) |
| rs2298532 | 0.0024 | A/G | G (0.5857) | 18 | 11496644 | 0.081 | 2.06 (0.89,4.88) |
| rs202091 | 0.0024 | A/G | G (0.2857) | 13 | 29775359 | 0.01 | 3.17 (1.25,8.33) |
| rs12141243 | 0.0024 | A/G | A (0.881) | 1 | 35016015 | 0.0017 | 14.11 (2.01,615.92) |
| rs4755711 | 0.0024 | A/G | G (0.5667) | 11 | 43364995 | 0.019 | 2.65 (1.13,6.44) |
| rs1864649 | 0.0024 | A/C | C (0.7143) | 17 | 51442621 | 0.016 | 3.25 (1.18,10.05) |
| rs6022639 | 0.0024 | A/G | G (0.1333) | 20 | 51706482 | 0.061 | 2.9 (0.89,10.47) |
| rs6022731 | 0.0024 | A/G | A (0.2857) | 20 | 51811531 | 0.0056 | 3.46 (1.37,9.08) |
| rs7334350 | 0.0024 | A/G | G (0.1762) | 13 | 53547811 | 0.0043 | 4.13 (1.42,13.28) |
| rs4628974 | 0.0024 | A/G | G (0.3238) | 16 | 64663264 | 0.1 | 2.02 (0.84,4.94) |
| rs527530 | 0.0024 | A/G | G (0.4524) | 13 | 74819568 | 0.0056 | 3.18 (1.35,7.72) |
| rs568772 | 0.0024 | A/C | A (0.4524) | 13 | 74821964 | 0.0056 | 3.18 (1.35,7.72) |
| rs1505231 | 0.0024 | A/G | G (0.4286) | 2 | 77503398 | 0.03 | 2.45 (1.04,5.88) |
| rs4975132 | 0.0024 | A/G | G (0.5429) | 4 | 79777795 | 0.02 | 2.56 (1.1,6.1) |
| rs1860610 | 0.0024 | A/C | C (0.0931) | 7 | 82794215 | 0.001 | 9.68 (1.98,94.26) |
| rs7146945 | 0.0024 | A/G | A (0.1286) | 14 | 91811259 | 0.016 | 3.97 (1.18,15.68) |
| rs2756916 | 0.0024 | A/C | C (0.1429) | 9 | 101757506 | 0.035 | 3.26 (1.02,11.61) |
| rs9308849 | 0.0024 | A/G | A (0.6635) | 2 | 101983905 | 0.027 | 2.6 (1.05,6.75) |
| rs6726051 | 0.0024 | A/G | A (0.6602) | 2 | 102055609 | 0.014 | 2.94 (1.16,7.94) |
| rs1048257 | 0.0024 | A/G | G (0.5667) | 14 | 104475429 | 0.011 | 2.86 (1.2,7.13) |
| rs2130519 | 0.0024 | A/G | G (0.1286) | 10 | 106556072 | 0.006 | 5.04 (1.39,23.16) |
| rs687928 | 0.0024 | A/G | A (0.4327) | 11 | 121501471 | 0.011 | 2.8 (1.2,6.72) |
| rs9419015 | 0.0024 | A/G | G (0.1394) | 10 | 134843257 | 0.77 | 1.35 (0.37,4.94) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs12534545 | 0.0024 | A/G | A (0.2048) | 7 | 139648647 | 0.015 | 3.4 (1.14,11.05) |
| rs6678283 | 0.0024 | A/G | G (0.419) | 1 | 207551434 | 0.0065 | 3 (1.28,7.24) |
| rs2487670 | 0.0025 | A/G | A (0.1048) | 1 | 3391300 | 0.017 | 6.21 (1.16,63.16) |
| rs2185639 | 0.0025 | A/G | G (0.1) | 1 | 3412884 | 0.017 | 6.21 (1.16,63.16) |
| rs390537 | 0.0025 | A/G | A (0.519) | 18 | 7157130 | 0.00085 | 4.1 (1.71,10.24) |
| rs11249984 | 0.0025 | A/G | A (0.1048) | 8 | 10198387 | 0.01 | 10.74 (1.3,500.03) |
| rs2210566 | 0.0025 | A/G | A (0.4466) | 20 | 12661699 | 0.0055 | 3.28 (1.36,8.25) |
| rs9967119 | 0.0025 | A/G | G (0.3476) | 18 | 19149047 | 0.035 | 2.61 (1.06,6.62) |
| rs4964058 | 0.0025 | A/G | A (0.5333) | 12 | 27414033 | 0.0062 | 3.13 (1.32,7.71) |
| rs35034 | 0.0025 | A/G | A (0.1779) | 12 | 31075619 | 0.0082 | 3.75 (1.28,12.13) |
| rs2746304 | 0.0025 | A/G | G (0.5619) | 6 | 39182513 | 0.019 | 2.68 (1.15,6.41) |
| rs7605661 | 0.0025 | A/G | A (0.6286) | 2 | 43536729 | 0.0052 | 3.35 (1.37,8.72) |
| rs2039541 | 0.0025 | A/G | A (0.6048) | 6 | 45805518 | 0.018 | 2.68 (1.12,6.68) |
| rs7244227 | 0.0025 | A/G | G (0.3429) | 18 | 46803943 | 0.04 | 2.39 (0.98,5.97) |
| rs4939652 | 0.0025 | A/G | A (0.3429) | 18 | 46903080 | 0.04 | 2.39 (0.98,5.97) |
| rs2027348 | 0.0025 | A/G | A (0.8619) | 14 | 48168021 | 0.012 | 6.33 (1.34,60.52) |
| rs6863219 | 0.0025 | A/G | A (0.49) | 5 | 66957234 | 0.03 | 2.48 (1.05,6.06) |
| rs7997970 | 0.0025 | A/G | G (0.8905) | 13 | 89868323 | 0.0035 | 11.74 (1.64,517.13) |
| rs12229563 | 0.0025 | A/G | A (0.9476) | 12 | 89962892 | 0.02 | Inf (1.36,Inf) |
| rs11612412 | 0.0025 | A/C | A (0.519) | 12 | 95925011 | 0.0055 | 3.14 (1.33,7.65) |
| rs1297355 | 0.0025 | A/G | A (0.5192) | 13 | 99912767 | 0.02 | 2.56 (1.1,6.1) |
| rs10850503 | 0.0025 | A/G | G (0.6333) | 12 | 108528787 | 0.00055 | 4.68 (1.78,13.55) |
| rs2278782 | 0.0025 | A/G | A (0.1748) | 4 | 111899758 | 0.042 | 2.92 (0.95,9.7) |
| rs7979852 | 0.0025 | A/C | C (0.5429) | 12 | 116621478 | 0.12 | 1.97 (0.85,4.64) |
| rs10857057 | 0.0025 | A/G | G (0.7524) | 4 | 119320688 | 0.0081 | 4.58 (1.36,20.06) |
| rs10886296 | 0.0025 | A/G | A (0.6714) | 10 | 120512222 | 0.0032 | 3.97 (1.45,12.22) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs672858 | 0.0025 | A/G | A (0.5333) | 4 | 125624402 | 0.0034 | 3.29 (1.4,7.99) |
| rs681139 | 0.0025 | A/G | G (0.9238) | 5 | 134618049 | 0.01 | 10.74 (1.3,500.03) |
| rs12663543 | 0.0025 | A/G | G (0.119) | 6 | 135559307 | 0.039 | 3.97 (1.05,18.68) |
| rs693437 | 0.0025 | A/G | G (0.2286) | 6 | 165914691 | 0.0097 | 3.55 (1.26,10.8) |
| rs4668014 | 0.0025 | A/G | A (0.875) | 2 | 168769272 | 0.0069 | 11.22 (1.56,494.7) |
| rs9828937 | 0.0025 | A/G | G (0.9095) | 3 | 178135842 | 0.00074 | Inf (2.58,Inf) |
| rs3821010 | 0.0025 | A/G | G (0.3762) | 2 | 178508162 | 0.026 | 2.62 (1.1,6.39) |
| rs7542189 | 0.0025 | A/G | A (0.4714) | 1 | 234738811 | 0.051 | 2.26 (0.97,5.41) |
| rs11970426 | 0.0026 | A/G | G (0.8798) | 6 | 160423 | 0.037 | 4.88 (0.98,47.67) |
| rs7305099 | 0.0026 | A/C | C (0.5762) | 12 | 845537 | 0.24 | 1.65 (0.72,3.88) |
| rs2147263 | 0.0026 | A/G | G (0.52) | 9 | 2177750 | 0.072 | 2.14 (0.9,5.17) |
| rs6908760 | 0.0026 | A/G | G (0.0905) | 6 | 11841763 | 0.017 | 6.21 (1.16,63.16) |
| rs1980696 | 0.0026 | A/G | A (0.8413) | 13 | 23557212 | 0.04 | 3.46 (1,15.48) |
| rs2290052 | 0.0026 | A/G | A (0.9) | 2 | 30185186 | 0.01 | 10.74 (1.3,500.03) |
| rs991570 | 0.0026 | A/G | A (0.419) | 5 | 35840965 | 0.0063 | 3.07 (1.31,7.41) |
| rs2592866 | 0.0026 | A/G | G (0.8714) | 13 | 57124943 | 0.032 | 4.13 (1.05,23.93) |
| rs831207 | 0.0026 | A/G | A (0.0762) | 13 | 59039048 | 0.067 | 4.18 (0.83,41.29) |
| rs574291 | 0.0026 | A/C | A (0.0762) | 13 | 59052823 | 0.067 | 4.18 (0.83,41.29) |
| rs7581802 | 0.0026 | A/C | C (0.1571) | 2 | 62001589 | 0.00078 | 10.37 (2.12,100.84) |
| rs1470025 | 0.0026 | A/G | A (0.7048) | 5 | 79458185 | 0.032 | 2.78 (1.04,8.1) |
| rs1034052 | 0.0026 | A/G | A (0.381) | 13 | 85346055 | 0.1 | 2.01 (0.83,4.97) |
| rs2725236 | 0.0026 | A/G | G (0.4905) | 4 | 89276285 | 0.019 | 2.68 (1.15,6.41) |
| rs1397793 | 0.0026 | A/G | A (0.5905) | 5 | 90507207 | 7.00E-04 | 4.31 (1.72,11.55) |
| rs7167842 | 0.0026 | A/G | A (0.6571) | 15 | 91356254 | 0.024 | 2.75 (1.09,7.37) |
| rs712600 | 0.0026 | A/G | G (0.2619) | 5 | 115686794 | 0.071 | 2.45 (0.93,6.69) |
| rs10955854 | 0.0026 | A/C | A (0.8) | 8 | 119167708 | 0.0044 | 4.16 (1.45,13.84) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs4733616 | 0.0026 | A/G | A (0.1905) | 8 | 128731277 | 0.12 | 2.45 (0.78,8.21) |
| rs1554816 | 0.0026 | A/G | G (0.2) | 6 | 144967923 | 0.0052 | 3.9 (1.4,11.81) |
| rs4274541 | 0.0026 | A/G | A (0.5865) | 2 | 147388592 | 0.018 | 2.77 (1.16,6.83) |
| rs13166175 | 0.0026 | A/C | A (0.219) | 5 | 166713648 | 0.011 | 4.03 (1.3,14.12) |
| rs10516032 | 0.0026 | A/G | G (0.9095) | 5 | 166726668 | 0.0019 | Inf (2.13,Inf) |
| rs6719664 | 0.0026 | A/G | A (0.9095) | 2 | 212350545 | 0.067 | 4.18 (0.83,41.29) |
| rs9903427 | 0.0027 | A/G | A (0.9286) | 17 | 16698929 | 0.52 | 1.7 (0.4,7.57) |
| rs7558682 | 0.0027 | A/C | A (0.8558) | 2 | 17375596 | 0.031 | 4.31 (1.1,24.99) |
| rs179196 | 0.0027 | A/C | C (0.9375) | 16 | 19204720 | 0.098 | 3.37 (0.72,21.43) |
| rs958635 | 0.0027 | A/G | G (0.4667) | 3 | 21047047 | 0.031 | 2.49 (1.06,6.02) |
| rs7384684 | 0.0027 | A/G | G (0.3333) | 7 | 22489764 | 0.0021 | 3.69 (1.51,9.36) |
| rs625812 | 0.0027 | A/G | G (0.3317) | 5 | 29536062 | 0.0035 | 3.57 (1.44,9.21) |
| rs1762594 | 0.0027 | A/G | A (0.3238) | 10 | 30715496 | 0.0023 | 3.94 (1.51,10.93) |
| rs8087061 | 0.0027 | A/C | A (0.9095) | 18 | 44177443 | 0.2 | 2.58 (0.61,12.85) |
| rs11038863 | 0.0027 | A/G | G (0.8048) | 11 | 46304900 | 0.018 | 4.52 (1.16,26.01) |
| rs2071358 | 0.0027 | A/C | A (0.2476) | 12 | 46652716 | 0.039 | 2.75 (1.02,7.72) |
| rs2239843 | 0.0027 | A/G | A (0.0905) | 22 | 48355848 | 0.069 | 3.49 (0.89,16.63) |
| rs1037426 | 0.0027 | A/G | G (0.4333) | 2 | 50186425 | 0.076 | 2.11 (0.91,5.01) |
| rs3861561 | 0.0027 | A/G | G (0.4333) | 2 | 50186729 | 0.076 | 2.11 (0.91,5.01) |
| rs13135792 | 0.0027 | A/G | G (0.3476) | 4 | 55393258 | 0.0015 | 3.71 (1.55,9.16) |
| rs7143202 | 0.0027 | A/G | G (0.8077) | 14 | 58563776 | 0.0015 | 6.54 (1.73,37.06) |
| rs180054 | 0.0027 | A/G | G (0.9333) | 17 | 65480267 | 0.13 | 3.62 (0.56,39.69) |
| rs1831143 | 0.0027 | A/G | A (0.1857) | 9 | 70629304 | 0.0017 | 4.9 (1.61,16.97) |
| rs2219 | 0.0027 | A/G | G (0.781) | 9 | 75509420 | 0.039 | 2.92 (0.99,9.86) |
| rs7991663 | 0.0027 | A/C | C (0.1762) | 13 | 79031300 | 0.17 | 2.25 (0.65,8.38) |
| rs9787677 | 0.0027 | A/G | A (0.6571) | 10 | 79671456 | 0.02 | 2.97 (1.12,8.65) |
| rs2170819 | 0.0027 | A/C | C (0.276) | 14 | 97046517 | 0.021 | 3.04 (1.08,9.02) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs7931273 | 0.0027 | A/G | A (0.319) | 11 | 100353225 | 0.013 | 2.85 (1.16,7.22) |
| rs823907 | 0.0027 | A/G | G (0.1905) | 9 | 101774479 | 0.018 | 3.23 (1.13,9.87) |
| rs873598 | 0.0027 | A/G | G (0.481) | 10 | 120413174 | 0.078 | 2.12 (0.91,5.07) |
| rs564037 | 0.0027 | A/G | G (0.4286) | 11 | 121519609 | 0.011 | 2.8 (1.2,6.72) |
| rs7629719 | 0.0027 | A/C | A (0.0619) | 3 | 131642632 | 0.18 | 3.23 (0.6,32.73) |
| rs4954609 | 0.0027 | A/G | G (0.1952) | 2 | 137003227 | 0.058 | 2.55 (0.91,7.53) |
| rs3811450 | 0.0027 | A/G | A (0.0857) | 1 | 151364105 | 0.017 | 6.21 (1.16,63.16) |
| rs12075 | 0.0027 | A/G | A (0.6095) | 1 | 155988427 | 0.018 | 2.68 (1.12,6.68) |
| rs4654164 | 0.0027 | A/C | A (0.2788) | 1 | 242380828 | 0.0078 | 3.47 (1.32,9.67) |
| rs6041524 | 0.0028 | A/G | A (0.3571) | 20 | 1266214 | 0.024 | 2.61 (1.07,6.51) |
| rs4150579 | 0.0028 | A/G | A (0.3048) | 11 | 18313756 | 0.00026 | 5.11 (1.97,14.15) |
| rs2467691 | 0.0028 | A/G | A (0.8333) | 8 | 25616924 | 0.032 | 3.32 (1.06,12.5) |
| rs10506018 | 0.0028 | A/G | G (0.1857) | 12 | 27387124 | 0.11 | 2.23 (0.83,6.12) |
| rs6488063 | 0.0028 | A/G | A (0.2286) | 12 | 32597583 | 0.043 | 3.06 (1.01,10.05) |
| rs1997884 | 0.0028 | A/G | G (0.3667) | 22 | 34428278 | 0.065 | 2.19 (0.91,5.41) |
| rs7280028 | 0.0028 | A/G | A (0.819) | 21 | 35338710 | 0.052 | 3.07 (0.97,11.61) |
| rs6973 | 0.0028 | A/C | A (0.2206) | 22 | 41883635 | 0.0031 | 4.73 (1.55,16.38) |
| rs1992691 | 0.0028 | A/G | A (0.5857) | 14 | 47653933 | 0.0025 | 3.79 (1.52,10.14) |
| rs2413987 | 0.0028 | A/G | A (0.476) | 15 | 48037645 | 0.019 | 2.68 (1.15,6.41) |
| rs4540741 | 0.0028 | A/G | G (0.4429) | 10 | 66571401 | 0.01 | 2.91 (1.24,7.03) |
| rs995506 | 0.0028 | A/G | G (0.4667) | 16 | 81516938 | 0.018 | 2.75 (1.16,6.75) |
| rs12597413 | 0.0028 | A/G | G (0.7286) | 16 | 86782559 | 0.024 | 2.75 (1.09,7.37) |
| rs4296871 | 0.0028 | A/G | A (0.1238) | 6 | 92297511 | 0.051 | 4.09 (0.91,25.4) |
| rs234219 | 0.0028 | A/G | G (0.9286) | 14 | 97104106 | 0.01 | 10.74 (1.3,500.03) |
| rs17025978 | 0.0028 | A/G | G (0.9048) | 1 | 110781653 | 0.012 | 6.33 (1.34,60.52) |
| rs2419627 | 0.0028 | A/G | G (0.7621) | 10 | 114157293 | 0.0097 | 3.42 (1.24,10.62) |
| rs6542345 | 0.0028 | A/C | C (0.6923) | 2 | 117453662 | 0.00036 | 5.41 (1.9,17.9) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs4751664 | 0.0028 | A/G | G (0.2714) | 10 | 120245905 | 0.08 | 2.21 (0.85,5.88) |
| rs9422967 | 0.0028 | A/G | A (0.2095) | 10 | 126116799 | 0.0052 | 3.9 (1.4,11.81) |
| rs1399928 | 0.0028 | A/G | A (0.2095) | 10 | 126125252 | 0.0052 | 3.9 (1.4,11.81) |
| rs2116798 | 0.0028 | A/G | G (0.1381) | 5 | 166199519 | 0.09 | 2.75 (0.76,11.29) |
| rs12633719 | 0.0028 | A/G | G (0.2524) | 3 | 189253308 | 0.018 | 3.23 (1.13,9.87) |
| rs10014577 | 0.0028 | A/G | A (0.2095) | 4 | 189911360 | 0.0021 | 5.43 (1.67,20.94) |
| rs4844687 | 0.0028 | A/G | A (0.6381) | 1 | 205089529 | 0.0091 | 3.15 (1.28,8.18) |
| rs7425448 | 0.0028 | A/G | G (0.8619) | 2 | 212384046 | 0.022 | 5.76 (1.2,55.34) |
| rs2788193 | 0.0028 | A/G | A (0.2714) | 1 | 214846622 | 0.0063 | 3.74 (1.38,10.77) |
| rs1885987 | 0.0029 | A/C | A (0.5913) | 17 | 2149775 | 0.031 | 2.42 (1.02,5.97) |
| rs4527134 | 0.0029 | A/G | A (0.7905) | 19 | 6884907 | 0.084 | 2.83 (0.89,10.75) |
| rs2967675 | 0.0029 | A/G | A (0.1394) | 19 | 8650484 | 0.09 | 2.75 (0.76,11.29) |
| rs4496807 | 0.0029 | A/C | A (0.1905) | 6 | 9951561 | 0.043 | 3.06 (1.01,10.05) |
| rs4791463 | 0.0029 | A/G | A (0.6238) | 17 | 11157311 | 0.11 | 2.08 (0.86,5.17) |
| rs1653751 | 0.0029 | A/G | A (0.281) | 2 | 23632414 | 0.03 | 2.65 (1.04,7.01) |
| rs2285688 | 0.0029 | A/G | A (0.7) | 7 | 24647699 | 0.0096 | 3.48 (1.26,10.73) |
| rs140129 | 0.0029 | A/G | A (0.5095) | 22 | 28475103 | 0.0063 | 3.07 (1.31,7.41) |
| rs131271 | 0.0029 | A/G | G (0.5095) | 22 | 28476136 | 0.0063 | 3.07 (1.31,7.41) |
| rs131272 | 0.0029 | A/G | G (0.5095) | 22 | 28478209 | 0.0063 | 3.07 (1.31,7.41) |
| rs131273 | 0.0029 | A/G | G (0.5095) | 22 | 28478275 | 0.0063 | 3.07 (1.31,7.41) |
| rs131285 | 0.0029 | A/G | G (0.5095) | 22 | 28482494 | 0.0063 | 3.07 (1.31,7.41) |
| rs1034716 | 0.0029 | A/G | G (0.3333) | 7 | 29207684 | 0.065 | 2.19 (0.91,5.41) |
| rs1955773 | 0.0029 | A/G | G (0.6286) | 14 | 29800649 | 0.0051 | 3.34 (1.33,8.92) |
| rs6989203 | 0.0029 | A/G | G (0.7429) | 8 | 41642902 | 0.0031 | 6.24 (1.66,35.3) |
| rs3764465 | 0.0029 | A/G | G (0.3476) | 18 | 46825370 | 0.04 | 2.39 (0.98,5.97) |
| rs3809325 | 0.0029 | A/G | A (0.5577) | 13 | 49164110 | 0.0094 | 3 (1.26,7.33) |
| rs12407003 | 0.0029 | A/G | A (0.1381) | 1 | 58651655 | 0.035 | 3.26 (1.02,11.61) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs6950870 | 0.0029 | A/G | G (0.8619) | 7 | 76594686 | 0.089 | 3.4 (0.84,20.02) |
| rs12606 | 0.0029 | A/G | G (0.6762) | 6 | 76681461 | 0.00019 | 5.76 (2.03,19.08) |
| rs4732493 | 0.0029 | A/C | A (0.181) | 7 | 82334552 | 0.035 | 3.26 (1.02,11.61) |
| rs1471106 | 0.0029 | A/G | G (0.3381) | 5 | 83041316 | 0.1 | 2.01 (0.83,4.97) |
| rs7514724 | 0.0029 | A/G | G (0.8619) | 1 | 91885868 | 0.043 | 3.32 (0.96,14.81) |
| rs518677 | 0.0029 | A/G | G (0.3333) | 11 | 99792469 | 0.00023 | 4.82 (1.95,12.49) |
| rs12197000 | 0.0029 | A/C | A (0.5524) | 6 | 106884765 | 0.019 | 2.61 (1.12,6.24) |
| rs687910 | 0.0029 | A/G | G (0.4279) | 11 | 121504397 | 0.011 | 2.8 (1.2,6.72) |
| rs2912787 | 0.0029 | A/G | A (0.8238) | 10 | 123315528 | 0.043 | 3.32 (0.96,14.81) |
| rs432519 | 0.0029 | A/C | C (0.1333) | 7 | 150450911 | 0.029 | 3.54 (1.03,14.13) |
| rs247999 | 0.0029 | A/G | A (0.3476) | 5 | 167417996 | 0.024 | 2.61 (1.07,6.51) |
| rs334062 | 0.0029 | A/G | A (0.5429) | 2 | 178825798 | 0.0033 | 3.4 (1.4,8.65) |
| rs6443559 | 0.0029 | A/C | C (0.319) | 3 | 179877688 | 0.0026 | 3.84 (1.5,10.33) |
| rs9290674 | 0.0029 | A/G | A (0.519) | 3 | 180144516 | 0.049 | 2.31 (0.99,5.48) |
| rs1501560 | 0.0029 | A/C | C (0.2619) | 1 | 207502186 | 0.018 | 2.9 (1.14,7.65) |
| rs10488368 | 0.003 | A/G | G (0.1095) | 8 | 180568 | 0.094 | 3.49 (0.74,22.16) |
| rs9314442 | 0.003 | A/G | G (0.1095) | 8 | 181226 | 0.094 | 3.49 (0.74,22.16) |
| rs4959232 | 0.003 | A/G | G (0.6571) | 6 | 3247216 | 0.0056 | 3.72 (1.36,11.45) |
| rs2570668 | 0.003 | A/G | G (0.2143) | 8 | 6017976 | 0.0021 | 4.54 (1.57,14.52) |
| rs4909945 | 0.003 | A/G | G (0.7136) | 11 | 10630315 | 0.059 | 2.35 (0.91,6.37) |
| rs6131568 | 0.003 | A/G | G (0.181) | 20 | 14242196 | 0.031 | 2.92 (1.01,9) |
| rs6740355 | 0.003 | A/G | A (0.4078) | 2 | 21635495 | 0.0084 | 3.1 (1.29,7.66) |
| rs1818744 | 0.003 | A/C | A (0.1476) | 6 | 24128722 | 0.035 | 3.26 (1.02,11.61) |
| rs4534931 | 0.003 | A/G | A (0.3041) | 18 | 25505604 | 0.0011 | 4.82 (1.69,14.93) |
| rs10829015 | 0.003 | A/C | A (0.3857) | 10 | 26844248 | 0.0075 | 3.11 (1.27,7.86) |
| rs9311139 | 0.003 | A/G | G (0.2286) | 3 | 36880627 | 0.0058 | 4.45 (1.45,15.49) |
| rs2837766 | 0.003 | A/G | A (0.3381) | 21 | 40950152 | 0.1 | 2.02 (0.84,4.94) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs3123670 | 0.003 | A/G | G (0.7718) | 10 | 43478989 | 0.013 | 3.47 (1.18,11.73) |
| rs9534565 | 0.003 | A/G | G (0.4762) | 13 | 46675374 | 0.0063 | 3.07 (1.31,7.41) |
| rs7805656 | 0.003 | A/G | G (0.3905) | 7 | 49955477 | 0.03 | 2.42 (1.02,5.86) |
| rs1954329 | 0.003 | A/C | C (0.481) | 14 | 52938344 | 0.011 | 2.83 (1.2,6.89) |
| rs920921 | 0.003 | A/C | C (0.8714) | 15 | 66573339 | 0.023 | 5.21 (1.07,50.48) |
| rs701662 | 0.003 | A/G | A (0.8619) | 6 | 68946749 | 0.07 | 3.03 (0.86,13.64) |
| rs11615839 | 0.003 | A/G | A (0.8571) | 12 | 69133885 | 0.11 | 2.76 (0.78,12.52) |
| rs8021398 | 0.003 | A/G | A (0.1048) | 14 | 75916679 | 0.0044 | 12.58 (1.58,577.21) |
| rs941174 | 0.003 | A/G | A (0.5571) | 12 | 76329438 | 0.047 | 2.32 (0.95,5.88) |
| rs12200596 | 0.003 | A/C | C (0.1476) | 6 | 80092691 | 0.0036 | 6.82 (1.68,40.27) |
| rs12148845 | 0.003 | A/G | G (0.2762) | 15 | 86504550 | 0.0071 | 6.08 (1.47,36.22) |
| rs10765594 | 0.003 | A/G | A (0.2619) | 11 | 92563132 | 0.01 | 3.17 (1.25,8.33) |
| rs11607191 | 0.003 | A/G | A (0.1333) | 11 | 99827638 | 0.039 | 3.97 (1.05,18.68) |
| rs9513875 | 0.003 | A/G | G (0.3073) | 13 | 100689460 | 0.06 | 2.26 (0.91,5.74) |
| rs9514301 | 0.003 | A/G | A (0.7333) | 13 | 104084580 | 0.037 | 2.57 (0.99,7.17) |
| rs10979414 | 0.003 | A/G | G (0.6238) | 9 | 108466908 | 0.0066 | 3.03 (1.28,7.38) |
| rs17863168 | 0.003 | A/G | A (0.9095) | 7 | 126187418 | 0.2 | 2.58 (0.61,12.85) |
| rs1505864 | 0.003 | A/G | A (0.2571) | 4 | 140088670 | 0.028 | 2.69 (1.03,7.31) |
| rs9289603 | 0.003 | A/G | G (0.4524) | 3 | 141527616 | 0.034 | 2.34 (1.01,5.58) |
| rs1368412 | 0.003 | A/G | G (0.5286) | 5 | 147163887 | 0.042 | 2.41 (1.01,5.86) |
| rs16826788 | 0.003 | A/G | A (0.0952) | 2 | 147635659 | 0.0071 | 6.08 (1.47,36.22) |
| rs7429131 | 0.003 | A/G | A (0.1202) | 3 | 149333600 | 0.038 | 3.14 (0.98,11.21) |
| rs6789040 | 0.003 | A/G | G (0.4429) | 3 | 178176291 | 0.0015 | 3.75 (1.58,9.23) |
| rs2455864 | 0.003 | A/G | A (0.5429) | 1 | 191581346 | 0.053 | 2.19 (0.94,5.17) |
| rs4659766 | 0.003 | A/G | A (0.481) | 1 | 233526657 | 0.0017 | 3.71 (1.56,9.14) |
| rs3791423 | 0.0031 | A/G | G (0.9476) | unknown | 0 | 0.13 | 5.66 (0.69,263.42) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs890032 | 0.0031 | A/G | G (0.7286) | 8 | 6011583 | 0.0071 | 3.39 (1.28,9.85) |
| rs2148358 | 0.0031 | A/G | G (0.4714) | 9 | 7375564 | 0.019 | 2.68 (1.15,6.41) |
| rs2963327 | 0.0031 | A/G | G (0.2905) | 5 | 8987159 | 0.00048 | 4.87 (1.83,13.95) |
| rs2500081 | 0.0031 | A/G | G (0.9048) | 6 | 14880876 | 0.0083 | 7.17 (1.38,71.92) |
| rs9396834 | 0.0031 | A/G | G (0.2667) | 6 | 18272052 | 0.12 | 2 (0.76,5.38) |
| rs2451646 | 0.0031 | A/G | A (0.9429) | 9 | 19142187 | 0.07 | 4.44 (0.75,47.1) |
| rs2331662 | 0.0031 | A/G | G (0.9381) | 14 | 21898729 | 0.4 | 2.83 (0.39,32.62) |
| rs854342 | 0.0031 | A/G | G (0.2714) | 14 | 24409289 | 0.024 | 3.02 (1.13,8.43) |
| rs7976928 | 0.0031 | A/G | G (0.5143) | 12 | 24876436 | 0.0017 | 3.71 (1.56,9.14) |
| rs6936465 | 0.0031 | A/G | A (0.5238) | 6 | 25308821 | 0.0055 | 3.14 (1.33,7.65) |
| rs5758837 | 0.0031 | A/C | C (0.581) | 22 | 41373512 | 0.0058 | 3.25 (1.36,8.13) |
| rs13133564 | 0.0031 | A/G | A (0.5714) | 4 | 44293233 | 0.011 | 2.94 (1.25,7.11) |
| rs6493274 | 0.0031 | A/C | A (0.819) | 15 | 45463781 | 0.014 | 4.24 (1.26,18.66) |
| rs6906753 | 0.0031 | A/G | A (0.0714) | 6 | 45779378 | 0.02 | Inf (1.36,Inf) |
| rs10506399 | 0.0031 | A/G | G (0.75) | 12 | 58460760 | 0.013 | 3.47 (1.18,11.73) |
| rs7304290 | 0.0031 | A/G | A (0.475) | 12 | 58576619 | 0.0078 | 3.27 (1.32,8.5) |
| rs963261 | 0.0031 | A/G | G (0.4667) | 2 | 60444453 | 0.011 | 2.93 (1.24,7.21) |
| rs11002470 | 0.0031 | A/G | G (0.6635) | 10 | 79682887 | 0.03 | 2.92 (1.09,8.56) |
| rs1387551 | 0.0031 | A/G | G (0.9238) | 8 | 82842822 | 0.0044 | 12.58 (1.58,577.21) |
| rs1557431 | 0.0031 | A/C | C (0.2548) | 11 | 83270045 | 0.001 | 4.97 (1.73,15.85) |
| rs1357548 | 0.0031 | A/G | G (0.7905) | 7 | 84727719 | 0.16 | 2.09 (0.73,6.62) |
| rs362813 | 0.0031 | A/G | G (0.5429) | 7 | 102808891 | 0.00095 | 3.81 (1.6,9.37) |
| rs694539 | 0.0031 | A/G | G (0.8238) | 11 | 113638629 | 0.096 | 2.5 (0.84,8.52) |
| rs7070744 | 0.0031 | A/G | G (0.5905) | 10 | 114187632 | 0.05 | 2.33 (0.99,5.64) |
| rs2293492 | 0.0031 | A/G | G (0.0952) | 7 | 128209017 | 0.0018 | 9.24 (1.86,90.63) |
| rs1538184 | 0.0031 | A/G | G (0.6381) | 6 | 130309754 | 0.0016 | 4.52 (1.66,13.88) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs908843 | 0.0031 | A/G | G (0.3667) | 8 | 139451045 | 0.041 | 2.39 (0.99,5.9) |
| rs707075 | 0.0031 | A/G | A (0.9143) | 2 | 155129678 | 0.0083 | 7.17 (1.38,71.92) |
| rs10798035 | 0.0031 | A/G | G (0.4712) | 1 | 182782074 | 0.011 | 2.83 (1.19,6.94) |
| rs4111008 | 0.0032 | A/G | A (0.9429) | 10 | 2304901 | 0.23 | 4.77 (0.55,226.37) |
| rs726688 | 0.0032 | A/G | A (0.2921) | 18 | 8493530 | 0.0072 | 3.6 (1.35,10.1) |
| rs4669862 | 0.0032 | A/G | A (0.9143) | 2 | 12671050 | 0.017 | 6.21 (1.16,63.16) |
| rs4293781 | 0.0032 | A/G | G (0.2857) | 4 | 14383260 | 0.0063 | 3.74 (1.38,10.77) |
| rs4977522 | 0.0032 | A/G | G (0.7905) | 9 | 18646065 | 0.024 | 3.14 (1.08,10.57) |
| rs2245409 | 0.0032 | A/G | G (0.1238) | 6 | 24889004 | 0.052 | 3.13 (0.89,12.67) |
| rs1948522 | 0.0032 | A/G | G (0.8048) | 9 | 27565785 | 0.0081 | 4.58 (1.36,20.06) |
| rs2088656 | 0.0032 | A/G | A (0.2667) | 12 | 32584741 | 0.018 | 3.23 (1.13,9.87) |
| rs11719120 | 0.0032 | A/G | G (0.1143) | 3 | 34815095 | 0.051 | 4.09 (0.91,25.4) |
| rs1434509 | 0.0032 | A/G | A (0.319) | 18 | 43092162 | 0.026 | 2.62 (1.1,6.39) |
| rs1560904 | 0.0032 | A/G | G (0.319) | 18 | 43093325 | 0.026 | 2.62 (1.1,6.39) |
| rs7220293 | 0.0032 | A/C | C (0.2524) | 17 | 49015851 | 0.039 | 2.75 (1.02,7.72) |
| rs2852474 | 0.0032 | A/G | A (0.2429) | 12 | 50947079 | 0.08 | 2.21 (0.85,5.88) |
| rs12942722 | 0.0032 | A/G | A (0.7184) | 17 | 51461536 | 0.016 | 3.19 (1.15,9.92) |
| rs3745038 | 0.0032 | A/G | G (0.2667) | 18 | 52634144 | 0.033 | 2.63 (1.05,6.79) |
| rs7552342 | 0.0032 | A/G | A (0.2476) | 1 | 54533450 | 0.039 | 2.75 (1.02,7.72) |
| rs26979 | 0.0032 | A/G | A (0.3429) | 5 | 57695990 | 0.025 | 2.61 (1.09,6.43) |
| rs399282 | 0.0032 | A/G | A (0.3429) | 5 | 57710235 | 0.025 | 2.61 (1.09,6.43) |
| rs12046441 | 0.0032 | A/C | C (0.1952) | 1 | 59637687 | 0.11 | 2.23 (0.83,6.12) |
| rs6784508 | 0.0032 | A/G | A (0.4286) | 3 | 61274670 | 0.032 | 2.5 (1.07,5.98) |
| rs1373698 | 0.0032 | A/G | A (0.8095) | 15 | 66592483 | 0.018 | 4.52 (1.16,26.01) |
| rs6566078 | 0.0032 | A/C | C (0.6422) | 18 | 71171843 | 0.029 | 2.52 (1.05,6.31) |
| rs4272572 | 0.0032 | A/G | G (0.4429) | 1 | 71273105 | 0.032 | 2.42 (1.02,5.92) |
| rs2817851 | 0.0032 | A/G | G (0.4429) | 1 | 71315724 | 0.032 | 2.42 (1.02,5.92) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs1864605 | 0.0032 | A/G | A (0.8905) | 8 | 77282562 | 0.02 | Inf (1.36,Inf) |
| rs2075013 | 0.0032 | A/G | A (0.5286) | 7 | 77618107 | 0.01 | 2.95 (1.26,7.12) |
| rs4703849 | 0.0032 | A/G | G (0.4857) | 5 | 81142712 | 0.0058 | 3.25 (1.36,8.13) |
| rs9934411 | 0.0032 | A/G | A (0.6762) | 16 | 81429793 | 0.022 | 2.94 (1.14,8.16) |
| rs2652735 | 0.0032 | A/G | G (0.5149) | 5 | 89448920 | 0.0016 | 3.79 (1.58,9.41) |
| rs4743039 | 0.0032 | A/G | G (0.5857) | 9 | 106779102 | 0.02 | 2.58 (1.09,6.32) |
| rs10978713 | 0.0032 | A/G | G (0.081) | 9 | 106919045 | 0.075 | 6.58 (0.83,301.83) |
| rs7537000 | 0.0032 | A/G | G (0.2238) | 1 | 108088344 | 0.031 | 2.92 (1.01,9) |
| rs7701590 | 0.0032 | A/C | C (0.9238) | 5 | 109018045 | 0.0044 | 12.58 (1.58,577.21) |
| rs1438663 | 0.0032 | A/G | A (0.1524) | 5 | 109055750 | 0.006 | 5.04 (1.39,23.16) |
| rs3740142 | 0.0032 | A/G | G (0.8095) | 10 | 114162758 | 0.011 | 3.86 (1.25,14.43) |
| rs476484 | 0.0032 | A/C | C (0.2619) | 10 | 115056869 | 0.033 | 2.63 (1.05,6.79) |
| rs6478300 | 0.0032 | A/C | C (0.7571) | 9 | 117115323 | 0.032 | 2.78 (1.04,8.1) |
| rs1361285 | 0.0032 | A/G | A (0.6143) | 6 | 118407462 | 5.00E-05 | 6.23 (2.3,19.11) |
| rs1433212 | 0.0032 | A/G | G (0.7961) | 4 | 125110579 | 0.014 | 4.23 (1.25,18.68) |
| rs4733581 | 0.0032 | A/G | G (0.2952) | 8 | 129016957 | 0.005 | 3.52 (1.37,9.48) |
| rs6759721 | 0.0032 | A/G | A (0.0962) | 2 | 147621015 | 0.0074 | 5.87 (1.42,35.02) |
| rs1610399 | 0.0032 | A/G | G (0.4853) | 1 | 201365515 | 0.0099 | 2.92 (1.22,7.15) |
| rs12035407 | 0.0032 | A/G | A (0.2333) | 1 | 236704699 | 0.011 | 3.41 (1.25,9.87) |
| rs2395594 | 0.0033 | A/G | G (0.9461) | unknown | 0 | 0.14 | 4 (0.67,42.46) |
| rs9296614 | 0.0033 | A/G | G (0.419) | 6 | 13257478 | 0.31 | 1.61 (0.68,3.83) |
| rs10025001 | 0.0033 | A/G | A (0.6429) | 4 | 14556198 | 0.066 | 2.28 (0.92,5.94) |
| rs10828351 | 0.0033 | A/G | G (0.7905) | 10 | 23180737 | 0.0081 | 4.58 (1.36,20.06) |
| rs1476355 | 0.0033 | A/C | C (0.6869) | 6 | 24086494 | 0.0092 | 3.49 (1.25,10.89) |
| rs17107115 | 0.0033 | A/G | A (0.7053) | 14 | 37334707 | 0.014 | 3.44 (1.22,10.81) |
| rs5750764 | 0.0033 | A/C | C (0.7) | 22 | 37909924 | 0.093 | 2.26 (0.89,6.07) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs10048286 | 0.0033 | A/G | A (0.1905) | 18 | 40288243 | 0.02 | 3.63 (1.15,12.83) |
| rs6002673 | 0.0033 | A/G | A (0.4524) | 22 | 41015794 | 0.011 | 2.93 (1.24,7.21) |
| rs7193955 | 0.0033 | A/G | G (0.2333) | 16 | 46680083 | 0.018 | 3.23 (1.13,9.87) |
| rs4998530 | 0.0033 | A/G | A (0.619) | 4 | 77854935 | 0.0043 | 3.57 (1.39,9.89) |
| rs502708 | 0.0033 | A/G | G (0.2) | 10 | 78649744 | 0.043 | 3.06 (1.01,10.05) |
| rs4753644 | 0.0033 | A/G | A (0.7714) | 11 | 94319471 | 0.0031 | 6.24 (1.66,35.3) |
| rs3019260 | 0.0033 | A/C | A (0.3524) | 8 | 102137980 | 0.11 | 1.91 (0.81,4.55) |
| rs1882640 | 0.0033 | A/G | A (0.219) | 2 | 141694729 | 0.064 | 2.49 (0.92,7.05) |
| rs1337072 | 0.0033 | A/G | G (0.1) | 1 | 158836316 | 0.0083 | 7.17 (1.38,71.92) |
| rs4391668 | 0.0033 | A/C | A (0.6333) | 1 | 205193320 | 0.072 | 2.21 (0.92,5.51) |
| rs3807551 | 0.0034 | A/G | A (0.919) | unknown | 0 | 0.017 | 6.21 (1.16,63.16) |
| rs9644299 | 0.0034 | A/G | G (0.3) | 8 | 6032068 | 0.011 | 3.13 (1.26,8.06) |
| rs3916092 | 0.0034 | A/G | G (0.7571) | 3 | 19921061 | 0.12 | 2.25 (0.83,6.62) |
| rs4436787 | 0.0034 | A/G | G (0.1905) | 16 | 20082234 | 0.039 | 2.75 (1.02,7.72) |
| rs586205 | 0.0034 | A/G | A (0.1048) | 13 | 21263974 | 0.017 | 6.21 (1.16,63.16) |
| rs739259 | 0.0034 | A/G | A (0.6667) | 22 | 25681133 | 0.016 | 2.95 (1.2,7.67) |
| rs933222 | 0.0034 | A/G | A (0.3) | 22 | 35943137 | 0.035 | 2.61 (1.06,6.62) |
| rs867043 | 0.0034 | A/G | A (0.7667) | 15 | 36297219 | 0.0024 | 4.44 (1.55,14.77) |
| rs551658 | 0.0034 | A/C | A (0.2) | 3 | 42999449 | 0.0043 | 4.13 (1.42,13.28) |
| rs2301382 | 0.0034 | A/G | A (0.1238) | 22 | 46213572 | 0.014 | 5.38 (1.27,32.42) |
| rs12159918 | 0.0034 | A/G | G (0.1238) | 22 | 46227133 | 0.014 | 5.38 (1.27,32.42) |
| rs10500285 | 0.0034 | A/G | G (0.8883) | 19 | 47364600 | 0.0066 | 10.63 (1.46,470.67) |
| rs9958194 | 0.0034 | A/G | A (0.6714) | 18 | 55871378 | 0.0075 | 3.35 (1.31,9.28) |
| rs412028 | 0.0034 | A/C | C (0.3398) | 5 | 57709620 | 0.025 | 2.61 (1.09,6.43) |
| rs4912406 | 0.0034 | A/C | C (0.1311) | 1 | 59874312 | 0.25 | 2.06 (0.57,7.85) |
| rs459950 | 0.0034 | A/G | A (0.5667) | 16 | 64379094 | 0.018 | 2.75 (1.16,6.75) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs1559290 | 0.0034 | A/G | G (0.9333) | 16 | 77239087 | 0.0044 | 12.58 (1.58,577.21) |
| rs2220748 | 0.0034 | A/C | C (0.6571) | 12 | 77526736 | 0.0071 | 3.39 (1.28,9.85) |
| rs11597111 | 0.0034 | A/G | G (0.9238) | 10 | 78410654 | 0.13 | 3.62 (0.56,39.69) |
| rs13033073 | 0.0034 | A/G | A (0.5143) | 2 | 107017425 | 0.0029 | 3.4 (1.43,8.35) |
| rs2080652 | 0.0034 | A/G | G (0.1952) | 12 | 114713454 | 0.1 | 2.26 (0.82,6.43) |
| rs2013676 | 0.0034 | A/G | A (0.8714) | 3 | 157294025 | 0.0022 | Inf (2.1,Inf) |
| rs1518186 | 0.0034 | A/G | G (0.2714) | 4 | 168969440 | 0.14 | 2 (0.81,5.02) |
| rs960700 | 0.0034 | A/G | A (0.181) | 4 | 174797743 | 0.035 | 2.82 (1.02,8.26) |
| rs6881402 | 0.0034 | A/G | A (0.9143) | 5 | 177451218 | 0.067 | 4.18 (0.83,41.29) |
| rs4894030 | 0.0034 | A/C | C (0.2619) | 2 | 179286728 | 0.053 | 2.4 (0.95,6.23) |
| rs2066136 | 0.0034 | A/C | A (0.3381) | 1 | 218619110 | 0.053 | 2.4 (0.95,6.23) |
| rs2760743 | 0.0035 | A/C | A (0.2762) | 17 | 1963829 | 0.083 | 2.19 (0.86,5.7) |
| rs923862 | 0.0035 | A/C | C (0.2667) | 17 | 1978132 | 0.083 | 2.19 (0.86,5.7) |
| rs1565763 | 0.0035 | A/G | G (0.2667) | 17 | 2002050 | 0.083 | 2.19 (0.86,5.7) |
| rs9898819 | 0.0035 | A/G | A (0.2762) | 17 | 2023102 | 0.083 | 2.19 (0.86,5.7) |
| rs2239080 | 0.0035 | A/G | A (0.3762) | 12 | 2424950 | 0.01 | 2.86 (1.2,6.98) |
| rs4798443 | 0.0035 | A/G | G (0.1857) | 18 | 6442944 | 0.029 | 3.54 (1.03,14.13) |
| rs206573 | 0.0035 | A/G | A (0.1333) | 18 | 10368228 | 0.069 | 3.49 (0.89,16.63) |
| rs9804873 | 0.0035 | A/G | G (0.1381) | 12 | 22391686 | 0.0018 | 9.24 (1.86,90.63) |
| rs1552788 | 0.0035 | A/C | A (0.3029) | 18 | 25528037 | 0.0068 | 3.55 (1.34,9.97) |
| rs7364180 | 0.0035 | A/G | G (0.2427) | 22 | 40543356 | 0.0015 | 4.5 (1.63,13.55) |
| rs1297371 | 0.0035 | A/G | A (0.5381) | 22 | 46231512 | 0.0034 | 3.29 (1.4,7.99) |
| rs12537160 | 0.0035 | A/G | A (0.5667) | 7 | 51508036 | 0.02 | 2.56 (1.1,6.1) |
| rs8111906 | 0.0035 | A/G | G (0.881) | 19 | 56586550 | 0.022 | 5.76 (1.2,55.34) |
| rs4718716 | 0.0035 | A/G | A (0.2) | 7 | 67101057 | 0.011 | 3.41 (1.25,9.87) |
| rs9293946 | 0.0035 | A/G | A (0.8814) | 6 | 74741703 | 0.17 | 2.46 (0.67,11.32) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs527589 | 0.0035 | A/G | G (0.1202) | 11 | 76832287 | 0.0092 | 6.93 (1.33,69.55) |
| rs798311 | 0.0035 | A/G | G (0.7981) | 7 | 77603210 | 0.014 | 3.62 (1.25,12.12) |
| rs7186068 | 0.0035 | A/G | G (0.3) | 16 | 79169299 | 0.0041 | 3.39 (1.38,8.57) |
| rs677911 | 0.0035 | A/G | A (0.6429) | 1 | 85117908 | 0.00028 | 4.99 (1.9,14.45) |
| rs12268765 | 0.0035 | A/G | G (0.0905) | 10 | 86605432 | 0.0046 | Inf (1.84,Inf) |
| rs4851407 | 0.0035 | A/G | G (0.3524) | 2 | 101217700 | 0.0075 | 3.11 (1.27,7.86) |
| rs4917434 | 0.0035 | A/C | A (0.9421) | 10 | 106698287 | 0.017 | 9.84 (1.12,469.41) |
| rs10778641 | 0.0035 | A/C | A (0.5625) | 12 | 107377428 | 0.031 | 2.43 (1.03,5.87) |
| rs2156801 | 0.0035 | A/G | G (0.3077) | 11 | 122159266 | 0.005 | 3.25 (1.36,8.01) |
| rs585166 | 0.0035 | A/G | A (0.1) | 11 | 133436223 | 0.0083 | 7.17 (1.38,71.92) |
| rs7464953 | 0.0035 | A/G | A (0.5238) | 8 | 135988174 | 0.011 | 2.8 (1.2,6.72) |
| rs4691057 | 0.0035 | A/C | A (0.776) | 4 | 156977447 | 0.0082 | 4.68 (1.39,20.58) |
| rs1946814 | 0.0035 | A/G | A (0.3952) | 2 | 178505183 | 0.01 | 2.86 (1.2,6.98) |
| rs4850546 | 0.0035 | A/C | A (0.4381) | 2 | 193271112 | 0.12 | 1.88 (0.81,4.42) |
| rs2882835 | 0.0036 | A/C | C (0.8798) | 7 | 10166541 | 0.0046 | Inf (1.91,Inf) |
| rs2823575 | 0.0036 | A/G | G (0.1048) | 21 | 16330327 | 0.07 | 4.44 (0.75,47.1) |
| rs12326518 | 0.0036 | A/G | G (0.3762) | 18 | 19139126 | 0.041 | 2.39 (0.99,5.9) |
| rs9608859 | 0.0036 | A/G | A (0.3558) | 22 | 28991831 | 0.005 | 3.25 (1.36,8.01) |
| rs2073320 | 0.0036 | A/G | G (0.6333) | 10 | 33593263 | 0.0036 | 3.86 (1.47,11.19) |
| rs1439859 | 0.0036 | A/G | A (0.3802) | 2 | 35355484 | 0.0055 | 3.22 (1.32,8.28) |
| rs7753322 | 0.0036 | A/C | C (0.6893) | 6 | 35856064 | 0.037 | 2.57 (0.99,7.17) |
| rs2643208 | 0.0036 | A/G | A (0.6429) | 15 | 36793090 | 0.0051 | 3.34 (1.33,8.92) |
| rs7001892 | 0.0036 | A/G | G (0.3619) | 8 | 37240117 | 0.04 | 2.39 (0.98,5.97) |
| rs7112307 | 0.0036 | A/G | G (0.8095) | 11 | 60355585 | 0.0016 | 6.72 (1.8,37.91) |
| rs12258285 | 0.0036 | A/G | G (0.3333) | 10 | 66601657 | 0.056 | 2.39 (0.96,6.08) |
| rs12754625 | 0.0036 | A/G | A (0.9135) | 1 | 71699517 | 0.038 | 5.13 (0.91,53.09) |
| rs1346133 | 0.0036 | A/G | A (0.1078) | 4 | 74139752 | 0.021 | 5.8 (1.08,58.91) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs7315214 | 0.0036 | A/G | G (0.4952) | 12 | 77548104 | 0.011 | 2.94 (1.25,7.11) |
| rs3121 | 0.0036 | A/G | G (0.3619) | 12 | 79605389 | 0.024 | 2.61 (1.07,6.51) |
| rs1305270 | 0.0036 | A/G | G (0.3619) | 12 | 79628576 | 0.024 | 2.61 (1.07,6.51) |
| rs1336050 | 0.0036 | A/C | C (0.1429) | 1 | 86400499 | 0.016 | 3.97 (1.18,15.68) |
| rs735027 | 0.0036 | A/G | G (0.3143) | 11 | 94941101 | 0.056 | 2.39 (0.96,6.08) |
| rs4255644 | 0.0036 | A/G | A (0.2381) | 13 | 109887574 | 0.05 | 2.42 (0.94,6.43) |
| rs3809278 | 0.0036 | A/C | C (0.881) | 12 | 110187905 | 0.032 | 4.13 (1.05,23.93) |
| rs2057926 | 0.0036 | A/G | G (0.1538) | 7 | 119486012 | 0.04 | 3.84 (1.01,18.06) |
| rs10787852 | 0.0036 | A/G | G (0.681) | 10 | 120422985 | 0.0056 | 3.72 (1.36,11.45) |
| rs880713 | 0.0036 | A/G | A (0.4857) | 2 | 128847844 | 0.011 | 2.94 (1.25,7.11) |
| rs2327832 | 0.0036 | A/G | A (0.7571) | 6 | 138014761 | 0.015 | 3.38 (1.16,11.33) |
| rs1572495 | 0.0036 | A/G | A (0.0952) | 1 | 158830959 | 0.0083 | 7.17 (1.38,71.92) |
| rs7744302 | 0.0036 | A/C | A (0.8221) | 6 | 164029507 | 0.052 | 3.07 (0.97,11.61) |
| rs2411385 | 0.0036 | A/G | G (0.3144) | 5 | 178827315 | 0.0079 | 3.83 (1.28,12.57) |
| rs1447323 | 0.0036 | A/G | A (0.6048) | 4 | 181527703 | 0.031 | 2.49 (1.06,6.02) |
| rs4972964 | 0.0036 | A/C | C (0.5) | 2 | 231302728 | 0.00073 | 4.06 (1.7,10.08) |
| rs3750417 | 0.0037 | A/G | A (0.1971) | 9 | 2044409 | 0.16 | 2.03 (0.73,5.85) |
| rs650895 | 0.0037 | A/G | A (0.0952) | 18 | 4366873 | 0.0096 | Inf (1.59,Inf) |
| rs9808246 | 0.0037 | A/G | A (0.3786) | 2 | 4925409 | 0.072 | 2.14 (0.91,5.15) |
| rs1615810 | 0.0037 | A/G | A (0.2524) | 8 | 5555216 | 0.19 | 1.82 (0.72,4.68) |
| rs2438481 | 0.0037 | A/G | A (0.2549) | 5 | 5649868 | 0.019 | 2.79 (1.09,7.36) |
| rs1149986 | 0.0037 | A/C | C (0.9143) | 10 | 9389527 | 0.13 | 3.62 (0.56,39.69) |
| rs6109781 | 0.0037 | A/C | C (0.115) | 20 | 13181011 | 0.00047 | 17.5 (2.36,781.11) |
| rs3846121 | 0.0037 | A/C | C (0.2381) | 3 | 14653303 | 0.015 | 3.4 (1.14,11.05) |
| rs6079891 | 0.0037 | A/G | G (0.6333) | 20 | 15510149 | 0.017 | 2.77 (1.12,7.19) |
| rs7907660 | 0.0037 | A/G | A (0.5571) | 10 | 16045847 | 0.011 | 2.8 (1.2,6.72) |
| rs1922124 | 0.0037 | A/G | A (0.4238) | 10 | 23246452 | 0.051 | 2.26 (0.97,5.41) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs11586125 | 0.0037 | A/G | G (0.2333) | 1 | 24122632 | 0.0027 | 4.28 (1.54,12.89) |
| rs7540557 | 0.0037 | A/G | G (0.2333) | 1 | 24123250 | 0.0027 | 4.28 (1.54,12.89) |
| rs7665027 | 0.0037 | A/G | G (0.1143) | 4 | 26163178 | 0.09 | 2.75 (0.76,11.29) |
| rs7541937 | 0.0037 | A/C | A (0.4952) | 1 | 35011075 | 0.0066 | 3.03 (1.28,7.38) |
| rs848596 | 0.0037 | A/G | A (0.5143) | 2 | 36715514 | 0.081 | 2.04 (0.88,4.81) |
| rs876960 | 0.0037 | A/G | G (0.3857) | 2 | 45228919 | 0.16 | 1.89 (0.8,4.53) |
| rs10486757 | 0.0037 | A/G | A (0.0952) | 7 | 50468067 | 0.0083 | 7.17 (1.38,71.92) |
| rs512716 | 0.0037 | A/C | C (0.1422) | 11 | 60333647 | 0.016 | 3.92 (1.15,15.57) |
| rs634392 | 0.0037 | A/G | A (0.9238) | 18 | 68367025 | 0.0049 | Inf (1.7,Inf) |
| rs943001 | 0.0037 | A/G | G (0.6538) | 13 | 70578522 | 0.0011 | 4.33 (1.69,12.06) |
| rs1107768 | 0.0037 | A/G | G (0.8) | 3 | 73959415 | 0.019 | 3.58 (1.15,13.44) |
| rs2276029 | 0.0037 | A/G | G (0.7475) | 11 | 76473807 | 0.052 | 2.49 (0.94,7.08) |
| rs1401838 | 0.0037 | A/G | G (0.3333) | 2 | 76544795 | 0.069 | 2.22 (0.94,5.37) |
| rs790381 | 0.0037 | A/G | A (0.2095) | 11 | 83312631 | 0.00082 | 5.38 (1.79,18.55) |
| rs4405983 | 0.0037 | A/G | G (0.6143) | 4 | 88743948 | 0.0058 | 3.19 (1.32,8.1) |
| rs742893 | 0.0037 | A/G | A (0.524) | 14 | 94222866 | 0.019 | 2.61 (1.12,6.24) |
| rs854542 | 0.0037 | A/G | A (0.7864) | 7 | 94566142 | 0.037 | 3.06 (1.04,10.35) |
| rs1939470 | 0.0037 | A/G | G (0.0667) | 11 | 95485565 | 0.02 | Inf (1.36,Inf) |
| rs4576532 | 0.0037 | A/G | A (0.4238) | 9 | 97359566 | 0.01 | 2.91 (1.24,7.03) |
| rs947950 | 0.0037 | A/G | A (0.5388) | 11 | 99913200 | 0.003 | 3.48 (1.47,8.49) |
| rs2850437 | 0.0037 | A/G | A (0.7667) | 11 | 101851298 | 0.0063 | 4.15 (1.35,15.46) |
| rs698030 | 0.0037 | A/G | A (0.5286) | 6 | 106781543 | 0.011 | 2.86 (1.22,6.89) |
| rs597443 | 0.0037 | A/G | G (0.5286) | 6 | 106876335 | 0.011 | 2.86 (1.22,6.89) |
| rs9846784 | 0.0037 | A/G | G (0.4429) | 3 | 140127711 | 0.0084 | 3.1 (1.29,7.66) |
| rs4648505 | 0.0038 | A/G | G (0.1286) | 1 | 3428453 | 0.039 | 3.97 (1.05,18.68) |
| rs2963363 | 0.0038 | A/G | A (0.2908) | 5 | 8981893 | 0.00021 | 6.29 (2.16,20.42) |
| rs4748234 | 0.0038 | A/G | G (0.5625) | 10 | 16069516 | 0.012 | 2.75 (1.18,6.58) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs549485 | 0.0038 | A/G | A (0.3429) | 11 | 17880340 | 0.024 | 2.61 (1.07,6.51) |
| rs2826505 | 0.0038 | A/G | G (0.7429) | 21 | 21059640 | 0.0013 | 5.69 (1.72,24.72) |
| rs10757193 | 0.0038 | A/G | G (0.5245) | 9 | 21147373 | 0.01 | 2.9 (1.21,7.14) |
| rs7656884 | 0.0038 | A/G | G (0.9238) | 4 | 23834298 | 0.17 | 2.92 (0.58,19.09) |
| rs2760139 | 0.0038 | A/C | A (0.4619) | 6 | 24589278 | 0.011 | 2.93 (1.24,7.21) |
| rs9467445 | 0.0038 | A/G | G (0.1857) | 6 | 25342863 | 0.029 | 3.54 (1.03,14.13) |
| rs2466062 | 0.0038 | A/G | A (0.7) | 8 | 32562632 | 0.057 | 2.41 (0.92,6.72) |
| rs7152432 | 0.0038 | A/G | A (0.6381) | 14 | 52936440 | 0.015 | 2.93 (1.17,7.85) |
| rs7326182 | 0.0038 | A/G | A (0.3429) | 13 | 53023625 | 0.13 | 2 (0.78,5.22) |
| rs1000380 | 0.0038 | A/G | G (0.1571) | 5 | 56921946 | 0.052 | 3.13 (0.89,12.67) |
| rs12532549 | 0.0038 | A/G | A (0.9333) | 7 | 67332047 | 0.04 | 8.98 (1.03,426.65) |
| rs2866277 | 0.0038 | A/G | A (0.2981) | 7 | 69204484 | 0.01 | 3.17 (1.25,8.33) |
| rs6564372 | 0.0038 | A/G | A (0.5238) | 16 | 71881575 | 0.077 | 2.15 (0.93,5.08) |
| rs10869554 | 0.0038 | A/G | A (0.15) | 9 | 75234809 | 0.038 | 3.14 (0.98,11.21) |
| rs526260 | 0.0038 | A/G | G (0.4381) | 18 | 75654161 | 0.031 | 2.49 (1.06,6.02) |
| rs1346452 | 0.0038 | A/G | G (0.6095) | 5 | 88422883 | 0.0029 | 3.58 (1.46,9.3) |
| rs7728690 | 0.0038 | A/G | G (0.6095) | 5 | 88446970 | 0.0029 | 3.58 (1.46,9.3) |
| rs1326848 | 0.0038 | A/G | A (0.125) | 10 | 92033133 | 0.014 | 5.38 (1.27,32.42) |
| rs3802195 | 0.0038 | A/C | A (0.1429) | 8 | 99209384 | 0.0036 | 6.82 (1.68,40.27) |
| rs4329039 | 0.0038 | A/G | G (0.5905) | 5 | 117821408 | 0.0066 | 3.05 (1.28,7.61) |
| rs17811014 | 0.0038 | A/G | G (0.781) | 3 | 161170731 | 0.073 | 2.44 (0.87,7.65) |
| rs30084 | 0.0038 | A/C | A (0.4333) | 5 | 169208420 | 0.048 | 2.27 (0.97,5.42) |
| rs1979570 | 0.0038 | A/G | A (0.2238) | 4 | 188483173 | 0.1 | 2.26 (0.82,6.43) |
| rs2685230 | 0.0039 | A/G | G (0.3606) | 2 | 427664 | 0.014 | 2.84 (1.17,7.09) |
| rs576567 | 0.0039 | A/G | A (0.5857) | 12 | 5164157 | 0.017 | 2.77 (1.12,7.19) |
| rs957610 | 0.0039 | A/C | A (0.1286) | 6 | 10207002 | 0.014 | 5.38 (1.27,32.42) |
| rs11605276 | 0.0039 | A/G | G (0.7885) | 11 | 13610087 | 0.0045 | 4.93 (1.48,21.54) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs1566066 | 0.0039 | A/G | G (0.1429) | 4 | 16326359 | 0.1 | 2.57 (0.77,9.39) |
| rs4922151 | 0.0039 | A/G | A (0.4286) | 8 | 20185372 | 0.048 | 2.27 (0.97,5.42) |
| rs10965002 | 0.0039 | A/G | A (0.9095) | 9 | 21445814 | 0.094 | 3.49 (0.74,22.16) |
| rs4269167 | 0.0039 | A/G | A (0.3762) | 4 | 25702025 | 0.24 | 1.69 (0.73,3.95) |
| rs1433840 | 0.0039 | A/G | G (0.2333) | 18 | 36255130 | 0.024 | 3.02 (1.13,8.43) |
| rs9631189 | 0.0039 | A/G | A (0.2667) | 21 | 36391146 | 0.035 | 2.82 (1.02,8.26) |
| rs10456444 | 0.0039 | A/G | G (0.5143) | 6 | 36830820 | 0.019 | 2.68 (1.15,6.41) |
| rs348560 | 0.0039 | A/G | G (0.8238) | 5 | 40348617 | 0.01 | 4.92 (1.28,28.16) |
| rs11082569 | 0.0039 | A/G | A (0.1587) | 18 | 43115969 | 0.011 | 3.86 (1.22,13.69) |
| rs603560 | 0.0039 | A/G | A (0.681) | 1 | 43333212 | 0.0075 | 3.35 (1.31,9.28) |
| rs2214979 | 0.0039 | A/G | A (0.524) | 17 | 50649284 | 0.011 | 2.86 (1.21,7.01) |
| rs11594325 | 0.0039 | A/G | A (0.5381) | 10 | 53152740 | 0.076 | 2.11 (0.91,5.01) |
| rs2406284 | 0.0039 | A/G | A (0.1952) | 1 | 57981063 | 0.094 | 2.3 (0.81,6.84) |
| rs11126387 | 0.0039 | A/G | A (0.4712) | 2 | 73079599 | 0.01 | 2.96 (1.24,7.32) |
| rs639525 | 0.0039 | A/G | A (0.8238) | 11 | 73782753 | 0.032 | 3.32 (1.06,12.5) |
| rs7170624 | 0.0039 | A/C | A (0.2356) | 15 | 86002046 | 0.11 | 2.23 (0.83,6.12) |
| rs13239875 | 0.0039 | A/G | A (0.3155) | 7 | 91360755 | 0.0075 | 3.1 (1.26,7.81) |
| rs4766567 | 0.0039 | A/G | G (0.8429) | 12 | 110170276 | 0.025 | 3.92 (1.15,17.32) |
| rs307533 | 0.0039 | A/G | A (0.3048) | 5 | 110784862 | 0.018 | 2.9 (1.14,7.65) |
| rs11195940 | 0.0039 | A/G | G (0.4554) | 10 | 114137024 | 0.033 | 2.37 (1.01,5.68) |
| rs1407184 | 0.0039 | A/G | A (0.4) | 6 | 117840410 | 0.025 | 2.61 (1.09,6.43) |
| rs10782186 | 0.0039 | A/G | G (0.5) | 6 | 117930201 | 0.03 | 2.52 (1.08,6) |
| rs4937216 | 0.0039 | A/G | G (0.6714) | 11 | 126273300 | 0.00083 | 4.82 (1.77,14.8) |
| rs7805545 | 0.0039 | A/G | G (0.8429) | 7 | 129203632 | 0.012 | 6.33 (1.34,60.52) |
| rs17049735 | 0.0039 | A/G | G (0.8095) | 4 | 138933608 | 0.015 | 3.38 (1.16,11.33) |
| rs4869943 | 0.0039 | A/G | A (0.1538) | 6 | 151159719 | 0.027 | 4.71 (1.09,28.83) |
| rs10863715 | 0.0039 | A/G | A (0.7238) | 1 | 205101560 | 0.012 | 3.18 (1.2,9.23) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs10848863 | 0.004 | A/G | G (0.7143) | 12 | 3432071 | 0.016 | 3.25 (1.18,10.05) |
| rs1884751 | 0.004 | A/G | G (0.3702) | 20 | 5345328 | 0.011 | 3.04 (1.2,8.02) |
| rs2274027 | 0.004 | A/G | G (0.181) | 10 | 6000505 | 0.12 | 2.45 (0.78,8.21) |
| rs1105004 | 0.004 | A/G | G (0.4333) | 16 | 6912217 | 0.011 | 2.93 (1.24,7.21) |
| rs2098839 | 0.004 | A/G | G (0.6857) | 11 | 10633563 | 0.071 | 2.17 (0.89,5.51) |
| rs4669835 | 0.004 | A/G | A (0.2714) | 2 | 12323221 | 0.08 | 2.21 (0.85,5.88) |
| rs10505879 | 0.004 | A/C | C (0.1905) | 12 | 22539123 | 0.016 | 3.97 (1.18,15.68) |
| rs3811180 | 0.004 | A/G | A (0.2619) | 14 | 22803565 | 0.056 | 2.39 (0.96,6.08) |
| rs3746731 | 0.004 | A/G | A (0.4596) | 20 | 23013209 | 0.027 | 2.52 (1.05,6.19) |
| rs4964005 | 0.004 | A/G | A (0.2788) | 12 | 26660000 | 0.045 | 2.59 (0.99,7.04) |
| rs315492 | 0.004 | A/G | A (0.1476) | 17 | 27059794 | 0.027 | 4.71 (1.09,28.83) |
| rs2032319 | 0.004 | A/G | A (0.3095) | 21 | 36295592 | 0.028 | 2.69 (1.03,7.31) |
| rs2835228 | 0.004 | A/G | G (0.3095) | 21 | 36297513 | 0.028 | 2.69 (1.03,7.31) |
| rs9979193 | 0.004 | A/G | A (0.6524) | 21 | 43219454 | 0.047 | 2.32 (0.95,5.88) |
| rs11913072 | 0.004 | A/G | G (0.2115) | 22 | 43340885 | 0.11 | 2.14 (0.8,5.89) |
| rs136094 | 0.004 | A/G | G (0.7356) | 22 | 45678886 | 0.079 | 2.45 (0.91,7.21) |
| rs935672 | 0.004 | A/G | A (0.4231) | 2 | 45957610 | 0.041 | 2.39 (0.99,5.9) |
| rs9473239 | 0.004 | A/G | G (0.149) | 6 | 48171647 | 0.032 | 3.42 (0.99,13.65) |
| rs8046199 | 0.004 | A/G | G (0.7327) | 16 | 49570048 | 0.016 | 3.19 (1.14,9.99) |
| rs1947558 | 0.004 | A/G | G (0.8039) | 5 | 67974317 | 0.051 | 2.95 (0.93,11.26) |
| rs1568828 | 0.004 | A/G | G (0.519) | 8 | 69284682 | 0.033 | 2.41 (1.03,5.78) |
| rs4852918 | 0.004 | A/G | A (0.6298) | 2 | 69827026 | 0.0045 | 3.58 (1.43,9.62) |
| rs11117001 | 0.004 | A/G | A (0.8) | 12 | 75979968 | 0.062 | 2.7 (0.91,9.17) |
| rs1457958 | 0.004 | A/G | G (0.3365) | 6 | 77460260 | 0.014 | 2.84 (1.17,7.09) |
| rs1390747 | 0.004 | A/G | A (0.3619) | 4 | 79808608 | 0.15 | 1.87 (0.78,4.52) |
| rs4975139 | 0.004 | A/G | A (0.5619) | 4 | 79812912 | 0.033 | 2.34 (1.01,5.56) |
| rs7185078 | 0.004 | A/G | A (0.3429) | 16 | 82790849 | 0.13 | 2 (0.79,5.11) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2148322 | 0.004 | A/C | A (0.2429) | 1 | 91834353 | 0.0063 | 3.74 (1.38,10.77) |
| rs733830 | 0.004 | A/G | A (0.319) | 2 | 101236837 | 0.01 | 3.17 (1.25,8.33) |
| rs751837 | 0.004 | A/G | G (0.1238) | 14 | 102554578 | 0.0038 | 8.18 (1.61,80.71) |
| rs3741639 | 0.004 | A/G | G (0.2333) | 12 | 126284147 | 0.0063 | 3.74 (1.38,10.77) |
| rs3829849 | 0.004 | A/G | A (0.3286) | 9 | 126470354 | 0.014 | 2.84 (1.17,7.09) |
| rs1519369 | 0.004 | A/G | A (0.3286) | 8 | 139463091 | 0.024 | 2.61 (1.07,6.51) |
| rs9307869 | 0.004 | A/G | G (0.2143) | 4 | 140068847 | 0.064 | 2.49 (0.92,7.05) |
| rs1856416 | 0.004 | A/G | A (0.2048) | 6 | 145328061 | 0.0017 | 4.9 (1.61,16.97) |
| rs1013192 | 0.004 | A/G | A (0.5048) | 5 | 169131681 | 0.032 | 2.5 (1.07,5.98) |
| rs983560 | 0.004 | A/G | A (0.4327) | 4 | 173321017 | 0.051 | 2.19 (0.94,5.23) |
| rs10520270 | 0.004 | A/G | A (0.1429) | 4 | 175421715 | 0.035 | 3.26 (1.02,11,61) |
| rs10520376 | 0.004 | A/G | A (0.9048) | 4 | 178578612 | 0.00074 | Inf (2.58,Inf) |
| rs2871133 | 0.004 | A/G | G (0.4762) | 4 | 181257259 | 0.033 | 2.41 (1.03,5.78) |
| rs7554115 | 0.004 | A/G | A (0.3077) | 1 | 218490977 | 0.078 | 2.33 (0.9,6.18) |
| rs11777063 | 0.0041 | A/G | G (0.3413) | 8 | 877909 | 0.013 | 2.98 (1.21,7.56) |
| rs10088247 | 0.0041 | A/G | A (0.7143) | 8 | 3671607 | 0.014 | 3.62 (1.25,12.12) |
| rs699549 | 0.0041 | A/G | A (0.2048) | 2 | 4198632 | 0.064 | 2.49 (0.92,7.05) |
| rs10772649 | 0.0041 | A/C | A (0.2048) | 12 | 13346982 | 0.015 | 3.4 (1.14,11.05) |
| rs1204250 | 0.0041 | A/G | G (0.6048) | 6 | 13985102 | 0.028 | 2.63 (1.09,6.68) |
| rs4814389 | 0.0041 | A/G | G (0.1762) | 20 | 15426379 | 0.0041 | 4.91 (1.5,19.08) |
| rs2106949 | 0.0041 | A/G | G (0.2762) | 16 | 18032061 | 0.0034 | 4.09 (1.52,11.75) |
| rs4072410 | 0.0041 | A/G | A (0.381) | 3 | 23664205 | 0.03 | 2.45 (1.04,5.88) |
| rs1434514 | 0.0041 | A/G | G (0.3053) | 18 | 43096405 | 0.017 | 2.96 (1.16,7.82) |
| rs11037452 | 0.0041 | A/G | A (0.6143) | 11 | 43471106 | 0.032 | 2.42 (1.02,5.92) |
| rs10507657 | 0.0041 | A/G | G (0.2) | 13 | 60206691 | 0.015 | 3.4 (1.14,11.05) |
| rs13379257 | 0.0041 | A/G | A (0.5524) | 14 | 62729878 | 0.081 | 2.04 (0.88,4.81) |
| rs3093273 | 0.0041 | A/G | G (0.9087) | 7 | 76473926 | 0.01 | 10.74 (1.3,500.03) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs10504616 | 0.0041 | A/G | A (0.081) | 8 | 77380620 | 0.02 | Inf (1.13,Inf) |
| rs10090760 | 0.0041 | A/G | A (0.3173) | 8 | 82320582 | 0.015 | 2.85 (1.19,7.01) |
| rs6563468 | 0.0041 | A/G | G (0.6287) | 13 | 85456901 | 0.16 | 1.91 (0.8,4.67) |
| rs6419391 | 0.0041 | A/C | A (0.38) | 12 | 104174374 | 0.069 | 2.21 (0.92,5.49) |
| rs260218 | 0.0041 | A/G | G (0.2048) | 9 | 109892930 | 0.043 | 3.06 (1.01,10.05) |
| rs27499 | 0.0041 | A/G | G (0.9118) | 5 | 115664875 | 0.094 | 3.49 (0.74,22.16) |
| rs7974836 | 0.0041 | A/G | G (0.524) | 12 | 116619449 | 0.12 | 1.87 (0.8,4.43) |
| rs7296549 | 0.0041 | A/G | G (0.5476) | 12 | 125455447 | 0.034 | 2.34 (1.01,5.58) |
| rs758564 | 0.0041 | A/G | G (0.5476) | 12 | 125457504 | 0.034 | 2.34 (1.01,5.58) |
| rs7085947 | 0.0041 | A/G | A (0.1942) | 10 | 130944851 | 0.014 | 4.21 (1.24,16.7) |
| rs17729858 | 0.0041 | A/G | A (0.0769) | 8 | 134743421 | 0.4 | 2.74 (0.37,31.56) |
| rs6749771 | 0.0041 | A/G | G (0.581) | 2 | 176895633 | 2.00E-04 | 4.6 (1.92,11.55) |
| rs2387396 | 0.0042 | A/G | A (0.5476) | 10 | 6400299 | 0.011 | 2.93 (1.24,7.21) |
| rs6912599 | 0.0042 | A/G | G (0.6952) | 6 | 9936504 | 0.032 | 2.78 (1.04,8.1) |
| rs6081242 | 0.0042 | A/G | A (0.7905) | 20 | 18537745 | 0.01 | 4.92 (1.28,28.16) |
| rs776776 | 0.0042 | A/G | A (0.5571) | 9 | 18647558 | 0.0029 | 3.4 (1.43,8.35) |
| rs680548 | 0.0042 | A/G | A (0.404) | 22 | 19290109 | 0.066 | 2.21 (0.92,5.41) |
| rs9465361 | 0.0042 | A/C | C (0.9048) | 6 | 19472965 | 0.027 | 4.71 (1.09,28.83) |
| rs4747449 | 0.0042 | A/C | C (0.4216) | 10 | 23239537 | 0.048 | 2.29 (0.97,5.55) |
| rs4564422 | 0.0042 | A/G | A (0.6) | 12 | 25308685 | 0.0014 | 4.04 (1.62,10.81) |
| rs4931019 | 0.0042 | A/G | A (0.1476) | 12 | 32637598 | 0.012 | 4.49 (1.21,20.85) |
| rs2009168 | 0.0042 | A/G | G (0.3762) | 22 | 34442354 | 0.025 | 2.61 (1.09,6.43) |
| rs2504246 | 0.0042 | A/G | A (0.0631) | 10 | 35225502 | 0.02 | Inf (1.36,Inf) |
| rs12184386 | 0.0042 | A/G | A (0.4476) | 10 | 35363136 | 0.0019 | 3.57 (1.5,8.86) |
| rs5767685 | 0.0042 | A/G | G (0.9238) | 22 | 46039196 | 0.13 | 3.62 (0.56,39.69) |
| rs1787114 | 0.0042 | A/C | A (0.3235) | 18 | 46734236 | 0.19 | 1.87 (0.73,4.84) |
| rs2347990 | 0.0042 | A/G | A (0.4905) | 7 | 47463638 | 0.12 | 1.93 (0.82,4.65) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs1012624 | 0.0042 | A/C | A (0.6952) | 6 | 53926221 | 0.093 | 2.26 (0.89,6.07) |
| rs1929753 | 0.0042 | A/G | A (0.1333) | 13 | 75709051 | 0.014 | 5.38 (1.27,32.42) |
| rs10105423 | 0.0042 | A/C | C (0.3476) | 8 | 139422897 | 0.041 | 2.39 (0.99,5.9) |
| rs2964243 | 0.0042 | A/G | G (0.5095) | 5 | 151902066 | 0.08 | 2.09 (0.9,4.92) |
| rs4657378 | 0.0042 | A/C | A (0.5524) | 1 | 161531469 | 0.044 | 2.47 (1.02,6.26) |
| rs11740641 | 0.0042 | A/C | A (0.1373) | 5 | 174100229 | 0.1 | 3.25 (0.69,20.7) |
| rs13084517 | 0.0042 | A/G | G (0.4571) | 3 | 175802374 | 0.12 | 1.93 (0.83,4.57) |
| rs2332838 | 0.0042 | A/G | A (0.2905) | 4 | 175901420 | 0.002 | 3.72 (1.5,9.58) |
| rs6882940 | 0.0042 | A/G | A (0.2981) | 5 | 179581595 | 0.0065 | 3.27 (1.32,8.45) |
| rs6714155 | 0.0042 | A/G | G (0.081) | 2 | 197649322 | 0.023 | 8.53 (1.13,383.04) |
| rs12096737 | 0.0042 | A/C | A (0.2048) | 1 | 222583289 | 0.035 | 2.82 (1.02,8.26) |
| rs1424356 | 0.0042 | A/G | A (0.1619) | 2 | 222783421 | 0.2 | 2.11 (0.69,6.69) |
| rs2111168 | 0.0043 | A/G | G (0.4286) | 12 | 5655745 | 0.12 | 1.87 (0.81,4.39) |
| rs2040666 | 0.0043 | A/G | G (0.3048) | 7 | 27741433 | 0.013 | 2.85 (1.16,7.22) |
| rs904549 | 0.0043 | A/G | A (0.2619) | 11 | 36058508 | 0.033 | 2.63 (1.05,6.79) |
| rs1022367 | 0.0043 | A/G | G (0.281) | 11 | 36716901 | 0.04 | 2.39 (0.98,5.97) |
| rs7240984 | 0.0043 | A/G | A (0.3317) | 18 | 37655531 | 0.014 | 2.97 (1.22,7.42) |
| rs956092 | 0.0043 | A/G | A (0.9048) | 11 | 44365738 | 0.0096 | Inf (1.59,Inf) |
| rs1003854 | 0.0043 | A/G | A (0.7286) | 21 | 44534535 | 0.00048 | 5.83 (1.93,21.5) |
| rs6493270 | 0.0043 | A/G | A (0.8667) | 15 | 45400695 | 0.012 | 6.33 (1.34,60.52) |
| rs1056995 | 0.0043 | A/G | G (0.8125) | 19 | 47283324 | 0.018 | 4.52 (1.16,26.01) |
| rs2367537 | 0.0043 | A/G | A (0.5714) | 17 | 66977481 | 0.0033 | 3.4 (1.4,8.65) |
| rs7986958 | 0.0043 | A/G | A (0.4333) | 13 | 69832617 | 0.049 | 2.31 (0.99,5.48) |
| rs803815 | 0.0043 | A/G | G (0.5238) | 13 | 70434610 | 0.033 | 2.34 (1.01,5.56) |
| rs3743990 | 0.0043 | A/G | A (0.9429) | 17 | 71102780 | 0.0019 | Inf (2.13,Inf) |
| rs7918564 | 0.0043 | A/G | G (0.5095) | 10 | 78571569 | 0.24 | 1.64 (0.71,3.82) |
| rs12340476 | 0.0043 | A/C | A (0.2476) | 9 | 82769783 | 0.08 | 2.21 (0.85,5.88) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs1743152 | 0.0043 | A/C | A (0.351) | 14 | 91148761 | 0.002 | 3.72 (1.5,9.58) |
| rs3792119 | 0.0043 | A/G | A (0.1429) | 2 | 99630599 | 0.0081 | 4.43 (1.33,17.33) |
| rs12425351 | 0.0043 | A/G | G (0.7952) | 12 | 112896572 | 0.024 | 3.14 (1.08,10.57) |
| rs6585465 | 0.0043 | A/G | G (0.3714) | 10 | 119610549 | 0.026 | 2.62 (1.1,6.39) |
| rs9828272 | 0.0043 | A/G | A (0.1571) | 3 | 165194199 | 0.015 | 3.4 (1.14,11.05) |
| rs4465021 | 0.0044 | A/G | A (0.3095) | 9 | 6837072 | 0.1 | 2.02 (0.84,4.94) |
| rs942531 | 0.0044 | A/G | A (0.6635) | 10 | 11888195 | 0.061 | 2.41 (0.95,6.48) |
| rs495441 | 0.0044 | A/G | G (0.7048) | 6 | 24096306 | 0.057 | 2.41 (0.92,6.72) |
| rs6923761 | 0.0044 | A/G | G (0.6762) | 6 | 39142050 | 0.0056 | 3.72 (1.36,11.45) |
| rs7937734 | 0.0044 | A/G | G (0.9109) | 11 | 42268062 | 0.017 | 9.96 (1.14,473.91) |
| rs7935663 | 0.0044 | A/G | G (0.8333) | 11 | 44648055 | 0.2 | 2.16 (0.65,8.41) |
| rs7307330 | 0.0044 | A/G | A (0.625) | 12 | 58492811 | 0.0087 | 3.13 (1.25,8.37) |
| rs10510809 | 0.0044 | A/G | A (0.9143) | 3 | 59414093 | 0.035 | 5.3 (0.94,54.86) |
| rs2366700 | 0.0044 | A/C | A (0.9143) | 3 | 59418462 | 0.035 | 5.3 (0.94,54.86) |
| rs7576017 | 0.0044 | A/G | G (0.6667) | 2 | 60071183 | 0.066 | 2.28 (0.92,5.94) |
| rs10455586 | 0.0044 | A/G | G (0.3714) | 6 | 66628219 | 0.32 | 1.5 (0.64,3.54) |
| rs2376734 | 0.0044 | A/G | A (0.1762) | 9 | 75206254 | 0.053 | 2.63 (0.9,8.18) |
| rs11714671 | 0.0044 | A/G | G (0.8558) | 3 | 79952602 | 0.11 | 3.84 (0.74,38.39) |
| rs2259391 | 0.0044 | A/G | A (0.4476) | 15 | 85831437 | 0.051 | 2.26 (0.97,5.41) |
| rs867744 | 0.0044 | A/G | G (0.8667) | 6 | 85878413 | 0.0064 | 6.92 (1.48,65.88) |
| rs2279577 | 0.0044 | A/G | G (0.4) | 12 | 91004311 | 0.03 | 2.45 (1.04,5.88) |
| rs10778656 | 0.0044 | A/G | G (0.8381) | 12 | 107636054 | 0.026 | 3.61 (1.05,16.04) |
| rs839549 | 0.0044 | A/C | A (0.6524) | 1 | 109195809 | 0.016 | 2.95 (1.2,7.67) |
| rs11742151 | 0.0044 | A/G | G (0.9381) | 5 | 118442692 | 0.04 | 8.98 (1.03,426.65) |
| rs1107811 | 0.0044 | A/G | G (0.7067) | 9 | 121405095 | 0.088 | 2.2 (0.83,6.19) |
| rs4001038 | 0.0044 | A/G | A (0.774) | 4 | 123243845 | 0.0045 | 4.93 (1.48,21.54) |
| rs2146204 | 0.0044 | A/C | A (0.8654) | 1 | 165605757 | 0.0062 | 7.22 (1.54,68.87) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs339567 | 0.0044 | A/G | G (0.267) | 1 | 191574391 | 0.019 | 2.79 (1.09,7.36) |
| rs2071402 | 0.0045 | A/G | A (0.5381) | 2 | 1396206 | 0.018 | 2.67 (1.13,6.42) |
| rs16865276 | 0.0045 | A/G | A (0.1524) | 2 | 6601910 | 0.02 | 3.63 (1.15,12.83) |
| rs10832084 | 0.0045 | A/C | C (0.2476) | 11 | 13584883 | 0.015 | 3.4 (1.14,11.05) |
| rs8065506 | 0.0045 | A/C | A (0.274) | 17 | 16468520 | 0.094 | 2.1 (0.85,5.26) |
| rs408718 | 0.0045 | A/G | G (0.3073) | 22 | 19750323 | 0.0036 | 3.94 (1.47,11.2) |
| rs10102717 | 0.0045 | A/G | A (0.4) | 8 | 19801093 | 0.0029 | 3.4 (1.43,8.35) |
| rs1994474 | 0.0045 | A/G | A (0.5) | 12 | 26838121 | 0.052 | 2.23 (0.96,5.28) |
| rs12783959 | 0.0045 | A/G | G (0.8095) | 10 | 26842367 | 0.0031 | 6.24 (1.66,35.3) |
| rs4346791 | 0.0045 | A/C | C (0.4238) | 5 | 36403210 | 0.0065 | 3 (1.28,7.24) |
| rs10506212 | 0.0045 | A/G | G (0.6952) | 12 | 41382291 | 0.0016 | 4.52 (1.66,13.88) |
| rs6493402 | 0.0045 | A/G | G (0.3333) | 15 | 48184796 | 0.088 | 2.19 (0.88,5.57) |
| rs2256982 | 0.0045 | A/G | A (0.3) | 10 | 70441901 | 0.0084 | 3.3 (1.25,9.2) |
| rs2639982 | 0.0045 | A/G | G (0.5762) | 18 | 71134403 | 0.034 | 2.34 (1.01,5.58) |
| rs6783760 | 0.0045 | A/G | G (0.8857) | 3 | 73619276 | 0.023 | 8.53 (1.13,383.04) |
| rs12344488 | 0.0045 | A/G | A (0.919) | 9 | 75655479 | 0.17 | 2.92 (0.58,19.09) |
| rs634294 | 0.0045 | A/G | A (0.7) | 1 | 85115018 | 0.0024 | 4.44 (1.55,14.77) |
| rs11107420 | 0.0045 | A/G | A (0.8905) | 12 | 93057281 | 0.035 | 5.3 (0.94,54.86) |
| rs255211 | 0.0045 | A/G | G (0.8619) | 5 | 94201137 | 0.2 | 2.16 (0.65,8.41) |
| rs17129493 | 0.0045 | A/G | A (0.919) | 11 | 96056987 | 0.027 | 4.71 (1.09,28.83) |
| rs4743196 | 0.0045 | A/G | A (0.381) | 9 | 98128807 | 0.0041 | 3.39 (1.38,8.57) |
| rs230487 | 0.0045 | A/C | A (0.7048) | 4 | 103746588 | 0.027 | 2.6 (1.05,6.75) |
| rs6579952 | 0.0045 | A/G | A (0.3462) | 5 | 152004950 | 0.041 | 2.39 (0.99,5.9) |
| rs9371379 | 0.0045 | A/G | G (0.4571) | 6 | 156004789 | 0.0037 | 3.23 (1.37,7.91) |
| rs3796578 | 0.0045 | A/G | A (0.8286) | 4 | 157006120 | 0.0034 | 7.53 (1.62,71.51) |
| rs1483030 | 0.0045 | A/G | G (0.8333) | 4 | 157011974 | 0.0034 | 7.53 (1.62,71.51) |
| rs2573083 | 0.0045 | A/G | A (0.5619) | 2 | 178670976 | 0.0018 | 3.54 (1.5,8.64) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs4700830 | 0.0045 | A/G | A (0.2286) | 5 | 178840871 | 0.058 | 2.55 (0.91,7.53) |
| rs11584662 | 0.0045 | A/C | C (0.4519) | 1 | 183312481 | 0.0062 | 3.13 (1.32,7.71) |
| rs7826500 | 0.0046 | A/C | C (0.181) | 8 | 1828620 | 0.015 | 3.4 (1.14,11.05) |
| rs10848840 | 0.0046 | A/C | C (0.1381) | 12 | 3269690 | 0.016 | 3.97 (1.18,15.68) |
| rs164632 | 0.0046 | A/G | A (0.2621) | 19 | 4090849 | 0.0016 | 5.19 (1.72,17.91) |
| rs6477115 | 0.0046 | A/C | C (0.3905) | 9 | 6827912 | 0.048 | 2.27 (0.97,5.42) |
| rs2157720 | 0.0046 | A/G | G (0.4903) | 22 | 16307240 | 0.077 | 2.12 (0.91,5.02) |
| rs913931 | 0.0046 | A/G | G (0.1505) | 9 | 21317141 | 0.044 | 2.95 (0.98,9.69) |
| rs2965402 | 0.0046 | A/G | A (0.8413) | 7 | 21521187 | 0.024 | 3.77 (1.1,16.78) |
| rs7403557 | 0.0046 | A/G | A (0.2619) | 15 | 24885725 | 0.039 | 2.75 (1.02,7.72) |
| rs410349 | 0.0046 | A/G | A (0.4714) | 21 | 27157065 | 0.051 | 2.26 (0.97,5.41) |
| rs2277693 | 0.0046 | A/C | C (0.355) | 17 | 43264182 | 0.022 | 2.71 (1.1,6.85) |
| rs9894630 | 0.0046 | A/G | A (0.4714) | 17 | 46526413 | 0.02 | 2.57 (1.1,6.19) |
| rs7974557 | 0.0046 | A/C | C (0.7108) | 12 | 56648046 | 0.13 | 2.08 (0.79,5.88) |
| rs10132229 | 0.0046 | A/G | G (0.9) | 14 | 64847313 | 0.035 | 5.3 (0.94,54.86) |
| rs2870957 | 0.0046 | A/C | C (0.2476) | 12 | 66772702 | 0.08 | 2.21 (0.85,5.88) |
| rs3743212 | 0.0046 | A/G | G (0.0762) | 15 | 73206596 | 0.012 | 9.56 (1.3,426.01) |
| rs276695 | 0.0046 | A/G | G (0.6667) | 6 | 76525835 | 0.00019 | 5.76 (2.03,19.08) |
| rs1786331 | 0.0046 | A/G | G (0.6905) | 8 | 101760866 | 0.071 | 2.17 (0.89,5.51) |
| rs1508884 | 0.0046 | A/C | A (0.9) | 5 | 115442511 | 0.094 | 3.49 (0.74,22.16) |
| rs17816967 | 0.0046 | A/G | A (0.36) | 6 | 115577822 | 0.017 | 2.72 (1.13,6.68) |
| rs784482 | 0.0046 | A/G | A (0.9048) | 5 | 115644187 | 0.094 | 3.49 (0.74,22.16) |
| rs7096525 | 0.0046 | A/G | G (0.2571) | 10 | 120538129 | 0.083 | 2.19 (0.86,5.7) |
| rs1282211 | 0.0046 | A/G | A (0.233) | 6 | 153239800 | 0.16 | 1.96 (0.7,5.63) |
| rs2501864 | 0.0046 | A/G | G (0.2524) | 1 | 158043553 | 0.035 | 2.82 (1.02,8.26) |
| rs931370 | 0.0046 | A/C | A (0.1952) | 4 | 188480941 | 0.15 | 2.06 (0.71,6.2) |
| rs1937235 | 0.0046 | A/C | A (0.5952) | 1 | 188882831 | 0.12 | 1.99 (0.85,4.75) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs16870615 | 0.0047 | A/G | A (0.1394) | 5 | 2929127 | 0.027 | 4.71 (1.09,28.83) |
| rs7506674 | 0.0047 | A/G | A (0.5143) | 18 | 3123302 | 0.0063 | 3.07 (1.31,7.41) |
| rs13099634 | 0.0047 | A/G | G (0.8429) | 3 | 12443463 | 0.012 | 6.33 (1.34,60.52) |
| rs6110157 | 0.0047 | A/G | G (0.1952) | 20 | 14003947 | 0.22 | 1.84 (0.62,5.6) |
| rs10483271 | 0.0047 | A/G | A (0.3143) | 14 | 21831090 | 0.15 | 1.84 (0.75,4.56) |
| rs1260719 | 0.0047 | A/C | C (0.5769) | 18 | 26824023 | 0.12 | 1.94 (0.83,4.62) |
| rs174709 | 0.0047 | A/G | G (0.4381) | 22 | 28149077 | 0.078 | 2.12 (0.91,5.07) |
| rs1909509 | 0.0047 | A/G | A (0.1857) | 12 | 32617966 | 0.02 | 3.63 (1.15,12.83) |
| rs16977665 | 0.0047 | A/G | A (0.0952) | 18 | 39624898 | 0.0019 | 14.52 (1.88,658.49) |
| rs7335572 | 0.0047 | A/G | G (0.0905) | 13 | 40286476 | 0.0022 | Inf (2.1,Inf) |
| rs2301690 | 0.0047 | A/G | A (0.3485) | 17 | 43245706 | 0.021 | 2.7 (1.08,6.9) |
| rs756847 | 0.0047 | A/G | G (0.0619) | 17 | 45513048 | 0.0096 | Inf (1.59,Inf) |
| rs10519257 | 0.0047 | A/G | G (0.2667) | 15 | 48176213 | 0.1 | 2.26 (0.82,6.43) |
| rs1927859 | 0.0047 | A/G | A (0.1762) | 13 | 52992734 | 0.052 | 3.13 (0.89,12.67) |
| rs198747 | 0.0047 | A/G | G (0.8667) | 11 | 61094821 | 0.054 | 3.76 (0.95,21.94) |
| rs925683 | 0.0047 | A/G | A (0.219) | 5 | 62456472 | 0.16 | 2.03 (0.73,5.85) |
| rs862707 | 0.0047 | A/G | G (0.1714) | 19 | 62558685 | 0.015 | 3.4 (1.14,11.05) |
| rs464817 | 0.0047 | A/G | A (0.4667) | 12 | 63601564 | 4.00E-04 | 4.42 (1.82,11.34) |
| rs2222544 | 0.0047 | A/G | G (0.2952) | 7 | 69214020 | 0.01 | 3.17 (1.25,8.33) |
| rs389540 | 0.0047 | A/G | A (0.7596) | 8 | 71033622 | 0.043 | 2.76 (0.98,8.63) |
| rs1986116 | 0.0047 | A/G | A (0.2571) | 14 | 76583597 | 0.011 | 4.03 (1.3,14.12) |
| rs4291215 | 0.0047 | A/C | C (0.3571) | 7 | 83913179 | 0.002 | 3.72 (1.5,9.58) |
| rs4843811 | 0.0047 | A/G | A (0.9238) | 16 | 86728864 | 0.094 | 3.49 (0.74,22.16) |
| rs2005613 | 0.0047 | A/G | G (0.2404) | 2 | 95845856 | 0.003 | 4.12 (1.48,12.43) |
| rs1541796 | 0.0047 | A/C | C (0.6095) | 12 | 99586550 | 0.017 | 2.81 (1.16,7.12) |
| rs1337144 | 0.0047 | A/G | G (0.7286) | 13 | 104110978 | 0.061 | 2.41 (0.95,6.48) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2174880 | 0.0047 | A/G | A (0.4381) | 11 | 115810193 | 0.053 | 2.19 (0.95,5.22) |
| rs7735219 | 0.0047 | A/C | C (0.2333) | 5 | 117632494 | 0.12 | 2 (0.76,5.38) |
| rs1947803 | 0.0047 | A/G | G (0.2333) | 5 | 117632864 | 0.12 | 2 (0.76,5.38) |
| rs7029536 | 0.0047 | A/G | G (0.7429) | 9 | 127189965 | 0.013 | 3.14 (1.22,8.7) |
| rs11016723 | 0.0047 | A/G | G (0.7048) | 10 | 130946769 | 0.0036 | 3.86 (1.47,11.19) |
| rs7014552 | 0.0047 | A/G | G (0.6333) | 8 | 143408330 | 0.11 | 2.08 (0.86,5.17) |
| rs12117452 | 0.0047 | A/G | A (0.2429) | 1 | 233973323 | 0.064 | 2.49 (0.92,7.05) |
| rs7022297 | 0.0048 | A/G | A (0.8762) | 9 | 1273982 | 0.04 | 4.69 (0.95,45.79) |
| rs1029773 | 0.0048 | A/G | G (0.1524) | 12 | 3270544 | 0.02 | 3.63 (1.15,12.83) |
| rs2039331 | 0.0048 | A/G | G (0.2) | 9 | 8978375 | 0.23 | 1.82 (0.64,5.3) |
| rs9349494 | 0.0048 | A/G | G (0.7333) | 6 | 13281133 | 0.13 | 2.09 (0.8,5.88) |
| rs1204168 | 0.0048 | A/G | G (0.4381) | 6 | 13944651 | 0.078 | 2.12 (0.91,5.07) |
| rs10140287 | 0.0048 | A/G | G (0.4524) | 14 | 21139619 | 0.12 | 1.97 (0.85,4.64) |
| rs1780327 | 0.0048 | A/G | G (0.5762) | 1 | 21628126 | 0.03 | 2.52 (1.05,6.26) |
| rs10805281 | 0.0048 | A/G | G (0.3269) | 4 | 28571347 | 0.091 | 2.1 (0.84,5.35) |
| rs10771794 | 0.0048 | A/G | G (0.5286) | 12 | 31074412 | 0.032 | 2.44 (1.05,5.81) |
| rs4952342 | 0.0048 | A/G | A (0.79) | 2 | 33258707 | 0.042 | 3.6 (1.04,16.02) |
| rs1705127 | 0.0048 | A/G | G (0.5238) | 2 | 33987653 | 0.03 | 2.42 (1.02,5.86) |
| rs4890477 | 0.0048 | A/G | A (0.3048) | 18 | 40257464 | 0.056 | 2.39 (0.96,6.08) |
| rs10048300 | 0.0048 | A/C | C (0.3) | 18 | 40283834 | 0.056 | 2.39 (0.96,6.08) |
| rs1511770 | 0.0048 | A/G | A (0.3) | 18 | 40284761 | 0.056 | 2.39 (0.96,6.08) |
| rs3884504 | 0.0048 | A/G | G (0.7333) | 2 | 43607204 | 0.032 | 2.78 (1.04,8.1) |
| rs16945848 | 0.0048 | A/G | A (0.1238) | 15 | 60913837 | 0.052 | 3.13 (0.89,12.67) |
| rs1073617 | 0.0048 | A/G | A (0.5952) | 16 | 64344665 | 0.03 | 2.52 (1.05,6.26) |
| rs6592590 | 0.0048 | A/G | A (0.4952) | 11 | 74058677 | 0.02 | 2.57 (1.1,6.19) |
| rs10504678 | 0.0048 | A/G | G (0.9333) | 8 | 79680931 | 0.017 | 6.21 (1.16,63.16) |
| rs12594095 | 0.0048 | A/G | G (0.2714) | 15 | 86508197 | 0.016 | 3.97 (1.18,15.68) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs10520676 | 0.0048 | A/G | A (0.2524) | 15 | 86510642 | 0.012 | 4.49 (1.21,20.85) |
| rs7328769 | 0.0048 | A/C | C (0.3381) | 13 | 92495230 | 0.00023 | 4.82 (1.95,12.49) |
| rs6468601 | 0.0048 | A/G | A (0.1095) | 8 | 98946713 | 0.26 | 1.95 (0.54,7.42) |
| rs699301 | 0.0048 | A/G | G (0.9048) | 3 | 99127342 | 0.023 | 8.53 (1.13,383.04) |
| rs11123857 | 0.0048 | A/G | A (0.6857) | 2 | 101062330 | 0.066 | 2.28 (0.92,5.94) |
| rs10739488 | 0.0048 | A/G | G (0.6762) | 9 | 117098903 | 0.066 | 2.28 (0.92,5.94) |
| rs7443402 | 0.0048 | A/C | A (0.1857) | 5 | 117711770 | 0.061 | 2.9 (0.89,10.47) |
| rs6874663 | 0.0048 | A/G | G (0.8462) | 5 | 143449989 | 0.022 | 5.76 (1.2,55.34) |
| rs3769215 | 0.0048 | A/G | A (0.2) | 2 | 173731004 | 0.011 | 4.03 (1.3,14.12) |
| rs6477405 | 0.0049 | A/C | C (0.3579) | 9 | 9710279 | 0.013 | 3.02 (1.23,7.68) |
| rs1471844 | 0.0049 | A/C | C (0.3286) | 12 | 24865112 | 0.0075 | 3.11 (1.27,7.86) |
| rs10182608 | 0.0049 | A/G | G (0.781) | 2 | 33691882 | 0.0063 | 4.15 (1.35,15.46) |
| rs2014842 | 0.0049 | A/G | G (0.8173) | 22 | 38033509 | 0.032 | 3.21 (1.01,12.16) |
| rs3088362 | 0.0049 | A/C | A (0.1286) | 13 | 43331630 | 0.052 | 3.13 (0.89,12.67) |
| rs7230692 | 0.0049 | A/G | A (0.1286) | 18 | 43774285 | 0.029 | 3.54 (1.03,14.13) |
| rs7226071 | 0.0049 | A/C | C (0.3069) | 17 | 66917957 | 0.02 | 2.88 (1.15,7.44) |
| rs966671 | 0.0049 | A/C | C (0.2952) | 16 | 79158463 | 0.0039 | 3.41 (1.38,8.78) |
| rs4235653 | 0.0049 | A/G | G (0.6635) | 5 | 80673285 | 0.059 | 2.38 (0.93,6.43) |
| rs1553585 | 0.0049 | A/G | A (0.7571) | 6 | 100915711 | 0.12 | 2.25 (0.83,6.62) |
| rs2888865 | 0.0049 | A/G | A (0.6) | 12 | 104585509 | 0.016 | 2.95 (1.2,7.67) |
| rs7976650 | 0.0049 | A/C | A (0.9272) | 12 | 105027938 | 0.029 | 4.56 (1.05,27.85) |
| rs10514623 | 0.0049 | A/G | G (0.1333) | 2 | 108422010 | 0.12 | 3.02 (0.75,14.69) |
| rs7455866 | 0.0049 | A/C | A (0.3571) | 7 | 123326886 | 0.0041 | 3.39 (1.38,8.57) |
| rs2461210 | 0.0049 | A/C | C (0.0619) | 10 | 123784538 | 0.075 | 6.58 (0.83,301.83) |
| rs4921165 | 0.0049 | A/C | C (0.2115) | 5 | 160212958 | 0.015 | 3.4 (1.14,11.05) |
| rs10520520 | 0.0049 | A/G | A (0.3095) | 4 | 183399226 | 0.1 | 2.01 (0.83,4.97) |
| rs1908976 | 0.005 | A/G | A (0.3857) | 5 | 2842932 | 0.00089 | 3.88 (1.63,9.58) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs12606686 | 0.005 | A/G | A (0.9048) | 18 | 9032622 | 0.094 | 3.49 (0.74,22.16) |
| rs177053 | 0.005 | A/C | A (0.0524) | 5 | 17236891 | 0.03 | Inf (0.92,Inf) |
| rs7627406 | 0.005 | A/C | A (0.9381) | 3 | 18143807 | 0.094 | 3.49 (0.74,22.16) |
| rs12677666 | 0.005 | A/G | A (0.6571) | 8 | 20186399 | 0.23 | 1.64 (0.69,4.03) |
| rs1472563 | 0.005 | A/G | G (0.5096) | 1 | 21585804 | 0.053 | 2.19 (0.94,5.17) |
| rs6557594 | 0.005 | A/G | A (0.1337) | 8 | 22711133 | 0.054 | 3.08 (0.87,12.53) |
| rs12062136 | 0.005 | A/C | C (0.07) | 1 | 24178312 | 0.02 | Inf (1.12,Inf) |
| rs2643364 | 0.005 | A/G | A (0.219) | 15 | 31663809 | 0.08 | 2.21 (0.85,5.88) |
| rs1529717 | 0.005 | A/G | G (0.7019) | 19 | 46575038 | 0.0015 | 4.49 (1.64,13.86) |
| rs2275442 | 0.005 | A/G | A (0.0619) | 10 | 59705158 | 0.16 | 5.72 (0.54,290.23) |
| rs4775551 | 0.005 | A/G | A (0.2048) | 15 | 60924375 | 0.016 | 2.95 (1.14,7.98) |
| rs9291919 | 0.005 | A/G | G (0.7286) | 5 | 67336205 | 0.014 | 3.62 (1.25,12.12) |
| rs9747992 | 0.005 | A/G | G (0.0762) | 17 | 75419435 | 0.11 | 3.7 (0.71,36.88) |
| rs2272316 | 0.005 | A/G | G (0.7381) | 7 | 76587610 | 0.087 | 2.24 (0.86,6.29) |
| rs12589344 | 0.005 | A/C | C (0.4238) | 14 | 85027423 | 0.12 | 1.93 (0.83,4.57) |
| rs466432 | 0.005 | A/G | A (0.3413) | 13 | 92488907 | 0.00023 | 4.82 (1.95,12.49) |
| rs2171186 | 0.005 | A/G | G (0.8286) | 11 | 94328585 | 0.025 | 3.92 (1.15,17.32) |
| rs1847393 | 0.005 | A/G | A (0.27) | 3 | 102062947 | 0.14 | 2.25 (0.77,6.83) |
| rs9519439 | 0.005 | A/G | G (0.7238) | 13 | 104113337 | 0.061 | 2.41 (0.95,6.48) |
| rs3819100 | 0.005 | A/G | A (0.6952) | 11 | 113672686 | 0.093 | 2.26 (0.89,6.07) |
| rs26529 | 0.005 | A/G | G (0.3905) | 5 | 115212349 | 0.048 | 2.27 (0.97,5.42) |
| rs2215348 | 0.005 | A/G | G (0.4381) | 7 | 123351834 | 0.03 | 2.48 (1.06,5.92) |
| rs11103115 | 0.005 | A/G | A (0.268) | 9 | 135753216 | 0.069 | 2.35 (0.87,6.57) |
| rs258799 | 0.005 | A/C | A (0.4095) | 5 | 142510153 | 0.018 | 2.67 (1.13,6.42) |
| rs17085526 | 0.005 | A/G | A (0.3857) | 6 | 154968617 | 0.0029 | 3.4 (1.43,8.35) |
| rs4679735 | 0.005 | A/G | G (0.1476) | 3 | 155654932 | 0.016 | 3.97 (1.18,15.68) |
| rs4861485 | 0.005 | A/G | A (0.7762) | 4 | 182868867 | 0.0033 | 5.78 (1.53,32.82) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs1583631 | 0.005 | A/C | C (0.7429) | 2 | 211888317 | 0.047 | 2.63 (0.94,8.21) |
| rs2049387 | 0.0051 | A/C | C (0.7) | 7 | 10232035 | 0.057 | 2.41 (0.92,6.72) |
| rs4797788 | 0.0051 | A/G | G (0.5429) | 18 | 13580752 | 0.051 | 2.28 (0.99,5.41) |
| rs955205 | 0.0051 | A/C | A (0.0762) | 5 | 16400660 | 0.075 | 6.58 (0.83,301.83) |
| rs4589352 | 0.0051 | A/G | G (0.6667) | 12 | 17773336 | 0.012 | 3.18 (1.2,9.23) |
| rs10764354 | 0.0051 | A/G | A (0.5971) | 10 | 23208734 | 0.074 | 2.13 (0.89,5.24) |
| rs2850323 | 0.0051 | A/G | G (0.7596) | 18 | 26825081 | 0.0075 | 3.81 (1.31,12.78) |
| rs396419 | 0.0051 | A/C | C (0.5433) | 14 | 26854279 | 0.03 | 2.48 (1.06,5.92) |
| rs2624272 | 0.0051 | A/G | G (0.7714) | 15 | 36790701 | 0.015 | 3.38 (1.16,11.33) |
| rs17030752 | 0.0051 | A/G | A (0.7667) | 2 | 43542144 | 0.0096 | 3.48 (1.26,10.73) |
| rs9614976 | 0.0051 | A/G | G (0.9327) | 22 | 43625043 | 0.12 | 2.92 (0.72,14.2) |
| rs6090921 | 0.0051 | A/G | A (0.2) | 20 | 46935183 | 0.015 | 3.4 (1.14,11.05) |
| rs17627715 | 0.0051 | A/G | G (0.8619) | 7 | 47590631 | 0.04 | 4.69 (0.95,45.79) |
| rs9323346 | 0.0051 | A/G | G (0.1952) | 14 | 58865754 | 0.053 | 2.63 (0.9,8.18) |
| rs7143698 | 0.0051 | A/G | A (0.1952) | 14 | 58873880 | 0.053 | 2.63 (0.9,8.18) |
| rs1019614 | 0.0051 | A/G | G (0.6381) | 2 | 60042808 | 0.11 | 2.08 (0.86,5.17) |
| rs7714712 | 0.0051 | A/C | A (0.3571) | 5 | 60768309 | 0.018 | 2.67 (1.13,6.42) |
| rs288783 | 0.0051 | A/C | A (0.9095) | 13 | 67363476 | 0.0046 | Inf (1.84,Inf) |
| rs1402910 | 0.0051 | A/G | A (0.7571) | 2 | 78962712 | 0.12 | 2.25 (0.83,6.62) |
| rs1829764 | 0.0051 | A/G | A (0.1286) | 11 | 80428642 | 0.39 | 1.67 (0.44,6.51) |
| rs778233 | 0.0051 | A/G | A (0.5857) | 5 | 84653232 | 0.028 | 2.63 (1.09,6.68) |
| rs186360 | 0.0051 | A/G | G (0.5) | 1 | 84704131 | 0.032 | 2.44 (1.05,5.81) |
| rs6919366 | 0.0051 | A/C | A (0.7) | 6 | 87169891 | 0.14 | 1.97 (0.77,5.32) |
| rs1859037 | 0.0051 | A/G | A (0.3095) | 7 | 91212923 | 0.014 | 2.84 (1.17,7.09) |
| rs6465347 | 0.0051 | A/G | G (0.3095) | 7 | 91338488 | 0.014 | 2.84 (1.17,7.09) |
| rs733957 | 0.0051 | A/G | A (0.3095) | 7 | 91351848 | 0.014 | 2.84 (1.17,7.09) |
| rs2079082 | 0.0051 | A/G | G (0.3095) | 7 | 91356442 | 0.014 | 2.84 (1.17,7.09) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs4265 | 0.0051 | A/G | A (0.3095) | 7 | 91364259 | 0.014 | 2.84 (1.17,7.09) |
| rs1063243 | 0.0051 | A/C | C (0.3095) | 7 | 91371578 | 0.014 | 2.84 (1.17,7.09) |
| rs2113070 | 0.0051 | A/C | A (0.12) | 5 | 94287596 | 0.018 | 6.12 (1.13,62.36) |
| rs7338702 | 0.0051 | A/G | G (0.8381) | 13 | 94383942 | 0.025 | 3.92 (1.15,17.32) |
| rs9323947 | 0.0051 | A/G | A (0.9429) | 14 | 96206106 | 0.17 | 2.92 (0.58,19.09) |
| rs1965397 | 0.0051 | A/C | A (0.1048) | 3 | 100866101 | 0.027 | 4.71 (1.09,28.83) |
| rs1148409 | 0.0051 | A/G | A (0.4667) | 12 | 105167367 | 0.08 | 2.05 (0.87,4.96) |
| rs4978664 | 0.0051 | A/G | G (0.6286) | 9 | 106899483 | 0.017 | 2.81 (1.16,7.12) |
| rs7970490 | 0.0051 | A/G | A (0.7333) | 12 | 110219158 | 0.057 | 2.41 (0.92,6.72) |
| rs2519543 | 0.0051 | A/G | G (0.4279) | 12 | 118384255 | 0.075 | 2.09 (0.89,4.96) |
| rs7825404 | 0.0051 | A/G | A (0.9381) | 8 | 124714693 | 0.16 | 5.72 (0.54,290.23) |
| rs1482294 | 0.0051 | A/G | A (0.4952) | 12 | 128235671 | 0.011 | 2.86 (1.2,7.13) |
| rs4955476 | 0.0051 | A/G | G (0.9) | 3 | 135913954 | 0.04 | 4.69 (0.95,45.79) |
| rs7627904 | 0.0051 | A/C | C (0.6762) | 3 | 140362636 | 0.0056 | 3.72 (1.36,11.45) |
| rs1338634 | 0.0051 | A/G | A (0.9238) | 1 | 161237847 | 0.035 | 5.3 (0.94,54.86) |
| rs2291270 | 0.0051 | A/C | C (0.5238) | 5 | 169078543 | 0.052 | 2.23 (0.96,5.28) |
| rs4240923 | 0.0051 | A/C | C (0.7429) | 1 | 213045654 | 0.015 | 3.38 (1.16,11.33) |
| rs9298820 | 0.0052 | A/G | G (0.3571) | 9 | 2167391 | 0.0075 | 3.11 (1.27,7.86) |
| rs7574256 | 0.0052 | A/C | C (0.1667) | 2 | 3772659 | 0.26 | 1.95 (0.54,7.42) |
| rs12964045 | 0.0052 | A/C | C (0.1286) | 18 | 13442635 | 0.23 | 2.38 (0.63,9.99) |
| rs932649 | 0.0052 | A/G | A (0.1779) | 8 | 18833654 | 0.44 | 1.63 (0.53,5.03) |
| rs2928679 | 0.0052 | A/G | A (0.519) | 8 | 23494920 | 0.02 | 2.57 (1.1,6.19) |
| rs550969 | 0.0052 | A/G | G (0.6905) | 6 | 24054846 | 0.0075 | 3.35 (1.31,9.28) |
| rs233896 | 0.0052 | A/C | C (0.4417) | 21 | 27246439 | 0.11 | 2.02 (0.86,4.81) |
| rs7684647 | 0.0052 | A/G | A (0.3942) | 4 | 44390774 | 0.0013 | 3.86 (1.57,9.82) |
| rs1015985 | 0.0052 | A/G | A (0.101) | 20 | 55243363 | 0.015 | 5.2 (1.23,31.35) |
| rs2574764 | 0.0052 | A/G | A (0.8333) | 19 | 61226336 | 0.0059 | 5.34 (1.4,30.46) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs10233199 | 0.0052 | A/G | G (0.77) | 7 | 63338386 | 0.018 | 3.76 (1.21,14.12) |
| rs2800265 | 0.0052 | A/G | A (0.4619) | 9 | 74750231 | 0.053 | 2.19 (0.95,5.22) |
| rs9565163 | 0.0052 | A/G | A (0.1667) | 13 | 74883232 | 0.18 | 2.17 (0.67,7.37) |
| rs4903399 | 0.0052 | A/G | A (0.1571) | 14 | 75844955 | 0.011 | 4.03 (1.3,14.12) |
| rs2503273 | 0.0052 | A/G | G (0.1571) | 1 | 87985811 | 0.052 | 3.13 (0.89,12.67) |
| rs891750 | 0.0052 | A/G | G (0.8) | 14 | 88086953 | 0.047 | 2.63 (0.94,8.21) |
| rs1378876 | 0.0052 | A/G | A (0.5625) | 4 | 97252528 | 0.01 | 2.99 (1.24,7.59) |
| rs10860363 | 0.0052 | A/G | G (0.5238) | 12 | 97651135 | 0.018 | 2.7 (1.15,6.49) |
| rs6538888 | 0.0052 | A/G | A (0.2143) | 12 | 97846351 | 0.039 | 2.75 (1.02,7.72) |
| rs10750642 | 0.0052 | A/G | A (0.681) | 11 | 101862489 | 0.022 | 2.94 (1.14,8.16) |
| rs1431010 | 0.0052 | A/G | A (0.5143) | 8 | 102338200 | 0.0065 | 3 (1.28,7.24) |
| rs2903281 | 0.0052 | A/G | A (0.2667) | 4 | 103753512 | 0.02 | 3.11 (1.13,9.04) |
| rs7115187 | 0.0052 | A/G | G (0.9048) | 11 | 105001548 | 0.094 | 3.49 (0.74,22.16) |
| rs6913816 | 0.0052 | A/C | C (0.6381) | 6 | 142080058 | 0.044 | 2.47 (1.02,6.26) |
| rs1989181 | 0.0052 | A/C | C (0.8558) | 6 | 142565558 | 0.051 | 2.95 (0.93,11.26) |
| rs10062528 | 0.0052 | A/C | A (0.768) | 5 | 160083400 | 0.031 | 3.29 (1.04,12.51) |
| rs2337552 | 0.0052 | A/G | G (0.581) | 5 | 168099870 | 0.032 | 2.42 (1.02,5.92) |
| rs10497560 | 0.0052 | A/G | G (0.819) | 2 | 180820819 | 0.0081 | 4.58 (1.36,20.06) |
| rs3860698 | 0.0052 | A/G | A (0.1286) | 4 | 188475813 | 0.0038 | 8.18 (1.61,80.71) |
| rs3111558 | 0.0053 | A/G | G (0.9279) | 16 | 7771215 | 0.083 | 7.08 (0.75,345.31) |
| rs9923450 | 0.0053 | A/G | A (0.6) | 16 | 9009889 | 0.0066 | 3.05 (1.28,7.61) |
| rs6033654 | 0.0053 | A/C | A (0.6143) | 20 | 13195500 | 0.072 | 2.21 (0.92,5.51) |
| rs4814789 | 0.0053 | A/G | G (0.7714) | 20 | 18697415 | 0.025 | 3.92 (1.15,17.32) |
| rs711717 | 0.0053 | A/G | A (0.1667) | 3 | 22985484 | 0.035 | 3.26 (1.02,11.61) |
| rs736081 | 0.0053 | A/G | G (0.6) | 13 | 23557726 | 0.0058 | 3.19 (1.32,8.1) |
| rs205138 | 0.0053 | A/G | A (0.2115) | 16 | 25523872 | 0.00073 | 4.92 (1.79,14.77) |
| rs2056317 | 0.0053 | A/G | A (0.8762) | 15 | 35350843 | 0.018 | 4.52 (1.16,26.01) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs12271660 | 0.0053 | A/G | G (0.9429) | 11 | 36618299 | 0.094 | 3.49 (0.74,22.16) |
| rs9315676 | 0.0053 | A/G | G (0.9381) | 13 | 38822894 | 0.035 | 5.3 (0.94,54.86) |
| rs6065285 | 0.0053 | A/G | G (0.2619) | 20 | 38982242 | 0.0026 | 3.84 (1.5,10.33) |
| rs913382 | 0.0053 | A/G | A (0.4952) | 1 | 40992975 | 0.25 | 1.61 (0.7,3.74) |
| rs38055 | 0.0053 | A/G | A (0.3762) | 5 | 52596401 | 0.012 | 2.75 (1.18,6.58) |
| rs10999171 | 0.0053 | A/C | C (0.4333) | 10 | 53168114 | 0.11 | 1.94 (0.83,4.59) |
| rs10271255 | 0.0053 | A/G | A (0.7381) | 7 | 63354597 | 0.014 | 3.62 (1.25,12.12) |
| rs461829 | 0.0053 | A/G | A (0.5857) | 12 | 63621984 | 0.0029 | 3.58 (1.46,9.3) |
| rs2300871 | 0.0053 | A/C | C (0.9333) | 14 | 65217447 | 0.07 | 4.44 (0.75,47.1) |
| rs2700565 | 0.0053 | A/G | G (0.1619) | 12 | 66144120 | 0.016 | 3.97 (1.18,15.68) |
| rs1567793 | 0.0053 | A/G | A (0.619) | 6 | 67180515 | 0.16 | 1.87 (0.79,4.58) |
| rs1418773 | 0.0053 | A/C | A (0.2524) | 13 | 76288165 | 0.05 | 2.42 (0.94,6.43) |
| rs16947728 | 0.0053 | A/G | A (0.1238) | 16 | 77081831 | 0.039 | 3.97 (1.05,18.68) |
| rs499418 | 0.0053 | A/G | A (0.3462) | 12 | 77507266 | 0.0055 | 3.12 (1.31,7.63) |
| rs11002468 | 0.0053 | A/G | G (0.6786) | 10 | 79682057 | 0.024 | 3.07 (1.08,9.74) |
| rs1590235 | 0.0053 | A/G | A (0.3857) | 6 | 85958845 | 0.069 | 2.22 (0.94,5.37) |
| rs2722814 | 0.0053 | A/G | A (0.1143) | 9 | 117942466 | 0.006 | 5.04 (1.39,23.16) |
| rs2285996 | 0.0053 | A/G | A (0.2524) | 7 | 123188157 | 0.016 | 2.95 (1.14,7.98) |
| rs1348658 | 0.0053 | A/G | A (0.9333) | 3 | 146421326 | 0.035 | 5.3 (0.94,54.86) |
| rs6803575 | 0.0053 | A/C | A (0.3429) | 3 | 178173998 | 0.011 | 3.13 (1.26,8.06) |
| rs2253771 | 0.0053 | A/G | A (0.2476) | 1 | 196800315 | 0.019 | 2.87 (1.15,7.4) |
| rs3024490 | 0.0053 | A/C | A (0.2333) | 1 | 203333706 | 0.0084 | 3.3 (1.25,9.2) |
| rs4807140 | 0.0054 | A/G | G (0.4567) | 19 | 1724999 | 0.005 | 3.37 (1.37,8.81) |
| rs4880562 | 0.0054 | A/G | A (0.6) | 10 | 2453585 | 0.05 | 2.27 (0.96,5.55) |
| rs231917 | 0.0054 | A/G | G (0.6346) | 11 | 2701683 | 0.005 | 3.36 (1.34,9.02) |
| rs10495525 | 0.0054 | A/G | A (0.2524) | 2 | 5937776 | 0.024 | 3.02 (1.13,8.43) |
| rs7234920 | 0.0054 | A/G | G (0.2115) | 18 | 6477465 | 0.026 | 3.27 (1.1,10.67) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs12444064 | 0.0054 | A/G | G (0.7598) | 16 | 7107887 | 0.043 | 2.76 (0.98,8.63) |
| rs6885471 | 0.0054 | A/G | G (0.2905) | 5 | 16432041 | 0.0075 | 3.11 (1.27,7.86) |
| rs549601 | 0.0054 | A/G | A (0.5286) | 22 | 25986432 | 0.019 | 2.61 (1.12,6.24) |
| rs703027 | 0.0054 | A/G | G (0.1714) | 10 | 28680921 | 0.015 | 3.4 (1.14,11.05) |
| rs6863342 | 0.0054 | A/G | G (0.6524) | 5 | 29122318 | 0.0052 | 3.35 (1.37,8.72) |
| rs6867873 | 0.0054 | A/G | G (0.6524) | 5 | 29122666 | 0.0052 | 3.35 (1.37,8.72) |
| rs10053621 | 0.0054 | A/G | A (0.6538) | 5 | 29152463 | 0.0052 | 3.35 (1.37,8.72) |
| rs4124634 | 0.0054 | A/G | G (0.6524) | 5 | 29164784 | 0.0052 | 3.35 (1.37,8.72) |
| rs4441609 | 0.0054 | A/G | G (0.419) | 3 | 36867721 | 0.018 | 2.75 (1.16,6.75) |
| rs7299435 | 0.0054 | A/G | G (0.8476) | 12 | 37842780 | 0.022 | 5.76 (1.2,55.34) |
| rs879321 | 0.0054 | A/C | C (0.181) | 2 | 38012788 | 0.012 | 4.49 (1.21,20.85) |
| rs3761097 | 0.0054 | A/G | G (0.0714) | 19 | 40982817 | 0.0096 | Inf (1.59,Inf) |
| rs6074015 | 0.0054 | A/G | G (0.6414) | 20 | 44145582 | 0.0018 | 4.18 (1.62,11.53) |
| rs649700 | 0.0054 | A/G | A (0.719) | 6 | 44753714 | 0.015 | 2.93 (1.17,7.85) |
| rs3826705 | 0.0054 | A/G | A (0.8762) | 19 | 47329072 | 0.0066 | 10.63 (1.46,470.67) |
| rs1613040 | 0.0054 | A/G | G (0.9048) | 15 | 58162378 | 0.0022 | Inf (2.1,Inf) |
| rs4902208 | 0.0054 | A/C | A (0.3281) | 14 | 62694173 | 0.15 | 1.91 (0.78,4.75) |
| rs1404676 | 0.0054 | A/G | A (0.7598) | 7 | 63173627 | 0.0044 | 5.04 (1.51,22.09) |
| rs999944 | 0.0054 | A/G | A (0.1286) | 2 | 64961504 | 0.052 | 3.13 (0.89,12.67) |
| rs7851675 | 0.0054 | A/C | C (0.1) | 9 | 89951860 | 0.014 | 5.38 (1.27,32.42) |
| rs718268 | 0.0054 | A/G | A (0.7524) | 6 | 100965880 | 0.073 | 2.44 (0.87,7.65) |
| rs6877440 | 0.0054 | A/G | G (0.1287) | 5 | 109071966 | 0.0049 | 7.63 (1.5,75.52) |
| rs10124515 | 0.0054 | A/G | A (0.1765) | 9 | 113153446 | 0.027 | 3.15 (1.05,10.29) |
| rs967099 | 0.0054 | A/G | A (0.3905) | 4 | 114476254 | 0.026 | 2.62 (1.1,6.39) |
| rs363221 | 0.0054 | A/G | G (0.0571) | 10 | 119008021 | 0.13 | 5.66 (0.69,263.42) |
| rs7101547 | 0.0054 | A/G | G (0.8238) | 11 | 132700125 | 0.087 | 2.6 (0.81,9.94) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs12535022 | 0.0054 | A/G | A (0.1923) | 7 | 138229160 | 0.23 | 1.82 (0.64,5.3) |
| rs996955 | 0.0054 | A/G | G (0.3571) | 8 | 139447407 | 0.065 | 2.19 (0.91,5.41) |
| rs625717 | 0.0054 | A/G | G (0.4714) | 6 | 145459728 | 0.033 | 2.41 (1.03,5.78) |
| rs1389717 | 0.0054 | A/G | G (0.6524) | 6 | 151133914 | 0.015 | 2.93 (1.17,7.85) |
| rs10015908 | 0.0054 | A/G | G (0.8048) | 4 | 187652061 | 0.032 | 3.32 (1.06,12.5) |
| rs6602311 | 0.0055 | A/G | A (0.2) | 10 | 8548252 | 0.1 | 2.26 (0.82,6.43) |
| rs10805347 | 0.0055 | A/G | G (0.6381) | 4 | 15517108 | 0.044 | 2.47 (1.02,6.26) |
| rs6531036 | 0.0055 | A/G | G (0.8619) | 2 | 17352466 | 0.054 | 3.76 (0.95,21.94) |
| rs1525902 | 0.0055 | A/G | G (0.1058) | 12 | 33870876 | 0.051 | 4.09 (0.91,25.4) |
| rs1529806 | 0.0055 | A/G | G (0.2762) | 18 | 43156991 | 0.04 | 2.39 (0.98,5.97) |
| rs2924250 | 0.0055 | A/G | G (0.4) | 17 | 43698118 | 0.01 | 2.88 (1.22,6.99) |
| rs10483612 | 0.0055 | A/C | C (0.1842) | 14 | 52104019 | 0.0048 | 4.68 (1.42,18.34) |
| rs7663168 | 0.0055 | A/C | A (0.8434) | 4 | 67166380 | 0.18 | 2.36 (0.65,10.84) |
| rs1434762 | 0.0055 | A/G | A (0.6733) | 8 | 69166175 | 0.0023 | 3.84 (1.49,10.72) |
| rs273464 | 0.0055 | A/G | A (0.2381) | 9 | 79058890 | 0.13 | 2 (0.78,5.22) |
| rs1439618 | 0.0055 | A/G | G (0.419) | 15 | 91250973 | 0.077 | 2.15 (0.93,5.08) |
| rs4451693 | 0.0055 | A/G | A (0.1476) | 11 | 98511000 | 0.052 | 3.13 (0.89,12.67) |
| rs2190358 | 0.0055 | A/G | G (0.681) | 2 | 102039742 | 0.039 | 2.58 (1.02,6.91) |
| rs10435337 | 0.0055 | A/C | A (0.2356) | 7 | 102924635 | 0.0067 | 3.6 (1.33,10.39) |
| rs228614 | 0.0055 | A/G | G (0.5714) | 4 | 103935840 | 0.047 | 2.36 (0.99,5.87) |
| rs1894671 | 0.0055 | A/G | G (0.3429) | 6 | 115916375 | 0.049 | 2.31 (0.99,5.48) |
| rs8600 | 0.0055 | A/C | A (0.2308) | 2 | 119776151 | 0.11 | 2.17 (0.79,6.19) |
| rs1428393 | 0.0055 | A/G | A (0.5194) | 5 | 122687008 | 0.031 | 2.48 (1.06,5.94) |
| rs1500847 | 0.0055 | A/G | G (0.5905) | 4 | 143190939 | 0.011 | 2.93 (1.24,7.21) |
| rs7463969 | 0.0055 | A/G | A (0.1287) | 8 | 143291920 | 0.0079 | 7.32 (1.4,73.3) |
| rs480110 | 0.0055 | A/G | A (0.5333) | 6 | 145447727 | 0.032 | 2.39 (1.03,5.67) |
| rs11737895 | 0.0055 | A/C | A (0.2714) | 4 | 156980332 | 0.12 | 2 (0.76,5.38) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs9814786 | 0.0055 | A/G | A (0.4) | 3 | 192294992 | 0.026 | 2.62 (1.1,6.39) |
| rs10802948 | 0.0055 | A/G | A (0.2143) | 1 | 237796675 | 0.16 | 2.03 (0.73,5.85) |
| rs13402622 | 0.0055 | A/G | A (0.7) | 2 | 240564340 | 0.015 | 2.93 (1.17,7.85) |
| rs1538714 | 0.0056 | A/G | A (0.1667) | 9 | 1314647 | 0.035 | 3.26 (1.02,11.61) |
| rs7976304 | 0.0056 | A/G | G (0.5429) | 12 | 5669475 | 0.08 | 2.09 (0.9,4.92) |
| rs6500756 | 0.0056 | A/C | A (0.0526) | 16 | 6079661 | 0.032 | Inf (0.9,Inf) |
| rs1658957 | 0.0056 | A/G | A (0.519) | 9 | 6662097 | 0.053 | 2.19 (0.94,5.17) |
| rs2578275 | 0.0056 | A/G | G (0.519) | 9 | 6663957 | 0.053 | 2.19 (0.94,5.17) |
| rs9378524 | 0.0056 | A/G | A (0.4) | 6 | 8070407 | 0.067 | 2.21 (0.92,5.38) |
| rs8111918 | 0.0056 | A/G | A (0.7857) | 19 | 14780710 | 0.045 | 2.83 (1.01,8.79) |
| rs869179 | 0.0056 | A/G | A (0.3476) | 1 | 21585435 | 0.014 | 2.84 (1.17,7.09) |
| rs2433084 | 0.0056 | A/C | C (0.8) | 8 | 25584964 | 0.096 | 2.5 (0.84,8.52) |
| rs7215616 | 0.0056 | A/G | G (0.9095) | 17 | 28547280 | 0.32 | 2.16 (0.48,11.1) |
| rs10486680 | 0.0056 | A/G | G (0.9381) | 7 | 38596814 | 0.13 | 3.62 (0.56,39.69) |
| rs10511953 | 0.0056 | A/G | G (0.2667) | 9 | 38705865 | 0.011 | 3.13 (1.26,8.06) |
| rs9532397 | 0.0056 | A/G | A (0.2762) | 13 | 39017410 | 0.0063 | 3.74 (1.38,10.77) |
| rs698613 | 0.0056 | A/G | G (0.9333) | 18 | 44781491 | 0.051 | 4.09 (0.91,25.4) |
| rs1807472 | 0.0056 | A/C | A (0.119) | 22 | 48249450 | 0.052 | 3.13 (0.89,12.67) |
| rs1395604 | 0.0056 | A/G | G (0.3883) | 16 | 49487455 | 0.046 | 2.33 (0.99,5.62) |
| rs9945952 | 0.0056 | A/G | A (0.2667) | 18 | 52853676 | 0.071 | 2.45 (0.93,6.69) |
| rs9536591 | 0.0056 | A/C | A (0.5762) | 13 | 53479088 | 0.017 | 2.81 (1.16,7.12) |
| rs10207939 | 0.0056 | A/C | A (0.7952) | 2 | 61165066 | 0.11 | 2.26 (0.8,7.12) |
| rs8071270 | 0.0056 | A/G | A (0.3125) | 17 | 66907543 | 0.022 | 2.73 (1.11,6.93) |
| rs1007874 | 0.0056 | A/G | A (0.3762) | 2 | 84280005 | 0.015 | 2.85 (1.19,7.01) |
| rs8041687 | 0.0056 | A/G | G (0.9143) | 15 | 86999564 | 0.0019 | Inf (2.13,Inf) |
| rs1158464 | 0.0056 | A/G | G (0.7129) | 5 | 88374545 | 0.006 | 3.64 (1.37,10.62) |
| rs4729015 | 0.0056 | A/C | A (0.3077) | 7 | 91206243 | 0.014 | 2.84 (1.17,7.09) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs7969106 | 0.0056 | A/C | C (0.1143) | 12 | 93115220 | 0.09 | 2.75 (0.76,11.29) |
| rs2106295 | 0.0056 | A/C | A (0.3667) | 7 | 93575940 | 0.00045 | 4.48 (1.79,11.77) |
| rs2310144 | 0.0056 | A/G | G (0.5381) | 2 | 101988279 | 0.052 | 2.23 (0.96,5.28) |
| rs1006817 | 0.0056 | A/C | A (0.2905) | 7 | 102849936 | 0.0012 | 4.3 (1.65,11.91) |
| rs17643292 | 0.0056 | A/G | A (0.1476) | 11 | 106023093 | 0.069 | 3.49 (0.89,16.63) |
| rs975666 | 0.0056 | A/G | G (0.4952) | 12 | 116625292 | 0.12 | 1.91 (0.83,4.48) |
| rs7749394 | 0.0056 | A/G | A (0.6286) | 6 | 119451228 | 0.23 | 1.75 (0.74,4.3) |
| rs70156 | 0.0056 | A/C | C (0.1524) | 9 | 122504061 | 0.1 | 2.57 (0.77,9.39) |
| rs7459733 | 0.0056 | A/G | G (0.2905) | 8 | 132006059 | 0.01 | 3.17 (1.25,8.33) |
| rs2267698 | 0.0056 | A/G | A (0.9476) | 7 | 139069977 | 0.13 | 3.62 (0.56,39.69) |
| rs214955 | 0.0056 | A/G | A (0.5429) | 6 | 152789820 | 0.0018 | 3.61 (1.53,8.86) |
| rs6702896 | 0.0056 | A/G | G (0.2524) | 1 | 164915068 | 0.51 | 1.35 (0.51,3.55) |
| rs9456079 | 0.0056 | A/G | A (0.8857) | 6 | 168602007 | 0.012 | 9.56 (1.3,426.01) |
| rs6663324 | 0.0056 | A/G | G (0.919) | 1 | 201678421 | 0.13 | 3.62 (0.56,39.69) |
| rs7775946 | 0.0057 | A/G | A (0.2476) | 6 | 2688244 | 0.28 | 1.66 (0.66,4.22) |
| rs1218342 | 0.0057 | A/G | A (0.7524) | 10 | 14350005 | 0.045 | 2.83 (1.01,8.79) |
| rs4721864 | 0.0057 | A/G | G (0.381) | 7 | 19866881 | 0.0055 | 3.14 (1.33,7.65) |
| rs12447858 | 0.0057 | A/G | G (0.1553) | 16 | 20076334 | 0.053 | 2.71 (0.94,8.36) |
| rs1496759 | 0.0057 | A/G | G (0.2238) | 16 | 20528494 | 0.0041 | 4.91 (1.5,19.08) |
| rs3087812 | 0.0057 | A/G | G (0.2238) | 16 | 20542276 | 0.0041 | 4.91 (1.5,19.08) |
| rs151328 | 0.0057 | A/G | A (0.2238) | 16 | 20556203 | 0.0041 | 4.91 (1.5,19.08) |
| rs17795538 | 0.0057 | A/G | G (0.119) | 14 | 24327620 | 0.09 | 2.75 (0.76,11.29) |
| rs12887023 | 0.0057 | A/C | A (0.2376) | 14 | 28590335 | 0.016 | 3.01 (1.17,8) |
| rs641027 | 0.0057 | A/G | G (0.1333) | 13 | 38877197 | 0.027 | 4.71 (1.09,28.83) |
| rs9677398 | 0.0057 | A/C | C (0.7095) | 2 | 43545786 | 0.024 | 2.75 (1.09,7.37) |
| rs4564645 | 0.0057 | A/G | A (0.3476) | 18 | 43868534 | 0.065 | 2.19 (0.91,5.41) |
| rs763391 | 0.0057 | A/G | G (0.281) | 7 | 68361691 | 0.13 | 2 (0.79,5.11) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs17085151 | 0.0057 | A/G | G (0.0857) | 13 | 68945111 | 0.094 | 3.49 (0.74,22.16) |
| rs7100913 | 0.0057 | A/G | G (0.8654) | 10 | 71318322 | 0.0064 | 6.92 (1.48,65.88) |
| rs1812680 | 0.0057 | A/G | G (0.3524) | 18 | 73001653 | 0.0079 | 3.1 (1.28,7.73) |
| rs349363 | 0.0057 | A/G | A (0.226) | 8 | 73594355 | 0.0058 | 4.45 (1.45,15.49) |
| rs7907270 | 0.0057 | A/G | A (0.4476) | 10 | 78550949 | 0.33 | 1.57 (0.68,3.66) |
| rs1382775 | 0.0057 | A/G | G (0.481) | 11 | 79673362 | 0.12 | 1.88 (0.81,4.42) |
| rs9925302 | 0.0057 | A/G | G (0.8857) | 16 | 85223804 | 0.04 | 4.69 (0.95,45.79) |
| rs4458614 | 0.0057 | A/G | A (0.7333) | 5 | 90476902 | 0.0078 | 3.88 (1.35,12.96) |
| rs12134841 | 0.0057 | A/C | C (0.0728) | 1 | 90489738 | 0.02 | Inf (1.13,Inf) |
| rs1508065 | 0.0057 | A/G | G (0.9375) | 11 | 94421495 | 0.083 | 7.08 (0.75,345.31) |
| rs7711674 | 0.0057 | A/G | G (0.1476) | 5 | 117320167 | 0.1 | 2.57 (0.77,9.39) |
| rs1331621 | 0.0057 | A/G | A (0.3952) | 9 | 119253916 | 0.048 | 2.25 (0.95,5.38) |
| rs10231282 | 0.0057 | A/G | A (0.4476) | 7 | 123301588 | 0.011 | 2.83 (1.2,6.89) |
| rs322812 | 0.0057 | A/G | G (0.4327) | 7 | 127338738 | 0.017 | 2.78 (1.18,6.73) |
| rs2031205 | 0.0057 | A/G | G (0.8524) | 6 | 131751634 | 0.01 | 4.92 (1.28,28.16) |
| rs6464111 | 0.0057 | A/G | G (0.281) | 7 | 149968521 | 0.035 | 2.61 (1.06,6.62) |
| rs717687 | 0.0057 | A/G | G (0.4854) | 6 | 153341329 | 0.01 | 2.89 (1.21,7.05) |
| rs10428211 | 0.0057 | A/G | G (0.3905) | 3 | 194437588 | 0.012 | 2.75 (1.18,6.58) |
| rs4370312 | 0.0058 | A/G | A (0.1058) | 5 | 1172722 | 0.065 | 2.8 (0.86,10.11) |
| rs4669825 | 0.0058 | A/G | G (0.476) | 2 | 12271667 | 0.033 | 2.41 (1.03,5.78) |
| rs10500796 | 0.0058 | A/G | A (0.7714) | 11 | 13655409 | 0.0063 | 4.15 (1.35,15.46) |
| rs1820446 | 0.0058 | A/C | C (0.6095) | 15 | 32693440 | 0.032 | 2.42 (1.02,5.92) |
| rs3194051 | 0.0058 | A/G | G (0.2714) | 5 | 35912031 | 0.018 | 2.9 (1.14,7.65) |
| rs10491434 | 0.0058 | A/G | G (0.2714) | 5 | 35913671 | 0.018 | 2.9 (1.14,7.65) |
| rs150795 | 0.0058 | A/G | G (0.4) | 21 | 42335448 | 0.048 | 2.27 (0.97,5.42) |
| rs1893948 | 0.0058 | A/G | G (0.6111) | 18 | 43963551 | 0.00034 | 4.45 (1.83,11.28) |
| rs1560525 | 0.0058 | A/C | C (0.4471) | 13 | 46598335 | 0.0066 | 3.03 (1.28,7.38) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs11172406 | 0.0058 | A/C | C (0.6394) | 12 | 56652873 | 0.31 | 1.63 (0.68,4.01) |
| rs706411 | 0.0058 | A/G | G (0.4667) | 1 | 58004642 | 0.08 | 2.05 (0.87,4.96) |
| rs1445978 | 0.0058 | A/G | G (0.6286) | 5 | 60890827 | 0.017 | 2.77 (1.12,7.19) |
| rs1561398 | 0.0058 | A/G | G (0.719) | 5 | 71522541 | 0.0013 | 5.69 (1.72,24.72) |
| rs6453597 | 0.0058 | A/G | G (0.9429) | 6 | 73469609 | 0.04 | 8.98 (1.03,426.65) |
| rs2951922 | 0.0058 | A/G | A (0.3932) | 6 | 76219570 | 0.007 | 3.25 (1.32,8.22) |
| rs9649304 | 0.0058 | A/G | G (0.0857) | 7 | 77737325 | 0.012 | 9.56 (1.3,426.01) |
| rs11647213 | 0.0058 | A/G | G (0.8143) | 16 | 85228015 | 0.01 | 4.92 (1.28,28.16) |
| rs7858600 | 0.0058 | A/G | A (0.481) | 9 | 102605917 | 0.02 | 2.57 (1.1,6.19) |
| rs9520220 | 0.0058 | A/G | A (0.4663) | 13 | 106307637 | 0.031 | 2.48 (1.06,5.94) |
| rs3803167 | 0.0058 | A/G | A (0.7885) | 12 | 110248306 | 0.01 | 4.05 (1.31,15.17) |
| rs11195191 | 0.0058 | A/G | G (0.0952) | 10 | 112325746 | 0.094 | 3.49 (0.74,22.16) |
| rs27634 | 0.0058 | A/G | A (0.2228) | 5 | 115722976 | 0.15 | 2.13 (0.74,6.35) |
| rs709611 | 0.0058 | A/G | G (0.7476) | 9 | 130077913 | 0.19 | 1.93 (0.73,5.48) |
| rs2501879 | 0.0058 | A/G | G (0.2476) | 1 | 158048038 | 0.064 | 2.49 (0.92,7.05) |
| rs6427703 | 0.0058 | A/G | G (0.3725) | 1 | 159666350 | 0.094 | 2.1 (0.85,5.26) |
| rs9815702 | 0.0058 | A/G | G (0.6779) | 3 | 165217916 | 0.00012 | 6.59 (2.18,24.37) |
| rs6890425 | 0.0058 | A/G | A (0.3048) | 5 | 177359136 | 0.15 | 1.84 (0.75,4.56) |
| rs1447322 | 0.0058 | A/G | A (0.3214) | 4 | 181531305 | 0.0093 | 3.21 (1.23,8.76) |
| rs6815342 | 0.0058 | A/G | A (0.1524) | 4 | 187320080 | 0.053 | 2.63 (0.9,8.18) |
| rs6725303 | 0.0058 | A/C | C (0.7308) | 2 | 211179533 | 0.0096 | 3.48 (1.26,10.73) |
| rs219870 | 0.0059 | A/C | C (0.3667) | 20 | 9550875 | 0.0055 | 3.12 (1.31,7.63) |
| rs745181 | 0.0059 | A/G | A (0.6333) | 11 | 11305404 | 0.01 | 2.99 (1.24,7.59) |
| rs2416678 | 0.0059 | A/G | G (0.1649) | 12 | 11448926 | 0.041 | 2.96 (0.95,10) |
| rs7867305 | 0.0059 | A/C | C (0.2621) | 9 | 12778784 | 0.0015 | 4.02 (1.57,10.84) |
| rs6105055 | 0.0059 | A/G | A (0.1095) | 20 | 13168348 | 0.0019 | 14.52 (1.88,658.49) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs707821 | 0.0059 | A/G | G (0.8857) | 6 | 14778866 | 0.0046 | Inf (1.84,Inf) |
| rs2102623 | 0.0059 | A/G | A (0.119) | 3 | 18181169 | 0.027 | 4.71 (1.09,28.83) |
| rs6132146 | 0.0059 | A/G | A (0.5095) | 20 | 18930974 | 0.011 | 2.83 (1.2,6.89) |
| rs10490750 | 0.0059 | A/G | A (0.7621) | 2 | 23623216 | 0.0013 | 5.7 (1.71,24.87) |
| rs2516400 | 0.0059 | A/G | A (0.3265) | 6 | 31589084 | 0.031 | 2.7 (1.07,7.03) |
| rs897506 | 0.0059 | A/C | C (0.7762) | 2 | 33698163 | 0.0063 | 4.15 (1.35,15.46) |
| rs2862999 | 0.0059 | A/G | G (0.2048) | 11 | 43848270 | 0.015 | 3.4 (1.14,11.05) |
| rs135796 | 0.0059 | A/G | G (0.0931) | 22 | 48268650 | 0.14 | 2.7 (0.74,11.15) |
| rs1407440 | 0.0059 | A/G | A (0.1346) | 13 | 49184219 | 0.12 | 2.92 (0.72,14.2) |
| rs5743289 | 0.0059 | A/G | A (0.1714) | 16 | 49314275 | 0.2 | 2.11 (0.69,6.69) |
| rs890869 | 0.0059 | A/G | A (0.181) | 19 | 60324855 | 0.0041 | 4.91 (1.5,19.08) |
| rs16927963 | 0.0059 | A/G | A (0.8714) | 10 | 72205498 | 0.023 | 5.21 (1.07,50.48) |
| rs1715489 | 0.0059 | A/G | G (0.1619) | 17 | 72790904 | 0.061 | 2.9 (0.89,10.47) |
| rs2246456 | 0.0059 | A/C | C (0.7524) | 8 | 73745292 | 0.032 | 2.78 (1.04,8.1) |
| rs1795970 | 0.0059 | A/G | G (0.2857) | 12 | 77372865 | 0.13 | 2 (0.79,5.11) |
| rs2067860 | 0.0059 | A/G | A (0.1333) | 16 | 82189931 | 0.0083 | 7.17 (1.38,71.92) |
| rs4773567 | 0.0059 | A/G | G (0.1381) | 13 | 89738714 | 0.051 | 4.09 (0.91,25.4) |
| rs10512379 | 0.0059 | A/G | A (0.1143) | 9 | 107599983 | 0.0019 | 14.52 (1.88,658.49) |
| rs2724661 | 0.0059 | A/G | G (0.681) | 11 | 123560412 | 0.042 | 2.43 (0.99,6.33) |
| rs948071 | 0.0059 | A/G | A (0.6905) | 11 | 123590672 | 0.042 | 2.43 (0.99,6.33) |
| rs322840 | 0.0059 | A/G | G (0.6442) | 7 | 127302926 | 0.005 | 3.37 (1.37,8.81) |
| rs6857927 | 0.0059 | A/C | C (0.4952) | 4 | 162617834 | 0.053 | 2.19 (0.95,5.22) |
| rs9685165 | 0.0059 | A/C | C (0.2067) | 4 | 175918687 | 0.0031 | 4.73 (1.55,16.38) |
| rs10911812 | 0.0059 | A/C | C (0.4615) | 1 | 182779865 | 0.031 | 2.43 (1.03,5.87) |
| rs12477557 | 0.0059 | A/C | A (0.2) | 2 | 199062376 | 0.0045 | 3.61 (1.37,10.03) |
| rs1569175 | 0.0059 | A/G | G (0.0714) | 2 | 200847460 | 0.075 | 6.58 (0.83,301.83) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs10754661 | 0.0059 | A/G | G (0.2095) | 1 | 235527214 | 0.15 | 2.06 (0.71,6.2) |
| rs7479065 | 0.006 | A/G | G (0.719) | 11 | 2025293 | 0.13 | 2.09 (0.8,5.88) |
| rs3843924 | 0.006 | A/G | A (0.5952) | 8 | 4065461 | 0.028 | 2.63 (1.09,6.68) |
| rs9313264 | 0.006 | A/G | A (0.2762) | 5 | 9014561 | 0.0034 | 4.09 (1.52,11.75) |
| rs6871960 | 0.006 | A/C | C (0.2762) | 5 | 9016871 | 0.0034 | 4.09 (1.52,11.75) |
| rs6880494 | 0.006 | A/C | C (0.2762) | 5 | 9030180 | 0.0034 | 4.09 (1.52,11.75) |
| rs3777253 | 0.006 | A/C | C (0.8667) | 5 | 9174591 | 6.00E-05 | Inf (3.54,Inf) |
| rs1399490 | 0.006 | A/G | A (0.8238) | 7 | 9592850 | 0.026 | 3.61 (1.05,16.04) |
| rs3765896 | 0.006 | A/G | G (0.1524) | 1 | 11008686 | 0.039 | 3.97 (1.05,18.68) |
| rs7098491 | 0.006 | A/C | A (0.0952) | 10 | 11864856 | 0.0096 | Inf (1.59,Inf) |
| rs1858941 | 0.006 | A/G | G (0.4471) | 7 | 20409719 | 0.018 | 2.77 (1.16,6.83) |
| rs6840416 | 0.006 | A/G | A (0.1143) | 4 | 25676864 | 0.2 | 2.58 (0.61,12.85) |
| rs12349820 | 0.006 | A/G | A (0.7238) | 9 | 27543876 | 0.047 | 2.63 (0.94,8.21) |
| rs313153 | 0.006 | A/G | G (0.1714) | 1 | 28155585 | 0.043 | 3.06 (1.01,10.05) |
| rs2957932 | 0.006 | A/G | G (0.7333) | 17 | 43685183 | 0.016 | 3.25 (1.18,10.05) |
| rs979746 | 0.006 | A/C | C (0.7333) | 17 | 43691111 | 0.016 | 3.25 (1.18,10.05) |
| rs11831940 | 0.006 | A/G | G (0.699) | 12 | 46476433 | 0.02 | 2.95 (1.12,8.31) |
| rs8082489 | 0.006 | A/G | G (0.5146) | 17 | 51470724 | 0.017 | 2.76 (1.17,6.72) |
| rs4412789 | 0.006 | A/G | A (0.7095) | 12 | 58528735 | 0.027 | 3.03 (1.09,9.4) |
| rs1480298 | 0.006 | A/G | A (0.3107) | 4 | 58568166 | 0.033 | 2.58 (1.03,6.61) |
| rs8103535 | 0.006 | A/C | A (0.2381) | 19 | 58853143 | 0.13 | 2 (0.78,5.22) |
| rs8103787 | 0.006 | A/G | A (0.2381) | 19 | 58853397 | 0.13 | 2 (0.78,5.22) |
| rs10191517 | 0.006 | A/G | G (0.7048) | 2 | 73470058 | 0.02 | 2.97 (1.12,8.65) |
| rs1410569 | 0.006 | A/G | G (0.7952) | 9 | 75536154 | 0.024 | 3.14 (1.08,10.57) |
| rs1479312 | 0.006 | A/G | A (0.919) | 11 | 79659821 | 0.094 | 3.49 (0.74,22.16) |
| rs4782607 | 0.006 | A/G | A (0.081) | 16 | 82911084 | 0.023 | 5.21 (1.07,50.48) |
| rs10016927 | 0.006 | A/C | C (0.4048) | 4 | 96922290 | 0.003 | 3.48 (1.47,8.49) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs4494277 | 0.006 | A/C | A (0.7) | 11 | 97309293 | 0.024 | 2.75 (1.09,7.37) |
| rs1943783 | 0.006 | A/G | A (0.4952) | 11 | 101863317 | 0.052 | 2.23 (0.96,5.28) |
| rs12338452 | 0.006 | A/G | A (0.0619) | 9 | 105011441 | 0.02 | Inf (1.13,Inf) |
| rs1808973 | 0.006 | A/G | A (0.481) | 1 | 112199876 | 0.08 | 2.09 (0.9,4.92) |
| rs1807211 | 0.006 | A/G | A (0.6524) | 1 | 112202490 | 0.042 | 2.43 (0.99,6.33) |
| rs1181770 | 0.006 | A/G | G (0.7619) | 7 | 140604578 | 0.014 | 4.24 (1.26,18.66) |
| rs2366240 | 0.006 | A/C | C (0.7667) | 7 | 140610332 | 0.014 | 4.24 (1.26,18.66) |
| rs12196375 | 0.006 | A/C | C (0.86) | 6 | 148754343 | 0.0063 | 6.89 (1.46,65.8) |
| rs2340252 | 0.006 | A/C | A (0.5048) | 4 | 157486823 | 0.081 | 2.06 (0.89,4.88) |
| rs13781 | 0.006 | A/G | A (0.2762) | 2 | 170764875 | 0.00048 | 4.87 (1.83,13.95) |
| rs10000456 | 0.006 | A/G | G (0.1875) | 4 | 176024671 | 0.035 | 3.26 (1.02,11.61) |
| rs6836502 | 0.006 | A/C | A (0.0619) | 4 | 181250382 | 0.4 | 2.83 (0.39,32.62) |
| rs2121639 | 0.006 | A/G | G (0.6143) | 4 | 181551448 | 0.032 | 2.42 (1.02,5.92) |
| rs7626795 | 0.006 | A/G | G (0.1238) | 3 | 191833163 | 0.17 | 2.25 (0.65,8.38) |
| rs6749960 | 0.006 | A/G | A (0.8476) | 2 | 197624630 | 0.07 | 3.03 (0.86,13.64) |
| rs2376187 | 0.006 | A/G | G (0.8476) | 2 | 197641790 | 0.07 | 3.03 (0.86,13.64) |
| rs10924375 | 0.006 | A/G | G (0.2667) | 1 | 242370122 | 0.016 | 2.95 (1.14,7.98) |
| rs2709064 | 0.0061 | A/G | A (0.5571) | 7 | 9315239 | 0.0062 | 3.13 (1.32,7.71) |
| rs8070243 | 0.0061 | A/G | A (0.4333) | 17 | 11692776 | 0.033 | 2.34 (1.01,5.56) |
| rs12192359 | 0.0061 | A/G | A (0.3714) | 6 | 13272634 | 0.05 | 2.42 (0.94,6.43) |
| rs10244904 | 0.0061 | A/G | G (0.7952) | 7 | 19812503 | 0.014 | 4.24 (1.26,18.66) |
| rs2749817 | 0.0061 | A/G | G (0.6857) | 20 | 23007255 | 0.0087 | 3.13 (1.25,8.37) |
| rs1892775 | 0.0061 | A/G | G (0.4381) | 21 | 32071786 | 0.072 | 2.21 (0.92,5.51) |
| rs2834368 | 0.0061 | A/G | G (0.1333) | 21 | 34341060 | 0.12 | 3.02 (0.75,14.69) |
| rs11170890 | 0.0061 | A/G | G (0.8571) | 12 | 37835456 | 0.022 | 5.76 (1.2,55.34) |
| rs9380854 | 0.0061 | A/G | A (0.0714) | 6 | 39465235 | 0.02 | Inf (1.36,Inf) |
| rs7165971 | 0.0061 | A/G | A (0.6905) | 15 | 53708305 | 0.099 | 2.13 (0.86,5.57) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs885355 | 0.0061 | A/G | G (0.2184) | 14 | 58633058 | 0.054 | 2.72 (0.96,8.06) |
| rs3825073 | 0.0061 | A/C | A (0.1346) | 11 | 64656846 | 0.0037 | 6.59 (1.62,38.97) |
| rs10131712 | 0.0061 | A/G | A (0.8905) | 14 | 69425371 | 0.0022 | Inf (2.1,Inf) |
| rs10957512 | 0.0061 | A/G | A (0.7952) | 8 | 71053049 | 0.045 | 2.83 (1.01,8.79) |
| rs1821016 | 0.0061 | A/G | G (0.0905) | 8 | 77322321 | 0.02 | Inf (1.13,Inf) |
| rs607345 | 0.0061 | A/G | A (0.4238) | 1 | 85108179 | 0.0055 | 3.12 (1.31,7.63) |
| rs4571541 | 0.0061 | A/G | A (0.3095) | 6 | 92885445 | 0.2 | 1.83 (0.73,4.6) |
| rs854561 | 0.0061 | A/G | A (0.3702) | 7 | 94591668 | 0.071 | 2.11 (0.88,5.16) |
| rs7136446 | 0.0061 | A/G | G (0.3667) | 12 | 101340982 | 0.0047 | 3.38 (1.41,8.37) |
| rs2375016 | 0.0061 | A/G | G (0.3524) | 12 | 107730380 | 0.048 | 2.25 (0.95,5.38) |
| rs17127145 | 0.0061 | A/G | A (0.9363) | 10 | 111920351 | 0.081 | 7.43 (0.79,362.98) |
| rs7077408 | 0.0061 | A/G | A (0.0619) | 10 | 118218753 | 0.02 | Inf (1.13,Inf) |
| rs1892968 | 0.0061 | A/G | A (0.3524) | 11 | 122815590 | 0.1 | 2.02 (0.84,4.94) |
| rs2479104 | 0.0061 | A/G | A (0.8762) | 9 | 123664655 | 0.054 | 3.76 (0.95,21.94) |
| rs9324820 | 0.0061 | A/G | G (0.9095) | 5 | 154793593 | 0.0066 | 10.63 (1.46,470.67) |
| rs4233808 | 0.0061 | A/G | G (0.1143) | 2 | 167300989 | 0.0071 | 6.08 (1.47,36.22) |
| rs1013729 | 0.0061 | A/C | C (0.2714) | 4 | 181883830 | 0.39 | 1.53 (0.61,3.82) |
| rs716756 | 0.0061 | A/G | G (0.4667) | 1 | 182802559 | 0.033 | 2.41 (1.03,5.78) |
| rs720971 | 0.0061 | A/G | G (0.4667) | 1 | 182817425 | 0.033 | 2.41 (1.03,5.78) |
| rs3741974 | 0.0062 | A/G | A (0.2429) | 12 | 1784534 | 0.035 | 2.82 (1.02,8.26) |
| rs3856852 | 0.0062 | A/G | G (0.8619) | 3 | 3206647 | 0.0035 | 11.74 (1.64,517.13) |
| rs17168763 | 0.0062 | A/G | G (0.881) | 7 | 15276553 | 0.07 | 3.03 (0.86,13.64) |
| rs12031938 | 0.0062 | A/C | A (0.2524) | 1 | 18302045 | 0.015 | 3.4 (1.14,11.05) |
| rs2285690 | 0.0062 | A/G | A (0.3269) | 7 | 24676002 | 0.045 | 2.31 (0.97,5.61) |
| rs2383811 | 0.0062 | A/G | G (0.0952) | 9 | 29406253 | 0.039 | 3.97 (1.05,18.68) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs136067 | 0.0062 | A/G | G (0.7429) | 22 | 45664516 | 0.079 | 2.41 (0.89,7.09) |
| rs8057505 | 0.0062 | A/G | G (0.8835) | 16 | 48262130 | 0.053 | 3.92 (0.98,22.88) |
| rs131718 | 0.0062 | A/G | G (0.3204) | 22 | 49348067 | 0.27 | 1.69 (0.65,4.41) |
| rs1538881 | 0.0062 | A/G | A (0.5817) | 1 | 52049060 | 0.01 | 2.91 (1.24,7.03) |
| rs2427032 | 0.0062 | A/G | G (0.1538) | 20 | 59297367 | 0.13 | 2.36 (0.79,7.41) |
| rs1071646 | 0.0062 | A/C | C (0.3429) | 15 | 61138893 | 0.1 | 2.04 (0.86,4.93) |
| rs17158154 | 0.0062 | A/C | A (0.1571) | 11 | 62876062 | 0.17 | 2.25 (0.65,8.38) |
| rs1927819 | 0.0062 | A/G | G (0.119) | 13 | 66664262 | 0.072 | 2.75 (0.89,9.1) |
| rs7207590 | 0.0062 | A/C | C (0.2794) | 17 | 69011846 | 0.0056 | 3.96 (1.42,11.98) |
| rs2808207 | 0.0062 | A/G | G (0.3952) | 6 | 76186935 | 0.0084 | 3.1 (1.29,7.66) |
| rs11948804 | 0.0062 | A/G | A (0.1095) | 5 | 78839534 | 0.12 | 3.02 (0.75,14.69) |
| rs2787942 | 0.0062 | A/G | G (0.1286) | 6 | 88496102 | 0.072 | 2.75 (0.89,9.1) |
| rs11020995 | 0.0062 | A/C | C (0.7163) | 11 | 94356646 | 0.012 | 3.18 (1.2,9.23) |
| rs7330977 | 0.0062 | A/C | C (0.3714) | 13 | 101535664 | 0.0021 | 3.69 (1.51,9.36) |
| rs316969 | 0.0062 | A/G | A (0.9143) | 1 | 104774715 | 0.07 | 4.44 (0.75,47.1) |
| rs1264901 | 0.0062 | A/G | A (0.9429) | 1 | 111699077 | 0.13 | 3.62 (0.56,39.69) |
| rs2146762 | 0.0062 | A/G | A (0.7762) | 6 | 119419347 | 0.37 | 1.67 (0.62,4.76) |
| rs10518319 | 0.0062 | A/G | G (0.1476) | 4 | 120169800 | 0.17 | 2.25 (0.65,8.38) |
| rs10819303 | 0.0062 | A/G | A (0.5667) | 9 | 127472508 | 0.0065 | 3 (1.28,7.24) |
| rs2402964 | 0.0062 | A/G | A (0.0714) | 7 | 128985109 | 0.041 | 7.53 (0.98,341.68) |
| rs6888242 | 0.0062 | A/G | A (0.3429) | 5 | 160964402 | 0.01 | 3.17 (1.25,8.33) |
| rs4365431 | 0.0062 | A/G | G (0.1286) | 2 | 168611925 | 0.052 | 3.13 (0.89,12.67) |
| rs973573 | 0.0062 | A/C | C (0.0905) | 4 | 188486207 | 0.01 | 10.74 (1.3,500.03) |
| rs13375803 | 0.0062 | A/G | G (0.9) | 1 | 205312949 | 0.0022 | Inf (2.1,Inf) |
| rs10497942 | 0.0062 | A/G | A (0.1476) | 2 | 211779425 | 0.79 | 1.24 (0.38,3.99) |
| rs1183141 | 0.0063 | A/G | G (0.8476) | 17 | 5785318 | 0.043 | 3.32 (0.96,14.81) |
| rs4702698 | 0.0063 | A/G | A (0.6619) | 5 | 10570908 | 0.032 | 2.78 (1.04,8.1) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs4910411 | 0.0063 | A/G | A (0.0571) | 11 | 11676195 | 0.0049 | Inf (1.7,Inf) |
| rs4237447 | 0.0063 | A/G | A (0.1505) | 10 | 15286352 | 0.15 | 2.06 (0.71,6.2) |
| rs249116 | 0.0063 | A/G | A (0.1952) | 5 | 17005807 | 0.016 | 3.97 (1.18,15.68) |
| rs10840745 | 0.0063 | A/C | A (0.4216) | 12 | 17601788 | 0.01 | 2.89 (1.21,7.05) |
| rs38147 | 0.0063 | A/G | G (0.6333) | 7 | 19825783 | 0.024 | 2.75 (1.09,7.37) |
| rs1534649 | 0.0063 | A/C | A (0.5429) | 8 | 19843921 | 0.012 | 2.75 (1.18,6.58) |
| rs1332180 | 0.0063 | A/G | G (0.919) | 9 | 21403085 | 0.2 | 2.58 (0.61,12.85) |
| rs12419174 | 0.0063 | A/G | A (0.9356) | 11 | 36515854 | 0.074 | 4.95 (0.88,51.31) |
| rs1998612 | 0.0063 | A/C | A (0.2333) | 13 | 42822997 | 0.016 | 2.95 (1.14,7.98) |
| rs6007220 | 0.0063 | A/G | A (0.1286) | 22 | 43347041 | 0.26 | 1.95 (0.54,7.42) |
| rs10857437 | 0.0063 | A/G | A (0.1667) | 10 | 50051927 | 0.061 | 2.9 (0.89,10.47) |
| rs346420 | 0.0063 | A/C | C (0.1) | 5 | 62564968 | 0.018 | 5.05 (1.21,30.27) |
| rs4529740 | 0.0063 | A/G | G (0.8667) | 1 | 63909062 | 0.043 | 3.32 (0.96,14.81) |
| rs755764 | 0.0063 | A/G | G (0.8476) | 8 | 65345636 | 0.01 | 4.92 (1.28,28.16) |
| rs12737233 | 0.0063 | A/G | G (0.181) | 1 | 70496956 | 0.035 | 3.26 (1.02,11.61) |
| rs10518334 | 0.0063 | A/G | G (0.181) | 1 | 70520289 | 0.035 | 3.26 (1.02,11.61) |
| rs495593 | 0.0063 | A/G | G (0.2381) | 13 | 71817801 | 0.17 | 2.01 (0.75,5.58) |
| rs798396 | 0.0063 | A/G | A (0.4571) | 12 | 76316841 | 0.12 | 1.99 (0.85,4.75) |
| rs2035620 | 0.0063 | A/G | G (0.0905) | 2 | 77940211 | 0.2 | 2.58 (0.61,12.85) |
| rs4693421 | 0.0063 | A/G | A (0.5667) | 4 | 83376575 | 0.0058 | 3.19 (1.32,8.1) |
| rs1992623 | 0.0063 | A/C | A (0.2952) | 16 | 85203281 | 0.0092 | 3.22 (1.25,8.7) |
| rs10249530 | 0.0063 | A/G | G (0.2714) | 7 | 90636972 | 0.04 | 2.39 (0.98,5.97) |
| rs1371357 | 0.0063 | A/G | A (0.1683) | 4 | 94980827 | 0.026 | 3.27 (1.1,10.67) |
| rs1954850 | 0.0063 | A/G | G (0.219) | 11 | 101883770 | 0.17 | 2.01 (0.75,5.58) |
| rs1766783 | 0.0063 | A/C | A (0.1905) | 1 | 119146938 | 0.23 | 1.82 (0.64,5.3) |
| rs10510103 | 0.0063 | A/G | G (0.3333) | 10 | 123614500 | 0.00045 | 4.48 (1.79,11.77) |
| rs2542615 | 0.0063 | A/G | A (0.6714) | 10 | 131018942 | 0.00042 | 5.14 (1.89,15.77) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2462870 | 0.0063 | A/C | A (0.2885) | 4 | 138954760 | 0.011 | 3.41 (1.25,9.87) |
| rs968538 | 0.0063 | A/G | A (0.6048) | 2 | 193305641 | 0.17 | 1.75 (0.75,4.17) |
| rs12117400 | 0.0063 | A/G | G (0.9231) | 1 | 212073589 | 0.13 | 3.62 (0.56,39.69) |
| rs4719607 | 0.0064 | A/G | A (0.649) | 7 | 2433192 | 0.0019 | 4.1 (1.55,11.93) |
| rs12506821 | 0.0064 | A/G | G (0.119) | 4 | 3321529 | 0.35 | 2.03 (0.51,8.75) |
| rs2327841 | 0.0064 | A/C | C (0.3048) | 20 | 14185257 | 0.15 | 1.84 (0.75,4.56) |
| rs10495650 | 0.0064 | A/C | C (0.1381) | 2 | 16464861 | 0.0038 | 8.18 (1.61,80.71) |
| rs9509036 | 0.0064 | A/C | C (0.7714) | 13 | 19576392 | 0.0063 | 4.15 (1.35,15.46) |
| rs964189 | 0.0064 | A/G | G (0.3619) | 8 | 20752615 | 0.16 | 1.89 (0.8,4.53) |
| rs7070284 | 0.0064 | A/G | A (0.5952) | 10 | 23193356 | 0.08 | 2.05 (0.87,4.96) |
| rs2886153 | 0.0064 | A/G | A (0.5952) | 10 | 23226630 | 0.08 | 2.05 (0.87,4.96) |
| rs4330872 | 0.0064 | A/G | A (0.881) | 1 | 24221676 | 0.11 | 3.7 (0.71,36.88) |
| rs10969885 | 0.0064 | A/G | G (0.625) | 9 | 30830471 | 0.034 | 2.75 (1.06,7.71) |
| rs1352774 | 0.0064 | A/G | G (0.5619) | 4 | 32003144 | 0.069 | 2.22 (0.94,5.37) |
| rs1570211 | 0.0064 | A/G | A (0.9231) | 14 | 33025576 | 0.083 | 7.08 (0.75,345.31) |
| rs1413611 | 0.0064 | A/G | G (0.419) | 10 | 36506897 | 0.03 | 2.52 (1.08,6) |
| rs2304102 | 0.0064 | A/G | G (0.6619) | 19 | 38159197 | 0.015 | 2.93 (1.17,7.85) |
| rs179271 | 0.0064 | A/G | A (0.5762) | 6 | 39187081 | 0.12 | 1.93 (0.83,4.57) |
| rs4812279 | 0.0064 | A/C | C (0.399) | 20 | 59210886 | 0.24 | 1.6 (0.69,3.76) |
| rs9883195 | 0.0064 | A/C | A (0.9327) | 3 | 59435360 | 0.081 | 7.32 (0.78,356.8) |
| rs10515181 | 0.0064 | A/G | G (0.8524) | 5 | 73751240 | 0.026 | 3.61 (1.05,16.04) |
| rs6467917 | 0.0064 | A/G | A (0.581) | 7 | 82214308 | 0.12 | 1.93 (0.82,4.65) |
| rs4916737 | 0.0064 | A/C | C (0.6048) | 5 | 88326523 | 0.011 | 2.86 (1.2,7.13) |
| rs362732 | 0.0064 | A/G | G (0.3333) | 7 | 102804019 | 0.002 | 3.72 (1.5,9.58) |
| rs156372 | 0.0064 | A/G | A (0.1714) | 5 | 174888400 | 0.006 | 5.04 (1.39,23.16) |
| rs2940527 | 0.0064 | A/G | G (0.1346) | 5 | 176144896 | 0.57 | 1.48 (0.41,5.41) |
| rs2362470 | 0.0065 | A/G | G (0.2596) | 12 | 5742820 | 0.19 | 1.81 (0.7,4.77) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs3021387 | 0.0065 | A/G | A (0.2304) | 2 | 8443521 | 0.0068 | 4.01 (1.36,13.01) |
| rs2456237 | 0.0065 | A/G | A (0.4619) | 5 | 9158283 | 0.019 | 2.68 (1.15,6.41) |
| rs2268115 | 0.0065 | A/C | A (0.4439) | 12 | 13760992 | 0.0088 | 3.11 (1.28,7.91) |
| rs7238647 | 0.0065 | A/G | A (0.4714) | 18 | 14017616 | 0.01 | 2.91 (1.24,7.03) |
| rs12149474 | 0.0065 | A/G | A (0.1429) | 16 | 25676665 | 0.061 | 2.9 (0.89,10.47) |
| rs7132644 | 0.0065 | A/G | G (0.8429) | 12 | 27053543 | 0.054 | 3.76 (0.95,21.94) |
| rs7146149 | 0.0065 | A/C | C (0.7286) | 14 | 28027974 | 0.037 | 2.57 (0.99,7.17) |
| rs9573959 | 0.0065 | A/G | G (0.2019) | 13 | 34934119 | 0.074 | 2.65 (0.86,8.78) |
| rs17406451 | 0.0065 | A/C | C (0.6524) | 2 | 43544127 | 0.016 | 2.95 (1.2,7.67) |
| rs2278868 | 0.0065 | A/G | A (0.4619) | 17 | 43617170 | 0.0019 | 3.57 (1.5,8.86) |
| rs8072282 | 0.0065 | A/G | G (0.4619) | 17 | 43631857 | 0.0019 | 3.57 (1.5,8.86) |
| rs4714885 | 0.0065 | A/G | A (0.7048) | 6 | 45963492 | 0.00083 | 4.82 (1.77,14.8) |
| rs5767764 | 0.0065 | A/G | A (0.5437) | 22 | 46220364 | 0.0029 | 3.46 (1.44,8.55) |
| rs1992298 | 0.0065 | A/G | A (0.1476) | 1 | 47898788 | 0.18 | 2.17 (0.67,7.37) |
| rs16945724 | 0.0065 | A/G | G (0.0625) | 17 | 57372670 | 0.0046 | Inf (1.84,Inf) |
| rs3745437 | 0.0065 | A/G | G (0.0762) | 19 | 59663041 | 0.13 | 5.66 (0.69,263.42) |
| rs308576 | 0.0065 | A/G | G (0.1584) | 13 | 61696876 | 0.0037 | 5.23 (1.59,20.4) |
| rs2367536 | 0.0065 | A/G | G (0.3048) | 17 | 66975870 | 0.003 | 3.77 (1.5,9.89) |
| rs877874 | 0.0065 | A/G | G (0.2157) | 17 | 75450416 | 0.039 | 2.66 (0.98,7.57) |
| rs4497612 | 0.0065 | A/G | G (0.1095) | 14 | 77130762 | 0.35 | 2.03 (0.51,8.75) |
| rs9601665 | 0.0065 | A/G | A (0.2048) | 13 | 81128027 | 0.00091 | 7.6 (1.9,44.59) |
| rs6936512 | 0.0065 | A/G | A (0.8524) | 6 | 85789267 | 0.025 | 3.92 (1.15,17.32) |
| rs926784 | 0.0065 | A/G | G (0.8029) | 14 | 96656675 | 0.34 | 1.77 (0.6,5.67) |
| rs12256664 | 0.0065 | A/C | A (0.9135) | 10 | 107359087 | 0.0043 | 7.91 (1.56,78.19) |
| rs7897726 | 0.0065 | A/G | G (0.7381) | 10 | 108733540 | 0.18 | 1.93 (0.7,5.74) |
| rs4870858 | 0.0065 | A/C | C (0.7115) | 8 | 124670174 | 0.0078 | 3.88 (1.35,12.96) |
| rs9375400 | 0.0065 | A/G | G (0.2762) | 6 | 125889153 | 0.5 | 1.48 (0.55,3.96) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs13387889 | 0.0065 | A/G | A (0.1667) | 2 | 128604438 | 0.015 | 5.2 (1.23,31.35) |
| rs4926386 | 0.0065 | A/C | A (0.0909) | 1 | 146699208 | 0.019 | 6.01 (1.12,61.09) |
| rs164122 | 0.0065 | A/G | G (0.649) | 1 | 159100015 | 0.0043 | 3.57 (1.39,9.89) |
| rs4862224 | 0.0065 | A/C | A (0.2624) | 4 | 184880945 | 0.013 | 3.74 (1.2,13.15) |
| rs10494662 | 0.0065 | A/G | G (0.9375) | 1 | 188507286 | 0.08 | 7.68 (0.82,375.05) |
| rs4973252 | 0.0065 | A/G | G (0.7212) | 2 | 230745884 | 0.0097 | 3.42 (1.24,10.62) |
| rs3809263 | 0.0066 | A/G | G (0.7143) | 12 | 643717 | 0.013 | 3.14 (1.22,8.7) |
| rs1817207 | 0.0066 | A/G | G (0.7476) | 11 | 5044288 | 0.015 | 3.38 (1.16,11.33) |
| rs3782631 | 0.0066 | A/G | G (0.5455) | 12 | 5666004 | 0.07 | 2.19 (0.92,5.34) |
| rs2290239 | 0.0066 | A/G | A (0.6905) | 12 | 7167525 | 0.15 | 2 (0.8,5.22) |
| rs6488801 | 0.0066 | A/C | C (0.3431) | 12 | 8017012 | 0.066 | 2.19 (0.9,5.42) |
| rs548304 | 0.0066 | A/G | A (0.2286) | 6 | 11745469 | 0.0084 | 3.3 (1.25,9.2) |
| rs243898 | 0.0066 | A/G | G (0.9135) | 20 | 13185714 | 0.098 | 3.37 (0.72,21.43) |
| rs1151924 | 0.0066 | A/G | A (0.9286) | 20 | 15505228 | 0.01 | 10.74 (1.3,500.03) |
| rs1998382 | 0.0066 | A/C | A (0.4167) | 20 | 18933842 | 0.019 | 2.61 (1.11,6.28) |
| rs2242540 | 0.0066 | A/G | G (0.5051) | 14 | 21480460 | 0.0015 | 3.84 (1.6,9.65) |
| rs9368310 | 0.0066 | A/G | A (0.519) | 6 | 21535122 | 0.052 | 2.23 (0.96,5.28) |
| rs12055945 | 0.0066 | A/G | G (0.755) | 7 | 22585564 | 0.49 | 1.47 (0.54,4.24) |
| rs5762049 | 0.0066 | A/C | C (0.5667) | 22 | 25986767 | 0.05 | 2.27 (0.96,5.55) |
| rs1546138 | 0.0066 | A/C | A (0.1287) | 5 | 28053573 | 0.0043 | 7.91 (1.56,78.19) |
| rs1540297 | 0.0066 | A/G | A (0.6476) | 22 | 34434965 | 0.12 | 1.93 (0.82,4.65) |
| rs11129738 | 0.0066 | A/G | G (0.2333) | 3 | 36876584 | 0.0082 | 3.75 (1.28,12.13) |
| rs6493393 | 0.0066 | A/G | A (0.519) | 15 | 48064237 | 0.032 | 2.44 (1.05,5.81) |
| rs2648005 | 0.0066 | A/G | A (0.5429) | 16 | 50124541 | 0.0029 | 3.43 (1.45,8.39) |
| rs11004401 | 0.0066 | A/G | G (0.8689) | 10 | 56024124 | 0.0046 | Inf (1.91,Inf) |
| rs9599648 | 0.0066 | A/G | A (0.3952) | 13 | 69826210 | 0.041 | 2.39 (0.99,5.9) |
| rs872470 | 0.0066 | A/G | G (0.0524) | 2 | 85440301 | 0.081 | 7.32 (0.78,356.8) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs11547160 | 0.0066 | A/G | A (0.0524) | 2 | 85448073 | 0.081 | 7.32 (0.78,356.8) |
| rs2273696 | 0.0066 | A/C | C (0.3762) | 10 | 102249696 | 0.042 | 2.41 (1.01,5.86) |
| rs11112661 | 0.0066 | A/G | G (0.4856) | 12 | 104582716 | 0.019 | 2.65 (1.13,6.44) |
| rs10502054 | 0.0066 | A/C | A (0.932) | 11 | 104747894 | 0.012 | Inf (1.3,Inf) |
| rs1834175 | 0.0066 | A/C | A (0.819) | 10 | 109667757 | 0.0034 | 7.53 (1.62,71.51) |
| rs4946069 | 0.0066 | A/G | G (0.3524) | 6 | 115697120 | 0.03 | 2.52 (1.08,6) |
| rs2660442 | 0.0066 | A/G | G (0.5571) | 4 | 142456078 | 0.011 | 2.86 (1.22,6.89) |
| rs537693 | 0.0066 | A/G | G (0.8762) | 6 | 159632547 | 0.012 | 6.33 (1.34,60.52) |
| rs811197 | 0.0066 | A/G | G (0.5381) | 3 | 175492753 | 0.033 | 2.34 (1.01,5.56) |
| rs1153702 | 0.0066 | A/G | A (0.6202) | 2 | 175746264 | 0.018 | 2.69 (1.12,6.73) |
| rs11132328 | 0.0066 | A/G | G (0.2571) | 4 | 186860442 | 0.088 | 2.19 (0.88,5.57) |
| rs9881941 | 0.0066 | A/C | A (0.4327) | 3 | 189368138 | 0.028 | 2.5 (1.05,6.04) |
| rs1933701 | 0.0066 | A/G | G (0.5048) | 1 | 189462162 | 0.034 | 2.34 (1.01,5.58) |
| rs17336419 | 0.0066 | A/C | C (0.2933) | 1 | 201608623 | 0.053 | 2.4 (0.95,6.23) |
| rs11594791 | 0.0067 | A/G | G (0.9286) | 10 | 3549601 | 0.13 | 3.62 (0.56,39.69) |
| rs1992203 | 0.0067 | A/C | A (0.1346) | 8 | 4744792 | 0.11 | 2.48 (0.74,9.07) |
| rs1537656 | 0.0067 | A/G | A (0.3238) | 6 | 8060391 | 0.13 | 2 (0.78,5.22) |
| rs4978113 | 0.0067 | A/G | G (0.219) | 9 | 21391106 | 0.13 | 2.36 (0.79,7.41) |
| rs2949528 | 0.0067 | A/G | G (0.6106) | 18 | 26041261 | 0.012 | 3.11 (1.2,8.67) |
| rs241707 | 0.0067 | A/G | G (0.4476) | 22 | 31703388 | 0.0058 | 3.19 (1.32,8.1) |
| rs1523785 | 0.0067 | A/G | G (0.7048) | 2 | 36649435 | 0.014 | 3.62 (1.25,12.12) |
| rs1304138 | 0.0067 | A/G | G (0.6571) | 3 | 39979370 | 0.028 | 2.63 (1.09,6.68) |
| rs3861062 | 0.0067 | A/C | A (0.898) | 11 | 44601861 | 0.13 | 3.22 (0.76,19.46) |
| rs1951479 | 0.0067 | A/G | G (0.4476) | 14 | 44806289 | 0.048 | 2.27 (0.97,5.42) |
| rs2350284 | 0.0067 | A/G | G (0.1905) | 6 | 65648275 | 0.072 | 2.75 (0.89,9.1) |
| rs7743515 | 0.0067 | A/G | G (0.1905) | 6 | 65653480 | 0.072 | 2.75 (0.89,9.1) |
| rs4074707 | 0.0067 | A/G | A (0.8619) | 3 | 67155642 | 0.032 | 4.13 (1.05,23.93) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs1804522 | 0.0067 | A/G | G (0.3073) | 17 | 73694638 | 0.031 | 2.63 (1.02,7.05) |
| rs4773668 | 0.0067 | A/G | A (0.5619) | 13 | 91598223 | 0.012 | 2.75 (1.17,6.62) |
| rs965770 | 0.0067 | A/G | A (0.2095) | 14 | 97652798 | 0.0082 | 3.75 (1.28,12.13) |
| rs7716581 | 0.0067 | A/G | G (0.3857) | 5 | 116719232 | 0.013 | 2.85 (1.16,7.22) |
| rs10818649 | 0.0067 | A/G | A (0.5857) | 9 | 122048869 | 0.032 | 2.42 (1.02,5.92) |
| rs921924 | 0.0067 | A/G | A (0.5143) | 12 | 123746863 | 0.053 | 2.19 (0.94,5.17) |
| rs917776 | 0.0067 | A/C | A (0.4048) | 9 | 126225898 | 0.015 | 2.85 (1.19,7.01) |
| rs992574 | 0.0067 | A/C | C (0.7952) | 10 | 130694659 | 0.073 | 2.44 (0.87,7.65) |
| rs2575580 | 0.0067 | A/G | G (0.0905) | 4 | 145962630 | 0.04 | 8.98 (1.03,426.65) |
| rs9384054 | 0.0067 | A/G | A (0.3571) | 6 | 153355207 | 0.088 | 2.19 (0.88,5.57) |
| rs1432926 | 0.0067 | A/G | G (0.2476) | 5 | 166206202 | 0.028 | 2.69 (1.03,7.31) |
| rs2193744 | 0.0067 | A/G | A (0.3571) | 3 | 175387679 | 0.069 | 2.22 (0.94,5.37) |
| rs11799843 | 0.0067 | A/G | G (0.0735) | 1 | 183040746 | 0.031 | Inf (1.01,Inf) |
| rs10497674 | 0.0067 | A/G | A (0.9286) | 2 | 187573988 | 0.04 | 8.98 (1.03,426.65) |
| rs3795801 | 0.0067 | A/G | G (0.815) | 1 | 224812474 | 0.03 | 4.29 (1.09,24.97) |
| rs13415546 | 0.0067 | A/G | G (0.3798) | 2 | 225821050 | 0.1 | 2.02 (0.84,4.94) |
| rs9956042 | 0.0068 | A/G | A (0.9143) | 18 | 3977533 | 0.17 | 2.92 (0.58,19.09) |
| rs531099 | 0.0068 | A/G | G (0.4762) | 1 | 5997921 | 0.0063 | 3.07 (1.31,7.41) |
| rs11998950 | 0.0068 | A/G | A (0.0952) | 9 | 6801507 | 0.2 | 2.58 (0.61,12.85) |
| rs6870451 | 0.0068 | A/G | G (0.2571) | 5 | 8991885 | 0.00023 | 5.93 (2.09,18.82) |
| rs7548659 | 0.0068 | A/C | C (0.2087) | 1 | 11041705 | 0.014 | 3.62 (1.23,11.71) |
| rs570061 | 0.0068 | A/G | A (0.2333) | 13 | 20497824 | 0.024 | 3.02 (1.13,8.43) |
| rs17163904 | 0.0068 | A/G | G (0.3571) | 1 | 26425248 | 0.0075 | 3.11 (1.27,7.86) |
| rs10122902 | 0.0068 | A/G | G (0.8381) | 9 | 27546780 | 0.026 | 3.61 (1.05,16.04) |
| rs4952097 | 0.0068 | A/G | A (0.5952) | 2 | 30232248 | 0.017 | 2.77 (1.12,7.19) |
| rs8127350 | 0.0068 | A/G | A (0.1) | 21 | 32470751 | 0.0044 | 12.58 (1.58,577.21) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs1873050 | 0.0068 | A/G | G (0.581) | 17 | 34282595 | 0.011 | 2.83 (1.2,6.89) |
| rs3123678 | 0.0068 | A/G | G (0.5481) | 10 | 43435666 | 0.081 | 2.04 (0.88,4.81) |
| rs2905503 | 0.0068 | A/G | A (0.9143) | 11 | 60607228 | 0.051 | 4.09 (0.91,25.4) |
| rs6546266 | 0.0068 | A/G | G (0.4333) | 2 | 66958516 | 0.012 | 2.75 (1.17,6.62) |
| rs2589142 | 0.0068 | A/G | A (0.2429) | 17 | 76458857 | 0.0063 | 3.74 (1.38,10.77) |
| rs7324822 | 0.0068 | A/G | G (0.7333) | 13 | 77904506 | 0.024 | 3.14 (1.08,10.57) |
| rs4877475 | 0.0068 | A/G | G (0.5429) | 9 | 79040585 | 0.17 | 1.79 (0.78,4.18) |
| rs790361 | 0.0068 | A/C | C (0.819) | 11 | 83304626 | 0.024 | 3.14 (1.08,10.57) |
| rs2954600 | 0.0068 | A/G | A (0.1286) | 12 | 88776452 | 0.035 | 5.3 (0.94,54.86) |
| rs2305971 | 0.0068 | A/G | G (0.3143) | 12 | 93105768 | 0.19 | 1.82 (0.72,4.68) |
| rs7357193 | 0.0068 | A/C | A (0.3667) | 7 | 93432064 | 0.056 | 2.39 (0.96,6.08) |
| rs1950771 | 0.0068 | A/G | A (0.6619) | 14 | 94334655 | 0.11 | 2.03 (0.83,5.17) |
| rs10519576 | 0.0068 | A/G | G (0.0667) | 5 | 118647435 | 0.075 | 6.58 (0.83,301.83) |
| rs1331616 | 0.0068 | A/G | A (0.2333) | 9 | 119227215 | 0.094 | 2.3 (0.81,6.84) |
| rs12411763 | 0.0068 | A/G | G (0.1571) | 10 | 132853583 | 0.061 | 2.9 (0.89,10.47) |
| rs2125431 | 0.0068 | A/G | G (0.4762) | 4 | 181242387 | 0.053 | 2.19 (0.95,5.22) |
| rs11741203 | 0.0069 | A/G | G (0.6429) | 5 | 2271025 | 0.001 | 4.39 (1.67,12.71) |
| rs11033879 | 0.0069 | A/G | G (0.9412) | 11 | 4764803 | 0.088 | 6.6 (0.7,322.33) |
| rs966099 | 0.0069 | A/G | G (0.7714) | 11 | 13714230 | 0.0063 | 4.15 (1.35,15.46) |
| rs1237183 | 0.0069 | A/G | A (0.2714) | 20 | 15446687 | 0.13 | 2 (0.78,5.22) |
| rs12710748 | 0.0069 | A/G | G (0.3333) | 2 | 21057955 | 0.025 | 2.61 (1.09,6.43) |
| rs7776857 | 0.0069 | A/C | C (0.3571) | 7 | 22528008 | 0.03 | 2.42 (1.02,5.86) |
| rs1049536 | 0.0069 | A/G | G (0.7286) | 22 | 28228908 | 0.15 | 2 (0.8,5.22) |
| rs7559825 | 0.0069 | A/G | A (0.3333) | 2 | 37659735 | 0.22 | 1.71 (0.71,4.15) |
| rs6789468 | 0.0069 | A/G | G (0.2381) | 3 | 37988497 | 0.035 | 2.82 (1.02,8.26) |
| rs8128695 | 0.0069 | A/G | G (0.3905) | 21 | 39338037 | 0.048 | 2.27 (0.97,5.42) |
| rs10416755 | 0.0069 | A/G | A (0.1524) | 19 | 39975287 | 0.053 | 2.63 (0.9,8.18) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2411759 | 0.0069 | A/G | A (0.4257) | 17 | 44426073 | 0.54 | 1.38 (0.58,3.32) |
| rs1552756 | 0.0069 | A/G | G (0.7333) | 12 | 51468260 | 0.02 | 2.97 (1.12,8.65) |
| rs400524 | 0.0069 | A/G | A (0.8619) | 4 | 66947433 | 0.04 | 4.69 (0.95,45.79) |
| rs1705244 | 0.0069 | A/G | A (0.519) | 12 | 69875513 | 0.12 | 1.88 (0.81,4.42) |
| rs943860 | 0.0069 | A/G | A (0.2429) | 9 | 75490268 | 0.12 | 2 (0.76,5.38) |
| rs7848094 | 0.0069 | A/G | A (0.2476) | 9 | 89955614 | 0.016 | 2.95 (1.14,7.98) |
| rs10134946 | 0.0069 | A/G | A (0.3238) | 14 | 104725651 | 0.075 | 2.06 (0.87,4.93) |
| rs9514649 | 0.0069 | A/G | A (0.3824) | 13 | 106742081 | 0.076 | 2.08 (0.89,4.98) |
| rs2396135 | 0.0069 | A/G | G (0.3952) | 7 | 108824135 | 0.069 | 2.22 (0.94,5.37) |
| rs10519507 | 0.0069 | A/C | A (0.9333) | 5 | 116741031 | 0.13 | 3.62 (0.56,39.69) |
| rs9883988 | 0.0069 | A/G | G (0.0571) | 3 | 131645093 | 0.3 | 2.78 (0.5,28.7) |
| rs6830355 | 0.0069 | A/G | A (0.3798) | 4 | 181386165 | 0.028 | 2.52 (1.06,6.12) |
| rs4975533 | 0.007 | A/G | G (0.1714) | 5 | 1041510 | 0.0097 | 3.55 (1.26,10.8) |
| rs4321285 | 0.007 | A/G | A (0.2524) | 18 | 4837173 | 0.071 | 2.45 (0.93,6.69) |
| rs2561858 | 0.007 | A/C | A (0.3476) | 5 | 5912422 | 0.048 | 2.25 (0.95,5.38) |
| rs4669724 | 0.007 | A/G | G (0.1619) | 2 | 11480967 | 0.061 | 2.9 (0.89,10.47) |
| rs2614176 | 0.007 | A/G | A (0.8286) | 11 | 37079412 | 0.0033 | 5.78 (1.53,32.82) |
| rs2802490 | 0.007 | A/G | G (0.5) | 10 | 43919019 | 0.33 | 1.58 (0.68,3.68) |
| rs1861739 | 0.007 | A/C | C (0.419) | 22 | 45452569 | 0.02 | 2.57 (1.1,6.19) |
| rs9526457 | 0.007 | A/G | A (0.6857) | 13 | 47624771 | 0.15 | 1.9 (0.78,4.85) |
| rs801145 | 0.007 | A/G | A (0.9238) | 7 | 51067256 | 0.4 | 2.83 (0.39,32.62) |
| rs8064100 | 0.007 | A/G | A (0.619) | 16 | 55282674 | 0.016 | 2.95 (1.2,7.67) |
| rs3018362 | 0.007 | A/G | G (0.6779) | 18 | 58233073 | 0.024 | 2.73 (1.08,7.34) |
| rs1045352 | 0.007 | A/C | C (0.3911) | 5 | 60868837 | 0.026 | 2.61 (1.09,6.4) |
| rs1812486 | 0.007 | A/G | G (0.1952) | 16 | 62143991 | 0.001 | 4.97 (1.73,15.85) |
| rs7133608 | 0.007 | A/G | G (0.2905) | 12 | 66089772 | 0.056 | 2.39 (0.96,6.08) |
| rs11077846 | 0.007 | A/G | A (0.8942) | 17 | 72119342 | 0.023 | 5.21 (1.07,50.48) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs7104259 | 0.007 | A/G | A (0.5971) | 11 | 74064089 | 0.018 | 2.68 (1.12,6.68) |
| rs1822610 | 0.007 | A/C | A (0.2667) | 13 | 92537980 | 0.002 | 3.72 (1.5,9.58) |
| rs7298918 | 0.007 | A/G | G (0.2238) | 12 | 93113480 | 0.094 | 2.3 (0.81,6.84) |
| rs180267 | 0.007 | A/G | A (0.3173) | 7 | 93185193 | 0.033 | 2.63 (1.05,6.79) |
| rs1905719 | 0.007 | A/G | A (0.1952) | 4 | 94774423 | 0.12 | 2.45 (0.78,8.21) |
| rs1836406 | 0.007 | A/C | A (0.3571) | 9 | 95621599 | 0.17 | 1.78 (0.76,4.21) |
| rs7484006 | 0.007 | A/G | G (0.3317) | 11 | 99680579 | 0.00045 | 4.48 (1.79,11.77) |
| rs1562221 | 0.007 | A/G | G (0.8905) | 11 | 104988839 | 0.094 | 3.49 (0.74,22.16) |
| rs17367786 | 0.007 | A/G | A (0.3905) | 13 | 106733679 | 0.032 | 2.39 (1.03,5.67) |
| rs483096 | 0.007 | A/G | A (0.1106) | 1 | 110078352 | 0.17 | 2.82 (0.56,18.46) |
| rs769563 | 0.007 | A/G | G (0.2905) | 3 | 110679604 | 0.024 | 3.02 (1.13,8.43) |
| rs7852204 | 0.007 | A/G | A (0.524) | 9 | 127453150 | 0.011 | 2.92 (1.24,7.07) |
| rs1571823 | 0.007 | A/G | A (0.2238) | 9 | 135484150 | 0.058 | 2.55 (0.91,7.53) |
| rs4634676 | 0.007 | A/G | A (0.3905) | 8 | 138336165 | 0.1 | 2.04 (0.86,4.93) |
| rs296513 | 0.007 | A/G | A (0.2143) | 1 | 197638130 | 0.02 | 3.11 (1.13,9.04) |
| rs4951693 | 0.007 | A/C | A (0.83) | 1 | 207530958 | 0.0016 | 13.43 (1.89,589.99) |
| rs868898 | 0.0071 | A/C | A (0.9167) | 8 | 806716 | 0.04 | 8.81 (1,420.96) |
| rs6751561 | 0.0071 | A/G | A (0.1538) | 2 | 6416761 | 0.012 | 4.49 (1.21,20.85) |
| rs1033638 | 0.0071 | A/G | A (0.1571) | 1 | 11020983 | 0.052 | 3.13 (0.89,12.67) |
| rs10492140 | 0.0071 | A/C | C (0.0619) | 12 | 13917276 | 0.04 | 8.98 (1.03,426.65) |
| rs6044080 | 0.0071 | A/G | A (0.6048) | 20 | 16461260 | 0.076 | 2.13 (0.9,5.21) |
| rs1697647 | 0.0071 | A/G | G (0.3454) | 8 | 16651012 | 0.059 | 2.26 (0.91,5.72) |
| rs11910740 | 0.0071 | A/G | G (0.0667) | 21 | 23130703 | 0.18 | 3.23 (0.6,32.73) |
| rs730493 | 0.0071 | A/G | A (0.9333) | 15 | 33165126 | 0.04 | 8.98 (1.03,426.65) |
| rs3780867 | 0.0071 | A/G | A (0.5194) | 10 | 33587815 | 0.018 | 2.67 (1.13,6.46) |
| rs11585 | 0.0071 | A/G | A (0.9029) | 11 | 33684281 | 0.16 | 3.07 (0.61,20.12) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2235776 | 0.0071 | A/G | G (0.8571) | 22 | 42702900 | 0.012 | 6.33 (1.34,60.52) |
| rs4919756 | 0.0071 | A/G | G (0.7381) | 12 | 51463589 | 0.032 | 2.78 (1.04,8.1) |
| rs855314 | 0.0071 | A/G | G (0.1905) | 1 | 63807132 | 0.072 | 2.75 (0.89,9.1) |
| rs2860216 | 0.0071 | A/G | A (0.7429) | 14 | 76075761 | 0.015 | 2.93 (1.17,7.85) |
| rs6574505 | 0.0071 | A/C | A (0.3317) | 14 | 78876950 | 0.21 | 1.77 (0.73,4.34) |
| rs4887466 | 0.0071 | A/G | A (0.5) | 15 | 84796753 | 0.051 | 2.28 (0.99,5.41) |
| rs10033018 | 0.0071 | A/G | A (0.8544) | 4 | 92575501 | 0.039 | 5.18 (1.06,50.28) |
| rs4762290 | 0.0071 | A/G | A (0.6714) | 12 | 95141211 | 0.037 | 2.57 (0.99,7.17) |
| rs807450 | 0.0071 | A/G | A (0.6667) | 14 | 98713688 | 0.23 | 1.75 (0.74,4.3) |
| rs7387186 | 0.0071 | A/G | G (0.7048) | 8 | 103781083 | 0.057 | 2.41 (0.92,6.72) |
| rs9520758 | 0.0071 | A/G | A (0.5143) | 13 | 107516512 | 0.032 | 2.44 (1.05,5.81) |
| rs25923 | 0.0071 | A/G | G (0.3429) | 5 | 110824997 | 0.015 | 2.85 (1.19,7.01) |
| rs10491527 | 0.0071 | A/G | G (0.2) | 9 | 119256347 | 0.094 | 2.3 (0.81,6.84) |
| rs13298216 | 0.0071 | A/G | G (0.0714) | 9 | 126250459 | 0.041 | 7.53 (0.98,341.68) |
| rs3780663 | 0.0071 | A/G | A (0.7619) | 9 | 127711073 | 0.0036 | 4.45 (1.46,16.54) |
| rs2622436 | 0.0071 | A/G | G (0.3286) | 10 | 131019646 | 0.00048 | 4.42 (1.79,11.42) |
| rs10930342 | 0.0071 | A/G | A (0.1905) | 2 | 151619135 | 0.15 | 2.06 (0.71,6.2) |
| rs901363 | 0.0071 | A/G | A (0.319) | 6 | 159148754 | 0.042 | 2.41 (1.01,5.86) |
| rs2085449 | 0.0071 | A/G | A (0.4048) | 5 | 167818328 | 0.048 | 2.27 (0.97,5.42) |
| rs17072678 | 0.0071 | A/G | G (0.7762) | 4 | 183339561 | 0.052 | 3.07 (0.97,11.61) |
| rs17534206 | 0.0072 | A/G | A (0.681) | 5 | 1811662 | 0.057 | 2.41 (0.92,6.72) |
| rs11078674 | 0.0072 | A/C | C (0.6942) | 17 | 7251197 | 0.054 | 2.53 (0.97,7.1) |
| rs4669821 | 0.0072 | A/C | C (0.4619) | 2 | 12265797 | 0.031 | 2.49 (1.06,6.02) |
| rs16885 | 0.0072 | A/G | G (0.7981) | 6 | 16414730 | 0.0062 | 7.22 (1.54,68.87) |
| rs1455915 | 0.0072 | A/G | A (0.101) | 8 | 29262045 | 0.054 | 3.95 (0.88,24.56) |
| rs1511168 | 0.0072 | A/C | C (0.8286) | 8 | 30013376 | 0.012 | 6.33 (1.34,60.52) |
| rs2422178 | 0.0072 | A/G | A (0.3571) | 11 | 36112154 | 0.041 | 2.39 (0.99,5.9) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2835263 | 0.0072 | A/G | G (0.4158) | 21 | 36352115 | 0.018 | 2.7 (1.12,6.8) |
| rs570941 | 0.0072 | A/C | C (0.1275) | 9 | 38263626 | 0.087 | 2.8 (0.77,11.55) |
| rs2050083 | 0.0072 | A/G | A (0.4524) | 20 | 40000537 | 0.0018 | 3.63 (1.5,9.24) |
| rs4794225 | 0.0072 | A/C | C (0.1394) | 17 | 43312590 | 0.016 | 3.97 (1.18,15.68) |
| rs17659514 | 0.0072 | A/G | A (0.6333) | 15 | 51208696 | 0.16 | 1.87 (0.79,4.58) |
| rs4883812 | 0.0072 | A/C | C (0.1333) | 13 | 53441517 | 0.0036 | 6.82 (1.68,40.27) |
| rs4087469 | 0.0072 | A/G | G (0.1333) | 13 | 53454709 | 0.0036 | 6.82 (1.68,40.27) |
| rs2671130 | 0.0072 | A/C | A (0.4524) | 7 | 56472758 | 0.0017 | 3.71 (1.55,9.31) |
| rs4286198 | 0.0072 | A/G | G (0.7714) | 18 | 62145332 | 0.052 | 3.07 (0.97,11.61) |
| rs2796188 | 0.0072 | A/G | A (0.4714) | 1 | 71406882 | 0.078 | 2.12 (0.91,5.07) |
| rs2298152 | 0.0072 | A/G | A (0.6952) | 1 | 72366751 | 0.037 | 2.57 (0.99,7.17) |
| rs2974154 | 0.0072 | A/G | A (0.8571) | 2 | 79993511 | 0.032 | 4.13 (1.05,23.93) |
| rs11019297 | 0.0072 | A/G | G (0.6346) | 11 | 87691891 | 0.047 | 2.36 (0.99,5.87) |
| rs12580153 | 0.0072 | A/G | G (0.7048) | 12 | 91320950 | 0.0044 | 4.16 (1.45,13.84) |
| rs16948292 | 0.0072 | A/G | A (0.9095) | 15 | 91959244 | 0.067 | 4.18 (0.83,41.29) |
| rs1537957 | 0.0072 | A/G | G (0.3429) | 9 | 98272545 | 0.22 | 1.69 (0.7,4.15) |
| rs11123751 | 0.0072 | A/C | A (0.8238) | 2 | 98828275 | 0.018 | 4.52 (1.16,26.01) |
| rs847669 | 0.0072 | A/G | A (0.9272) | 6 | 108730548 | 0.075 | 4.29 (0.72,45.57) |
| rs10507250 | 0.0072 | A/G | A (0.125) | 12 | 113358516 | 0.069 | 3.49 (0.89,16.63) |
| rs4870976 | 0.0072 | A/G | G (0.3476) | 8 | 127547507 | 0.018 | 2.9 (1.14,7.65) |
| rs10235144 | 0.0072 | A/G | A (0.5333) | 7 | 139561280 | 0.052 | 2.23 (0.96,5.28) |
| rs7000748 | 0.0072 | A/G | A (0.3571) | 8 | 143406692 | 0.15 | 1.87 (0.78,4.52) |
| rs3753539 | 0.0072 | A/G | A (0.2429) | 1 | 168447842 | 0.024 | 3.02 (1.13,8.43) |
| rs11730323 | 0.0072 | A/G | G (0.7905) | 4 | 184211639 | 0.011 | 3.86 (1.25,14.43) |
| rs1573185 | 0.0072 | A/G | A (0.2381) | 1 | 220589799 | 0.05 | 2.42 (0.94,6.43) |
| rs2445300 | 0.0073 | A/C | C (0.6476) | 11 | 4988060 | 0.071 | 2.17 (0.89,5.51) |
| rs10500683 | 0.0073 | A/G | A (0.4905) | 11 | 7145579 | 0.012 | 2.75 (1.17,6.62) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs6079855 | 0.0073 | A/G | G (0.2667) | 20 | 15433148 | 0.016 | 2.95 (1.14,7.98) |
| rs7919238 | 0.0073 | A/G | A (0.8143) | 10 | 18432132 | 0.043 | 3.32 (0.96,14.81) |
| rs932055 | 0.0073 | A/G | A (0.1875) | 12 | 22591655 | 0.016 | 3.97 (1.18,15.68) |
| rs655258 | 0.0073 | A/G | G (0.4571) | 9 | 23532707 | 0.033 | 2.41 (1.03,5.78) |
| rs12265119 | 0.0073 | A/G | A (0.1619) | 10 | 26848807 | 0.029 | 3.54 (1.03,14.13) |
| rs12200498 | 0.0073 | A/G | A (0.2381) | 6 | 35793933 | 0.16 | 2.03 (0.73,5.85) |
| rs2076125 | 0.0073 | A/G | A (0.7762) | 22 | 38035851 | 0.073 | 2.44 (0.87,7.65) |
| rs1906342 | 0.0073 | A/G | A (0.4762) | 17 | 40659391 | 0.081 | 2.06 (0.89,4.88) |
| rs6941971 | 0.0073 | A/G | A (0.9048) | 6 | 45695572 | 0.041 | 7.53 (0.98,341.68) |
| rs1973688 | 0.0073 | A/G | A (0.3) | 15 | 56369832 | 0.0045 | 3.61 (1.37,10.03) |
| rs1412906 | 0.0073 | A/C | C (0.1683) | 13 | 64812067 | 0.32 | 1.72 (0.59,5.09) |
| rs12535057 | 0.0073 | A/G | G (0.781) | 7 | 75133689 | 0.073 | 2.44 (0.87,7.65) |
| rs898962 | 0.0073 | A/G | A (0.5048) | 16 | 80055453 | 0.0033 | 3.46 (1.46,8.5) |
| rs11935974 | 0.0073 | A/G | G (0.9286) | 4 | 83930909 | 0.07 | 4.44 (0.75,47.1) |
| rs10504814 | 0.0073 | A/G | A (0.1143) | 8 | 86735603 | 0.1 | 2.57 (0.77,9.39) |
| rs1391496 | 0.0073 | A/G | A (0.9381) | 6 | 92624521 | 0.46 | 1.86 (0.3,13.38) |
| rs2871432 | 0.0073 | A/G | A (0.5433) | 2 | 101975892 | 0.078 | 2.08 (0.89,4.92) |
| rs7134360 | 0.0073 | A/G | A (0.6333) | 12 | 104190413 | 0.076 | 2.13 (0.9,5.21) |
| rs3098211 | 0.0073 | A/G | G (0.7718) | 8 | 104535041 | 0.0045 | 4.93 (1.48,21.54) |
| rs8624 | 0.0073 | A/G | A (0.7067) | 10 | 114177777 | 0.024 | 2.73 (1.08,7.34) |
| rs9491396 | 0.0073 | A/G | A (0.7095) | 6 | 125735926 | 0.087 | 2.24 (0.86,6.29) |
| rs1572049 | 0.0073 | A/G | A (0.0905) | 9 | 135723012 | 0.0019 | 14.52 (1.88,658.49) |
| rs4668094 | 0.0073 | A/G | G (0.5143) | 2 | 169345138 | 0.034 | 2.34 (1.01,5.58) |
| rs4915451 | 0.0073 | A/C | A (0.5429) | 1 | 197391511 | 0.00095 | 3.81 (1.6,9.37) |
| rs3763057 | 0.0074 | A/G | G (0.5762) | 5 | 7398815 | 0.0029 | 3.56 (1.42,9.5) |
| rs628674 | 0.0074 | A/G | A (0.7476) | 18 | 20331033 | 0.093 | 2.26 (0.89,6.07) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2797777 | 0.0074 | A/C | A (0.7762) | 6 | 37618910 | 0.024 | 3.14 (1.08,10.57) |
| rs2722425 | 0.0074 | A/G | A (0.1048) | 8 | 40603396 | 0.01 | 10.74 (1.3,500.03) |
| rs9615958 | 0.0074 | A/G | A (0.1154) | 22 | 45071641 | 0.11 | 3.84 (0.74,38.39) |
| rs12215131 | 0.0074 | A/C | A (0.3883) | 6 | 47817655 | 0.31 | 1.62 (0.68,3.87) |
| rs1539997 | 0.0074 | A/G | A (0.8238) | 18 | 57370047 | 0.054 | 3.76 (0.95,21.94) |
| rs10509213 | 0.0074 | A/G | A (0.0524) | 10 | 66403510 | 0.46 | 1.86 (0.3,13.38) |
| rs4578268 | 0.0074 | A/G | A (0.2143) | 10 | 67310408 | 0.27 | 1.64 (0.62,4.35) |
| rs1620673 | 0.0074 | A/G | A (0.2143) | 10 | 67342767 | 0.27 | 1.64 (0.62,4.35) |
| rs11997859 | 0.0074 | A/G | A (0.6058) | 8 | 78343625 | 0.07 | 2.18 (0.89,5.56) |
| rs10485303 | 0.0074 | A/G | A (0.8429) | 6 | 85877962 | 0.026 | 3.61 (1.05,16.04) |
| rs1216471 | 0.0074 | A/C | C (0.4143) | 11 | 99900062 | 0.0056 | 3.18 (1.35,7.72) |
| rs698172 | 0.0074 | A/G | A (0.432) | 5 | 127182826 | 0.0032 | 3.37 (1.42,8.26) |
| rs16763 | 0.0074 | A/C | C (0.4356) | 9 | 133822319 | 0.03 | 2.45 (1.04,5.96) |
| rs7739143 | 0.0074 | A/C | C (0.3447) | 6 | 144874527 | 0.014 | 2.97 (1.22,7.42) |
| rs9478809 | 0.0074 | A/G | A (0.9238) | 6 | 151164461 | 0.4 | 2.83 (0.39,32.62) |
| rs3775085 | 0.0074 | A/C | A (0.8619) | 4 | 157180984 | 0.012 | 9.56 (1.3,426.01) |
| rs6885463 | 0.0074 | A/G | A (0.581) | 5 | 169889654 | 0.078 | 2.12 (0.91,5.07) |
| rs838704 | 0.0074 | A/G | G (0.8714) | 3 | 191299292 | 0.043 | 3.32 (0.96,14.81) |
| rs12027815 | 0.0074 | A/G | G (0.781) | 1 | 191676879 | 0.045 | 2.83 (1.01,8.79) |
| rs6793017 | 0.0075 | A/G | G (0.781) | 3 | 2554127 | 0.047 | 2.63 (0.94,8.21) |
| rs2986687 | 0.0075 | A/G | A (0.1524) | 9 | 3291887 | 0.016 | 3.97 (1.18,15.68) |
| rs1812310 | 0.0075 | A/G | A (0.8095) | 4 | 5339650 | 0.026 | 3.61 (1.05,16.04) |
| rs11022839 | 0.0075 | A/G | A (0.819) | 11 | 13444537 | 0.0081 | 4.58 (1.36,20.06) |
| rs1459014 | 0.0075 | A/G | A (0.819) | 11 | 13513831 | 0.0081 | 4.58 (1.36,20.06) |
| rs912410 | 0.0075 | A/G | G (0.7952) | 13 | 19582769 | 0.0031 | 6.24 (1.66,35.3) |
| rs7873308 | 0.0075 | A/G | A (0.4667) | 9 | 23415710 | 0.02 | 2.57 (1.1,6.19) |
| rs7931259 | 0.0075 | A/C | A (0.3619) | 11 | 25346524 | 0.19 | 1.82 (0.72,4.68) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs11702795 | 0.0075 | A/C | A (0.2115) | 21 | 32304363 | 0.0043 | 4.13 (1.42,13.28) |
| rs12433092 | 0.0075 | A/G | A (0.1381) | 14 | 43932545 | 0.027 | 4.71 (1.09,28.83) |
| rs3782479 | 0.0075 | A/C | C (0.0865) | 12 | 50588085 | 0.04 | 7.82 (1.02,355.05) |
| rs1075440 | 0.0075 | A/G | A (0.6667) | 16 | 52348407 | 0.071 | 2.17 (0.89,5.51) |
| rs6572971 | 0.0075 | A/G | A (0.1619) | 14 | 54280838 | 0.053 | 2.63 (0.9,8.18) |
| rs12782802 | 0.0075 | A/G | G (0.2692) | 10 | 71079849 | 0.13 | 2.1 (0.83,5.49) |
| rs1587563 | 0.0075 | A/G | G (0.6476) | 4 | 79091742 | 0.028 | 2.63 (1.09,6.68) |
| rs2169452 | 0.0075 | A/G | A (0.1286) | 16 | 81401442 | 0.3 | 1.86 (0.6,6.01) |
| rs8732 | 0.0075 | A/G | G (0.9143) | 4 | 83709000 | 0.069 | 3.49 (0.89,16.63) |
| rs8025158 | 0.0075 | A/G | A (0.2667) | 15 | 86514696 | 0.012 | 4.49 (1.21,20.85) |
| rs1262181 | 0.0075 | A/G | A (0.3667) | 11 | 95532144 | 0.042 | 2.41 (1.01,5.86) |
| rs228617 | 0.0075 | A/G | A (0.4667) | 4 | 103937991 | 0.032 | 2.42 (1.02,5.92) |
| rs1334925 | 0.0075 | A/G | A (0.7524) | 6 | 119454618 | 0.51 | 1.47 (0.56,4.03) |
| rs7736276 | 0.0075 | A/G | A (0.1117) | 5 | 139316132 | 0.098 | 3.37 (0.72,21.43) |
| rs697651 | 0.0075 | A/C | C (0.2692) | 2 | 155148786 | 0.03 | 2.65 (1.04,7.01) |
| rs2305619 | 0.0075 | A/G | A (0.4) | 3 | 158637563 | 0.0084 | 3.1 (1.29,7.66) |
| rs1114237 | 0.0075 | A/G | A (0.919) | 3 | 158658745 | 0.069 | 3.49 (0.89,16.63) |
| rs3845724 | 0.0075 | A/G | A (0.4095) | 2 | 169435739 | 0.049 | 2.31 (0.99,5.48) |
| rs1990606 | 0.0075 | A/G | A (0.5905) | 2 | 171326131 | 0.011 | 2.86 (1.2,7.13) |
| rs2688492 | 0.0075 | A/G | A (0.7129) | 3 | 196985676 | 0.032 | 2.72 (1.01,8) |
| rs10924303 | 0.0075 | A/G | A (0.2079) | 1 | 242173075 | 0.062 | 2.56 (0.94,7.29) |
| rs15971 | 0.0076 | A/G | G (0.0952) | 19 | 367834 | 0.23 | 2.38 (0.63,9.99) |
| rs4735827 | 0.0076 | A/G | A (0.4853) | 8 | 843666 | 0.012 | 2.78 (1.18,6.76) |
| rs2682119 | 0.0076 | A/C | A (0.9327) | 11 | 6307685 | 0.02 | 9.44 (1.08,449.04) |
| rs11080571 | 0.0076 | A/G | G (0.2143) | 18 | 12328470 | 0.0063 | 3.74 (1.38,10.77) |
| rs7235296 | 0.0076 | A/G | G (0.2143) | 18 | 12339445 | 0.0063 | 3.74 (1.38,10.77) |
| rs6416020 | 0.0076 | A/G | A (0.8286) | 11 | 18573760 | 0.15 | 2.3 (0.76,7.89) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs10084714 | 0.0076 | A/G | G (0.2019) | 3 | 20158336 | 0.053 | 2.63 (0.9,8.18) |
| rs4983039 | 0.0076 | A/G | G (0.719) | 14 | 24495062 | 0.057 | 2.41 (0.92,6.72) |
| rs4963804 | 0.0076 | A/G | A (0.3143) | 12 | 24874298 | 0.011 | 3.13 (1.26,8.06) |
| rs4867517 | 0.0076 | A/G | G (0.5286) | 5 | 33549198 | 0.081 | 2.04 (0.88,4.81) |
| rs9615901 | 0.0076 | A/G | G (0.8476) | 22 | 47183633 | 0.15 | 3.06 (0.74,18.19) |
| rs1873363 | 0.0076 | A/G | A (0.8762) | 5 | 73692476 | 0.0064 | 6.92 (1.48,65.88) |
| rs10509500 | 0.0076 | A/G | G (0.0667) | 10 | 86655684 | 0.02 | Inf (1.36,Inf) |
| rs12860685 | 0.0076 | A/G | G (0.2476) | 13 | 92481066 | 0.0013 | 4.19 (1.64,11.26) |
| rs2978157 | 0.0076 | A/G | G (0.6905) | 8 | 95156972 | 0.071 | 2.17 (0.89,5.51) |
| rs1190922 | 0.0076 | A/G | A (0.835) | 14 | 100189117 | 0.018 | 4.5 (1.15,25.97) |
| rs2846293 | 0.0076 | A/G | G (0.4905) | 11 | 122812472 | 0.12 | 1.88 (0.81,4.42) |
| rs2109149 | 0.0076 | A/G | A (0.4905) | 12 | 125476335 | 0.081 | 2.06 (0.89,4.88) |
| rs12641839 | 0.0076 | A/G | A (0.1667) | 4 | 125785266 | 0.029 | 3.54 (1.03,14.13) |
| rs1027560 | 0.0076 | A/G | G (0.6333) | 12 | 129129616 | 0.042 | 2.43 (0.99,6.33) |
| rs9375677 | 0.0076 | A/C | A (0.5952) | 6 | 130273736 | 0.044 | 2.47 (1.02,6.26) |
| rs1016315 | 0.0076 | A/G | G (0.8942) | 10 | 131742295 | 0.064 | 4.35 (0.86,42.9) |
| rs1319087 | 0.0076 | A/G | G (0.2571) | 6 | 133938800 | 0.13 | 2 (0.79,5.11) |
| rs1153711 | 0.0076 | A/C | A (0.8714) | 2 | 175738064 | 0.023 | 5.21 (1.07,50.48) |
| rs3764712 | 0.0077 | A/G | G (0.5524) | 20 | 1261762 | 0.03 | 2.48 (1.06,5.92) |
| rs2779736 | 0.0077 | A/C | A (0.2143) | 9 | 6769505 | 0.02 | 3.11 (1.13,9.04) |
| rs1926390 | 0.0077 | A/C | C (0.8286) | 9 | 7315964 | 0.026 | 3.61 (1.05,16.04) |
| rs526061 | 0.0077 | A/G | A (0.7857) | 10 | 21354610 | 0.0045 | 4.93 (1.48,21.54) |
| rs10510535 | 0.0077 | A/G | A (0.919) | 3 | 23068395 | 0.16 | 5.72 (0.54,290.23) |
| rs2305572 | 0.0077 | A/G | G (0.7905) | 2 | 33660367 | 0.011 | 3.86 (1.25,14.43) |
| rs1436613 | 0.0077 | A/G | G (0.0905) | 8 | 35716697 | 0.04 | 8.98 (1.03,426.65) |
| rs2534575 | 0.0077 | A/G | G (0.3571) | 7 | 38144714 | 0.03 | 2.65 (1.04,7.01) |
| rs135821 | 0.0077 | A/C | C (0.1048) | 22 | 48276869 | 0.18 | 2.17 (0.67,7.37) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs135823 | 0.0077 | A/G | A (0.1048) | 22 | 48278532 | 0.18 | 2.17 (0.67,7.37) |
| rs1395605 | 0.0077 | A/G | A (0.3905) | 16 | 49495030 | 0.048 | 2.27 (0.97,5.42) |
| rs751595 | 0.0077 | A/G | A (0.1714) | 10 | 50634662 | 0.00028 | 7.58 (2.19,33.95) |
| rs2877724 | 0.0077 | A/G | G (0.4952) | 17 | 51471381 | 0.012 | 2.75 (1.17,6.62) |
| rs2419405 | 0.0077 | A/G | G (0.5294) | 2 | 60062833 | 0.078 | 2.08 (0.89,4.92) |
| rs2112931 | 0.0077 | A/G | A (0.781) | 5 | 67963902 | 0.084 | 2.83 (0.89,10.75) |
| rs657315 | 0.0077 | A/G | A (0.2524) | 11 | 69041893 | 0.083 | 2.19 (0.86,5.7) |
| rs1692420 | 0.0077 | A/G | G (0.0762) | 5 | 71266499 | 0.075 | 6.58 (0.83,301.83) |
| rs948971 | 0.0077 | A/G | G (0.3952) | 11 | 76614481 | 0.11 | 1.91 (0.81,4.55) |
| rs2301134 | 0.0077 | A/G | A (0.4952) | 4 | 91116123 | 0.25 | 1.61 (0.7,3.76) |
| rs4984419 | 0.0077 | A/G | G (0.581) | 15 | 94344078 | 0.051 | 2.26 (0.97,5.41) |
| rs10493986 | 0.0077 | A/G | G (0.2837) | 1 | 103282148 | 0.064 | 2.29 (0.95,5.66) |
| rs696562 | 0.0077 | A/G | A (0.4048) | 9 | 106888921 | 0.048 | 2.25 (0.95,5.38) |
| rs12345511 | 0.0077 | A/G | G (0.7476) | 9 | 109724854 | 0.047 | 2.63 (0.94,8.21) |
| rs7513088 | 0.0077 | A/C | C (0.396) | 1 | 112250281 | 0.057 | 2.36 (0.95,5.96) |
| rs10847527 | 0.0077 | A/G | G (0.0857) | 12 | 127096138 | 0.11 | 3.7 (0.71,36.88) |
| rs10739754 | 0.0077 | A/G | G (0.7571) | 9 | 129499993 | 0.12 | 2.25 (0.83,6.62) |
| rs1501626 | 0.0077 | A/G | G (0.0952) | 3 | 144477520 | 0.012 | 9.56 (1.3,426.01) |
| rs9886592 | 0.0077 | A/C | A (0.4856) | 8 | 144563279 | 0.12 | 1.9 (0.82,4.49) |
| rs10485061 | 0.0077 | A/G | A (0.6) | 6 | 154763890 | 0.018 | 2.68 (1.12,6.68) |
| rs10037137 | 0.0077 | A/C | A (0.3447) | 5 | 160973555 | 0.0094 | 3.25 (1.26,8.78) |
| rs7440833 | 0.0077 | A/G | A (0.781) | 4 | 168713236 | 0.01 | 4.92 (1.28,28.16) |
| rs171718 | 0.0077 | A/G | G (0.5667) | 5 | 171075771 | 0.02 | 2.58 (1.09,6.32) |
| rs12616750 | 0.0077 | A/G | A (0.2476) | 2 | 198904907 | 0.019 | 2.87 (1.15,7.4) |
| rs12615065 | 0.0077 | A/C | C (0.2108) | 2 | 202799866 | 0.064 | 2.49 (0.92,7.05) |
| rs696958 | 0.0077 | A/G | G (0.6333) | 1 | 205022397 | 0.016 | 2.95 (1.2,7.67) |
| rs6992802 | 0.0078 | A/G | G (0.9381) | 8 | 5534186 | 0.31 | 4.2 (0.32,226.71) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs219861 | 0.0078 | A/G | A (0.5381) | 20 | 9545555 | 0.0063 | 3.07 (1.31,7.41) |
| rs2038095 | 0.0078 | A/G | G (0.3495) | 1 | 13970305 | 0.064 | 2.29 (0.95,5.66) |
| rs8107904 | 0.0078 | A/G | A (0.619) | 19 | 14759248 | 0.08 | 2.05 (0.87,4.96) |
| rs287294 | 0.0078 | A/G | G (0.8571) | 2 | 17293337 | 0.054 | 3.76 (0.95,21.94) |
| rs207338 | 0.0078 | A/G | A (0.5143) | 4 | 18730028 | 0.051 | 2.26 (0.97,5.41) |
| rs1111766 | 0.0078 | A/G | A (0.3857) | 9 | 20543571 | 0.069 | 2.22 (0.94,5.37) |
| rs3174040 | 0.0078 | A/G | G (0.1952) | 8 | 23487352 | 0.072 | 2.75 (0.89,9.1) |
| rs932299 | 0.0078 | A/C | C (0.595) | 22 | 25985563 | 0.023 | 2.77 (1.11,7.31) |
| rs237179 | 0.0078 | A/G | G (0.719) | 16 | 26570108 | 0.14 | 1.97 (0.77,5.32) |
| rs9315760 | 0.0078 | A/G | G (0.2476) | 13 | 39618994 | 0.064 | 2.49 (0.92,7.05) |
| rs1020445 | 0.0078 | A/G | G (0.281) | 2 | 46284926 | 0.016 | 2.95 (1.14,7.98) |
| rs1033060 | 0.0078 | A/G | A (0.619) | 18 | 46680315 | 0.02 | 2.58 (1.09,6.32) |
| rs7333965 | 0.0078 | A/G | A (0.6333) | 13 | 70482247 | 0.053 | 2.19 (0.93,5.29) |
| rs10223066 | 0.0078 | A/G | A (0.7143) | 5 | 71697344 | 7.00E-04 | 6.1 (1.85,26.42) |
| rs4590377 | 0.0078 | A/G | G (0.2548) | 7 | 77352208 | 0.18 | 1.94 (0.74,5.13) |
| rs10509384 | 0.0078 | A/G | A (0.6952) | 10 | 78693188 | 0.037 | 2.57 (0.99,7.17) |
| rs962877 | 0.0078 | A/G | G (0.3894) | 16 | 83021026 | 0.017 | 2.74 (1.15,6.64) |
| rs9301366 | 0.0078 | A/G | G (0.881) | 13 | 108737543 | 0.054 | 3.76 (0.95,21.94) |
| rs17621621 | 0.0078 | A/C | A (0.1571) | 1 | 109110959 | 0.029 | 3.54 (1.03,14.13) |
| rs1981035 | 0.0078 | A/C | C (0.8381) | 9 | 109742680 | 0.04 | 4.69 (0.95,45.79) |
| rs12735195 | 0.0078 | A/G | G (0.7095) | 1 | 117956681 | 0.014 | 3.62 (1.25,12.12) |
| rs2799465 | 0.0078 | A/G | A (0.8476) | 9 | 123624163 | 0.043 | 3.32 (0.96,14.81) |
| rs13116100 | 0.0078 | A/C | A (0.8143) | 4 | 125172029 | 0.026 | 3.61 (1.05,16.04) |
| rs1881955 | 0.0078 | A/G | A (0.1635) | 3 | 154703913 | 0.04 | 3.84 (1.01,18.06) |
| rs317789 | 0.0078 | A/G | G (0.319) | 6 | 159071702 | 0.056 | 2.39 (0.96,6.08) |
| rs10494421 | 0.0078 | A/G | A (0.1) | 1 | 161470385 | 0.012 | 4.49 (1.21,20.85) |
| rs1434035 | 0.0078 | A/G | G (0.8429) | 4 | 179905930 | 0.0018 | 8.18 (1.77,77.07) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs339651 | 0.0078 | A/G | G (0.5286) | 1 | 191629311 | 0.17 | 1.79 (0.78,4.18) |
| rs3860880 | 0.0079 | A/C | A (0.5333) | 8 | 799497 | 0.0066 | 3.03 (1.28,7.38) |
| rs13176914 | 0.0079 | A/G | G (0.8) | 5 | 3322658 | 0.024 | 3.14 (1.08,10.57) |
| rs3849841 | 0.0079 | A/G | G (0.6714) | 8 | 4062687 | 0.024 | 2.75 (1.09,7.37) |
| rs7234270 | 0.0079 | A/G | G (0.3048) | 18 | 19426015 | 0.1 | 2.01 (0.83,4.97) |
| rs9318316 | 0.0079 | A/G | G (0.5857) | 13 | 23582163 | 0.011 | 2.86 (1.2,7.13) |
| rs2173009 | 0.0079 | A/G | G (0.8) | 5 | 34584769 | 0.2 | 2.16 (0.65,8.41) |
| rs2028812 | 0.0079 | A/G | A (0.381) | 13 | 43397013 | 0.0029 | 3.4 (1.43,8.35) |
| rs2093939 | 0.0079 | A/G | G (0.125) | 13 | 43774701 | 0.04 | 3.84 (1.01,18.06) |
| rs10082730 | 0.0079 | A/G | G (0.1143) | 12 | 44288969 | 0.027 | 4.71 (1.09,28.83) |
| rs11768782 | 0.0079 | A/G | A (0.9) | 7 | 63410316 | 0.23 | 2.38 (0.63,9.99) |
| rs1023237 | 0.0079 | A/G | A (0.1476) | 13 | 70107159 | 0.1 | 2.57 (0.77,9.39) |
| rs812462 | 0.0079 | A/G | G (0.835) | 1 | 72668058 | 0.019 | 3.48 (1.11,13.12) |
| rs10778102 | 0.0079 | A/G | G (0.3571) | 12 | 77529888 | 0.01 | 2.88 (1.22,6.99) |
| rs929346 | 0.0079 | A/G | G (0.7885) | 7 | 80107555 | 0.069 | 2.56 (0.91,8.04) |
| rs244853 | 0.0079 | A/G | G (0.5048) | 16 | 82857274 | 0.018 | 2.72 (1.16,6.56) |
| rs2295135 | 0.0079 | A/C | A (0.8088) | 14 | 87963963 | 0.11 | 2.29 (0.8,7.28) |
| rs1598490 | 0.0079 | A/G | G (0.8462) | 15 | 92536132 | 0.067 | 3.16 (0.9,14.24) |
| rs227284 | 0.0079 | A/G | G (0.3571) | 4 | 103964838 | 0.042 | 2.41 (1.01,5.86) |
| rs10981576 | 0.0079 | A/G | A (0.8333) | 9 | 112825211 | 0.084 | 2.83 (0.89,10.75) |
| rs7860625 | 0.0079 | A/G | A (0.1476) | 9 | 113144641 | 0.12 | 2.45 (0.78,8.21) |
| rs9320591 | 0.0079 | A/G | A (0.1019) | 6 | 117602819 | 0.089 | 3.02 (0.86,12.24) |
| rs1421361 | 0.0079 | A/C | A (0.4238) | 7 | 130917037 | 0.076 | 2.11 (0.91,5.01) |
| rs1424593 | 0.0079 | A/C | A (0.5714) | 7 | 131412256 | 0.24 | 1.7 (0.73,4.01) |
| rs10102742 | 0.0079 | A/G | G (0.4238) | 8 | 132373690 | 0.12 | 1.99 (0.85,4.75) |
| rs7842889 | 0.0079 | A/G | A (0.2857) | 8 | 135842789 | 0.0063 | 3.74 (1.38,10.77) |
| rs2107443 | 0.0079 | A/G | G (0.6286) | 5 | 136450112 | 0.039 | 2.58 (1.02,6.91) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs1858104 | 0.0079 | A/G | A (0.1762) | 2 | 165520243 | 0.00039 | 8.43 (2.13,49.17) |
| rs13410779 | 0.0079 | A/G | G (0.1381) | 2 | 173429126 | 0.029 | 3.54 (1.03,14.13) |
| rs6788054 | 0.0079 | A/G | A (0.1048) | 3 | 187451619 | 0.32 | 2.16 (0.48,11.1) |
| rs4672781 | 0.0079 | A/G | A (0.1476) | 2 | 216436563 | 0.0014 | 6.23 (1.76,28.19) |
| rs2948556 | 0.0079 | A/G | G (0.3762) | 2 | 226835132 | 0.01 | 2.88 (1.22,6.99) |
| rs3806215 | 0.0079 | A/G | A (0.7238) | 1 | 229502565 | 0.037 | 2.57 (0.99,7.17) |
| rs12479184 | 0.0079 | A/C | C (0.6714) | 2 | 240171688 | 0.093 | 2.26 (0.89,6.07) |
| rs657074 | 0.008 | A/G | G (0.0941) | 12 | 3920871 | 0.023 | 4.18 (1.13,19.46) |
| rs4725269 | 0.008 | A/C | C (0.5619) | 7 | 10133654 | 0.017 | 2.81 (1.16,7.12) |
| rs1848038 | 0.008 | A/G | A (0.4667) | 4 | 16352655 | 0.08 | 2.09 (0.9,4.92) |
| rs248 | 0.008 | A/G | A (0.9278) | 8 | 19855106 | 0.14 | 4 (0.67,42.46) |
| rs7198577 | 0.008 | A/G | A (0.381) | 16 | 22766504 | 0.03 | 2.45 (1.04,5.88) |
| rs1418759 | 0.008 | A/G | G (0.1571) | 10 | 28573724 | 0.18 | 2.17 (0.67,7.37) |
| rs2081727 | 0.008 | A/G | G (0.2714) | 22 | 31690605 | 0.05 | 2.42 (0.94,6.43) |
| rs2268247 | 0.008 | A/G | G (0.0905) | 21 | 34098840 | 0.18 | 3.23 (0.6,32.73) |
| rs2839591 | 0.008 | A/G | G (0.6333) | 21 | 43159655 | 0.11 | 2 (0.84,4.89) |
| rs2256817 | 0.008 | A/G | G (0.6089) | 21 | 44539814 | 0.0022 | 3.85 (1.51,10.46) |
| rs6949474 | 0.008 | A/G | G (0.5667) | 7 | 48107203 | 0.05 | 2.27 (0.96,5.55) |
| rs4865140 | 0.008 | A/C | A (0.8571) | 4 | 57378127 | 0.0035 | 11.74 (1.64,517.13) |
| rs2039970 | 0.008 | A/G | G (0.3238) | 13 | 59808292 | 0.13 | 2 (0.79,5.11) |
| rs1486503 | 0.008 | A/G | G (0.3905) | 4 | 61784732 | 0.03 | 2.48 (1.06,5.92) |
| rs12854171 | 0.008 | A/G | A (0.1) | 13 | 61815188 | 0.0019 | 14.52 (1.88,658.49) |
| rs1871238 | 0.008 | A/C | A (0.2381) | 2 | 70485969 | 0.26 | 1.81 (0.68,4.9) |
| rs6424414 | 0.008 | A/G | G (0.6429) | 1 | 71167491 | 0.0043 | 3.57 (1.39,9.89) |
| rs1514399 | 0.008 | A/G | A (0.2476) | 4 | 71271883 | 0.088 | 2.19 (0.88,5.57) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs7816119 | 0.008 | A/G | G (0.2429) | 8 | 73942565 | 0.0021 | 4.54 (1.57,14.52) |
| rs3738846 | 0.008 | A/G | G (0.7816) | 2 | 74003460 | 0.022 | 3.3 (1.13,11.12) |
| rs7330431 | 0.008 | A/G | G (0.1952) | 13 | 74765908 | 0.13 | 2.36 (0.79,7.41) |
| rs2328943 | 0.008 | A/G | G (0.681) | 13 | 74918850 | 0.02 | 2.97 (1.12,8.65) |
| rs7622781 | 0.008 | A/C | A (0.3048) | 3 | 80256257 | 0.053 | 2.4 (0.95,6.23) |
| rs6939852 | 0.008 | A/G | G (0.125) | 6 | 83148896 | 0.045 | 3.39 (0.96,13.83) |
| rs10858840 | 0.008 | A/G | G (0.8952) | 12 | 88165016 | 0.2 | 2.58 (0.61,12.85) |
| rs7687945 | 0.008 | A/G | G (0.4952) | 4 | 91121877 | 0.24 | 1.62 (0.7,3.8) |
| rs6720836 | 0.008 | A/G | A (0.5429) | 2 | 101979046 | 0.081 | 2.04 (0.88,4.81) |
| rs2299356 | 0.008 | A/G | G (0.5049) | 7 | 102903773 | 0.003 | 3.38 (1.41,8.4) |
| rs6937943 | 0.008 | A/G | A (0.1905) | 6 | 103706730 | 0.02 | 3.63 (1.15,12.83) |
| rs1330001 | 0.008 | A/G | A (0.8333) | 10 | 109656676 | 0.0034 | 7.53 (1.62,71.51) |
| rs3757759 | 0.008 | A/C | C (0.6476) | 7 | 127288765 | 0.0058 | 3.19 (1.32,8.1) |
| rs10086471 | 0.008 | A/G | A (0.5381) | 8 | 127461308 | 0.082 | 2.01 (0.87,4.72) |
| rs2268545 | 0.008 | A/G | A (0.3524) | 1 | 165100676 | 0.1 | 2.01 (0.83,4.97) |
| rs4265959 | 0.008 | A/G | A (0.1286) | 2 | 237235049 | 0.00078 | 10.37 (2.12,100.84) |
| rs4571012 | 0.008 | A/C | A (0.1286) | 2 | 237235806 | 0.00078 | 10.37 (2.12,100.84) |
| rs7582574 | 0.008 | A/G | G (0.1286) | 2 | 237242365 | 0.00078 | 10.37 (2.12,100.84) |
| rs2074265 | 0.0081 | A/C | A (0.519) | 19 | 15094581 | 0.0066 | 3.03 (1.28,7.38) |
| rs1390669 | 0.0081 | A/G | A (0.9476) | 5 | 21667361 | 0.07 | Inf (0.7,Inf) |
| rs1689825 | 0.0081 | A/G | G (0.25) | 17 | 27982433 | 0.088 | 2.45 (0.85,7.31) |
| rs404890 | 0.0081 | A/C | A (0.3286) | 6 | 32306845 | 0.15 | 1.85 (0.77,4.53) |
| rs2849015 | 0.0081 | A/G | A (0.3286) | 6 | 32306914 | 0.15 | 1.85 (0.77,4.53) |
| rs1501727 | 0.0081 | A/G | A (0.8238) | 5 | 33599737 | 0.025 | 3.92 (1.15,17.32) |
| rs10495818 | 0.0081 | A/G | G (0.7429) | 2 | 34501650 | 0.38 | 1.58 (0.61,4.33) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2045014 | 0.0081 | A/C | A (0.1373) | 15 | 34777452 | 0.015 | 4.07 (1.2,16.14) |
| rs846381 | 0.0081 | A/G | G (0.2714) | 7 | 41944281 | 0.0058 | 4.45 (1.45,15.49) |
| rs3934824 | 0.0081 | A/G | A (0.4) | 2 | 48984859 | 0.01 | 2.86 (1.2,6.98) |
| rs11896677 | 0.0081 | A/G | G (0.5381) | 2 | 50124682 | 0.076 | 2.13 (0.9,5.21) |
| rs6545138 | 0.0081 | A/G | G (0.5381) | 2 | 50133158 | 0.076 | 2.13 (0.9,5.21) |
| rs7181032 | 0.0081 | A/G | A (0.0857) | 15 | 55801999 | 0.13 | 5.66 (0.69,263.42) |
| rs10490098 | 0.0081 | A/G | A (0.1442) | 2 | 59041592 | 0.017 | 3.83 (1.13,15.15) |
| rs1952202 | 0.0081 | A/G | G (0.8286) | 14 | 69431827 | 0.022 | 5.76 (1.2,55.34) |
| rs11869620 | 0.0081 | A/G | G (0.5476) | 17 | 70326171 | 0.053 | 2.19 (0.94,5.17) |
| rs214260 | 0.0081 | A/G | G (0.8619) | 14 | 72732382 | 0.087 | 2.6 (0.81,9.94) |
| rs961196 | 0.0081 | A/G | A (0.2095) | 14 | 90204414 | 0.035 | 2.82 (1.02,8.26) |
| rs986178 | 0.0081 | A/G | A (0.1058) | 10 | 124079065 | 0.019 | 6.01 (1.12,61.09) |
| rs753353 | 0.0081 | A/G | A (0.6346) | 11 | 134167337 | 0.042 | 2.45 (0.99,6.39) |
| rs4397357 | 0.0081 | A/G | G (0.219) | 8 | 135829416 | 0.018 | 3.23 (1.13,9.87) |
| rs728937 | 0.0081 | A/G | A (0.5286) | 5 | 146465875 | 0.12 | 1.91 (0.83,4.48) |
| rs2390732 | 0.0081 | A/G | G (0.3905) | 2 | 169431474 | 0.049 | 2.31 (0.99,5.48) |
| rs6857566 | 0.0081 | A/G | G (0.2714) | 4 | 181875811 | 0.52 | 1.39 (0.55,3.49) |
| rs3096851 | 0.0081 | A/C | C (0.3571) | 2 | 204589388 | 0.035 | 2.61 (1.06,6.62) |
| rs782257 | 0.0082 | A/G | A (0.1952) | unknown | 0 | 0.053 | 2.63 (0.9,8.18) |
| rs10864368 | 0.0082 | A/G | G (0.519) | 1 | 8852579 | 0.03 | 2.45 (1.04,5.88) |
| rs2876687 | 0.0082 | A/G | A (0.3854) | 6 | 25320537 | 0.019 | 2.83 (1.12,7.34) |
| rs12254001 | 0.0082 | A/G | A (0.2571) | 10 | 28651569 | 0.05 | 2.42 (0.94,6.43) |
| rs4533436 | 0.0082 | A/G | G (0.5048) | 2 | 29591363 | 0.082 | 2.01 (0.87,4.72) |
| rs8000058 | 0.0082 | A/G | G (0.7905) | 13 | 30174225 | 0.0081 | 4.58 (1.36,20.06) |
| rs6490477 | 0.0082 | A/C | A (0.7905) | 13 | 30174586 | 0.0081 | 4.58 (1.36,20.06) |
| rs11052055 | 0.0082 | A/G | G (0.2857) | 12 | 32576176 | 0.13 | 2 (0.79,5.11) |
| rs909446 | 0.0082 | A/G | G (0.4905) | 21 | 35962518 | 0.032 | 2.44 (1.05,5.81) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2018721 | 0.0082 | A/G | G (0.3095) | 21 | 36344836 | 0.028 | 2.69 (1.03,7.31) |
| rs10793449 | 0.0082 | A/G | A (0.7333) | 10 | 43416766 | 0.18 | 2.08 (0.76,6.17) |
| rs4695718 | 0.0082 | A/G | A (0.8905) | 4 | 44253865 | 0.0066 | 10.63 (1.46,470.67) |
| rs13144404 | 0.0082 | A/G | G (0.8905) | 4 | 44276613 | 0.0066 | 10.63 (1.46,470.67) |
| rs2066843 | 0.0082 | A/G | A (0.2857) | 16 | 49302700 | 0.05 | 2.42 (0.94,6.43) |
| rs4801054 | 0.0082 | A/G | G (0.419) | 18 | 52569790 | 0.075 | 2.09 (0.89,4.96) |
| rs614397 | 0.0082 | A/G | A (0.1619) | 11 | 63640561 | 0.029 | 3.54 (1.03,14.13) |
| rs6564251 | 0.0082 | A/G | A (0.9381) | 16 | 71866705 | 0.16 | 5.72 (0.54,290.23) |
| rs2590943 | 0.0082 | A/G | A (0.1952) | 1 | 72586477 | 0.053 | 2.63 (0.9,8.18) |
| rs4944158 | 0.0082 | A/G | A (0.3619) | 11 | 76703887 | 0.075 | 2.06 (0.87,4.93) |
| rs6453423 | 0.0082 | A/G | A (0.2381) | 5 | 78304645 | 0.071 | 2.45 (0.93,6.69) |
| rs2559658 | 0.0082 | A/G | A (0.1952) | 10 | 79415275 | 0.27 | 1.64 (0.62,4.35) |
| rs17085898 | 0.0082 | A/G | A (0.2524) | 9 | 82797193 | 0.13 | 2 (0.78,5.22) |
| rs7765824 | 0.0082 | A/C | A (0.6667) | 6 | 119191337 | 0.099 | 2.13 (0.86,5.57) |
| rs12513015 | 0.0082 | A/C | A (0.8221) | 4 | 137951259 | 0.031 | 4.31 (1.1,24.99) |
| rs6535932 | 0.0082 | A/G | G (0.5481) | 4 | 154907268 | 0.02 | 2.56 (1.1,6.1) |
| rs6846231 | 0.0082 | A/G | A (0.1238) | 4 | 165168017 | 0.052 | 3.13 (0.89,12.67) |
| rs547641 | 0.0082 | A/G | A (0.0762) | 1 | 204545264 | 0.041 | 7.53 (0.98,341.68) |
| rs9308908 | 0.0082 | A/G | A (0.7573) | 2 | 239732433 | 0.0063 | 4.15 (1.35,15.46) |
| rs2959802 | 0.0083 | A/G | A (0.4375) | 8 | 6459403 | 0.05 | 2.31 (0.99,5.52) |
| rs1801133 | 0.0083 | A/G | A (0.4238) | 1 | 11790644 | 0.077 | 2.15 (0.93,5.08) |
| rs1516913 | 0.0083 | A/G | G (0.7048) | 2 | 18453127 | 0.096 | 2.11 (0.83,5.69) |
| rs6751655 | 0.0083 | A/G | G (0.1095) | 2 | 24067129 | 0.2 | 2.58 (0.61,12.85) |
| rs6036746 | 0.0083 | A/G | G (0.2095) | 20 | 24284795 | 0.058 | 2.55 (0.91,7.53) |
| rs1474734 | 0.0083 | A/G | G (0.2095) | 20 | 24293144 | 0.058 | 2.55 (0.91,7.53) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2418453 | 0.0083 | A/G | G (0.6238) | 18 | 25932285 | 0.013 | 3.14 (1.22,8.7) |
| rs2837773 | 0.0083 | A/C | C (0.3857) | 21 | 40961880 | 0.026 | 2.62 (1.1,6.39) |
| rs6008384 | 0.0083 | A/G | A (0.8286) | 22 | 44976467 | 0.032 | 4.13 (1.05,23.93) |
| rs6519993 | 0.0083 | A/G | A (0.8286) | 22 | 44976998 | 0.032 | 4.13 (1.05,23.93) |
| rs6022204 | 0.0083 | A/G | A (0.9381) | 20 | 51052745 | 0.13 | 3.62 (0.56,39.69) |
| rs12932558 | 0.0083 | A/G | G (0.5857) | 16 | 54600670 | 0.00018 | 4.91 (1.96,13.19) |
| rs11862192 | 0.0083 | A/G | A (0.8143) | 16 | 56122970 | 0.0024 | 5.3 (1.6,23.09) |
| rs7571389 | 0.0083 | A/G | G (0.8286) | 2 | 62159102 | 0.043 | 3.32 (0.96,14.81) |
| rs878927 | 0.0083 | A/G | A (0.5952) | 8 | 73428781 | 0.081 | 2.06 (0.89,4.88) |
| rs2297210 | 0.0083 | A/C | A (0.581) | 13 | 74796597 | 0.0016 | 3.81 (1.55,9.93) |
| rs2274736 | 0.0083 | A/G | A (0.7) | 14 | 88008405 | 0.099 | 2.13 (0.86,5.57) |
| rs483333 | 0.0083 | A/C | C (0.2381) | 11 | 95590836 | 0.26 | 1.81 (0.68,4.9) |
| rs11732439 | 0.0083 | A/C | A (0.2381) | 4 | 97864848 | 0.053 | 2.4 (0.95,6.23) |
| rs1145411 | 0.0083 | A/G | G (0.419) | 11 | 99752169 | 0.00044 | 4.18 (1.75,10.38) |
| rs9322528 | 0.0083 | A/G | A (0.5238) | 6 | 156045639 | 0.01 | 2.91 (1.24,7.03) |
| rs9392465 | 0.0084 | A/C | A (0.6286) | 6 | 3107377 | 0.0036 | 3.86 (1.47,11.19) |
| rs10495540 | 0.0084 | A/G | A (0.7837) | 2 | 6721854 | 0.0017 | 6.41 (1.71,36.21) |
| rs7790025 | 0.0084 | A/C | A (0.281) | 7 | 7674750 | 0.053 | 2.4 (0.95,6.23) |
| rs742453 | 0.0084 | A/G | A (0.2571) | 20 | 16900341 | 0.033 | 2.63 (1.05,6.79) |
| rs2829443 | 0.0084 | A/G | A (0.4143) | 21 | 25164231 | 0.01 | 2.91 (1.24,7.03) |
| rs2154618 | 0.0084 | A/G | G (0.4143) | 21 | 25174725 | 0.01 | 2.91 (1.24,7.03) |
| rs2242752 | 0.0084 | A/G | A (0.4857) | 21 | 35904622 | 0.032 | 2.44 (1.05,5.81) |
| rs2242753 | 0.0084 | A/G | A (0.4857) | 21 | 35907988 | 0.032 | 2.44 (1.05,5.81) |
| rs6071961 | 0.0084 | A/C | C (0.3667) | 20 | 38231161 | 0.042 | 2.41 (1.01,5.86) |
| rs3746250 | 0.0084 | A/G | A (0.6796) | 19 | 40527422 | 0.056 | 2.44 (0.94,6.81) |
| rs7075288 | 0.0084 | A/G | A (0.4) | 10 | 43390593 | 0.0065 | 3 (1.28,7.24) |
| rs2123978 | 0.0084 | A/C | A (0.9038) | 2 | 52861638 | 0.013 | 10.16 (1.4,450.25) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs929809 | 0.0084 | A/G | A (0.619) | 5 | 56927014 | 0.032 | 2.42 (1.02,5.92) |
| rs1505664 | 0.0084 | A/G | A (0.4857) | 4 | 61961111 | 0.033 | 2.41 (1.03,5.78) |
| rs1333099 | 0.0084 | A/G | A (0.419) | 13 | 72589237 | 0.03 | 2.52 (1.08,6) |
| rs8054840 | 0.0084 | A/C | A (0.1238) | 16 | 73408413 | 0.1 | 2.57 (0.77,9.39) |
| rs11892930 | 0.0084 | A/G | G (0.6106) | 2 | 78187841 | 0.0087 | 3.12 (1.24,8.37) |
| rs2616646 | 0.0084 | A/G | G (0.1298) | 10 | 78559423 | 0.12 | 3.02 (0.75,14.69) |
| rs8024569 | 0.0084 | A/G | G (0.4143) | 15 | 94425267 | 0.018 | 2.7 (1.15,6.49) |
| rs6889798 | 0.0084 | A/G | G (0.9143) | 5 | 97513897 | 0.051 | 4.09 (0.91,25.4) |
| rs1888578 | 0.0084 | A/G | G (0.7095) | 14 | 100188208 | 0.047 | 2.63 (0.94,8.21) |
| rs11591110 | 0.0084 | A/C | C (0.8333) | 1 | 104920693 | 0.052 | 3.07 (0.97,11.61) |
| rs4730273 | 0.0084 | A/C | C (0.7238) | 7 | 107073470 | 0.0036 | 4.45 (1.46,16.54) |
| rs17133647 | 0.0084 | A/C | C (0.0583) | 5 | 111061008 | 0.02 | Inf (1.13,Inf) |
| rs10509928 | 0.0084 | A/C | A (0.1429) | 10 | 112462093 | 0.029 | 3.54 (1.03,14.13) |
| rs17154831 | 0.0084 | A/G | A (0.1714) | 10 | 128150265 | 0.029 | 3.54 (1.03,14.13) |
| rs2318832 | 0.0084 | A/C | A (0.9) | 8 | 140075791 | 0.15 | 3.06 (0.74,18.19) |
| rs2291442 | 0.0084 | A/G | A (0.281) | 5 | 175950061 | 0.018 | 2.9 (1.14,7.65) |
| rs2241262 | 0.0084 | A/G | A (0.7667) | 2 | 207853281 | 0.11 | 2.26 (0.8,7.12) |
| rs2260032 | 0.0084 | A/G | A (0.481) | 1 | 218493874 | 0.12 | 1.96 (0.85,4.59) |
| rs1915275 | 0.0084 | A/G | G (0.1524) | 1 | 235473832 | 0.17 | 2.25 (0.65,8.38) |
| rs893465 | 0.0085 | A/G | G (0.851) | 8 | 4749173 | 0.031 | 4.31 (1.1,24.99) |
| rs12631463 | 0.0085 | A/G | A (0.1905) | 3 | 9302287 | 0.02 | 3.63 (1.15,12.83) |
| rs969485 | 0.0085 | A/G | G (0.2619) | 11 | 13359619 | 0.17 | 2.01 (0.75,5.58) |
| rs822261 | 0.0085 | A/G | G (0.519) | 8 | 16597092 | 0.17 | 1.81 (0.78,4.25) |
| rs3802329 | 0.0085 | A/C | C (0.6667) | 8 | 19311007 | 0.16 | 1.87 (0.79,4.58) |
| rs2247559 | 0.0085 | A/G | G (0.3048) | 18 | 23081954 | 0.08 | 2.21 (0.85,5.88) |
| rs6713865 | 0.0085 | A/G | G (0.1381) | 2 | 23811459 | 0.016 | 3.97 (1.18,15.68) |
| rs6497748 | 0.0085 | A/G | G (0.4667) | 16 | 24595962 | 0.05 | 2.33 (0.99,5.64) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs9613212 | 0.0085 | A/G | A (0.6619) | 22 | 25282328 | 0.0043 | 3.57 (1.39,9.89) |
| rs79137 | 0.0085 | A/G | G (0.3333) | 22 | 25982061 | 0.065 | 2.19 (0.91,5.41) |
| rs206319 | 0.0085 | A/G | G (0.3238) | 13 | 31884219 | 0.042 | 2.41 (1.01,5.86) |
| rs7899442 | 0.0085 | A/G | G (0.649) | 10 | 32995111 | 0.044 | 2.47 (1.02,6.26) |
| rs8016889 | 0.0085 | A/G | A (0.3762) | 14 | 37316191 | 0.057 | 2.36 (0.95,5.99) |
| rs7163861 | 0.0085 | A/G | A (0.7286) | 15 | 59420077 | 0.27 | 1.81 (0.68,5.12) |
| rs3843712 | 0.0085 | A/G | G (0.9327) | 16 | 65491654 | 0.038 | 5.13 (0.91,53.09) |
| rs4565172 | 0.0085 | A/G | A (0.6143) | 5 | 66908117 | 0.047 | 2.32 (0.95,5.88) |
| rs7779884 | 0.0085 | A/C | C (0.5) | 7 | 76594044 | 0.11 | 1.92 (0.81,4.63) |
| rs6862921 | 0.0085 | A/C | A (0.6619) | 5 | 85074731 | 0.066 | 2.28 (0.92,5.94) |
| rs305413 | 0.0085 | A/G | G (0.2857) | 1 | 87944137 | 0.0027 | 4.28 (1.54,12.89) |
| rs8006346 | 0.0085 | A/G | A (0.1381) | 14 | 91783836 | 0.006 | 5.04 (1.39,23.16) |
| rs1514797 | 0.0085 | A/G | A (0.8381) | 12 | 99683430 | 0.022 | 5.76 (1.2,55.34) |
| rs7947643 | 0.0085 | A/G | A (0.8571) | 11 | 100069553 | 0.0064 | 6.92 (1.48,65.88) |
| rs3945761 | 0.0085 | A/G | G (0.6394) | 9 | 112826948 | 0.03 | 2.52 (1.05,6.26) |
| rs2197777 | 0.0085 | A/G | G (0.2571) | 12 | 124356099 | 0.0084 | 3.3 (1.25,9.2) |
| rs1961455 | 0.0085 | A/G | A (0.7048) | 8 | 135014863 | 0.016 | 3.25 (1.18,10.05) |
| rs12141387 | 0.0085 | A/G | G (0.4038) | 1 | 143728657 | 0.079 | 2.04 (0.88,4.84) |
| rs670307 | 0.0085 | A/G | A (0.8) | 6 | 153236046 | 0.047 | 2.63 (0.94,8.21) |
| rs11753617 | 0.0085 | A/G | G (0.1905) | 6 | 158171530 | 0.024 | 3.02 (1.13,8.43) |
| rs10916290 | 0.0085 | A/G | A (0.2238) | 1 | 220591110 | 0.071 | 2.45 (0.93,6.69) |
| rs11784100 | 0.0086 | A/G | G (0.3333) | 8 | 929604 | 0.015 | 2.85 (1.19,7.01) |
| rs2879747 | 0.0086 | A/C | C (0.8714) | 11 | 5471468 | 0.089 | 3.4 (0.84,20.02) |
| rs4701728 | 0.0086 | A/G | G (0.7667) | 5 | 6439041 | 0.057 | 2.41 (0.92,6.72) |
| rs12671658 | 0.0086 | A/C | C (0.4571) | 7 | 7615521 | 0.17 | 1.81 (0.77,4.36) |
| rs4926285 | 0.0086 | A/C | A (0.4952) | 19 | 13392611 | 0.0062 | 3.13 (1.32,7.71) |
| rs209440 | 0.0086 | A/C | A (0.4762) | 16 | 22700629 | 0.17 | 1.81 (0.78,4.28) |

| **SNP** | **p-value (RelDAS28)** | **polymorphism** | **Allele associated with non-response (Allele Frequency)** | **Chr** | **Physical Positions** | **p-values (Non-responders vs. Good responders)** | **Odds Ratio (OR) being non-responders** |
|---|---|---|---|---|---|---|---|
| rs11627187 | 0.0086 | A/C | C (0.0714) | 14 | 23821574 | 0.041 | 7.53 (0.98,341.68) |
| rs79037 | 0.0086 | A/G | A (0.2905) | 22 | 25757733 | 0.018 | 2.9 (1.14,7.65) |
| rs4692195 | 0.0086 | A/G | G (0.2548) | 4 | 26995034 | 0.012 | 4.87 (1.34,22.38) |
| rs4572240 | 0.0086 | A/G | A (0.4286) | 13 | 29648616 | 0.019 | 2.61 (1.12,6.24) |
| rs10418577 | 0.0086 | A/G | A (0.4952) | 19 | 34217651 | 0.018 | 2.75 (1.16,6.75) |
| rs1476002 | 0.0086 | A/G | A (0.1716) | 22 | 35955288 | 0.13 | 2.36 (0.79,7.41) |
| rs6451309 | 0.0086 | A/G | G (0.3952) | 5 | 36815495 | 0.075 | 2.09 (0.89,4.96) |
| rs1995281 | 0.0086 | A/G | G (0.3952) | 5 | 36816808 | 0.075 | 2.09 (0.89,4.96) |
| rs1452786 | 0.0086 | A/G | A (0.2981) | 2 | 50328486 | 0.11 | 2.17 (0.79,6.19) |
| rs292445 | 0.0086 | A/G | G (0.2952) | 18 | 54048700 | 0.056 | 2.39 (0.96,6.08) |
| rs843713 | 0.0086 | A/G | A (0.3) | 2 | 54417721 | 0.039 | 2.75 (1.02,7.72) |
| rs4935116 | 0.0086 | A/G | A (0.2095) | 10 | 56071276 | 0.001 | 4.97 (1.73,15.85) |
| rs1561168 | 0.0086 | A/G | A (0.9333) | 1 | 64827340 | 0.035 | 5.3 (0.94,54.86) |
| rs11180738 | 0.0086 | A/G | G (0.8429) | 12 | 74579927 | 0.043 | 3.32 (0.96,14.81) |
| rs2328944 | 0.0086 | A/C | A (0.519) | 13 | 74967577 | 0.17 | 1.84 (0.79,4.31) |
| rs7329914 | 0.0086 | A/G | G (0.4905) | 13 | 77931326 | 0.019 | 2.65 (1.13,6.44) |
| rs7992108 | 0.0086 | A/G | G (0.4952) | 13 | 77952391 | 0.019 | 2.65 (1.13,6.44) |
| rs3759964 | 0.0086 | A/G | A (0.8592) | 16 | 82547590 | 0.0062 | 7.22 (1.54,68.87) |
| rs9351015 | 0.0086 | A/G | A (0.1863) | 6 | 85650780 | 0.041 | 3.03 (0.94,10.82) |
| rs237435 | 0.0086 | A/G | G (0.2) | 1 | 93742555 | 0.015 | 3.4 (1.14,11.05) |
| rs10487135 | 0.0086 | A/G | G (0.0905) | 7 | 94802586 | 0.04 | 4.69 (0.95,45.79) |
| rs1109164 | 0.0086 | A/G | G (0.0913) | 2 | 106063704 | 0.0093 | Inf (1.41,Inf) |
| rs7333523 | 0.0086 | A/G | G (0.7048) | 13 | 107790856 | 0.032 | 2.78 (1.04,8.1) |
| rs11806304 | 0.0086 | A/G | A (0.0769) | 1 | 183104522 | 0.018 | Inf (1.17,Inf) |
| rs2740349 | 0.0087 | A/G | G (0.119) | 17 | 595248 | 0.039 | 3.97 (1.05,18.68) |
| rs7940766 | 0.0087 | A/G | A (0.4857) | 11 | 2007134 | 0.081 | 2.06 (0.89,4.88) |
| rs996708 | 0.0087 | A/G | A (0.7667) | 20 | 2558285 | 0.051 | 2.59 (0.97,7.58) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs16945681 | 0.0087 | A/G | A (0.8762) | 18 | 3926384 | 0.07 | 3.03 (0.86,13.64) |
| rs4798491 | 0.0087 | A/G | G (0.6619) | 18 | 6748921 | 0.013 | 3.14 (1.22,8.7) |
| rs1926381 | 0.0087 | A/G | G (0.7667) | 9 | 7329425 | 0.045 | 2.83 (1.01,8.79) |
| rs1542538 | 0.0087 | A/G | A (0.1048) | 19 | 8213183 | 0.0038 | 8.18 (1.61,80.71) |
| rs7111218 | 0.0087 | A/G | A (0.8702) | 11 | 8372699 | 0.054 | 3.76 (0.95,21.94) |
| rs12200114 | 0.0087 | A/G | A (0.3048) | 6 | 8966417 | 0.35 | 1.63 (0.58,4.6) |
| rs12616649 | 0.0087 | A/G | A (0.101) | 2 | 23802565 | 0.12 | 3.02 (0.75,14.69) |
| rs900820 | 0.0087 | A/C | A (0.3571) | 4 | 27485354 | 0.0041 | 3.39 (1.38,8.57) |
| rs728088 | 0.0087 | A/G | G (0.7238) | 14 | 37282222 | 0.079 | 2.41 (0.89,7.09) |
| rs10507466 | 0.0087 | A/G | A (0.2952) | 13 | 37361657 | 0.064 | 2.19 (0.89,5.48) |
| rs10015266 | 0.0087 | A/G | A (0.9095) | 4 | 41476761 | 0.012 | 6.33 (1.34,60.52) |
| rs2104142 | 0.0087 | A/G | A (0.3238) | 6 | 43317717 | 0.064 | 2.19 (0.89,5.48) |
| rs4922536 | 0.0087 | A/C | A (0.5143) | 10 | 48168383 | 0.24 | 1.69 (0.73,3.95) |
| rs6458687 | 0.0087 | A/G | A (0.3238) | 6 | 49511241 | 0.003 | 3.77 (1.5,9.89) |
| rs9369923 | 0.0087 | A/G | G (0.7644) | 6 | 49987964 | 0.057 | 2.41 (0.92,6.72) |
| rs12910051 | 0.0087 | A/G | A (0.8952) | 15 | 56367817 | 0.022 | 5.76 (1.2,55.34) |
| rs10877921 | 0.0087 | A/G | A (0.1381) | 12 | 61526939 | 0.00077 | 16.57 (2.2,744.21) |
| rs10514948 | 0.0087 | A/C | A (0.119) | 5 | 62077938 | 0.23 | 2.38 (0.63,9.99) |
| rs10136789 | 0.0087 | A/G | A (0.8857) | 14 | 62426006 | 0.15 | 3.06 (0.74,18.19) |
| rs7320723 | 0.0087 | A/G | A (0.0905) | 13 | 96727155 | 0.069 | 3.49 (0.89,16.63) |
| rs2073791 | 0.0087 | A/C | A (0.7667) | 7 | 103716940 | 0.015 | 3.38 (1.16,11.33) |
| rs7539636 | 0.0087 | A/G | G (0.6422) | 1 | 105840144 | 0.065 | 2.26 (0.9,5.91) |
| rs971850 | 0.0087 | A/G | G (0.4952) | 8 | 111116637 | 0.17 | 1.79 (0.78,4.18) |
| rs1794815 | 0.0087 | A/G | A (0.3095) | 6 | 112070396 | 0.0045 | 3.61 (1.37,10.03) |
| rs3920209 | 0.0087 | A/G | G (0.3143) | 8 | 115081540 | 0.033 | 2.63 (1.05,6.79) |
| rs1716466 | 0.0087 | A/G | G (0.4048) | 12 | 118326580 | 0.31 | 1.61 (0.68,3.83) |
| rs784852 | 0.0087 | A/G | A (0.4048) | 12 | 118358595 | 0.31 | 1.61 (0.68,3.83) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs3922897 | 0.0087 | A/G | G (0.4) | 8 | 121624945 | 0.011 | 2.8 (1.2,6.72) |
| rs10100935 | 0.0087 | A/G | G (0.3762) | 8 | 143423627 | 0.048 | 2.25 (0.95,5.38) |
| rs1467662 | 0.0087 | A/G | A (0.181) | 1 | 153878247 | 0.061 | 2.9 (0.89,10.47) |
| rs7689380 | 0.0087 | A/C | C (0.1106) | 4 | 172591034 | 0.43 | 1.57 (0.49,5.18) |
| rs2493151 | 0.0087 | A/G | A (0.2429) | 1 | 227185296 | 0.5 | 1.48 (0.55,3.96) |
| rs10488610 | 0.0088 | A/G | A (0.919) | 7 | 2380175 | 0.0038 | 8.18 (1.61,80.71) |
| rs11778675 | 0.0088 | A/G | G (0.5952) | 8 | 3283036 | 0.0058 | 3.19 (1.32,8.1) |
| rs12487836 | 0.0088 | A/C | A (0.0571) | 3 | 6900815 | 0.13 | 3.62 (0.56,39.69) |
| rs2697684 | 0.0088 | A/G | A (0.2381) | 4 | 17112227 | 0.0097 | 3.55 (1.26,10.8) |
| rs10225081 | 0.0088 | A/G | A (0.1202) | 7 | 22637964 | 0.1 | 2.57 (0.77,9.39) |
| rs962656 | 0.0088 | A/G | G (0.1095) | 12 | 25095620 | 0.014 | 5.38 (1.27,32.42) |
| rs1872686 | 0.0088 | A/G | G (0.9286) | 16 | 34285183 | 0.07 | 4.44 (0.75,47.1) |
| rs470113 | 0.0088 | A/G | G (0.1762) | 22 | 39054114 | 0.02 | 3.63 (1.15,12.83) |
| rs12484697 | 0.0088 | A/G | A (0.1762) | 22 | 39060972 | 0.02 | 3.63 (1.15,12.83) |
| rs8128440 | 0.0088 | A/G | A (0.6238) | 21 | 43197434 | 0.076 | 2.13 (0.9,5.21) |
| rs10495927 | 0.0088 | A/G | G (0.3095) | 2 | 46264150 | 0.14 | 2 (0.81,5.02) |
| rs10486748 | 0.0088 | A/G | G (0.8762) | 7 | 50800146 | 0.089 | 3.4 (0.84,20.02) |
| rs497254 | 0.0088 | A/G | G (0.3667) | 18 | 54029897 | 0.03 | 2.45 (1.04,5.88) |
| rs6567128 | 0.0088 | A/G | A (0.2356) | 18 | 55706948 | 0.058 | 2.55 (0.91,7.53) |
| rs715064 | 0.0088 | A/G | G (0.0952) | 20 | 59084122 | 0.17 | 2.92 (0.58,19.09) |
| rs1073572 | 0.0088 | A/G | G (0.7571) | 17 | 66467656 | 0.014 | 4.24 (1.26,18.66) |
| rs6566644 | 0.0088 | A/C | A (0.4857) | 18 | 68461160 | 0.012 | 2.75 (1.18,6.58) |
| rs12361940 | 0.0088 | A/G | A (0.9333) | 11 | 72634968 | 0.28 | 2.38 (0.44,16.17) |
| rs7192903 | 0.0088 | A/G | G (0.0905) | 16 | 76122403 | 0.027 | 4.71 (1.09,28.83) |
| rs2277547 | 0.0088 | A/G | G (0.3333) | 15 | 76869486 | 0.041 | 2.39 (0.99,5.9) |
| rs1359011 | 0.0088 | A/C | C (0.8738) | 6 | 85746544 | 0.0093 | 5.38 (1.39,30.92) |
| rs1150345 | 0.0088 | A/C | A (0.2429) | 11 | 95014185 | 0.19 | 1.82 (0.72,4.68) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs7176226 | 0.0088 | A/G | G (0.3381) | 15 | 96491926 | 0.13 | 2 (0.79,5.11) |
| rs11193816 | 0.0088 | A/G | G (0.8447) | 10 | 109711663 | 0.0058 | 6.89 (1.45,66.11) |
| rs7008254 | 0.0088 | A/G | G (0.381) | 8 | 109972734 | 0.015 | 2.85 (1.19,7.01) |
| rs12660061 | 0.0088 | A/G | A (0.6429) | 6 | 130273301 | 0.0071 | 3.39 (1.28,9.85) |
| rs354843 | 0.0088 | A/G | G (0.3029) | 4 | 142546409 | 0.0078 | 3.47 (1.32,9.67) |
| rs6724627 | 0.0088 | A/G | G (0.6429) | 2 | 171333769 | 0.0051 | 3.34 (1.33,8.92) |
| rs897124 | 0.0088 | A/G | A (0.4619) | 2 | 221688067 | 0.053 | 2.19 (0.95,5.22) |
| rs3750729 | 0.0089 | A/G | A (0.481) | 10 | 1058802 | 0.053 | 2.19 (0.93,5.29) |
| rs923376 | 0.0089 | A/G | A (0.6571) | 17 | 3339836 | 0.14 | 1.97 (0.77,5.32) |
| rs4724592 | 0.0089 | A/C | C (0.8095) | 7 | 5168192 | 0.0059 | 5.34 (1.4,30.46) |
| rs12103335 | 0.0089 | A/G | G (0.8429) | 16 | 7084057 | 0.01 | 4.92 (1.28,28.16) |
| rs8093929 | 0.0089 | A/C | A (0.2524) | 18 | 7344669 | 0.011 | 3.41 (1.25,9.87) |
| rs2531853 | 0.0089 | A/C | C (0.4905) | 17 | 9143979 | 0.012 | 2.75 (1.17,6.62) |
| rs4792349 | 0.0089 | A/C | A (0.5) | 17 | 13138311 | 0.03 | 2.48 (1.06,5.92) |
| rs10840837 | 0.0089 | A/G | A (0.4952) | 12 | 17787481 | 0.018 | 2.75 (1.16,6.75) |
| rs1945327 | 0.0089 | A/G | G (0.0857) | 11 | 21190821 | 0.023 | 8.53 (1.13,383.04) |
| rs756803 | 0.0089 | A/G | A (0.0571) | 16 | 22916766 | 0.46 | 2.34 (0.39,24.81) |
| rs7213724 | 0.0089 | A/C | C (0.3571) | 17 | 28713170 | 0.041 | 2.39 (0.99,5.9) |
| rs4934677 | 0.0089 | A/C | C (0.1942) | 10 | 35188685 | 0.15 | 2.13 (0.74,6.35) |
| rs11073332 | 0.0089 | A/G | G (0.3429) | 15 | 36604906 | 0.0092 | 3.22 (1.25,8.7) |
| rs986868 | 0.0089 | A/G | A (0.6095) | 15 | 55740856 | 0.17 | 1.81 (0.78,4.28) |
| rs517948 | 0.0089 | A/G | G (0.4714) | 18 | 63701127 | 0.00076 | 4.11 (1.72,10.19) |
| rs1856317 | 0.0089 | A/G | G (0.3429) | 1 | 66727993 | 0.19 | 1.82 (0.72,4.68) |
| rs11126175 | 0.0089 | A/C | A (0.7452) | 2 | 68235778 | 0.0078 | 3.88 (1.35,12.96) |
| rs10777224 | 0.0089 | A/C | C (0.1762) | 12 | 89010777 | 0.061 | 2.9 (0.89,10.47) |
| rs12303180 | 0.0089 | A/G | G (0.1762) | 12 | 89025034 | 0.061 | 2.9 (0.89,10.47) |
| rs1336708 | 0.0089 | A/G | G (0.2524) | 13 | 101763004 | 0.016 | 2.95 (1.14,7.98) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs6974751 | 0.0089 | A/C | A (0.1) | 7 | 105821060 | 0.094 | 3.49 (0.74,22.16) |
| rs2295279 | 0.0089 | A/G | G (0.8238) | 6 | 108132109 | 0.07 | 3.03 (0.86,13.64) |
| rs2185029 | 0.0089 | A/G | G (0.4667) | 1 | 111798072 | 0.17 | 1.76 (0.76,4.14) |
| rs878073 | 0.0089 | A/G | A (0.1429) | 5 | 112398210 | 0.029 | 3.54 (1.03,14.13) |
| rs4644087 | 0.0089 | A/C | A (0.4806) | 6 | 127522847 | 0.032 | 2.44 (1.05,5.81) |
| rs13152384 | 0.0089 | A/C | C (0.3429) | 4 | 137975676 | 0.11 | 1.91 (0.81,4.55) |
| rs1933695 | 0.0089 | A/G | A (0.181) | 1 | 189496477 | 0.2 | 2.11 (0.69,6.69) |
| rs10775437 | 0.009 | A/G | G (0.9476) | 18 | 6833259 | 0.23 | 4.77 (0.55,226.37) |
| rs2168856 | 0.009 | A/G | A (0.5952) | 17 | 9336422 | 0.24 | 1.7 (0.73,4.01) |
| rs317891 | 0.009 | A/G | A (0.1857) | 4 | 15801405 | 0.02 | 3.63 (1.15,12.83) |
| rs2052937 | 0.009 | A/G | G (0.245) | 2 | 26448345 | 0.66 | 1.29 (0.5,3.3) |
| rs11750666 | 0.009 | A/G | G (0.5333) | 5 | 31577962 | 0.24 | 1.64 (0.71,3.82) |
| rs422951 | 0.009 | A/G | G (0.3786) | 6 | 32296361 | 0.11 | 1.97 (0.83,4.72) |
| rs1320301 | 0.009 | A/G | G (0.6714) | 19 | 54634244 | 0.21 | 1.87 (0.75,4.89) |
| rs7844344 | 0.009 | A/G | G (0.619) | 8 | 58869556 | 0.11 | 2.03 (0.83,5.17) |
| rs11677744 | 0.009 | A/G | A (0.8095) | 2 | 62105089 | 0.084 | 2.83 (0.89,10.75) |
| rs17102710 | 0.009 | A/G | A (0.1952) | 12 | 65268381 | 0.053 | 2.63 (0.9,8.18) |
| rs641909 | 0.009 | A/G | G (0.2905) | 1 | 70299381 | 0.0092 | 3.22 (1.25,8.7) |
| rs9318562 | 0.009 | A/G | G (0.4904) | 13 | 77945084 | 0.019 | 2.65 (1.13,6.44) |
| rs4732558 | 0.009 | A/G | G (0.3667) | 7 | 83813266 | 0.0041 | 3.39 (1.38,8.57) |
| rs8036454 | 0.009 | A/G | G (0.8714) | 15 | 93891749 | 0.012 | 9.56 (1.3,426.01) |
| rs3102542 | 0.009 | A/G | G (0.4381) | 8 | 96919800 | 0.077 | 2.15 (0.93,5.08) |
| rs2817676 | 0.009 | A/G | A (0.3333) | 10 | 98809307 | 0.0041 | 3.39 (1.38,8.57) |
| rs11123239 | 0.009 | A/G | A (0.281) | 2 | 114811718 | 0.04 | 2.39 (0.98,5.97) |
| rs7093850 | 0.009 | A/G | A (0.4216) | 10 | 130197583 | 0.028 | 2.5 (1.05,6.04) |
| rs6999950 | 0.009 | A/G | A (0.5524) | 8 | 136004065 | 0.02 | 2.56 (1.1,6.1) |
| rs2400170 | 0.009 | A/C | C (0.5143) | 5 | 144526123 | 0.081 | 2.06 (0.89,4.88) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs10514622 | 0.009 | A/C | C (0.4905) | 2 | 169354285 | 0.078 | 2.12 (0.91,5.07) |
| rs6657332 | 0.009 | A/C | C (0.6286) | 1 | 234156936 | 0.044 | 2.47 (1.02,6.26) |
| rs1045714 | 0.0091 | A/G | A (0.8365) | 7 | 2426823 | 0.0034 | 7.53 (1.62,71.51) |
| rs9405611 | 0.0091 | A/G | G (0.6202) | 6 | 3089027 | 0.0034 | 3.84 (1.45,11.17) |
| rs399377 | 0.0091 | A/G | A (0.2238) | 1 | 4745748 | 0.039 | 2.75 (1.02,7.72) |
| rs3915499 | 0.0091 | A/G | G (0.719) | 16 | 15818244 | 0.38 | 1.58 (0.61,4.33) |
| rs242243 | 0.0091 | A/G | G (0.8762) | 17 | 17190331 | 0.0035 | 11.74 (1.64,517.13) |
| rs4321005 | 0.0091 | A/G | G (0.5245) | 12 | 17806489 | 0.011 | 2.93 (1.24,7.21) |
| rs10219714 | 0.0091 | A/G | G (0.3942) | 12 | 19010402 | 0.045 | 2.31 (0.97,5.61) |
| rs6075750 | 0.0091 | A/G | A (0.2381) | 20 | 20953246 | 0.024 | 3.02 (1.13,8.43) |
| rs7175782 | 0.0091 | A/G | G (0.6857) | 15 | 22702180 | 0.057 | 2.41 (0.92,6.72) |
| rs10519249 | 0.0091 | A/G | G (0.6095) | 15 | 48055662 | 0.05 | 2.27 (0.96,5.55) |
| rs12326572 | 0.0091 | A/C | C (0.0714) | 18 | 52642214 | 0.04 | 4.69 (0.95,45.79) |
| rs11763184 | 0.0091 | A/G | G (0.4095) | 7 | 56307345 | 0.00034 | 4.4 (1.83,11.05) |
| rs774381 | 0.0091 | A/G | G (0.2667) | 12 | 65726355 | 0.071 | 2.45 (0.93,6.69) |
| rs1912118 | 0.0091 | A/G | G (0.5714) | 2 | 69866038 | 0.0058 | 3.19 (1.32,8.1) |
| rs1517176 | 0.0091 | A/G | G (0.0952) | 16 | 72142031 | 0.39 | 1.67 (0.44,6.51) |
| rs10498910 | 0.0091 | A/G | A (0.8762) | 6 | 77627595 | 0.067 | 4.18 (0.83,41.29) |
| rs6678176 | 0.0091 | A/G | A (0.2905) | 1 | 99712848 | 0.064 | 2.19 (0.89,5.48) |
| rs399945 | 0.0091 | A/C | C (0.3204) | 5 | 106686308 | 0.019 | 2.79 (1.09,7.36) |
| rs10507266 | 0.0091 | A/G | G (0.9286) | 12 | 114915295 | 0.0049 | Inf (1.7,Inf) |
| rs1186361 | 0.0091 | A/G | A (0.6165) | 10 | 128915566 | 0.0032 | 3.42 (1.4,8.75) |
| rs1837955 | 0.0091 | A/G | G (0.2476) | 3 | 138896119 | 0.018 | 2.9 (1.14,7.65) |
| rs13237949 | 0.0091 | A/G | G (0.5952) | 7 | 139559688 | 0.044 | 2.47 (1.02,6.26) |
| rs2304066 | 0.0091 | A/G | G (0.281) | 5 | 145760885 | 0.03 | 2.65 (1.04,7.01) |
| rs4662539 | 0.0091 | A/G | A (0.4381) | 2 | 148266411 | 0.032 | 2.42 (1.02,5.92) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs836650 | 0.0091 | A/G | G (0.2667) | 2 | 168587006 | 0.08 | 2.21 (0.85,5.88) |
| rs4274574 | 0.0091 | A/G | G (0.7667) | 2 | 176055303 | 0.087 | 2.6 (0.81,9.94) |
| rs4566335 | 0.0091 | A/G | G (0.7667) | 2 | 176087341 | 0.087 | 2.6 (0.81,9.94) |
| rs3817586 | 0.0091 | A/G | G (0.081) | 1 | 183035955 | 0.02 | Inf (1.36,Inf) |
| rs7608493 | 0.0091 | A/G | G (0.5762) | 2 | 193336155 | 0.24 | 1.7 (0.73,4.01) |
| rs1898917 | 0.0091 | A/G | G (0.5762) | 2 | 193364326 | 0.24 | 1.7 (0.73,4.01) |
| rs12459191 | 0.0092 | A/G | A (0.2019) | 19 | 3889782 | 0.0081 | 4.43 (1.33,17.33) |
| rs7973281 | 0.0092 | A/G | A (0.6238) | 12 | 5149428 | 0.039 | 2.58 (1.02,6.91) |
| rs4425309 | 0.0092 | A/C | C (0.1923) | 4 | 6042361 | 0.058 | 2.55 (0.91,7.53) |
| rs4804164 | 0.0092 | A/G | G (0.101) | 19 | 11363291 | 0.32 | 2.16 (0.48,11.1) |
| rs1357558 | 0.0092 | A/G | G (0.8667) | 8 | 16857203 | 0.0035 | 11.74 (1.64,517.13) |
| rs974821 | 0.0092 | A/G | A (0.9381) | 7 | 17346165 | 0.17 | 2.92 (0.58,19.09) |
| rs5997592 | 0.0092 | A/G | G (0.6311) | 22 | 28933650 | 0.044 | 2.47 (1.02,6.26) |
| rs764882 | 0.0092 | A/G | G (0.1429) | 4 | 37204750 | 0.014 | 5.38 (1.27,32.42) |
| rs4832928 | 0.0092 | A/G | G (0.6667) | 4 | 37467955 | 0.066 | 2.28 (0.92,5.94) |
| rs1506888 | 0.0092 | A/G | G (0.6905) | 12 | 56683240 | 0.21 | 1.74 (0.69,4.58) |
| rs1445594 | 0.0092 | A/G | A (0.6857) | 13 | 57573300 | 0.0075 | 3.35 (1.31,9.28) |
| rs1329699 | 0.0092 | A/G | G (0.2647) | 13 | 60196350 | 0.016 | 3.1 (1.2,8.38) |
| rs872311 | 0.0092 | A/G | G (0.635) | 18 | 64326366 | 0.099 | 2.13 (0.87,5.37) |
| rs17516342 | 0.0092 | A/G | G (0.2095) | 13 | 66580629 | 0.13 | 2.36 (0.79,7.41) |
| rs9294664 | 0.0092 | A/G | G (0.6048) | 6 | 66654970 | 0.11 | 2.08 (0.86,5.17) |
| rs10518014 | 0.0092 | A/G | G (0.7429) | 4 | 67428674 | 0.099 | 2.13 (0.86,5.57) |
| rs1331500 | 0.0092 | A/G | G (0.5143) | 9 | 90443086 | 0.032 | 2.5 (1.07,5.98) |
| rs11613634 | 0.0092 | A/G | A (0.3702) | 12 | 95761539 | 0.056 | 2.39 (0.96,6.08) |
| rs6707103 | 0.0092 | A/G | A (0.1048) | 2 | 103453029 | 0.57 | 1.41 (0.39,5.12) |
| rs10496370 | 0.0092 | A/G | G (0.1048) | 2 | 103475855 | 0.57 | 1.41 (0.39,5.12) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs7835207 | 0.0092 | A/G | A (0.8048) | 8 | 143205285 | 0.026 | 3.61 (1.05,16.04) |
| rs2927585 | 0.0092 | A/G | A (0.9476) | 5 | 152769814 | 0.13 | 3.62 (0.56,39.69) |
| rs3095966 | 0.0092 | A/G | A (0.5762) | 4 | 181877011 | 0.17 | 1.81 (0.77,4.36) |
| rs860317 | 0.0092 | A/G | G (0.3619) | 3 | 196745138 | 0.048 | 2.25 (0.95,5.38) |
| rs2012243 | 0.0092 | A/G | A (0.4048) | 2 | 217237593 | 0.018 | 2.7 (1.15,6.49) |
| rs6657371 | 0.0092 | A/G | G (0.7905) | 1 | 229473793 | 0.039 | 2.92 (0.99,9.86) |
| rs13345388 | 0.0093 | A/G | G (0.5144) | 19 | 702553 | 0.077 | 2.12 (0.91,5.02) |
| rs2740810 | 0.0093 | A/C | C (0.2429) | 8 | 3825663 | 0.28 | 1.66 (0.66,4.22) |
| rs285545 | 0.0093 | A/G | G (0.3) | 2 | 4219600 | 0.15 | 1.84 (0.75,4.56) |
| rs11780345 | 0.0093 | A/G | G (0.5979) | 8 | 23107911 | 0.21 | 1.81 (0.74,4.49) |
| rs1912842 | 0.0093 | A/C | A (0.1524) | 4 | 34695492 | 0.3 | 1.86 (0.6,6.01) |
| rs2291782 | 0.0093 | A/G | G (0.7048) | 5 | 40867565 | 0.0071 | 3.39 (1.28,9.85) |
| rs1492310 | 0.0093 | A/G | G (0.5524) | 12 | 42835702 | 0.02 | 2.56 (1.1,6.1) |
| rs559396 | 0.0093 | A/G | A (0.4381) | 2 | 45011401 | 0.0036 | 3.23 (1.37,7.83) |
| rs6508077 | 0.0093 | A/G | A (0.1905) | 18 | 47384120 | 0.052 | 3.13 (0.89,12.67) |
| rs4239943 | 0.0093 | A/G | G (0.6515) | 22 | 47640120 | 0.096 | 2.13 (0.86,5.54) |
| rs4806674 | 0.0093 | A/G | A (0.481) | 19 | 59153350 | 0.032 | 2.42 (1.02,5.92) |
| rs3017664 | 0.0093 | A/G | G (0.3571) | 18 | 74947085 | 0.018 | 2.64 (1.12,6.39) |
| rs610243 | 0.0093 | A/G | G (0.1095) | 16 | 76505221 | 0.2 | 2.58 (0.61,12.85) |
| rs6973950 | 0.0093 | A/C | C (0.4476) | 7 | 76586481 | 0.17 | 1.83 (0.79,4.29) |
| rs3743057 | 0.0093 | A/G | G (0.75) | 15 | 76876062 | 0.01 | 4.05 (1.31,15.17) |
| rs651861 | 0.0093 | A/C | A (0.4476) | 11 | 78359137 | 0.0063 | 3.07 (1.31,7.41) |
| rs7169450 | 0.0093 | A/G | A (0.3952) | 15 | 94445469 | 0.018 | 2.7 (1.15,6.49) |
| rs7674376 | 0.0093 | A/G | A (0.3077) | 4 | 97880516 | 0.045 | 2.31 (0.97,5.61) |
| rs1391201 | 0.0093 | A/G | G (0.5714) | 8 | 109772471 | 0.01 | 2.99 (1.24,7.59) |
| rs2466650 | 0.0093 | A/G | A (0.1619) | 11 | 123615652 | 0.12 | 2.45 (0.78,8.21) |
| rs553995 | 0.0093 | A/G | A (0.1619) | 11 | 123700734 | 0.12 | 2.45 (0.78,8.21) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs31483 | 0.0093 | A/G | A (0.0777) | 5 | 126774176 | 0.0093 | Inf (1.41,Inf) |
| rs2952888 | 0.0093 | A/G | A (0.3238) | 4 | 131193492 | 0.0055 | 3.12 (1.31,7.63) |
| rs2645954 | 0.0093 | A/G | G (0.8524) | 3 | 131710140 | 0.087 | 2.6 (0.81,9.94) |
| rs4909911 | 0.0093 | A/G | G (0.6857) | 8 | 135634437 | 0.087 | 2.24 (0.86,6.29) |
| rs7008121 | 0.0093 | A/G | A (0.6857) | 8 | 135635086 | 0.087 | 2.24 (0.86,6.29) |
| rs1511693 | 0.0094 | A/C | A (0.2115) | 18 | 1070791 | 0.15 | 2.06 (0.71,6.2) |
| rs2253698 | 0.0094 | A/G | A (0.5476) | 20 | 1493617 | 0.0066 | 3.03 (1.28,7.38) |
| rs201061 | 0.0094 | A/G | A (0.5429) | 6 | 6675511 | 0.25 | 1.61 (0.7,3.74) |
| rs1862458 | 0.0094 | A/G | G (0.8) | 19 | 8930792 | 0.024 | 3.14 (1.08,10.57) |
| rs1862460 | 0.0094 | A/C | A (0.8) | 19 | 8930892 | 0.024 | 3.14 (1.08,10.57) |
| rs3745255 | 0.0094 | A/G | G (0.6733) | 19 | 10529452 | 0.0048 | 3.37 (1.35,8.85) |
| rs897360 | 0.0094 | A/G | A (0.6068) | 11 | 11720424 | 0.028 | 2.63 (1.09,6.68) |
| rs6962343 | 0.0094 | A/G | A (0.3398) | 7 | 13683988 | 0.028 | 2.52 (1.06,6.12) |
| rs2822786 | 0.0094 | A/G | A (0.3221) | 21 | 14876464 | 0.038 | 2.5 (1.02,6.25) |
| rs6960653 | 0.0094 | A/G | G (0.3238) | 7 | 15207873 | 0.13 | 2 (0.78,5.22) |
| rs134938 | 0.0094 | A/G | A (0.119) | 22 | 25756911 | 0.052 | 3.13 (0.89,12.67) |
| rs144848 | 0.0094 | A/C | A (0.702) | 13 | 31804729 | 0.0073 | 3.83 (1.3,13) |
| rs7613051 | 0.0094 | A/G | G (0.7524) | 3 | 33040343 | 0.057 | 2.41 (0.92,6.72) |
| rs296901 | 0.0094 | A/G | G (0.4714) | 13 | 33268101 | 0.011 | 2.83 (1.2,6.89) |
| rs7236910 | 0.0094 | A/G | G (0.1381) | 18 | 41133085 | 0.23 | 2.38 (0.63,9.99) |
| rs949016 | 0.0094 | A/G | G (0.25) | 18 | 43173030 | 0.029 | 2.72 (1.04,7.41) |
| rs4260852 | 0.0094 | A/C | C (0.9) | 7 | 48128244 | 0.04 | 4.69 (0.95,45.79) |
| rs930516 | 0.0094 | A/G | G (0.6857) | 13 | 70225002 | 0.061 | 2.41 (0.95,6.48) |
| rs1822059 | 0.0094 | A/G | G (0.0619) | 8 | 73828294 | 0.13 | 5.66 (0.69,263.42) |
| rs2958414 | 0.0094 | A/C | A (0.0619) | 8 | 73849754 | 0.13 | 5.66 (0.69,263.42) |
| rs4969205 | 0.0094 | A/G | A (0.4571) | 17 | 74109718 | 0.034 | 2.34 (1.01,5.58) |
| rs727708 | 0.0094 | A/G | G (0.5238) | 7 | 102924895 | 0.01 | 2.95 (1.26,7.12) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs6859143 | 0.0094 | A/G | A (0.681) | 5 | 103418563 | 0.002 | 4.77 (1.57,17.68) |
| rs7444078 | 0.0094 | A/G | G (0.4857) | 5 | 104226959 | 0.011 | 2.94 (1.25,7.11) |
| rs4980245 | 0.0094 | A/G | A (0.8416) | 10 | 124783587 | 0.017 | 4.7 (1.2,27.19) |
| rs7827545 | 0.0094 | A/G | G (0.7041) | 8 | 135635749 | 0.074 | 2.48 (0.91,7.35) |
| rs12473643 | 0.0094 | A/C | C (0.5529) | 2 | 166874079 | 0.031 | 2.43 (1.04,5.82) |
| rs4955779 | 0.0094 | A/C | C (0.5905) | 3 | 169100499 | 0.12 | 1.99 (0.85,4.75) |
| rs11782142 | 0.0095 | A/C | A (0.3857) | 8 | 889726 | 0.0055 | 3.12 (1.31,7.63) |
| rs4319993 | 0.0095 | A/G | G (0.3429) | 20 | 1250727 | 0.014 | 2.84 (1.17,7.09) |
| rs6053374 | 0.0095 | A/G | G (0.3905) | 20 | 5335789 | 0.041 | 2.39 (0.99,5.9) |
| rs3935435 | 0.0095 | A/G | G (0.7864) | 1 | 6506441 | 0.21 | 2.11 (0.68,7.36) |
| rs6035707 | 0.0095 | A/C | A (0.9417) | 20 | 20770149 | 0.04 | Inf (1.08,Inf) |
| rs4692256 | 0.0095 | A/G | G (0.419) | 4 | 27420987 | 6.70E-05 | 5.24 (2.13,13.5) |
| rs7815173 | 0.0095 | A/G | A (0.4857) | 8 | 27939020 | 0.019 | 2.68 (1.15,6.41) |
| rs6571608 | 0.0095 | A/G | G (0.3143) | 14 | 33232524 | 0.53 | 1.41 (0.57,3.48) |
| rs1522590 | 0.0095 | A/G | A (0.6143) | 2 | 33966714 | 0.12 | 1.86 (0.8,4.45) |
| rs11632283 | 0.0095 | A/C | A (0.4515) | 15 | 35395326 | 0.078 | 2.12 (0.91,5.07) |
| rs4720444 | 0.0095 | A/G | G (0.4524) | 7 | 42988995 | 0.01 | 2.95 (1.26,7.12) |
| rs6749927 | 0.0095 | A/G | G (0.3714) | 2 | 43396207 | 0.15 | 1.84 (0.75,4.56) |
| rs1912155 | 0.0095 | A/G | G (0.6524) | 17 | 43903666 | 0.11 | 2.08 (0.86,5.17) |
| rs2221747 | 0.0095 | A/G | A (0.3429) | 16 | 58229372 | 0.033 | 2.63 (1.05,6.79) |
| rs7445990 | 0.0095 | A/G | A (0.8) | 5 | 62939856 | 0.014 | 4.24 (1.26,18.66) |
| rs1968681 | 0.0095 | A/G | G (0.3238) | 11 | 79971598 | 0.035 | 2.61 (1.06,6.62) |
| rs604948 | 0.0095 | A/G | G (0.5667) | 9 | 80108681 | 0.053 | 2.19 (0.95,5.22) |
| rs4635138 | 0.0095 | A/G | G (0.125) | 12 | 88798361 | 0.064 | 4.67 (0.78,49.6) |
| rs11736229 | 0.0095 | A/G | G (0.2427) | 4 | 92988975 | 0.006 | 3.66 (1.34,10.66) |
| rs1371360 | 0.0095 | A/C | C (0.1714) | 4 | 95044420 | 0.031 | 2.92 (1.01,9) |
| rs4559216 | 0.0095 | A/G | G (0.1238) | 8 | 98963286 | 0.41 | 1.65 (0.48,5.83) |

| **SNP** | **p-value (RelDAS28)** | **polymorphism** | **Allele associated with non-response (Allele Frequency)** | **Chr** | **Physical Positions** | **p-values (Non-responders vs. Good responders)** | **Odds Ratio (OR) being non-responders** |
|---|---|---|---|---|---|---|---|
| rs2298752 | 0.0095 | A/G | A (0.2115) | 4 | 103534249 | 0.088 | 2.19 (0.88,5.57) |
| rs10816965 | 0.0095 | A/G | G (0.9143) | 9 | 110148645 | 0.07 | 4.44 (0.75,47.1) |
| rs1588327 | 0.0095 | A/C | A (0.5288) | 2 | 110248808 | 0.031 | 2.48 (1.06,5.94) |
| rs10885330 | 0.0095 | A/C | A (0.4524) | 10 | 114062134 | 0.081 | 2.04 (0.88,4.81) |
| rs7027882 | 0.0095 | A/G | A (0.7238) | 9 | 115030627 | 0.037 | 2.57 (0.99,7.17) |
| rs7588987 | 0.0095 | A/C | C (0.5286) | 2 | 125228134 | 0.081 | 2.04 (0.88,4.81) |
| rs7098205 | 0.0095 | A/G | G (0.1381) | 10 | 130329472 | 0.051 | 4.09 (0.91,25.4) |
| rs6878439 | 0.0095 | A/G | G (0.8143) | 5 | 136968115 | 0.084 | 2.83 (0.89,10.75) |
| rs923738 | 0.0095 | A/G | A (0.2476) | 2 | 140790516 | 0.2 | 1.83 (0.73,4.6) |
| rs1735091 | 0.0095 | A/G | G (0.0905) | 7 | 152433653 | 0.2 | 2.58 (0.61,12.85) |
| rs6540664 | 0.0095 | A/G | A (0.4619) | 1 | 207720288 | 0.019 | 2.65 (1.13,6.44) |
| rs10187555 | 0.0095 | A/G | A (0.3762) | 2 | 218746016 | 0.43 | 1.45 (0.62,3.37) |
| rs3791428 | 0.0095 | A/G | A (0.2163) | 2 | 239776700 | 0.001 | 4.97 (1.73,15.85) |
| rs10165056 | 0.0096 | A/G | G (0.2667) | 2 | 4249382 | 0.14 | 2 (0.81,5.02) |
| rs10795851 | 0.0096 | A/G | G (0.1762) | 10 | 11289246 | 0.0017 | 4.9 (1.61,16.97) |
| rs16961126 | 0.0096 | A/G | A (0.8143) | 16 | 13101912 | 0.096 | 2.5 (0.84,8.52) |
| rs527634 | 0.0096 | A/G | A (0.1667) | 9 | 15191745 | 0.054 | 3.08 (0.87,12.53) |
| rs875462 | 0.0096 | A/G | A (0.7788) | 6 | 15646415 | 0.014 | 4.1 (1.2,18.14) |
| rs9396593 | 0.0096 | A/G | G (0.7788) | 6 | 15700538 | 0.014 | 4.1 (1.2,18.14) |
| rs7004724 | 0.0096 | A/G | A (0.5825) | 8 | 20791419 | 0.17 | 1.75 (0.75,4.11) |
| rs719785 | 0.0096 | A/G | G (0.3714) | 14 | 21048133 | 0.056 | 2.39 (0.96,6.08) |
| rs3098543 | 0.0096 | A/G | A (0.7115) | 15 | 25492753 | 0.059 | 2.38 (0.93,6.43) |
| rs6006393 | 0.0096 | A/G | A (0.1048) | 22 | 28914580 | 0.2 | 2.58 (0.61,12.85) |
| rs1419633 | 0.0096 | A/G | A (0.5381) | 6 | 29481192 | 0.018 | 2.7 (1.15,6.49) |
| rs680798 | 0.0096 | A/G | G (0.3714) | 18 | 30772788 | 0.0055 | 3.14 (1.33,7.65) |
| rs6831060 | 0.0096 | A/C | A (0.6667) | 4 | 34738187 | 0.02 | 2.97 (1.12,8.65) |
| rs11944089 | 0.0096 | A/G | A (0.6714) | 4 | 34742846 | 0.02 | 2.97 (1.12,8.65) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs619660 | 0.0096 | A/C | C (0.2071) | 3 | 43002953 | 0.014 | 3.57 (1.2,11.66) |
| rs4469289 | 0.0096 | A/G | G (0.068) | 6 | 45344252 | 0.074 | 6.83 (0.86,313.42) |
| rs12432604 | 0.0096 | A/G | A (0.7762) | 14 | 57285593 | 0.18 | 1.93 (0.7,5.74) |
| rs1787492 | 0.0096 | A/C | A (0.732) | 18 | 62256840 | 0.15 | 2.24 (0.76,7.27) |
| rs4671614 | 0.0096 | A/G | G (0.1857) | 2 | 64955107 | 0.035 | 3.26 (1.02,11.61) |
| rs3169646 | 0.0096 | A/G | A (0.3476) | 17 | 67911078 | 0.17 | 1.75 (0.75,4.17) |
| rs12322717 | 0.0096 | A/G | A (0.8143) | 12 | 72862357 | 0.062 | 2.7 (0.91,9.17) |
| rs7417520 | 0.0096 | A/C | A (0.098) | 1 | 83861311 | 0.15 | 2.55 (0.7,10.52) |
| rs2599545 | 0.0096 | A/G | G (0.4095) | 15 | 85659703 | 0.026 | 2.62 (1.1,6.39) |
| rs10223241 | 0.0096 | A/G | A (0.8041) | 5 | 88448144 | 0.0099 | 3.95 (1.26,14.97) |
| rs9445243 | 0.0096 | A/G | G (0.4333) | 6 | 95160301 | 0.053 | 2.19 (0.95,5.22) |
| rs9301167 | 0.0096 | A/G | A (0.5095) | 13 | 106316852 | 0.081 | 2.04 (0.88,4.81) |
| rs17203139 | 0.0096 | A/C | A (0.0571) | 3 | 120687907 | 0.11 | 3.7 (0.71,36.88) |
| rs2242601 | 0.0096 | A/G | A (0.6875) | 7 | 142610661 | 0.098 | 2.11 (0.84,5.53) |
| rs1029942 | 0.0096 | A/G | A (0.9476) | 5 | 148290344 | 0.07 | Inf (0.7,Inf) |
| rs1416620 | 0.0096 | A/G | G (0.1762) | 1 | 213285526 | 0.012 | 4.49 (1.21,20.85) |
| rs17682 | 0.0097 | A/G | G (0.649) | 2 | 4731323 | 0.071 | 2.17 (0.89,5.51) |
| rs2432793 | 0.0097 | A/G | A (0.3381) | 6 | 5337377 | 0.1 | 2.02 (0.84,4.94) |
| rs4813825 | 0.0097 | A/G | A (0.3714) | 20 | 7245420 | 0.00034 | 4.38 (1.81,11.08) |
| rs7219526 | 0.0097 | A/G | A (0.2286) | 17 | 9236034 | 0.24 | 1.82 (0.66,5.07) |
| rs6033987 | 0.0097 | A/G | A (0.9095) | 20 | 14536168 | 0.023 | 8.53 (1.13,383.04) |
| rs4757441 | 0.0097 | A/G | A (0.2381) | 11 | 16859250 | 0.23 | 1.82 (0.64,5.3) |
| rs6102209 | 0.0097 | A/G | G (0.2667) | 20 | 38969725 | 0.0056 | 3.46 (1.37,9.08) |
| rs751933 | 0.0097 | A/G | A (0.4952) | 6 | 39272099 | 0.24 | 1.68 (0.73,3.91) |
| rs12405914 | 0.0097 | A/G | G (0.0657) | 1 | 44540668 | 0.0094 | Inf (1.57,Inf) |
| rs1198316 | 0.0097 | A/G | G (0.9038) | 13 | 49268206 | 0.15 | 2.92 (0.71,17.37) |
| rs3848265 | 0.0097 | A/C | A (0.2286) | 16 | 58777335 | 0.08 | 2.21 (0.85,5.88) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs4776280 | 0.0097 | A/G | G (0.2163) | 15 | 64188464 | 0.073 | 2.59 (0.82,8.73) |
| rs1790956 | 0.0097 | A/C | A (0.4515) | 18 | 65613506 | 0.003 | 3.51 (1.46,8.84) |
| rs10878918 | 0.0097 | A/G | A (0.1298) | 12 | 67806523 | 0.15 | 2.65 (0.73,10.91) |
| rs7948192 | 0.0097 | A/G | G (0.0583) | 11 | 73244833 | 0.65 | 2.08 (0.23,25.89) |
| rs7907311 | 0.0097 | A/G | G (0.1476) | 10 | 81849467 | 0.13 | 2.36 (0.79,7.41) |
| rs12156341 | 0.0097 | A/C | C (0.0762) | 8 | 84861182 | 0.11 | 3.7 (0.71,36.88) |
| rs17370612 | 0.0097 | A/G | G (0.3048) | 12 | 85763081 | 0.0084 | 3.3 (1.25,9.2) |
| rs3847535 | 0.0097 | A/G | A (0.29) | 11 | 92197672 | 0.0058 | 3.47 (1.37,9.14) |
| rs4400148 | 0.0097 | A/G | A (0.5238) | 5 | 96014543 | 0.02 | 2.56 (1.1,6.1) |
| rs1052270 | 0.0097 | A/G | A (0.6442) | 9 | 97354979 | 0.027 | 2.6 (1.05,6.75) |
| rs10494143 | 0.0097 | A/G | A (0.9126) | 1 | 112179056 | 0.075 | 4.29 (0.72,45.57) |
| rs548032 | 0.0097 | A/G | A (0.6381) | 2 | 127461866 | 0.016 | 2.95 (1.2,7.67) |
| rs11061598 | 0.0097 | A/G | A (0.9238) | 12 | 130542231 | 0.12 | 3.02 (0.75,14.69) |
| rs7844565 | 0.0097 | A/G | A (0.2143) | 8 | 139755924 | 0.061 | 2.9 (0.89,10.47) |
| rs4869777 | 0.0097 | A/G | G (0.0619) | 6 | 153436247 | 0.13 | 5.66 (0.69,263.42) |
| rs10491971 | 0.0098 | A/G | A (0.1286) | 12 | 3638603 | 0.28 | 1.9 (0.57,6.58) |
| rs6679380 | 0.0098 | A/G | G (0.2115) | 1 | 9467504 | 0.0086 | 3.8 (1.34,11.63) |
| rs17150863 | 0.0098 | A/G | A (0.0905) | 10 | 11871117 | 0.02 | Inf (1.36,Inf) |
| rs2380422 | 0.0098 | A/G | G (0.1786) | 2 | 12299987 | 0.13 | 2.35 (0.76,7.83) |
| rs6530859 | 0.0098 | A/G | A (0.2429) | 8 | 15284960 | 0.0052 | 3.9 (1.4,11.81) |
| rs1994952 | 0.0098 | A/G | A (0.2429) | 8 | 15287177 | 0.0052 | 3.9 (1.4,11.81) |
| rs834367 | 0.0098 | A/G | G (0.1683) | 6 | 19110272 | 0.0081 | 4.43 (1.33,17.33) |
| rs916048 | 0.0098 | A/C | C (0.6649) | 14 | 21860760 | 0.09 | 2.17 (0.85,5.8) |
| rs227011 | 0.0098 | A/G | G (0.0714) | 14 | 22060602 | 0.3 | 2.78 (0.5,28.7) |
| rs10221443 | 0.0098 | A/G | A (0.2048) | 18 | 25458399 | 0.0021 | 4.54 (1.57,14.52) |
| rs1602737 | 0.0098 | A/G | G (0.4429) | 18 | 25560823 | 0.078 | 2.12 (0.91,5.07) |
| rs10512729 | 0.0098 | A/G | G (0.319) | 5 | 40262019 | 0.1 | 2.04 (0.86,4.93) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs6907771 | 0.0098 | A/G | G (0.4135) | 6 | 42327272 | 0.03 | 2.45 (1.04,5.88) |
| rs1338559 | 0.0098 | A/G | A (0.4) | 10 | 43350623 | 0.012 | 2.75 (1.18,6.58) |
| rs9931467 | 0.0098 | A/G | G (0.1952) | 16 | 49892985 | 0.16 | 2.03 (0.73,5.85) |
| rs7195911 | 0.0098 | A/G | A (0.3333) | 16 | 54543081 | 0.011 | 3.13 (1.26,8.06) |
| rs1177285 | 0.0098 | A/G | A (0.6048) | 2 | 61261788 | 0.11 | 2.08 (0.86,5.17) |
| rs566908 | 0.0098 | A/G | G (0.5049) | 11 | 64593946 | 0.0035 | 3.29 (1.38,8.09) |
| rs8009944 | 0.0098 | A/C | A (0.7115) | 14 | 68109341 | 0.027 | 2.6 (1.05,6.75) |
| rs550005 | 0.0098 | A/C | A (0.8824) | 13 | 68976329 | 0.011 | 10.36 (1.4,462.16) |
| rs7397056 | 0.0098 | A/C | C (0.7452) | 12 | 73040857 | 0.029 | 2.88 (1.03,8.95) |
| rs10206082 | 0.0098 | A/G | A (0.4524) | 2 | 99618542 | 0.011 | 2.86 (1.22,6.89) |
| rs6590809 | 0.0098 | A/C | C (0.8619) | 11 | 100065729 | 0.012 | 6.33 (1.34,60.52) |
| rs1224147 | 0.0098 | A/G | A (0.6952) | 13 | 107754759 | 0.087 | 2.24 (0.86,6.29) |
| rs10849931 | 0.0098 | A/G | A (0.7905) | 12 | 110216528 | 0.073 | 2.44 (0.87,7.65) |
| rs10923615 | 0.0098 | A/C | A (0.7714) | 1 | 118818978 | 0.045 | 2.83 (1.01,8.79) |
| rs11167761 | 0.0098 | A/G | G (0.767) | 5 | 141218527 | 0.069 | 2.56 (0.91,8.04) |
| rs9389972 | 0.0098 | A/G | G (0.8143) | 6 | 142406321 | 0.11 | 2.26 (0.8,7.12) |
| rs1015419 | 0.0098 | A/G | A (0.2143) | 1 | 173499109 | 0.071 | 2.45 (0.93,6.69) |
| rs6043374 | 0.0099 | A/G | G (0.8798) | 20 | 1557952 | 0.006 | 11.06 (1.52,490.22) |
| rs7048976 | 0.0099 | A/C | C (0.5143) | 9 | 2163296 | 0.018 | 2.67 (1.13,6.42) |
| rs10094300 | 0.0099 | A/G | A (0.5476) | 8 | 4074703 | 0.032 | 2.42 (1.02,5.92) |
| rs17379732 | 0.0099 | A/G | A (0.9381) | 6 | 6287179 | 0.16 | 5.72 (0.54,290.23) |
| rs6474967 | 0.0099 | A/C | A (0.4952) | 9 | 15750988 | 0.02 | 2.57 (1.1,6.19) |
| rs7934372 | 0.0099 | A/G | G (0.8524) | 11 | 20375604 | 0.0033 | 5.78 (1.53,32.82) |
| rs2269636 | 0.0099 | A/G | G (0.9286) | 22 | 24593017 | 0.07 | 4.44 (0.75,47.1) |
| rs9982288 | 0.0099 | A/G | A (0.5143) | 21 | 29498951 | 0.012 | 2.75 (1.17,6.62) |
| rs10491998 | 0.0099 | A/G | A (0.1429) | 12 | 40585149 | 0.016 | 3.97 (1.18,15.68) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs2485652 | 0.0099 | A/G | G (0.3939) | 1 | 44264503 | 0.029 | 2.66 (1.04,6.98) |
| rs2330183 | 0.0099 | A/G | G (0.5773) | 21 | 45777720 | 0.17 | 1.79 (0.76,4.26) |
| rs728962 | 0.0099 | A/G | G (0.8125) | 16 | 48889951 | 0.01 | 4.92 (1.28,28.16) |
| rs6097667 | 0.0099 | A/G | A (0.819) | 20 | 51965103 | 0.0034 | 7.53 (1.62,71.51) |
| rs2038406 | 0.0099 | A/G | A (0.4286) | 14 | 59118832 | 0.053 | 2.19 (0.93,5.29) |
| rs984297 | 0.0099 | A/G | A (0.3952) | 8 | 72697290 | 0.0084 | 3.1 (1.29,7.66) |
| rs3904725 | 0.0099 | A/C | A (0.219) | 10 | 84081011 | 0.02 | 3.11 (1.13,9.04) |
| rs11830104 | 0.0099 | A/G | A (0.0905) | 12 | 92532947 | 0.027 | 4.71 (1.09,28.83) |
| rs2660852 | 0.0099 | A/C | A (0.3429) | 12 | 94948016 | 0.025 | 2.61 (1.09,6.43) |
| rs6543092 | 0.0099 | A/G | G (0.2692) | 2 | 101801580 | 0.13 | 2.1 (0.83,5.49) |
| rs1334616 | 0.0099 | A/G | G (0.1476) | 10 | 106349053 | 0.0029 | 5.62 (1.57,25.6) |
| rs4918120 | 0.0099 | A/G | A (0.1476) | 10 | 106372348 | 0.0029 | 5.62 (1.57,25.6) |
| rs534259 | 0.0099 | A/G | G (0.1905) | 10 | 110937567 | 0.039 | 3.97 (1.05,18.68) |
| rs10884707 | 0.0099 | A/G | G (0.1905) | 10 | 110959073 | 0.039 | 3.97 (1.05,18.68) |
| rs979755 | 0.0099 | A/G | A (0.8524) | 4 | 125106582 | 0.07 | 3.03 (0.86,13.64) |
| rs4845394 | 0.0099 | A/G | A (0.381) | 1 | 151621360 | 0.0055 | 3.12 (1.31,7.63) |
| rs2789426 | 0.0099 | A/G | G (0.9) | 1 | 156741132 | 0.17 | 2.5 (0.69,11.44) |
| rs244903 | 0.0099 | A/G | G (0.419) | 5 | 167846088 | 0.01 | 2.88 (1.22,6.99) |
| rs1426938 | 0.0099 | A/G | A (0.3143) | 4 | 175875478 | 0.0079 | 3.1 (1.28,7.73) |
| rs7580839 | 0.0099 | A/G | G (0.7762) | 2 | 197498541 | 0.26 | 1.78 (0.64,5.34) |
| rs11558492 | 0.0099 | A/G | A (0.8429) | 1 | 227714826 | 0.043 | 3.32 (0.96,14.81) |
| rs4735919 | 0.01 | A/G | G (0.1571) | 8 | 712942 | 0.053 | 2.63 (0.9,8.18) |
| rs91315 | 0.01 | A/G | A (0.4541) | 5 | 1908301 | 0.17 | 1.81 (0.78,4.28) |
| rs6046513 | 0.01 | A/G | A (0.7952) | 20 | 19913641 | 0.32 | 1.93 (0.63,6.72) |
| rs7162153 | 0.01 | A/G | A (0.1048) | 15 | 24962153 | 0.017 | 6.21 (1.16,63.16) |
| rs2620705 | 0.01 | A/G | A (0.7905) | 12 | 26131006 | 0.24 | 1.93 (0.67,6.13) |
| rs9611324 | 0.01 | A/G | G (0.1857) | 22 | 39105662 | 0.02 | 3.63 (1.15,12.83) |

| SNP | p-value (RelDAS28) | polymorphism | Allele associated with non-response (Allele Frequency) | Chr | Physical Positions | p-values (Non-responders vs. Good responders) | Odds Ratio (OR) being non-responders |
|---|---|---|---|---|---|---|---|
| rs220256 | 0.01 | A/G | A (0.5381) | 21 | 42351168 | 0.032 | 2.44 (1.05,5.81) |
| rs10509319 | 0.01 | A/G | A (0.1429) | 10 | 71072908 | 0.061 | 2.9 (0.89,10.47) |
| rs6983160 | 0.01 | A/C | A (0.6333) | 8 | 73028560 | 0.16 | 1.87 (0.79,4.58) |
| rs1481853 | 0.01 | A/G | A (0.6333) | 8 | 73033665 | 0.16 | 1.87 (0.79,4.58) |
| rs16896680 | 0.01 | A/G | G (0.8398) | 8 | 99223461 | 0.017 | 4.72 (1.21,27.18) |
| rs1417907 | 0.01 | A/G | A (0.6952) | 13 | 107144507 | 0.02 | 2.97 (1.12,8.65) |
| rs3908999 | 0.01 | A/G | A (0.7) | 13 | 107150852 | 0.02 | 2.97 (1.12,8.65) |
| rs2964356 | 0.01 | A/C | A (0.3476) | 5 | 162559574 | 0.015 | 2.85 (1.19,7.01) |
| rs1529127 | 0.01 | A/G | A (0.4952) | 2 | 180775058 | 0.052 | 2.23 (0.96,5.28) |
| rs6821153 | 0.01 | A/C | A (0.4048) | 4 | 187019756 | 0.018 | 2.64 (1.12,6.39) |
| rs963550 | 0.01 | A/C | C (0.7571) | 1 | 202061900 | 0.11 | 2.26 (0.8,7.12) |

It is understood that Table 13 not only includes the heterozygotic SNP genotypes depicted in column 3, but also homozygotic SNP genotypes described in column 4. For example, Table 13 includes not only rs9611324 (A/G) but also rs9611324 (G/G). It is understood that both the the heterozygotic and homozyogotic SNP genotypes of Table 13 are predictive in the methods described herein.

Other Embodiments

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

In view of the foregoing, it will be appreciated that the invention described therein inter alia relates to the following items:

1. A method of predicting the response of a subject to a therapy comprising an anti-TNF agent, the method comprising:

providing a biological sample obtained from a subject that has an immune disorder; and
measuring the expression level of one or more genes in the biological sample, wherein the one or more genes comprise at least one gene selected from the group consisting of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2,
wherein at least one of (i) an elevated expression level, as compared to a healthy individual, of one or more of ANKIB 1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1, or (ii) a reduced expression level, as compared to a healthy individual, of one or more of ANKRD12, CAMK2G-, CASP5, CXorf52, DNAH1, EEA1, FAM44A, FOXJ3, HDAC4, MNT, MXRA7, PTCH1, SEL1L, or SFRS2 predicts that the subject will respond to a therapy comprising an anti-TNF agent.

2. A method of predicting the response of a subject to a therapy comprising an anti-TNF agent, the method comprising:

providing a biological sample obtained from a subject that has an immune disorder; and
measuring the expression level of one or more genes in the biological sample, wherein the one or more genes comprise at least one gene selected from the group consisting of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PITCH1, SEL1L, and SFRS2,
wherein at least one of (i) an elevated expression level, as compared to a healthy individual, of one or more of ANKRD12, CAMK2G-, CASP5, CXorf52, DNAH1, EEA1, FAM44A, FOXJ3, HDAC4, MNT, MXRA7, PITCH1, SEL1L, or SFRS2, or (ii) a reduced expression level, as compared to a healthy individual, of one or more of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 predicts that the subject will not respond to a therapy comprising an anti-TNF agent.

3. A method of treating an immune disorder, the method comprising administering to a subject in need thereof an effective amount of a therapy comprising an anti-TNF agent, wherein the subject has been identified as having at least one of (i) an elevated expression level, as compared to a healthy individual, of one or more of ANKIB1, ARF1, ARF5, BRWD2, CALM2, CLTB, COL4A3BP, C9orf80, EGLN2, HDAC5, LGALS9, MYLIP, PCBP2, PGK1, RBBP4, REF1, RPA3, SERF2, SLC25A39, SRGAP2, TUG1, YIPF6, ZNF294, or ZFP36L1, or (ii) a reduced expression level, as compared to a healthy individual, of one or more of ANKRD12, CAMK2G-, CASP5, CXorf52, DNAH1, EEA1, FAM44A, FOXJ3, HDAC4, MNT, MXRA7, PTCH1, SEL1L, or SFRS2.

4. The method of item 1, comprising:

determining that the expression level of one or more of (i) ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294 are elevated, as compared to a healthy individual, or (ii) ANKRD12, CAMK2G-, CASP5, CXorf52, DNAH1. EEA1, FAM44A, FOXJ3, HDAC4, MNT, MXRA7, PTCH1, SEL1L, or SFRS2 are reduced, as compared to a healthy individual; and
selecting a therapy comprising an anti-TNF agent for the subject.

5. The method of item 2, comprising:

determining that the expression level of one or more of (i) ANKRD12, CAMK2G-, CASP5, CXorf52, DNAH1, EEA1, FAM44A, FOXJ3, HDAC4, MNT, MXRA7, PTCH1, SEL1L, or SFRS2 are elevated, as compared to a healthy individual, or (ii) ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294 are reduced, as compared to a healthy individual; and
selecting a therapy comprising a non-anti-TNF agent for the subject.

6. The method of any of items 1, 2, 4 or 5, wherein the RNA level of the one or more genes is measured.

7. The method of any of items 1, 2, 4 or 5, wherein the protein level of the one or more genes is measured.

8. The method of item 1, further comprising creating a record indicating that the subject is likely to respond to a therapy comprising an anti-TNF agent, if the expression level of one or more of (i) ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 are elevated, as compared to a healthy individual, or (ii) ANKRD12, CAMK2G-, CASP5, CXorf52, DNAH1, EEA1, FAM44A, FOXJ3, HDAC4, MNT, MXRA7, PTCH1, SEL1L, or SFRS2 are reduced, as compared to a healthy individual.

9. The method of item 2, further comprising creating a record indicating that the subject is not likely to respond to a therapy comprising an anti-TNF agent, if the expression level of one or more of (i) ANKRD12, CAMK2G-, CASP5, CXorf52, DNAH1, EEA1, FAM44A, FOXJ3, HDAC4, MNT, MXRA7, PTCH1, SEL1L, or SFRS2 are elevated, as compared to a healthy individual, or (ii) ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 are reduced, as compared to a healthy individual.

10. The method of item 8 or 9, wherein the record is created on a computer readable medium.

11. The method of any of items 1-10, wherein the expression level of at least one gene selected from the group consisting of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PITCH1, SEL1L, and SFRS2 is elevated or reduced, as compared to a healthy individual, by at least about 1.5 fold.

12. The method of any of items 1, 2, or 4-11, wherein the method comprises measuring the expression level of at least five genes selected from the group consisting of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2.

13. The method of item 3, wherein the subject has been identified as having elevated or reduced expression levels, as compared to a healthy individual, of at least five genes selected from the group consisting of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2.

14. The method of item 12 or 13, wherein the at least five genes are selected from the group consisting of ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6.

15. The method of any one of items 12-14, wherein the at least five genes comprise five or more of CLTB, COL4A3BP, CXorf52, FAM44A, MXRA7, PGK1, SFRS2, or YIPF6.

16. The method of any of items 1, 2, or 4-11, wherein the method comprises measuring the expression level of at least eight genes selected from the group consisting of ANKIB 1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PITCH1, SEL1L, and SFRS2.

17. The method of item 16, wherein the at least eight genes comprise eight or more of ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6.

18. The method of item 16 or 17, wherein the at least eight genes comprise CLTB, COL4A3BP, CXorf52, FAM44A, MXRA7, PGK1, SFRS2, and YIPF6.

19. The method of any of items1, 2, or 4-11, wherein the method comprises measuring the expression level of at least 24 genes selected from the group consisting of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2.

20. The method of item 19, wherein the at least 24 genes comprise ANKIB 1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, YIPF6.

21. A method of predicting the response of a subject to a therapy comprising an anti-TNF agent, the method comprising:

providing a biological sample obtained from a subject that has an immune disorder; and
detecting the presence or absence of one or more SNP genotypes in the biological sample, wherein the one or more SNP genotypes comprise at least one SNP genotype selected from the group consisting of rs 11778767 (A/A), rs 11780500 (A/A), rs11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), and rs868856 (G/G),
wherein the presence of one or more of rs11778767 (A/A), rs11780500 (A/A), rs11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), or rs868856 (G/G) predicts that the subject will respond to a therapy comprising an anti-TNF agent.

22. A method of predicting the response of a subject to a therapy comprising an anti-TNF agent, the method comprising:

providing a biological sample obtained from a subject that has an immune disorder; and
detecting the presence or absence of one or more SNP genotypes in the biological sample, wherein the one or more SNP genotypes comprise at least one SNP genotype selected from the group consisting of rs11778767 (A/G), rs 11778767 (G/G), rs11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), rs868856 (A/A), rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs 1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), and any of the SNP genotypes depicted in Table 13,
wherein the presence of one or more of rs11778767 (A/G), rs11778767 (G/G), rs11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), rs868856 (A/A), rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), or any of the SNP genotypes depicted in Table 13 predicts that the subject will not respond to a therapy comprising an anti-TNF agent.

23. A method of treating an immune disorder, the method comprising administering to a subject in need thereof an effective amount of a therapy comprising an anti-TNF agent, wherein the subject has been identified as having one or more single nucleotide polymorphisms (SNPs) genotypes selected from the group consisting of rs11778767 (A/A), rs11780500 (A/A), rs11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), and rs868856 (G/G).

24. The method of item 21, wherein the method comprises detecting the presence or absence of five or more SNP genotypes selected from the group consisting of rs11778767 (A/A), rs11780500 (A/A), rs11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), and rs868856 (G/G).

25. The method of item 22, wherein the method comprises detecting the presence or absence of five or more SNP genotypes selected from the group consisting of rs11778767 (A/G), rs1778767 (G/G), rs11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), and rs868856 (A/A).

26. The method of item 22, wherein the method comprises detecting the presence or absence of five or more SNP genotypes selected from the group consisting of any of rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), and rs6071980 (C/C).

27. The method of any of items 1-26, further comprising prescribing a therapy comprising an anti-TNF agent for the subject.

28. The method of any of items 1-27, further comprising administering to the subject an anti-TNF agent.

29. The method of any of items 1-28, wherein the subject has an inflammatory disorder.

30. The method of any of items 1-29, wherein the subject has rheumatoid arthritis or Crohn's disease.

31. The method of any of items 1-30, wherein the subject is a human.

32. A composition comprising at least five polynucleotides that selectively hybridize to each of at least five genes selected from the group consisting of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2.

33. The composition of item 32, wherein the at least five genes are selected from the group consisting of ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6.

34. The composition of item 32 or 33, wherein the at least five genes are selected from the group consisting of CLTB, COL4A3BP, CXorf52, FAM44A, MXRA7, PGK1, SFRS2, and YIPF6.

35. A composition comprising at least five polynucleotides that selectively hybridize to each of at least five SNP genotypes selected from the group consisting of rs11778767 (A/A), rs11780500 (A/A), rs11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), rs868856 (G/G), rs11778767 (A/G), rs11778767 (G/G), rs11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), rs868856 (A/A), rs983332 (A/C), rs928655 (A/G), rs 13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T); rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), and any of the SNP genotypes depicted in Table 13.

36. The composition of item 35, wherein the at least five polynucleotides selectively hybridize to each of at least five SNP genotypes selected from the group consisting of rs11778767 (A/A), rs11780500 (A/A), rs11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), rs868856 (G/G), rs11778767 (A/G), rs11778767 (G/G), rs 11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), and rs868856 (A/A).

37. The composition of item 35, wherein the at least five polynucleotides selectively hybridize to each of at least five SNP genotypes selected from the group consisting of any of rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), and rs6071980 (C/C).

38. The composition of any one of items 32-37, wherein the at least five polynucleotides are bound to a solid support.

39. A kit comprising:

an array comprising a plurality of polynucleotides bound to a solid support, wherein the plurality comprises at least five polynucleotides that selectively hybridize to each of at least five genes selected from the group consisting of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2; and

instructions for detecting the presence or amount of one of more of the polynucleotides in a sample.

40. A kit comprising:

an array comprising a plurality of polynucleotides bound to a solid support, wherein the plurality comprises at least five polynucleotides that selectively hybridize to each of at least five SNP genotypes selected from the group consisting of rs11778767 (A/A), rs11780500 (A/A), rs11780500 (G/G), rs1422422 (A/A), rs1441209 (A/A), rs1968201 (A/A), rs2028446 (A/A), rs2028446 (A/G), rs2028446 (G/G), rs2170331 (A/G), rs2170331 (G/G), rs2814707 (A/G), rs2814707 (G/G), rs3019293 (A/A), rs3087615 (A/C), rs3087615 (C/C), rs3849942 (A/G), rs3849942 (G/G), rs437943 (A/A), rs4562286 (A/A), rs4976592 (A/A), rs6531358 (A/A), rs6665006 (A/A), rs6665006 (A/G), rs7046653 (A/G), rs7046653 (G/G), rs774359 (A/A), rs868856 (A/G), rs868856 (G/G), rs 11778767 (A/G), rs 11778767 (G/G), rs 11780500 (A/G), rs1422422 (A/G), rs1422422 (G/G), rs1441209 (A/G), rs1441209 (G/G), rs1968201 (A/G), rs2170331 (A/A), rs2814707 (A/A), rs3019293 (A/G), rs3019293 (G/G), rs3849942 (A/A), rs437943 (A/G), rs437943 (G/G), rs4562286 (A/G), rs4562286 (G/G), rs4976592 (A/G), rs4976592 (G/G), rs6531358 (A/G), rs6531358 (G/G), rs6665006 (G/G), rs7046653 (A/A), rs774359 (A/G), rs774359 (G/G), rs868856 (A/A), rs983332 (A/C), rs928655 (A/G), rs13393173 (A/G), rs437943 (A/G), rs10945919 (A/G), rs854555 (A/C), rs854548 (A/G), rs854547 (A/G), rs7046653 (A/G), rs868856 (C/T), rs774359 (C/T), rs2814707 (A/G), rs3849942 (A/G), rs6028945 (G/T), rs6138150 (C/T), rs6071980 (C/T), rs1800896 (A/G), rs3024490 (A/C), rs231726 (A/G), rs3096851 (A/C), rs6708660 (A/G), rs2523619 (A/G), rs3915971 (A/G), rs9264869 (A/G), rs2239804 (A/G), rs2395175 (A/G), rs2395185 (A/C), rs2516049 (A/G), rs660895 (A/G), rs7026551 (A/C), rs4803455 (A/C), rs983332 (A/A), rs928655 (A/A), rs13393173 (A/A), rs437943 (G/G), rs10945919 (G/G), rs854555 (A/A), rs854548 (A/A), rs7046653 (A/A), rs868856 (T/T), rs774359 (C/C), rs2814707 (A/A), rs3849942 (A/A), rs6028945 (T/T), rs6138150 (T/T), rs6071980 (C/C), rs1800896 (A/A), rs3024490 (A/A), rs231726 (A/A), rs3096851 (C/C), rs6708660 (A/A), rs2523619 (G/G), rs3915971 (A/A), rs9264869 (A/A), rs2239804 (A/A), rs2395175 (G/G), rs2395185 (C/C), rs2516049 (A/A), rs660895 (A/A), rs7026551 (C/C), rs4803455 (C/C), and any of the SNP genotypes depicted in Table 13; and

instructions for detecting the presence or amount of one of more of the SNP genotypes in a sample.

41. The kit of item 39 or 40, further comprising one or more reagents for isolating nucleic acid from a sample.

42. The kit of any of items 39-41, further comprising a means for amplifying a nucleic acid.

## Claims

1. A method of predicting the response of a human subject to a therapy comprising an anti-TNF agent, the method comprising:

   providing a biological sample obtained from a human subject that has an immune disorder; and
   measuring the expression level of at least five genes selected from the group consisting of CLTB (SEQ ID NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEQ ID NO: 32) and YIPF6 (SEQ ID NO: 36) in the biological sample, wherein at least five of

   (i) an elevated expression level, as compared to a healthy individual, of CLTB (SEQ ID NO: 10), COL4A3BP (SEQ ID NO: 11), PGK1 (SEQ ID NO: 25), or YIPF6 (SEQ ID NO: 36), or (ii) a reduced expression level, as compared to a healthy individual, of CXorf52 (SEQ ID NO: 12), FAM44A (SEQ ID NO: 16), MXRA7 (SEQ ID NO: 22), or SFRS2 (SEQ ID NO: 32) predicts that the human subject will respond to a therapy comprising an anti-TNF agent.

2. A method of predicting the response of a human subject to a therapy comprising an anti-TNF agent, the method comprising:

   providing a biological sample obtained from a human subject that has an immune disorder; and
   measuring the expression level of at least five genes selected from the group consisting of CLTB (SEQ ID NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEQ ID NO: 32) and YIPF6 (SEQ ID NO: 36) in the biological sample, wherein at least five of (i) an elevated expression level, as compared to a healthy individual, of one or more of CXorf52 (SEQ ID NO: 12), FAM44A (SEQ ID NO: 16), MXRA7 (SEQ ID NO: 22), or SFRS2 (SEQ ID NO: 32), or (ii) a reduced expression level, as compared to a healthy individual, of one or more of CLTB (SEQ ID NO: 10), COL4A3BP (SEQ ID NO: 11), PGK1 (SEQ ID NO: 25), or YIPF6 (SEQ ID NO: 36) predicts that the human subject will not respond to a therapy comprising an anti-TNF agent.

3. The method of claim 1, comprising:

   determining that the expression level of at least five of (i) CLTB (SEQ ID NO: 10), COL4A3BP (SEQ ID NO: 11), PGK1 (SEQ ID NO: 25), or YIPF6 (SEQ ID NO: 36) are elevated, as compared to a healthy individual, or (ii) CXorf52 (SEQ ID NO: 12), FAM44A (SEQ ID NO: 16), MXRA7 (SEQ ID NO: 22), or SFRS2 (SEQ ID NO: 32) are reduced, as compared to a healthy individual; and
   selecting a therapy comprising an anti-TNF agent for the human subject.

4. The method of claim 2, comprising:

   determining that the expression level of at least five of (i) CXorf52 (SEQ ID NO: 12), FAM44A (SEQ ID NO: 16), MXRA7 (SEQ ID NO: 22), or SFRS2 (SEQ ID NO: 32) are elevated, as compared to a healthy individual, or (ii) CLTB (SEQ ID NO: 10), COL4A3BP (SEQ ID NO: 11), PGK1 (SEQ ID NO: 25), or YIPF6 (SEQ ID NO: 36) are reduced, as compared to a healthy individual; and
selecting a therapy comprising a non-anti-TNF agent for the human subject.

**5.** The method of claim 1, further comprising creating a record indicating that the human subject is likely to respond to a therapy comprising an anti-TNF agent, if the expression level of at least five of (i) CLTB (SEQ ID NO: 10), COL4A3BP (SEQ ID NO: 11), PGK1 (SEQ ID NO: 25), or YIPF6 (SEQ ID NO: 36) are elevated, as compared to a healthy individual, or (ii) CXorf52 (SEQ ID NO: 12), FAM44A (SEQ ID NO: 16), MXRA7 (SEQ ID NO: 22), or SFRS2 (SEQ ID NO: 32) are reduced, as compared to a healthy individual.

**6.** The method of claim 2, further comprising creating a record indicating that the human subject is not likely to respond to a therapy comprising an anti-TNF agent, if the expression level at least five of (i) CXorf52 (SEQ ID NO: 12), FAM44A (SEQ ID NO: 16), MXRA7 (SEQ ID NO: 22), or SFRS2 (SEQ ID NO: 32) are elevated, as compared to a healthy individual, or (ii) CLTB (SEQ ID NO: 10), COL4A3BP (SEQ ID NO: 11), PGK1 (SEQ ID NO: 25), or YIPF6 (SEQ ID NO: 36) are reduced, as compared to a healthy individual.

**7.** The method of any of the preceding claims, wherein the expression level of at least five genes selected from the group consisting of CLTB (SEQ ID NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEQ ID NO: 32) and YIPF6 (SEQ ID NO: 36) is elevated or reduced, as compared to a healthy individual, by at least about 1.5 fold.

**8.** The method of any of the preceding claims, wherein the method comprises measuring the expression level of at least eight genes selected from the group consisting of CLTB (SEQ ID NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEQ ID NO: 32), and YIPF6 (SEQ ID NO: 36).

**9.** An anti-TNF agent for use in treating an immune disorder in a human subject, wherein the human subject has been identified as having at least five of (i) an elevated expression level, as compared to a healthy individual, of CLTB (SEQ ID NO: 10), COL4A3BP (SEQ ID NO: 11), PGK1 (SEQ ID NO: 25), or YIPF6 (SEQ ID NO: 36), or (ii) a reduced expression level, as compared to a healthy individual, of CXorf52 (SEQ ID NO: 12), FAM44A (SEQ ID NO: 16), MXRA7 (SEQ ID NO: 22), or SFRS2 (SEQ ID NO: 32).

**10.** The anti-TNF agent for use as in claim 9, wherein the expression level of at least five genes selected from the group consisting of CLTB (SEQ ID NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEQ ID NO: 32) and YIPF6 (SEQ ID NO: 36) is elevated or reduced, as compared to a healthy individual, by at least about 1.5 fold.

**11.** The anti-TNF agent for use as in claim 9, wherein the human subject has been identified as having at least eight of (i) an elevated expression level, as compared to a healthy individual, of CLTB (SEQ ID NO: 10), COL4A3BP (SEQ ID NO: 11), PGK1 (SEQ ID NO: 25), or YIPF6 (SEQ ID NO: 36), or (ii) a reduced expression level, as compared to a healthy individual, of CXorf52 (SEQ ID NO: 12), FAM44A (SEQ ID NO: 16), MXRA7 (SEQ ID NO: 22), or SFRS2 (SEQ ID NO: 32).

**12.** The method of or the anti-TNF agent for the use as in any of the preceding claims, wherein the human subject has an inflammatory disorder.

**13.** The method of or the anti-TNF agent for the use as in any of the preceding claims, wherein the human subject has rheumatoid arthritis or Crohn's disease.

**14.** A composition comprising at least five polynucleotides that selectively hybridize to each of at least five genes selected from the group consisting of CLTB (SEQ ID NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEQ ID NO: 32) and YIPF6 (SEQ ID NO: 36).

**15.** A kit comprising:

an array comprising a plurality of polynucleotides bound to a solid support, wherein the plurality comprises at least five polynucleotides that selectively hybridize to each of at least five genes selected from the group consisting of CLTB (SEQ ID NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEQ ID NO: 32) and YIPF6 (SEQ ID NO: 36); and

instructions for detecting the presence or amount of one of more of the polynucleotides in a sample.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 94293707 P
- US 6270766 B
- WO 06122187 A
- WO 2004096273 A
- EP 1536012 A
- WO 2006119115 A
- US 5559012 A
- US 5888510 A
- US 20010001663 A
- WO 0674399 A
- US 5656272 A
- US 5919452 A
- US 2004086915 A
- EP 0543942 A
- US 7101663 B
- US 6812341 B
- US 5445934 A
- US 6027880 A
- US 6057100 A
- US 6156501 A
- US 6261776 A
- US 6576424 A
- US 20030013208 A
- US 2004171068 A
- US 4582789 A
- US 4563417 A
- EP 1234058 A
- US 20060008823 A
- US 20030059813 A
- US 6410231 B
- US 6193969 A
- US 6403087 B
- US 5925351 B
- US 6896885 B
- US 7060667 B
- US 5427779 A
- US 6197599 B
- US 5902723 B
- US 5871928 B
- US 5981180 A, Chandler
- US 5028545 A, Soini
- US 4499052 A, Fulwyler
- US 20040179267 A
- US 20040132205 A
- US 20040130786 A
- US 20040130761 A
- US 20040126875 A
- US 20040125424 A
- US 20040075907 A
- US 6916661 B

### Non-patent literature cited in the description


- **SANDBORN et al.** *Gut,* 2004, vol. 53 (10), 1485-1493
- **CHOY et al.** *Rheumatology,* 2002, vol. 41 (10), 1133-1137
- **KAUSHIK et al.** *Expert Opinion on Biological Therapy,* 2005, vol. 5 (4), 601-606
- **SAMBROOK et al.** Molecular Cloning: A Laboratory Manual. Cold Spring Harbor Laboratory Press, November 1989, vol. 1, 2, 3
- **GIBSON et al.** *Genome Res.,* 1999, vol. 6 (10), 995-1001
- **ZHANG et al.** *Environ. Sci. Technol.,* 2005, vol. 39 (8), 2777-2785
- Current Protocols in Immunology. Wiley, 1995
- **HAYMES et al.** Nucleic Acid Hybridization, A Practical Approach. IRL Press, 1985
- **AUSUBEL et al.** Current Protocols in Molecular Biology. John Wiley & Sons, 1989
- **OLIVER et al.** *J. Mol. Diagnostics,* 2000, vol. 2 (4), 202-208
- **TYAGI et al.** *Nat. Biotechnol.,* 1998, vol. 16, 49-53
- **ABRAVAYA et al.** *Clin. Chem. Lab. Med.,* 2003, vol. 41, 468-474
- **MULLAH et al.** *Nucleos Nucleot,* 1999, vol. 18, 1311-1312
- **LAMY et al.** *Nucleic Acids Research,* 2006, vol. 34 (14), e100
- **AUSUBEL et al.** Current Protocols in Molecular Biology. John Wiley & Sons, 1999, vol. 47
- **HARLOW ; LANE.** Antibodies: A Laboratory Manual. Cold Spring Harbor Laboratory Press, 1988
- **HARLOW ; LANE.** Using Antibodies: A Laboratory Manual. Cold Spring Harbor Press, 1999
- **TIETZ.** Clinical Chemistry. W.B. Saunders, 1999
- **BRAUN et al.** *Ann. Rheum. Dis.,* 2003, vol. 62, 1023-1024
- **FELSON et al.** *Arthritis and Rheumatism,* 1995, vol. 38 (6

- **PREVOO et al.** *Arthritis and rheumatism,* 1995, vol. 38, 44-48
- **VAN GESTEL et al.** *Arthritis and rheumatism,* 1998, vol. 41, 1845-1850
- **DUERR et al.** *Science,* 2006, vol. 314, 1461-1463
- **PE'ER et al.** *Nat Genet,* 2006, vol. 38, 663-667
- **DENG et al.** *Genetics,* 2001, vol. 159, 1319-1323
- **SELDIN et al.** *PLoS genetics,* 2008, vol. 4, e5
- **PRICE et al.** *Nat Genet,* 2006, vol. 38, 904-909
- **KENT et al.** *Genome Res,* 2002, vol. 12, 996-1006
- **FISHER.** *Journal of the Royal Statistical Society,* 1922, vol. 85, 87-94
- **PADYUKOV et al.** *Ann Rheum Dis,* 2003, vol. 62, 526-529
- **TURNER et al.** *Eur J Immunogenet,* 1997, vol. 24, 1-8
- **MAROTTE et al.** *Ann Rheum Dis,* 2006, vol. 65, 342-347
- **KANG et al.** *Rheumatology (Oxford,* 2005, vol. 44, 547-552
- **CRISWELL et al.** *Arthritis and rheumatism,* 2004, vol. 50, 2750-2756
- **KOSS et al.** *Genes Immun,* 2000, vol. 1, 185-190
- **LI et al.** *Genome Biol,* 2001, vol. 2
- **IRIZARRY et al.** *Nucleic Acids Res,* 2003, vol. 31, e15
- **WU et al.** *Journal of the American Statistical Association,* 2004, vol. 99, 909-917
- **GENETELMAN et al.** Bioinformatics and Computational Biology Solutions using R and Bioconductor. Springer, 2005
- **BREIMAN, L.** *Machine Learning,* 2001, vol. 45, 5-32
- **FRANSEN et al.** *Clin Exp Rheumatol,* 2005, vol. 23, 93-99
- **SMYTH, G. K.** *Stat Appl Genet Mol Biol,* 2004, vol. 3
- **BRIEMAN.** *Machine Learning,* 2001, vol. 45, 5-32
- **GENTLEMAN et al.** Bioinformatics and Computational Biology Solutions using R and Bioconductor. Springer

SEQUENCE LISTING

<110> BIOGEN IDEC MA INC.
THE FEINSTEIN INSTITUTE FOR MEDICAL RESEARCH

<120> BIOMARKERS FOR PREDICTING ANTI-TNF RESPONSIVENESS OR NON-RESPONSIVENESS

<130> BIO13505PCTEPD1

<150> PCT/US08/66180
<151> 2008-06-06

<150> 60/942,937
<151> 2007-06-08

<160> 82

<170> PatentIn version 3.5

<210> 1
<211> 6081
<212> DNA
<213> Homo sapiens

<400> 1
cgcagcggcc ggagagggat ggggggcgcc cacccagtct gagcctcgcc gcgggcgcct 60

tcggctcacg cagcgcttcc cgcgggccgc cagtgcgcac cctcggccac atcagcctcc 120

gcctggcggg tgcacccggg ccggcgagga aggggcaacc gtccgggtgg gtggggcggg 180

ctgggtacct gaggtcacca gctcggctgt agaggcaggg gcggcggagg cggaactgcg 240

gagttgctgg gtccaccgac ccttaccctc agcgagagaa gtaaccaatg tgaaagatgt 300

tgcagaagtg ttccagaagt ggctgaagat agaaggaaaa aagtgccact gcctatcaga 360

aaaaacaaaa caaaacatgg gaaatacaac caccaaattc cgtaaagcac tcatcaatgg 420

tgatgaaaac ctggcctgcc aaatatatga aaacaatcct cagctaaaag aatctcttga 480

tccaaataca tcttatgggg agccctacca gcacaatact ccattacatt atgctgctag 540

acatggaatg aataaaatat tagggacttt tcttggtaga gatggaaatc caaataaacg 600

gaatgtgcac aatgaaacat ctatgcattt gttgtgtatg ggacctcaaa ttatgatatc 660

tgaaggagcc cttcatcctc gcttggcacg ccccacagaa gatgatttca gaagagcaga 720

ttgtctgcag atgatcttaa aatggaaagg agcaaaactt gaccagggtg aatatgagag 780

agcagctatt gatgctgttg ataacaaaaa aaacacaccc ttgcactatg ctgctgcctc 840

agggatgaaa gcctgtgtag agcttttagt aaaacatgga ggagacttgt ttgctgagaa 900

tgaaaataaa gatactcctt gtgattgtgc tgaaaagcaa caccacaaag atttggccct 960

caatctggaa tctcaaatgg tattctcacg ggatcccgag gctgaagaaa tagaagctga 1020

atatgctgca ttagacaaac gagagccata tgaaggatta aggcctcagg atcttcgtag 1080

actaaaagat atgcttattg tggaaactgc agacatgctt caggctcctc tctttactgc 1140
tgaagcttta cttcgagctc atgactggga cagggagaaa ttacttgaag cttggatgtc 1200
caacccggag aactgctgcc aacgatcagg tgttcaaatg ccaactccac caccaagtgg 1260
gtataatgcc tgggacacgc tcccatctcc aagaactcca aggactacac gctcttctgt 1320
cacctcccca gatgaaatca gcttatctcc tggggattta gacaccagtt tgtgtgacat 1380
ttgtatgtgc agtatctctg tatttgaaga ccctgtggat atgccctgtg gacatgactt 1440
ttgtagagga tgttgggagt cgtttttgaa tctgaaaatt caagaaggtg aagctcacaa 1500
catttttttgc cctgcatatg attgcttcca acttgtacct gtggatatca tagaaagtgt 1560
agtttcaaag gagatggaca aacgatacct acagtttgat attaaggcct ttgttgaaaa 1620
taatcctgcc attaaatggt gtcctactcc aggctgtgac agagcagtaa gactaacgaa 1680
acaagggtca aatacatctg gatctgatac actcagcttc ccattgctga gagctcctgc 1740
tgttgattgt ggaaaaggac acctcttctg ctgggagtgc cttggtgaag cacatgagcc 1800
ttgtgactgc caaacatgga agaattggct gcaaaaaata accgaaatga aaccagaaga 1860
acttgtggga gttagtgaag cctacgagga tgccgccaat tgtctctggt tattaactaa 1920
ctccaagcct tgtgccaact gtaagtctcc aatacagaag aatgaaggct gcaatcacat 1980
gcagtgtgct aagtgcaagt atgacttttg ctggatttgc cttgaagagt ggaaaaaaca 2040
tagttcgtcc actggaggtt attacagatg tactcgctat gaagtcattc aacacgtgga 2100
ggagcaatcc aaggaaatga ctgtggaggc tgagaaaaaa cacaaacgat ttcaggaact 2160
tgacagattt atgcactatt atacaagatt taaaaaccat gagcatagtt atcagctaga 2220
acaacgcctt cttaaaacag ccaaagaaaa gatggagcaa ttgagcagag ctctcaaaga 2280
aactgaagga ggctgtccag ataccacttt cattgaagat gcagttcatg tgctcttaaa 2340
aactcggcgc attctcaagt gttcttatcc atatggattt ttcttggaac ctaaaagcac 2400
aaagaaagaa atttttgaac taatgcaaac agacctagaa atggtcactg aagaccttgc 2460
ccagaaagtc aataggcctt accttcgcac accccgccac aagatcatca aagcagcatg 2520
ccttgtacag cagaagaggc aagaattcct ggcatctgtg gctcggggag tagctcctgc 2580
agactcacca gaagctccaa ggcgcagctt tgctggtgga acatgggatt gggaatattt 2640
aggatttgca tcaccagagg aatatgctga atttcagtat cggaggaggc acagacaacg 2700
tcgtcgagga gatgttcaca gtctactcag taatcctcca gaccctgatg agccaagtga 2760
aagcacttta gatattccag aaggcggcag cagcagccgc aggcctggca catccgtggt 2820
aagttctgca tctatgagtg tgctgcacag ctcttccctg cgtgactaca cccctgccag 2880
tcgctctgaa aaccaggact ctcttcaggc tctgagttcc ttggatgaag acgatcccaa 2940
tatacttctt gcaatacagt tatcactgca agagtctggg ctggccctcg atgaagaaac 3000

tagagacttc ctcagtaatg aagcatcctt aggtgcgata ggcacttctt taccttccag 3060
gctggactct gtccccagaa atacagatag ccctcgggct gcattgagca gctctgagct 3120
tttggaactt ggtgacagcc tcatgagact aggagcagag aatgacccat tttcaactga 3180
caccctgagc tcacaccctc tcagtgaggc aagaagtgat ttctgtccct catctagtga 3240
tcctgactca gctggccagg accccaacat caatgacaat cttctcggca acatcatggc 3300
ttggtttcat gacatgaacc ctcagagtat tgccctgatt cctccagcaa ctacagaaat 3360
cagtgcagat tcccagctcc cctgtatcaa agatgggtca gaaggtgtga aggatgtgga 3420
actggtgctg ccagaagatt caatgtttga agatgccagt gtcagtgaag gtagaggaac 3480
ccagatagaa gaaaatcctt tggaagaaaa tattctggcg ggggaagcag catctcaagc 3540
tggtgacagt ggtaacgagg cagccaacag aggagatggt tcagatgttt caagtcaaac 3600
acctcaaacc tcaagtgact ggcttgaaca agtacattta gtgtgaactg cacacatctg 3660
ggctctaaat gaattacagg tacagatggt atgctaggtg gagtatgctt gatagagact 3720
ttgattcact taattccaac tcagtgataa accactgaca ttagggttga atacagagaa 3780
gttcccttga atggtagctt catttttat tttaacctta cagggaattt cctttgtact 3840
taattgaata gcttttcccc tttttgctga caaaaagaag agcaagagaa agagaaacaa 3900
aaatgaaata aataagttgt attccacact ctaagaaaat gcagtcctct atttagccta 3960
ggcttgacaa tacttaaatt gaacatttaa actaaaggct tactccctaa tctttgggtg 4020
gctttccttt aaaaaaaaaa aaaaagtttt cttcattcta gaaatttatt ttggataaat 4080
ccgataacat atatgtcctc aatctctttg tgctcttcca taacttactt ccttttttgtc 4140
tgagcaatgt gaattgaagt ctctttagta ccacatctac catagtgtaa ttagttttaa 4200
ttttcacatg aatcaaaggt ttcctttcat gtctatttac agtccaattg tgccaaactc 4260
ttacttgtgt gctgactaac aaggcattta ggtgtgcagc atcctagagt gctccagggc 4320
agtgtcagcg ttctcgggag taaaaggtgc cacttggtag caatgatatt ccagaattaa 4380
atgggttttt gttgccatgg agactgcatt tatataaatg tagcctgtag cttaagttaa 4440
ctaaacctaa tgctgctgtt aaaaacagtt tattttaata ttaaaataca gttgattagc 4500
aacagcggtg ctgtatttta agagacactt tattggaagt gcaatcatag ttatttgttt 4560
tcacaatttt acagtgcatt ctaattactg atgggtgcaa ttacttttaa tcgtgtttta 4620
taaaatagaa aaaaagtgga gttttcatga gttatagtaa atcccacgat tattaagaaa 4680
ttcagtaaaa catcctgcgc aacatgttac cgtgcctttg cctaacctaa atggatagtt 4740
gccagttaaa taagtgagta attcaaattt caatgtctct tctgaagtaa ctatgctatg 4800
aattgcaaag acctccataa aaccacccat ggccttgctt ttacactaac tataacaact 4860

aaatgttcaa tcagtttgtt tgcctaacta gcaaatgctg acatgtgttt gttctactgc 4920

gcaatactca tttgctgtgt gattactgtt tagtgttgaa aaaaatcaac ttcctagtta 4980

tcagtgtctt actgtgaaga aaatactggt cttagttgta attaggatac aatggtacag 5040

tgtgtaatta aaactagagt aaactgttgg aatggctgtt ttacttaaat attatcaaaa 5100

ctagcataac ataagcaaaa tagataagta caacactcca tttagtgttt tgccagattg 5160

ttaccagaag tctacagata ccaaactttc agttctgagt ttgtacaggc aagtcctggg 5220

ctgggtaaaa agttatatta atattgttat ccacaagaga tgtgattatg ggttttgatt 5280

actttttttt tttccaaacc ctgcttttga aatatccttg tacttaaaat tcatattgct 5340

aagacactgt attagaatat ttaatattcc ccagatcctc ttaggataaa ctgtgggaat 5400

cctcctatgc catggatatc aaaggtccac attagttttt atttctccag tgatcagaaa 5460

cattgatatc aatccctatt aaattagtgg ggggaatatt aactttatct acagtgtatt 5520

actgtatatt aaactgaaat agtccattaa aggatttttt tataaattta ttttggatta 5580

aaaatatcaa caccaataag tttttagacc aagttgtaat ttttccaata tagagtcttt 5640

gcatcacact gaggcatctt gcacagctgc agttaaggtg agaaagaatg ctctgtgtga 5700

agacagtgta cacaatgggt tccggtttcc ttgcaccttg tgcagtatcc tttatttctg 5760

tgctgttctc tcctgagcat gaaaaatgat cattatccaa tttgtatttc cttggtacat 5820

attttaaaaa caacacagtc attgacttta caattcagta atgaagtttg gcaaagccta 5880

ttttgtaaac aagttaattt tataatgtaa aaaaaaaaag ttaatctaac cttgacttgt 5940

tatttgcact ttcatagtct atacttgata cattcccact ttatatacag taggattcta 6000

caaacgtgta gatgtttggc caaatgaatg ctgttaataa tatgtaaaat tctttgatta 6060

aacatttatt acttaaacta c 6081

<210> 2
<211> 10459
<212> DNA
<213> Homo sapiens

<400> 2
gtgctcgcgt cgcagccaat cgcggggcga cgctgtcctg ggagcgagga ggctgtggtg 60

agagacggac cgagaccgga gatgttttca agcccggctc cggcggcttt acaggcggct 120

gcagcggcga cgaagacaac gacagcgacg gctacgccga agcactcgtt ccgggggtga 180

agcctcctgc gccggccttg cctcggatcc aggatgagaa gactgataaa agaagaagct 240

agctgaacag ctgtaaaatg cccaaatctg ggttcacaaa accaattcag agtgaaaatt 300

ctgacagtga cagcaatatg gtagagaaac catatggaag aaagagtaaa gacaagattg 360

catcctacag caaaactcca aaaattgaac gaagtgatgt gagcaaggag atgaaagaga 420

aatcatccat gaaacgtaaa cttcctttta ctattagccc atcaagaaat gaagaacgag 480
attcagacac agagaaagaa ggtccagaaa agaagaagac aaaaaaggaa gctggaaata 540
agaaatccac accagttagc attctttttg gttatccact ctctgagcga aaacagatgg 600
cacttcttat gcagatgaca gcaagagaca acagtccaga ttccacacca aatcatccat 660
cacaaacaac gcctgcccaa aagaaaactc ccagttcttc atctcgacag aaagataaag 720
ttaataaaag aaatgaacgt ggtgaaactc ctttacacat ggctgctatt cgaggagatg 780
tgaaacaagt taaagaatta ataagtttag gggcaaatgt gaatgtgaaa gattttgcag 840
gttggacacc actgcatgaa gcttgcaatg ttggatatta cgatgttgct aagatactta 900
tagcagctgg agcagatgtt aacacacaag gattagatga tgacactcca ctccatgatt 960
ctgctagtag tgggcacaga gatatagtaa agctgttact tcgtcacggt ggaaatccat 1020
ttcaagctaa taaacatggg gagcgtccag tggatgtagc agaaacagag gagttggagt 1080
tgctactaaa aagagaggtg cctttatctg atgatgatga aagttacaca gattccgaag 1140
aggctcaatc tgtaaatcct tctagtgttg atgaaaatat tgactctgaa acagagaaag 1200
actctctcat ctgtgaaagt aaacagatac ttcccagtaa aacacctctt ccatctgccc 1260
ttgatgagta tgagttcaaa gatgatgatg atgaagaaat taataagatg attgatgata 1320
ggcatattct taggaaagaa caacgaaaag aaaatgaacc tgaagcagaa aaaactcatt 1380
tatttgcaaa acaggagaaa gccttctatc ctaaatcatt taaaagtaaa aaacaaaagc 1440
catctagggt cttatattca agtactgaaa gttctgatga agaagctctt cagaataaaa 1500
agatttctac ttcatgttcc gtcatccctg aaacatcaaa ttctgatatg caaaccaaaa 1560
aggaatatgt agtttcaggt gaacacaaac agaaaggcaa agttaaaaga aaattgaaaa 1620
atcagaataa aaataaagag aaccaagagc taaagcaaga aaaggaagga aaagaaaata 1680
caagaataac aaacttgaca gtaaatactg gactagattg ttcagaaaag accagagagg 1740
aggggaactt taggaaatct tttagcccaa aagatgatac ttcattacat ttatttcata 1800
tttccactgg taaatctccc aaacattctt gtggattaag tgaaaaacag tcaacaccac 1860
taaaacaaga acatactaaa acatgtttat caccaggaag ttctgaaatg tcattacagc 1920
ctgatcttgt tcggtatgat aatacagaat ctgaattctt gccagaaagt tcaagtgtaa 1980
aatcttgtaa gcataaggaa aaaagcaaac atcagaaaga tttccactta gaatttggtg 2040
aaaaatcaaa tgccaaaata aaggatgaag atcatagtcc aacatttgaa aattcagatt 2100
gcacactgaa aaaaatggat aaagaaggta aaacattaaa aaaacataaa ttgaagcata 2160
aagagaggga aaaagaaaag cataaaaaag aaattgaagg tgaaaaggaa aaatacaaaa 2220
ctaaggatag tgccaaagaa ctgcagagga gtgtggaatt tgatagagaa ttttggaaag 2280
agaatttttt taaaagtgat gaaactgaag atctcttttt aaatatggaa catgaatcct 2340

taacattaga aaaaaaatca aaattggaaa aaaacatcaa agatgataaa tcaaccaagg 2400
aaaagcatgt gtcaaaagag aggaacttta aagaggaacg agacaagatt aaaaaggaaa 2460
gcgagaaatc ttttagggag gaaaaaataa aagatctaaa agaagagaga gaaaacatac 2520
ccacagataa agactcagaa tttacttctt tgggtatgag tgccattgag gaatctatag 2580
ggcttcattt agtggaaaag gaaatagaca ttgaaaaaca agaaaaagcat ataaaggaaa 2640
gtaaagaaaa acctgagaag cgatctcaaa ttaaagaaaa ggacattgag aagatggaaa 2700
gaaaaacctt tgaaaaagaa aagaagataa aacatgagca taagtcagaa aaagacaaat 2760
tagatcttag tgaatgtgtt gataaaataa aagaaaagga caagctatat tcgcatcaca 2820
cagaaaaatg ccataaagaa ggtgagaaga gcaaaaatac tgctgctatt aaaaaaactg 2880
acgacagaga gaaaagtaga gaaaagatgg ataggaaaca tgacaaagaa aagcctgaaa 2940
aagagaggca tctagcagaa agcaaagaaa agcacttgat ggagaaaaaa aataaacaat 3000
cagataatag tgaatacagt aaatcagaaa aaggcaaaaa taaagaaaaa gacagggagc 3060
tagataaaaa ggaaaaatct agagataaag aaagtataaa tataactaac tccaaacaca 3120
tacaggaaga aaaaaaatca agtatagtag acggtaataa agcacaacat gaaaaaccct 3180
tatcccttaa agaaaaaaca aaagatgaac ctttgaaaac tccagatgga aaagaaaaag 3240
ataaaaaaga taaagatata gatagataca aagaacgaga caaacataaa gataaaattc 3300
aaataaatag cttactcaaa ctaaaatctg aagcagataa gcctaaacct aagtcatcac 3360
cagcatcaaa agatacccga cctaaagaaa agaggttagt gaatgatgat ttaatgcaga 3420
caagttttga acgaatgcta agccttaaag acctagaaat agaacagtgg cacaaaaaac 3480
ataaggaaaa aattaagcaa aaagaaaagg aacggttgag aaaccgaaac tgtttagaac 3540
ttaaaataaa agataaagaa aaaacaaagc atacaccaac tgaatccaaa aataaagaac 3600
ttactaggtc aaagagttca gaagtgactg atgcatatac caaggagaaa caacctaaag 3660
atgctgtaag taacagatca caatctgttg acaccaaaaa tgtaatgact ttagggaagt 3720
catcttttgt ttcagataat agcttaaaca ggtctcctag atcagaaaat gaaaagccgg 3780
gtctcagctc cagatctgta tccatgattt ctgttgctag ttcagaagat tcctgccata 3840
ctacagtgac aaccccaagg cctccagttg agtatgactc tgactttatg ttagagagtt 3900
cagaatccca aatgtccttt tcccagtcac ctttttttgtc aattgccaaa tctcctgctc 3960
ttcatgaaag ggaattggac agcctggctg acttgccgga gcggattaaa ccaccatatg 4020
caaacagact ttcaacatcc catcttaggt catcttctgt agaagatgtt aaactaatta 4080
taagcgaggg gagacctacc atagaagttc gaagatgtag catgccttct gtcatttgtg 4140
aacataccaa acaattccaa acaatatcag aagagagcaa tcaaggtagc ttattaactg 4200

tgccaggaga tactagtcct tctcccaaac ctgaggtatt ctcaaatgtg cctgaaagag 4260
acctttcaaa tgtatctaac atacattcca gttttgcaac ttctccaact ggagcttcaa 4320
acagcaagta tgtttcagct gatagaaatc tcatcaagaa tactgcccca gtgaacactg 4380
taatggacag tccagtgcat ttagagccat ctagtcaggt tggtgtgatc cagaataaat 4440
catgggagat gcctgttgat agactagaga cattaagcac cagagacttt atctgcccaa 4500
attctaacat acctgatcaa gaatcctctc ttcagagttt ttgtaattct gaaaataagg 4560
tattgaaaga aaatgctgat tttttatccc tgcgccagac tgaactgcca ggaaactctt 4620
gtgctcagga tccggcatcc tttatgcctc cacagcagcc ttgctctttc cccagccaat 4680
cactttcaga tgctgaatcg atttctaaac atatgtcttt gtcatatgtt gctaatcaag 4740
agccaggtat tttacaacaa aaaaatgcag ttcagattat tagttctgct ttagatactg 4800
ataatgaatc tacaaaagat acagaaaata cttttgtcct aggagatgtt caaaaaacag 4860
atgcctttgt cccagtgtac tctgacagca ctattcaaga agcatcacca aactttgaga 4920
aagcttatac tttacctgtg ttaccatcag aaaaggactt taatggaagt gatgcctcta 4980
cccagctaaa tacacattat gcatttagca aactaactta caagtcttcc agtggccatg 5040
aagttgagaa tagcacaact gatactcagg tcatttcaca tgaaaaagaa aacaaactgg 5100
agagtttggt tttaactcat ttgagtaggt gtgattctga tttatgtgaa atgaatgcag 5160
ggatgccaaa aggaaaccta aatgaacaag atccaaaaca ttgtcctgaa agtgaaaagt 5220
gtttgctttc catagaagat gaggaatctc aacaaagcat tttatcaagt ctggaaaacc 5280
attcacagca gtcaactcaa ccagaaatgc ataaatatgg tcagttagtt aaagtagaat 5340
tagaagaaaa tgccgaagat gataaaactg aaaaccaaat ccctcaaaga atgactagaa 5400
acaaagcaaa tacaatggca aatcaaagca aacagattct tgctagctgt acactattat 5460
cagaaaaaga cagtgaatcc tcatctccta gaggaagaat aagattaact gaagatgacg 5520
atcctcaaat tcaccatcca cggaaaagga aagtgtcacg tgtacctcag cctgtgcaag 5580
tgagtccctc tttactacaa gcaaaagaga aaactcagca atctctggca gccattgtag 5640
attctctaaa actagatgag attcagccat acagttcaga gagagcaaat ccatattttg 5700
aatacttgca cataaggaaa aaaatagaag aaaaacgcaa attactgtgt agtgtgattc 5760
ctcaagcacc tcagtactat gacgaatatg taacatttaa cggatcatat ctcctggatg 5820
gaaacccctt aagcaagatt tgtattccca caattacacc accaccttca ctgtcagatc 5880
cacttaaaga gctttttcga caacaggaag ttgtaaggat gaaactacgt ttgcaacaca 5940
gtattgaaag ggaaaaactc attgtatcca acgaacaaga agttttgcga gttcattaca 6000
gagctgcaag aacattggca aatcaaacac tgccatttag tgcttgtact gttttgctgg 6060
atgccgaagt atacaatgta ccattggact ctcagtctga tgacagtaaa acttctgtga 6120

gggatcgctt taatgcaaga caattcatgt cttggttaca agatgtggat gataaatttg 6180

acaaattaaa gacctgtctt ttaatgaggc aacaacatga agctgcggct ttaaatgctg 6240

tccagaggtt agaatggcag ctcaaactcc aggaacttga tcctgccacc tataaatcta 6300

tcagcattta cgaaatccag gagttttatg ttccccttgt tgatgttaac gacgactttg 6360

aattgactcc tatatagcag tcagtacttc ctgatggtat tgtcctaaac tggtgatgct 6420

caagcattat actgtggaat actgcctttt gacaaaaata ctcatgcctt tacaattgtt 6480

agtaaagttc gattatagtt ggttatgtag taaacactgt cattttataa aaaatgagaa 6540

ttattttgga tcttagatcc aaacacagtt tctaatagaa aactattatt tatattggga 6600

aaggtaacta ttgcattaga gcatgttggc agactggtag gtatttaaaa agttgagaat 6660

ctgctaacag cgctggaagt tgttagcgct ctaagtaata agataaccac tagtattcaa 6720

atctctttca ggttttatta aaaaatatat atcaataaac taaaaggttc aattcctacc 6780

aaatagtttc taatgtggga gaaaaacttg gcacaaaatt tcttcagttt attatctgta 6840

aattgtacag ttttcttttt gaaagtttta atattgtctt ccttttttaat aacttatttt 6900

atacatattg tgcagatgta aatcttgtaa ttaatggtca aactgtataa agggattggt 6960

agtcaaaaca tgtacaaaga aatacctgta aaactgtttt gtctcatgtt ttattggacc 7020

aaagttgtgg tttgtatgga gtgtagtagt agtgtgtaca ggtagaaaac ttttaaatac 7080

agcatgcagg tgtttcagtt agcttgtttt catcaccata actgcaaaga tgtggcttag 7140

ttgtattgca tgcttcctat aatttaactc tccataattg atgcctgcag tagtgtaagg 7200

catttcatac tagtctcctc tagtagacct gtgacttact gtgttggaca tattatttag 7260

acttagtcat acaaagaaac ttagctcttt tttcatctca cagtaaagcc tatttcccca 7320

ggaaaaaaat aaatgccttt gaatgaaaat tctgaaattg taaatgtcta ttttaatatt 7380

cacctatgaa agaatctgtg aatatatgta aatacgttta ataaatttta ttggtcatgt 7440

taaatcattg taaaactttt ttacattgct taatgtttta agcttaatag cctttgcact 7500

tttaaaataa aaaccaagta tgcaaatcaa agatatttgg tagtcaaaat aagtaaaaga 7560

aatataggaa tattccagtc aatctctgaa atgtttatga aaaacaggtt aatatgttgt 7620

attttttttcc ttgtatcaag atgcaaaaca taatttgcaa aattttataa ttgaaataaa 7680

acttggtatg ctgttttata ataaattagc aatatacttt aaaaaaaatc cagtttctcc 7740

tataacatga tgtaaattaa atattcagga atatttcagg tctgaaatct gctgctgaac 7800

ttgttagaca tttttgaaag gaaaattagg ttagcgtaca atttatcata aatatatgag 7860

gtaaaatttg caaaactttc cacagtactt tcttgaatta taatactgaa tgttttctgt 7920

tccgatagag aaaagtgaag caaatatgtg aggaatgaag tgatctagtg caagttggta 7980

attccagcta atgggagaga atgtaaatgt gcaaaggtac atattaagct tagggatttt 8040
tgaattttta cgtcagttta tattttaatt cttactgtac ttggcatgcg caataaagca 8100
gcacatgtaa aaaaaaaata cacagtgcaa ggactttatt ataaaggttg gatgtagttt 8160
tccttagaaa tttaggtgag agattttggc ctttttttatt ggataaattg ggccagaaag 8220
gcagggtaga tttcgaagca ctggttctgt tgaagttaag tttattaagc ctgggaacat 8280
taaaagctaa tttataaaag caatactttt taatatgaaa acttactgca aagtttgttt 8340
atacttttgc ctaaaaagga aattggatgg gatactgtgg caaatcataa aaaaccagat 8400
aattgaactt tgaagttata gaaaatcaga gaggggtaag tttatagggc attttgttct 8460
gatggttcaa ccagaggtct gggaaatagc actgttggcc caaacagaac aggcttttag 8520
aagataaaag cgacaagaag gaatctggtg aattttagtc atcccagctt tttagtctta 8580
accacagttc tcactctctt aaatggtacc tcaaaaagct ggagcctctc tgccatgatt 8640
atgcttctac aaatttcttt tataaagaga ctcaaagcta atgatagctt aaaagaaaag 8700
ttaatgcctt ctcattggaa atgtataatc aaataagtag ttaagggctt ttggtattaa 8760
agatattctg aagctctgaa atgctagaaa aaaatttgga atggagtata tgcctgaaaa 8820
ggttttggat tcagaaagaa aaaggatggt tagtttaatc agtgattctt tttaaactct 8880
tcaaatatca tgaacaagat actaaattgt acctaaggat ttgtatttct ttacaatttg 8940
ttctaaatat ctgtttaatg actagttgat atttgtgcat gttatttaat aaagagttat 9000
atttttatag aaaaaaagag tgaaatgtgt gctaactgtt tttttactta attttacttg 9060
ggcagctagc aaaattgcag aaatatgcat cctgggaaaa gaaacagcct ttgaagaatt 9120
agcctttcaa gttcaaatct atttaataat gagaagtctc acaagtgaat ttttaagtac 9180
aggcatacct cagacgtact ttaggttcca gaccatctca gtaaagcaaa taccacaaca 9240
aagcgagtca ggaggaattt tttggtttcc cagtgcatat aaaagttttg tttatactat 9300
attaagtgtg caatagcatt atgtctaaaa atatgtacat aagtttaaaa atattttatt 9360
gctaaaaatg gtaacaaagt gagcacatgc tgttggaaaa agagcaccaa tagacttgct 9420
tgaagcaggg ttgccacaaa ccttcaattt gtaaaaaacg ccaatatgta caaagcacaa 9480
taaagcaaag cacaatagaa caggattgcc tgtattagac atgctacaaa cttcataact 9540
ggaaacatct caaagacccc atgaagctca tttgaatggg acttaacaat tagacagtta 9600
ttttagaaat tgagtgcaga cctaaataca tagttttcca aaaagaaaat tattgtctct 9660
gatatcttaa aacataaaaa cccaaaattt tatatagaag aaattgactc tgtaaaacgc 9720
aatgaaatag tcctcttttt aaacagttta aaggaagcat tttcaccgtt tgtaaaaatt 9780
atttttaaat atttagggca aaatttttgt tagataataa tggaaaagct tgtgtgagtt 9840
tagtggttaa aatatcttgt aattcatcat tatttaagtg acttcttggg agccgtcttt 9900

gtacctaaaa tggagttttt ttttaagcct ccacagagat agtcacccaa agtatttcca 9960

gtcagtaaaa gtagaattca tagaaaaaac tgaggcaaat taaaacaatt ccattaatca 10020

aaatggcttt aaacaaatta agtattagca taaaaatagc aaaaagtaca actaaaaaaa 10080

tggttgggtt ttcccagtgg ttaaatgcta tataataact gcaaataaaa gtttttttgt 10140

acatggacag cgtcctcata aaagaaaata ggccaggcca ggcgcagtgg ctcgcgcctg 10200

taatcccagc actttgggag gccaaggcgg gcggatcacg aggtcaggag atcgagacca 10260

tcctggctaa cacggtgaaa ccccgtctct actaaacaaa atgcaaaaaa tcagccgggt 10320

gtggccgcgg gcgcctgtag ttccagctac tcgggaggct gaggcaggag aatggcgtga 10380

gcctgggagg cggagcttgc agtgagccga gatcgtgcca ctgcactcca gcctgggcga 10440

cagagcgaga ctccgtctc 10459

<210> 3
<211> 1986
<212> DNA
<213> Homo sapiens

<400> 3
ccttacccgg cgtgccccgc gcccggaggc gctgacgtgg ccgccgtcag agccgccatc 60

ttgtgggagc aaaaccaacg cctggctcgg agcagcagcc tctgaggtga gggcgagggg 120

cgcgggccgg tgtgggccgc agagacgttg gagccggcgg gggctgggga ctggcctcgg 180

ggcgacttga ggtgtccctg gccagtgtcc ttccacctgt ccacaagcat ggggaacatc 240

ttcgccaacc tcttcaaggg cctttttggc aaaaaagaaa tgcgcatcct catggtgggc 300

ctggatgctg cagggaagac cacgatcctc tacaagctta agctgggtga gatcgtgacc 360

accattccca ccataggctt caacgtggaa accgtggagt acaagaacat cagcttcact 420

gtgtgggacg tgggtggcca ggacaagatc cggcccctgt ggcgccacta cttccagaac 480

acacaaggcc tgatcttcgt ggtggacagc aatgacagag agcgtgtgaa cgaggcccgt 540

gaggagctca tgaggatgct ggccgaggac gagctccggg atgctgtcct cctggtgttc 600

gccaacaagc aggacctccc caacgccatg aatgcggccg agatcacaga caagctgggg 660

ctgcactcac tacgccacag gaactggtac attcaggcca cctgcgccac cagcggcgac 720

gggctctatg aaggactgga ctggctgtcc aatcagctcc ggaaccagaa gtgaacgcga 780

ccccccctccc tctcactcct cttgccctct gctttactct catgtggcaa acgtgcggct 840

cgtggtgtga gtgccagaag ctgcctccgt ggtttggtca ccgtgtgcat cgcaccgtgc 900

tgtaaatgtg gcagacgcag cctgcggcca ggctttttat ttaatgtaaa tagtttttgt 960

ttccaatgag gcagtttctg gtactcctat gcaatattac tcagcttttt ttattgtaaa 1020

aagaaaaatc aactcactgt tcagtgctga gaggggatgt aggcccatgg gcacctggcc 1080

tccaggagtc gctgtgttgg gagagccggc cacgcccttg gctttagagc tgtgttgaaa 1140

tccattttgg tggttggttt ttaacccaaa ctcagtgcat tttttaaaat agttaagaat 1200

ccaagtcgag aacacttgaa cacacagaag ggagaccccg cctagcatag atttgcagtt 1260

acggcctgga tgccagtcgc cagcccagct gttcccctcg ggaacatgag gtggtggtgg 1320

cgcagcagac tgcgatcaat tctgcatggt cacagtagag atccccgcaa ctcgcttgtc 1380

cttgggtcac cctgcattcc atagccatgt gcttgtccct gtgctcccac ggttcccagg 1440

ggccaggctg ggagcccaca gccaccccac tatgccgcag gccgccctac ccaccttcag 1500

gcagcctatg ggacgcaggg ccccatctgt ccctcggtcg ccgtgtggcc agagtgggtc 1560

cgtcgtcccc aacactcgtg ctcgctcaga cactttggca ggatgtctgg ggcctcacca 1620

gcaggagcgc gtgcaagccg ggcaggcggt ccacctagac ccacagcccc tcgggagcac 1680

cccacctctg tgtgtgatgt agctttctct ccctcagcct gcaagggtcc gatttgccat 1740

cgaaaaagac aacctctact tttttctttt gtattttgat aaacactgaa gctggagctg 1800

ttaaatttat cttggggaaa cctcagaact ggtctatttg gtgtcgtgga acctcttact 1860

gctttcaata cacgattagt aatcaactgt tttgtatact tgttttcagt tttcatttcg 1920

acaaacaagc actgtaatta tagctattag aataaaatct cttaactatt tcaaaaaaaa 1980

aaaaaa 1986

<210> 4
<211> 978
<212> DNA
<213> Homo sapiens

<400> 4
ccgcgtcggt gcccgcgccc ctccccgggc cccgccatgg gcctcaccgt gtccgcgctc 60

ttttcgcgga tcttcgggaa gaagcagatg cggattctca tggttggctt ggatgcggct 120

ggcaagacca caatcctgta caaactgaag ttgggggaga ttgtcaccac catcccaacc 180

ataggcttca atgtagaaac agtggaatat aagaacatct gtttcacagt ctgggacgtg 240

ggaggccagg acaagattcg gcctctgtgg cggcactact tccagaacac tcagggcctc 300

atctttgtgg tggacagtaa tgaccgggag cgggtccaag aatctgctga tgaactccag 360

aagatgctgc aggaggacga gctgcgggat gcagtgctgc tggtatttgc caacaagcag 420

gacatgccca acgccatgcc cgtgagcgag ctgactgaca agctggggct acagcactta 480

cgcagccgca cgtggtatgt ccaggccacc tgtgccaccc aaggcacagg tctgtacgat 540

ggtctggact ggctgtccca cgagctgtca aagcgctaac cagccagggg caggcccctg 600

atgcccggaa gctcctgcgt gcatccccgg gatgaccaga ctcccggact cctcaggcag 660

tgccctttcc tcccactttt cctcccccat agccacaggc ctctgctcct gctcctgcct 720

gcatgttctc tctgttgttg gagcctggag ccttgctctc tgggcacaga ggggtccact 780

ctcctgcctg ctgggaccta tggaaggggc ttcctggcca aggccccctc ttccagagga 840

ggagcaggga tctgggtttc cttttttttt tctgttttgg gtgtactcta ggggccaggt 900

tgggaggggg aaggtgaggg cttcgggtgg tgctataatg tggcactgga tcttgagtaa 960

taaatttgct gtggtttg 978

<210> 5
<211> 4746
<212> DNA
<213> Homo sapiens

<400> 5
agcacaaccg cctaagcggc ggaactctgg agagtgcgga acctaggatc ctaggaaact 60

gaaggcaacg gaacctgggg gcgatggaat ccgggagcgg tggaacccgg agcctgtaga 120

acttgaatct agaggaacct gacagcggcg ccgagggggc cacccggact ctgtttggaa 180

cggaagcaca gtgtccgccg cttcctggtt gcgggtcagc gcccaggtcc tgggctggcc 240

gccgggatgt tgccctacac agtgaacttc aaggtgtcgg cgcgcaccct cacgggggcc 300

ctcaacgccc acaacaaggc ggcggtggac tggggctggc aaggtttaat tgcttatgga 360

tgtcattcac ttgtggtagt gattgattcc attactgccc aaactcttca agttttagaa 420

aagcataaag ctgatgttgt aaaggttaaa tgggccaggg aaaactatca ccataacatt 480

ggctcaccat attgcttacg gttagcttct gctgatgtca atgggaagat catcgtctgg 540

gatgtagcag caggagtagc tcagtgtgag atccaagagc atgccaagcc tatccaggat 600

gttcagtggt tgtggaatca agatgcttcc cgcgatttac tgcttgctat ccacccgcca 660

aattacattg tgctctggaa tgccgacact ggcaccaaac tatggaagaa gagctatgca 720

gataacattc tttctttttc ttttgaccct tttgatccct cacatttaac tttgcttacc 780

agcgagggta ttgttttcat ctcagacttc tccccatcca agcctccctc aggccctggg 840

aaaaaagttt acatatccag cccacactct agcccagctc ataacaagct ggccacagcc 900

acaggtgcca agaaagctct aaataaagta aaaattttaa tcactcaaga gaaacctagt 960

gctgaattca taactctcaa tgattgcctt cagttggcat acctgccttc aaaaaggaat 1020

cacatgttgt tgctctatcc tcgagagatt ttaatccttg accttgaggt gaatcagacg 1080

gtgggtgtga ttgcaataga acgcacagga gttccatttt tacaggtaat accctgcttt 1140

cagcgtgatg gtttattttg tctacatgaa aatggttgta taactttacg tgttcgaaga 1200

tcttataata acatttttac cacttcaaat gaggaaccag atccagatcc agttcaggag 1260

cttacctatg atttacgaag ccagtgtgat gcaatcaggg tgacaaaaac cgtccgtccc 1320

ttcagtatgg tgtgctgtcc tgtcaatgag aatgcagccg ccctcgtagt gagtgatggc 1380

agggtcatga tatgggaact caagtctgca gtttgtaatc gaaattcacg gaacagtagt 1440

tctggtgtgt cacctttata ttcaccagtg tctttctgtg gaattcctgt aggagtgcta 1500

cagaataaac tcccagacct ttccttagat aacatgattg ggcaaagtgc aattgctggg 1560

gaagaacatc ccagaggttc aattctgcgg gaagtgcacc tcaagttcct gctgacggga 1620

ctgctttcag gactgcccgc accacagttt gctattcgta tgtgtccacc gttgaccaca 1680

aaaaacatca agatgtatca gccactgctg gctgttggta caagtaatgg ttctgtcctg 1740

gtgtaccatc tcaccagtgg tctgctacac aaagagttaa gcatccactc atgtgaagtc 1800

aagggtattg aatggacaag tttgactagt tttctttctt ttgctacctc aacaccaaac 1860

aatatgggat tagtgagaaa tgaacttcaa ctggttgatc ttccaacagg taggagcatt 1920

gcttttcgtg gtgaaagagg caatgatgaa tctgccatcg aaatgattaa agtatctcat 1980

ttgaagcagt atttggcagt cgtattcaga gataaacccc tggagctatg ggatgttagg 2040

acttgtaccc ttcttagaga gatgtccaaa aacttcccta caataactgc tttggagtgg 2100

tcaccatctc acaacttgaa gagcctgaga aagaagcaac ttgcaactcg agaggccatg 2160

gcccgccaga ccgtagtctc agacacagag ctgagtattg ttgaatcatc tgtgatcagc 2220

ttgctgcagg aggcagaaag taaatctgaa cttagtcaga acatctctgc ccgggaacat 2280

tttgtattta ccgatattga tggccaagtg tatcatctca ctgttgaagg aaactcagta 2340

aaagacagtg ctcggattcc accagatgga agtatgggta gtattacctg catcgcttgg 2400

aaaggtgata cattagtgct tggagatatg gatggaaatt taaatttctg ggacttgaaa 2460

ggcagagtat ccagaggaat acccacacac cgaagttggg tgaggaagat tcgttttgct 2520

cctggtaaag gaaatcaaaa attaatagca atgtacaatg atggagctga agtgtgggat 2580

actaaagagg ttcagatggt gagcagttta agaagtggca gaaatgtgac ctttcgtata 2640

ttggatgtgg actggtgtac gtcagataaa gtgatcttgg cctcagatga tgggtgcatc 2700

agagtcctag agatgtctat gaagtctgcg tgctttagaa tggatgaaca agagttaacc 2760

gagcctgtgt ggtgccccta tctccttgtt ccaagggcct ctcttgcctt gaaagccttc 2820

ttattacacc agccttggaa tggacagtat tctttggaca tttctcatgt tgactatcca 2880

gaaaatgaag aaataaagaa tctcctccaa gaacagttga attcattgtc taatgacata 2940

aagaaactgt tgcttgatcc agaattcact ctcttgcaga ggtgcctgct tgtttcaagg 3000

ctctatggtg atgaatcgga gctgcacttc tggactgtcg ctgcccacta cctgcacagc 3060

ttatcccagg aaaagtcagc cagcacaaca gctcctaaag aagctgctcc tcgagacaaa 3120

ctgagcaacc cactggatat atgctatgac gtgctctgtg aaaatgccta ctttcagaaa 3180

tttcagctag aaagggttaa tctgcaggaa gtgaaacggt caacttatga tcatacaagg 3240

aaatgtacag accagctact gctcttgggt caaacagaca gagctgtgca gttgctgttg 3300

gaaacaagtg cagataacca gcattattac tgtgattcac tgaaagcctg tttagtcact 3360

actgtcacct cgtcaggccc ctctcagagc accattaagt tggtggcaac gaatatgatt 3420

gccaatggca aattggcaga gggcgttcag ttgctctgcc tgatagataa ggctgcagac 3480

gcctgccgct acctgcagac atacggcgag tggaatcggg ctgcatggct ggcaaaagtc 3540

cgtttgaatc ctgaggagtg tgccgatgtt ttaaggcggt gggttgacca cctttgttct 3600

ccacaagtca atcagaaatc aaaggctctc ctggttctcc tctctctggg ctgctttttt 3660

agcgtggcag agacgcttca cagcatgaga tactttgata gagcagcctt atttgtggaa 3720

gcttgcctca agtatggagc atttgaagtc actgaggaca cagagaaact catcactgct 3780

atatatgcag attatgcccg gagtttgaag aacctcggtt ttaagcaggg agcagttctc 3840

tttgcttcaa aagccggagc agctggcaaa gacttattga atgagcttga gtcccccaag 3900

gaagaaccca ttgaagagtg acagcttaat aaatgccagg gaatctgacc tggaaggcag 3960

atgggagggg gctggtctgg ctgtggccac cgtcacagtc caggatgaag aggagtacag 4020

ggtcctgtga gctgtttgac cactgttcta agactatgtg tgcccaaaag cacataagca 4080

tctatgttga gagtaagttt gtatcctgcg ttggtctcag aaagaacgtg aatgcttaag 4140

attttgaaag tacataatat tttatacttt gggagagagc tttaagagtc cctggaaata 4200

ctttttaatt tttttaactt aaaattcaag agactgaatc acttttctca ttgattaaat 4260

gtaaagatta ttgagaaacc tatagtaaat gaaatttgtg agatgttttc tcaaatatat 4320

gctgtgcctg tacttatata cagtctttca agagagatac aaacaaggca gaaacattta 4380

aactagtatt aaaggtagtt taccaaagca tttttgttt tcttaccttg aaaacacaga 4440

accgttaatt ccttggttta agcagttgct aagttttgta attttaggct cagaggacca 4500

taggaggttt taagatttat gtttagtccg ataggtgagg tctttgatat tttgaatttt 4560

aactcctttt atgatacatc acagtaacct catttttgaa gtctttcttt gtactttaat 4620

gttctctctg ttctaatagt tgaagtatga gatgtaacta ttataaactg ttgctgaaaa 4680

cataaatgtc tgtaacttac aaacatgata aataaattaa aaattccatc caggaaaaaa 4740

aaaaaa 4746

<210> 6
<211> 1532
<212> DNA
<213> Homo sapiens

<400> 6
agcgcagcgg gggcgggaag gttgtagtgc cgcgagttga gctcctcttg cctaagtggt 60

cgcgcccccct ttaagagcag cgattgtaag gagaggcggt cccggtgtcc tcgggtccca 120

ggtgattgtg aagtgctgac caattgccac tggacatact tgaaacaaaa taggaaaatg 180

gcagcaaact cttcaggaca aggttttcaa aacaaaaata gagttgcaat cttggcagaa 240

ctggacaaag agaaaagaaa actacttatg cagaaccagt cttcaacaaa tcatcctgga 300

gctagcattg cactctcgag accctctctt aataaggact tccgggatca cgctgagcag 360

cagcatattg cagcccaaca gaaggcagct ttgcagcatg ctcatgcaca ttcatctgga 420

tacttcatca ctcaagactc tgcatttggg aaccttattc ttcctgtttt acctcgcctt 480

gacccagaat gaagaaaaca tttgcgatgg aaaagtgact ttgtaatatc aaatgccaaa 540

gctactatca ttcagtgcta catgaactgt gactttaaga attttggtga actttgatat 600

tttttgtttg tctgaaagaa aggaatgtgt aagtgaaagc tgaaagaaga ataaccagga 660

tgatgagagc tgtggaagct gtatcgtcca aggaattgat tatgtaccgt gactgtaact 720

tttttgtaat gctgtttaac tctcaatcag actgtgaact ggatggtcac gaagtcattc 780

cccaactcct agcaagtttg actgaatata tcatgtccac agtagatttt caagaatcat 840

ttatagtact taactttaaa gaaacaaggc tgcttttaaa aaatgaacta ataggcttaa 900

atcaattgca tccatatttg ctgtttatag gattgctatc agtatacctt ttgcgtttat 960

agtcaacatg tatcatcctg aaatattctt tctggactta taactacttc cccctttttc 1020

actttaaaac aaacctcaag aataaattac taaccagtct taaccatctt ttataaacat 1080

atgctcttat aaatgttgtg actagatgca attaaaaata atagggaatg tggtaggttt 1140

ttaatttgta catcctctta tttagtgtta ccacataaat gatgagtttg tgtggttctg 1200

ttttccattt ttgttctaac tgaaaacttt ttggctgggc acggtgcctc atgcctgtaa 1260

tcccagcact ttgggaggcc aaggcgggca gatcacttga gatcaggagt ttgagaccag 1320

cctggccaac atggtgaaac cctgtctcta ctaaaagtat aaaaaattag ccatgtgtgg 1380

tggcacacgc ctgtaatccc agctactcag gaggctgagg caggagaatc gcttgaacct 1440

gggaggcagg ggttgcagtg agctgagacg gtgtcactgc actccagcct gggtgacagt 1500

gagtctttgt ctcaaagaaa aaaaaaaaaa aa 1532

<210> 7
<211> 1309
<212> DNA
<213> Homo sapiens

<400> 7
ctcgtttgcg atgttccgtt atctggatgc ggcggaggga tctggcggag ggaggtgttt 60

atgaggcgct gggggcggag gaggcgaatt agtccgagtg gagagagcga gctgagtggt 120

tgtgtggtcg cgtctcggaa accggtagcg cttgcagcat ggctgaccaa ctgactgaag 180

agcagattgc agaattcaaa gaagcttttt cactatttga caaagatggt gatggaacta 240

taacaacaaa ggaattggga actgtaatga gatctcttgg gcagaatccc acagaagcag 300

agttacagga catgattaat gaagtagatg ctgatggtaa tggcacaatt gacttccctg 360

aatttctgac aatgatggca agaaaaatga aagacacaga cagtgaagaa gaaattagag 420

aagcattccg tgtgtttgat aaggatggca atggctatat tagtgctgca gaacttcgcc 480

atgtgatgac aaaccttgga gagaagttaa cagatgaaga agttgatgaa atgatcaggg 540

aagcagatat tgatggtgat ggtcaagtaa actatgaaga gtttgtacaa atgatgacag 600

caaagtgaag accttgtaca gaatgtgtta aatttcttgt acaaaattgt ttatttgcct 660

tttctttgtt tgtaacttat ctgtaaaagg tttctcccta ctgtcaaaaa aatatgcatg 720

tatagtaatt aggacttcat tcctccatgt tttcttccct tatcttactg tcattgtcct 780

aaaaccttat tttagaaaat tgatcaagta acatgttgca tgtggcttac tctggatata 840

tctaagccct tctgcacatc taaacttaga tggagttggt caaatgaggg aacatctggg 900

ttatgccttt tttaaagtag ttttctttag gaactgtcag catgttgttg ttgaagtgtg 960

gagttgtaac tctgcgtgga ctatggacag tcaacaatat gtacttaaaa gttgcactat 1020

tgcaaaacgg gtgtattatc caggtactcg tacactattt ttttgtactg ctggtcctgt 1080

accagaaaca ttttctttta ttgttacttg ctttttaaac tttgtttagc cacttaaaat 1140

ctgcttatgg cacaatttgc ctcaaaatcc attccaagtt gtatatttgt tttccaataa 1200

aaaaattaca atttacccaa tggttgctct gcatctgagt catttaactg ttgaagtcta 1260

ataattttga aaataaaata tggcattggt ttctgcttgg taaaaaaa 1309

<210> 8
<211> 3638
<212> DNA
<213> Homo sapiens

<400> 8
agtctcgcgg tgctgccggg ctcagccccg tctcctcctc ttgctccctc ggccgggcgg 60

cggtgactgt gcaccgacgt cggcgcgggc tgcaccgccg cgtccgcccg cccgccagca 120

tggccaccac cgccacctgc acccgtttca ccgacgacta ccagctcttc gaggagcttg 180

gcaagggtgc tttctctgtg gtccgcaggt gtgtgaagaa aacctccacg caggagtacg 240

cagcaaaaat catcaatacc aagaagttgt ctgcccggga tcaccagaaa ctagaacgtg 300

aggctcggat atgtcgactt ctgaaacatc caaacatcgt gcgcctccat gacagtattt 360

ctgaagaagg gtttcactac ctcgtgtttg accttgttac cggcggggag ctgtttgaag 420

acattgtggc cagagagtac tacagtgaag cagatgccag ccactgtata catcagattc 480

tggagagtgt taaccacatc caccagcatg acatcgtcca cagggacctg aagcctgaga 540

acctgctgct ggcgagtaaa tgcaagggtg ccgccgtcaa gctggctgat tttggcctag 600

ccatcgaagt acagggagag cagcaggctt ggtttggttt tgctggcacc ccaggttact 660

tgtcccctga ggtcttgagg aaagatccct atggaaaacc tgtggatatc tgggcctgcg 720

gggtcatcct gtatatcctc ctggtgggct atcctccctt ctgggatgag gatcagcaca 780

agctgtatca gcagatcaag gctggagcct atgatttccc atcaccagaa tgggacacgg 840

taactcctga agccaagaac ttgatcaacc agatgctgac cataaaccca gcaaagcgca 900

tcacggctga ccaggctctc aagcacccgt gggtctgtca acgatccacg gtggcatcca 960

tgatgcatcg tcaggagact gtggagtgtt tgcgcaagtt caatgcccgg agaaaactga 1020

agggtgccat cctcacgacc atgcttgtct ccaggaactt ctcagctgcc aaaagcctat 1080

tgaacaagaa gtcggatggc ggtgtcaagg agccacaaac cactgtggta cacaacgcta 1140

cagatgggat caagggctcc acagagagct gcaacaccac cacagaagat gaggacctca 1200

aagtgcgaaa acaggagatc attaagatta cagaacagct gattgaagcc atcaacaatg 1260

gggactttga ggcctacacg aagatttgtg atccaggcct cacttccttt gagcctgagg 1320

cccttggtaa cctcgtggag gggatggatt tccataagtt ttactttgag aatctcctgt 1380

ccaagaacag caagcctatc cataccacca tcctaaaccc acacgtccac gtgattgggg 1440

aggacgcagc gtgcatcgcc tacatccgcc tcacccagta catcgacggg cagggtcggc 1500

ctcgcaccag ccagtcagaa gagacccggg tctggcaccg tcgggatggc aagtggctca 1560

atgtccacta tcactgctca ggggcccctg ccgcaccgct gcagtgagct cagccacagg 1620

ggctttagga gattccagcc ggaggtccaa ccttcgcagc cagtggctct ggagggcctg 1680

agtgacagcg gcagtcctgt ttgtttgagg tttaaaacaa ttcaattaca aaagcggcag 1740

cagccaatgc acgcccctgc atgcagccct cccgcccgcc cttcgtgtct gtctctgctg 1800

taccgaggtg ttttttacat ttaagaaaaa aaaaaaagaa aaaaagattg tttaaaaaaa 1860

aaaggaatcc ataccatgat gcgttttaaa accaccgaca gcccttgggt tggcaagaag 1920

gcaggagtat gtatgaggtc catcctggca tgagcagtgg ctcacccacc ggccttgaag 1980

aggtgagctt ggcctctctg gtccccatgg acttaggggg accaggcaag aactctgaca 2040

gagctttggg ggccgtgatg tgattgcagc tcctgaggtg gcctgcttac cccaggtcta 2100

ggaatgaact tctttggaac ttgcataggc gcctagaatg gggctgatga gaacatcgtg 2160

accatcagac ctacttggga gagaacgcag agctcccagc ctgctgtgga ggcagctgag 2220

aagtggtggc ctcaggactg agagcccgga cgttgctgta ctgtcttgtt tagtgtagaa 2280

gggaagagaa ttggtgctgc agaagtgtac ccgccatgaa gccgatgaga aacctcgtgt 2340

tagtctgaca tgcactcact catccatttc tataggatgc acaatgcatg tgggccctaa 2400

tattgaggcc ttatccctgc agctaggagg gggaggggtt gttgctgctt tgcttcgtgt 2460

tttcttctaa cctggcaagg agagagccag gccctggtca gggctcccgt gccgcctttg 2520

```
gcggttctgt ttctgtgctg atctggacca tctttgtctt gccttttcac ggtagtggtc     2580
cccatgctga ccctcatctg ggcctgggcc ctctgccaag tgcccctgtg ggatgggagg     2640
agtgaggcag tgggagaaga ggtggtggtc gtttctatgc attcaggctg cctttggggc     2700
tgcctccctt cttattcttc cttgctgcac gtccatctct tttcctgtct ttgagattga     2760
cctgactgct ctggcaagaa gaagaggtgt ccttacagag gcctctttac tgaccaactg     2820
aagtatagac ttactgctgg acaatctgca tgggcatcac cctccccgc atgtaaccca      2880
aaagaggtgt ccagagccaa ggcttctacc ttcattgtcc ctctctgtgc tcaaggagtt     2940
ccattccagg aggaagagat ctataccta agcagatagc aaagaagata atggaggagc      3000
aattggtcat ggccttggtt tccctcaaaa caacgctgca gatttatctg cacaaacatc     3060
tccacttttg ggggaaaggt gggtagattc cagttccctg gactaccttc aggaggcacg     3120
agagctggga gaagaggcaa agctacaggt ttacttggga gccagctgag aagagagcag     3180
actcacaggt gctggtgctt ggatttagcc aggctcctcc gagcacctca tgcatgtccc     3240
agcccctggg ccctagccct ttcctgccct gcagtctgca gtgccagcac gcaaatccct     3300
tcaccacagg gtttcgtttt gctggcttga agacaaatgg tcttagaatt cattgagacc     3360
catagcttca tatggctgct ccagccccac ttcttagcat tcttactcct cttctggggc     3420
taatgtcagc atctatagac aatagactat taaaaaatca ccttttaaac aagaaacgga     3480
aggcatttga tgcagaattt ttgcatgaca acatagaaat aatttaaaaa tagtgtttgt     3540
tctgaatgtt ggtagaccct tcatagcttt gttacaatga aaccttgaac tgaaaatatt     3600
taataaaata acctttaaac agtcaaaaaa aaaaaaaa                             3638
```

```
<210> 9
<211> 1275
<212> DNA
<213> Homo sapiens

<400> 9
agtgctgtac aaagagacag aggctgttag ctatggctga agacaaccac aaaaaaaaaa       60
cagttaagat gttggaatac ctgggcaaag atgttcttca tggtgttttt aattatttgg      120
caaaacacga tgttctgaca ttgaaggaag aggaaaagaa aaaatattat gataccaaaa      180
ttgaagacaa ggccctgatc ttggtagact ctttgcgaaa gaatcgcgtg gctcatcaaa      240
tgtttaccca aacacttctc aatatggacc aaaagatcac cagtgtaaaa cctcttctgc      300
aaatcgaggc tggaccacct gagtcagcag aatctacaaa tatactcaaa ctttgtcctc      360
gtgaagaatt cctgagactg tgtaaaaaaa atcatgatga gatctatcca ataaaaaaga      420
gagaggaccg cagacgcctg gctctcatca tatgcaatac aaagtttgat cacctgcctg      480
caaggaatgg ggctcactat gacatcgtgg ggatgaaaag gctgcttcaa ggcctgggct      540
```

acactgtggt tgacgaaaag aatctcacag ccagggatat ggagtcagtg ctgagggcat 600

ttgctgccag accagagcac aagtcctctg acagcacgtt cttggtactc atgtctcatg 660

gcatcctaga gggaatctgc ggaactgcgc ataaaaagaa aaaaccggat gtgctgcttt 720

atgacaccat cttccagata ttcaacaacc gcaactgcct cagtctaaag gacaaaccca 780

aggtcatcat tgtccaggcc tgcagaggtg aaaaacatgg ggaactctgg gtcagagact 840

ctccagcatc cttggcactc atctcttcac agtcatctga gaacctggag gcagattctg 900

tttgcaagat ccacgaggag aaggacttca ttgctttctg ttcttcaaca ccacataacg 960

tgtcctggag agaccgcaca aggggctcca tcttcattac ggaactcatc acatgcttcc 1020

agaaatattc ttgctgctgc cacctaatgg aaatatttcg gaaggtacag aaatcatttg 1080

aagttccaca ggctaaagcc cagatgccca ccatagaacg agcaaccttg acaagagatt 1140

tctacctctt tcctggcaat tgaaaatgaa accacaggca gcccagccct cctctgtcaa 1200

catcaaagag cacatttacc agtatagctt gcatagtcaa tatttggtat ttcaataaaa 1260

gtaaagactg tatct 1275

<210> 10
<211> 1130
<212> DNA
<213> Homo sapiens

<400> 10
agtgacagcc agccgggccc ggtggcggag aggaagtgcg gtccgcgcca agcccgtccc 60

cgccgacgcc ggctccccgc ggctcgggtg acagcgtcgc ggccgccgga cgcagcgcgg 120

ggcaggcgcg ggcagagccg agcgcagcgg aggctccggc ggaggcgcgg ggaaaatggc 180

tgatgacttt ggcttcttct cgtcgtcgga gagcggtgcc ccggaggcgg cggaggagga 240

cccggcggcc gccttcctgg cccagcagga gagcgagatt gcaggcatag agaacgacga 300

gggcttcggg gcacctgccg gcagccatgc ggcccccgcg cagccgggcc ccacgagtgg 360

ggctggttct gaggacatgg ggaccacagt caatggagat gtgtttcagg aggccaacgg 420

tcctgctgat ggctacgcag ccattgccca ggctgacagg ctgacccagg agcctgagag 480

catccgcaag tggcgagagg agcagaggaa acggctgcaa gagctggatg ctgcatctaa 540

ggtcacggaa caggaatggc gggagaaggc caagaaggac ctggaggagt ggaaccagcg 600

ccagagtgaa caagtagaga agaacaagat caacaaccgg gcatccgagg aggctttcgt 660

gaaggaatcc aaggaggaga ccccaggcac agagtgggag aaggtggccc agctatgtga 720

cttcaacccc aagagcagca agcagtgcaa agatgtgtcc cgcctgcgct cggtgctcat 780

gtccctgaag cagacgccac tgtcccgcta ggtgcctgct aggtgcatgg ccacagagca 840

tgggctgggc ctgggcacag gaggagcagc tgctttggtc ggggtggaga ctcgcagcag 900

```
ctgctaccca cagcctattc cactcctccc catctccagg cgctgggagg ggggccctca      960

ccccatcacg cctcgctccc tcctggccct ctggtccagc ccctcacgcc tcctctcagt     1020

ctactcaatt gtgactgtcc ctcctgatgt attttttttc ttggcttaaa gggtgtgttg     1080

ttgactcttt ttacacttat ttattatcat tctcacttct ctggaagcca                1130


<210> 11
<211> 5494
<212> DNA
<213> Homo sapiens

<400> 11
aaacgaagcc cacccaccga ctgacaaggc cccaagggga caagcgatcc ccgcgcggga       60

tactcacccg ttacctcagg atcgcgacta caactcccag gaggctgcgc gagcgacgga      120

ccaacgccct tcccagaatg cagcacagct gcatccctac cccgccctct cctttctccg      180

ctcctcctgc ttttctaccc gtcgtcaccc gggagagccg gagggggcta agttcgggtg      240

gcagcgccgg gcgcaacgca ggggtcacgg cgacggcggc ggcggctgac ggctggaagg      300

gtaggcttcc ttcaccgctc gtcctccttc ctcgctccgc tcggtgtcag gcgcggcggc      360

ggcgcggcgg gcggacttcg tccctcctcc tgctcccccc cacaccggag cgggcactct      420

tcgcttcgcc atccccccgac ccttcacccc gaggactggg cgcctcctcc ggcgcagctg      480

agggagcggg ggccggtctc ctgctcggtt gtcgagcctc catgtcggat aatcagagct      540

ggaactcgtc gggctcggag gaggatccag agacggagtc tgggccgcct gtggagcgct      600

gcggggtcct cagtaagtgg acaaactaca ttcatgggtg gcaggatcgt tgggtagttt      660

tgaaaaataa tgctctgagt tactacaaat ctgaagatga aacagagtat ggctgcagag      720

gatccatctg tcttagcaag gctgtcatca cacctcacga ttttgatgaa tgtcgatttg      780

atattagtgt aaatgatagt gtttggtatc ttcgtgctca ggatccagat catagacagc      840

aatggataga tgccattgaa cagcacaaga ctgaatctgg atatggatct gaatccagct      900

tgcgtcgaca tggctcaatg gtgtccctgg tgtctggagc aagtggctac tctgcaacat      960

ccacctcttc attcaagaaa ggccacagtt tacgtgagaa gttggctgaa atggaaacat     1020

ttagagacat cttatgtaga caagttgaca cgctacagaa gtactttgat gcctgtgctg     1080

atgctgtctc taaggatgaa cttcaaaggg ataaagtggt agaagatgat gaagatgact     1140

ttcctacaac gcgttctgat ggtgacttct tgcatagtac caacggcaat aaagaaaagt     1200

tatttccaca tgtgacacca aaaggaatta atggtataga ctttaaaggg gaagcgataa     1260

cttttaaagc aactactgct ggaatccttg caacactttc tcattgtatt gaactaatgg     1320

ttaaacgtga ggacagctgg cagaagagac tggataagga aactgagaag aaaagaagaa     1380

cagaggaagc atataaaaat gcaatgacag aacttaagaa aaaatcccac tttggaggac     1440
```

cagattatga agaaggccct aacagtctga ttaatgaaga agagttcttt gatgctgttg 1500
aagctgctct tgacagacaa gataaaatag aagaacagtc acagagtgaa aaggtgagat 1560
tacattggcc tacatccttg ccctctggag atgccttttc ttctgtgggg acacatagat 1620
ttgtccaaaa gccctatagt cgctcttcct ccatgtcttc cattgatcta gtcagtgcct 1680
ctgatgatgt tcacagattc agctcccagg ttgaagagat ggtgcagaac cacatgactt 1740
actcattaca ggatgtaggc ggagatgcca attggcagtt ggttgtagaa gaaggagaaa 1800
tgaaggtata cagaagagaa gtagaagaaa atgggattgt tctggatcct ttaaaagcta 1860
cccatgcagt taaaggcgtc acaggacatg aagtctgcaa ttatttctgg aatgttgacg 1920
ttcgcaatga ctgggaaaca actatagaaa actttcatgt ggtggaaaca ttagctgata 1980
atgcaatcat catttatcaa acacacaaga gggtgtggcc tgcttctcag cgagacgtat 2040
tatatctttc tgtcattcga aagataccag ccttgactga aaatgaccct gaaacttgga 2100
tagtttgtaa tttttctgtg gatcatgaca gtgctcctct aaacaaccga tgtgtccgtg 2160
ccaaaataaa tgttgctatg atttgtcaaa ccttggtaag cccaccagag ggaaaccagg 2220
aaattagcag ggacaacatt ctatgcaaga ttacatatgt agctaatgtg aaccctggag 2280
gatgggcacc agcctcagtg ttaagggcag tggcaaagcg agagtatcct aaatttctaa 2340
aacgttttac ttcttacgtc caagaaaaaa ctgcaggaaa gcctattttg ttctagtatt 2400
aacagtgact gaagcaaggc tgtgtgacat tccatgttgg agaaaaaaag aaaaaaaaaa 2460
gctgaatgct ctaagctgga acgtaggatc tatagccttg tctgtggccc aagaccttgg 2520
ccttgtgtac aaaaatgaca aaatattgca atagcaaagc tgaacatcta acactagcta 2580
tctcttgcta gatctccttg ctcagcatat aactataaat acatgtaaaa ttacatgtat 2640
atggctatat ttttatttgc ttgctcctag aagagaaaaa aaaatcaact ttgaatcaca 2700
actaggaatt gatgctttaa tttttggata ctttttcaga atttttaatt tactatggtc 2760
cggcctaaga tcctctgttg tatcaggttt tgtgcacaaa agaaaagcac aaaagttgaa 2820
tgcacatggg gcatgtgctt tctgtgcacc aaatatctgg atgaggttct tttttcaggc 2880
ctacagtcaa atctgtgtcc agaatttttt gacttttttg ctttgtataa tcatagaatt 2940
cattgctgct gatttctata atgattcatg ttgtcatgtg tctcttaata actgagggct 3000
gtcagtaacc tgtgattttg ccttttctat agtcttactc ccatgaagaa ccttggttct 3060
gatggagaaa gtgaaaagct ttatttcttc ccctagatat ctttatattt ctattatatt 3120
ttttagttgt gtactgtgta ctagagattt ttttcagttt gttatgaaca caatttggta 3180
agccctaaat tggttctgcc tgtctccaaa cagaaacatc tgtacaaatc ttgttggtat 3240
agactacttt ctggaaaatg gtcaagataa gttcatgttt tcttgaaatt tctaagatag 3300

tatatggtat cacttgttta aagcaaatca gactgagttt gacatttaat tcaatatttc 3360
tggtattcag taacgggtat atatgtttgt tcttccagtt tgggtcagtt taaaagatat 3420
gttgcaaagt atacatagaa aatgtgagca atgcctctct ttgccttttg atcagaaact 3480
tcagcagagc ggtaaggatt ccacatgatt taaactgaaa tgcttttctt tgttgctgta 3540
agaacttaaa atgtaaaata cctttttcag tttaagtcct gtaaacaaca ttgaagcatg 3600
gagatgaggc aaggaatagt actcactgaa gttgaaatga ctgcccactt caaaatcttc 3660
attgtgttta cacaccagtg tatttataca aatcagaggc attttgtaga tgctttgctg 3720
acttgttcag ctctgtaaaa acacagaaat cagacccatt ttgtaaagcg gaaaatcatg 3780
ttacatggaa catgtcctgt atatatcaca tacatggtaa tggagtctta atgataagtg 3840
caagataata atttaatgat gggattagtc tgatcgctta atatgcacaa tcctggaagt 3900
gaattacttg catcagatat agtgatattt attattctgt acagagagaa aaatacatat 3960
aaaacatatg cttacattac atgcacgcgg atttcatgct ccataatctt ttctattttt 4020
taatttacct ttctgtaaat gatgtgcatg gaatatgcct tatagaaaaa tgctgttcat 4080
aatttgacta cgtggaaaag tgcctatatg gtggtaatgc tagtaaggca aataagacaa 4140
attatcatgt tggtttacta catcaccagt taacatttta tattgtgatg tttaaaaaag 4200
aaaaatttat acctcaaatg tgtattttat tttacaatca gctgtggggt atggggttgg 4260
gatgggagaa tgggggggtt ggggagggca ggtttattca ccatagccgc tgataagaat 4320
cttcaaaaaa attctatatg cgcactataa atgtttctct gtttgccatt tctggtaact 4380
atcatgaaca cagacagtta actctttcat aactgaattg gatagcttta ttttacagaa 4440
gtatggcaag tttacaaagc aatatctaaa tctaattatc attagttgca tttggactaa 4500
atgtgatgat atacttttgc aattgattct gtaaataaaa ggattacact aaaatatttg 4560
tattaaaaga agaaaagata acattttacc tttagataac tgcacttgta cctcactaga 4620
gttaatccca cccaatcaga ttgagaaata aattggggaa tgtggaaaga gtccaaaaga 4680
ggtcagaatt tggagaggta ctggccttct ggacaacatt tagaccctct acaattattt 4740
tcattaagct gattcctaca tcctgaatat tcatgttttc tcatctacag atatttgtct 4800
tcccccaaac taaaagaaaa aaaactaccc tttactctct tttctactca gttactcttt 4860
tgtgctatgt tagaaacttg aaatatattg gtgatgtggg gattttgtcc ctgactgccc 4920
actgtacagg acaagagagt acagtgtttc agttggaatt caggactcct ggttttgagg 4980
tagaggatga tcactgcagt acttggtttg gaattgccac aggggtagct aaaccaaagg 5040
agggttatat ctgcaaggga ggtgtaagaa ggcaaaataa ggaaaaggag gaatgggttt 5100
tctatttgtt cagtttcatc aactaattta tacacttaat acaacttcag tgtcaattgc 5160
tattaagaaa tttttagttg ggctgagctg gttctcttgt gaaattgtgc tggttatctt 5220

taagcttatc agttatttgt ccaattaaac acttttcacc agtatttagt ccgagttgta 5280

cagacgatgt atttggattt tgtcatggtt catctacaga ctcaaaacat aatcatttta 5340

aagtaccttg ggagtgtgta gagtaacttc tataatagct ttatgatcct gatgatgttt 5400

tttaaacaca ataaagttgg atcttccatg ttacaatcac agaattaaaa ccagtattta 5460

aagtggaaaa gtattaaaat attatggaca aata 5494

<210> 12
<211> 713
<212> DNA
<213> Homo sapiens

<400> 12
tttcgttttt gcagagatgc aggtattgtc atgttgcctg ggctggtctc aaactcctgg 60

gttcaagcaa tcctcccacc tgtgcctccc taagtgttgg gattacaggc atgagccgct 120

gcctccccgc agcatggggg gacttcttat aagcattttt atttatttat ttggaattcg 180

gatggataga tcatgttggc ctgataatgt atgtgcaaga gcacagactc tggggtctca 240

tggacaagcc ccactctgcc ccttgcttaa tgtctctgag cctcagtttc ctcatctgta 300

acaagggcag aaatgccatc cgtgtgcagc agagtaccga cgaaaggatg gatgcaatgc 360

tcttacggca gtgccccacc caaggcacaa ggaaaaacca cgaaagcaat tccagtttac 420

accacgtccc aaactggatt ttccattcga ctataattcc tcccaataag ggctcaaaac 480

gttgtctaag gaaagtcgat tggttgttgc ccagggctgg aggagttggg ggaaaacggg 540

gagtgactgc tgatggagac agagtttcct tttgagatga tgagaatgtc ctgaagctgg 600

tcatgctgat ggttgcacgt atctatgaat atactaaaaa ccatcgaatt gtacacttta 660

gacatgtgaa ttgtgtagca tgtgaattaa acctcaagtc gttattttaa aaa 713

<210> 13
<211> 13126
<212> DNA
<213> Homo sapiens

<400> 13
gggggggtacc caacctatac gcagacccat tgagcagcca gactgcccta aacagggcta 60

tggagtcctt gtcccttcta agagctgtgg acaccttgcc tccagttccc ctcacagtcc 120

tttcttcctt ctgcagtgat cactgagtac ctgtgtgaat gccaacaaga aaaataaaac 180

cacagtttgg attcaggaaa ggttgcacca ccatcaggtc ttgaagggag cttcggctgg 240

ggcatctccc tgagaagcag catggagcag cctaacagta aaggctatag cctgggaagg 300

acccctcagg gcccagagtg cagcagtgct cctgcagtcc aagtggggac ccacaggggc 360

ctagagtata acccggggaa gattcttcca ggatcagact atgggttggg aaatcctcca 420

gcccttgacc ccaagctccc acatttaccc ctgccccccgg ccccacccac actctcagac 480
ttggggcagc cacggaagtc acccctgaca ggcactgata agaagtaccc gctgatgaag 540
cagcgtgggt tctactccga catcctcagc cctggaacct tagatcaact tggggaggta 600
tgtcgtggcc cccgaatgag ccagaacctc ctgcggcagg ctgaccttga caagttcacc 660
ccaagagtcg gaagctttga ggttcctgaa gacttccagg agcgcatgga gcagcagtgc 720
atcgggtcca ccacccggct gctcgcccag actgacttcc cactgcaggc ctacgagccc 780
aagatgcagg tgcctttcca ggtgctgcca ggccagcatc ctcgcaagat tgagatcgag 840
aggaggaaac agcagtacct gagcctggac attgagcagt tgctgttcag ccagggcatc 900
gactccaaca agctcatgcc caggcacctg gaccaccagc acccccaaac catcgaacag 960
ggccatgacc caatcttccc catctacctc ccactgaagg tatttgacaa tgaggacttt 1020
gactgccgga ctcccagaga gtggatcaac atgggcttgg agccagggtc tctggacagg 1080
aaacctgtcc cgggaaaagc cctcttgccc actgatgact tcctggggca tgaggacccc 1140
aagagtcaga agctgaagta caaatggtgc gaggtcggcg tcctggacta cgacgaggag 1200
aagaagctat acctggtaca caagacagac gagaaaggcc tggtgcgaga tgagatgggg 1260
aggcccatcc tgaatgcagg ggtcaccact gaaggaaggc caccccttca ggtctgtcag 1320
tactgggtgc cacggatcca gcttctcttc tgcgctgagg acccttgcat gttcgcacaa 1380
cgtgtggtcc aggccaacgc cctgcgcaag aacacggaag cactgctgct ctacaacttg 1440
tatgtggact gcatgccctc tgacggccag catgtcatca gtgaacagag cctgagcaag 1500
atcaagcagt gggccctgag cacgcctcgg atgcgcaaag gcccctcggt tctagagcac 1560
ctcagcagtc ttgccagaga agtgagcctg gactatgagc gcagcatgaa caagatcaac 1620
tttgaccacg ttgtctcttc caagcccgag accttctcct acgtcaccct ccccaagaag 1680
gaggaggagc aggtgcctga gcgagggctg gtgagtgtcc ccaagtacca cttctgggag 1740
cagaaggagg acttcacttt cgtgtccctg ctcacacggc cagaggtcat cacggccctc 1800
agcaaggtga gggccgagtg caacaaggtg accgccatgt ccctgttcca ctcgagcctc 1860
tccaagtaca gccacctgga ggaatttgag cagatccagt cacagacctt ctcccaggtg 1920
cagatgttcc tcaaggacag ctggatcagc tcgctaaagg tggccatgcg cagcagcctg 1980
cgcgacatga gcaagggctg gtacaacctc tacgagacca actgggaggt gtacctcatg 2040
tccaagctgc gcaagctgat ggagctggtg aagtacatgc tgcaggacac actgcgcttc 2100
ctggtgcagg actcacttgc cagcttctca cagttcatca gcgacacctg ttgcagcgtg 2160
ctcaactgca ccgatgacat ggtctggggt gacgacttaa ttaacagccc ctacaggccc 2220
cggaagaatc ccctgttcat catggacctg gtgctggaca gctctggggt gcactatagc 2280
accccactgg agcagtttga ggcatctctg ctgaacctct tcgacaaggg catcctggcc 2340

acccatgccg tgccccagct ggagaagctg gtgatggagg acatcttcat cagcggtgac 2400
cccctgctgg agtccgtggg ccttcatgag ccactggtgg aagagctacg ggccaccatt 2460
gccagtgccg tgtccaaggc catgatccca ctgcaggcct acgccaagga gtaccgaaag 2520
tacctggagc tgaacaacaa tgacattgcc tcctttctca aaacctacca gacgcagggc 2580
ctgttggccc aggaggtgcg ggaggtagtg ctcacccacc tgcgggagaa ggagatcctg 2640
gacagctcgc tgcccagcag catcatcatt gggcctttct acatcaacac cgacaatgtc 2700
aagcagagcc tgtccaagaa acgcaaggcc ctggccactt ccgtgctgga catccttgcc 2760
aagaacctgc ataaggaggt ggatagcatc tgcgaggagt tccgcagcat cagccgcaag 2820
atctatgaga agcccaacag cattgaggag ctggctgagc tgcgagagtg gatgaagggc 2880
atcccggaga ggctggtggg cctggaggag cggattgtga aggtcatgga tgactaccag 2940
gtcatggatg aattcctcta caacctcagc tcagatgact tcaatgacaa atggattgcc 3000
agcaactggc cttctaagat ccttgggcag atagagctgg tgcagcagca gcatgtggag 3060
gatgaggaga agttccgcaa aatccagatc atggatcaga acaacttcca agagaagctg 3120
gaagggctgc agctggtagt agctggcttc tccatccatg tggagatttc acgtgcacac 3180
gagatcgcca acgaggtgcg gcgtgtcaag aagcagctga aggactgcca gcagctggcc 3240
atgctctaca acaaccgcga gcgcatcttc agcttgccca tcaccaatta tgacaagctc 3300
tccaggatgg tgaaggagtt ccaaccctac ctggaccttt ggaccacagc gtctgactgg 3360
ctgcgctggt cggagagctg gatgaatgac cccctctctg ccatcgatgc tgagcagctg 3420
gagaagaacg tggttgaagc cttcaagacc atgcacaagt gcgtgaagca gtttaaggac 3480
atgccagcct gccaggaagt ggccttggac atccgggccc gcatcgagga gttcaaacca 3540
tacatcccac tgatccaggg gctgcgcaac cctggcatgc ggatccggca ctgggagaca 3600
ctgtccaacc agatcaacat caatgtcagg cccaaggcca acctgacctt tgctcgctgc 3660
ctggagatga acctgcagga ccatatcgag agcatcagca aggtggctga ggtggctggc 3720
aaggagtacg ccatcgagca ggcactggac aagatggaga aggagtggtc gaccatcctg 3780
ttcaatgtac tgccctacaa ggcgacagac acctacatcc tgaagagccc ggacgaggcc 3840
tcacagctgc tggacgacca catcgtcatg acccagaata tgtcattttc accctacaag 3900
aagccctttg agcagcgcat caactcctgg gagaacaaac tgaagctgac ccaggaggtt 3960
ctggaggagt ggctgaactg tcagcggtcc tggctctacc tggagcccat ctttagctct 4020
gaggacatca accagcagct gcctgtggag agcaagcgct accagaccat ggagcggatc 4080
tggaagaaga tcatgaagaa tgcctacgag aaccgggagg tgatcaatgt gtgttccgac 4140
ctgagaatgc tggacagcct gcgggactgc aacaagattc tggacctggt gcagaagggc 4200

ctcagcgagt atctggagac caagaggagc gccttcccca gattctactt cctgtcagat 4260
gatgaactac tagagatctt gtcgcagaca aaggacccca cggccgtgca gccacacctg 4320
cgcaagtgct tcgagaacat cgctcggctg ctattccagg aggacctgga gatcacgcac 4380
atgtactcag ccgaggggga ggaggtacag ttgtgcttct ccatctaccc ctccagcaac 4440
gtggaggact ggctgcggga ggtggagcgc agcatgaagg ccagtgtgca cgacatcatt 4500
gagaaggcca tcagggccta ccccacgatg cccaggaccc agtgggttct gaactggcct 4560
ggccaggtga ccatcgctgg gtgccagacc tactggacca tggaggtggc agaggctctg 4620
gaggccggca acctcagaag ccaactgttc cccagctct gccagcagct cagtgatctg 4680
gtggcccttg tgcgggggaa gctgtcccgc atgcagcggg cagtgctgtc agcgctaatc 4740
gtcattgagg tccatgccaa ggacgtggtg agcaagctaa tccaggagaa cgtggtcagc 4800
gtgaatgact tccagtggat ctcacagctg aggtactact ggacaaataa tgacctgtat 4860
atccgtgctg tgaatgctga gttcatctat ggctatgagt acctgggcaa cagtgggagg 4920
ctggtgatca cgcccctcac cgacaggtgc tacctgacac tgaccggagc tctgcacctc 4980
aagtttgggg gtgccccagc tggcccagct ggcacaggca aaactgagac caccaaagac 5040
ctgggtaagg ccttggccat acagaccgtt gtgttcaact gctctgacca gctcgacttc 5100
atggccatgg gcaagttctt caagggcctg gccagtgctg gggcctgggc ctgcttcgac 5160
gagttcaatc gcatcgacat cgaggtgctg tctgtggtgg cgcagcagat caccaccatc 5220
cagaaggcgc agcagcagcg ggtggaacgc ttcatgtttg agggtgtgga gatcccactg 5280
gtgccatcct gcgcagtgtt tatcaccatg aacccgggct acgctggccg cacggagctg 5340
cctgacaatc tgaaggcgct cttccgaccc gtggccatga tggttccaga ttacgccatg 5400
atcactgaga tctccctcta ttcctttggc tttaatgagg ccagtgtgct ggctaagaag 5460
atcacaacca ccttcaagct gtcttctgag cagctcagct cccaggatca ctatgacttc 5520
gggatgagag ccgtgaaaac tgtgatctcg gctgctggga acctcaagcg agaaaacccc 5580
agcatgaatg aggagctgat ctgcctccgg gccatccgtg atgtgaacgt gcccaagttc 5640
ctgcaggagg acctcaagct cttctctggc atcgtgtccg acctgtttcc caccatcaag 5700
gaggaggaca cggactacgg catcctggat gaggccatcc gcgaggcctg caggaacagc 5760
aacctcaagg atgtggaggg cttcctgaca aagtgcatcc agctctacga gaccacggtg 5820
gtacgacacg gcctcatgct cgtcgggccc acaggctccg gcaagagtac ttgttacaga 5880
gtcctggcag ctgccatgac gtcactgaaa gggcagccat ccatcagtgg tggcatgtac 5940
gaggctgtca actactacgt gctcaacccc aagtccatca cgatgggcca gctgtacggg 6000
gagtttgacc tcctcaccca tgagtggaca gacgggatat tctcctcgtt catccgggcg 6060
ggggccatca cctccgacac caacaagaag tggtacatgt tcgatgggcc ggtggatgcc 6120

atctggattg agaacatgaa cacggtgctg gatgacaaca agaagctgtg cctcagctct 6180
ggggagatca tcaagctcac agaggcaatg accatgatgt tcgaggtgca agacctggcg 6240
gtggcttcac cagctacagt ctcccgctgt ggcatggtgt acctggagcc cagcatcctg 6300
gggctcatgc ccttcatcga gtgctggctg aggaagctgc ctcccttgct gaagccctat 6360
gaggagcatt tcaaggccct ctttgtcagc ttcctggagg aatccatctc cttcgttcgg 6420
tcctcagtga aggaggtgat cgcctcaacc aactgcaacc tgaccatgag cctcctcaag 6480
ctgctggact gcttcttcaa gccctttctg cctagagagg gcctcaagaa aataccctct 6540
gaaaagctga gtcgcatcgt agagttgatc gagccctggt tcatcttctc cctgatctgg 6600
agcgtgggtg ccactgggga cagcagtggc cgcaccagtt tcagccactg gctaaggctc 6660
aagatggaga acgaacagct gactctgctt ttcccagaag aggggctggt gttcgattac 6720
aggctggagg acgcgggcat cagtggcacc aacgacagtg aggatgaaga ggaggaatac 6780
aagcaggttg cctgggtgaa gtggatggac tcctcagctc cattcaccat ggtaccagac 6840
accaactact gcaacatcat tgtgcccacc atggacaccg tgcagatgtc ccatttactg 6900
gacatgctgc tcaccaacaa gaagcccgtg ctgtgcattg ggccaacagg cacggggaag 6960
acgctcacca tctctgacaa gctcctcaag aacctggcac tggattacat cagccacttc 7020
ctcaccttct cagcccgcac ttcagccaac cagacccagg acttcattga cagcaagctg 7080
gacaagaggc ggaagggtgt gtttggacca cctctggggc gcaactttat cttcttcatc 7140
gatgacctga acatgccggc cctggagacc tacggtgcac agccacccat cgagctgttg 7200
cgccagtgga tggaccacgg cggctggtac gaccgcaaga tcattggtgc cttcaagaac 7260
ctagtggaca tcaactttgt ctgtgccatg ggcccccggg gtggaggcag gaacaccgtc 7320
accccgcggc tgatgcgtca cttcaactac ctgtctttcg ctgagatgga cgaggtcagc 7380
aagaaacgca tcttctccac catcctgggc aactggttgg atggactcct tggagaaaaa 7440
agctaccggg agcgtgtgcc tggggccccc cacattgccc acttcacgga gccccttgtg 7500
gaagccacca tcatggtgta tgcaaccatc acctcccagc tgctgcccac tccagccaag 7560
tcccactaca ccttcaacct gagggacctc tccaaggtct tccaaggcat gctcatggct 7620
gacccggcca aggtcgagga ccaagtgcag ctgctgcgac tgtggtatca cgagaactgc 7680
cgcgtgttcc gggaccgact ggtgaatgag gaggaccgca gctggttcga ccagctcctc 7740
aagcgctgca tggagcagtg ggaggtgacc ttcaacaagg tctgcccctt ccagcccatt 7800
ctttacgggg acttcatgtc accaggctcc gatgtcaagt cctacgagct catcaccagt 7860
gagagtaaga tgatgcaggt gatagaggag tacatagagg actacaacca gatcaacacg 7920
gccaagctga agctggtcct cttcatggac gccatgagcc acatctgtcg catcagccgc 7980

accctacgcc aggcgctggg caatgcactc ctgctgggcg tgggtggcag cggccgcagc 8040

tccctcacaa ggctcgcctc gcacatggcc gagtacgagt gcttccagat tgaactatcc 8100

aagaactacg gcatgtccga gtggcgagat gatgtgaaga aggtcctgct caaggcgggc 8160

ctacagaacc tacccatcac cttcctcttc tcagacaccc agatcaagaa cgaatccttc 8220

ctggaagata tcaacaacgt cctaaactct ggtgacattc ccaatctgta tactgcggac 8280

gagcaggacc agatcgtcag caccatgcgg ccctatatcc aggagcaggg cctacagccc 8340

accaaggcca acctcatggc tgcttacaca gggcgtgtgc gcagcaacat ccacatggtg 8400

ctgtgcatga gccccatcgg agaggtcttc cgagctcgtc tgaggcagtt tccctccctg 8460

gtcaactgct gtaccatcga ctggtttaac gagtggccgg cagaagccct gaagtctgtg 8520

gccaccgtgt tcctcaatga gatcccagaa ctggaatcct cccaggaaga aatccaagga 8580

ctgatccagg tctgtgtgta catccaccag tcggtgtcca agaagtgcat cgagtacctg 8640

gcagagctga cccgccacaa ctatgtgacc cccaagagct acttggagct gcttcatatt 8700

ttctccatcc tcatcgggca gaagaaactg gagctgaaaa ctgccaagaa ccgcatgaag 8760

agcggcctcg acaagctgct gcgcacttct gaggatgtag ccaagatgca ggaggacctg 8820

gagagtatgc accccctgct ggaggaggct gccaaggaca ccatgctcac catggagcag 8880

atcaaggtgg atacggccat cgccgaggag acccggaatt cagtgcagac agaggagatc 8940

aaagccaatg agaaggccaa gaaggcacaa gctattgctg acgatgccca gaaggacctg 9000

gacgaggcgt tgccagccct ggatgcggct ctggccagcc tgcgcaacct caacaagaac 9060

gatgtgaccg aggtacgtgc catgcagcgg ccaccccccgg gtgtgaaact ggtcatagaa 9120

gctgtgtgca ttatgaaagg catcaagccc aagaaggtgc ctggagaaaa gccaggcacc 9180

aaggtggatg actactggga gcctggcaag gggctgctgc aggacccggg ccacttcctt 9240

gagagcctct tcaagtttga caaggacaac attggggatg tggtgatcaa agccatccag 9300

ccgtacatcg ataatgaaga gttccagcca gccaccattg ccaaggtgtc caaggcttgc 9360

acctccatct gccagtgggt gcgcgccatg cacaagtacc actttgtggc caaggccgtg 9420

gagcccaagc ggcaagccct gctggaggcc caggatgacc tgggggtgac acagaggatc 9480

ctggatgagg caaaacagcg ccttcgtgag gtggaggacg gcatcgccac aatgcaggct 9540

aagtaccggg aatgcattac caagaaggag gagctggagc tgaagtgtga gcagtgtgag 9600

cagcggctgg gccgagctgg caagctcatc aacgggctgt cggatgagaa ggtgcgctgg 9660

caggagacgg tggagaacct gcagtacatg ctcaacaaca tctccggcga tgtcctggtg 9720

gccgctggct ttgtggccta cctgggcccc ttcacgggcc agtaccgcac ggtgctctac 9780

gacagctggg tcaagcagct caggagccac aatgtcccac acacctccga gcccacgcta 9840

atcgggacgc tggggaaccc tgtgaagatc cgatcgtggc agatcgctgg cctccccaac 9900

gacacactgt cagtggagaa cggggtcatc aaccagtttt cccagcgctg gacccacttc 9960
attgaccctc agagccaggc caacaaatgg atcaagaaca tggagaagga caatgggctg 10020
gatgtgttca agttgagtga ccgcgacttc ctgcgcagca tggagaacgc catccgcttt 10080
ggcaagccat gtctcctgga gaacgtgggc gaggagctag acccagccct ggagccagtg 10140
ctgctcaagc agacgtacaa gcagcaggga aacacggtgc tgaagctggg ggacacggtg 10200
atcccctacc atgaggactt caggatgtac atcaccacca agctgcccaa cccacactac 10260
acgcccgaga tctccaccaa actcaccctc atcaacttca ccctgtcgcc cagtggccta 10320
gaggaccagc tactgggcca ggtagtggca gaggagcgac ccgacctgga ggaggccaag 10380
aaccagctga ttatcagtaa tgccaagatg cgccaggagc tgaaggacat tgaggaccag 10440
atcctgtacc ggctcagctc ctccgagggc aaccctgtag atgacatgga actcatcaag 10500
gtgctggaag cctccaagat gaaggctgct gagatccagg ccaaagtcag gattgcagag 10560
cagacggaga aggacatcga cctgacgcgc atggagtaca tacccgtggc catccgcacc 10620
cagatcctct tcttctgtgt gtccgacctg gccaacgtgg accccatgta ccagtactcc 10680
cttgagtggt ttctcaacat cttcctctcg ggcatcgcca actcagagag agcagacaac 10740
ctgaagaagc gcatctccaa catcaaccgc tacctgacct acagcctcta cagcaacgtc 10800
tgccgcagcc tctttgagaa gcacaagctg atgtttgcct tcctgctgtg tgttcgcatc 10860
atgatgaacg agggcaaaat caaccagagt gagtggcgat acctcctgtc tggggggctcc 10920
atctcgatca tgactgagaa tccggcaccg gactggctgt cagaccgggc ttggcgagac 10980
atcctagcac tctcgaacct gccaaccttt tcctccttct cttccgactt cgtgaagcac 11040
ctctcagaat tccgggtcat cttcgacagc cttgagcccc accgggagcc tttgcctggc 11100
atctgggacc agtacctaga ccagttccag aagctgctag tcctccgctg cctgcgtggg 11160
gacaaggtta ccaacgccat gcaggacttt gtggccacca acctggagcc acgcttcatt 11220
gaaccccaga cagccaatct gtcagtggtg ttcaaagact ccaactccac cacacccctc 11280
atctttgtgc tgtcacccgg cacagaccct gctgccgacc tctacaagtt tgccgaagaa 11340
atgaagttct ccaaaaagct ctctgccatc tccctgggcc aggggcaggg ccctcgggca 11400
gaagccatga tgcgcagctc catagagagg ggcaaatggg tcttcttcca gaactgccac 11460
ctggcaccaa gctggatgcc agccctagaa cgcctcatcg agcacatcaa ccccgacaag 11520
gtacacaggg acttccgcct ctggctcacc agcctgccca gcaacaagtt cccagtgtcc 11580
atcctgcaga acggctccaa gatgaccatt gagccgccac gcggtgtcag ggccaacctg 11640
ctgaagtcct atagtagcct tggtgaagac ttcctcaact cctgccacaa ggtgatggag 11700
ttcaagtctc tgctgctgtc tctgtgcttg ttccatggga acgccctgga gcgccgtaag 11760

tttgggcccc tgggcttcaa catcccctat gagttcacgg atggagatct gcgcatctgc 11820

atcagccagc tcaagatgtt cctggacgaa tatgatgaca tcccctacaa ggtcctcaag 11880

tacacggcag gggagatcaa ttacgggggc cgtgtcactg atgactggga ccggcgctgc 11940

atcatgaaca tcttggagga cttctacaac cctgacgtgc tctcccctga gcacagctac 12000

agcgcctcgg gcatctacca ccagatcccg cctacctacg acctccacgg ctacctctcc 12060

tacatcaaga gcctcccact caatgatatg cctgagatct ttggcctgca tgacaatgcc 12120

aacatcacct ttgcccagaa cgagacgttc gccctcctgg gcaccatcat ccagctgcaa 12180

cccaaatcat cttctgcagg cagccagggc cgggaggaga tagtggagga cgtcacccaa 12240

aacattctgc tcaaggtgcc tgagcctatc aacttgcaat gggtgatggc caagtaccca 12300

gtgctgtatg aggaatcaat gaacacagta ctagtacaag aggtcattag gtacaatcgg 12360

ctgctgcagg tgatcacaca gacactgcaa gacctactca aggcactcaa ggggctggta 12420

gtgatgtcct ctcagctgga gctgatggct gccagcctgt acaacaatac tgtgcctgag 12480

ctctggagtg ccaaggccta cccatcgctc aagcctctgt catcatgggt catggacctg 12540

ctgcaacgcc tggactttct gcaggcctgg atccaagatg gcatcccagc tgtcttctgg 12600

atcagtggat tcttcttccc ccaggctttc ttaacaggca ctctgcagaa ttttgcccgc 12660

aaatttgtca tctccattga caccatctcc tttgatttca aggtgatgtt tgaggcacca 12720

tcagagttaa cacaaagacc ccaagtaggg tgctatatcc atggattatt cctggaaggt 12780

gcccgctggg atccagaggc cttccagctg gctgagtctc agcccaagga gctgtacaca 12840

gagatggccg ttatctggct cttgccaaca cccaaccgca aggcccagga ccaggacttt 12900

tacctgtgcc ccatctacaa gacactgact cgtgctggaa cactatcaac cacaggacac 12960

tctaccaact atgtcattgc tgtggagatc cccacccatc agccccagcg acactggata 13020

aagcgtggtg tggccctcat ctgtgccctg gactactaga ctcagacaga agggctgggg 13080

ccattaaagc tgaattttct aagcagtcca aaaaaaaaaa aaaaaa 13126

<210> 14
<211> 8019
<212> DNA
<213> Homo sapiens

<400> 14
atctgtcagt tacgggggct gcccggcggg cgggctggag gcgctcgcag gctgcctagt 60

cgttcgctcg ctctctctcg cgtgctccct ctcgccgcct gtggggaagt cggggccgcc 120

cgagcggccg tcgccaccgc ctcgccccga cctccttccc ggtcccctcc cggcccagtc 180

cctcccgccc gcgcaccccg agtagtgagt ggccccgcgc agggtctgga gagtcaccgc 240

ggcggcgccg ggtggtggtt aaaccatgtt aaggaggatt ttacagagga ctcctgggag 300

agttggctct caaggttctg atttagattc atcagcaact cctataaaca cagtggacgt 360
caataatgaa agctcttcag agggtttcat atgtccccag tgtatgaaat ctcttggatc 420
tgctgatgaa cttttcaaac attatgaagc tgttcatgat gctggtaatg actcaggtca 480
tggaggagag tctaatcttg ctttgaagcg agatgatgta acactgctca gacaagaggt 540
ccaagaccta caggcttcac ttaaggaaga aaaatggtac tcggaagaat taaagaagga 600
attagaaaaa tatcaagggc tgcagcagca agaggccaaa cctgatgggt tggtgactga 660
ttcatcagca gaactacagt ctttggaaca gcaattagaa gaagcccaaa cagaaaattt 720
taatattaag caaatgaaag acttatttga acagaaagca gcccaacttg ctactgaaat 780
tgcagatata aagtcaaagt atgatgaaga aaggagtctt cgagaagctg ctgaacaaaa 840
agtgacacgt ctgacagaag aattaaacaa agaggcaact gtaattcaag atctgaagac 900
ggaactgctt cagagacctg gtatagaaga tgttgccgtg ctaaagaaag aactggtcca 960
agttcaaaca ctaatggata acatgacctt ggaacgtgag cgagaatctg aaaaactcaa 1020
agatgaatgc aaaaaattgc agtcacaata tgctagctca gaggccacaa taagccagct 1080
aaggagtgaa cttgccaaag gcccccagga agttgctgta tatgtacagg aactacaaaa 1140
actgaaaagt tcagttaatg aattaacaca aaaaaatcag accttgacag aaaacttgct 1200
gaaaaaagaa caagactata ctaagttaga ggagaaacat aatgaagaat ctgtgagtaa 1260
aaagaatatt caggcaaccc ttcatcaaaa agacctagat tgtcaacagc ttcagtcaag 1320
attgtctgca tctgaaacct cactgcatag aatacatgta gaactaagtg aaaaaggaga 1380
agctactcaa aagctcaaag aagaattatc tgaggtagag accaagtacc agcatctaaa 1440
ggcggagttt aagcagctac aacaacagag agaagaaaag gagcagcatg ggttacaact 1500
ccaaagtgaa attaatcaat tacatagcaa acttctggag acagagcgcc aactagggga 1560
agctcatggt aggctgaagg aacagagaca gctttcaagt gaaaagttga tggataaaga 1620
acaacaagtg gctgatttac aactcaaact ttctcggtta gaagagcagt tgaaggaaaa 1680
agttacaaat tctacagaat tgcagcatca attagataaa acaaagcaac agcatcaaga 1740
acaacaggct cttcagcaaa gcaccacggc aaaacttcga gaagctcaga atgatttgga 1800
acaagttcta cgtcaaattg gcgataagga ccaaaagatc cagaaccttg aagctttatt 1860
acagaagagt aaagaaaata tttcattact agaaaaagaa agagaagatc tttatgcaaa 1920
aattcaggct ggtgaaggag agactgctgt tcttaaccag ttacaagaaa aaaaccatac 1980
actacaggag caagtaactc aactaacaga gaagctgaag aatcagtcag aaagtcataa 2040
acaagcccag gagaatttgc atgaccaggt acaagagcag aaggcacatc ttagagctgc 2100
acaagaccgt gtcctttccc tagaaactag tgtcaatgaa ttaaatagtc aattaaatga 2160
aagcaaggag aaggtctccc agcttgacat acagattaaa gccaaaaccg aactattact 2220

atcagcagaa gcagcaaaaa ctgctcaaag agctgatctt cagaatcatt tggacacagc 2280
tcaaaatgca ttacaagata aacagcagga gttaaataag attactactc agttggatca 2340
ggtcactgca aagttacaag acaagcaaga acattgcagt cagctggaaa gtcatcttaa 2400
agaatataaa gagaaatacc tctctttaga acagaaaacc gaagagctag aaggtcaaat 2460
taagaaacta gaagctgata gtcttgaagt taaagcaagc aaggagcagg ctttgcaaga 2520
tctacaacag caaagacagc tgaacacaga tttagagctc agagccacag aattgagtaa 2580
acaacttgaa atggagaagg aaatagtatc cagtacaaga ttggatctac agaaaaaatc 2640
tgaagccctt gaaagtatca agcaaaagct taccaagcaa gaggaagaaa aacaaatcct 2700
gaaacaagat tttgaaactt taagtcaaga aacaaagatt cagcatgagg aattgaataa 2760
cagaattcaa acaacagtaa cagaactaca aaaagtgaaa atggagaaag aagctttaat 2820
gacagagctt tctacagtaa aggacaaact atcaaaagtt tctgattctt tgaaaaactc 2880
taaaagtgaa tttgaaaagg agaatcagaa aggaaaagcc gctatattag acttggaaaa 2940
aacttgcaaa gaattaaagc atcaacttca agtgcagatg gaaaacacac ttaaggaaca 3000
gaaggaactg aaaaagtcac ttgaaaaaga gaaggaggct tctcatcagt tgaaattgga 3060
actcaattca atgcaggaac aacttataca ggcccagaat actttaaaac aaaatgaaaa 3120
agaagagcaa caacttcagg ggaacataaa tgagctaaag caatcaagtg aacagaagaa 3180
aaaacaaatt gaagcactcc aaggagagct taaaattgct gttttacaga agacagagct 3240
tgagaataaa ctacagcagc agttaacaca ggcagcccag gaacttgcag cagagaaaga 3300
gaaaatatca gtattacaaa acaactatga aaaaagtcag gaaactttca aacagcttca 3360
atctgatttc tatgggaggg aatctgaact tctagccacc aggcaagatc ttaagtctgt 3420
agaagagaag ctttctctag cacaggagga cttgatttca aacagaaatc aaattggaaa 3480
tcaaaataaa ttgattcaag aactgaagac tgccaaggct acattggagc aggattcagc 3540
aaagaaagaa cagcaattgc aggagcgatg taaagcacta caagacattc agaaagaaaa 3600
gtcactgaaa gaaaaagaac tggtaaatga gaagtctaaa ttggcagaga tagaagaaat 3660
taaatgtaga caagaaaaag aaatcactaa actaaacgaa gaactcaagt cccacaaact 3720
agaaagcata aaggagataa caaatcttaa agatgctaaa cagcttctaa ttcagcagaa 3780
attagaactt caaggaaaag cggactccct gaaggcagct gttgaacagg agaagagaaa 3840
tcagcagata ctaaaagacc aggtgaaaaa ggaagaagag gagctgaaga aagaatttat 3900
tgagaaagaa gctaagttgc attccgaaat aaaagaaaag gaagtaggaa tgaagaagca 3960
tgaagaaaat gaggctaaac ttaccatgca gattacagca ttaaatgaaa acttaggcac 4020
tgtgaagaag gagtggcaat ctagtcaacg gagagttagt gagcttgaga aacaaacgga 4080

tgacttacgg ggtgaaattg cagtattaga agcaacggtt cagaataatc aagatgaaag 4140
gagagcacta ctggaaagat gtcttaaagg agaaggtgaa atagaaaagc ttcaaaccaa 4200
agtattagaa ttgcaaagaa agctggataa tacaactgca gcagtgcagg agctgggcag 4260
agaaaaccaa tcacttcaga tcaaacatac acaagcgttg aatagaaagt gggccgaaga 4320
caatgaagta caaaactgta tggcctgtgg gaaaggcttt tcagtaacag tgagacggca 4380
tcactgccga cagtgtggaa atatcttctg tgctgaatgt tcagccaaaa atgccttaac 4440
tccttcctcc aagaagcctg ttcgtgtctg tgatgcatgt ttcaatgact tgcaaggata 4500
atgggttatc acaacttcag agtaatatta cactaacatt agatttttaa taaatgtact 4560
taatagaggt cttggactac tatttggttt ggacactggt tgcaatacta gaccaaatag 4620
gaattagtat attttggaat gttatggata tcaagtaaaa ctcttctatt tttgtgagac 4680
ttgggcttat catctttcat tacttttttc ccatttagcc cctggaatag ctttcatgcc 4740
aagtttagaa ttagccaatg aaatgtaaat aaactttgga cagaaaatag caatgtattt 4800
ttttaatata atttcattat tcacaaatga agaatgcaat tccatagctt acttctttct 4860
cctaaattta atgtacaaaa atgtacatgg atgatattat cacttgttgc tgtttttgtt 4920
gcacaatagc acattaatta tattaaatat tctctttgtg aagttatgca cagaactgta 4980
acactttcct ctgccttttt ctcttatatg tgggttcatg gttcagttgc attgttatgt 5040
tatgaattta ctacttgggt gttctaaata cagtttatct taaaatatgt agtaatatga 5100
attcaaaaat ggtttttgtt ttaacaatat taattaaatt ttcttgggtg gattttataa 5160
aaacctctgt aaaatcaatt tgtttactta tagttatctg gggttgtatt gcaaagaata 5220
gggttaattt ttcttttgt ctttttctgt ctctttctta aactcccacg cattgtccat 5280
ttagaatctt agaaagcttc ttaccctcgc tgtcagttgg aggatgaata ataaacattg 5340
tataatgttc ttattctcgg tttcttgtaa gtgggaagtc ttttgagatc agccttcata 5400
tcataccctt attttgtctc tctagcatct tcaggaacct gagtttgctt tttacataga 5460
tctgattttt tcctttattt ggtttaacat tttcaagcat agatcagctt catgtatttg 5520
ttttataatt ttgcatctta aagctagtaa ctaatattgc tgtggtctta ttggtgaaaa 5580
caaaatataa aatgataaga agacattaaa tattttactt tctatttctt accctattgg 5640
gaagaatttt taaaagttgt aaaattgact taaagttaaa atggttctga atttggtgtt 5700
ccatgtcaac tgttcccagt tttaatttta actttgcaaa aataatgttg tgaaattccc 5760
tttaagtaac taacacctag aaatagagta tcaccaaaaa caaatgtatt taggaatccc 5820
agttacaata aaaactgaaa cagcatggct aagcatctta aggataatta agaaattagc 5880
ttatttgtat gctgaacaat ggaatcgaac tttaatttag catagtctta ttaatttgga 5940
aaattgtaaa acatctcaag agagaagtta ttaatattgg tttattaatc tttctagaga 6000

agaagtgtag ttatacattc attcagtgca atcattttat aacagtagtt aatatttcat 6060
ttataatagt tcttaatata catttgcttt caaatattct gaaatgaaat tatattcatg 6120
ctatacctac agtgtatgta aactaaagag atcactacat atttgtttaa ttatttaaaa 6180
aaaaatttaa gtaaaccctg ttgtcattag ctattaagaa cattgattaa gtccctggac 6240
ttggataaac atatatcctt gaacatatat aatgaagaaa tacagtggct ctttattaaa 6300
aataatagtt ggataatata aactgaacta tttatgcatt tttatatact tataaatcct 6360
tccaaatagt tttaattcta tccttttaca tataaataac ttaataagtg tgctggaaaa 6420
acacagatat tcacagcacc actgtttttt tttttttttt gagataataa attccatgag 6480
aaatctgggt ttgaatattt gtttactttg tctcctaatt gaacaccact ccaggccttc 6540
tgtctgtctc ccctttaccc ccaaaatact cacaaaaaaa ttttaagaca acaagtaacc 6600
atatataggt gtttgaatga ttttctcatt tttatctaat ttcatttcat aagtcccgag 6660
taatttacct accataggct actatactga taatataaat gaaaccgaac atttttttgct 6720
actaactctc cccaatttaa tgtgttttcg aaataaaaat ttaaattttt ttccttttaa 6780
ttaaaaagtc atctttgaag tccttattgg ctgtacattt tacatgtttg ctggtactat 6840
tattttgtca gtcagttaaa gctggcatgt acagctcttg gctttaatga aaagcacatt 6900
gacataatgt tagtaaattc caaaccccgg cacagaatgt gagttaaaat taagtcttgc 6960
tgggttagtg tacaataaac tatacctaca gacttttttt taatagaaag aagacaaagc 7020
tgctggtata ggatttgttc ctttgaagaa aaaatgaggg aaacaaacac aaaaacctca 7080
atgcagtgta taaataacat tttgttcaac tacctcttaa tgtggaatta tctactttaa 7140
tagtttcctg acagtaatgt taaatagtaa ctgccaaatt tgttattttc ccatctctct 7200
taaaaaagtc tttatgatta ttttatatag ttttgagaac tttaaagcca cttttttta 7260
accttacatt tgcataaaaa tgtttagctt ttaagtagag agcaaattat gatcatatat 7320
tttgatattc atgacctgtt tgactatagg agttttttta aaaaaatgca ctttggctat 7380
aaaaccatgg atgatttgat ccataagatt taaatgtgcc accattatag tattcctaga 7440
catgagcttg atgaatggta ttctgtaatt ataacgtgcc acacattatt gtgtcttaat 7500
tgcccttagc ctgaatttta atgatcaatt tgttattgtt gcagatgtga atattgtgca 7560
taaacttact aaatttatgt aaaattgtat aaaatagaat tagaagtcac taagttcttt 7620
ctgtgtagaa gtaataaatt tattgtaaca caatgcagtt gtgtatatga cattctgtaa 7680
ttccttgaac tggatcatat attcataagt tctgtagata cttatgcatg aacattttct 7740
catttagttt ttgggttcat tatttgtatt gtgtttacta cttgtgatca tgtagttgtg 7800
ccttactttg tgagaaaggt tagctcagta aatactgcaa tttctaaact cagtgattgg 7860

aaggttatta attataaatg taactgataa agtacgtgac agcatttaaa tctgtataaa 7920

gaacaatgga aggatcctta ttgaattgtt gcttttttt aatatgttta aatattatat 7980

taaaaacatt tctttctaaa ctaaaaaaa aaaaaaaaa 8019

<210> 15
<211> 2111
<212> DNA
<213> Homo sapiens

<400> 15
gctttcccct gcctgcctgt ctctagtttc tctcacatcc cttttttttt ttcctttctc 60

tagccaccct gaagggtccc ttcccaagcc cttagggacc gcagaggact tggggaccag 120

caagcaaccc ccagggcacg agaagagctc ttgctgtctg ccctgcctca ccctgcccca 180

cgccaggccc ggtggccccc agctgcatca agtggaggcg gaggaggagg cggaggaggg 240

tggcaccatg ggcccgggcg gtgccctcca tgcccggggg atgaagacac tgctgccatg 300

gacagcccgt gccagccgca gcccctaagt caggctctcc ctcagttacc agggtcttcg 360

tcagagccct tggagcctga gcctggccgg gccaggatgg gagtggagag ttacctgccc 420

tgtcccctgc tcccctccta ccactgtcca ggagtgccta gtgaggcctc ggcagggagt 480

gggacccca gagccacagc cacctctacc actgccagcc ctcttcggga cggttttggc 540

gggcaggatg gtggtgagct gcggccgctg cagagtgaag gcgctgcagc gctggtcacc 600

aaggggtgcc agcgattggc agcccagggc gcacggcctg aggcccccaa acggaaatgg 660

gccgaggatg gtggggatgc cccttcaccc agcaaacggc cctgggccag gcaagagaac 720

caggaggcag agcgggaggg tggcatgagc tgcagctgca gcagtggcag tggtgaggcc 780

agtgctgggc tgatggagga ggcgctgccc tctgcgcccg agcgcctggc cctggactat 840

atcgtgccct gcatgcggta ctacggcatc tgcgtcaagg acagcttcct gggggcagca 900

ctgggcggtc gcgtgctggc cgaggtggag gccctcaaac ggggtgggcg cctgcgagac 960

gggcagctag tgagccagag ggcgatcccg ccgcgcagca tccgtgggga ccagattgcc 1020

tgggtggaag gccatgaacc aggctgtcga agcattggtg ccctcatggc ccatgtggac 1080

gccgtcatcc gccactgcgc agggcggctg ggcagctatg tcatcaacgg gcgcaccaag 1140

gccatggtgg cgtgttaccc aggcaacggg ctcgggtacg taaggcacgt tgacaatccc 1200

cacggcgatg ggcgctgcat cacctgtatc tattacctga atcagaactg ggacgttaag 1260

gtgcatggcg gcctgctgca gatcttccct gagggccggc ccgtggtagc caacatcgag 1320

ccactctttg accggttgct cattttctgg tctgaccggc ggaaccccca cgaggtgaag 1380

ccagcctatg ccaccaggta cgccatcact gtctggtatt ttgatgccaa ggagcgggca 1440

gcagccaaag acaagtatca gctagcatca ggacagaaag gtgtccaagt acctgtatca 1500

cagccgccta cgcccaccta gtggccagtc ccagagccgc atggcagaca gcttaaatga 1560

cttcaggaga gccctgggcc tgtgctggct gctccttccc tgccaccgct gctgcttctg 1620

actttgcctc tgtcctgcct ggtgtggagg gctctgtctg ttgctgagga ccaaggagga 1680

gaagagacct ttgctgcccc atcatggggg ctggggttgt cacctggaca gggggcagcc 1740

gtggaggcca ccgttaccaa ctgaagctgg gggcctgggt cctaccctgt ctggtcatga 1800

ccccattagg tatggagagc tgggaggagg cattgtcact tcccaccagg atgcaggact 1860

tggggttgag gtgagtcatg gcctcttgct ggcaatgggg tgggaggagt acccccaagt 1920

cctctcactc ctccagcctg gaatgtgaag tgactcccca accccttggg ccatggcagg 1980

caccttttgg actgggctgc cactgcttgg gcagagtaaa aggtgccagg aggagcatgg 2040

gtgtggaagt cctgtcagcc aagaaataaa agtttacctc agagctgcaa aaaaaaaaaa 2100

aaaaaaaaaa a 2111

<210> 16
<211> 10542
<212> DNA
<213> Homo sapiens

<400> 16
ccgctggttc cgtaacaaca tcccgttggc ttccctcagg cggcgggacc agtgcagccg 60

ccgcctccca ggatcgtccg ccggtcaggg cccttgccct ccccggcaca ggccaccatg 120

gccaccaacc cacagccgca gccgcctcct ccggcgccgc cgcctccccc gccgcagccg 180

cagccgcagc caccgccgcc gccgccgggc cccggggctg gccccggcgc gggcggggcg 240

ggcggcgcgg gtgcgggcgc cggggacccg cagctcgtgg ccatgatcgt gaaccacctc 300

aagagccagg ggctcttcga ccagttccgc agagactgcc tggccgacgt ggacaccaag 360

cctgcgtatc agaatctgag acagcgtgtt gacaactttg ttgcaaatca cttggcaact 420

cacacatgga gtccgcatct caataagaac cagctaagaa acaacattag acaacaagtc 480

ctcaaatcag gaatgttgga gtctggtatt gaccgaatta tttctcaggt tgtggaccca 540

aagatcaacc acacattcag acctcaggta gagaaagctg tgcatgagtt tttggccacg 600

ctaaatcaca aagaggaagg aagtggcaac acagctcccg atgatgagaa accagacact 660

tcccttatta cacaaggtgt tcctactcct gggcccagtg ctaatgtagc caatgatgcc 720

atgtcgatat tggaaaccat aacttctctt aaccaagaag ccagtgctgc tagggcttca 780

acagaaacat caaatgccaa gaccagtgag agagcgtcaa aaaaacttcc atctcagcca 840

accactgata ctagtactga caaagaaaga acttcagagg acatggctga taaagaaaaa 900

tctacagctg actctggagg tgaaggactg gaaacagccc caaagtctga agagttcagc 960

gacctcccct gtccagtcga agaaattaaa aattacacaa aagagcataa taatttaatt 1020

ctgctaaata aggatgttca acaggaaagc agtgagcaaa aaaataaatc aacagacaaa 1080
ggtgaaaaga agccagacag caatgagaaa ggagaaagaa agaaagaaaa gaaggaaaag 1140
actgaaaaga aatttgatca ctcaaaaaag agtgaagata cacagaaagt taaagatgaa 1200
aaacaagcaa aggaaaaaga agtagagagt ttaaaacttc cttcagaaaa gaacagtaat 1260
aaagctaaaa ctgttgaagg gacaaaagaa gatttctctt tgatagattc tgatgtggat 1320
ggacttacag acatcacagt tagctctgtt cataccagtg acctttcatc ttttgaagaa 1380
gatactgagg aggaagttgt aacgtctgat agcatggaag aaggagagat tacgtcagat 1440
gatgaagaga agaacaaaca gaataaaaca aaaactcaaa ctagtgattc tagtgaagga 1500
aaaacaaaaa gtgtacggca tgcgtatgtc cacaaaccat atctttactc aaaatactat 1560
agtgattctg atgatgagct tactgtagaa caacgacgac agtccattgc caaagaaaaa 1620
gaagagaggc ttttaagaag gcaaatcaat agagaaaaac ttgaagaaaa acgaaaacag 1680
aaagcagaaa agacaaagtc ttcaaaaacc aagggtcaag gcaggagtag tgtggactta 1740
gaagaatcat caacaaagag tttggaacct aaagccgcca gaattaaaga agtccttaaa 1800
gaacggaaag ttttagaaaa aaaagtagcc ttaagcaaaa agagaaaaaa agattcaagg 1860
aatgttgaag agaactccaa aaagaaacag caatatgaag aagattccaa agaaaccctt 1920
aaaacaagtg agcattgtga aaaggaaaaa atttcttctt caaaggagct gaagcatgtt 1980
catgcaaaaa gtgaaccaag taaacctgcc cggagacttt cagagtcttt gcatgtagtt 2040
gacgaaaaca aaaatgaatc caaattagaa agagaacata aaagacggac atctacccct 2100
gttatcatgg agggggtaca ggaagagact gacacaagag atgtaaaaag gcaagtagaa 2160
cgctcagaaa tttgcaccga agagccccag aaacagaaaa gcacacttaa aaacgaaaag 2220
catctaaaga aagatgattc tgaaacacca catttgaaaa gcctacttaa gaaagaggtg 2280
aaatcctcca aggagaagcc tgaaagagag aaaactccat cggaagacaa attgtctgtg 2340
aaacataaat ataaaggtga ttgtatgcat aaaacaggtg atgagactga gcttcactct 2400
tctgagaaag gtttaaaagt agaggaaaat attcaaaagc aaagtcaaca aacaaagctt 2460
tcttcagatg ataaaaccga acgaaaaagt aaacatagga atgaaaggaa attatcagta 2520
ttaggcaaag atggaaagcc agtttctgaa tatattataa aaacagatga gaatgttcgt 2580
aaagaaaaca acaaaaaaga gagacgcttg tcagctgaaa aaactaaggc agagcacaaa 2640
tcaagaaggt caagtgattc taaaattcag aaagattctc tgggttccaa gcaacatggt 2700
atcacattac agagaagaag tgaaagttat tcggaagata agtgtgatat ggactccact 2760
aacatggata gtaatttgaa accagaagag gttgttcaca aggagaaacg acgaacaaag 2820
agcttgttag aagagaaact tgtgttgaag tctaaatcaa aaactcaagg caaacaggta 2880
aaagttgtag aaacagaatt acaagaaggt gccacaaaac aggcaaccac tccaaaacca 2940

gacaaggaga agaacacaga agaaaatgac tcagaaaaac agcgtaagtc taaagttgaa 3000
gacaaacctt ttgaagaaac tggtgttgaa cctgtattag agactgcttc ttcttcagca 3060
catagtacac agaaggattc tagtcataga gccaagttac cattagcaaa ggagaaatat 3120
aagagtgata aagactccac ttccaccagg cttgagagaa agttgtcaga tggccacaaa 3180
agcagaagct taaagcatag tagtaaagac ataaaaaaga aggacgaaaa taaatcagat 3240
gacaaggatg gtaaagaagt tgacagtagt catgaaaagg ccagaggtaa tagttcactc 3300
atggaaaaga aattaagtag aaggttgtgc gaaaatcgga gaggaagctt gtcacaagaa 3360
atggccaaag gagaagaaaa attagcagca aacactttga gcactcccag cggttcctcc 3420
cttcagagac caaaaaagag tggtgatatg acattgatcc ctgaacaaga gccaatggaa 3480
attgattctg agccaggtgt tgaaaatgtg tttgaagtat ctaaaaccca agacaaccgc 3540
aataataatt ctcagcaaga cattgactct gaaaatatga aacaaaaaac ttctgccact 3600
gttcaaaagg atgaattgag aacttgcaca gcagattcaa aagcaacagc tccagcttat 3660
aagccaggcc gtggaacagg agttaatagt aattctgaaa agcatgccga tcatagaagc 3720
accttgacca agaaaatgca tatacaaagt gctgtgtcca aaatgaaccc tggggagaaa 3780
gaacccattc atagaggaac tactgaagtg aatatagatt ctgaaactgt tcatagaatg 3840
ttactgagtg ccccatcaga aaatgatagg gtacagaaga atttgaaaaa cacagctgct 3900
gaagaacatg ttgctcaagg agatgccact cttgaacatt ccacaaattt agactcctca 3960
ccatccttaa gttcagtgac tgttgtgcct ctgagggaat cgtatgatcc agatgtaatt 4020
cctctgtttg acaaaagaac tgttttggaa ggtagcacag ccagcacctc ccctgcggat 4080
cactctgctc tccctaacca aagtctgact gttagggaat cagaagtcct taagacaagt 4140
gacagcaaag aaggtggtga aggtttcaca gtagatacac cagcaaaagc aagcatcact 4200
agcaaaagac acattccaga agctcaccag gctactttat tggatggtaa acaaggaaag 4260
gtaatcatgc ctcttggaag taagttaacg ggcgtgattg tggaaaatga gaatattacc 4320
aaagaaggtg gcttagtgga catggccaag aaagaaaatg acttaaatgc agagcccaat 4380
ttaaagcaga caattaaagc aacagtagag aatggcaaga aggatggcat tgctgttgat 4440
catgttgtag gcctgaatac agaaaaatat gctgaaactg tcaaacttaa gcataaaaga 4500
agcccaggta aagtaaaaga catatcaatt gatgttgaaa gaaggaatga aaacagtgag 4560
gtagacacca gtgctggaag tggctctgca ccctctgttt tacaccaaag gaacggacaa 4620
actgaggatg tggcaactgg gcctaggaga gcagaaaaga cttctgttgc cactagtact 4680
gaagggaagg acaaagatgt caccttaagt ccagtgaagg ctgggcctgc cacaaccact 4740
tcttcagaaa caagacaaag tgaggtggct ttgccttgca ccagcattga ggcagatgaa 4800

ggcctcataa taggaacaca ttccagaaat aatcctcttc atgttggtgc agaagccagt 4860
gaatgcactg tttttgctgc agctgaagaa ggtggggctg ttgtcacaga gggatttgct 4920
gaaagtgaaa ccttcctcac aagcactaag gaaggggaaa gtggggagtg tgctgtggct 4980
gaatctgagg acagagcagc agacctactg gctgtgcatg cagttaaaat cgaagccaat 5040
gtaaatagcg ttgtgacaga ggaaaaggat gatgctgtaa ccagtgcagg ctctgaagaa 5100
aaatgtgatg gttctttaag tagagactca gaaatagttg aaggaactat tacttttatt 5160
agtgaagttg aaagtgatgg agcagttaca agtgctggaa cagagataag agcaggatct 5220
ataagcagtg aagaggtgga tggctcccag ggaaatatga tgagaatggg tcccaaaaaa 5280
gaaacagagg gcactgtgac atgtacagga gcagaaggca gaagtgataa ctttgtgatc 5340
tgctcagtaa ctggagcagg gccccgggag gaacgcatgg ttacaggtgc aggtgttgtc 5400
ctgggagata atgatgcacc accaggaaca agtgccagcc aagaaggaga tggttctgtg 5460
aatgatggta cagaaggtga gagtgcagtc accagcacgg ggataacaga agatggagag 5520
gggccagcaa gttgcacagg ttcagaagat agcagcgaag gctttgctat aagttctgaa 5580
tcggaagaaa atggagagag tgcaatggac agcacagtgg ccaaagaagg cactaatgta 5640
ccattagttg ctgctggtcc ttgtgatgat gaaggcattg tgactagcac aggcgcaaaa 5700
gaggaagacg aggaaggga ggatgttgtg actagtactg gaagaggaaa tgaaattggg 5760
catgcttcaa cttgtacagg gttaggagaa gaaagtgaag gggtcttgat ttgtgaaagt 5820
gcagaagggg acagtcagat tggtactgtg gtagagcatg tggaagctga ggctggagct 5880
gccatcatga atgcaaatga aaataatgtt gacagcatga gtggcacaga gaaaggaagt 5940
aaagacacag atatctgctc cagtgcaaaa gggattgtag aaagcagtgt gaccagtgca 6000
gtctcaggaa aggatgaagt gacaccagtt ccaggaggtt gtgagggtcc tatgactagt 6060
gctgcatctg atcaaagtga cagtcagctc gaaaaagttg aagataccac tatttccact 6120
ggcctggtcg gggtagtta cgatgttctt gtatctggtg aagtcccaga atgtgaagtt 6180
gctcacacat caccaagtga aaaagaagat gaggacatca tcacctctgt agaaaatgaa 6240
gagtgtgatg gtctcatggc aactacagcc agtggtgata ttaccaacca gaatagctta 6300
gcaggggggta aaaatcaagg caaagttttg attatttcca ccagtaccac aaatgattac 6360
acccctcagg taagcgcaat tacagatgtg gaaggaggtc tctcagatgc tctgagaact 6420
gaagaaaata tggaaggtac cagagtaacc acagaagaat ttgaggcccc catgcccagt 6480
gcagtctcag gagatgacag ccaactcact gccagcagaa gtgaagagaa agatgagtgt 6540
gccatgattt ccacaagcat aggggaagaa ttcgaattgc ctatctccag tgcaacaacc 6600
atcaagtgtg ctgaaagtct tcagccggtt gctgcagcag tggaagaaag ggctacaggt 6660
ccagtcttga taagcaccgc cgactttgag gggcctatgc ccagtgcgcc cccagaagct 6720

gaaagtcctc ttgcctcaac cagcaaggag gagaaggatg aatgtgctct catttccact 6780
agcatagcag aagaatgtga ggcttctgtt tccggtgtag ttgttgaaag tgaaaatgag 6840
cgagctggca cagtcatgga agaaaaagac gggagtggca tcatctctac gagctcggtg 6900
gaagactgtg agggcccagt gtccagtgct gtccctcaag aggaaggcga cccctcagtc 6960
acaccagcgg aagagatggg tgacaccgcc atgatttcca caagcacctc tgaagggtgt 7020
gaagcagtca tgattggtgc tgtcctccag gatgaagatc ggctcaccat cacaagagta 7080
gaagacttga gcgatgctgc catcatctcc accagcacag cagaatgtat gccaatttcc 7140
gccagcattg acagacatga agagaatcag ctgactgcag acaacccaga agggaacggt 7200
gacctgtcag ccacagaagt gagcaagcac aaggtcccca tgcccagcct aattgctgag 7260
aataactgtc ggtgtcctgg gccagtcagg ggaggcaaag aaccgggtcc cgtgttggca 7320
gtgagcaccg aggaggggca caacgggcca tcagtccaca agccctctgc agggcaaggc 7380
catccaagtg ctgtttgtgc ggaaaaagaa gagaagcatg gcaaggagtg ccccgaaata 7440
ggaccatttg caggaagagg acagaaagag agcactttac acctcataaa tgcagaagag 7500
aagaatgtat tgttgaactc ccttcagaaa gaagataaga gcccagagac agggacagca 7560
gggggcagta gcacagcaag ttattcagca ggaaggggct tagaggggaa tgctaactca 7620
cctgcccacc tgagaggacc agaacagacg tctgggcaga cggctaagga tccctctgtc 7680
agcattcgct atttggcagc agtaaacacc ggtgctataa aagctgatga catgccacct 7740
gttcaaggga ccgtggctga gcattccttt cttcctgctg agcagcaggg gtctgaagac 7800
aacttgaaaa ccagtaccac caaatgtatt actggccaag aatcaaaaat tgctccttcc 7860
cacacaatga tccctccagc tacttacagt gtagctctgt tggctcctaa atgtgagcag 7920
gacttgacta taaagaatga ttatagtggc aaatggactg atcaagcatc tgctgagaaa 7980
acaggagatg ataacagcac aaggaaatca ttccctgagg aaggagacat aatggttact 8040
gtgtcttctg aagaaaatgt gtgtgacata ggcaatgaag agtctccatt gaatgttttg 8100
ggaggattga aactgaaagc caacttgaaa atggaggctt atgtgccttc agaggaagag 8160
aaaaatggtg aaattctggc accaccagaa agtctgtgtg ggggaaagcc aagtggaata 8220
gctgaactcc agagggagcc tttgttggtg aatgaatcac taaatgttga aaattcaggc 8280
ttcagaacaa atgaagaaat tcatagcgaa tcttataaca aaggagagat atccagtggt 8340
agaaaagaca acgcagaagc cataagcggt cacagtgttg aagcagatcc taaagaggtt 8400
gaagaggaag aaaggcatat gcctaaaaga aaaagaaagc agcattatct ctcttcagaa 8460
gatgaaccag atgataatcc agatgtcctg gattccagaa tagaaacagc acaaaggcag 8520
tgtcctgaaa cggagccaca tgacacaaag gaagagaact ccagagattt ggaagaatta 8580

cctaaaacca gttctgagac aaatagcact acctcaaggg tcatggaaga aaaagatgaa 8640
tatagcagca gtgaaactac tggtgaaaag ccagagcaga acgatgatga caccataaaa 8700
tctcaggagg aagatcagcc aataattatt aaaaggaaaa gaggaagacc tcgcaaatac 8760
cctgtagaaa caacgttaaa aatgaaagac gactccaaaa cagatactgg cattgtcact 8820
gtagaacaat ctccatctag cagcaaactg aaagtaatgc aaacagatga atccaataaa 8880
gaaacagcta acctacaaga aagaagtata agcaatgatg atggtgaaga aaaaatagta 8940
acaagtgtgc gtcggagagg aagaaaaccc aaacgttctc tcactgtatc agatgatgct 9000
gaatcctcag agccagaaag aaaacgccag aaatcagttt ctgatccagt ggaggacaag 9060
aaagagcagg agtctgatga ggaagaggaa gaagaggaag aggacgagcc ttcaggagcc 9120
accacaagat ccaccaccag atcagaggct cagagatcaa agacacagct ctccccttct 9180
atcaagcgca agagagaagt cagccctcct ggggcccgaa caagaggcca gcaaagggtg 9240
gaggaagccc ctgtgaaaaa agcgaagcga taatcctgac cactgctgcc ctaggcttat 9300
ggaggaacac ggtggagagg aaagagacat gccttggtgg ccataggctt ctctttaacc 9360
aggaaaaaga tatgcatgtg ctgtaagtcc ctaggtgcaa gctttttctt gttatgtttt 9420
aaacagcttt ataaactatt gttcatagaa gatattatgt acatttattt cagataaagg 9480
acaataagtt tactttgtat ctgaactcaa aacaaagtag ttgtatattt taacattcaa 9540
aattgggatt tcccaatgtg acacatcatg aatgcaaacc cctccagccc atcagacgcc 9600
aggctgccta ctggtaatct gtgtatagta tataaacatg taaaaatagg ttgtatttta 9660
ctctatgtat gatgctaatc aatgaacact ttatttattt tacagagaaa acttatctgt 9720
gaactttact atatatctgt ttattttact ttattttttt tttaaataaa aagggtttta 9780
aatgctatgc agtcattagt agaaaatttt ttaggactct gcctgctctg taactatctt 9840
aatatgatct ggcagaaact cgcatgtatc caagtaaagt agtttagcta aagaaaggtt 9900
cttcattgct tttctgttca cagttgtggc tctgtttttt aagaatgtaa cttgttttta 9960
gattatactt gcatctgtga ctttactacc agccacgttg acacaaaaca ggttctggtt 10020
caggtaaagt tgcgtcagtc acctgcagca gaaatccctc ttcattcctc ttctctgtgt 10080
tcattcctct tctgtgctgt tctgaagctt ctaccaatac tctttccata ttgtcttttt 10140
cagtgaagag aaatgcattc aagattaggt ccctcctgtc tatccagttt caggatttta 10200
tgttgtttta tacacagtta tttcagtata gaaactggct ttattgccaa gtgttttttt 10260
aaacatgttt taactctcat atgagcaaac tgtccaactt cagtttttca taagattaaa 10320
cttcttacga tcaaatttgt ctcttgcaat gatgtgatga gttgccaaat aattgagatt 10380
attttaaaat gttttgttca tattcttgtt ttataattaa aatttacatt cagtgtgtat 10440
gggttttttt ttttattttg actcttaatg taaggtggat atttctgtca ttttacatgg 10500

tttcttactg agattttata tataaattat aaaatgttta cc 10542

<210> 17
<211> 5246
<212> DNA
<213> Homo sapiens

<400> 17
agttccgggt gcgcggcccc ggatgttggc tgctcttgct gccgccgccg ctctcggggc 60

tgctcggggc ctcgaccgct cgcagtaggc accgttggga cctttcccgg gggggcggag 120

acgcacggag cggccggggg ctcggccggg ccgtcccggc ctgcgcagcc tgagattctc 180

tttgtggcca gatgggtttg tatggacagg cttgtccatc tgtaacttca ttaaggatga 240

catctgaact ggagagcagc ctaacgtcta tggactggtt accacagctc accatgagag 300

cagccatcca aaaatctgat gctacacaaa atgcacatgg aacaggaatt tctaagaaga 360

atgcactcct tgacccaaat acaactctgg accaggaaga agtccaacag cacaaagatg 420

ggaaacctcc atacagttat gccagcctca ttacatttgc aattaatagc tcacccaaaa 480

agaaaatgac tttaagtgaa atttatcagt ggatttgtga taacttccca tattatagag 540

aggctggcag tggttggaag aattccatac gacataatct gtcattgaac aaatgtttcc 600

ttaaagtgcc tcgatctaag gatgaccctg gaaaggggtc ctactgggca atagacacca 660

atccgaagga agatgtgctg cctactcggc caaagaagag ggcacgatct gtagaacggg 720

cctcaactcc atatagcata gattcagatt ctttgggaat ggagtgtatt atttcgggaa 780

gtgcctctcc aactctggca atcaacactg tgactaacaa agtaacattg tataacactg 840

atcaggatgg tagtgatagc ccacgcagta gccttaacaa cagtctctca gaccagagtt 900

tggcatctgt taatttgaac agtgttggaa gtgtgcatag ttatacaccg gtgacaagcc 960

atccagaatc agtctctcaa tcattaactc ctcagcagca accacagtac aaccttccag 1020

aaagagacaa gcaactactt ttctcagaat ataattttga agatcttagt gcctcatttc 1080

ggagccttta taagtcagtt tttgagcagt cacttagtca acaaggtttg atgaacatcc 1140

cttctgaatc ttcccagcag tcccacactt catgtaccta tcagcactct cccagcagta 1200

cagtgagcac tcacccacac agcaaccaaa gcagcctgtc caacagtcat ggcagtggcc 1260

tcaacaccac aggcagtaat tcggttgcac aggtctcact gtctcacccc cagatgcaca 1320

cacagccatc tccacatcct ccccatcgac cgcatggttt accgcagcat ccgcagcgtt 1380

ccccacaccc agcaccacac ccacagcaac acagccagct ccagtcccct caccccccagc 1440

atccctctcc acatcaacac atacagcacc atccgaacca tcagcatcag acgttaacac 1500

atcaggcacc cccacccccca caacaggtat cctgtaattc tggtgtttca aatgattggt 1560

atgcgacact tgatatgcta aaagaaagct gtcgaattgc cagcagtgtt aattggtcag 1620

atgtagacct ttcacagttt caaggtctga tggagagtat gagacaggca gatctcaaga 1680
actggtcttt agaccaggtt cagtttgccg atctttgttc ttctcttaat cagttcttta 1740
cacaaactgg ccttatacat tcacagagta atgttcaaca aaatgtttgt catggtgcca 1800
tgcatccaac aaaaccttcc caacacattg gaacaggaaa tttgtacata gattctaggc 1860
aaaatctccc tccttcagtg atgccacccc ctggttatcc tcatatccca caggcactca 1920
gcactccagg aacaacgatg gcaggccatc acagagccat gaaccagcag cacatgatgc 1980
cttcccaagc cttccagatg cggcgttccc tgcctccaga tgacatccag gatgactttg 2040
attgggattc aattgtgtag ggcttgtttc tgcaagacac cagaccctaa cgttaccttt 2100
ctgtgcagtg aagggaaagg tttaagagaa tccagttgag aaaacaaact tgctaatcac 2160
tttaccaatg ttatcaaaat tacttttgaa gacaatcaga aggattttag ctggataact 2220
tactgctttt atctgaccca agcaagtact acatgtttgt ctccctgcca gctgccctat 2280
gtagctccta actgttgtgt gatttggacg gctttttgca tatttgtgtc agtttgatgt 2340
taaccacaag tgccagactg atttttcaga cggagcctat tttgctgcaa gcagtttata 2400
taaagataca tatgtgtaaa tatatgtaca aaaattactg aaaggcttca gtttttttcta 2460
attggattat tatgtcttga aagttattgt cagtttttat tccttgttag gctattttct 2520
gcaggatgct tttaactgat gtaggaaact gaaaggaaat agatttttttc caaaacccag 2580
ttccccttat ttaatctttt ttagaaatgt gggtaatgaa ttctatctaa taagtcaagg 2640
aaaccagaat ttgacacact ccaacaatcc aaaggggcat gttgctcctg agcagcatga 2700
agaactgacc aaattggttt tgatgcttgg gggatcatag agtatttatg tctgcttttc 2760
taaatctgca ttataatagc tctaaaattt gttgattggt aagaaattgg gcattgcttg 2820
gctctttaaa cacatcagtg cttccacatt cacctatgta tttattattc aaaagtgtca 2880
ttttaatatt tattgctacc ttctgtgaat gcttagctcc tgtcgggttc attaaggaga 2940
aatgtgtctg aaagcacaga actattatta tttttttctt tttttgagat ggagtcttgc 3000
cctgttgccc aggctggagt gcagtggcac gatctcggct cactgcaacc tccgcctccc 3060
gggttcaagc aattctcctg cctcagcctc ccgagtagct gggagtgcag gtgcacaccg 3120
ccatgcctgg ctgatttttt gtattttagt agagatgggg tttcaccatg ttgcctgggc 3180
tggttgcaaa ctcttgagct caggcagtct gcctgcctca gcctcctaga gtgctgggat 3240
tacagacatg agccactgcg cctggcccag cgttactttt cttgataaga atttacagat 3300
aagacactca gagagacatt gtgtcattct ctatcatcac tccttttacc atgtgaatag 3360
atttctctgt cttggcattc ttgctgattc agacttatta gttaaatcgg atttttctgg 3420
aatttgaatc agatttcatt tgggcaacaa cagcagggca gggtttctaa agcaggtttc 3480

cccactcatc taggttgtct tgaaaggagg atagagccac ttacagtttt atttgcttca 3540
gtgtttaatg tagatattat gtggcctgct gtttctgttg tcttgtatgt ctgtgtatgc 3600
atgacatttg gtccctttct ttgaaatgca gcccctctta cgggtttttt agtggtggtg 3660
gtttgttttg tttcatatca tatcacagtt tgcatggact gattatctta gttttatgat 3720
aaaactagaa gaaatgaggg ttttttttta atgaatagat tttgaattga ttctttagac 3780
cccccaaaaa gtgtcagttc taatggggaa atatattcca tcaagtcaaa gagtaataac 3840
tgctcactag ttgcttttta gcatctctgg tcttatcagc catgctaaat cactttaatt 3900
agccttagtg attctatggt tgagtaatct ctacttgaac taaacaaaca tcttttgttt 3960
ctgtgtgtgt gtgctgtgtg tgagagtgtg cgcgcgcgtg tgtgtgtgtg tgttttaatg 4020
gagtgttgcc ttgaatgaat cactgggaag ccagccatgg taagggctgg tgaggttggg 4080
gagaaaggaa gagctttatg tttctctgtt gtttggaccc tacttggcat gaaaaaggaa 4140
gctcagttcc agccccttgg atcaacgaaa atcagaggat tctggaaagg cagccaactt 4200
gcgcctctta gaaggatcag aggcaagatg agatggcagc ctgcagagta aatgcttgaa 4260
aaaagagggg tgattttcaa tggtttgctt aagtcactgt tttctagaca ccaaaatagc 4320
tgttttgaaa ctgttttaat tgcttgggta gcaatgtgca ctttaaacaa tttggatatt 4380
ggaatgcagt ctcatactga gtgatttgag tagaaccact gatgatgatt ttataaattg 4440
tgtgaagcga atttcccatt tggcaatcat ttactgattt gcagtgatga atatttttat 4500
gagaatttaa acttagcaag aatggccatg gaggcaaagc cttcacccag acccatccca 4560
ctctcctgtg atccaggtgg tccaggagcc caggacaggc cttttctgtg ggccctggcc 4620
agacagggtt acctggtgag gtgcagagag tccctctagt ggccattttg tatggtagtt 4680
gctaatgcag aacaagttct gtcttgggct taaattgact gaagacttta gggggaaaga 4740
atagtaaatg catgtaaaca aatggggaca ctctgttcag gagaataatc cgactggcat 4800
ttgtggcagt ttttgaaatg taaatgtatt catgtgtgtt cttgtaaata cgtgtcgctc 4860
agatgtcctt tgaagtggga gggaatcaat ccggggataa tttcaaatgg aatagagtat 4920
tttgatattg ttcattcaga gggtgatgtg tacatatcta tattgtatat atgtgatgaa 4980
aatgcattgg ctttttgtgc agatacaacc tgctctctgt actgctgttg gacagtcagt 5040
gttttaatgt ttctacagtt ttgctattgc acgatttcat attttgcctc tatgatgaac 5100
ggcaaccatt atttgtaact gtttagtgct gtaaagaaat attccaagtg tcattaggat 5160
tgttgctgcc agaactgata tgcatgaatg gcacttaaaa taaatatatt atgttaactc 5220
tatttacaac aaaaaaaaaa aaaaaa 5246

<210> 18
<211> 8980

<212> DNA
<213> Homo sapiens

<400> 18
ggaggttgtg gggccgccgc cgcggagcac cgtccccgcc gccgcccgag cccgagcccg 60

agcccgcgca cccgcccgcg ccgccgccgc cgccgcccga acagcctccc agcctgggcc 120

cccggcggcg ccgtggccgc gtcccggctg tcgccgcccg agcccgagcc cgcgcgccgg 180

cgggtggcgg cgcaggctga ggagatgcgg cgcggagcgc cggagcaggg ctagagccgg 240

ccgccgccgc ccgccgcggt aagcgcagcc ccggcccggc gcccgcgggc cattgtccgc 300

cgcccgcccc gcgccccgcg cagcctgcag gccttggagc ccgcggcagg tggacgccgc 360

cggtccacac ccgcccgcg cgcggccgtg ggaggcgggg gccagcgctg gccgcgcgcc 420

gtgggacccg ccggtcccca gggccgcccg gccccttctg gacctttcca cccgcgccgc 480

gaggcggctt cgcccgccgg ggcgggggcg cgggggtggg cacggcaggc agcggcgccg 540

tctcccggtg cggggcccgc gcccccgag caggttcatc tgcagaagcc agcggacgcc 600

tctgttcaac ttgtgggtta cctggctcat gagaccttgc cggcgaggct cggcgcttga 660

acgtctgtga cccagccctc accgtcccgg tacttgtatg tgttggtggg agtttggagc 720

tcgttggagc tatcgtttcc gtggaaattt tgagccattt cgaatcactt aaaggagtgg 780

acattgctag caatgagctc ccaaagccat ccagatggac tttctggccg agaccagcca 840

gtggagctgc tgaatcctgc ccgcgtgaac cacatgccca gcacggtgga tgtggccacg 900

gcgctgcctc tgcaagtggc cccctcggca gtgcccatgg acctgcgcct ggaccaccag 960

ttctcactgc ctgtggcaga gccggccctg cgggagcagc agctgcagca ggagctcctg 1020

gcgctcaagc agaagcagca gatccagagg cagatcctca tcgctgagtt ccagaggcag 1080

cacgagcagc tctcccggca gcacgaggcg cagctccacg agcacatcaa gcaacaacag 1140

gagatgctgg ccatgaagca ccagcaggag ctgctggaac accagcggaa gctggagagg 1200

caccgccagg agcaggagct ggagaagcag caccgggagc agaagctgca gcagctcaag 1260

aacaaggaga agggcaaaga gagtgccgtg gccagcacag aagtgaagat gaagttacaa 1320

gaatttgtcc tcaataaaaa gaaggcgctg gcccaccgga atctgaacca ctgcatttcc 1380

agcgaccctc gctactggta cgggaaaacg cagcacagtt ccccttgacca gagttctcca 1440

ccccagagcg gagtgtcgac ctcctataac cacccggtcc tgggaatgta cgacgccaaa 1500

gatgacttcc ctcttaggaa aacagcttct gaaccgaatc tgaaattacg gtccaggcta 1560

aagcagaaag tggccgaaag acggagcagc cccctgttac gcaggaaaga cgggccagtg 1620

gtcactgctc taaaaaagcg tccgttggat gtcacagact ccgcgtgcag cagcgcccca 1680

ggctccggac ccagctcacc caacaacagc tccgggagcg tcagcgcgga gaacggtatc 1740

gcgcccgccg tccccagcat cccggcggag acgagtttgg cgcacagact tgtggcacga 1800

gaaggctcgg ccgctccact tcccctctac acatcgccat ccttgcccaa catcacgctg 1860
ggcctgcctg ccaccggccc ctctgcgggc acggcgggcc agcaggacgc cgagagactc 1920
acccttcccg ccctccagca gaggctctcc cttttccccg gcacccacct cactccctac 1980
ctgagcacct cgcccttgga gcgggacgga ggggcagcgc acagccctct tctgcagcac 2040
atggtcttac tggagcagcc gccggcacaa gcacccctcg tcacaggcct gggagcactg 2100
cccctccacg cacagtcctt ggttggtgca gaccgggtgt ccccctccat ccacaagctg 2160
cggcagcacc gcccactggg gcggacccag tcggccccgc tgccccagaa cgcccaggct 2220
ctgcagcacc tggtcatcca gcagcagcat cagcagtttc tggagaaaca caagcagcag 2280
ttccagcagc agcaactgca gatgaacaag atcatcccca agccaagcga gccagcccgg 2340
cagccggaga gccacccgga ggagacggag gaggagctcc gtgagcacca ggctctgctg 2400
gacgagccct acctggaccg gctgccgggg cagaaggagg cgcacgcaca ggccggcgtg 2460
caggtgaagc aggagcccat tgagagcgat gaggaagagg cagagccccc acgggaggtg 2520
gagccgggcc agcgccagcc cagtgagcag gagctgctct tcagacagca agccctcctg 2580
ctggagcagc agcggatcca ccagctgagg aactaccagg cgtccatgga ggccgccggc 2640
atccccgtgt ccttcggcgg ccacaggcct ctgtcccggg cgcagtcctc acccgcgtct 2700
gccaccttcc ccgtgtctgt gcaggagccc cccaccaagc cgaggttcac gacaggcctc 2760
gtgtatgaca cgctgatgct gaagcaccag tgcacctgcg ggagtagcag cagccacccc 2820
gagcacgccg ggaggatcca gagcatctgg tcccgcctgc aggagacggg cctccggggc 2880
aaatgcgagt gcatccgcgg acgcaaggcc accctggagg agctacagac ggtgcactcg 2940
gaagcccaca ccctcctgta tggcacgaac cccctcaacc ggcagaaact ggacagtaag 3000
aaacttctag gctcgctcgc ctccgtgttc gtccggctcc cttgcggtgg tgttggggtg 3060
gacagtgaca ccatatggaa cgaggtgcac tcggcggggg cagcccgcct ggctgtgggc 3120
tgcgtggtag agctggtctt caaggtggcc acaggggagc tgaagaatgg ctttgctgtg 3180
gtccgccccc ctggacacca tgcggaggag agcacgccca tgggcttttg ctacttcaac 3240
tccgtggccg tggcagccaa gcttctgcag cagaggttga gcgtgagcaa gatcctcatc 3300
gtggactggg acgtgcacca tggaaacggg acccagcagg ctttctacag cgaccccagc 3360
gtcctgtaca tgtccctcca ccgctacgac gatgggaact tcttcccagg cagcggggct 3420
cctgatgagg tgggcacagg gcccggcgtg ggtttcaacg tcaacatggc tttcaccggc 3480
ggcctggacc cccccatggg agacgctgag tacttggcgg ccttcagaac ggtggtcatg 3540
ccgatcgcca gcgagtttgc cccggatgtg gtgctggtgt catcaggctt cgatgccgtg 3600
gagggccacc ccacccctct tgggggctac aacctctccg ccagatgctt cgggtacctg 3660

acgaagcagc tgatgggcct ggctggcggc cggattgtcc tggccctcga gggaggccac 3720
gacctgaccg ccatttgcga cgcctcggaa gcatgtgttt ctgccttgct gggaaacgag 3780
cttgatcctc tcccagaaaa ggttttacag caaagaccca atgcaaacgc tgtccgttcc 3840
atggagaaag tcatggagat ccacagcaag tactggcgct gcctgcagcg cacaacctcc 3900
acagcggggc gttctctgat cgaggctcag acttgcgaga acgaagaagc cgagacggtc 3960
accgccatgg cctcgctgtc cgtgggcgtg aagcccgccg aaaagagacc agatgaggag 4020
cccatggaag aggagccgcc cctgtagcac tccctcgaag ctgctgttct cttgtctgtc 4080
tgtctctgtc ttgaagctca gccaagaaac tttcccgtgt cacgcctgcg tcccaccgtg 4140
gggctctctt ggagcaccca gggacaccca gcgtgcaaca gccacgggaa gcctttctgc 4200
cgcccaggcc cacaggtctc gagacgcaca tgcacgcctg ggcgtggcag cctcacaggg 4260
aacacgggac agacgccggc gacgcgcaga cacacggaca cgcggaagcc aagcacactc 4320
tggcgggtcc cgcaagggac gccgtggaag aaaggagcct gtggcaacag gcggccgagc 4380
tgccgaattc agttgacacg aggcacagaa aacaaatatc aaagatctaa taatacaaaa 4440
caaacttgat taaaactggt gcttaaagtt tattacccac aactccacag tctctgtgta 4500
aaccactcga ctcatcttgt agcttatttt tttttaaaga ggacgttttc tacggctgtg 4560
gcccgcctct gtgaaccata gcggtgtgcg gcgggggtc tgcacccggg tgggggacag 4620
agggaccttt aaagaaaaca aaactggaca gaaacaggaa tgtgagctgg gggagctggc 4680
ttgagtttct caaaagccat cggaagatgc gagtttgtgc cttttttttt attgctctgg 4740
tggatttttg tggctgggtt ttctgaagtc tgaggaacaa tgccttaaga aaaaacaaac 4800
agcaggaatc ggtgggacag tttcctgtgg ccagccgagc ctggcagtgc tggcaccgcg 4860
agctggcctg acgcctcaag cacgggcacc agccgtcatc tccggggcca ggggctgcag 4920
cccggcggtc cctgttttgc tttattgctg tttaagaaaa atggaggtag ttccaaaaaa 4980
gtggcaaatc ccgttggagg ttttgaagtc caacaaattt taaacgaatc caaagtgttc 5040
tcacacgtca catacgattg agcatctcca tctggtcgtg aagcatgtgg taggcacact 5100
tgcagtgtta cgatcggaat gctttttatt aaaagcaagt agcatgaagt attgcttaaa 5160
ttttaggtat aaataaatat atatatgtat aatatatatt ccaatgtatt ccaagctaag 5220
aaacttactt gattcttatg aaatcttgat aaaatattta taatgcattt atagaaaaag 5280
tatatatata tatataaaat gaatgcagat tgcgaaggtc cctgcaaatg gatggcttgt 5340
gaatttgctc tcaaggtgct tatggaaagg gatcctgatt gattgaaatt catgttttct 5400
caagctccag attggctaga tttcagatcg ccaacacatt cgccactggg caactaccct 5460
acaagtttgt actttcattt taattatttt ctaacagaac cgctcccgtc tccaagcctt 5520
catgcacata tgtacctaat gagtttttat agcaaagaat ataaatttgc tgttgatttt 5580

tgtatgaatt ttttcacaaa aagatcctga ataagcattg ttttatgaat tttacatttt 5640

tcctcaccat ttagcaattt tctgaatggt aataatgtct aaatcttttt cctttctgaa 5700

ttcttgcttg tacatttttt tttacctttc aaaggttttt aattattttt gtttttattt 5760

ttgtacgatg agttttctgc agcgtacaga attgttgctg tcagattcta ttttcagaaa 5820

gtgagaggag ggaccgtagg tcttttcgga gtgacaccaa cgattgtgtc tttcctggtc 5880

tgtcctagga gctgtataaa gaagcccagg ggctcttttt aactttcaac actagtagta 5940

ttacgagggg tggtgtgttt ttcccctccg tggcaagggc agggagggtt gcttaggatg 6000

cccggccacc ctgggaggct tgccagatgc cgggggcagt cagcattaat gaaactcatg 6060

tttaaacttc tctgaccaca tcgtcaggat agaattctaa cttgagtttt ccaaagacct 6120

tttgagcatg tcagcaatgc atggggcaca cgtggggctc tttacccact tgggtttttc 6180

cactgcagcc acgtggccag ccctggattt tggagcctgt ggctgcaagg aacccaggga 6240

cccttgttgc ctggtgaacc tgcagggagg gtatgattgc ctgaccagga cagccagtct 6300

ttactctttt tctcttcaac agtaactgac agtcacgttt tactggtaac ttattttcca 6360

gcacatgaag ccaccagttt cattccaaag tgtatattgg gttcagactt gggggcagaa 6420

gttcagacac accgtgctca ggagggaccc agagccgagt ttcggagttt ggtaaagttt 6480

acagggtagc ttctgaaatt aactcaaact tttgaccaaa tgagtgcaga ttcttggatt 6540

cacttggtca ctgggctgct gatggtcagc tctgagacag tggtttgaga gcaggcagaa 6600

cggtcttggg acttgtttga ctttcccctc cctggtggcc actctttgct ctgaagccca 6660

gattggcaag aggagctggt ccattcccca ttcatggcac agagcagtgg cagggcccag 6720

ctagcaggct cttctggcct ccttggcctc attctctgca tagccctctg gggatcctgc 6780

cacctgccct cttaccccgc cgtggcttat ggggaggaat gcatcatctc actttttttt 6840

tttaagcaga tgatgggata acatggactg ctcagtggcc aggttatcag tgggggggact 6900

taattctaat ctcattcaaa tggagacgcc ctctgcaaag gcctggcagg gggaggcacg 6960

tttcatctgt cagctcactc cagcttcaca aatgtgctga gagcattact gtgtagcctt 7020

ttctttgaag acacactcgg ctcttctcca cagcaagcgt ccagggcaga tggcagagga 7080

tctgcctcgg cgtctgcagg cgggaccacg tcagggaggg ttccttcatg tgttctccct 7140

gtgggtcctt ggacctttag ccttttttctt cctttgcaaa ggccttgggg gcactggctg 7200

ggagtcagca agcgagcact ttatatccct ttgagggaaa ccctgatgac gccactgggc 7260

ctcttggcgt ctgccctgcc ctcgcggctt cccgccgtgc cgcagcgtgc ccacgtgccc 7320

acgccccacc agcaggcggc tgtcccggag gccgtggccc gctgggactg gccgcccctc 7380

cccagcgtcc cagggctctg gttctggagg gccactttgt caaggtgttt cagtttttct 7440

ttacttcttt tgaaaatctg tttgcaaggg gaaggaccat ttcgtaatgg tctgacacaa 7500

aagcaagttt gatttttgca gcactagcaa tggactttgt tgtttttctt tttgatcaga 7560

acattccttc tttactggtc acagccacgt gctcattcca ttcttctttt tgtagacttt 7620

gggcccacgt gttttatggg cattgataca tatataaata tatagatata aatatatatg 7680

aatatatttt tttaagtttc ctacacctgg aggttgcatg gactgtacga ccggcatgac 7740

tttatattgt atacagattt tgcacgccaa actcggcagc tttggggaag aagaaaaatg 7800

cctttctgtt cccctctcat gacatttgca gatacaaaag atggaaattt ttctgtaaaa 7860

caaaaccttg aaggagagga gggcggggaa gtttgcgtct tattgaactt attcttaaga 7920

aattgtactt tttattgtaa gaaaaataaa aaggactact taaacatttg tcatattaag 7980

aaaaaaagtt tatctagcac ttgtgacata ccaataatag agtttattgt atttatgtgg 8040

aaacagtgtt ttagggaaac tactcagaat tcacagtgaa ctgcctgtct ctctcgagtt 8100

gatttggagg aattttgttt tgttttgttt tgtttgtttc cttttatctc cttccacggg 8160

ccaggcgagc gccgcccgcc ctcactggcc ttgtgacggt ttattctgat tgagaactgg 8220

gcggactcga aagagtcccc ttttccgcac agctgtgttg actttttaat tacttttagg 8280

tgatgtatgg ctaagatttc actttaagca gtcgtgaact gtgcgagcac tgtggtttac 8340

aattatactt tgcatcgaaa ggaaaccatt tcttcattgt aacgaagctg agcgtgttct 8400

tagctcggcc tcactttgtc tctggcattg attaaaagtc tgctattgaa agaaaaagaa 8460

agcgaacagt ttttgtttgt tttttttgcc gtgtgtgctc catagtggag gcgctatttt 8520

ccaattgatg agaatgacaa acatatataa tctatctatc tatctatcta tctatctatc 8580

tatacagggg gcttgaacct tactcaccca agagcttctt acggaatgtg gtagaaaacc 8640

aagttgtaac gacactgtaa cctacctgat gcctgttcgc gcccgccgtg agctgcgcac 8700

tggccgtggc caccattcac ctctgtaatt taatccgttt ctcttggatt gtctggacgt 8760

gcccgatggt tctttctttt gctcagtgga gttggaggtt ttgtgtttgg ttttctcatt 8820

cttgtcttgt tttgtgtggg tggattttca ccgaccaatg atatcctctt ctgacggtca 8880

ccttctttcc acttcactgg agtccagtat tctgtaccac atcacgcaac gtgttatctt 8940

gtggtgtaaa taaagactgc gttacttgcc ctcccaaaaa 8980

<210> 19
<211> 5327
<212> DNA
<213> Homo sapiens

<400> 19
cgaatgttgt tgttggtggc ggcggcgagc ggagccggag gagccgccgc aaagatggag 60

gagccgtcga ggaggtgctg ccgccgctgc cgccgccgct gctgccgccg ccgcccgcga 120

agccggagct cgagccgcag cggggatgcc gttctgagtg cctgactgcc tcgccccgaa 180
ggatggcctc ggatgggcat tagaggcacg gcggccccgg gctcccgtcc cgtccgtctg 240
tctgttatcg tctgtctctc ttgacatcac cgcagctcca ccccctcccg tcccagcccc 300
caacgccagc ttcctgcagg cccagagccg gcatgaactc tcccaacgag tcggcagatg 360
ggatgtcagg tcgggaacca tccttggaaa tcctgccgcg gacttctctg cacagcatcc 420
ctgtgacagt ggaggtgaag ccggtgctgc caagagccat gcccagttcc atgggggtg 480
ggggtggagg cagccccagc cctgtggagc tacggggggc tctggtgggc tctgtggacc 540
ccacactgcg ggagcagcaa ctgcagcagg agctcctggc gctcaagcag cagcagcagc 600
tgcagaagca gctcctgttc gctgagttcc agaaacagca tgaccacctg acaaggcagc 660
atgaggtcca gctgcagaag cacctcaagc agcagcagga gatgctggca gccaagcagc 720
agcaggagat gctggcagcc aagcggcagc aggagctgga gcagcagcgg cagcgggagc 780
agcagcggca ggaagagctg gagaagcagc ggctggagca gcagctgctc atcctgcgga 840
acaaggagaa gagcaaagag agtgccattg ccagcactga ggtaaagctg aggctccagg 900
aattcctctt gtcgaagtca aaggagccca caccaggcgg cctcaaccat tccctcccac 960
agcaccccaa atgctgggga gcccaccatg cttctttgga ccagagttcc cctccccaga 1020
gcggcccccc tgggacgcct ccctcctaca aactgccttt gcctgggccc tacgacagtc 1080
gagacgactt ccccctccgc aaaacagcct ctgaacccaa cttgaaagtg cgttcaaggc 1140
taaaacagaa ggtggctgag cggagaagca gtcccctcct gcgtcgcaag gatgggactg 1200
ttattagcac ctttaagaag agagctgttg agatcacagg tgccgggcct ggggcgtcgt 1260
ccgtgtgtaa cagcgcaccc ggctccggcc ccagctctcc caacagctcc cacagcacca 1320
tcgctgagaa tggctttact ggctcagtcc ccaacatccc cactgagatg ctccctcagc 1380
accgagccct ccctctggac agctccccca accagttcag cctctacacg tctccttctc 1440
tgcccaacat ctccctaggg ctgcaggcca cggtcactgt caccaactca cacctcactg 1500
cctccccgaa gctgtcgaca cagcaggagg ccgagaggca ggccctccag tccctgcggc 1560
agggtggcac gctgaccggc aagttcatga gcacatcctc tattcctggc tgcctgctgg 1620
gcgtggcact ggagggcgac gggagccccc acgggcatgc ctccctgctg cagcatgtgc 1680
tgttgctgga gcaggcccgg cagcagagca ccctcattgc tgtgccactc cacgggcagt 1740
ccccactagt gacgggtgaa cgtgtggcca ccagcatgcg gacggtaggc aagctcccgc 1800
ggcatcggcc cctgagccgc actcagtcct caccgctgcc gcagagtccc caggccctgc 1860
agcagctggt catgcaacaa cagcaccagc agttcctgga gaagcagaag cagcagcagc 1920
tacagctggg caagatcctc accaagacag gggagctgcc caggcagccc accacccacc 1980
ctgaggagac agaggaggag ctgacggagc agcaggaggt cttgctgggg gagggagccc 2040

tgaccatgcc ccgggagggc tccacagaga gtgagagcac acaggaagac ctggaggagg 2100
aggacgagga agacgatggg gaggaggagg aggattgcat ccaggttaag gacgaggagg 2160
gcgagagtgg tgctgaggag gggcccgact tggaggagcc tggtgctgga tacaaaaaac 2220
tgttctcaga tgcccagccg ctgcagcctt tgcaggtgta ccaggcgccc ctcagcctgg 2280
ccactgtgcc ccaccaggcc ctgggccgta cccagtcctc ccctgctgcc cctgggggca 2340
tgaagagccc cccagaccag cccgtcaagc acctcttcac cacaggtgtg gtctacgaca 2400
cgttcatgct aaagcaccag tgcatgtgcg ggaacacaca cgtgcaccct gagcatgctg 2460
gccggatcca gagcatctgg tcccggctgc aggagacagg cctgcttagc aagtgcgagc 2520
ggatccgagg tcgcaaagcc acgctagatg agatccagac agtgcactct gaataccaca 2580
ccctgctcta tgggaccagt cccctcaacc ggcagaagct agacagcaag aagttgctcg 2640
gccccatcag ccagaagatg tatgctgtgc tgccttgtgg gggcatcggg gtggacagtg 2700
acaccgtgtg gaatgagatg cactcctcca gtgctgtgcg catggcagtg ggctgcctgc 2760
tggagctggc cttcaaggtg gctgcaggag agctcaagaa tggatttgcc atcatccggc 2820
ccccaggaca ccacgccgag gaatccacag ccatgggatt ctgcttcttc aactctgtag 2880
ccatcaccgc aaaactccta cagcagaagt tgaacgtggg caaggtcctc atcgtggact 2940
gggacattca ccatggcaat ggcacccagc aggcgttcta caatgacccc tctgtgctct 3000
acatctctct gcatcgctat gacaacggga acttctttcc aggctctggg gctcctgaag 3060
aggttggtgg aggaccaggc gtggggtaca atgtgaacgt ggcatggaca ggaggtgtgg 3120
acccccccat tggagacgtg gagtacctta cagccttcag gacagtggtg atgcccattg 3180
cccacgagtt ctcacctgat gtggtcctag tctccgccgg gtttgatgct gttgaaggac 3240
atctgtctcc tctgggtggc tactctgtca ccgccagatg ttttggccac ttgaccaggc 3300
agctgatgac cctggcaggg ggccgggtgg tgctggccct ggagggaggc catgacttga 3360
ccgccatctg tgatgcctct gaggcttgtg tctcggctct gctcagtgta gagctgcagc 3420
ccttggatga ggcagtcttg cagcaaaagc ccaacatcaa cgcagtggcc acgctagaga 3480
aagtcatcga gatccagagc aaacactgga gctgtgtgca gaagttcgcc gctggtctgg 3540
gccggtccct gcgagaggcc caagcaggtg agaccgagga ggccgagact gtgagcgcca 3600
tggccttgct gtcggtgggg gccgagcagg cccaggctgc ggcagcccgg gaacacagcc 3660
ccaggccggc agaggagccc atggagcagg agcctgccct gtgacgcccc ggcccccatc 3720
cctctgggct tcaccattgt gattttgttt attttttcta ttaaaaacaa aaagtcacac 3780
attcaacaag gtgtgccgtg tgggtctctc agccttgccc ctcctgctcc tctacgctgc 3840
ctcaggcccc cagccctgtg gcttccacct cagctctaga agcctgctcc ctctgcaggg 3900

ggtggtggtg tcttcccagc cctgtcccat gtgtccctcc ccccattttc ctgcattctg 3960

tctgtccttt tcctccttgg agcctgggcc agctcaaggt gggcacgggg gcccagacag 4020

tactctccag ttctggggcc ccccgagtga ggagggaacg ggaagtcggt gccttggttt 4080

cagctgattt ggggggaaat gccttaattt cactctcctc ccttctccag cctcagggga 4140

ggatctggag gatccactac tgtctttaag atgcagagtg gaggggaggt gggcacccac 4200

cctgcgattc tccacccttt ccccttcttt cgtcctcacc atctctgcag acccctctcc 4260

tcctccttcc tcttggtctc agcactgatg ggaggctggt gcccaagctg tggcctgcag 4320

tctgtgagga gggctgtctt gcctcacact cctcacagcc tacttcccct tccccggggc 4380

tgagagggtg aaagtgtgtg gggaaggaga ggactggttt cctgggttct caggggccag 4440

gaggagtaac agaaccaggt ctgctcccca ccttactcgg atggcctccc tgcccctctg 4500

ctggcacagc ctgggcaagg ggagaaggtg gtccctgcag aggggctcca ggctggtgag 4560

agcccccctg ctgtcaggac cagattttcc cagccatcca gcatgctgcg gggagaaggg 4620

gcagaggctc acctccctcc tggggccttt tgttttggat cctggggatg gtgagaatgg 4680

aggttctaga aggggtaagg ccagaaccca gggatccagg agtcggctct cagctggagc 4740

ttccatacct tctgggctcc ctttgctgac caccagccca agggagctaa gaccaggagg 4800

gggctgggcg ctgtcccttc tctttcccag gagccctgcc aggggctgtg ggcctacaag 4860

gcttccaggg gatgccatcc agcctgtagg aaaccaaaga tgggaagtgg ctcctagggg 4920

gctgactctt ccttcctcct cctccccagt accacatata ctttctctcc ttctatctcc 4980

agggccccac caatctgttt acatatttat tatcctatgg gggcctgagc aggattgagg 5040

gagccagggg aggggcagga gtcccagcac catcggttca tagtgtgctt gtgtgtttgt 5100

tttagatcct cctgggggat ggggatgggg ccaggctcag tgtactaggc ctctctgtgc 5160

tgagccccag gctcccggcc ccttacccac tctctccctg tggctggtct ggttctcatg 5220

taaacccact ccttgctttg tctccctgga tatggatttc agttaagtat tttgtaaccc 5280

gttacactgt gtgtccttgt gtaaataaac ttgtttctgg cagtgcc 5327

<210> 20
<211> 1739
<212> DNA
<213> Homo sapiens

<400> 20
tccccattga ataacagcca agttgctttg gtttctattt ctttgttaag tcgttccctc 60

tacaaaggac ttcctagtgg gtgtgaaagg cagcggtggc cacagaggcg gcggagagat 120

ggccttcagc ggttcccagg ctccctacct gagtccagct gtcccctttt ctgggactat 180

tcaaggaggt ctccaggacg gacttcagat cactgtcaat gggaccgttc tcagctccag 240

tggaaccagg tttgctgtga actttcagac tggcttcagt ggaaatgaca ttgccttcca 300

cttcaaccct cggtttgaag atggagggta cgtggtgtgc aacacgaggc agaacggaag 360

ctgggggccc gaggagagga agacacacat gcctttccag aaggggatgc cctttgacct 420

ctgcttcctg gtgcagagct cagatttcaa ggtgatggtg aacgggatcc tcttcgtgca 480

gtacttccac cgcgtgccct tccaccgtgt ggacaccatc tccgtcaatg gctctgtgca 540

gctgtcctac atcagcttcc agcctcccgg cgtgtggcct gccaacccgg ctcccattac 600

ccagacagtc atccacacag tgcagagcgc ccctggacag atgttctcta ctcccgccat 660

cccacctatg atgtaccccc accccgccta tccgatgcct ttcatcacca ccattctggg 720

agggctgtac ccatccaagt ccatcctcct gtcaggcact gtcctgccca gtgctcagag 780

gttccacatc aacctgtgct ctgggaacca catcgccttc cacctgaacc cccgttttga 840

tgagaatgct gtggtccgca acacccagat cgacaactcc tgggggtctg aggagcgaag 900

tctgccccga aaaatgccct tcgtccgtgg ccagagcttc tcagtgtgga tcttgtgtga 960

agctcactgc ctcaaggtgg ccgtggatgg tcagcacctg tttgaatact accatcgcct 1020

gaggaacctg cccaccatca acagactgga agtgggggc gacatccagc tgacccatgt 1080

gcagacatag gcggcttcct ggccctgggg ccgggggctg gggtgtgggg cagtctgggt 1140

cctctcatca tccccacttc ccaggcccag cctttccaac cctgcctggg atctgggctt 1200

taatgcagag gccatgtcct tgtctggtcc tgcttctggc tacagccacc ctggaacgga 1260

gaaggcagct gacggggatt gccttcctca gccgcagcag cacctggggc tccagctgct 1320

ggaatcctac catcccagga ggcaggcaca gccagggaga ggggaggagt gggcagtgaa 1380

gatgaagccc catgctcagt cccctcccat cccccacgca gctccacccc agtcccaagc 1440

caccagctgt ctgctcctgg tgggaggtgg cctcctcagc ccctcctctc tgacctttaa 1500

cctcactctc accttgcacc gtgcaccaac ccttcacccc tcctggaaag caggcctgat 1560

ggcttcccac tggcctccac cacctgacca gagtgttctc ttcagaggac tggctccttt 1620

cccagtgtcc ttaaaataaa gaaatgaaaa tgcttgttgg cacattcaaa aaaaaaaaaa 1680

aaaaaaaaaa aaaaaaaaaa aaaaaaaaaa aaaaaaaaaa aaaaaaaaaa aaaaaaaaa 1739

<210> 21
<211> 4865
<212> DNA
<213> Homo sapiens

<400> 21
gcagtgccgc cttttttttt tttttgcatc ccattttttt aaatttgcaa ttttatattt 60

tgcaaatatt ttgagagaca ttgatttttc tccccgtgct cccccgttct tccctgcgga 120

gtgcgctgcg ccgcccagcc ctgtcgcccc ccggaggtga tccctccctc ctgcctgccc 180

gccagcctga cctgtgcccg gctcgcgggc cgcagcctcg gccccggcgc gcccccggca 240
gctctcggcg cgatgagcat agagacgcta ctggaggcgg cccgcttcct ggaatggcaa 300
gcgcagcaac aacagagagc acgtgaggag caggagcggc ttcgcttgga gcaggagcga 360
gagcaggaac agaagaaggc caatagcctg gccaggctgg cacataccct tcctgtggag 420
gaaccccgca tggaggcgcc acccctgcct ctgtctccac cggctccccc gccggcaccc 480
ccaccaccac ttgccacccc tgccccactg actgtcatcc ctatccctgt ggtgaccaac 540
tcccctcagc ctctaccccc acccccaccc ttgcccgcgg cagcccagcc tctgcccctg 600
gcgcctcgtc agccggccct ggttggcgcc cccggactca gcattaagga gcctgccccc 660
ctgcccagca ggccgcaggt gcccacccct gctcccctac tgccggactc gaaggccacc 720
attccaccca atggcagccc caagcctttg cagcccctcc ccacgcctgt cctgaccata 780
gcgccacacc ctggagtcca gcctcagctg gccccccagc agccgccccc acccacgctg 840
gggaccctga agttggcacc agctgaagaa gtcaaatcca gtgaacagaa gaagaggccc 900
ggggggatcg gaaccagaga agtccacaac aaattggaga agaacaggag ggcccatctg 960
aaagagtgct ttgagaccct gaagcggaac atccccaacg tggatgacaa gaagacgtcc 1020
aatctgagcg tgctgcggac ggcgctgcgg tacatccagt ccctgaagag gaaggagaag 1080
gaatatgagc atgaaatgga gcggctggca cgtgagaaga ttgccacgca gcagcggctg 1140
gcagagctca agcacgagct gagccagtgg atggacgtac tggagattga ccgcgtgctg 1200
cggcagacgg gccagcccga ggatgaccag gcctccacct ccaccgcctc tgagggtgag 1260
gacaacatag acgaggatat ggaggaggac cgggcgggcc tgggcccacc taagctgagc 1320
catcgtcccc agccggagct gctgaagtcc accctgccac cccccagcac cacccctgcg 1380
cctctgcctc cacacccaca ccctcacccc cactccgtgg ccctacctcc tgcccacctc 1440
cccgtgcagc agcagcagcc acagcagaag acccctctgc cagcccctcc tcccccaccg 1500
gctgcccctg cccagacact ggtgccagct ccagcccatc tggtggcgac ggctgggggt 1560
ggctccacgg tcatcgccca cacagccacc actcacgctt cagtcatcca gactgtgaac 1620
cacgttctgc aggggccagg cggcaagcac atcgcccaca tcgccccctc ggcccccagc 1680
cctgcggtgc aactggcgcc tgccacaccc cccattgggc acatcactgt gcaccctgcc 1740
accctcaacc atgtggccca cctgggctcc cagctgccct tgtacccgca gcccgtggca 1800
gtgagccaca tcgcccacac cctctcgcac cagcaagtca acggcacggc cggcctgggg 1860
cccccggcta ctgtcatggc aaagccggcc gtgggggctc aggtggtgca ccacccccag 1920
ctggtgggcc agaccgtgct caaccctgtg accatggtca ccatgccctc cttcccagtc 1980
agcacactca agctggcttg aggacgaggc cactcagagg cccccagtgg ggacagggag 2040
ggggacctgt cccccactct ctcacccacc agctccacac attccagcca ggcccaggcc 2100

agcccccccca cccacccccca ggcctcctag gggaaggggg tgcaaagact ctgagccaag 2160
ggagggaagg gccaccctgc tgcactagga cttggtaaga tgactctgag aaaatgcgag 2220
actctgatgg aatgtgccac ctgtccggcc cagtgccagc tccagtgccg ctcctgcttc 2280
ccctccctac cctcggaaat cagtgcgatg tggacgtcac gctccctgac ttctccccg 2340
ccctgccccg ccttcgtgtg ctgctgctgc tattgctgtc tggtgaggtg gcccaggccc 2400
ccggctttcc tccggagcct catgttctct tcccaggcct ttggaggga aatggggaaa 2460
gcagaactga agccacttgg cccagaaagc tgcggattgg ggtgataagg ggccttgctc 2520
tgagcacagg tgacagatca taggaagtgg ctggtctgga gtcccacccg gacaggtggg 2580
gcctcagcct ggggctctct gacccggttg cagtcactgt gattcgttac cgtagatact 2640
acttaaaatg attctctacc aacaataaac caaacccagc cactgccaag cttcctgtgc 2700
cctcacccca atccctgcca ctgggctctg gacctcagga gggcaggctg aggtggggaa 2760
ggagggactt ggctgtcctt cccccttcccc gtcccctgca gcctgggtct ggatgggagg 2820
agagccactg ggcccctgtc cccagtcccc agtccccagc cctgggcttg gctcttggct 2880
tccagaaggc agcaaagagg ggcgctgtcc tgcgttcagc ccctgatctc tgacctctgc 2940
tgagtgctgg gcactgctcc aagggacagg tgggcctggc ggcctgtggg tttgggtgcc 3000
tcctgcagtt tgggagacat ggaccagcat ctggtcttgt ttccaggagc atagaagcca 3060
catcgttgag acatcaggaa ggtaaaaacc cagcggctta gccaagccct aagcctgtcc 3120
ccagaccaac cctgggacct atacagaaca gagggccaga gctagggctg ctgcttctgc 3180
tccagcccct ttgcctctgt cctcccatcc cctcaacacc ctgcttctcc cggggacgct 3240
tttgagtggg ccctgcccgg ggagctgcag agcagcagca cctttctctg agaagaggtc 3300
cttggttggg tcaaggacag ggctgagcgt ggaagggga ggagtcaggg gctctgtgtt 3360
aggatgcggc tttctctgcc tctgggcagc ctgctttggc ctttccttgt atgtgggtgt 3420
ttattacaag tggctttgtg tcagacacgc tcggctcccc acctggacac acactcacca 3480
gtggcctttc agtgtagcgg gaggaagccg gtgtccctgg atgtgaagct cacactgatg 3540
ggctggggca ggggcctggg ccggcgaggg ggccgggggg aggggacaga gctgaggatt 3600
tcctggagtg ccctgcaggc acagaggagg tcagcaaagg tcttgaatag attttctctg 3660
gaaataaaga atccttagat gcctaaaaat tcccttcctg ttccctcctg gtcctggaca 3720
cctcccaggg gactgttcct tatttctctc tcctggtgtg ggtaaaggga cagttacaaa 3780
ccaggtcacc atcctcagag gctgagccct gtacccaccc cagcacagcc acctccgcca 3840
gaccccgtgg ctctgggaga gccagctttg ctattcccat tgtgacagcg atggcaggcc 3900
tgacccacgg ggcagttaga gcagcctcgt ggagctcgcc cctcccctca gcctgcccac 3960

ctctccctgc atcctgaggc cagtggcccc cgggcctgca cagatacctc atcatccacc 4020

cgctgcccct ccccgtccct gtcccccagc atcgggggcc tgcaaatcta gtgccgaatg 4080

actatgtcca gattggtgac gatgggtgtt gctgtttttc ttgtcgtttg tgaacgctgt 4140

gatgctgtgt gcccaagtgg gcagtcacca cccagccctt ccttgggcgt ctcccccaga 4200

gtgctgtcgg gaccacatct gtcctcaccc tgtgggaccg cctggccctc cctccctagc 4260

ccttccagcc tgggacacac acacacacac acacacacac acacgcacgc acacgcacac 4320

atcttacctc tcatgcgtgt tttacctttt gatgttcaga gtggctcact ggctgggagt 4380

ccttacctcg gggagagggg gaggttggtt ccttgggggg ccaaagaagg cagggaatgc 4440

ctggagggta actgggggc caccatgacc ccttctctct ccagaaacag ctgcttctcc 4500

ccccatccca gggtcccacc cccaaccccc agaggtggcc cttgtttaca gtgaggactc 4560

ggccactgtg tctctgtttc ctgaaatata aactgtagcg accccagact gtagagattt 4620

ttatgtgttt ggatacatct gctgtgtgga aaaaaaaaaa aactacaaaa accctaattt 4680

tgtacatact gtatttttac tattgaactg tattctagtg gctgttcatg ctccaagact 4740

ttagttaccg agacatgaat actatccatg taataagcac ttgcctggaa taaaatataa 4800

aactgaaata aacctgcact gaaacctgag atggagctgc caaaaaaaaa aaaaaaaaaa 4860

aaaaa 4865

<210> 22
<211> 2507
<212> DNA
<213> Homo sapiens

<400> 22
ctggccggca ctcggcggcc gcggcgcgat ggaggcgccg gccgagctac tggccgcgct 60

gcctgcgctg gccaccgcgc tggcccttct gctcgcctgg ctactggtgc ggcgtggggc 120

ggccgcgagc ccggagcctg cccgcgcgcc cccggaaccc gcgccccccgg ccgaggccac 180

cggggccccg gcgccgtccc gcccctgcgc ccccgagccg gcggcctcgc ccgcggggcc 240

ggaggagcct ggagagcccg cggggctggg ggagctcggg gagcctgcgg gaccggggga 300

gcccgaaggg ccaggggatc ccgcggcggc gccagcggag gcggaggagc aggcggtgga 360

ggcgaggcag gaagaggagc aggacttgga tggtgagaag gggccatcat cggaagggcc 420

tgaggaggag gacggagaag gcttctcctt caaatacagc cccgggaagc tgaggggaaa 480

ccagtacaag aagatgatga ccaaagagga gctggaggag gagcagagag ttcagaagga 540

acagctggct gccatcttca agctcatgaa agacaacaag gagacgtttg gcgagatgtc 600

cgacggcgac gtgcaggagc agctccggct ctacgacatg tagactgcgc ccacgggatg 660

cacagcggcc atgcctgatg ctgtccccac ccttgcccca tgcccttgtt ctttccagac 720

ttctgtaggc ctaattttcc cttataaaca tagatgcagg cgtacattct ataggtactt 780
gccgagttct tgcagtgtgt atattacttt gcaggaatca aaattgtttt attcagaaga 840
caaagtccct gacccctctg gtttctgctt gctggtgagg ttccagctgt tattgggctc 900
tgaggccctc tcctgatcca aatctttttt tgtttgtttg agatggagtc tcactctgtc 960
tcccaggctg gagtgcagtg gcgtgatctt ggctcactgc aacctccacc tccctgattc 1020
aagcgactct cctgcctcag cctcccgagt agctgggatt gcaggcatgc accatcacac 1080
ctggctaatt ttgtattttc agtagagaca gcgttttgcc atgctggcca ggctcgtctc 1140
gaactcctga cctcaggtga tccgcccacc ttggcctccc aaagtgctgg gattacacgc 1200
gtgagccacc atgcccggcc aatactacac tcttattctg ccttacgagg atttctggaa 1260
gcttctgtaa ctgtagggaa gaaagctgta ggggagcatg gtttttttgt tttgtttttg 1320
tttttgagac gaggtctcac tgtcacccag gctggagtgc agtggtgcca tcatgactca 1380
ctgcagcctt gacctcccag gcgcacaaaa tcctcctctc agcctgagta actgggacta 1440
caggtgtgta ccactacatc cagctaaagg agagcgtttt tttctttttc tttttgagac 1500
agagtctcac tctggcgcct ggtcactgtg cccttcacct tctgggttca agtgattctc 1560
ctgcctcagc ctccctagta gctgggatta caggcatgca ccaccatacc cagctaattt 1620
ctgtattttt agtagagatg gagtttcacc atgttggcca ggctggtctc gaactcctga 1680
cctcaggtga tccacctgcc tcggcctccc aaagtgctag gattacaggc gtgagccacc 1740
atgcccagct gggagcgttc tttcagcaaa aagtctaatt cagagactca ccttccatcc 1800
tggcactaaa caatagtact agaggtaaga tctgccaaga atttctctca cttaagcagg 1860
atacttactt taaatataat taggcaagac ttatttgagt acttgcctaa ttgtatttaa 1920
ttagaactat gctggatgct gtagacagaa gaaataaaat actcaacttg gtccctatct 1980
tccagcaggt tagaggcctt tagggaagaa aagcctgtgt gcagggaaag agaacctcac 2040
cagaaagtat tcaataaaat gccaaagtga tcttacagaa cagtgcctgt gacaccggag 2100
gggattgttc tgtctttgtg agtgaacata caatgatggg gataactggt cctcatacca 2160
tgcaggtccc atttctgatc accccactgg caggacaatt cacagttctt gagcaaactg 2220
agttagggag ggtatgctaa cgacagaacc tatgcaagtt cttagaaata ttttttgttt 2280
gctgaaataa agctcaatca acacactacc ttaaaaaaaa agtggagcag tggcatttcc 2340
atgcctcatg ctcaccatca tgaggttaac ttcccgccgt cgcctcttgc ctgccagggg 2400
tttttcggga gcccctctct gttccactct gattcctttt gtttcttgct gaccattcac 2460
tctctgtgtc tgttgttgga tccaaatacg agtgtttttc tttcaaa 2507

<210> 23
<211> 3086

<212> DNA
<213> Homo sapiens

<400> 23
agttgggctg ctggagtgcg gcgccaccgc ggaggacagg ggcagctggc gggcagcggg 60

tgagggggtg gcggggacgc gagtggcggc cgcggggccc cggacaaggg tccgcagagc 120

tgcagccttc gagggccagc cctctccgag tccggggctg ggtcccacca gtgacaaggc 180

ggcagccccg cgcacaccaa agagaaggcg gctgtggcgg cagcggcagc cccagccatg 240

ctgtgttatg tgacgaggcc ggacgcggtg ctgatggagg tggaggtgga ggcgaaagcc 300

aacggcgagg actgcctcaa ccaggtgtgc aggcgactgg gaatcataga agttgactat 360

tttggactgc agtttacggg tagcaaaggt gaaagtttat ggctaaacct gagaaaccgg 420

atctcccagc agatggatgg gctagcccct tacaggctta aacttagagt caagttcttc 480

gtggagcctc atctcatctt acaggagcag actaggcata tctttttctt gcacatcaag 540

gaggccctct tggcaggcca cctcttgtgt tccccagagc aggcagtgga actcagtgcc 600

ctcctggccc agaccaagtt tggagactac aaccagaaca ctgccaagta taactatgag 660

gagctctgtg ccaaggagct ctcctctgcc accttgaaca gcattgttgc aaaacataag 720

gagttggagg ggaccagcca ggcttcagct gaataccaag ttttgcagat tgtgtcggca 780

atggaaaact atggcataga atggcattct gtgcgggata gcgaagggca gaaactgctc 840

attggggttg gacctgaagg aatctcaatt tgtaaagatg actttagccc aattaatagg 900

atagcttatc ctgtggtgca gatggccacc cagtcaggaa agaatgtata tttgacggtc 960

accaaggaat ctgggaacag catcgtgctc ttgtttaaaa tgatcagcac cagggcggcc 1020

agcgggctct accgagcgat aacagagacg cacgcattct acaggtgtga cacagtgacc 1080

agcgccgtga tgatgcagta tagccgtgac ttgaagggcc acttggcatc tctgtttctg 1140

aatgaaaaca ttaaccttgg caagaaatat gtctttgata ttaaaagaac atcaaaggag 1200

gtgtatgacc atgccaggag ggctctgtac aatgctggcg ttgtggacct cgtttcaaga 1260

aacaaccaga gcccttcaca ctcgcctctg aagtcctcag aaagcagcat gaactgcagc 1320

agctgcgagg gcctcagctg ccagcagacc cgggtgctgc aggagaagct acgcaagctg 1380

aaggaagcca tgctgtgcat ggtgtgctgc gaggaggaga tcaactccac cttctgtccc 1440

tgtggccaca ctgtgtgctg tgagagctgc gccgcccagc tacagtcatg tcccgtctgc 1500

aggtcgcgtg tggagcatgt ccagcacgtc tatctgccaa cgcacaccag tcttctcaat 1560

ctgactgtaa tctaatctgt tgtgcttttg ttggacttgg catgtttcca tgaactgcac 1620

tattataaac tattaaaatg atagattgtg gagaaagtaa ttattccaac acccatctgc 1680

catgcgatgt taaaaaaaaa aaaaaggaag aaaaataaca cagctactcc tcactgcaaa 1740

aacatatcca tgcgtagaat caacaactcc agtcatggga ccaggaggag ctctgggacg 1800

cagacacatt ccttggatgt tgattttttt tatgatctag taaaggaata ggtaaagtct 1860

ttgatgtcag tgaagtggca acatagccaa aaagttgggt accttttagg aaatgatgtt 1920

gtaagtctcc ttaatgtatc ctgaggtaag tttcctactg gcagcagatt ttgtaagaat 1980

tacttttaag aatttcattc tttttgtatg gtcatggagc tccaaccatt tttaatagga 2040

aagtcttttg taaattgttg tcgttttaat gtcatttctg tctttataac ttgatcaaga 2100

atgattggaa ggcaaacagg tttacaaatc aattctgtga cttttaaaaa gttgacaatg 2160

ttgtcagatt taaaccagtg tggctagtaa aaagcagctc actcaatgtg ggtggctccc 2220

tattccttta cgctccccct atccctaccc cacaagcctt tcgattataa aatactacca 2280

atcttgttat aagattactg tggagtagtc aagtactccc cgggccttct gagctggtgg 2340

aatattttat ttcagactga aaacagagag cactctcctt gggaagggaa agcggagctt 2400

gctgagtgag agatggagcc tcatggtgta caactgaggg tagttaactc atcacttctc 2460

ccaagcactc gatcccagct tcacccactg gtgttgcttt gcttgaactg ttcaagcctt 2520

ttatagcctt accataagta tttagatatg gtgtcctttt ctgtttttgg gggggggagtt 2580

ttgttgtgtt tttttaaagt aagtgcttaa gtattaactt tgggttgtcc cctctgtatg 2640

tttcgaaggg gttttggttc tttttgcttc tgttttctta aacatgtttt ccactcccac 2700

ttgggcattt tggaagctgg tcagctagca ggttttctgg gatgtcggga gacctagatg 2760

accttatcgg gtgcaatact agctaaggta aagctagaaa cctacactgt cactttactg 2820

agatttctga gtatactttt catattgcct taatgtagca gtaatgtgtt tatgcatttg 2880

tttctttgca cagacatttt gtcaaatatt aaaactctac ttttttatgg cacatattag 2940

catataagcc tttattccaa gaggtattta ttttttcact tgtaaaaaaa taatgtttcc 3000

acgtaaagaa ctctgttata tcctagagga ctctgtcttt tatattcggg ataataaaga 3060

ctttaaagca aaaaaaaaaa aaaaaa 3086

<210> 24
<211> 3082
<212> DNA
<213> Homo sapiens

<400> 24
cccagaccag cagaggcagc agccggagca gccgcagcct gcgccctctc ccgcccgccc 60

gccctccgcc cgcccgcccg ccctccgccg ccctccaccc gccccggggt ctctttcccc 120

cttcctcctc ctcctcctcc acccccccctt cctcctccgc ccgcccgcgg ggcccccctc 180

gccttcccgc ccgcccctat tgttccgccc ccggcctccc gcccttcccc ttcccgcccg 240

ctcccctttt cccctcagtc gcctcgcgcc tgcagttttt ggctttcacc cccaaccagt 300

gaccaaagac ttgaccactc aaagtccagc tccccagaac actgctcgac atggacaccg 360

gtgtgattga aggtggatta aatgtcactc tcaccatccg gctacttatg catggaaagg 420
aagttggcag tatcatcgga aagaaaggag aatcagttaa gaagatgcgc gaggagagtg 480
gtgcacgtat caacatctca gaagggaatt gtcctgagag aattatcact ttggctggac 540
ccactaatgc catcttcaaa gcctttgcta tgatcattga caaactggaa gaggacataa 600
gcagctctat gaccaatagc acagctgcca gtagaccccc ggtcaccctg aggctggtgg 660
tccctgctag tcagtgtggc tctctcattg gaaaaggtgg atgcaagatc aaggaaatac 720
gagagagtac aggggctcag gtccaggtgg caggggatat gctacccaac tcaactgagc 780
gggccatcac tattgctggc attccacaat ccatcattga gtgtgtcaaa cagatctgcg 840
tggtcatgtt ggagtccccc ccgaagggcg tgaccatccc gtaccggccc aagccgtcca 900
gctctccggt catctttgca ggtggtcagg cctataccat tcaaggacag tatgccattc 960
cacagccaga tttgaccaag ctgcaccagt tggcaatgca acagtctcat tttcccatga 1020
cgcatggcaa caccggattc agtggcattg aatccagctc tccagaggtg aaaggctatt 1080
gggcaggttt ggatgcatct gctcagacta cttctcatga actcaccatt ccaaacgatt 1140
tgattggctg cataatcggg cgtcaaggcg ccaaaatcaa tgagatccgt cagatgtctg 1200
gggcgcagat caaaattgcg aacccagtgg aaggatctac tgataggcag gttaccatca 1260
ctggatctgc tgccagcatt agcctggctc aatatctaat caatgtcagg ctttcctcgg 1320
agacgggtgg catgggggagc agctagaaca atgcagattc atccataatc cctttctgct 1380
gttcaccacc acccatgatc catctgtgta gtttctgaac agtcagcgat tccaggtttt 1440
aaatagtttg taaattttca gtttctacac actttatcat ccactcgtga ttttttaatt 1500
aaagcgtttt aattcctttc tctgttcagc tgttgatgct gagatccata tttagtttta 1560
taagcttctc cctggttttt ttttttggc tcatgaattt ttctgtttgt catggaaatg 1620
taagagtgga atattaatac atttcagttt agttctgtaa tgtcaggaat ttttcaaaaa 1680
aattaaaaga tggactggag ctttttcttt gtgaatagaa actggatgcc acagtgattc 1740
atgtgggttt tattcctctt gtcttgctgt tatttttgta ccttttatcc ctcaaaggac 1800
ccttcttggg ttttgaatgg aagcctttat tccggttaag atgttttctt ctattttacc 1860
acttccatct ttttttgtgg ccctcgatcc tatttttccc tgactccatg cttggttggc 1920
ccttataaaa cttgtgccca aaagattgag gattagactt tccgaggact tacctgtcct 1980
aggggagtag gcaagcactt ccactaggga gggggtgggg gaaaggaatg acacatgaca 2040
tacatggcat acacattaag cagttgatca tatgtctgac tgggttccag tttcttggga 2100
atgttggtcc ccttgttcag gcttgcatat tttaaactaa aaatttcagt ctattgtttt 2160
tagtaacttc atttatagtc ctccataaca agttagaagg atgtatctgc taccatttat 2220

tcctataatt ttagaaagtt ggggcttgac attatactca tttagtgaga gtagatgcaa 2280

aaaagtggag gggcaggaga acttctccag acacctcaga taaagtccgg agcccaaggc 2340

tttatcttaa ccatgtatgg taccccattc attcatcaag aaaaccctca acagctgggc 2400

ctgcatggag tgttatattt caaggttttt cacaggggtt acagtaggac agtccccacc 2460

ccaatcaggc accaggataa aagcagggac ttaaacagca ccccggttct tcagcctgag 2520

ccatcacatg ctatcagtct cctaacctcc ccctgggcct taagacaggg cttgggcaga 2580

gaagataaat ggtgggacaa aaaaatgagt tacattgcca cctgagaaac ctcagagggg 2640

aggacccagc cttagcctcc ctcctcccaa gtgcaaaatg tgtaaacaga gtaaacggaa 2700

cagaaaagtg cagtctaagt ggttttctct cctgcccctc ccaccgcccc tccccccacc 2760

ccctattatt tggggataaa gaatataaag acaaccctgg cttttctatt gccttgttgc 2820

ttgctgaata taaggaatgg ggtggggcag gaaggggctt gcccttagcc acagctctac 2880

ggctgtgcct cattcatttc cacagctgcc agtgtcccta gagtttatca ggtgaattgg 2940

tcaggggatc agtctccctc gagcctgact tacggctggg acagccccat ctttctgttg 3000

attatgtggc gcatatatat atatatatgt atatatatat aatttatata aatatttctc 3060

tatgtaaaaa aaaaaaaaaa aa 3082

<210> 25
<211> 2439
<212> DNA
<213> Homo sapiens

<400> 25
gagagcagcg gccgggaagg ggcggtgcgg gaggcggggt gtggggcggt agtgtgggcc 60

ctgttcctgc ccgcgcggtg ttccgcattc tgcaagcctc cggagcgcac gtcggcagtc 120

ggctccctcg ttgaccgaat caccgacctc tctccccagc tgtatttcca aaatgtcgct 180

ttctaacaag ctgacgctgg acaagctgga cgttaaaggg aagcgggtcg ttatgagagt 240

cgacttcaat gttcctatga agaacaacca gataacaaac aaccagagga ttaaggctgc 300

tgtcccaagc atcaaattct gcttggacaa tggagccaag tcggtagtcc ttatgagcca 360

cctaggccgg cctgatggtg tgcccatgcc tgacaagtac tccttagagc cagttgctgt 420

agaactcaaa tctctgctgg gcaaggatgt tctgttcttg aaggactgtg taggcccaga 480

agtggagaaa gcctgtgcca acccagctgc tgggtctgtc atcctgctgg agaacctccg 540

ctttcatgtg gaggaagaag ggaagggaaa agatgcttct gggaacaagg ttaaagccga 600

gccagccaaa atagaagctt tccgagcttc actttccaag ctagggggatg tctatgtcaa 660

tgatgctttt ggcactgctc acagagccca cagctccatg gtaggagtca atctgccaca 720

gaaggctggt gggtttttga tgaagaagga gctgaactac tttgcaaagg ccttggagag 780

cccagagcga cccttcctgg ccatcctggg cggagctaaa gttgcagaca agatccagct 840

catcaataat atgctggaca aagtcaatga gatgattatt ggtggtggaa tggcttttac 900

cttccttaag gtgctcaaca acatggagat tggcacttct ctgtttgatg aagagggagc 960

caagattgtc aaagacctaa tgtccaaagc tgagaagaat ggtgtgaaga ttaccttgcc 1020

tgttgacttt gtcactgctg acaagtttga tgagaatgcc aagactggcc aagccactgt 1080

ggcttctggc atacctgctg gctggatggg cttggactgt ggtcctgaaa gcagcaagaa 1140

gtatgctgag gctgtcactc gggctaagca gattgtgtgg aatggtcctg tgggggtatt 1200

tgaatgggaa gcttttgccc ggggaaccaa agctctcatg gatgaggtgg tgaaagccac 1260

ttctaggggc tgcatcacca tcataggtgg tggagacact gccacttgct gtgccaaatg 1320

gaacacggag gataaagtca gccatgtgag cactgggggt ggtgccagtt tggagctcct 1380

ggaaggtaaa gtccttcctg ggtggatgc tctcagcaat atttagtact ttcctgcctt 1440

ttagttcctg tgcacagccc ctaagtcaac ttagcatttt ctgcatctcc acttggcatt 1500

agctaaaacc ttccatgtca agattcagct agtggccaag agatgcagtg ccaggaaccc 1560

ttaaacagtt gcacagcatc tcagctcatc ttcactgcac cctggatttg catacattct 1620

tcaagatccc atttgaattt tttagtgact aaaccattgt gcattctaga gtgcatatat 1680

ttatattttg cctgttaaaa agaaagtgag cagtgttagc ttagttctct tttgatgtag 1740

gttattatga ttagctttgt cactgtttca ctactcagca tggaaacaag atgaaattcc 1800

atttgtaggt agtgagacaa aattgatgat ccattaagta aacaataaaa gtgtccattg 1860

aaaccgtgat tttttttttt ttcctgtcat actttgttag gaagggtgag aatagaatct 1920

tgaggaacgg atcagatgtc tatattgctg aatgcaagaa gtggggcagc agcagtggag 1980

agatgggaca attagataaa tgtccattct ttatcaaggg cctactttat ggcagacatt 2040

gtgctagtgc ttttattcta acttttattt ttatcagtta cacatgatca taatttaaaa 2100

agtcaaggct tataacaaaa aagccccagc ccattcctcc cattcaagat tcccactccc 2160

cagaggtgac cactttcaac tcttgagttt ttcaggtata tacctccatg tttctaagta 2220

atatgcttat attgttcact tctttttttt ttattttta aagaaatcta tttcatacca 2280

tggaggaagg ctctgttcca catatatttc cacttcttca ttctctcggt atagttttgt 2340

cacaattata gattagatca aaagtctaca taactaatac agctgagcta tgtagtatgc 2400

tatgattaaa tttacttatg taaaaaaaaa aaaaaaaaa 2439

<210> 26
<211> 7957
<212> DNA
<213> Homo sapiens

<400> 26

gcgcccgccg tgtgagcagc agcagcggct ggtctgtcaa ccggagcccg agcccgagca 60
gcctgcggcc agcagcgtcc tcgcaagccg agcgcccagg cgcgccagga gcccgcagca 120
gcggcagcag cgcgccgggc cgcccgggaa gcctccgtcc ccgcggcggc ggcggcggcg 180
gcggcaacat ggcctcggct ggtaacgccg ccgagcccca ggaccgcggc ggcggcggca 240
gcggctgtat cggtgccccg ggacggccgg ctggaggcgg gaggcgcaga cggacggggg 300
ggctgcgccg tgctgccgcg ccggaccggg actatctgca ccggcccagc tactgcgacg 360
ccgccttcgc tctggagcag atttccaagg ggaaggctac tggccggaaa gcgccgctgt 420
ggctgagagc gaagtttcag agactcttat ttaaactggg ttgttacatt caaaaaaact 480
gcggcaagtt cttggttgtg ggcctcctca tatttggggc cttcgcggtg ggattaaaag 540
cagcgaacct cgagaccaac gtggaggagc tgtgggtgga agttggagga cgagtaagtc 600
gtgaattaaa ttatactcgc cagaagattg gagaagaggc tatgtttaat cctcaactca 660
tgatacagac ccctaaagaa gaaggtgcta atgtcctgac cacagaagcg ctcctacaac 720
acctggactc ggcactccag gccagccgtg tccatgtata catgtacaac aggcagtgga 780
aattggaaca tttgtgttac aaatcaggag agcttatcac agaaacaggt tacatggatc 840
agataataga atatctttac ccttgtttga ttattacacc tttggactgc ttctgggaag 900
gggcgaaatt acagtctggg acagcatacc tcctaggtaa acctcctttg cggtggacaa 960
acttcgaccc tttggaattc ctggaagagt taaagaaaat aaactatcaa gtggacagct 1020
gggaggaaat gctgaataag gctgaggttg gtcatggtta catggaccgc ccctgcctca 1080
atccggccga tccagactgc cccgccacag cccccaacaa aaattcaacc aaacctcttg 1140
atatggccct tgttttgaat ggtggatgtc atggcttatc cagaaagtat atgcactggc 1200
aggaggagtt gattgtgggt ggcacagtca agaacagcac tggaaaactc gtcagcgccc 1260
atgccctgca gaccatgttc cagttaatga ctcccaagca aatgtacgag cacttcaagg 1320
ggtacgagta tgtctcacac atcaactgga acgaggacaa agcggcagcc atcctggagg 1380
cctggcagag gacatatgtg gaggtggttc atcagagtgt cgcacagaac tccactcaaa 1440
aggtgctttc cttcaccacc acgaccctgg acgacatcct gaaatccttc tctgacgtca 1500
gtgtcatccg cgtggccagc ggctacttac tcatgctcgc ctatgcctgt ctaaccatgc 1560
tgcgctggga ctgctccaag tcccagggtg ccgtggggct ggctggcgtc ctgctggttg 1620
cactgtcagt ggctgcagga ctgggcctgt gctcattgat cggaatttcc tttaacgctg 1680
caacaactca ggttttgcca tttctcgctc ttggtgttgg tgtggatgat gtttttcttc 1740
tggcccacgc cttcagtgaa acaggacaga ataaaagaat cccttttgag gacaggaccg 1800
gggagtgcct gaagcgcaca ggagccagcg tggccctcac gtccatcagc aatgtcacag 1860
ccttcttcat ggccgcgtta atcccaattc ccgctctgcg ggcgttctcc ctccaggcag 1920

cggtagtagt ggtgttcaat tttgccatgg ttctgctcat ttttcctgca attctcagca 1980
tggatttata tcgacgcgag gacaggagac tggatatttt ctgctgtttt acaagcccct 2040
gcgtcagcag agtgattcag gttgaacctc aggcctacac cgacacacac gacaataccc 2100
gctacagccc cccacctccc tacagcagcc acagctttgc ccatgaaacg cagattacca 2160
tgcagtccac tgtccagctc cgcacggagt acgaccccca cacgcacgtg tactacacca 2220
ccgctgagcc gcgctccgag atctctgtgc agcccgtcac cgtgacacag gacaccctca 2280
gctgccagag cccagagagc accagctcca caagggacct gctctcccag ttctccgact 2340
ccagcctcca ctgcctcgag ccccccctgta cgaagtggac actctcatct tttgctgaga 2400
agcactatgc tcctttcctc ttgaaaccaa aagccaaggt agtggtgatc ttcctttttc 2460
tgggcttgct gggggtcagc ctttatggca ccacccgagt gagagacggg ctggacctta 2520
cggacattgt acctcgggaa accagagaat atgactttat tgctgcacaa ttcaaatact 2580
tttctttcta caacatgtat atagtcaccc agaaagcaga ctacccgaat atccagcact 2640
tactttacga cctacacagg agtttcagta acgtgaagta tgtcatgttg gaagaaaaca 2700
aacagcttcc caaaatgtgg ctgcactact tcagagactg gcttcaggga cttcaggatg 2760
catttgacag tgactgggaa accgggaaaa tcatgccaaa caattacaag aatggatcag 2820
acgatggagt ccttgcctac aaactcctgg tgcaaaccgg cagccgcgat aagcccatcg 2880
acatcagcca gttgactaaa cagcgtctgg tggatgcaga tggcatcatt aatcccagcg 2940
ctttctacat ctacctgacg gcttgggtca gcaacgaccc cgtcgcgtat gctgcctccc 3000
aggccaacat ccggccacac cgaccagaat gggtccacga caaagccgac tacatgcctg 3060
aaacaaggct gagaatcccg gcagcagagc ccatcgagta tgcccagttc cctttctacc 3120
tcaacggctt gcgggacacc tcagactttg tggaggcaat tgaaaaagta aggaccatct 3180
gcagcaacta tacgagcctg ggctgtcca gttaccccaa cggctacccc ttcctcttct 3240
gggagcagta catcggcctc cgccactggc tgctgctgtt catcagcgtg gtgttggcct 3300
gcacattcct cgtgtgcgct gtcttccttc tgaacccctg gacggccggg atcattgtga 3360
tggtcctggc gctgatgacg gtcgagctgt tcggcatgat gggcctcatc ggaatcaagc 3420
tcagtgccgt gcccgtggtc atcctgatcg cttctgttgg cataggagtg gagttcaccg 3480
ttcacgttgc tttggccttt ctgacggcca tcggcgacaa gaaccgcagg gctgtgcttg 3540
ccctggagca catgtttgca cccgtcctgg atggcgccgt gtccactctg ctgggagtgc 3600
tgatgctggc gggatctgag ttcgacttca ttgtcaggta tttctttgct gtgctggcga 3660
tcctcaccat cctcggcgtt ctcaatgggc tggttttgct tcccgtgctt ttgtctttct 3720
ttggaccata tcctgaggtg tctccagcca acggcttgaa ccgcctgccc acaccctccc 3780

ctgagccacc ccccagcgtg gtccgcttcg ccatgccgcc cggccacacg cacagcgggt 3840
ctgattcctc cgactcggag tatagttccc agacgacagt gtcaggcctc agcgaggagc 3900
ttcggcacta cgaggcccag cagggcgcgg gaggccctgc ccaccaagtg atcgtggaag 3960
ccacagaaaa ccccgtcttc gcccactcca ctgtggtcca tcccgaatcc aggcatcacc 4020
caccctcgaa cccgagacag cagccccacc tggactcagg gtccctgcct cccggacggc 4080
aaggccagca gccccgcagg gacccccca gagaaggctt gtggccaccc ccctacagac 4140
cgcgcagaga cgcttttgaa atttctactg aagggcattc tggccctagc aatagggccc 4200
gctggggccc tcgcggggcc cgttctcaca accctcggaa cccagcgtcc actgccatgg 4260
gcagctccgt gcccggctac tgccagccca tcaccactgt gacggcttct gcctccgtga 4320
ctgtcgccgt gcacccgccg cctgtccctg ggcctgggcg gaacccccga gggggactct 4380
gcccaggcta ccctgagact gaccacggcc tgtttgagga cccccacgtg cctttccacg 4440
tccggtgtga gaggagggat tcgaaggtgg aagtcattga gctgcaggac gtggaatgcg 4500
aggagaggcc ccggggaagc agctccaact gagggtgatt aaaatctgaa gcaaagaggc 4560
caaagattgg aaacccccca cccccacctc tttccagaac tgcttgaaga gaactggttg 4620
gagttatgga aaagatgccc tgtgccagga cagcagttca ttgttactgt aaccgattgt 4680
attattttgt taaatatttc tataaatatt taagagatgt acacatgtgt aatataggaa 4740
ggaaggatgt aaagtggtat gatctggggc ttctccactc ctgccccaga gtgtggaggc 4800
cacagtgggg cctctccgta tttgtgcatt gggctccgtg ccacaaccaa gcttcattag 4860
tcttaaattt cagcatatgt tgctgctgct taaatattgt ataatttact tgtataattc 4920
tatgcaaata ttgcttatgt aataggatta ttttgtaaag gtttctgttt aaaatatttt 4980
aaatttgcat atcacaaccc tgtggtagta tgaaatgtta ctgttaactt tcaaacacgc 5040
tatgcgtgat aatttttttg tttaatgagc agatatgaag aaagcacgtt aatcctggtg 5100
gcttctctag gtgtcgttgt gtgcggtcct cttgtttggc tgtgcgtgtg aacacgtgtg 5160
tgagttcacc atgtactgta ctgtgatttt ttttttgtct tgttttgttt ctctacactg 5220
tctgtaacct gtagtaggct ctgacctagt caggctggaa gcgtcaggat atcttttctt 5280
cgtgctggtg agggctggcc ctaaacatcc acctaatcct ttcaaatcag cccggcaaaa 5340
gctagactct cctcgtgtct acggcatctc ttatgatcat tggctgccat ccaggacccc 5400
aatttgtgct tcagggggat aatctccttc tctcggatca ttgtgatgga tgctggaacc 5460
tcagggtatg gagctcacat cagttcatca tggtgggtgt tagagaattc ggtgacatgc 5520
ctagtgctga gccttggctg ggccatgaga gtctgtatac tctaaaaagc atgcagcatg 5580
gtgcccctct tctgaccaac acacacacga cccctccccc aacaccccca aattcaagag 5640
tggatgtggc cctgtcacag gtagaaaaac ctatttagtt aattctttct tggcccacag 5700

tctcccagaa atgatgtttt gagtccctat agtttaaact ccctctctta aatggagcag 5760
ctggttgagg ctttctagat ctgtttgcat cttctttaaa actaagtggt gagcatgcat 5820
tgtggtgtag aggcaggcat tatgtaggat aagagctccg gggggattct tcatgcacca 5880
gtgtttaggg tacgtgcttc ctaagtaaat ccaaacattg tctccatcct ccccgtcatt 5940
agtgctcttt caatgtgatg tgggaaagca ggaggatgga cacaccccac tgaaagatgt 6000
aggcaggggc aggtctctca accaggcata tttttaaaag ttgcttctgt actggttctc 6060
ttcttttgct ctgaggtgtg ggctccctca tctcgtaacc agagaccagc acatgtcagg 6120
gaagcaccca gtgtcggctc cccatccaaa tccacaccag caccttgtta cagacaagaa 6180
gtcagaggaa agggcggggt ccctgcaggg ctgaagccta agctactgtg aggcgctcac 6240
gagtggcagc tcctgttact ccccttttaaa ttacctggga aatcttaaca gaaaggtaat 6300
gggcccccag aaatacccac agcatagtga cctcagaccc tgatactcac cacaaaactt 6360
ttaagatgct gattgggagc cgcttgtggc tgctgggtgt gtgtgtgtgt gtgtgcgtgc 6420
gtgcgtgtgt gtgtgtctct gctggggacc ctggccaccc ccctgctgct gtcttggtgc 6480
ctgtcaccca catggtctgc catcctaaca cccagctctg ctcagaaaac gtcctgcgtg 6540
gaggagggat gatgcagaat tctgaagtcg acttccctct ggctcctggc gtgccctcgc 6600
tcccttcctg agcccagctc gtgttgcgcc ggaggctgcg cggcccctga tttctgcatg 6660
gtgtagaact ttctccaata gtcacattgg caaagggaga actggggtgg gcggggggtg 6720
gggctggcag ggaattagaa tttctctctc tcttttaata gttttatttt gtctgtcctg 6780
tttgttcatt tggatgtttt aatttttaaa aaaaaaaaaa ctttgctgat atttataatt 6840
ttgtatcata agaatgtttt cctctacagt atttgtcatg ccagtttata acaaaaaaaa 6900
atgcagggat tttatttcta ttggaaacat tacagctatg ttttactttt ggacagaatt 6960
tttatttgta tagagtgctt actaatgtta aatagttcag agtatataac atttacatta 7020
aggactcatg gtaggtttta gggtaaggag tttaaaggaa ataaatattc aaactgggtc 7080
tcattgccaa ttttggtgga aatgagtttg tgtcatttca attacaaaga taaaagtatg 7140
ccatataatt tatttatatg aagatttatt tttgtagtgt acatagtagt catcaagtct 7200
tttgacagaa gtatattttt aaagaattta tatgtgatga atccataatg tctggaactt 7260
tgctgagaca tgagtgggca cagttttcat tgtaaattac agcaaggaaa gaaaatgttt 7320
aacagtgtta agagagtcag agcagagtgg atattcatgc gattatgaag tgtttattag 7380
ttaccattgg cgacctagca tgcttctcat ttcaaacctt ggaaggtgaa aatgtacaaa 7440
ctctctaaat aattaatgtt caaacactga tagaaattct aacatgaata aaaaataata 7500
taacttgttg gttatatctt tgtttgtaga atgctttttt tctttaaaat acttgggaga 7560

gacagttagt gttggagcca tcagaaactg ttgtttcatc ttgctgacct tgtgacactc 7620

ttcttttttg ctctatctga tggagagagt tttaggtttt cctccttttt gttttgtata 7680

aatagtgggt ataagcatag ctctgttaca tggatgtatt acaaagtggg ggctgagctt 7740

ttagtgtgat catcacccaa atagtgtaca ttgtacccat taattaattt cttatcacct 7800

gaccctattt ttgttttctc ataccaagaa acttcctaag ttaaccatca aaattagtcc 7860

ttctgtcatc tctcttagca tacttatttc ccttatgtga tgcctaataa tgagggaaca 7920

taaataaagc caaattcaaa gataaaaaaa aaaaaaa 7957

<210> 27
<211> 7940
<212> DNA
<213> Homo sapiens

<400> 27
gctcccattg gctgatgttg gcgcgaaggt gcgcgagtca gccctcgcgc tgggggcgca 60

ggaaacaata gaggccgcgc gcacagagcg agctcttgca gcctccccgc cctcccgca 120

acgctcgacc ccaggattcc cccggctcgc ctgcccgcca tggccgacaa ggaagccttc 180

gacgacgcag tggaagaacg agtgatcaac gaggaataca aaatatggaa aaagaacacc 240

ccttttcttt atgatttggt gatgacccat gctctggagt ggcccagcct aactgcccag 300

tggcttccag atgtaaccag accagaaggg aaagatttca gcattcatcg acttgtcctg 360

gggacacaca catcggatga acaaaaccat cttgttatag ccagtgtgca gctccctaat 420

gatgatgctc agtttgatgc gtcacactac gacagtgaga aaggagaatt tggaggtttt 480

ggttcagtta gtggaaaaat tgaaatagaa atcaagatca accatgaagg agaagtaaac 540

agggcccgtt atatgcccca gaacccttgt atcatcgcaa caaagactcc ttccagtgat 600

gttcttgttt ttgactatac aaaacatcct tctaaaccag atccttctgg agagtgcaac 660

ccagacttgc gtctccgtgg acatcagaag gaaggctatg ggctttcttg gaacccaaat 720

ctcagtgggc acttacttag tgcttcagat gaccatacca tctgcctgtg ggacatcagt 780

gccgttccaa aggagggaaa agtggtagat gcgaagacca tctttacagg gcatacggca 840

gtagtagaag atgtttcctg gcatctactc catgagtctc tgtttgggtc agttgctgat 900

gatcagaaac ttatgatttg ggatactcgt tcaaacaata cttccaaacc aagccactca 960

gttgatgctc acactgctga agtgaactgc ctttctttca atccttatag tgagttcatt 1020

cttgccacag gatcagctga caagactgtt gccttgtggg atctgagaaa tctgaaactt 1080

aagttgcatt cctttgagtc acataaggat gaaatattcc aggttcagtg gtcacctcac 1140

aatgagacta ttttagcttc cagtggtact gatcgcagac tgaatgtctg ggatttaagt 1200

aaaattggag aggaacaatc cccagaagat gcagaagacg ggccaccaga gttgttgttt 1260

attcatggtg gtcatactgc caagatatct gatttctcct ggaatcccaa tgaaccttgg 1320
gtgatttgtt ctgtatcaga agacaatatc atgcaagtgt ggcaaatggc agagaacatt 1380
tataatgatg aagaccctga aggaagcgtg gatccagaag gacaagggtc ctagatatgt 1440
ctttacttgt tgtgatttta gactcccctt ttttcttctc aaccctgaga gtgatttaac 1500
actggttttg agacagactt tattcagcta tccctctata taataggtac caccgataat 1560
gctattagcc caaaccgtgg gtgttttcta aatattaata ggggggcttg attcaacaaa 1620
gccacagact taacgttgaa attttcttca ggaattttct agtaacccag gtctaaagta 1680
gctacagaaa ggggaatatt atgtgtgatt atttttcttc ttatgctata tccccaagtt 1740
tttcagactc atttaagtaa aggctagagt gagtaaggaa tagagccaaa tgaggtaggt 1800
gtctgagcca tgaagtataa atactgaaag atgtcacttt tattcaggaa ataggggggag 1860
attcaagtca tatagattcc tactcgaaaa tcttgacacc tgactttcca ggatgcacat 1920
tttcatacgt agaccagttt cctcttggtt tcttcagtta agtcaaaaca acacgttcct 1980
ctttccccat atattcatat atttttgctc gttagtgtat ttcttgagct gttttcatgt 2040
tgtttatttc ctgtctgtga aatggtgttt tttttttgt tgttggtttt tttttttttt 2100
tttttaactt gggaccacca agttgtaaag atgtatgttt ttacctgaca gttataccac 2160
aggtagactg tcaagttgag aagagtgaat caataacttg tatttgtttt aaaaattaaa 2220
ttaatccttg ataagagttg cttttttttt ttaggagtta gtccttgacc actagtttga 2280
tgccatctcc attttgggtg acctgtttca ccagcaggcc tgttactctc catgactaac 2340
tgtgtaagtg cttaaaatgg aataaattgc ttttctacat aaccccatgc tgatgggttt 2400
tatttagtat aaaacatcca tcaaacacca gtctctggct tctagaagag tccttcagat 2460
gacagttgtt gtccatggtc tttgactatc aagagcagaa ttaaatgtaa tagtcccaga 2520
gctgtagaaa agaactttac tccttcccag ggaaagtgaa agacataaaa cactgaatca 2580
gaggtggcac agattagtct ttgataaggt aacgtttctt tgaagtctat ctgtagagaa 2640
ctacatggac ttccaagagt gtcaaaggca gtgtggtaga gagaatttaa ggcaagattt 2700
aaatttggaa aaggtgcttg aacctttttct cagaggtttt atttccccag tatgtttttc 2760
actggggcct ttacttaggt tagaaataat aggctttgaa ggcctctatc accagatgca 2820
ataaccagat aaaattcctg ttttttccca atcgcttagt tttttgttgt tgttgttttt 2880
taactgagta gatcattctg acccagaact actttcatga ggtaagatct ttgggaaaat 2940
ctgaatagcg ttaaccatta gattcaaatc tcaaatggtt tcttttcaag tctagttgtt 3000
ttagagtata gtgagaaata ccttgacaca attttaagag taaactatat gggtcagcat 3060
atccttgaac aaaaagtaga ctttgtaaaa gtattcattt aaattctaac actcgtggca 3120
caaaagaatg gaaattgtaa acccatgtaa tggaaattgg ctatcttttt gaccccacat 3180

gtgcccctca aaaatgtttt tggtttgggt caacacaagg caagatacat tctttaaaat 3240

actcccagat gtgtccatac attcatcctt cactcagtgc atatgtgagg gttgttgctg 3300

gaagacagga ggctcatctt tcctttcctt ggtgcattga gatcagtatc aacagcagat 3360

gaaatagaat ccagcaaaga gttgacatgt tctgcctccg gccaactcta gaatcttttt 3420

aagcaggtca gccagtattt gcaacttcca caggatgaat tgcttgccaa gtttctggca 3480

ctcttgtctg gttggaagag tacatccaaa gggtacttag tgatcctttg ctaagaagtt 3540

ttttgctgtt tccgggttac agatttggcc atatatttct aaacagcccc tgtaaagttg 3600

aaagaaaaag tttataacag tgaacttctg aggtttagtt actgcaggct ttgttgagaa 3660

gagattgtta cagtgtgatt tatggatgat cagggatgac tttcccctag caaatatttg 3720

gatgcctcct gtttgtcaaa tagaatgaat ggtgatggtg atgggaggga tagttaaacg 3780

ttttctctgc taggttaact tcttacaggt ataattacaa tgcctgaaat tctgtagttt 3840

catttctttg gattagtcgt tgtcttttcc agattgtaca caatctgatc aacacaaagg 3900

tagttagtag atcattaacc tcaattgcaa ggttataatt tctcaaacat taagcatatt 3960

atcagtcatg tggattcaaa cacagtataa gaaaatcctc aaggctgggt gtgatggctt 4020

atgcctgtaa tcccagctac ttagcaggct gaggcaggag tattgtttga acccaggagg 4080

cagagttgca gtgagccaag atcgtgccac tactccagcc tgggcaaaat agcaagaccc 4140

gacccccccat ctctactaaa aagaaattta aaaaaataaa atcctagaaa attagaaaaa 4200

gcaacaatag ttacttgtgg gccaggcgcg gtggttcacg cctgtaatcc cagcactttg 4260

agaggctgag gcacgtggat cacaaggtca ggagttcaag accagcctgg ccaagatggt 4320

gaaaccccgt ttctactaaa aatacaaaaa ctagctggcc gtggtggcat gctcctgtag 4380

tcccagctac tcaggaggct gaggcaggaa aatcacttga acccaggagg tggaggttgc 4440

agtgaactga gaccgtgcca ctgcactcca gcctgggtgg cagagcgaga ctgtctcaaa 4500

aaaaaaagaa agttgtgaat ttgatgtaag cttaggaaat gaataaaatt tataggcatc 4560

tgtataatgt acaacttgac acggactttc ttttatcctt agtttctttc acggactcta 4620

gaacttttat cagaatatac tggtaaaaca ttgggggagg gatcctgagt aggtgattgg 4680

tcagaaagat gccttcagtt ttgtcagtgt ctaaaagtta agtctgttta ggccaagcat 4740

ggtggctcac acctgaaatc ccagcactct gggaggccga ggcaagtgga tcacaaggtc 4800

aggagatgag accatcttag ccaacatggt gaaaccccgt ctctactaaa atacaaaaaa 4860

attagccagg cgtggtggtg cgtgcctata atcccagcta cttgggaggc tgaggcaggg 4920

gaatcgcttg aacccgggag gcagaggtca cgccactgca ctccagcctg gcaacagagc 4980

aagactccgt ctcaaaaaaa aaaaaaaaaa aaaagagtaa gtctgtgtaa catgaacatc 5040

tctgcttcca cccaaaacca cagcctttga atattatata aggaacttaa tggatagata 5100
tgtttattat ttttgatagc acaactgctt tctctgctat tataaggaaa actgagaata 5160
gcaggtgggt agggtaggat gaggaaacaa gatgcccaaa gcctagatgc cacagaattc 5220
atggtgataa tcagggcata ttttgagtcc tactagaaac aaacattcca aatgaactct 5280
gaatgcctga ctcaggcgtt ttggaggttt gggttatccc cttgtcatta ggcacacaag 5340
ggtttttgt tgtttttgtt ttttgggttt ttgttttgtt ttgaggcagt ctcactctgt 5400
tgtacaagct ggagtgctgt attgtgatct tgactcactg caacctctgc ctcctggttc 5460
aagcgattct cctgccttgg cctcctgagt agctgggatt ataagtgcct gccactatgc 5520
ccggctaatt tttgtatttt tagtggagat ggagttttgc catgttggcc aggctggtct 5580
tgaactcctg actccaggtg atccaccctc ctcagcctcc caaagtgcta ggattacagg 5640
cgtgagccac cccgtccggc ctgtttttaa ggcattaatt agtattgtta ggaaagcagt 5700
aacaatgcaa acaccactct tctcttcaca aagatcacct tgagactgtg tctccattcc 5760
acctgcctga gaagtgggag catcagcctg ttccaggctc ttgggtagta gcatagccct 5820
ttaaaaagag agagccattt tccatgtgtt tttggataag cacaatttga aaatcatttc 5880
ccaaatcctc tttttgtttt tgattctaag gtaaaatttt ccctaagccc tcccaccatc 5940
ccctcagcca gtattagatg agatttgtat agcagcagaa actgacttat aagtagagag 6000
ctcttcagca agactgagcc ttagctgttc catctctttg ttcttctgtt gctggagttg 6060
caccccattt cttaactgcc tctggcgttc ttccatttcc tccagctgtt cctgcatgag 6120
atggccaaga acatttctaa tgagccaaac aataaaaact cacattgtcc actcttactt 6180
ataaaacact tttttgttca ttgtttaatc ttgatagcag tattgaggct ggtatttata 6240
tgataggtta tgaaacaggt tcaaagaagt tgtgtcttgg aaaaaaagtg acaatgcttt 6300
tgaaaatgat gacgaaaaag gcatcttgtc tgttaaccac agcttgcttt aatagaatcc 6360
tgggagggtg attgggactt tttagtatta caacccttagt gtcattgagg aggattttgg 6420
tctagttagt gggctgagtt tcatatacct ctccctccat gtgcaggttt gttaagataa 6480
ttggtagttt ttaataatat aaaatactta agttgaaata caaaagtgtg gcaacaatta 6540
ttaaatattg gctagaattc taggagagtt acacaactag tggaagtcca tgtttagaaa 6600
ataaatggct tgtttaagga aaagtttttg tgtccaaagc tccttaaagt cagagagatt 6660
tctacctggt acttaacatc atatggaaat tgatgcttta gtgagggtgt tggctatcct 6720
attgctaatt tcctgcatcc ttttttcttc tttatttttg tatagagaca gggtctcgct 6780
atgttgccca ggctggtctt gttcctgggc tcaagcagtc ctcccgcctc ggtctcccaa 6840
agtgctggga ttacaggtgt gagccactgt gcccagctta tcctttttttc attacacaaa 6900
aagactgaat ttggttagtt ctaagttgga agataaagat ggtatgcaca ggaggccctt 6960

gggagccctc agataacttt ctcattcttc cagaatcagg ctgggatgca ttctgtaaat 7020

tttccctgcc taggatgtat acctgaggaa taaggtaagg aagatgtcag caagtcagtc 7080

tctggtttac ctgctagctg gcatggatcc ttaaggaagc aggagggagt tgggaagaga 7140

ggaaggggtg aagttggtat cttttaaagc gagagtgatt ttacctcaga ttttgaagaa 7200

tactaaggaa tccagttgtt ggggtacatg ctattattag aaggatctag ataatttgtc 7260

ctctgagtca tacttgacat tgtacctgtg gcacatcaat ccgcactgtt tgatactctg 7320

gctgaatctc agctttcacc aacattgtca aaggaccttt tttagtgccc agccatgcct 7380

aagagtgtgt catctgaaga gggaagcatc tgcatactgc tgtcctgatt gctcagtcct 7440

cactacctac cagacccgtt ggtaaggtac aaaagtacat gcttggaaaa gcagtctgca 7500

ccaccagtga taagctgtga cagagtggaa cagcctcaat gaaatgaagg aaggattgct 7560

acagtggcat taaggatggt ctcttaatcc tgtgttaacc actagattaa ctttacaatc 7620

aactcaaaat ccttcaaagg ctttccactt tctttagtgg cattcagacc ccctctagtt 7680

tgacccctac ctccaacttg aacctctgtt actcttccgt atgaacattt tcctctagcc 7740

ctggactact agtaccgaag tcactagtca cataggactc atttgaaata tgactagtct 7800

caattgagat gtaatgtaag tgtaaaatac acagcagatt tctaagacag cacacaaaat 7860

gtaaaatatg tcaaaaatat ttgatactga ttacatgttg aaatatatgt gttgggttaa 7920

ataaaatgca ttaaagttaa 7940

<210> 28
<211> 3118
<212> DNA
<213> Homo sapiens

<400> 28
agcccttggc cccgccctcg cgccatcttg gggccctgg aggcggcgcc gcggaggacg 60

gagcggaagt gctcgctgca gcttcccgga gccggagcgc agcgcctgcg gccgcccgtg 120

ccccgccgtc ctccttcccg cggccgtgag ggagaccgcg gctcggccgt agcggagctg 180

cgagttacag aatgtctgaa ggggacagtg tgggagaatc cgtccatggg aaaccttcgg 240

tggtgtacag atttttcaca agacttggac agatttatca gtcctggcta gacaagtcca 300

caccctacac ggctgtgcga tgggtcgtga cactgggcct gagctttgtc tacatgattc 360

gagtttacct gctgcagggt tggtacattg tgacctatgc cttggggatc taccatctaa 420

atcttttcat agcttttctt tctcccaaag tggatccttc cttaatggaa gactcagatg 480

acggtccttc gctacccacc aaacagaacg aggaattccg cccctttcatt cgaaggctcc 540

cagagtttaa attttggcat gcggctacca agggcatcct tgtggctatg gtctgtactt 600

tcttcgacgc tttcaacgtc ccggtgttct ggccgattct ggtgatgtac ttcatcatgc 660

tcttctgtat cacgatgaag aggcaaatca agcacatgat taagtaccgg tacatcccgt 720
tcacacatgg gaagagaagg tacagaggca aggaggatgc cggcaaggcc ttcgccagct 780
agaagcggga ctgaggctgc ctcacgtgtt gcaagaacag ttttgagcca ttgttaacaa 840
tgcctttttt cttcacataa agtagttgat tacgagggag tcaaattttc tttttaaaaa 900
ggagcttcaa tgatttgtaa ctgaaatatc aggttctaga agaaactggc gcttaaacca 960
aatcgcatgg atttcttttt cagtgacgtt caagtgtttc tcacggatgg aattctagtc 1020
agctgcaggc gggaagccag gcgggtggag cccatgggag caagggcgag tggccggtcc 1080
ccgctgtgcc aggtgggcag gcaggagcaa ggcctgcgag ggaggaacgg gccgctcccc 1140
gccagccgcc ttccccagca gccgcaggtg gtgccagcca ctccacagag cccgagggat 1200
gatctagcct gattcctgcg tgtccgaaag aacttaacgt tttaaaggtg attgtcaagt 1260
aactgtgtgg ggttctaatg ccagtttcct aattccatct cactggagat gtttaaagtt 1320
ggcctctatc ctaatgactc aaaacttggt tcttaactac catgattgct tttgagggcc 1380
cggaattata aatatatatt atattttaat tgtttgagat tattttgaca catttctttg 1440
atacgtagag tgttttgttt ttaatttaaa tctgtcctca tgcaaccctc catgaggggc 1500
agcgaagctg gcagggagca gactggcttt gtaggttcag cactcggccc cccactgcgg 1560
gagaggcgga acccacttgc atgtcagcgt ttttgattcg agaaaagaaa tactctcaac 1620
gttttaccaa gtgattttac ctccaccttt actaaagtct ttacctaaaa catggcagtc 1680
gctggacaca ggaaagccca ccttttgttt ggccttttcg aaaggtgacc catattgcac 1740
agcagaacat cacagctgtg gtcccagatg agacactgac atgcgagtga aggcctctcc 1800
tcctgggccc cgggctgcgc aggctcctca ctctgggcgg tgtttcctgt ctcagaattg 1860
acacggtgaa tgcttagtgt ctggattttc ttgtaccagt gtttacatat ctgacatcga 1920
gctcctctaa gaggccacgt tcaagcttgt gtgtccctga cccaagatag ccagtgctgc 1980
tcccaggtgg tacttctggt accgtgttga gacacttggg attctcagac tgtggacagg 2040
agtgtttgtc atttttcata ctgttttctt aataagcgct caggcctaag gtgtgacagg 2100
aagtcgcacg cgcttggcca gagcacagtg aagcaaagga ctgggtgctg atggatggag 2160
ccacggcggc atctgcccac ccggccgcag cccccagtgc ctctcctggt ggtcctccca 2220
gtctagaggg tcacggcccc cccgccctcc tccgtctctg gcaagctgac cttgactaac 2280
ccaggaatac agggtcatcc tcattcctaa gtaagtcaaa cagcaagaca tggtttgcgc 2340
gggtctttgc cggaagccgg tcctgctggc caggtgtttt acgtcagcag ggaaatgtgg 2400
cacacgccct cgaggcattt taacactgcg cttcaggaaa tctcaagttc catcttgtgt 2460
tagtaacgta cccacatttt gctggagtta gtttattaaa gatgcctacg gtgaactctc 2520

tggcgcaggt taaatgcagt tttgaaaacc tggaaacatc aaatggaggc gggaaatagg 2580

ctggggccga gctgaggggc tgaacacagc agtgaccgtg ggtcagcagg tcgcctgccc 2640

agcaggcccc ccaggagagg gctcgggcgc ccctggcagc ccccataccc ccaggacctg 2700

gctcgtgagt gcgtctgggt caggaagaga cctctctgtg cgtctcaggc tgagatgcag 2760

atttctgttt tctaaaactg gaagcgacct tgacgtgtat tgaaggtgtg tgtgccaaat 2820

gcttccgacg gaggtgctgg ccttggttgg tttctctctg ccccgtgtgg tcatcaagtc 2880

ctgggggatg tgctctgccc agccgccctc ggggagagca gcgccgcctc ccatggggcc 2940

gtggggctgc tgttctcact gcactggctg aagcaacccg ccagcctccg tgccccaccc 3000

cacccagcac gcactcattc agtccattgc cttaacacaa gcctgatggg gctgttttct 3060

cacaatataa acgaataaag tgtcttctgg cctacttctg aaaaaaaaaa aaaaaaa 3118

<210> 29
<211> 1613
<212> DNA
<213> Homo sapiens

<400> 29
aagctactca gataagaggc tccaagagga catttttgga tgtgaaaaac aatgagaagg 60

aggacaacac acatttacaa tcgtcttaat tttgtactca gaaaaaggat gtgaagacaa 120

tgcacaggga atacaatagt ttcagatctg tgtacagttt ccttttgctt catctcctgc 180

aacaatgtaa tgaagacacc atgatatcat taacatttca cacaaaagga aaatgaggct 240

gaaatggtgt gggcaaggcc caggaatctg gagcatccct aaccaagcag cagagcacct 300

gggatagaga aagtgctcaa gaatgttcac ttactgatta ctacaatcaa aaaaagatac 360

gacactaatt taccacattc ttcttactta ttttatgaga tactattctt ccaaggtgga 420

gaaagtggag aaagtagagt gacgcagcta agggagtaaa tcgaccctca gccaacaagt 480

ggcaaaagcc tgaagaaagt gatcaagatc actgatgacc ccgcggccca tctccaaggg 540

ggcgggtatc acaaccccga cgccacacca cgtatcattc cgcaaaactc ccgcgcctcc 600

cacgcagaac tggcaagagg gaaggcgaga cagcagtgaa cagctggtac gcagcaccca 660

cagcaccgcg gcagcagcta gtgccgactc ccgcctagct cttttgactc tgttcgcggg 720

aagaatgggg aaacagtaag gttgcggcgc ctcccgcgag acgaggtacc tgaggctggc 780

cccgcagtcc cccgccgcac cagcaccgga gcttcacacc ccacttccgg ggtcaagtca 840

ccgccgggaa tcctgtgatc gcagaaaggt agtctcaggt tccgccccta tccaagtccc 900

gcctccactg cctctcgccc tgtatctgtc aacttccggg acgccgcgcg tcactaagca 960

gccaatctcc acttccggac tcatccagcc ccttctccac ccctttcaga gacagcgcga 1020

ttgcgattta ggtttccgcg catttaattg gcgaagctgg agcgctagtc ttcgctgatt 1080

ggtgccgaga aatctgcccc atagacaccc gcggggcgca cagtttcagt cgtccgtggg 1140

tttcccgcca gccgcagtct tggaccataa tcatggtgga catgatggac ttgcccaggt 1200

cgcgcatcaa cgccggcatg ctagctcaat tcatcgacaa gcctgtctgc ttcgtaggga 1260

ggctggaaaa gattcatccc accggaaaaa tgtttattct ttcagatgga gaaggaaaaa 1320

atggaaccat cgagttgatg gaaccccttg atgaagaaat ctctggaatt gtggaagtgg 1380

ttggaagagt aaccgccaag gccaccatct tgtgtacatc ttatgtccag tttaaagaag 1440

atagccatcc ttttgatctt ggactttaca atgaagctgt gaaaattatc catgacttcc 1500

ctcagtttta tcctttaggg attgtgcaac atgattgatc ttgatggatt ttcatacgat 1560

tgtaaatgag ctatattaaa gtctattaaa ggaaaaaaaa aaaaaaaaaa aaa 1613

<210> 30
<211> 6593
<212> DNA
<213> Homo sapiens

<400> 30
gcggaggaag gagactagag caggaagagc agcggcgagg cggcggtggt ggctgagtcc 60

gtggtggcag aggcgaaggc gacagctcta ggggttggca ccggccccga gaggaggatg 120

cgggtccgga tagggctgac gctgctgctg tgtgcggtgc tgctgagctt ggcctcggcg 180

tcctcggatg aagaaggcag ccaggatgaa tccttagatt ccaagactac tttgacatca 240

gatgagtcag taaaggacca tactactgca ggcagagtag ttgctggtca aatatttctt 300

gattcagaag aatctgaatt agaatcctct attcaagaag aggaagacag cctcaagagc 360

caagaggggg aaagtgtcac agaagatatc agctttctag agtctccaaa tccagaaaac 420

aaggactatg aagagccaaa gaaagtacgg aaaccagctt tgaccgccat tgaaggcaca 480

gcacatgggg agccctgcca cttccctttt cttttcctag ataaggagta tgatgaatgt 540

acatcagatg ggagggaaga tggcagactg tggtgtgcta caacctatga ctacaaagca 600

gatgaaaagt ggggcttttg tgaaactgaa gaagaggctg ctaagagacg gcagatgcag 660

gaagcagaaa tgatgtatca aactggaatg aaaatcctta atggaagcaa taagaaaagc 720

caaaaaagag aagcatatcg gtatctccaa aaggcagcaa gcatgaacca taccaaagcc 780

ctggagagag tgtcatatgc tcttttattt ggtgattact tgccacagaa tatccaggca 840

gcgagagaga tgtttgagaa gctgactgag gaaggctctc ccaaggqaca gactgctctt 900

ggctttctgt atgcctctgg acttggtgtt aattcaagtc aggcaaaggc tcttgtatat 960

tatacatttg gagctcttgg gggcaatcta atagcccaca tggttttggg ttacagatac 1020

tgggctggca tcggcgtcct ccagagttgt gaatctgccc tgactcacta tcgtcttgtt 1080

gccaatcatg ttgctagtga tatctcgcta acaggaggct cagtagtaca gagaatacgg 1140

ctgcctgatg aagtggaaaa tccaggaatg aacagtggaa tgctagaaga agatttgatt 1200
caatattacc agttcctagc tgaaaaaggt gatgtacaag cacaggttgg tcttggacaa 1260
ctgcacctgc acggagggcg tggagtagaa cagaatcatc agagagcatt tgactacttc 1320
aatttagcag caaatgctgg caattcacat gccatggcct ttttgggaaa gatgtattcg 1380
gaaggaagtg acattgtacc tcagagtaat gagacagctc tccactactt taagaaagct 1440
gctgacatgg gcaacccagt tggacagagt gggcttggaa tggcctacct ctatgggaga 1500
ggagttcaag ttaattatga tctagccctt aagtatttcc agaaagctgc tgaacaaggc 1560
tgggtggatg ggcagctaca gcttggttcc atgtactata atggcattgg agtcaagaga 1620
gattataaac aggccttgaa gtattttaat ttagcttctc agggaggcca tatcttggct 1680
ttctataacc tagctcagat gcatgccagt ggcaccggcg tgatgcgatc atgtcacact 1740
gcagtggagt tgtttaagaa tgtatgtgaa cgaggccgtt ggtctgaaag gcttatgact 1800
gcctataaca gctataaaga tggcgattac aatgctgcag tgatccagta cctcctcctg 1860
gctgaacagg gctatgaagt ggcacaaagc aatgcagcct ttattcttga tcagagagaa 1920
gcaagcattg taggtgagaa tgaaacttat cccagagctt tgctacattg gaacagggcc 1980
gcctctcaag gctatactgt ggctagaatt aagctcggag actaccattt ctatgggttt 2040
ggcaccgatg tagattatga aactgcattt attcattacc gtctggcttc tgagcagcaa 2100
cacagtgcac aagctatgtt taatctggga tatatgcatg agaaaggact gggcattaaa 2160
caggatattc accttgcgaa acgtttttat gacatggcag ctgaagccag cccagatgca 2220
caagttccag tcttcctagc cctctgcaaa ttgggcgtcg tctatttctt gcagtacata 2280
cgggaaacaa acattcgaga tatgttcacc caacttgata tggaccagct tttgggacct 2340
gagtgggacc tttacctcat gaccatcatt gcgctgctgt tgggaacagt catagcttac 2400
aggcaaaggc agcaccaaga catgcctgca cccaggcctc cagggccacg gccagctcca 2460
ccccagcagg aggggccacc agagcagcag ccaccacagt aataggcact gggtccagcc 2520
ttgatcagtg acagcgaagg aagttatctg ctgggaacac ttgcatttga tttaggacct 2580
tggatcagtg gtcacctccc agaagaggca cggcacaagg aagcattgaa ttcctaaagc 2640
tgcttagaat ctgatgcctt tattttcagg gataagtaac tcttacctaa actgagctga 2700
atgtttgttt cagtgccata tggaataaca actttcagtg gcttttttt ttcttttctg 2760
gaaacatatg tgagacactc agagtaatgt ctactgtatc cagctatctt tcttggatcc 2820
ttttggtcat tatttcagtg tgcataagtt cttaatgtca accatcttta aggtattgtg 2880
catcgacact aaaaactgat cagtgtaaaa aggaaaaccc agttgcaagt ttaaacgtgt 2940
tcgaaagtct gaaaatagaa cttgcctttt aagttaaaaa aaaaaaaagc tatcttgaaa 3000
atgttttgga actgcgataa ctgagaaact cttaccagtc cacatgcaat tagacatatt 3060

cagcatattt gttattttaa aagggagggt tgggaggttt cttattggtg attgtcacac 3120
ggtataccat actcctctcc ttcaaagaat gaaaggcctt gttaaggagt tttttgtgag 3180
ctttacttct ttggaatgga atatacttat gcaaaacctt gtgaactgac tccttgcact 3240
aacgcgagtt tgccccacct actctgtaat ttgcttgttt gttttgaata tacagagcct 3300
tgatccagaa gccagaggat ggactaagtg ggagaaatta gaaaacaaaa cgaactctgg 3360
ttggggtact acgatcacag acacagacat acttttccta aagttgaagc atttgttccc 3420
aggatttatt ttactttgca tttctttttg cacaaagaac acatcacctt cctgaattct 3480
ttaaatatga aatatcattg ccagggtatg gcttacagtg actactatta taatactaaa 3540
actcagagaa tcaaagatgg attaaactca gtggttgatg aaagccaaaa cctgtttgta 3600
ctgttctata ctattcaggt atctttttat ttctgatagt tttatattat aatagaaagc 3660
cagccactgc ttagctatca tagtcaccat tttctcactg ttaacattag gaaaatcaag 3720
gctactatgc ttcaggattg tctggttaaa tagtatggga aaaaaactga agagtttcac 3780
ataattacac acgtgaaata attaaagctt aaactgaatt tgtatttcat tttattgtca 3840
gatggtggtg ttcaccagcc tgtatcttgt ctgagactgc attcgtatct gagcaggttt 3900
tctatgccta ctgatgtcag tatgtttata ctaaccttca tgcttttttc ccagaatccc 3960
tcatctgcca gaaaacttga aaagtttatt gcttgtagag ttgtactgct ttgatttttg 4020
aagttggggt agtagttaga actagattta actagtctat aatgaacatg aaggctttta 4080
tatatgaagt tgtatacctt tttgtgttta gagaattatg ggaaacctgg taagcaaaac 4140
tttcctccca gataattgct tccaaattcg aagagttagt caccaagaga gccatatgta 4200
tgaaagcgta tctgtgaaag gtaggaaact tacccccccct aagtgtaatg ttgctttagg 4260
caactcttgt aaatagtgag acttgtttgg tctcttacat gtagagattt gagtgcagtt 4320
ggtacagtac tttggtgtct ccaccactgt cccttctccc cgcttcaaaa taagtgtaat 4380
ccacggtagc agccacactt cctttagaag gaactgttat aatttattta aaagttgaaa 4440
aaccacccaa gatgactacc aactttcact tttttcttct gccatccacc ctcatttttc 4500
ctttagcaag atttttatat ctaactttcc ttccctccat tgagtacgtg ctttgagaaa 4560
acatttctta aaacagtgtg tgccacctaa ggctggatgg gaaagtgcag tcttgttgtt 4620
catataaaaa cacacttctt attagtttac cacttgcctt tttctattgt taatgttctg 4680
aatttccttt tcttggcttg tttctacttc attttaccct gggtcacttg ctgccagcag 4740
tttgtgaatg gtgtctttca aataacttag ttcttatggc ttcacttaaa gactgtctca 4800
aaaatacttt gctctcttct tctttttttgt tcatgggaca tggtacctaa gcaaatagga 4860
gttgggtttg gtttttctcc taaaataatg ctcaatactt acctaatcaa atggcatcca 4920

tttgaataaa atgacaataa ctaaagctag ttaatgtcag tgacattaaa ctaactccag 4980

gattcaggag ttttaatgtt agaatttaga tttaacagat agagtgtggc ttcatttgtc 5040

catggtagcc catctctcct aagacctttt ctagtctgtc ttcctgcctt cgaacttgat 5100

gacagtaaaa ccctgtttag tattctcttg tgcatttggt ttgttggtta gccgactgtc 5160

ttgaaactat tcattttgct tctagtttta ttttacagag gtagcattgg tgggtttttt 5220

tttttttttc tgtctctgtg tttgaagttt cagtttctgt tttctaggta aggcttattt 5280

ttgattagca gtcaatggca aagaaaaagt aaatcaaaga tgacttcttt tcaaaatgta 5340

ttgtttagca cttaactcag atgaatttat aaattattaa tcttgatact aaggatttgt 5400

tacttttttg catattaggt taatttttac cttacatgtg agagtcttac cactaagcca 5460

ttctgtctct gtactgttgg gaagttttgg aaacccctgc cagtgatctg gtgatgatct 5520

gatgatttat ttaaagagcc gttgatgcct ccaggaaact taagtatttt attaatatat 5580

atataggaat ttttttttat tttgctttgt ctttctctcc cttcttttat cctcatgttc 5640

attcttcaaa ccagtgtttt ggaagtatgc atgcaggcct ataaatgaaa aacacaattc 5700

tttatgtgta tagcatgtgt attaatgtct aactacatac gcaaaaactt cctttacaga 5760

ggttcggact aacatttcac atgcacattt caaaacaaga tgtgtcatga aaacagcccc 5820

tttacctgcc aagacaagca gggctatatt tcagtgacag ctgatatttg ttttgaaagt 5880

gaatctcata atatatatat gtattacaca ttattatgac tagaagtatg taagaaatga 5940

tcagaacaaa agaaaatttc tattttcatg caaatatttt tcatcagtca tcactctcaa 6000

atataaatta aaatataaca ctcctgaatg cctgaggcac gatctggatt ttaaatgtgt 6060

ggtattcatt gaaaagaagc tctccaccca cttggtattt caagaaaatt taaaacgatc 6120

ccaaggaaag atgatttgta tgttaaagtg actgcacaag taaaagtcca atgttgtgtg 6180

catgaaaagg attccttggt tatgtgcagg gaatcatctc acatgctgtt tttcctattt 6240

ggtttgagaa acaggctgac actattctct ttgattagaa aataaactca taaaactcat 6300

aatgttgata taatcaagat gtaaccacta taaatatgta gaagaggaag ttttaaaaga 6360

ccttaagctg gcattgtgaa ggaacaccat ggtagactct ttttgtaaat gtattttgta 6420

tttaatgaaa tgcagtataa aggttggtga agtgtaatat aattgtgtaa acaaatcctg 6480

ttaatagaga gatgtacaga atcgttttgt actgtatctt gaaacttgtg aaataaagat 6540

tccacctctg gttatcctgt atgctgtaat ataccacaaa aaaaaaaaaa aaa 6593

<210> 31
<211> 2611
<212> DNA
<213> Homo sapiens

<400> 31

aggaacgact gtgctacgtt gccagaaggg gcgggacctg caacgtccga cagaacgagg 60
ggacgtaacg gaggcaggtt ggagccgctg ccgtcgccat gacccgcggt aaccagcgtg 120
agctcgcccg ccagaagaat atgaaaaagc agagcgactc ggttaaggga aagcgccgag 180
atgacgggct ttctgctgcc gcccgcaagc agagggactc ggagatcatg cagcagaagc 240
agaaaaaggc aaacgagaag aaggaggaac ccaagtagct ttgtggcttc gtgtccaacc 300
ctcttgccct tcgcctgtgt gcctggagcc agtcccacca cgctcgcgtt tcctcctgta 360
gtgctcacag gtcccagcac cgatggcatt ccctttgccc tgagtctgca gcgggtccct 420
tttgtgcttc cttcccctca ggtagcctct ctccccctgg gccactcccg gggtgaggg 480
ggttacccct tcccagtgtt ttttattcct gtggggctca ccccaaagta ttaaaagtag 540
ctttgtaatt ccttgagcgc ctggtttgac tggggacttg gggggatggg gttggaagaa 600
tgactgccct ttcccaccaa aaaagggaga actctttaga ttcagattgt gggtatgtag 660
acttaataag tgaaacatca cagaagaagc ctttattata caatgacaac caaacaagta 720
ctccggatat gcagtagagg aatcctctaa gaaccataga gacttctttt ctgtgatttt 780
tgttccccac ccttgaacac catctctagg atggagttgg cctaagagtg aatgctgcaa 840
gatctgtgtt tatgcctctt ttcctcattc ttcctcagtt tgttcgtctg cttgaaagtt 900
ggccaaaaaa tcctgctgct caccgacttc ccgtggtcag ctgctgtcaa gcgttcactt 960
tctcttctgt cattcctcat ggaatgaggg tggttttgtc ttcccgcttc ccttgacctc 1020
aaaatcagga ttaaaacctg ggtagcctc tgtgctcctt tcttctatgc cctggtttgt 1080
tctgtggttc tgggcttctt atatccgtgt gcccagggct gaactcctta ttttcctttc 1140
tccaagggca gagccgagtc ttcagtccct gttggtcttt ccccaccccc acttccagcc 1200
caagagccag gaaagggctg gtgccacact gtctgctggg atcagcggtg gttctttgag 1260
ctgctgattt gggtgttagg ctcttgagct gggatgcaga tgtaacagta gctccagtga 1320
gtcagacact ctgcccagca cattagactg tgtttgacca cttcttccag ttcatagtat 1380
tgacttcagc ccaaacggag ataactccct gtgtgtcctt gaggtattga gctgggctgg 1440
acagctcccc ttgagccaac tctaggagta caatgtcagg ggaaccccag tttgtgaaaa 1500
ggacttagac tggaggatat ttgttatctg gggatatgat gcggtggcgg cggcgcctca 1560
agataagggg ctggggtttc tgggtggggg gccaacagag tggtgccagt aacagcccca 1620
gatagaggag tacgcaggcc cagcatgagg caaccttgac ccagaaggtg gcccagctac 1680
ccttgatgaa ggtcttttcc agttctgctc cctcatagct gtgtaaccaa aggctctggt 1740
tagagaatat gaagggcctt agcttttaga cctgttctac ctcctcacca aatataatgg 1800
cagacccatg tgtgtctgga atggccttga attgctcttt ccttaaaata gctagctctt 1860
caggagagta tctaaggccc actccatctt acctgaacca gttggtaagg gtaaccatga 1920

catagagtga ggcaaggaag aagacgaagt ggaaggcaga atagttgtag gaaagatgct 1980

ggacttggac tggaggagct ggaggggttt cttggtcagc tggcctcgca gccccacccc 2040

tttgccctgg agagaggaaa tggctgctgg gagcagagct gctgaaacac ctcttcccct 2100

ctcccccaac tacctttgtt aaggctcttg agggttctta tggcactcca cagagatcta 2160

ccacttctta tggttcctca cttggcactc acctttgtct gcctccactg tttcagggca 2220

gcagaaacac agtgagggct tctgcaaaac agaacgcagg ttttggaatg gtcttaaaag 2280

atgtgagggt gttaatctag gaaacttccc ccgtgaaaag attggtctag tattaaaaag 2340

tggaggcaca cctgggttca aattctagct ccagcatata agtggctgtg cagactttgg 2400

taagatgttt aatcttttgt gcctcgattt ctccatttgt aaaatggagc aaatacctac 2460

ctcacagggt tgttgtgagg gttaaattaa atgagattat gtaaaagtat ctagcacagt 2520

tgcctagcac attgtgggta ctcaataaaa ggtaacagca gctataatct gagcattctg 2580

ggtagaggtt ggtaaaaaaa aaaaaaaaaa a 2611

<210> 32
<211> 2922
<212> DNA
<213> Homo sapiens

<400> 32
gttgttactt aggtgcgcta gcctgcggag cccgtccgtg ctgttctgcg gcaaggcctt 60

tcccagtgtc cccacgcgga aggcaactgc ctgagaggcg cggcgtcgca ccgcccagag 120

ctgaggaagc cggcgccagt tcgcggggct ccgggccgcc actcagagct atgagctacg 180

gccgcccccc tcccgatgtg gagggtatga cctccctcaa ggtggacaac ctgacctacc 240

gcacctcgcc cgacacgctg aggcgcgtct tcgagaagta cgggcgcgtc ggcgacgtgt 300

acatcccgcg ggatcgctac accaaggagt cccgcggctt cgccttcgtt cgctttcacg 360

acaagcgcga cgctgaggac gctatggatg ccatggacgg ggccgtgctg gacggccgcg 420

agctgcgggt gcaaatggcg cgctacggcc gcccccccgga ctcacaccac agccgccggg 480

gaccgccacc ccgcaggtac gggggcggtg gctacggacg ccggagccgc agccctaggc 540

ggcgtcgccg cagccgatcc cggagtcgga gccgttccag gtctcgcagc cgatctcgct 600

acagccgctc gaagtctcgg tcccgcactc gttctcgatc tcggtcgacc tccaagtcca 660

gatccgcacg aaggtccaag tccaagtcct cgtcggtctc cagatctcgt tcgcggtcca 720

ggtcccggtc tcggtccagg agtcctcccc cagtgtccaa gagggaatcc aaatccaggt 780

cgcgatcgaa gagtcccccc aagtctcctg aagaggaagg agcggtgtcc tcttaagaaa 840

atggtaatgt ctgggaatcc gagacacata accctaattc ataaatggga tttggggtag 900

gtctttttga gtcgtgttaa tgtaagaatg actcctatca ttaggagtgc tgctcggagg 960

ttactcacct ttgggagtaa tactgaagag aggggtctgc agaaaggatg tgtatgaagc 1020
ttagataata atggctgttt cgtaaactgt ttgagaccta ttaatgaaaa tgactatttc 1080
ttgctgtttt tatccaacgt ctgcattttc cccctttaaa gctgcggtct cctgtttgat 1140
aaaagaatat tggccagtat tgcagatttt aactgatttg gctgatcctc cagggaccag 1200
tttctgtggg cgtgtattgg agcaggtttg tctttaaatg ttaaagatgc actatcctct 1260
tagagaaaca atcagttcaa ctattgttgt actgactggg acttcatatt ctaatggatg 1320
tggcaaaaga attgcaataa gaagcagtga acatttggaa ccccaaaaga aagttacagg 1380
tattgcactg ggtggggaaa ggatagtgtg tctttaactc ttaaattgtt tggtcctatt 1440
ttttaaaaag gaaagggccc taagtagctc agatattaaa gtagtattct caattaccaa 1500
atgtttcatt tgaaacaatt tatcttaatg aaatatagac caattctctg atctcgagtt 1560
gtttttgttt ggatacagcc cttttttttt tctttttttt tcttcccctt acctttcttc 1620
accttggtta tttggccagg aatacgtaaa ttcaaacttg tacatgctga tggtagcctt 1680
tgtgaaattt tcctaattgg gccttttaaa aacatggctg ggtggaacat ttctgtaccc 1740
tactggtttg accagagcct tagtaagtac gtgcctgaaa ctgaaaccat gtgcacttta 1800
atggaaggta agctgaactt ctttcttttc aaacctagat gtatcggcaa gcagtgtaaa 1860
cggaggactt ggggaaaaag gaccacatag tccatcgaag aagagtcctt ggaacaagca 1920
actggctatt gaaaaggtta ttttgtaaca tttgtctaac tttttacttg tttaagcttt 1980
gcctcagttg gcaaacttca ttttatgtgc cattttgttg ctgttattca aatttcttgt 2040
aatttagtga ggtgaacgac ttcagatttc attattggat ttggatattt gaggtaaaat 2100
ttcattttgt tatatagtgc tgactttttt tgtttgaaat taaacagatt ggtaacctaa 2160
tttgtggcct cctgactttt aaggaaaacg tgtgcagcca ttacacacag cctaaagctg 2220
tcaagagatt gactcggcat tgccttcatt ccttaaaatt aaaaacctac aaaagttggt 2280
gtaaatttgt atatgttatt tacattcaga tctaaatggt aatctgaacc caaatttgta 2340
taaagacttt tcaggtgaaa agacttgatt ttttgaaagg attgtttatc aaacacaatt 2400
ctaatctctt ctcttatgta tttttgtgca ctaggcgcag ttgtgtagca gttgagtaat 2460
gctggttagc tgttaaggtg gcgtgttgca gtgcagagtg cttggctgtt tcctgttttc 2520
tcccgattgc tcctgtgtaa agatgccttg tcgtgcagaa acaaatggct gtccagttta 2580
ttaaaatgcc tgacaactgc acttccagtc acccgggcct tgcatataaa taacggagca 2640
tacagtgagc acatctagct gatgataaat acaccttttt ttccctcttc cccctaaaaa 2700
tggtaaatct gatcatatct acatgtatga acttaacatg gaaaatgtta aggaagcaaa 2760
tggttgtaac tttgtaagta cttataacat ggtgtatctt tttgcttatg aatattctgt 2820

attataacca ttgtttctgt agtttaatta aaacattttc ttggtgttag cttttctcag 2880

aaaaaaaaaa aaaaaaaaaa aaaaaaaaaa aaaaaaaaaa aa 2922

<210> 33
<211> 1628
<212> DNA
<213> Homo sapiens

<400> 33
ggggcggggc ccccttggct gctttcggcg tcggcctagg tgcgctgcga gcgcgcgcgg 60

accgcgcaca ggcggcggag ccggtatggg cccgcctgac cctgggcgcc gcgccgcacg 120

agcaccagcc tagagccagg actgaagctt caagatggct gaccaggacc ctgcgggcat 180

cagccccctc cagcaaatgg tggcctcagg caccggggct gtggttacct ctctcttcat 240

gacacccctg gacgtggtga aggttcgcct gcagtctcag cggccctcca tggccagcga 300

gctgatgcct tcctccagac tgtggagcct ctcctatacc aaattgccct cctctctcca 360

atccacaggg aagtgcctcc tgtattgcaa tggtgtcctg gagcctctgt acctgtgccc 420

aaatggtgcc cgctgtgcca cctggtttca agaccctacc cgcttcactg gcaccatgga 480

tgccttcgtg aagatcgtga ggcacgaggg caccaggacc ctctggagcg gcctccccgc 540

caccctggtg atgactgtgc cagctaccgc catctacttc actgcctatg accaactgaa 600

ggccttcctg tgtggtcgag ccctgacctc tgacctctac gcacccatgg tggctggcgc 660

gctggcccgc ctgggcaccg tgactgtgat cagccccctg gagcttatgc ggacaaagct 720

gcaggctcag catgtgtcgt accgggagct gggtgcctgt gttcgaactg cagtggctca 780

gggtggctgg cgctcactgt ggctgggctg ggggccccact gcccttcgag atgtgccctt 840

ctcagccctg tactggttca actatgagct ggtgaagagc tggctcaatg ggttcaggcc 900

gaaggaccag acttctgtgg gcatgagctt tgtggctggt ggcatctcag ggacggtggc 960

tgcagtgctg actctaccct ttgacgtggt aaagacccaa cgccaggtcg ctctgggagc 1020

gatggaggct gtgagagtga acccccctgca tgtggactcc acctggctgc tgctgcggag 1080

gatccgggcc gagtcgggca ccaagggact ctttgcaggc ttccttcctc ggatcatcaa 1140

ggctgccccc tcctgtgcca tcatgatcag cacctatgag ttcggcaaaa gcttcttcca 1200

gaggctgaac caggaccggc ttctgggcgg ctgaaagggg caaggaggca aggaccccgt 1260

ctctcccacg gatggggaga gggcaggagg agacccagcc aagtgccttt tcctcagcac 1320

tgagggaggg ggcttgtttc ccttccctcc cggcgacaag ctccagggca gggctgtccc 1380

tctgggcggc ccagcacttc ctcagacaca acttcttcct gctgctccag tcgtggggat 1440

catcacttac ccacccccca agttcaagac caaatcttcc agctgccccc ttcgtgtttc 1500

cctgtgtttg ctgtagctgg gcatgtctcc aggaaccaag aagccctcag cctggtgtag 1560

tctccctgac ccttgttaat tccttaagtc taaagatgat gaacttctgc aaaaaaaaaa 1620

aaaaaaaa 1628

<210> 34
<211> 3714
<212> DNA
<213> Homo sapiens

<400> 34
cggacgcgtg ggttggattt tttccgtgga tctatcaatt cacaattcga atttggaaga 60

aagaaggaaa acatgacgtc tccagccaaa ttcaaaaagg ataaggagat catagcagag 120

tacgatactc aggtcaaaga gatccgtgct cagctcacag agcagatgaa atgcctggac 180

cagcagtgtg agcttcgggt gcaactgttg caggacctcc aggacttctt ccgaaagaag 240

gcagagattg agatggacta ctcccgcaac ctggagaagc tggcagaacg cttcctggcc 300

aagacacgca gcaccaagga ccagcaattc aagaaggatc agaatgttct ctctccagtc 360

aactgctgga atctcctctt aaaccaggtg aagcgggaaa gcagggacca taccaccctg 420

agtgacatct acctgaataa tatcattcct cgatttgtac aagtcagcga ggactcagga 480

agactcttta aaaagagtaa agaagtcggc cagcagctcc aagatgattt gatgaaggtc 540

ctgaacgagc tctactcggt gatgaagaca tatcacatgt acaatgccga cagcatcagt 600

gctcagagca aactaaagga ggcggagaag caggaggaga agcaaattgg taaatcggta 660

aagcaggagg accggcagac cccacgctcc cctgactcca cggccaacgt tcgcattgag 720

gagaaacatg tccggaggag ctcagtgaag aagattgaga agatgaagga gaagcgtcaa 780

gccaagtaca cggagaataa gctgaaggcc atcaaagccc ggaatgagta cttgctggct 840

ttggaggcaa ccaatgcatc tgtcttcaag tactacatcc atgacctatc tgaccttatt 900

gatcagtgtt gtgacttagg ctaccatgca agtctgaacc gggctctacg caccttcctc 960

tctgctgagt taaacctgga acagtcgaag catgagggtc tggatgccat cgagaatgca 1020

gtagaaaacc tggatgccac cagtgacaag cagcgcctca tggagatgta caacaacgtc 1080

ttctgccccc ctatgaagtt tgagtttcag ccccacatgg gggatatggc ttcccagctc 1140

tgtgcccagc agcctgtcca gagtgagctg gtacagagat gccaacaact gcagtctcgc 1200

ttatccactc taaagattga aaacgaagag gtaaagaaga caatggaggc caccctgcaa 1260

accatccagg acattgtgac tgtcgaggac tttgatgtgt ctgactgctt ccagtacagc 1320

aactccatgg agtccgtcaa gtccacggtc tctgaaacct tcatgagcaa gcccagcatt 1380

gctaagagga gagccaacca gcaagagaca gagcagtttt atttcacaaa aatgaaagag 1440

tacctggagg gcaggaacct catcaccaag ttacaagcca agcatgacct tctgcagaaa 1500

accctgggag aaagtcagcg gacagattgc agtctagcca ggcgcagctc aactgtgagg 1560

aaacaggact ccagccaggc aattcctctg gtggtggaaa gctgtatccg gtttatcagc 1620
agacacggac tacagcatga aggaattttc cgggtgtcag gatcccaggt ggaagtgaat 1680
gacatcaaaa atgcctttga gagaggagag gaccccctgg ctggggacca gaacgaccat 1740
gacatggatt ccatagctgg tgtcctgaag ctttacttcc gggggctgga acaccctctc 1800
ttccccaagg acatctttca tgacctgatg gcctgcgtca caatggacaa cctgcaggag 1860
agagctctgc acatccggaa agtcctccta gtcctgccca aaaccactct gattatcatg 1920
agatacctct ttgccttcct caatcattta tcacagttca gtgaagagaa catgatggac 1980
ccctacaacc tcgccatctg cttcgggccc tcgctaatgt cagtgccaga gggccacgac 2040
caggtgtcct gccaagccca cgtgaatgag ctgatcaaaa ccatcatcat ccagcatgag 2100
aacatcttcc caagccccag ggagctggag ggccctgtct acagcagagg aggaagcatg 2160
gaggattact gtgatagccc tcatggagag actacctcgg ttgaagactc aacccaggat 2220
gtgaccgcag agcaccacac gagcgatgac gaatgtgagc ccatcgaggc cattgccaag 2280
tttgactacg tgggccggac agcccgagag ctgtccttta agaagggagc atccctgctg 2340
ctttaccagc gggcttccga cgactggtgg gaaggccggc acaatggcat cgacggactc 2400
atcccccatc agtacatcgt ggtccaagac accgaggacg gtgtcgtgga gaggtccagc 2460
cccaagtctg agattgaggt catttctgag ccacctgaag aaaaggtgac agccagagcg 2520
ggggccagct gtcccagtgg gggtcatgta gccgatattt atcttgcaaa catcaacaag 2580
taagctctgc ttttcatttt ctgctcccct gaatgacttg caacacccag cctcaccctc 2640
tggcctaacc cccatctcca ttcctgtgct gcacgtaggg ctcccagctc ccccagccta 2700
acagtttgca tgtggtcatt gctgctgcaa ggcggacagg gctgaggatg ctgctacaag 2760
cctcggggca ggtccaggtc tccagctagc tgccctcgtg ctgtggaagg gtgctttact 2820
gtgtgttccc gcagtgtctg tccacccaga cctttgtggc agtcttacag ctaaaacttt 2880
gaccaaagct ttggtcactt tatgcaacct ggttttgtac tgtttctcag aggtgccttc 2940
ttttttccaa tccatactca aataatagtc tttgatgtct gtcttccttg acccgtgttc 3000
gtgcaaagat tcagagtctg tgtgtggctt ctactaggct gatgttacac caggtgggtt 3060
tattgagata tcatgtgtct gttcctcccc ctgtcctgca ttcactcctg tggaggaaag 3120
gaggccacga tgtccctaag gaaagctttg tcctgagctc ttcattcatt ggctaacccc 3180
tagctccctt ttcttctgcc ctttcacacc aggagaaata attttccatt ttgttcctat 3240
tgctttggcc ttttgtatta ttctaccccc ttagtccctt tgcagatccc cactcctgct 3300
cagcaggctc ttacctctga cccccagctt tcattgtggc tgttagcaac atcctggggt 3360
ttaaactcca cccacgcccg atctggctgt ctagagggat tctacgcctg cgtgctgccg 3420
cctccccaag aggcattcag gttattggag aactaatctc atctcaaggg gccagacacc 3480

aagtcccaaa gcctacagac ctctttccgc caggccctga aacctggccc cgtgccagca 3540

ggatgacaag ccccagggcg ctcctgatga atatggattg gagatgatgt acagttttta 3600

ttcccctctg gcttttgagg aatgaaatga tttgcacttt gaaaacctgt taaccgtagc 3660

ctctggacac tgagactgga aggagaataa aggatgcttg ttgtttttaa acta 3714

<210> 35
<211> 7115
<212> DNA
<213> Homo sapiens

<400> 35
gcggccgcgg gatttggagc ggccggggag gcgggggtgg ccggggccgg cttggaggcc 60

tggcgccacc cttcggggcc tgcaaggacc cagttggggg ggcaggaggg ggccggagga 120

tggttggttg tgggatttct actttgcctt ttcctcctta tgccgcctta gtgaggggcg 180

ggagctctgg cggcagcccc ggggtgggga gacgagctcc ggagtcggaa gagctgggtt 240

ttcttccggg cctagccacc agttggcgga gtgaccttag gcgagtcact ctgtaatttg 300

tctgcgcctc agtttcctcc tctgcctatc aatgtgtgtg gggttgaaat cgctttgtaa 360

actataaagc gtgggtgtac gtaaaggatg gttattgttt ataatttttt ttgagttgta 420

agaaaactta gcagttcccc aatccttggg ttttgaacct gggaaccttg gattggagtt 480

ggggatcccc aaacttcctg aaattgtggg aatgtgcggt ttgggggaat gatgggaatt 540

tgtgggaatg tgcgttttag gggaatgatg atccatcgct agcaagtttt ccaagggggc 600

tgtgacccag aagagttaag aatcacaatt tcttcatgct acagagagga aactgaggcc 660

tagatgtcat ttgggaccct tcacaaccat tttgaagccc tgtttgagtc cctgggatat 720

gtgagctgtt tctatgcata atggatattc ggggttaaca acagtcccct gcttggcttc 780

tattctgaat ccttttcttt caccatgggg tgcctgaagg gtggctgatg catatggtac 840

aatggcaccc agtgtaaagc agctacaatt aggagtggat gtgttctgta gcatcctatt 900

taaataagcc tattttatcc tttggcccgt caactctgtt atctgctgct tgtactggtg 960

cctgtacttt tctgactctc attgaccata ttccacgacc atggttgtca tccattactt 1020

gatcctactt tacatgtcta gtctgtgtgg ttggtggtga ataggcttct ttttacatgg 1080

tgctgccagc ccagctaatt aatggtgcac gtggactttt agcaagcggg ctcactggaa 1140

gagactgaac ctggcatgga attcctgaag atgtttgggg tttttttctt tcttaatcga 1200

aagttaacat tgtctgaaaa gttttgttag aactactgcg gaacctcaaa atcagtagat 1260

ttggaagtga ttcaaagcta aactttttcc ttggccctcc ttgtgttcta attgcttgca 1320

agtgtaatac taggatgtcc aagatgccag tttttgcttc tttgttagtt gtcagctgct 1380

tttatcaaat ttcaggccat tatccaacaa acactataaa aatgtttgaa caattggatt 1440

tcaaacattt tcgttttgtg gagtggtgct caccaagtgg tacagcccta agcaagtgaa 1500
cacaaacaca tttaagtgta ttttgtctga ttagatgtta gccagttatg ctatttcatt 1560
caaatgtctg aaaaaatcaa ttgactattc ccttttccta aagggcagag acagataatc 1620
tcacttccag agaaatgact tggagaaaaa aaagtgttgg tctttttgct cttttgtaat 1680
taaatccgga tgtacctcaa aagacttaag actgtggtga taagatgctt tcctcagcag 1740
aaaggaggga aaaaaaacaa ctggaactca aagcttgaaa ttctgtggca aaacatgaga 1800
tgtccaggat tggaggttga aaagatttca ctacagtgtt ctgcaatagt tggagcagat 1860
aactttcagt gtagccacag ccatggactc cagatttcca gattttcaag acctggacct 1920
ggaacccgaa agagcttgtc acgatgcggc aggaacactg gaggtagatt ttttttattt 1980
tttgaatttt gggactgttg accttgctgt gagaaaagag acaacgactg agcaagcact 2040
accaccagca ctgttactgg gaattagaag acctgagttt ctgtccagac cctcagtgca 2100
aactgaggat gctccatcca aagtgaatta tgtcctgtgc ctcctgattg ctgagtgttc 2160
acctggacct tctgactacc ttccctgtgc tattccatca gcctacagac ctggtacctg 2220
gatttttgcc cgagatgatt cctaccacct tactactgac gaagacaccc attccagtgg 2280
accactgtga cccaggaggc attcagccat catgatgtgg cctttacctc cactcctgtc 2340
ttgttctacc cagattcagc acagcccttt atagtgaagt cagagtcctc aagccaaata 2400
gctaaagctg ttttatcaca acaaaggcct agtttgttcc atgagtgtgc atttcatttc 2460
ttcagttaaa gccttcagag acacacaata aatttggacc aggggatttt ttagttatta 2520
atgctctctg aagaaaggca acatcttttt gagagcagca ttggaccaca ccccacaatc 2580
tcaaatgatt gaaattcatg aacatctagg atcccgtgaa ggtcactgga ccctgttttt 2640
tctacttcaa atcctgtagt agcctactga atgagaaaac atattctgac ccattgggat 2700
caaatcaaag gcacagtgaa ctcctcatag catcttcttt ggaattactc aggaaccaga 2760
actttttaca caaatgtaag aaattctacc aaggagtccc cttacctaac agcatctcac 2820
aaggctgcac cagattccag aaaaggcttc tcttgataca tcaaggtaga acctctatgc 2880
attttgtgac cgacttattc ttagatcatt ggttttccaa aggctttgtg gccatgaagc 2940
cctttgagtg aaaactgtgc agaagcccag agtaaaagtg aagctgctct ggatgaagta 3000
gtgaagcaag agtaggggcc tgaatcctgc tacaactatc ttcctttacc accgtggtga 3060
cacctaaggg gacttcctta caacaccttg aactcttccg aacacagttt gaaaaccact 3120
gccccagaca gcaatatgtt tgacctgaat ggcattccaa tcttttctgt acctccactc 3180
agcacagttc atgttcagta gatgctgaac attcttagaa atactgtgtg tgaacttaga 3240
aaagtgcaag aagacaggca tgtctttgac cccaggaatg atcatttgct gaagatggtg 3300

tcaagtgaac ctagattaac agccctccac tccagatgga tatccagtga ttcctagaat 3360
gggatatagc cagagaacaa ttctatgcac cctacactga cagactccct taagcaacac 3420
cagatgctct actggtactt gaagtacatg actttgaagt cttgaccctc catgaatacc 3480
tgaattatca gcaagcgggt tttgaagctg gtgcctcatt gaggccatat tagagcaact 3540
tgtacatttg acctcttgtt atcagccatg gtactctact tcgtgtgcaa gagataacta 3600
tgaaagccaa attcaaatac tggcaacatt tcctaaaggg gctcaatatc tatcattcgt 3660
cttcttttcc aaactacaca tcactgtatg actcaaccag tagcagttat attgcccctt 3720
ggtttttatt cagtttaact actgtttcca agataaatga gctaataagc tttaaaaaaa 3780
aaaaaaaaaa aggctgaatt ctttttcttt catcactggc atatctgcct attctccaga 3840
attattatga ctattcagct cactttaaca gttgaacttc aagcgacaat ctttgaacac 3900
cccttctcat gtgatttaaa atgaaaccat ttggaaaagt ttcttctagc cagtaataga 3960
tttttttttt aattgctctg ccttgtgccg agagatgttc ttttaagatg aatcttttga 4020
tgtctgatac caccaaatat aggtggtagg gagagttgga ggctggccct ttgagcaggc 4080
cattagctta cttgctgggc atttccgata gcttattgcc tacctttttg ctggaaacaa 4140
actgatttga aaaacaaaat ctatgaagac tgcagctaag gattttatcg gtagacttaa 4200
gagcttttgt ccttgtggat attttagtgg aaccacatca gtctcaatac tgtcatttta 4260
cactgactca gagcagctga cttcattcct tgccatgata tatatttaag gcaggcattg 4320
taacagacat aaagacaact tatctgtttc agcaggaagg attcagttta tgaactctca 4380
gaccagatca tgttgaacaa ggagactttg atgtgtgtca tgagaaaact cattctttac 4440
ttcccagtca atttaaaggc cagctatcct gagctactcg aatgaatgca ctggttaaac 4500
attggaaata gtttgtttat atccttgtct ctctctaggc caattgtgat tacatgactc 4560
gactctacat ctcgtcaaac aaggcctagg tctggttgct gtagactgct cgccctcaac 4620
aaataaaatc tggttgacta gcctccttgt atatacaact attatttgtt aagaagaaat 4680
tatcgtcaat tttctactac cttccaattg tcagctcttt ttttcctctc tggtttttcc 4740
tatactttac agaaaaagac attgatctat actgccattc cctctaatcc tgccatactc 4800
agtcaaaagg aatgacttaa gatgaagatg atcatctgct cgagtctaaa atatacattg 4860
tatataagaa ttggtgatta gaaaagcaaa aaacctaaaa cttaaatcta ggagtctgta 4920
tactgtctcc atgtctccat gcctcagatc tcatctaaat ctttgaacag caccattcaa 4980
ccaatctgag gccttgactt gcttgtaaga tgattctcag agatcggctg agttaaaaaa 5040
gatgacgact tgattaccaa agaaagtagg gccaactttg acaaatctgg ctctgctgac 5100
cctgtcactc ccagatgtag catagactcc taaacagaac ctcaagtctg attgaggata 5160
aggccttctc ctgagctgaa agttctttgg cagatgagca agaaactgaa agctgatgta 5220

cctgactggc tctgtaagat cagaaaactg tatccagaat aagccctatg gattaacccc 5280
tgagtaccca gagtaaaaac taatttacag aacttcctta ttgatctgct ggttcttcca 5340
gatcatattc tggctattgg tatggctggc ctttctgaag gtaccctgct tgtctatttt 5400
cctgactcag ctcttgcctg ccttttttcac atgttgctgc aattagactc accgtgagga 5460
ctacagtcaa tttcagtcta tcttgtgccc aatacaacaa ggatttttaa tagtaacaac 5520
ccacacctca cccactagga ctcaatgttc acaacaggaa ggaccattgc tgcatactcc 5580
ttgaccagca actttttttga agatattttt aagtgcagag taggcctcta ttcctgtatg 5640
taattgttca ttttcagcac ctggaacctc atctatcggg tctggaagga atacagcagt 5700
tcgaaagccg cgtccatttc tctccttcag tagtgcagaa atgagtccga ttcaccagta 5760
cacacagaac tgtaccagtt caacctagca aaagaagaaa agtttccact gtacttaaaa 5820
tttacagctg actcaaattg cctcacagaa ttatttgatg tagaaggcta gttgtcttac 5880
ttcagatcag caggacagtt gggctctcag actcatgacc actgagtttg cttgtgttga 5940
aactgtggtt tcatccaaca tatgctattg gacatgatta ttattccatt caaatggatt 6000
acagacttct tgaggacagg acaaacttat ctctcatggt gttttttttag aatactttta 6060
taaccaagga agaaaccatg ccagctgtta ccattcaact tcttaagcag agattaagct 6120
ttttcatatc tgttcttatc ctggacatca gtagttttta attgcccagc atccgttcca 6180
tcttgtaaca actccctgat gtttcttaaa accacctctt cctattttca gtctgtggtt 6240
tggacagtct gacccaacct tgagctttgt gggtgaacat gtaattcaga cctcatcaat 6300
cagcaaatcc atctgaactg tggaggagaa gctctcttta ctgagggtgc tttagctttg 6360
taggatgaaa acctcaaact aacagggcct accatgtaga gaatgaagcc agtgcagggg 6420
aaagcagagc caaaatatgg agagacttga atcctgatga cagcgtttgt gcccctggat 6480
ccaaccgtgc ctgaagctag aatatcccct ggacttttca gttatgtgaa ccaataaata 6540
cccttttttg cttaagttac tttgagttgg gtttctgtta cttgaaattg aatccacact 6600
aatatatcta ccaacattga gacttgacag atccaagtat ttattaagct agaggtcatg 6660
gtcactgaaa ttactttcca aagtggaaga caaaatgaaa caggaactga gggaatattt 6720
aagatcccac agaagcgtaa aaatgacatg gtagaaagta atagaaaacc taaatgtctg 6780
tcattaaagg ataggttaag gtgtggttca gccatatagg aatatctcgt atctgttaaa 6840
atgaataaag tacattcatt gtgtatggaa aaatggccat gatacattag gtgaaacaag 6900
ttattaatag aaaagtgtac agtgtgaact cattttaaaa tgtgtgtgct tatgtttata 6960
aatgcataga aaggtctatt cacagctttc tttgaacagt gtagatcaca tgaaactttc 7020
aactttatac atttctgtat taatatttta cactacccac attattttta aactttattt 7080

taaataaaga atttttaaaa ttaaaaaaaa aaaaa 7115

<210> 36
<211> 1209
<212> DNA
<213> Homo sapiens

<400> 36
gacccgggag aaggagggcc aagatggcgg aagcggagga gtctccagga gacccgggga 60

cagcatcgcc caggcccctg tttgcaggcc tttcagatat atccatctca caagacatcc 120

ccgtagaagg agaaatcacc attcctatga gatctcgcat ccgggagttt gacagctcca 180

cattaaatga atctgttcgc aataccatca tgcgtgatct aaaagctgtt gggaaaaaat 240

tcatgcatgt tttgtaccca aggaaaagta atactctttt gagagattgg gatttgtggg 300

gccctttgat cctttgtgtg acactcgcat taatgctgca aagagactct gcagatagtg 360

aaaaagatgg agggccccaa tttgcagagg tgtttgtcat tgtctggttt ggtgcagtta 420

ccatcaccct caactcaaaa cttcttggag ggaacatatc tttttttcag agcctctgtg 480

tgctgggtta ctgtatactt cccttgacag tagcaatgct gatttgccgg ctggtacttt 540

tggctgatcc aggacctgta aacttcatgg ttcggctttt tgtggtgatt gtgatgtttg 600

cctggtctat agttgcctcc acagctctcc ttgctgatag ccagcctcca aaccgcagag 660

ccctagctgt ttatcctgtt ttcctgtttt actttgtcat cagttggatg attctcacct 720

ttactcctca gtaaatcagg aatgggaaat taaaaaccag tgaattgaaa gcacatctga 780

aagatgcaat tcaccatgga gctttgtctc tggcccttat ttgtctaatt ttggaggtat 840

ttgataactg agtaggtgag gagattaaaa gggagccata tagcactgtc accccttatt 900

tgaggaactg atgtttgaaa ggctgttctt ttctctctta atgtcatttc tttaaaaata 960

catgtgcata ctacacacag tatataatgc ctccttaagg catgatggag tcaccgtggt 1020

ccatttgggt gacaaccagt gacttgggaa gcacatagat acatcttaca agttgaatag 1080

agttgataac tattttcagt tttgagaata ccagttcagg tgcagctctt aaacacattg 1140

ccttatgact attagaatat gcctctcttt tcataaataa aaatacatgg tctaaaaaaa 1200

aaaaaaaaa 1209

<210> 37
<211> 3022
<212> DNA
<213> Homo sapiens

<400> 37
cggagtcaga aaggcgaggg gcgccgggaa ctggcgtgtg ggactccaga caggagaggc 60

tgcgccttcc ccgcaccggg accttcgcga cacaccagat cctcgcccct ggctcgcgcg 120

aacgcacagg atgaccacca ccctcgtgtc tgccaccatc ttcgacttga gcgaagtttt 180

atgcaagggt aacaagatgc tcaactatag tgctcccagt gcagggggtt gcctgctgga 240
cagaaaggca gtgggcaccc ctgctggtgg gggcttccct cggaggcact cagtcaccct 300
gcccagctcc aagttccacc agaaccagct cctcagcagc ctcaagggtg agccagcccc 360
cgctctgagc tcgcgagaca gccgcttccg agaccgctcc ttctcggaag gggcgagcg 420
gctgctgccc acccagaagc agcccggggg cggccaggtc aactccagcc gctacaagac 480
ggagctgtgc cgcccctttg aggaaaacgg tgcctgtaag tacggggaca agtgccagtt 540
cgcacacggc atccacgagc tccgcagcct gacccgccac cccaagtaca agacggagct 600
gtgccgcacc ttccacacca tcggcttttg cccctacggg ccccgctgcc acttcatcca 660
caacgctgaa gagcgccgtg ccctggccgg ggcccgggac ctctccgctg accgtccccg 720
cctccagcat agctttagct ttgctgggtt tcccagtgcc gctgccaccg ccgctgccac 780
cgggctgctg gacagcccca cgtccatcac cccacccct attctgagcg ccgatgacct 840
cctgggctca cctaccctgc ccgatggcac caataaccct tttgccttct ccagccagga 900
gctggcaagc ctctttgccc ctagcatggg gctgcccggg ggtggctccc cgaccacctt 960
cctcttccgg cccatgtccg agtcccctca catgtttgac tctcccccca gccctcagga 1020
ttctctctcg gaccaggagg gctacctgag cagctccagc agcagccaca gtggctcaga 1080
ctccccgacc ttggacaact caagacgcct gcccatcttc agcagacttt ccatctcaga 1140
tgactaagcc agggtaggga gggacctcct gcctactcca gcccctaccc tgcacccaca 1200
tcccataccc tcttctccct acccatccca ttccccacag gccctacatt aacaaggtta 1260
agctcaaccc ctttccccca gcacctcaga atgtgccctc cctctccccc tcataacccc 1320
acctaacata aggacaagtc aatttgtcag tagcttcttc tggcttgaaa ccccctccct 1380
ggattttata gcccacttac catgcataac agacaagtcc catattttgt cagtagatgc 1440
cttttttttt ccggcttaag ccttaagtgc caaatcacaa gagaaaaagc agtaacagtt 1500
tacagaagca acttagtgcc ttgtaatcta actttgtcac tgtgactaca ttacctcttc 1560
agcgccagag ggcacccgtg ggcctcccgg agcctctgcc catggcgggg tggagacccg 1620
gaaccagcag ccccctccac tggcgacaca actgcacctt ccctcatttc agtctcccgc 1680
acacttattc ctcctcccct cttcccggtg gcacctctcc acctgtaccc gccccccac 1740
ccccccacc cctgcccctt ggaagagttg ttgccagacc agggttttgg gggaaacctg 1800
tcttgacatt caaaaccttt ttcttcccga tctgaacccc tgttgactaa tcttgcctgg 1860
gtttgtgtag gtctgcagga aggaaggctg aaaaagcgga cgaagatttt gacttaagtg 1920
ggactttgtg atttaatttt ttcttttttt taagtgggga ggaaggggaa gctagatgga 1980
ctaggagaga cttgattttg gtgctaaagt tccccagttc atatgtgaca tctttttaaa 2040

aaaaataaca acaaaaaaaa aatgagagaa aagctaaaaa aaaaaaagta aggggtgagc 2100

agttaatggt attcattcca catacaatat ctgtgtaaaa cgatttcctg tagaagtagc 2160

tttaatggtt tttgctctag aataccgtag gtctatcctt agagcactca cgccatgctt 2220

tcttccctgg gttttaaact tcatataact ttcagaaatt ggagagcaaa aattttgctt 2280

gtcactgcac atcaatataa aaaagcttat ttaacttatc aaaacgtatt tattgccaaa 2340

ctatgctttt ttttgttaat tttgttcata tttatcggga tgacaaatcc atagaatata 2400

ttcttttatg ttaaattatg atcttcatat taatcttaaa attttgtgac gtgtcttttt 2460

cctttttttc cacagtttta atatattatt cttcaacgac attttttgta actttacact 2520

tttttggtta ttttatttta aaaaaatgaa aaattaattt aaaaaaatgc aaaaaactgt 2580

tggattattt attttagaaa ttcccccctt tgtgttggac tgcaaattga gtttctttct 2640

ctttaggcct ttcacaacta ggactgagaa tgtatgtaaa agttctgtga cagtacagaa 2700

ggaaaacaac tttttatgta tagcttctaa aaggggaaaa aaaaaaaaag agaaaccctt 2760

tgacttccac gtgcccatct caagacattc cactcacaga tttgaggttc tggattccag 2820

gtctggagtt ttccaatgtt aatgtaaaca gaactggcac acacacatta agatgaatgt 2880

aattattatt cctcttgctg gtcactaccg tcgctttcta tttctctttc tttgtgtgaa 2940

tttatttaaa agaaaaaaaa actttttgta acgactattt gcagtttaaa aatcaataaa 3000

ccccgttttt tcaagaaaca tt 3022

<210> 38
<211> 7756
<212> DNA
<213> Homo sapiens

<400> 38
agtggggcag caaatggaca gggtgggtgg cggaaaaggg cccgggggaa gttattacag 60

ggtgtcctct tccgccgcca gaagccggaa gttgtgtccc ggacgtgtca accggggtct 120

gagtgctcag agtacagctg caaccgcgac catgggcggg aagaacaagc agcgaactaa 180

agggaacctg aggccttcaa acagtggccg agctgcagaa ctccttgcca aagaacaggg 240

aacagtgcct ggatttattg gttttggaac atctcagagt gacctaggct atgttcctgc 300

tattcaagga gctgaagaaa ttgacagtct tgtagattct gatttccgaa tggtgctgcg 360

gaaactttca aagaaagatg tcaccacaaa attaaaagct atgcaggaat ttggaaccat 420

gtgtacagag agagacacag aaactgtgaa aggagttctt ccatattggc caagaatttt 480

ttgcaaaatt tcacttgatc atgaccgtcg cgtccgagaa gccacacaac aagcttttga 540

aaaacttatc cttaaagtaa agaaacagtt ggctccctac ttaaaaagtt taatgggata 600

ttggctaatg gctcagtgtg atacttacac accagctgcg tttgcagcaa aagatgcatt 660

tgaagcggct tttcctccaa gcaagcaacc tgaagccata gcattttgta aggatgaaat 720

tacaagtgtg ctgcaggatc atcttataaa agaaacacct gatacactca gtgacccgca 780

aactgttcca gaggaagaaa gagaagctaa attctaccgg gttgtaactt gttccttatt 840

ggcattaaag agattacttt gccttttacc tgataatgag cttgattctc tggaggagaa 900

atttaagtct cttttatcac agaataagtt ttggaagtat ggaaaacaca gtgtacctca 960

gattcgctca gcttattttg agttagtctc tgcattgtgc cagcgcattc cacagttgat 1020

gaaagaggaa gcatccaaag tgagcccatc agttctactt agcattgatg acagtgaccc 1080

aattgtctgc ccagctctct gggaagctgt actctataca cttacaacta ttgaggactg 1140

ttggcttcat gtaaatgcaa aaaagagtgt gtttcccaag ctatcaactg tgattcgtga 1200

aggtggtcgg ggtctagcta ctgtcatata tccttacctt ctgccattca tcagcaagct 1260

ccctcagtcc atcacaaatc caaagttgga tttcttcaaa aatttcctca cgtctctagt 1320

tgctgggctg tcaacagaga gaactaaaac cagctcttta gagtcctcgg cagtaatatc 1380

tgcttttttt gaatgcttac gttttataat gcagcaaaac ttaggtgagg aagagattga 1440

acagatgctc gtcaatgatc agttgatccc ttttattgat gcagttctca aagacccagg 1500

attgcaacat gggcagctat ttaaccattt agcagaaact ctaagttcct gggaagccaa 1560

agcagacacg gaaaaagatg aaaaaacagc tcacaacttg gagaacgtac tgatacattt 1620

ctgggaaaga ctgtcagaga tctgtgttgc gaaaatcagt gagccagaag ctgatgttga 1680

gtccgttttg ggtgtatcta acctattaca ggtgcttcag aagccgaaga gctcattgaa 1740

gtcaagtaaa aaaaaaaatg gtaaggttag atttgctgat gagatacttg aaagcaataa 1800

agagaatgaa aaatgtgtat cttcagaagg agagaagatt gaaggctggg aattaacaac 1860

tgaaccttct ctcactcata attcttcagg ccttttgtct cctctaagga aaaaaccttt 1920

ggaagactta gtctgtaaac tcgcagatat aagtattaat tatgtcaatg aacgaaagtc 1980

agagcaacat ctaaggtttc tttctactct gcttgactcc ttttcttcaa gccgagtatt 2040

taaaatgcta cttggtgatg aaaaacagag tattgtccaa gccaaacctc ttgaaatagc 2100

caagcttgta caaaaaaatc ctgcggtgca gtttttatac cagaaactga taggttggct 2160

aaatgaagat caaaggaagg attttggttt cctggtggac attttgtaca gtgctctccg 2220

gtgctgtgac aatgatatgg aaagaaaaaa agtcttggat gatctaacca aggtggactt 2280

gaaatggaat tctcttctta agattattga aaaggcatgt cctagttcag ataaacatgc 2340

tttagtaact ccttggctca aaggcgatat ccttggtgag aaattggtca acttggcaga 2400

ttgtctttgt aatgaggact tggaatccag ggtatcttca gaatctcact tctcagaaag 2460

atggactctt ctaagcttgg tattatccca acatgttaaa aatgattact tgattggaga 2520

cgtatatgtt gaaagaatca ttgttagact tcatgaaact ttattcaaaa caaagaaatt 2580

atcagaagct gaaagcagtg actcatcagt gtcttttatc tgtgatgtgg cctataacta 2640
tttcagctca gcgaaaggat gcttgctaat gccatcatct gaagatttat tattaactct 2700
ctttcagtta tgtgctcaga gcaaagaaaa aacacatttg ccagattttc ttatctgtaa 2760
actgaaaaat acttggctct ctggtgtaaa tttattggtt catcaaactg acagttcata 2820
taaagagagt accttcctac atttgtctgc tctgtggctg aagaaccaag ttcaggcttc 2880
atctttggat atcaacagtc tccaagtcct cttgtctgct gttgatgatt tgctaaatac 2940
acttctagag agtgaagatt cttatcttat gggagtttat attggaagtg taatgccgaa 3000
cgacagtgaa tgggaaaaga tgaggcagtc tcttcctatg cagtggttac atagacctct 3060
tttagaggga agattgagtt tgaattatga atgtttcaaa acagatttta aggaacagga 3120
cataaagaca cttcccagcc atttgtgtac ttcagcatta ttgagcaaaa tggtcttaat 3180
tgcactgaga aaggaaacag tcttagaaaa taatgagctt gagaaaataa ttgcagaact 3240
gctttattca ctgcagtggt gtgaagaatt agataaccca cctatttttc taattggatt 3300
ttgtgaaata cttcaaaaaa tgaatattac gtatgataac ttacgtgtac ttggtaatac 3360
gtcgggcctt ttgcagctgt tatttaacag gtccagagaa catggcacac tgtggtctct 3420
tattattgct aagttgatcc tttcccgaag catttcatct gatgaagtaa aaccacatta 3480
taagagaaaa gaaagttttt ttccactaac tgaaggcaat ttgcatacca ttcaaagtct 3540
atgtccattt ttgtcaaaag aagaaaagaa agaatttagt gctcaatgta tacctgctct 3600
tttgggctgg actaagaaag atctttgcag cactaatgga ggttttggac atcttgccat 3660
tttcaattct tgtctgcaaa ccaaaagtat agatgatgga gagctattac atggaatatt 3720
aaaaatcata atatcctgga agaaagagca tgaagatatt tttcttttca gttgtaatct 3780
atcagaagca agtccagagg tactgggtgt aaatatagaa ataatccggt ttctttccct 3840
atttctgaaa tactgctcat ccccttttggc agagagtgag tgggacttca tcatgtgctc 3900
catgttggct tggttggaga caacaagtga gaatcaggca ttgtattcta ttccacttgt 3960
gcaactgttt gcctgtgtca gctgtgattt ggcctgtgac ctcagtgctt tctttgattc 4020
cacaactctg gataccattg gcaatcttcc tgtaaatcta atcagtgaat ggaaagaatt 4080
tttttcccaa ggcatccaca gtttgctttt acctattttg gtgactgtta caggagaaaa 4140
caaagatgtg tctgaaacat cctttcagaa tgcaatgctg aaacccatgt gtgaaacatt 4200
aacgtatatc tcaaaggaac agctattgag tcacaaactt cctgcaagat tagttgctga 4260
ccaaaaaaca aacttaccag aatatctcca gactttgtta aatacattgg ccccattact 4320
cctcttcaga gctaggcctg tgcaaattgc tgtttatcat atgctataca aattgatgcc 4380
tgaattacca cagtatgatc aggataatct aaagtcatac ggagatgaag aagaagagcc 4440

agccttgtca ccaccagcag cactgatgtc tcttcttagc attcaagagg acttactaga 4500
aaatgttttg gggtgtattc ctgttggaca gatagttact attaaaccac tgagtgaaga 4560
cttctgttat gttctgggat accttctcac ttggaaatta atactaactt tcttcaaagc 4620
tgcatcatca cagcttcggg ctttgtattc catgtatctt cggaaaacaa agagtttgaa 4680
taaattgctc tatcacctgt tcaggcttat gccagaaaat ccaacctatg cagagacagc 4740
agttgaggtc ccaaataagg accctaaaac attctttact gaggagctcc agctgagtat 4800
tagagaaaca acaatgcttc cataccacat tccacacttg gcttgttcag tctatcatat 4860
gacattaaaa gacttgcctg ccatggttag gttgtggtgg aatagcagtg agaagcgtgt 4920
tttcaatatt gtggatagat ttacaagcaa gtatgtcagc agtgttcttt cttttcaaga 4980
aatatcttct gtacaaacaa gtacacaact atttaatggc atgacggtta aagctcgagc 5040
tactactcga gaggtaatgg ctacttatac tattgaggac atagttattg aacttataat 5100
acaactgcct tcaaattatc cactgggttc aataatagta gaaagtggga aaagagtagg 5160
agtagctgtt cagcagtggc ggaactggat gctgcagtta agcacttacc tcacccatca 5220
gaatggaagt attatggaag gcttagcttt atggaaaaat aacgtagaca aacgttttga 5280
gggtgttgaa gattgcatga tctgtttctc agtcattcac ggtttcaact attcccttcc 5340
caaaaaagcc tgtagaacat gcaagaaaaa attccattca gcctgcttgt acaaatggtt 5400
tacatctagc aacaaatcca cttgtccact gtgtcgtgag acgtttttct gagatttttt 5460
tcactggaag ggatccctga agtacatcaa acaaaggcat tggatttgga tccgtctgaa 5520
agtgtggatg tggggaagcc agtgagcatt acttttaaat aggaccttct ctggaaaatt 5580
attttggtta atgtaataat cctaaaatca ggtttactta actttagatt gctttgtaaa 5640
tgtttggaaa ctttctttta caaaagaata ttacatattt gagagaaaat atttctatta 5700
atggtttaat ttctttgtat atttaaaaat aaatatgaaa aacccctaaa ttacagaatt 5760
ggaatttgtg aaaagattgc acaactatat tattgataaa cccattccgt ttgttgatct 5820
gtgttttaga atagtccaag tgaagcaaaa cgaactgaga gagctgattt acatctatag 5880
atattttaag ccttatttat tagttatgac aataaattag tattctgata tttgtagatt 5940
attttaatga taaaaatgac actaagctat taatacggct tgtcttaaga tatctggagg 6000
catgctctga aatcctatat aaaagagttt aaactgaact ttaaggtgcc tccatttatc 6060
aagggttatg aaactcagaa aattaaattc ttgatgctga cctttttgct tgtgggagaa 6120
ttatttctat atattagtga aatttgaatt gtaaacttgt cagtgaattt atcactctga 6180
gcgagtctct agagggtgag aatttgagag aaatagtaat ttgattattt cattcatgaa 6240
gcggagtggt agttatcctt ctattgagag ggaagaaata aatgagtatt actgaaactt 6300
aaagatcagt ggaaaaaaaa aacatttaaa aatgttgtag gagactttca attttggaag 6360

caaactgagc tattcttacc agccaaagtt atactaaata agagacttgg gggagcaaat 6420

gtttttcagc cttcagaaat gaaagttaag gattttagca ctaggtaaaa ttcagtataa 6480

taggctgaag tggagtaagt gaaaacctgc cttttgccac tcttaaaaat tgtgcccaaa 6540

atataaaagt gtggaacttt agaacttgga ataattttat tgcagtcttc cattacatgg 6600

aaatagcata tctaatatct aggttacttg agagaccagc taatcatctc tgttgcacat 6660

gtttaattgg caaaaagcaa ttcatgataa ataaaattac tgctttccat ctactgggta 6720

aaatgactat tgaaatagta tgaatgtggt cagaggatta tagttgagag tgaagtacta 6780

tgtgtgagtt atagatctct cgaattatat ttatagatgc agtgtcctgc ccagttttgt 6840

ttgcctccta cattttactg taaaatattt attgtcttct agccttgagc ctctgagggt 6900

cagtaagtga agtacagata gcaaaatttt actacctcgt taaccctttt cttaaaatat 6960

tctctatctt tctatgtctc tttctgacat agtaatccca aaggattgtg ttactccccg 7020

tgaaagttat tacttttcct ttaaaaatgg ttttataata agactgttta aaacctttcc 7080

agtattggta catcttggct tctggcccaa accccaagca gaaaagaaaa tggaataatg 7140

gagcattgtt tttccacatt agtattaggg catcagattc ttggtgaaac actataatta 7200

agtagttata attaaataac tgttcttcat acttaggact cttaatacat ttctttaaga 7260

cttgtaagta taattgtaaa gcttgttaac tgtttatata ctaaagaaaa agctttacaa 7320

gataagactg aaattcagaa taccacatct gtacagcgtt gacaactcta actaaaagga 7380

ttacagccct taaaatttta gcttaaggac attttaaaat tgcatgagat tagcaaaatt 7440

attaatttaa aaccccaaag taaattgcaa attttaaaat ttatttttga ctcttttatc 7500

acatgctcca atgtatgtac ataagtgtct gtggcttaaa taatatgttg ttatttgata 7560

catttttgtg gtgcaagtaa ttaatgttat tatttgattg ttataccata tgtgcccatt 7620

aaaattcttt ttaattaaaa atatttcaaa tatgcagaaa aataataata taaatgccct 7680

tgtacccttc acatactatt ttgccatatt tgcttctgat ttttaaataa ataaaatgtt 7740

actcttttgt acttct 7756

<210> 39
<211> 601
<212> DNA
<213> Homo sapiens

<400> 39
ggaaggtcag tgaatgaaga ttaaatagac tgtctttctg ttctttgtct tctttctgac 60

aagaacagct gtgacattct caatgacaaa gaaggaacaa tttgcagaga cgctgggcca 120

aattttcaaa attgagttcc taatttacac atgaaaaata tgtgaccttg aggatgatta 180

ttggaacaaa cacagatttt acatgtgtgt ggttgggagt gtttgcatca ctgcagttct 240

gcctttgcac aaatgcaccc aagctgaagc tcactggaca gatgcaattc ctagccatgc 300

rtgagtcctt gggcagctaa tgtttcagct ttgaaccttt ctctttcaat agtaaatttc 360

caaacacaaa gtggatcata gacacataca aatgtacttt tctacttttt caaagttttg 420

gtgtagattt gtagtatggc tttaatgtgg tgtacctatt gctgtgcagt aatgtctggc 480

aatgggtttg cctgagttta tagtgagaat gtctttaaca tccatgagac ccagggatct 540

aaaaaattgt ttagaaaata gctcagaatg aggcctggta tggtggcttg cacttgtagt 600

c 601

<210> 40
<211> 1000
<212> DNA
<213> Homo sapiens

<400> 40
ctactccgtt acctcttgca cagacttgct ccccactggt ctccttgtct tagttttctc 60

ccctgttcta gtctatgcta catacagttg acaaggtacc ttactttaaa acaaaagtaa 120

aacaagctga tcatactgct ctcctagtta aacaaaaata ctctaacagc tctccattac 180

ccaaaggaaa agctcttaac tgcttcatat cgcatacaag ttatttcaga gtctaccttg 240

ttacccgtat ctctgattac tctccatctc ataccttaaa tttccagaca cactcagctt 300

tgctaggtct tgggaattga aagtatgtat ttggagttca taaatgttca ctgaaccatt 360

taagaaaagt gcttgtttac aaacaaatga aactaaatac agacttagaa ctgcaacata 420

tgagaaacaa aaaaaaaaat tctttacatg tactccttta aggtatggcg cctatgtgga 480

aacttttata gggagtagcr ttcctacacc tggctgtgca tgagagccac ctgtgaccca 540

agcccacact cctttgaggt tctgattcag taggttttta aaagctctac cggtgactct 600

gacatagacc cagagttaag cttcactcta ccagtgaata gactgtatat ccttcagggg 660

aaaaaaaaaa aactctctct ctctctcttt ttttttctga agcagaatct cattctgcca 720

cctaggctgg agtgcagtgg caccatgtcg gctcacagca atgtctgcct cctgggttcg 780

agtgattctc gtgcctcaac ctcccaagta gctgggatta caggcctgtg tcaccacgcc 840

cagctaattt ttgtagtttt ggtagagatg gggttttgcc atgttgacca ggcttgtctt 900

gaactcctgg cctcatgtgt tctgcctgcc tcagcttcca aaagtgctga gactataggt 960

gtgaaccacc atgcccgcca aaaaaaaatt ctttaaatat 1000

<210> 41
<211> 1001
<212> DNA
<213> Homo sapiens

<400> 41

ctagttctag ccaccagata gaaacaaaaa ccttaagtaa aatacgtcaa cattcagaag 60

ttcaaataac ctctatcacg ttttacgcaa tgtctagcac tctatcaaag atcacaaggc 120

acagacaaaa acaataaaaa acaagagaaa caatataaaa tagaaagaga cccacaggag 180

attaacattt aaaagattag cagacagaat tagactataa ttaatatgtt ctaggatatg 240

agcctaggca gcgaagtgag accctatctc ttcaaaaaat caaaaaaatt agccaggcat 300

ggtaccacac gactgtggtt ccagctactc aggaggctga agtgggaaga tcacttgagc 360

ccaggaggcc ggggctatag tgagccatga tcacaccact gcactacagc ctgagtgaca 420

gagcaagacc ctgtctcaaa aataaaaata aaaaataaaa ataaattatg ttcttggata 480

taaaatacaa gatttctgga yaatatcttt aaaaggctaa aaggaatcac taacctagaa 540

ttctttaccc agcaaatata ctgttcagaa gtgaagtgag ggtcagacat ggtggctcac 600

atcagtaatc ccagcactat gggaagccaa ggcaggagaa ttgcttgaga ccaggagttg 660

aagaccagcc tgggcaatat agccagaccc catctctaca aaataaaatt gttttaatta 720

gccaggcatg atggcatgct cctgaagtcc tagttactca ggaggctaag gctggaggat 780

cgcttgagcc caggaattca aggttgcagt gaactgtgat cacaccactg cattctagcc 840

tgggcaaaag agcgagaacc tgtctcagaa aaaggaaaaa agaaaacatg aagtaaaata 900

acaacatttt caccaaaaaa aaaaaaaaaa aagacattat catcataccc accacacata 960

aggaaatact aacagatatt cttcagacaa ctgagaaaca c 1001

<210> 42
<211> 542
<212> DNA
<213> Homo sapiens

<400> 42
tatttattta gtgttgataa cacttttttt ggagtaattt catctagttg catagtttca 60

tgtcttttgg tgttctcatt gccttgaatg ccatcctttt ttttctatct gtacaggtac 120

taaaaatggc cttttatttt tattccattt gttttctcct ttgtctcagt cacagtgtag 180

ccctttgcta gaagagcccc rtggagttag ctaaattgta gctgtctcag ggctttccat 240

gttagatttt tgtatcattg aggttttgag tcttacctgg gatagagacc atcttgttca 300

tctctatttt taacaatact tacttttaat ttttggcatt aaaaatttga actctcattt 360

taagatgttg aaaaatacag aaaaacattt tttaaagaat ataaagtaag ccataatccc 420

attgtttggc aacaaatacc ggtaaccttt taatatattt tcagtttctt ttatatggca 480

gaaatttata tgtaaataaa cagtgaccag atagacattg ttttctctca tttctgagca 540

tt 542

<210> 43

<211> 687
<212> DNA
<213> Homo sapiens

<400> 43
atatttactt ctccacctcc tgcaactatt ttttaaatac ttcctgaatc atctcaattt 60

ttccaagtaa aactatacat attaggtttt aaaatatctc atattcctat ggcacttgtc 120

ccctttacaa ggtactttca tttaatctca ttcaaactct acaaatgttt gtaaggaagg 180

cagatattta cctacagcta tgcttctcca atctggctgc aacctagaat taactaggga 240

ctttctagaa attccatgct caggccatac cacagaccaa gtaaaccaga atctttgggg 300

gtgggccagg crtgggtatt tttagttgtc ccctccagtg atctgacgag tctaatatgt 360

agcagaagct gggaaccact aactgttcct tttgttcccc cagctccaaa gacacctcct 420

catctccttg tagaaaggag atcaagactc cttattcgtc cattgcagag ctaaggaaac 480

cagggctcag gaaggttaag tggtttgctg ctaagaggaa gggccagaaa cagaatccaa 540

aactccggac ccggggagca ttcaaccctg gcttaatgtg cttctactgc atgccagcca 600

ctgagctatc cattaaccaa gaatagaaaa gacgcaggaa attatgccat tctattacgg 660

cacaggatgc tatggagcct gggctgg 687

<210> 44
<211> 601
<212> DNA
<213> Homo sapiens

<400> 44
actacagact gaaaataaaa ttctggatat ggattaagga aatctgcatt ttacataagt 60

tctccccatt gattcttatg cacagtaaaa gttggaaatt tttgtatctt aaaatgaaat 120

aaatgggtgg agctcaagta tgtgcatttt gaaaagctac cttggtgact ttgtatattc 180

tattttcttt cccccatcag actggtagat tttcaatagt gcacttcttt tgaaccagga 240

taaaatctac tcgtaaggtc attcttgttt tctggcattt gaacgatggt gctttctgct 300

raaacagcat tctaagataa tagggttatt cctgcctctg ccttttccta gatcacttat 360

cttggtctac taataacagg gcatccatca acaaagaaac accaccatct ttcatgttct 420

gttattaatt ggactctctg gactactgtt atctctggta ctctaaaaaa gaaaatttta 480

tagtgtctac tggtgggact aaatatcttt ttacctacct acctataggt aaactgtact 540

ccttcctcca agaggcaaat tataggtggt acacattttt ttttcagatt agttttgttc 600

a 601

<210> 45
<211> 913
<212> DNA
<213> Homo sapiens

<220>
<221> modified_base
<222> (226)..(227)
<223> a, c, t, g, unknown or other

<220>
<221> modified_base
<222> (800)..(899)
<223> a, c, t, g, unknown or other

<400> 45
tcaatgctcc ctggcaggca ggaggacagg tgctattgcc ctgttgggac agatgaaaaa 60

cagacacagg gaggatgagt gatttgccct gactatagag tggcagggcc aaggcagagc 120

ccaggcctcc tgcacctagg tcagtgttcc tcccagttac agtctaaact ggaatggcag 180

gcaaagcccc tgtggaaggg gaaggtgaag ctcaaatcaa agctcnncca gagactttcc 240

agatatctga agaagtcctg atgtcactgc cccggtcctt ccccaggtag agcaacactc 300

ctcgccgcaa cccaactggc tctccttact ttctacacac acacacacac acacacacac 360

acacacacac acacacacaa atccaagaca acactactaa ggcttctttg ggarggggaa 420

gtagggatag gtaagaggaa agtaagggac ctcctatcca gcctccatgg aatcctgact 480

tcttttcctt gttatttcaa cttcttccac cccatctttt aaactttaga ctccagccac 540

agaagcttac aactaaaaga aactctaagg ccaatttaat ccaaggtttc attctatgtg 600

ctggagatgg tgtacagtag ggtgaggaaa ccaaattctc agttggcact ggtgtaccct 660

tgtacaggtg atgtaatatc tctgtgcctc agtttgctca ctataaaata gagacggtag 720

gggtcatggt gagcactacc tgactagcat ataagaagtt tcagcaagtg ggggatcctc 780

tagagtcgcg acctcagcan nnnnnnnnnn nnnnnnnnnn nnnnnnnnnn nnnnnnnnnn 840

nnnnnnnnnn nnnnnnnnnn nnnnnnnnnn nnnnnnnnnn nnnnnnnnnn nnnnnnnnng 900

gattttggat tca 913

<210> 46
<211> 749
<212> DNA
<213> Homo sapiens

<400> 46
ccccatggct tgccatttat tcctttaaag gggaaattc tcagcttgga gtgatttaat 60

cttatgcaac aaatgctgtg gctcctggga cttatgtagt cttggcaggc agcagaatgc 120

cccagaactt atctctgcag gcttacccat aatactgatg tgtttgtttt gtcaccgtct 180

atttttcctg cccattttcc aggaaggccc taagctgtag cacctgacac acactgccaa 240

tgtctagctt catgcacctt ccagctacct tcttcaactc agttacataa atatctttct 300

gcctaggtct attttccagg ccaatggttc ccagccatgg tggtatcaca gcacactggg 360

gtgttgcaat gagctggggt gtaagacatt tgttactaac aacagagttc caaagtctac 420

taatgactta ctttgtaata cataaagaga attcaagtta atccccataa tttgccacac 480

tcaattgcac tgaacatacy ttcttaggat gggctgtgat tcttagggtc ggctctaagc 540

atctttattt tcaaggaagc aatttttagt tttaagggaa aaaaactcat gttgttatct 600

tttatgaatt gtccatctca caagacagtg tagtgtcaag ggttaggaac agtggtctga 660

gttcacaccc cagctccacc gctaactagt gatataacct tgagcaagtt tcctcagcct 720

ctctgagttc tttctcctac aaattgagg 749

<210> 47
<211> 1001
<212> DNA
<213> Homo sapiens

<400> 47
agagaaataa tataccctct ctctagaaat agagagaggg tatattattc tcaaatttca 60

aagataatgc ccaactatac ctaggtagaa catcaaaaaa atgaaataaa aaaggaagag 120

taatgcttaa aaaattctgt acaatgacat ctcctcagaa ttgtaaacta tataagtaaa 180

cttatataaa gctttgtagg atggagttgt aatgaaagaa tcttctttaa agttaaattg 240

tcaatcatct ataaaggcaa cataaaatat tcagtgacaa aagcttaaaa acactgcagg 300

ttagttatct tcatagactt gcaccaactc agttctcccc actttttcac ttgcagtttt 360

caaggataac attgcagcat gctctgggag tgtagcatcc tgagctagag agggactgtt 420

gaaacagctc cagctctgct ccagtgccct gtgtaacacc acgcctttca acattgcagc 480

ccagtgcact gtctcagcct ycagatataa aacccagggc acgctgcttt tcagggtccc 540

taagctgtgg tgcaagtagt gcatgcacag acagtctctg tctatgccaa gcagctttcc 600

tgagccttga ggagctggct ggtaatgaat tctaatcttt tgttgtccct tgtgcctatt 660

tgtgagtaat caatctactt catgtcactt gttgtagagg aagtgttctg actcaccaga 720

gtcaaacagg ttggtaacca gtgaacctgc ttcacacata ctttaattaa aaattcttca 780

tgtacatttc acaataatta aaagatgaat taaaaataaa aattcaagaa tggaaaatcc 840

atgtcattag cagtacagat aacgtcatcc caaatttgaa aaatttgaat taaattaaat 900

ttaaagaata acaagaattt aaaaagctct acaatattgg ccatggctat tgtttaaaaa 960

acttacagtg attacataaa aatatcatgg tattactcca t 1001

<210> 48
<211> 654
<212> DNA
<213> Homo sapiens

<400> 48

```
aaaaatgagg gttaattttc acattattag gtttgtatca tttaaaaaga ggtcacaatg        60
acttagaggt gtagctgtta ttagctataa agttcacatg cacaaactgg ccaagagagt       120
cttaattaca ctgttcatta ttctaggtgg aacattactt agattcagta tacccatttt       180
aattagagag aatgtaatta aagttaaccc atgccttcta cgtcacaggc tgtaaaacac       240
gtttaactaa aataaaaaag tataaaccca aaggcatcca tctcagcatt tctatcagct       300
actgggagct cagcatcttg tggaaagtac aggacttctg cctatccaat gaagtctcca       360
cacacatatg aaagaaagta cagagtataa aatacatcta gtacatggtc aaggaaaatt       420
tcaatgtgtc tagtacgtgg tcaaagaaaa tctcaaaatc tcactggagt agaaagagtt       480
aaagagatat tcgttgatct gtgggaaaaa aagaaggaaa tacagagara aaaaggaggt       540
aatcatataa aacttaaaaa taatattttc atttcaattt gtcctttgtg tagctaaaag       600
tatattaaaa gttttcaata tacacttttt tttaagtaaa agtggggcac tata             654
```

<210> 49
<211> 801
<212> DNA
<213> Homo sapiens

<400> 49

```
agactggagc cgtgatcaag gtgcccgtct atagaaaggg atgacagacc ctgaagcttt        60
gggataccag atgatacgaa tgtctgattc gttctagcaa agagaaatca agagccacat       120
ctgttcctac cttgtctctt aaatgcatta catttccttt ccccaccctg ttttcctccc       180
tcagatatct ccatatacgt cttcctctga ccacacctaa ctcacctcca tgtgccttac       240
agaggccccc tgcgttctgc tgcattgctt ttgcgcaatc ccttgatgat gaagatggtc       300
ccaataatga tgcccaccag acccacagtc aggcccaggg cacacaccac gttctctgta       360
gtctctggga gagggcttgg agcatcaaac tctgggggga rataaggcag agtacatggt       420
aatgagtgtg ggaagaagta acactcagaa tcaaatgtag ggttcttaat aaagagaaaa       480
gttattcata gattaaagaa gaggaagaca gaatgatgta ttgagagaat tacagattaa       540
aaaaaggagg aagctggata gtaggagaag acagttatat taattgtagc agatattatg       600
aaacagttag aacaccgagt ttcacacaag catcatagga ggaaaccttg aaataaagga       660
gattgagtgt tggtccatac cccagtgctt gagaagaggc tcatccaagc cccagtgctc       720
caccctgcag tcgtaaacgt cctcagttga gggcaggaag gggagatagt ggaacttgcg       780
gaaaaggtgg tcttccctgg g                                                 801
```

<210> 50
<211> 801
<212> DNA
<213> Homo sapiens

<400> 50
gccaggggct ccagagtcag ttcctcctcc aacatggctc ctagcacacg cctctgctca 60

tgtctcttct tcagcatggt actcgcattc ctgctacttc ccatttcttc atttctcacc 120

tccaaatatt ataatccttc attgtcacac ccatcaagac taatcccaaa ttttgcctcc 180

accgtcagat ttgctgacac tttaagctcg tggatttctc ctcttttgtt tcatagctat 240

acttggtttc acctctaata atttcaagca tgcatgtctt ttctacctag ttagatcagg 300

aacttctcca agacagggat aatcacaact ttaggcacac agtagatgct ccaataaatg 360

aattgaggag ttggggaact aggacatcca ggaccgtttt ycatacagaa cccatctgtg 420

ttttcttagg cagtcccagc ttaatctctt tcccatttga tcatcagaac tttgtgcagg 480

tctactcatc ttttggctta aaccttaaag actctggact gtgagttcca gctgcctact 540

ctgtcccact cattctactt tgatcttaat ccagtcttac ttccaatctt tttctttctt 600

tgttgaattc ttatttttat tctttttctc ttttgaattg ttggttgttg ctaaaattga 660

ctatattttt atacccagtc tgagccttct tccgtatcca cagttaatag ttcattcaac 720

ttagttggga ctgagctcgt gaaacattga tgtcactgtg ttgttaactg gagcaaagcc 780

agttgagaag ttcaagactt t 801

<210> 51
<211> 998
<212> DNA
<213> Homo sapiens

<400> 51
gtggtggaag ggaagctaaa agaaggggct ggaggttctc agaattcaaa ccacacaaac 60

aaatgaagta ttgaggtccc agacttgatc tgggcccagt gtgaaagccc taacttattt 120

ctccagaaga atatgtcctc tggttttaga cttggcactg tggggagaac cagagtgatc 180

tatggtggat atacacacaa acatagacac acatatttgc atttagtaat ttttgtaaaa 240

tttccatttg cttctctgat cctgtctgta tctttgggaa tagatgtaag aatattacat 300

ctctcaggct tgctctgccc caggtttctg aacgtggaat acatttctcc agggaaactc 360

agtattatga gatttgggag gtggaagtta ggccacagcc atctcaggga caggtttcac 420

agacatgagt tttggcagca gccttgtgtt ctaaagacat ttactcctag gggctctaga 480

ggatctgcaa catcagcaga rgcttcctgt gggttcctga tcttttaaaa ttagggttct 540

gcagtgactt ctgctcctcc agacccccta acagttttaa gggctaattc cctgtaatat 600

attcagttct gcttagactg attacaggga ttcctatttc ttgactgaat tctcatggct 660

atagtggctc gtcaccattt gacatcacca agaagtcctc attcaggtgc ctttggaaat 720

tccctcaaac acacaggaaa ttagagtttg aaagaaaacg gagaaccatg agcactgtcc 780

aaataggaac ttctctccta tcacagagaa agggaactga aagtcatttc tcaagtctcc 840

caaatttagt aatctcacaa gaagaaccaa tcagtgttct aggactaaac agtgtcataa 900

gttgctgagc aacaacttgg attgaagatg ctattataat atatgaaatg tctttgaatt 960

taccatgttt ttctcaagca ccatttaaga acaaggca 998

<210> 52
<211> 1364
<212> DNA
<213> Homo sapiens

<400> 52
caaccacttc aaatcatttc ctcccatatt cttccctatt tcaataagca gcaccaccat 60

ccacctattt atcaaggcaa aatacttaga aataagttac atttaatcca ttaacaagac 120

atgcaaaaag acatcccaag tctgttcact ttatctggat ctgtctttgt cactactaca 180

ctacatgaag ccaaaaattt ktcttccctg gagaattctg ctgttctcca cttgtgaacc 240

ccaacaatcc aatctccaca tagtagctag aattattttt aaaattgaat attatcgggg 300

gacctgcccg gataatcacg tagtttcttt tctattttcc taagcatcgg ccggcttgag 360

aaataaaggg acagagtaca aaagagagaa attttaaagc tgggggagac atcacacgtt 420

ggtaggatcc acagtgcccc acaagccaca aaaaccagca agtttttatt agggattttc 480

aaaaggggag ggagtgtgcg aataggtgtg ggtgacagac atcaagtact taacagggta 540

atagaatatc acaaggtaag tggaggcagg gcgagatcac aggaccacag gaccgaggcg 600

aaattaaaat tgctaatgaa gtttcaggca ccattgtcat caataacatc ttatcaggag 660

acatggtttt gagatcaacc gatctgatca aaatttatta ggtgggaatt tcctcttcct 720

aataagcctg ggagtgctat gggagactgg aatctatctc acctctgcaa tctcgaccag 780

aagagatagg tacgccccgg gggggccagt tcagagacct acccctaggt gcgcattctc 840

tttctcaggg acattccatg ctgagaaaaa gaattcagca atatttctcc catttgcttt 900

tgaaagaaga gaaatatggc tctgttctgc ctggctcacc ggcagtcaga gtttaaggtt 960

atctctctta ttccctgaac aattgctgtt atcctgttct tttttcaagg tgcccacatt 1020

tcatattgct caaacacaca tactgtacaa tttgtgcagt taatgcaatt atcacatagt 1080

cctgaggcga cgtacatcct cctcggctga taggattaag agattaaagt aaaggcaggc 1140

ataggaaatc acaagggtat tgattgggga agtgataagt gtccatgaaa tctttacaat 1200

ttatgtttag agattgcagt aaagacaggc ataagaaatt acaaaagtat taatttgggg 1260

aactaataaa tgtgcataaa atcttcacaa tccacattct tctgttctgg cttcagccgg 1320

tccctctgtt tggggtccct gacttcccgc aacataatat taat 1364

<210> 53
<211> 801

<212> DNA
<213> Homo sapiens

<400> 53
cgccggatgt ttgggtcttc tggagctctc tcctgctctt ctggtatatc cacaaacagt 60

cttgtcaaaa ctggatcctg atacttcata caattacttg cttcgggacc tcttgagatt 120

catttctcgt ttcttcattt cctcaccaaa tgctggagtc cattgagtga gaaggggcta 180

gcctcagcca tgtgtttact ttggaggcct agtcaagagc ttttctagaa catcctgtgg 240

gaatcagtgg acttgcccca atattttctc attattttca cactattcac tgttcccatt 300

caatgcggct gctgagctat tattttgctt tctgtctagg cactggtaac cctggctgga 360

gcgccatctt ggctgattct ccatatctta ttgtccttcc rtgactcaca tgtgctactg 420

tccgttaacc atgctctttg tcacaaagca caattctcag cttcccccca gctcctactc 480

cttctcccca acacttgcac ttacagaaat gtttccttcc catttccctt ttctgtatct 540

accatctttt atctgcccta atatctacaa aaatatcctc agttctcttg aagtaaaaat 600

ggacactttt aaaattgttc atgtaagtat ggatgtatat atctataaat ttatgtatac 660

atatttatgt attcatggat attggtgaat aaatgtcagg gtagccctac tttttacacc 720

cgcttctctg gtcacaatac agatgaactg tctgcatttg ggtgggaggc agtgagagaa 780

gcatcgattg attggcatga t 801

<210> 54
<211> 1617
<212> DNA
<213> Homo sapiens

<400> 54
gaccctgagg ctaggacaga tgtcactgct gcacatggaa attggacatc actactgtca 60

ctcagccatc tttactaaaa tgctttctgc acagtcctag cctctctgtc acctcattct 120

ggctgagtct ggcagtgata taggagttaa gaagaaatta tttagacagg tagtgagggt 180

acacaaggct tttaatggaa agcaggctcc aaattatttt cttttctaac aaggagcagc 240

ctgtaatatc aagcttcaga cacagacaag taagctggaa gtttgcacgg gtgaatgcca 300

gcagctgtcc aataggaata ggacgcctgg gactgggtat gttcacaatg gcggctccat 360

cttcccttct ctttgccagg cacgtttaca gtaaggagca gacaacatgt caccggccaa 420

gtggaaagcc catttgcata ataggatgag ggtggggtga acagccttcc acacgcacta 480

tgtaaatatc acacctggta caaccaacct gtgggcccta cataaatcag acaccgtctc 540

ctcaagcctg cctgtaaaat ccgatgcact cccattccag gctggaattc ccttttgggt 600

gcccctctct ctcacaagaa ggagctgttt ccctttgtct ttcttttgcc tattaaacca 660

tttgatccta actcactcct cgtgtgtcag tgtccttaat cttcttggcg tgagatgacg 720

aacacttagt atttacctga gacaatgaca cctcttcagc aggtggtcat cagactggtg 780

gagcttaaaa ctcatgtcca tgagcaactg caggggtgac tggrgtgttg ttgtcctctt 840

ccagggaggg aggtggtgtc tgtctcctct caacatttta catgttgtaa accaaaaata 900

aacatcaaag ccaccccttc ccaatcattt taatggacac cctcagccag ggtgctcaaa 960

agttaacctg aaagactggc tcaggccacc aagggaagca ggtattgaac atgtcttatt 1020

atgccctctt ccctcttgga attcaggaaa agttgaccag catttaccat caacacagac 1080

cttcagtctg atcagaaaca tttacaatct attctctctg aagcctggca cctggaggcg 1140

tcatctgcat gagaattttg gactccacaa ccttttatca taacccagac attcctttct 1200

attgataata actcaaccaa ttgccaatca gaaaaattaa aaatctactt ataacccaga 1260

agcactaccc cgcaaccctt gcttgcttca aattgttcca actttctgga ccgaaccaat 1320

gtatagctta aatgtatttg attgatgtct catatcttcc ttaaatgtgt aaaaccaacc 1380

tgtgcctcaa ccaccttgag cacatgttct cggtctcctg agggctgtgt catgggctgt 1440

ggtcactcat ttttggctca gaataaatct ctttaaatat gttactgcgt ttgactcttt 1500

tcattgacag tgtggaattc tgatgtagta agagggttca agtgctggag tgtgaggggt 1560

ggggaaaagt gacaaatttt aattcttgga gcagtgatcc aggacgagaa ctttatc 1617

<210> 55
<211> 501
<212> DNA
<213> Homo sapiens

<400> 55
tcaggcaact ggggctaaat tttgcttggc tctctaagaa atgtaaagaa tgcctcctgt 60

aattgctcac ctcaagtatt tattcattgg ctctcgtgct ttattggttg tccctgagga 120

ctttagccct ctctcactgc agcacagaca ctgtgctttc tcctagtttc tgtggcaagt 180

gacaggagcc cacctcaaac taaagcaaaa gggacttcat tggctcttgt agctaggaat 240

tccagggttg rcactggctt tgggcactac tggatgcagg aattcaaaca atgtcttcaa 300

ctctttcttt tggtgtttct ctcagctgtg cttctcttgt cgtttctttt tcccatttta 360

cagataagtt catccgtaac tgagagaggt gaaaagggga tggctgcaga gaactctggc 420

ttatatcatc cttgcttgct gacctcaagg tccatgtata aattctcaga gaagaagccc 480

tctggttggt gatgcttgga a 501

<210> 56
<211> 1001
<212> DNA
<213> Homo sapiens

<400> 56
aaacaaaatc acaaatggca tactcatttt ctcttatgag aaaagaaaaa tagctcagag 60

tagtctgagc tatgtgaggt atgcaaaatg actctcttag gcccagagag tcatgagtat 120
ggaacttcag tcatgcctct tccaacccat gcccaggggc aactgtgtaa aagcattttg 180
ttcctgtcta gctgcctcac ccactatctt tatgttcctg aactttgtga tacaaacaaa 240
aaatgaatag ccaatcaaga gcttatgtta tttcaatgta aattcctggt aacaatttag 300
gaattgcctc ttcttttttc cttacacccc acttgtaact gctattcatc agaaggtcat 360
tctcaagctt tgggctggag taaactctat acttaatcat attttctgaa tctcatcatt 420
taaggttgac aaaatatata gtttatgaat tacggtgtct ctttaaattc cttatatctt 480
acccctgaag gtgatggcat ytggaggtga ttaagtcata agggctaccc tcatgaatgg 540
aattagtccc ttacaaaaga ggtctgagga agctgtttac ctcttctacc atgtgaagac 600
acgggggaaa atggctgtct atgaatcagg aagttggcct tcaccagaca ctgaatctgc 660
caggcccttg atcttggacc tcctagcctc cttatgtttt ctttgcttgg aaatccattc 720
atttatccaa caaacatgta ttaattgatt ataacgttct gagtacttat gtgtagcaga 780
cattgtagtc tgttgtctaa ttacaatttc cccatttttt ctacataatc ggattccaat 840
tttgttctgt tgacctaaag gaagaagtgg agacaaaatt aatataagta gagagtttat 900
ttgggtcaag ttggaagatt gcagcctggg agcacagatt caagttgcca tgaatatgca 960
ctccagttag cagcagttat aagtagaatt ttaaagggaa a 1001

<210> 57
<211> 511
<212> DNA
<213> Homo sapiens

<400> 57
ctagacattc tcctggaact ataagaaccc tcctcctgcg cctccacctc catccccaac 60
acctattccc ccaaacttaa attcttaaga gaatcctaga tcaagccatg ggtttggtga 120
gttaagctaa gccagatgat acagtaaatg tgcaggaaac ctgccttata aagtaaatgc 180
gttctctctc gtgctgagaa acttataaga tcctgctggc gctctatact ttattggcta 240
ggagaagtaa agaaakgtct gattcgaggt gaagatgctc cccatgcctt gcagcaggga 300
aatttaaatt gcctctgctt agagcgtttc cagacctgaa agaccagtgg tttagggaag 360
cactctacat gagggaaacc tgcattagaa ggagcttctt aatccctggg atctttccaa 420
gctaaactgg atgtctacag tggggagaaa gaaaagcaga gaacaggaca tgaggggggc 480
tcaaggcccc gaagggttga cataggtgtc c 511

<210> 58
<211> 601
<212> DNA
<213> Homo sapiens

<400> 58
aagatgaaag atacatattc tcagcttttg taacaaggat ctttttacac atgacccaat 60

agtaactcag gactacttac atcaattctt gcttcaagct cagggatggg aatccttcta 120

ttatagcata acatttgcct accaatatct tcacaaattg gagttgaacc tgtttaaaga 180

gaaggaaaaa ttaactattg gaatacagat caaccatatg aaaacagaac ctatatcttt 240

atttttacca tcaagctgca acaacatgtt tgttttcaga agatttctgg ctcgtgcaac 300

mtcactttct gtgacacttg tacagagtcg catcctaaaa aacgtggaaa aacagtagta 360

gttggaaaat gttgtatttc atgagttaat gacctaagaa tgatgatgcc atcttctaca 420

caaaacatac aggtgattac aatttaattc caatgctaag caaaagctaa aatgattatt 480

ttccccagta ttaatttctc aaaaatgata cataaacatg cctaattaca cattagcaga 540

caaaggcttt atacaaatgg gctgatcaca ttttcagaaa tgctataagc atcttgagac 600

t 601

<210> 59
<211> 1001
<212> DNA
<213> Homo sapiens

<400> 59
atagaacaaa aatactgccc tttcaatgag tcaaagggaa ctctttcaat tcaaaggccg 60

actgtctttg aattgggaat aggtttttgt ttttgtttct gttttcctgc ttctggattc 120

aaactgaaac atcagctctt catgtgtctt cagcctgctg gtcttcagac agaaattaca 180

ccattggctc tcctgattct caggcctttg gactctaaga ttagaatcat accattggct 240

ttcctgggtc tgcagtttgt caactgcagg tcttggaaat tttcagcctc caaaagtgta 300

tgagccaatt ctgtataata aatctgtctc tctcttactc cctctctctc gattctgttt 360

ccccaaagaa ccctgactaa tacatggata taataatttt acacttgaac atcacaaaat 420

ctcttgatgt tgaaagggag atcttgattc ccatgttttc ataataagca ctttgggaga 480

ccatgaatct tcatgtgtag mtctttttgg atcacttggt ctagtgtata gacttggacc 540

tgagacccag tttctaatcc catttctgtt accaagtggc tgtgtgatta gggacaaatt 600

acttctcctc tctgtgctcc attttctcat ccctatgaga aggatcacag gggattatcc 660

ccagtgtttt ttttgtactt ctaaaatcct agggatttaa attttttaag taggaatggg 720

atcatgtcct ttgcagggac atggatggat ctggaagcca ttatcctcag gaacagaaca 780

caggaacaga aaaccaaatg ctgcatgttc tcacttgtaa gtgggagctg aacaatgaga 840

acacatggac acagggagag gaacaacaca cactggggcc tgttggtggg gcaggggagg 900

gagagcatca ggagaaatag ctaatgcagg tggagcttaa tacctaggtg atgggttgac 960

aggtgcagca aaccaccatg gcacacaaca ctacctcttc t 1001

<210> 60
<211> 957
<212> DNA
<213> Homo sapiens

<400> 60
tagagcacag cagtccccaa caacaataaa tcacacattc tttccaagag tacatgaaac 60

atgtaccaag atagaccgta ttttgagcca tgaaacaaat cttgataaat ttaaaaggat 120

tcaagtcata gaaaatatgt tctctgacca caatggaatt aaattattaa ccaataacaa 180

atatctggga aaacctcaaa aacttggaca ccagcgcttt taaaagacta aataatttct 240

aaattatctg tgttgggggg aaaagagaaa tggattagag agcaaaaagg gtatcagagt 300

gctgtggtac gatttttatg aagagtggaa cagaatctgc ctttggcgtt tccccactac 360

agcccattct tcacattgat aacagcatga tccttctaaa attaaatcta acgatcactt 420

ctgcttaatg gctctccaac acttacagaa ttaggtccaa aattctagca cagtttctgt 480

tcatctttct aacctttctt cccacaggtc tagctagtac gtatttcttt trttgcattt 540

attacactat tcctttgctt atctatctcc ccacctaggc taaagaacaa gattcttgtc 600

tttttcattt ttgtgtctca gtgcctagca tggtgccagg cacacagcat gcttccagta 660

aatgttagct ggatggatgt aatgagtata ttaaatatta atttatttgt ttttccccaa 720

aaagaattat ttcctgcaaa tcaaggaaat tgctttcttt atataatcaa aaacttattt 780

tcccagaaga ttcttcatta aaaattaagc ctatgcacaa cctagctcta aagtttcaaa 840

gattttaggc agcaattttt caatcttttt gaagtaatac atttgaatct tttcaaattt 900

ctgtttctgc atttgtgcca caccatctca tctcttgctg aaatgttttt gttaaat 957

<210> 61
<211> 1001
<212> DNA
<213> Homo sapiens

<400> 61
caaattcctt gcctggatgc aaccactgtt accagtttca gcaagaaatt aagacagaaa 60

ttaaagttct gctcctcata ataatcacga tcaattatcc ctgcttatac agtaacccct 120

ccttttccca acagcctcct agcatgagga acccaaattg aacaggtaac aaacataccc 180

ctaaacgtaa tgtaacttgt actctgttcc atggtaggaa cttccttctc tgggatccca 240

aactcctaga ccagctgagt acgttgaggg gatgggaagc accaacaccc cacgcgggtt 300

accggaagtg ctggtgagta tagtcattcc ttcttccagc actggtggtt agagacttac 360

ttactctacc tgttggagac ttggcactat gtaacagctg ctgccatagc ctaagtgtat 420

gcttcatcct gaaggacatt acccatcctg ataggatgtg atcttcaaga taaaacccag 480

ctgcaccctc aagagaccat yccactgctc tatcagctgg cagcgtgtgt gatgcagtat 540

ggggtagaac caggagatct catggtcgtg tgcccactac cagacagcgt tcgctataaa 600

gtaggtccct tagtctgggg caatgttatg tggttcccct attagaagat catacatatg 660

tgagccctta gataacgttt ctgagtgtgg cactatggga agaaagaaca aactcatacc 720

gctaatacaa ggcaagctaa atctgacaga acccctgcct ttttcattgt gaaaaagtaa 780

gagtagaatc aatttgccat caaattgctc attggcctcc ataagcaaag gtaatctgtt 840

ggagtttatt tatcgttgga aggtaggttg tgacaataac cacgtcagcc atgctgaagg 900

gagcccaagc cttgggaccc acccacagtg catctcttcc accatagata ctgctttcaa 960

gagcctgttt catgagcact gggatggtta aggacagagg c 1001

<210> 62
<211> 822
<212> DNA
<213> Homo sapiens

<400> 62
ttttattgtt cacatagtac tttgattgac aagtattagt tcctttgatc ctgagagtaa 60

ttccataaca cacacaaggc aaagatcagt atctatgctt tgcaaataaa gagagaaaga 120

cagaatggta aactggctta ccgaggagta aagcaccgtt ggtgtcaaaa gaaagcaaag 180

cttaggatat acactatcct gtagttcacc ctacgtgaca tgaaatggtc agtttgaaag 240

gtctgccacc caaggcatga tatctttcat ttgaaagaag tatatttatt ttataattat 300

gaaactaaac aaaaccaaac trtggttgct ataagttcta tatggtgctc agcacagtta 360

cacaagtaat taaacacatt ttattgtttt cagggcacaa tgatatgtta caaaactctc 420

tatcacttcc accaatgctg gaaaaactca ggaaaacata tcagaataca tgagtgctat 480

ggtctgaata tatgtgtctc tccgaaattc atatattgag ataccaaccg ccaaagtcat 540

gttagtagga agtggggtct ttaggaagtg atcatggtag cagtgccctg aggacttgga 600

tcagtgtctt aacaaaagag ggccctgaaa gcagccttac cccttatgtc atgtgagaac 660

acagagaaag tgctatctat gaatcagaaa gagggccctc accagatacc aaatctgcca 720

gcaccttaat attggactta cagcctccaa aacaatgatg aataaatgtc tataaactaa 780

tagtttttga tgttttcttg cagtaggcca aacagactaa ga 822

<210> 63
<211> 5135
<212> DNA
<213> Homo sapiens

<400> 63
cagcaacacc tccaaaaata atcactttta ttagtttttt gtgaaacctt tatataaata 60

```
tgaatacata ttcttaattc ctcccctttg aatatataag aagcatatac acacccttct      120
gtataaatat tgtcatgttg tctactgttt tctcttgaca aaactatgtt agagatcttt      180
ccatacaggt taatcatcca taatccaaaa atctgaaatc caaaatgctc caagatctga      240
aatttttga gcaccaacat gatacccaca atggaaaatg ccacacctga cctcatgtaa      300
tgagtcacag tcaaactgta agcacataac acaatttaag gaaaaaagat cctccccagc      360
cccttcagc tctgacatat cttttccatg catgcacaga ttctcccacc caagcacatc      420
caaagaaggg taataaaatg gcacaggtac acgccagacg caccaatgat aggttcccca      480
cgatgcccca cacggagcca agactgtgca ttactcactg tgggtttttt tgcttattct      540
ctgctttgtt ggataaagac aatgttgaaa atctgagtaa gtgacagaaa aagaggaagc      600
atttatgctt atctctagcc aaaaagtcaa gctgctggat aaactggaaa gcagtatggg      660
aaatgtctta caaaagagca tggtgttgga atgaccacca tatacaacct gaagaaacag      720
aaggataaat tgttgaagtt ctatgctgaa gtgtcaatga acagaagtta atgaaaaatt      780
tttcttaatt gcagaaaacc aaatgaagat ctcaaatgtg tactgaaaga gtaaatctgt      840
cagcacggca gagaaaacat actacttaat ggtatggtgc tcatgaaaca agcaaagata      900
tatcatgatg aactgaaaat tgaagggaac tatgaatatt caacaggctg attgcagaaa      960
tttaagaaaa gacacattaa tttttcaaag atttgtggtg aataaagcaa ctgctgatca     1020
tgaagaagca gaaaaattca ttgacaagtc tgccaaggcc atcactgatg aaaatctgac     1080
accagaacaa gtctataatg ctgatgaaac atcacagttt agtgttagtt ccccagaaag     1140
actctgacta cagcccctac aggataagaa tgccatggac agagtaactg tcctgggagg     1200
tgctaatgca gaaggcacac taagtgtaaa cctgctgtgt gggaaagatt gggttcatta     1260
ttagactaac aaaaaggcat gggtcactag ggacatcttt tctgattggt ttcacaaaca     1320
ttttgtagca gtggttcagg tttgctgcag ggaagctgga ctggatgatg gctacaagat     1380
tttgttattc cttgacaact gttctgctca tcctccagct gaaaaatctc attaaatata     1440
atgtttattc catgtacttt tccccaaatg tgacttcatt aattcagcaa gtgagcaggg     1500
tatcctcaga tcaataaaga ataaaaataa aaacactttc ttgaacagcc tacttgcagc     1560
agtgaacaga aatatgagcg tggaaggttt tcaaaagcag ttcagcattt agaatgctgt     1620
atatgctatt gtcgatgact ggaacacagt atctaaagac acagttgtgc atacctggca     1680
caatctctag cttgcaacta tgttcagtaa cgatgatgaa caaggcaatg actttgaagg     1740
attctgtatg tcaagataaa aaaaaaaaaa aataggccag gagcggtggc tcacgcctgt     1800
aatcccagca ctttggaagg ccgaggcggt ggatcacgag gtcaggagac cgagaccatc     1860
ctggctaaca cggtgaaacc ccgtctctac taaaaatacg aaaaataagc caggcatggt     1920
ggcgggtacc tgtagtccca gctacttgcg aggctgaggc aggagaatgg cgtgaaccca     1980
```

ggaggcggag cttgccgtga gccgagatcg cgccgctgca ctccagcctg ggcgacagag 2040
caagactccg tctcaaaaaa aaaaaattaa ttaaaaattt taaaaataaa taaataaaaa 2100
taaaaaggtg tttgacttcc ttacatatac aaaaaaatac cttcagagtc tgtcagtaag 2160
ttggaagaaa tggatatcaa ataagttttt aacattgata atgagtctcc agttgctcat 2220
tcattgacca atggtaaaat agcagaaatg ggctgggcac ggtggctcac gcctgtattc 2280
cagcactttg ggaggccgag gtaggcgtat cctttgagct caggagttcg agaccagccc 2340
gggcaaacgg cggaaccctg tctctattaa aaaatacaaa acgtttggtg ggcatgatgg 2400
cacatgcctg tggtcccagc tacttgggag actgagcctg ggagatggag gttgcagtga 2460
gccgagatca cgccactgca ctccagcctg ggtggcagag ttagacccta tctcaaaaaa 2520
agaaaaaaga aatagcagaa atggttctga atcaaggtta tggtagtagt aatgacaatg 2580
aagatgatgt taacattgca gaaaaagtgc cggaagacaa catggtgtga tgggcttatt 2640
ttaggactag agtgcattct taacagaata ggaaatcacg tcactttata acatcaaaga 2700
gagacttcta agacaaaagc cactgttaat gaggcagacg attctcgagg aaacatttta 2760
aaaagccatc caagcaatgg ggaaaggatt ccctatttaa taaatggtgc tgggaaaact 2820
ggctagccat atgtagaaag ctgaaactgg atcccttcct tacaccttat acaaaaatta 2880
attcaagatg gattaaagat ttacatgtta gacctaaaac cagaaaaacc ctagaagaaa 2940
acctaggcaa taccattcag gacataggca tgggtaagga cttcatgtct aaaacactaa 3000
aagcaatggc aacgaaagcc aaaattgaca aatgggatct aattaaacta aagagcttct 3060
gcacagcaaa agaaactacc atcagagtga acaggcaacc tacagaatgg gagaaaattt 3120
ttgcaatcta ctcatctgac aaagggctaa tatccagaat ctacgatgaa ctcaaacaaa 3180
tttacaagaa aaaaacaaac aaccccatca aaaagtgggc aaaggatatg aacacacact 3240
tctcaaaaga agacatttat gcagccaaaa gacacatgaa aacatgctca tcatcactga 3300
ccatcagaga aatgcaaatc aaaaccacaa tgagatacca tctcacacca gttagaatgg 3360
caatcattaa aaagtcagga aacaacaggt gctggagagg atgtggagaa ataggaacac 3420
ttttacactg ttggtgggac tgtaaactag ttcaaccatt gtggaagaca gtgtggcgat 3480
tcctcaggga tctagaacta gaaataccgt ttgacccagc catcccatta ctgggtatat 3540
acccaaagga ttataaatca tgctgctata aagacacatg cacacgtatg tttattgcgg 3600
cactattcac aatagcaaag acttggaacc aacccaaatg tccaacaatg atagactgga 3660
ttaagaaaat gtggtacata tacaccatgg aatactatgc agccataaaa aatgatgagt 3720
tcatgtcctt tgtagggaca tggatgaagc tggaaaccat cattctgagc aaactatcac 3780
aaggacaaaa aaccaaacac cgcatgttct cactcatagg tgggaattga acgatgagaa 3840

cacatggaca caggaagggg aacatcgcac accggggacg gttgtggggt gggtggagcg 3900

ggcagggata gcattaggag atatacctaa tgctaaatga cgagttaatg ggtgcagcac 3960

accaacatgg cacatgtata catatgtaac aaacctgcac attgtgcaca tgtaccctaa 4020

aacttaaagt ataataataa taaaataaaa taaaataaaa taaaaaagcc atccagcaga 4080

atgtttcctt atccctagca gacctatttc ctggcccctc agctgcttct gatgtttctt 4140

ctcaactaaa aaaaaaacca cacagtgtat aatagtaacc tttcaatcaa aaatcagcat 4200

cataggtgaa aactaaaagc ctgccgttgt ttgttgttcc tgttgtttaa aagctgatag 4260

aggtattctg gtaatgccac tgtgctgctt cattaccctg aacatatttt ttttccactg 4320

tattaatgtt atgccatatt ttttgcagtt aagtacttat gcatgagcaa gtataagaaa 4380

atgattgctt atcagtagca tgtaaattca gagtcagaaa tgaaggtgat gccaaacaac 4440

tccacagact gtccacatgg gtggctgaga tagtgacacc tttcctttct gatggttcaa 4500

tgtatgcaaa ctttgtttca tgcagaaaat tacttaaaat attgtataac attacctaca 4560

ggctatgtgt ataaggtaga tacaaaaatt tcatgtttgg ccttgggtcc cagccccaaa 4620

atatctcatt atgtatatgc aaatactcca aaaaacaaaa caaaacaaaa ttcagaaatc 4680

tgaaacattt atggtcccaa gcatctcaga taagggattc tcaacctgaa taaagcatca 4740

atgttctttt ttacagctgc atcctattca attacagaga tatagcatat cttacttgcc 4800

cagtcttttg cttatggaca tccaggttct cttttgatat gayaaacaat gctgcactga 4860

gtaaccttat atataagtca atgaatgaat gaatgaatga atgaatgaat gaatatatat 4920

aaacttatat ataagtcaat gaatgaataa gtcatgtgct agtatatatg tggaataaat 4980

tcccagaaat gaaactgaga atacagacac attcacacac acataaatat aaaagtagat 5040

atgctgacaa ataagtatat aatcatgtaa taatataatt ttaataatgt agtataacat 5100

aagatcacta ttgtcataac tatatggtaa ttatt 5135

<210> 64
<211> 1001
<212> DNA
<213> Homo sapiens

<400> 64
agccgaaggt gttttcttgc cttctcgagg cctgtttcct cattggtgaa atgctaagcc 60

ccactcagcg tggacagccc acaatgctaa aattctgctg ctaacattct gggccgggcg 120

cagtggctca cgcctgtaat cccagcactt tgggagccca aggtgggtgg atcacttgag 180

gtcaggagtt caagaccagc ctggccaaca tagtgaaacc ccatctctac taaaaataca 240

aaaattagcc aggcatggtg gcacacgcct gtagtcgcca ctatttggga ggctgaggca 300

ggagaattgc ttgagcccgg gagacacaga ctgcagtgag ccaagatcac gccactgcac 360

tccagcctgg gcgacaggac gagacttcgt ctcaaaataa taataataaa taaaataaaa 420

ttctgctgca aacattctgg ggtttactga gcatacggtt ctgcaaattt gacaccctga 480

attctcagta acttagaagt matttctaat gattccggct gggcacggtg gctcacgcct 540

gttatcccag cactttggga ggccaaggcg ggtggatctc ctgaggtcag gagttcaaga 600

ccagcctggc caacaaagtg aaacctcgtc tctactaaaa atacaaaaat tagccaggcg 660

tggtggcaca cgcctgtaat cccagctact caggaggctg aggcaggaga atcacttgaa 720

cccaagacac agaggttgca gtgagccgag attgcaccac tgcactctag ccagggtgac 780

agagtgagac tctgtctcaa aaaaaaaaaa agtcattttg tgattcaaat gtttgataat 840

atcccaagat ttccacttct agagcctgag aagctttaga agactaatgt tctataaacc 900

caagagtctg ccatgccgca cctagatgct gcaggtaccc aacatctgtg tagaataatt 960

ttctgggcca ggcacagtcg ctcacgcttg taatcccagc a 1001

<210> 65
<211> 601
<212> DNA
<213> Homo sapiens

<400> 65
caaattacac ttgatttgcc tccaggaaga aaatagtaca ttgttatcat ttggtcaaga 60

tttcatattc aaaaaagagt aaagtgacaa tatagttcaa aaatcttgtt agatgctgtg 120

gaaacctcac ccggcagtag tgatagctgc cacgtactga gggattttat agacatttac 180

attttcactt gtcctcacct tccagatgag gcagctgtgg cccagagagg ttagggaact 240

taccgaggtc acacagcaag ttggcaagag ggttggggtt tcagtctggg ctctctgact 300

ycgcagccaa gtgcctgcca gtgctgcatt gtctcaaggt atttggcgtg ttgatgtgac 360

atcactcacc ctccttgagt tctctgacat ttctcagagc gtttgtctgt gtgtcctgat 420

ttgagaatta gttaagctct cgcactctcc ggggtgcagt gcctggaatt caggcactgg 480

aggaagtcct gcctccttcc tggagctcgc agccagatcc cacagatcct gctctgttgg 540

acagtgagga tgggaggtgt ggcccagggg ttggcaggat ggtagtgggg tgcagagtag 600

a 601

<210> 66
<211> 801
<212> DNA
<213> Homo sapiens

<400> 66
gataagtatg ccaggagtca gtgtccgtgt gagcacatgc tggccaggcc tcgggatctg 60

ggttaggatc gcaaacatgt tttcttcatc ttcatgagga caaattcttt ttatacaagc 120

tggacttctt caatcattct ttatgaacaa agggagaaca ggaaagctct gtttaatgaa 180

cttcccaatc cagttaggag taaaaagtag aatacacacc acatttggag cactttatgt 240

taggattccg cccaaaatat accatagttg catgaaaccg taagctcact ggtagctgga 300

actgggtttg ttggagggac catcataatc actgcctcta aagcaaggcc tttccaatga 360

cagtagtgag taaatgagca tggactaaat aaagaacaca kgaacatcta agttaccggg 420

ctctacatat tcaagttgtt gtgctgagaa atctgtctgg agccatgaac ttcaatcagt 480

tctcccatcg tccctgccag atgggacttc agtttctgtc ctcaccacaa ggctgagtca 540

caaacccagg ctgacactgg gaatgtggca tttaaactgg tgcttaccaa gcttataaga 600

gtttctttga gtgacccggg agaaggggat ggtaagacaa agaaaaccac tgattggaaa 660

agggggcatg aggagtccag tcctcaattc tcgtacaggt gggggtaaag agggaaattc 720

aagcattccc ttagtatttt gcttccacct ccctgttccc acctcattct ttgtaactca 780

aatcagaacc tcctctctct a 801

<210> 67
<211> 801
<212> DNA
<213> Homo sapiens

<400> 67
aacatagatt tgggagcccc agaaagttaa ctttaccatt ctattggtca agatctctag 60

gaccagtcca gatataagag aaagggaatt aaactcaact tctcaatgca aggagcatca 120

acagtacaca gaaagaactg tacacagaag gaactgaaga cagttatcaa cttctgtatg 180

aattggctaa aataccatat ctaatagcac ctgctactgt tctagcatat aatcagtgct 240

tcgataatgt aaagaacatg cagtcaagta ctctttaaaa ctcaagcaca gcttgtgtgg 300

ttatgatgac tgattccttt tcctgtttgc tttaagatgc acagcctgac aggattttta 360

ttttctgttt ggggaacaga atactaacag gtcatgttta ytgagcattt ctgacatgct 420

gagctctttt cacaaatgat tgaatttgaa cccctccaca aacctttcag gtagataatc 480

tgtgtccttg aattctgcaa ataagaagag taaggcttgg agaattttgt gacttttcct 540

ggatttcata gcatataagt agcaaatgaa tgagaataac ccaatttagc taatatcagt 600

gactgagctc taaactgcta tgtactagaa aaaaatctgc aaatataaat atattaaatt 660

atattttatt atatatttta tgaattataa tttttaattt ttactaattt tgtattattt 720

ttatcctagt ttattaaaat attccacata ataaattatg gtatattctt tctatggaat 780

acactgtaac tatcacaaag a 801

<210> 68
<211> 801
<212> DNA
<213> Homo sapiens

<400> 68
cgtgagccac tgcacccagc ccatttcttc tacattttct tgtttgtaca cctaaatttt 60

attctttctt ccgcacaact cagagtcttt tgcattttgc tttcctgtct tttaaaggta 120

tctttatgtg tatgtataaa atattttata tattatataa acatacataa tacatagtta 180

tatataatat atattatata ttatacataa atatgaccca acttcttcta acttcacatc 240

tgtatttcca gctctttggg aagcttaaat caacttcaaa acctccacaa atgaactcac 300

tatctcacgt gctaagctca ccagcagcca ggcacccagg atggaagctc tgtcaagtag 360

ttctcacact cagttggcaa attcagattc caaaagtctc ytgcggctct cagaccccat 420

gccctttccc agccactgtg ctcaatagcc ctgtgtttcc acagttccca cttctctgtc 480

ctgacacacc ctgagtcttg gctcccttta aaactgttgg tttcaatttt taataatcag 540

aaaaggtata acaaatgctc ttgcattcac acaaaaatca atggggttag catatacttc 600

agtttataac aaacatttct aaagtgattg taccatctta cattcttgcc aaagtacaat 660

agttcctctt gctccacagc cttgcctttg aggctgtcag ttatttttc ccccgttata 720

tccagaaata accattgtta acattttcta atctctctct ctctctttat atatcaggat 780

aatgccacat agattattcc t 801

<210> 69
<211> 869
<212> DNA
<213> Homo sapiens

<400> 69
aaggccacaa ttagaggtaa caattgtgct ctgaatataa gcaatgtaag agctgtttgt 60

cttataaact ggtctttttc taggaaacag aagtactgac tcttgcagtc ataaacacag 120

gtgggttgct ctcttagtgt cttgatcttt ccttttctgg taaatcatca ctctgaaacc 180

atatttttcc ttgattgaaa ytgagtcata gaagcttaga ataatggaaa gtttgccggg 240

cgcgatgtct cacgcctgta atcccagcac tttgggagtc cgaggcggtc agatcacgag 300

gtcaggagtt cgagaccatc ctggctaaca cagtgaaacc cagtctctac taaaaatgca 360

aaaaatttag ccgggtgtgg cggtgtgtgc ctgtagtccc agctgctggg gaggctgagg 420

caggagaatg gcgtgaaccc gggaggtgga gtttgcagtg agccaagata gcgccactgt 480

actccaacct gggtgacaga gcaagactcc gtctcaaaaa agaaaaaaaa aaaaaagaat 540

actgggaagt ttgaactaga aggcgcacta ataataatct gtgtgataat gtatctatta 600

tctaggccta tatgatactg tagtccatgt tatgattggc catgactttg aatttgaaaa 660

aggataaaaa agaggacata aagtagacga gattgtgtaa aaatgtggtg ggatcaatag 720

gttaactttc caatgagatc aaaaaacaaa gtaatgatct ggagaaatgg gaggtgatga 780

tcaaagagtg ggattcaaaa gatgaaattc taaggaaaac aattggagga aaaacattca 840

aggaactgag ggggagataa aatcttcac 869

<210> 70
<211> 801
<212> DNA
<213> Homo sapiens

<400> 70
cttgggaggc taaggcagga gaattgcttg aacccgggag gcagatgttg cagtgggctg 60

agatctcgcc actgcactcc agcctgggtg acagagcgag actctgtctc taaaataata 120

ataataataa aaaaatgttt tacacaccta catgaacctt aaaaattaaa gattggagct 180

atgtgtatga gatagtaaca ctcattaaaa agggcaagtt ttggttaatt aagacagtag 240

gaggcgtaga gaaaatataa tgaaacgata tgtaagggaa acgaaggatg aaagatgcag 300

gcagggagag gagtactgtc tgatgggagt gaagattctt ccttcaggaa tggaagggga 360

tgcacagagt gaagccaccc aacaaaaaca agacttgtat rgctatagat ggaagggaaa 420

tcaaccagga aattattttg gaaatcccag tgtagttaca agtctaggaa gtaatagtta 480

gaatgaagag tttgtattta ctgagcacta atattctcat aatcatgcta ggaaatatca 540

cttgataaag atagaaagat gagtcgttta agaaggaata catgtctctt ttcagaggca 600

gtacccgcag gcagttctca aacccagaat ctgcctcatc agtctcagtt caggttactt 660

cgtttcaggg aaggcattcc agcttcttgc actaggcatt tcaagaaaga aaagcattcc 720

tggaaatccc caatcggtca caggcatgtt tctccaacca ataggaatag agaatgaggc 780

agcatcactt gtctccagca g 801

<210> 71
<211> 601
<212> DNA
<213> Homo sapiens

<400> 71
ctaatattgg tcttttggcc aatagtgaaa aagaacatgt ttttttcttt acagttcttc 60

gaatactttc agcctagaaa gtttaagaca gagaggactg gtaattatgg ttagataatt 120

ctagatttac ttctaaacat ctgaatgcta gaatatggaa gatgagaggg catcttttga 180

aattagagac tcatctgggg cacatttatg gaaattaagt ctttgtatac tataaataat 240

tgcaagaggt agacatgaaa tataggtggg actgctgtgc ttcacacact ctatcacaaa 300

ytgaattcta actggtataa atctagaaac aaacaaacct ggaggagggc cagttaaagg 360

caattaatga ctatacaaaa agattgaaca aagtaaaaat cagctactac aattacaaga 420

tagatgtcat gaagtgcaac catatcttct cctatctctc aagaattttc ttggcaacag 480

aactctggat tagatgaact atatttctaa gattttgaaa ttagaaagac aaaatgaaag 540

ccagaattac tgagagccaa aaataaaaat attctttaga cattttatat atttgaataa 600

a 601

<210> 72
<211> 1001
<212> DNA
<213> Homo sapiens

<400> 72
aactcctaac aggttggtta aaagccaaca gtaacagcca tgagaaaatc tttaaactag 60

attaggaggg gctaagatac caggcattgg gcattcccta gtctccccgc tccatttatt 120

cggaatgcct tcaactgtac cattcaacca cagttattca aaagcctctg tccttagacc 180

tctgttcttt tttcctttca tgctctccac caaagactgc aatcttacta tgatattttt 240

ggagaaaact cacagatcta catctctgtt tttgttatat cacccaaact aatcccctat 300

ctctaacatc tgacttaagt atttccacca gggtatgcca tcattatctc atactcaaca 360

cataaaaaac aaaaaaactt actcatcttt atcaaaccat tccctctttc aacttccttt 420

gttttgttaa catcaacccc atttgtctgg tgaccaagac ttagaatcac agaagcaacc 480

atgaactccc tagtaacctc yactattcac attcagttgg ttcatgtggc agatggcgtt 540

tttcaaagat ggctaccaca acatctccta tctgcgtata cttctacaat atgatcttgc 600

cactttgctg tcaaaaggta gactgaatcc aggctggtcc taagatgcac ttaaaatcaa 660

aggaatgtga ctaaagcaat gcaatgagac ttccaaggct gggtcagaaa agagcataca 720

gcttctggct gattctcttg gaatactctc tctgggggaa gcaagctgcc atataagaat 780

ttcaacccca agactaccat gctggggtga gaggctacat gtagtcccaa ctaggcccag 840

ccttctagct agtcccacca aggtgccata aatgcgagtg aagttgtctt aggtcccttg 900

tgaccagttc atctgccagc tgaatgccac caactgaact cagtaaatac cctaaggaag 960

aatcacccag ctgagccctg cccaaattgt tgagatacaa a 1001

<210> 73
<211> 1115
<212> DNA
<213> Homo sapiens

<400> 73
gtatgaatgg gcagaggtgc tgccagaagt agatatattg acaccaacca gaagtggcag 60

aaaagtggac aatctctcct cccccatata aacttacaac agtccttgaa ggaatgaatg 120

cactaaagat aagagcgtga ttaaaattat taataggatg ttaatttatg ctcagaacat 180

gcaatttaag aatgcatgaa matacttaaa cagcctagaa aaatttgcac ctgccagctg 240

ggcgtggtgg ctcgcgccca taatcccagc acattgggag gtcaaggtgg acggatcact 300

tgaggtcagg agtttgagac cagcctggtc aacatggtga aactccgtct ctactaaaaa 360

tacaaaaatt agccaggcgt agtggcatgt gcctgtaatc ccacctactt gggaggctga 420

ggcaggagaa ttgcttgaac ctgggaggcg gaggttgcag tgagccaaga ttgcaccact 480

gcactctagc ctgggtgaca gagcaagact ccatctcaaa aaaaaaaaaa aaaagaaaaa 540

aaattgcaac tgccaagtgg ctaaaatgaa gaaataagtc tttatgaata acacataaat 600

cccccaaaat cctggataaa tgagatggga tagaaaacat atatatatac aatgcacata 660

tgtacacaga tatatgtata tacacatatg ccatatgcgt atacacatat ataatcataa 720

aattatatat ctctataaat atatataatt tctataagta tacagaatgt atacatataa 780

ggattaaatt tttttgtgtg tgtgagacgg agtcttgctc tgttgccagg ctgcagtgca 840

gttgtgccat ctcggctcac tgcaacctct gcctcccagg ttcaagtgat tctcctgcct 900

cagcctctga gtagctggga atacaggcgc acgccaccat gcccggctaa tttttttgtat 960

ttttagtaca gacggggttt caccgtgtta gccagggtgg tcttgatctc ctgacctcgt 1020

ggtctgcccg cctaggcctc ccaaagtgct gggattaaag gcgtgagcca ccatgcctga 1080

ccaacattgt tattttcaaa aaaattgtga ggata 1115

<210> 74
<211> 701
<212> DNA
<213> Homo sapiens

<400> 74
tcccacagga gatgagattt gaatgtgggc catttaaaga tgaataggaa tttgtctggt 60

aggagacaag aaagggcatt tgaagcagtg gaaacagcat gtgcaaagac atgtagtgtg 120

aaagcacctg ttctcttggg gaggaaccta gggtggctca ggagctggga gtaaggagtg 180

ggagaggagg tccagatggg ttccttgact tgccgtaagg tcctacagca aaacaactcc 240

aaagcccacc taattaggtt tcttgactct cactccagtg ctctttacac tggaccttga 300

tgcttttagc accaataaaa tgtagccacg tgaaactaga atcattttta acataataaa 360

taccatcgat gctgtaggca caatgggtag cttaaaagag ggtctgagga aagcccagaa 420

aagcaaattc gaaggagaaa tgtaacaaca atgacaccta ccagaataaa aagagtattt 480

atcaaagccc tagatagcac rtcatacagc cttttttttt tttaattcag aaaataaagg 540

ataccaggac agaggaagat gataataata gtgataataa ttgcaacaaa cggtcctagt 600

gtctcatact ttaaaaaaat cctcctttgt atatattatt tcttcggaac gatgagataa 660

ttatgtagtt tggttaaaat taatatgcaa tccataacta a 701

<210> 75
<211> 695
<212> DNA

<213> Homo sapiens

<400> 75
ggctcttagg ttaaacacac tcatgcctct gatactccat catgagccta aaggaaaaga 60

ctgtgaacat aaaagtgaat actttatact tttacttctc ttttattaaa agtaaaattt 120

catgaaaatc tgtaactgtg aagaaacttt aaaacagaat ataagataat acatgtaaag 180

caactagtaa aggaactaac atgtaggcac tcaacaaata ctggctattt ctagaagaaa 240

tgtaaatagg aaatgttagc tatgagctat tattaagtgt ttttatgttc caggcactgt 300

tctaagtgct ttatattatt tatcttactc aatgcttata acaacccyac acattaggta 360

ctattactat tattgccatt ttacagatga ggaaataggt gtatagagaa ttcaggcacc 420

ttgcccacgg gtacacagca ttaatccagg gagtctggtt taagggcaca aactcttaag 480

tactaaactc cactgctgga tggaaaaaga tcagtataaa tatgaataat tttgttctac 540

gcctaaataa cttaagttca tctacagtac aacttaatat gaaaggattc tgttagcttt 600

aatgagaagt aaaacaagaa accagaatca agcaaggggc catgatttct tgtctgggat 660

ggaaactcgg tttctttaaa tagcaaatgg aataa 695

<210> 76
<211> 576
<212> DNA
<213> Homo sapiens

<400> 76
ctgtcttttc aaatttactt tttattaagg atgtacctgt gcactattct aaagctaagc 60

atttaaggat aaagtccaga aataaaatgt ccatattttc ttttcttttc tttttttttt 120

tttttttctg tcatctgtca cccagactgg actgcagtgg tgccatcatg tcttactgca 180

gcctcaaact cctgggctca agtggtcctc ctgcttcagc ttcccaagta gctgggacta 240

caggcatgtg ccaccacccc cagccaaaac atccatattt tctattagaa tggtttacat 300

tatgatgggg gatggggagc tgggggaggg ctgtacattc ccaaaagaaa gtagtgttat 360

aggaaatcca aatgcrcttc agtgattcac tcctgaaaag cagcctgggt atgttggcag 420

attttgtttg catttgtctt gcgtatttaa agtctcagga aatctagtct tggtccaaaa 480

cactggagaa aaaaataaca ccagacaaaa cacatttgag taacttgaag tagcagcata 540

gaataggccc aactaagaag ttggaggggc aaagag 576

<210> 77
<211> 601
<212> DNA
<213> Homo sapiens

<400> 77
ttcaaaaagt ttcaaatgac attccttact ctttggtttt cagaggcatt caagcaaaat 60

atcacagtgg tcttttgttt tctgaccaat ctaacaatac ctgtgattaa atttaatttg 120

ttagtgtaaa taaatttctt gccaatgagt attattgttg ttagacaacg aatgcaataa 180

aaagaaatac tgaggtctgt tgaagcaatt ttttttccct cttccatttt caccttcaac 240

taaaacaagc cttgactggg taaacctgta tcaattgtca aagcatatat ctgtcaatca 300

rgagcaaagt gcacattttt catggaaatg aaactaagtc ttaactgtgg taaagtgtaa 360

attggcagct agaaattaca tttgaaagca caattatgga actgggcata aagttaaaag 420

agcaaagcct gcactcgaag cttcctcgcc cgtcattttg ttcccctgtc gctgcttcac 480

catcaaagtc tctgatatct ctcagccagg aaaacctctg tgacaaatcc attaagtagt 540

cagtctcaca tagcgagggg caagctaagg aagaatcagg accatcgtag agtatggggt 600

t 601

<210> 78
<211> 511
<212> DNA
<213> Homo sapiens

<400> 78
catttcttca attctattaa acataccatt cagcaaccct aatagctaaa aactagcaaa 60

aggcacaaag ggttgccatt aaacatgatc acaattgtcc tgcaaaccta gagataaaag 120

gtaagagagc cataatgtaa atggaaatac acatttgttt cttttttttt taacccaaga 180

gatacgggct ttaacacttt tttttttcct gttgggcagg tgaaatggac tcttaaaact 240

gagctttaac aatacmcagc agtagacaaa tatgtcaaga tgatatcata tatatgatgt 300

aagtaataag tatccttagt agatactgaa aactcagtaa atgtgcaaat acctatattg 360

aagtaggttg caaattctta cttgtaccca aaatagttca ctgaataaag accgtggaca 420

aaaaatggga cctttgagta ctgtaactaa ggtggaagat gagatcttct aagccagaat 480

aattccagct atttatcttg ccacctgcag g 511

<210> 79
<211> 690
<212> DNA
<213> Homo sapiens

<400> 79
ctgctgggta taagtctaag gtctgacaga agcctttgca gagacacctg ctgctttcag 60

gactgcctgg gactgaggga atcccccaca caaaaggctt ctgttggatt tctatgtgya 120

gttgagaatg cttaaatgac aagggaagaa aaccacaagt gaaagagaaa gacggactag 180

gagaagtaaa tggaaatacg gttgacaaaa ggctatgcca gtgcaaaaag tgagtatgaa 240

gcaccagcga aaagctggaa agcaaactca aaatggctag gaagaagacc aggcggcagt 300

atccacattt tctaaaacta ccgtgggagc catgattatc taaaatcccc gctagatatt 360

atcacttgaa acatcatgtc tcactctttt agtctgtgta agttaccagt ccttccagaa 420

taagtaggaa caattctgag cccctggcat gtaatggtat agctctatgc cagtcaattt 480

gtcagccctg gcacaacata agtgaataca ggcagccttg aaatgtgtac gttatgtaag 540

ctgcactttc tcctttctgt gctgaccaca ttccatagtg ttctagataa gcaaaggggc 600

atcctgtttg gttgtcagtg ctactcgatg ggtatgccat gaccccttta gagatgtgac 660

accaggcagg gcgcagtggc tcacacctgt 690

<210> 80
<211> 801
<212> DNA
<213> Homo sapiens

<400> 80
ttagggaatc agtatcacaa acaaaggctc agaagaaaga acacacaggg agtctggggg 60

aactgtcagc agttcagggt agagattaga gaaagaggag cacaggggca aaaagcaagc 120

ttggagcagt gagcagattc aatgactaag gcttgtgggg ttggggagaa cctttggctt 180

ttatccgagg acaatgtgca gcactggcag cactgaagtc aggaagaacc ttgatcagat 240

ctgcattcca gaatatcact ttggtgaagt gtgaagaatg aactgagaga tgctagactg 300

aatacatgga gaagaagagg ttcggggaaa ccctggcagg aactgtaggg agaatgttag 360

gatggaggaa aaggtagaaa ggaccctgag agatctgagt ratcaagacc cagtgtttca 420

cacatggaaa atgaggtgga aaaggagaaa ggtccccatg tgaacagcac tccatctgga 480

aaaatgacat aaaacaaggg aatttggccc atgacataag aggtccttgg gctcacatgg 540

tattgagtga tcagggagga gtttagttga gttcacctct acagacaggt attgagtgcc 600

aggtatgctg tcatcagggt cataaggaaa tcaaaggtat ctgcctcata tctttgtgac 660

ttacatgtct gatcctgctt aagaactatg ccaatccccg actttccagg acccccagta 720

attttgtcgt gtccatgtgg gaagtgagct gaggcttggc aagaggatct tagcccatat 780

ggtccaaaaa atagtagaaa t 801

<210> 81
<211> 801
<212> DNA
<213> Homo sapiens

<400> 81
ttcaaagaaa tctaagtgct ttttcttcct tccataggca aaagaggtct tccagaggtt 60

cctggagtca cagatggtga tagaggaatc catcttgcag tcagataaag ccctcactga 120

tagagagaag gcagtagcag gtatggggca gggctcagct tacacaggag gggtacgttt 180

atacaatgcc ctctaacaga tctaacagga aaaccttccc aaaatgagga atttcctctt 240

ctgtggaaca cacactctgc taaatcttaa aataacaagg ggagctatga ttgtacatca 300

gccagacagg cagagccttt cccatcacat tctcaaatgc gctcttaatg gtgtggacct 360

gggtgaattc tgtgatttct caccctgcct ttcagatttg rtttagtctc agctcagaaa 420

agtggagatg aatcttcacc tattattttc ttctctttct aataccttca tggagcagtg 480

gatcgggcca agaaggaggc agctgagaag gaacaggaac ttttaaaaca gaaattacag 540

gagcagcagc aacagatgga ggctcaagat aagagtcgca aggaaaacat agcccaactg 600

aaggagaagc tgcagatgga gagagaacac ctactgagag agcagattat gatgttggag 660

cacacgcaga aggtaagtct gcccttggcc tcagcaggcc agacggtcct cacccaggta 720

cctcaggaag ggctggtgaa ggttacagca acatcatgaa aggaagcaca tcaccatttg 780

ctgcttggca atcactaaaa g 801

<210> 82
<211> 1494
<212> DNA
<213> Homo sapiens

<400> 82
gacatcagaa ggtctataac ctagaagtaa agacaaaatg aaataaaaca gctccaaaat 60

caatcttcaa caggattaag gtgatctgca agtactcctt gctgccaaaa gaggtcttaa 120

ccctcttaga aagaagataa caccatacag actctctata attttaatgg aatcaataca 180

caaggtccag cgtccaacta maatgtatga agtatgttaa aaaaaagaaa aaactagtga 240

ccgaagccaa gcagaaacag agctacagat gattcagata ttagaattat taaacaggga 300

cattagaata actatattca agaaaagaga gaaaaacaat ttcagcaggt actaataatc 360

tatattaaaa atcaaatgga atgtataggt ctgaaaaata taacaagtga aattaggaat 420

tcaacagaaa gatttaatag aaacttagac agagaggaaa ttagagtttg taaactagaa 480

tacaggccag cttagagtgt ctagaccaga gcactgagag aaaaatatgg aagttaggga 540

gaagagtaga agagatatgt aggacatagt gaaaatgtct aacatgtaat tgaagttcca 600

caaagaaagg agaataaaca tgacacagaa acaatatttg aagaaaaact agttgagagt 660

ttctaaaact gaaagacatc aaaccacaga ttcattaaac tttaaaaacc aaacaggata 720

aagaaaatta tatctaagca caagatagta aaacatacta aaaatcaaag acagagaaac 780

tcttaaaaag tcagaggtaa aaagacacat tgacttcaaa gaagcagtaa taattcttgg 840

ggcttatata tcaatattga tttttcagt ggacaccaga gaacaatgaa aaacagcttt 900

taaatactgg aagaaaatga ctagtcacct ataattttat agcagtcaaa atattcctca 960

aaaattagag tgataaaaca tcttattatc ccagaaataa agacatgctc aagaaattgt 1020

aataagacat gggaaccagt ctgaatgggc tcccactggc caaatccagg ataattcaaa 1080

```
caccaaataa ataataacag taagagattg tacttcattg aataataatt tgcgaatcca     1140
tacaaatagt aaatacatta ttgaattaat taaagtagaa tgttaactaa aaatgtagag     1200
gaaattatgg agttaaaaaa taccatttca caaccatcat agtagtaatt tatcgagcag     1260
gaatcatcaa tggatgatga aactacttgg tgaaaatttg atgagaaaat gggatattta     1320
cataatctca aaatatcttc ccacaaatta cttattaatt acagagacat aatgtgcctt     1380
ctaatgtgat gcactgataa ggaaggatac attacttctg tgttatttct gctaaaaatg     1440
tataacttga atctaatcat aagaaaaagt gaggattagt ctgaaaaaca tctg           1494
```